# EUROPEAN PATENT APPLICATION

(11) **EP 4 745 134 A1**
(43) Date of publication of application: **20.05.2026**
(21) Application number: 24842316.2
(22) Date of filing: 12.07.2024
(51) Int. Cl.: C07D 401/14, C07D 401/12, C07D 471/04, C07D 491/04, C07D 495/04, A61K 31/4355, A61K 31/4365, A61K 31/437, A61P 35/00

(54) **PROTEIN ARGINASE METHYLTRANSFERASE-5 INHIBITOR AND MEDICAL USE THEREOF**

(30) Priority: 14.07.2023 CN 202310864981
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: WU, Liang, Taizhou, Jiangsu 225316 (CN); ZHANG, Runtao, Taizhou, Jiangsu 225316 (CN); WANG, Tielin, Taizhou, Jiangsu 225316 (CN); LI, Lanli, Taizhou, Jiangsu 225316 (CN); QIN, Yuhua, Taizhou, Jiangsu 225316 (CN)
(74) Representative: Regimbeau
(86) International application number: PCT/CN2024/105252
(87) International publication number: WO 2025/016323

(57) **Abstract**

The present invention relates to a protein arginase methyltransferase-5 inhibitor and the medical use thereof. Specifically, the present invention relates to a compound as represented by general formula (I), a preparation method therefor, a pharmaceutical composition containing same, and the use thereof as a PRMT5 (protein arginase methyltransferase-5) inhibitor in the treatment of diseases related to PRMT5 activity. The definition of each group in general formula (I) is the same as defined in the description.

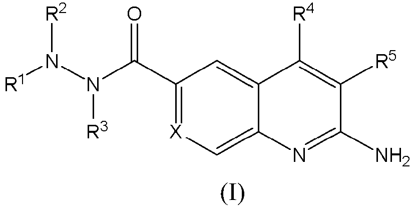

## Description

### FIELD OF THE INVENTION

The present invention relates to the field of pharmaceutical technology, and specifically relates to a new protein arginase methyltransferase-5 inhibitor, a pharmaceutical composition containing the same, a preparation method therefor, and a use thereof as a PRMT5 (protein arginase methyltransferase-5) inhibitor in the treatment of diseases related to PRMT5 activity.

### BACKGROUND OF THE INVENTION

The treatment of cancer can be broadly divided into two categories of cytotoxic therapy and targeted therapy. The cytotoxic therapy may result in extensive toxicity, while the targeted therapy has the advantage of selectively targeting tumor cells.

PRMT5 (protein arginase methyltransferase-5) is a type II arginine methyltransferase, which symmetrically dimethylate proteins involved in transcription and signal transduction by catalyzing the transmethylation reaction of S-adenosylmethionine (SAM), thereby achieving various essential cellular functions such as regulation of cell cycle progression, apoptosis and DMA damage response. PRMT5 is overexpressed in various cancers including glioblastoma, leukemia/lymphoma, prostate cancer and colorectal cancer, and is associated with poor prognosis. The data from genome-wide genetic perturbation screening using shRNA have revealed the importance of PRMT5 activity for cancer cell lines with MTAP (methylthioadenosine phosphorylase) delection (Kruykov et al, 2016; Marjon et al, 2016 and Markarov et al, 2016).

The first-generation PRMT5 inhibitors are mainly SAM competitive or non-competitive inhibitors, and lack selectivity towards cells with MTAP delection. The inhibition of PRMT5 in normal cells leads to dose-limiting toxicity, such as thrombocytopenia, anemia, and neutropenia.

MTAP delection causes the accumulation of methylthioadenosine (MTA) as a cellular metabolite in cancer cells. MTA, an intermediate in the methionine compensation pathway, has a weak inhibitory effect on PRMT5 and can form a PRMT5/MTA complex by competition with SAM. Novel inhibitors are developed, which may bind to and stabilize the PRMT5/MTA complex to enhance the inhibitory effect of MTA on PRMT5, and achieve the retention of PRMT5 activity in wild-type cells while inhibiting PRMT5 activity in cancer cells with MTAP delection, thereby effectively improving the therapeutic index.

At present, the second-generation PRMT5 inhibitors have been disclosed in published patent applications including WO2021163344, WO2022115377, WO2022132914, WO2022169948, WO2022192745, WO2021050915, WO2023278564 and WO2022026892.

### SUMMARY OF THE INVENTION

After serious study, the inventors have designed and synthesized a series of carbohydrazide compounds, which exhibit inhibitory activity against PRMT5 (protein arginase methyltransferase-5) and can be developed into drugs for preventing or treating diseases related to PRMT5 activity.

Therefore, an objective of the present invention is to provide a compound of general formula (I) or a tautomer, mesomer, racemate, enantiomer, diastereomer thereof, or a mixture thereof, or an oxynitride thereof, or a pharmaceutically acceptable salt thereof, 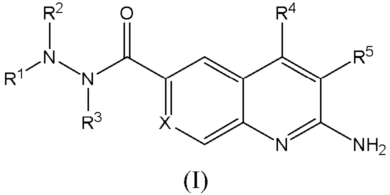 wherein:
X is CR⁶ or N;
R¹ and R² are each independently selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, -B(OH)₂, alkyl, -OR^{a}, haloalkyl, haloalkoxy, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R³ is selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
or, R¹ and R², or R¹ and R³, together with the nitrogen atom to which they are attached, form a nitrogen-containing heterocyclyl or nitrogen-containing heteroaryl, and the nitrogen-containing heterocyclyl or nitrogen-containing heteroaryl is optionally further substituted by one or more groups selected from the group consisting of halogen, -NR^{a}R^{b}, nitro, cyano, hydroxy, thiol, carboxyl, oxo, methylene, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -(CH2)_{q}-OR^{a}, alkyl, alkoxy, haloalkyl, haloalkoxy, deuterated alkyl, deuterated alkoxy, alkenyl, alkynyl, cycloalkyl, heterocyclyl, aryl and heteroaryl;
R⁴ and R³ are each independently selected from the group consisting of hydrogen, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -CH₂-R⁷, -NR^{a}R^{b}, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxyl, ester, amido, oxo, alkyl, alkoxy, haloalkyl, haloalkoxy, alkenyl, alkynyl, cycloalkyl, heterocyclyl, aryl and heteroaryl;
or, R⁴ and R⁵, together with the atoms to which they are attached, form a heteroaryl, heterocyclyl, aryl or cycloalkyl, and the heteroaryl, heterocyclyl, aryl or cycloalkyl is optionally further substituted by one or more groups selected from the group consisting of halogen, amino, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R⁶ is selected from the group consisting of hydrogen, halogen, amino, nitro, cyano, hydroxy, thiol, carboxyl, ester, oxo, -NR^{a}R^{b}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, ester, oxo, alkyl, alkoxy, haloalkyl, haloalkoxy, alkenyl, alkynyl, cycloalkyl, heterocyclyl, aryl and heteroaryl;
R⁷ is selected from the group consisting of heteroaryl, aryl, heterocyclyl and cycloalkyl, and the heteroaryl, aryl, heterocyclyl and cycloalkyl are optionally further substituted by one or more groups selected from the group consisting of deuterium, halogen, amino, -NR^{a}R^{b}, nitro, cyano, hydroxy, thiol, carboxyl, oxo, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, oxo, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, -OR^{a}, -(CH2)_{q}-OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxyl, -NR^{c}R^{d}, -C(=O)OR^{c}, -C(=O)R^{c}, -C(=O)NR^{c}R^{d}, -OC(=O)R^{c}, -NR^{c}C(=O)R^{d}, -S(=O)₂R^{c}, -NR^{c}S(=O)₂R^{d}, -S(=O)₂NR^{c}R^{d}, -S(=O)R^{c}, -P(=O)R^{c}R^{d}, -NR^{c}S(=O)₂R^{d}, alkyl, alkoxy, alkenyl, alkynyl, cycloalkyl, heterocyclyl, aryl and heteroaryl;
or R^{a} and R^{b}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
R^{c} and R^{d} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxyl, alkyl, alkoxy, alkenyl, alkynyl, cycloalkyl, heterocyclyl, aryl and heteroaryl;
or R^{c} and R^{d}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
p is an integer from 1 to 6;
q is an integer from 1 to 6.

In a preferred embodiment, provided is the compound of general formula (I) or a tautomer, mesomer, racemate, enantiomer, diastereomer thereof, or a mixture thereof, or an oxynitride thereof, or a pharmaceutically acceptable salt thereof according to the present invention, wherein,
X is CR⁶ or N;
R¹ is selected from the group consisting of hydrogen, C₁₋₆ alkyl, C₂₋₆ alkenyl, C₂₋₆ alkynyl, C₃₋₆ cycloalkyl and 4 to 6 membered heterocyclyl, wherein the C₁₋₆ alkyl, C₂₋₆ alkenyl, C₂₋₆ alkynyl, C₃₋₆ cycloalkyl and 4 to 6 membered heterocyclyl are optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy;
R² is selected from the group consisting of hydrogen, C₁₋₆ alkyl, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₁₋₆ alkyl, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, -NR^{a}R^{b}, -S(=O)₂R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R³ is selected from the group consisting of hydrogen, -(CH₂)ₚ-R⁷, C₁₋₆ alkyl, C₂₋₆ alkenyl, C₂₋₆ alkynyl, C₃₋₆ cycloalkyl and 4 to 10 membered heterocyclyl, wherein the C₁₋₆ alkyl, C₂₋₆ alkenyl, C₂₋₆ alkynyl, C₃₋₆ cycloalkyl and 4 to 10 membered heterocyclyl are optionally further substituted by one or more groups selected from the group consisting of deuterium, halogen, amino, nitro, cyano, hydroxy, thiol, carboxyl, C₁₋₆ alkyl, C₁₋₆ alkoxy, C₁₋₆ haloalkyl and C₁₋₆ haloalkoxy;
or, R¹ and R², together with the nitrogen atom to which they are attached, form a 5 to 10 membered nitrogen-containing heterocyclyl or 5 to 10 membered nitrogen-containing heteroaryl, and the 5 to 10 membered nitrogen-containing heterocyclyl or 5 to 10 membered nitrogen-containing heteroaryl is optionally further substituted by one or more groups selected from the group consisting of halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy;
or, R¹ and R³, together with the nitrogen atom to which they are attached, form a 5 to 10 membered nitrogen-containing heterocyclyl, and the 5 to 10 membered nitrogen-containing heterocyclyl is optionally substituted by one or more groups selected from the group consisting of halogen, hydroxy, nitro, cyano, hydroxy, thiol, carboxyl, oxo, methylene, -(CH2)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, C₆₋₁₀ aryl and 5 to 10 membered heteroaryl;
R⁴ is selected from the group consisting of hydrogen, C₁₋₆ alkyl and C₃₋₆ cycloalkyl;
R⁵ is selected from the group consisting of hydrogen, halogen, amino, nitro, cyano, hydroxy, C₁₋₆ alkyl, C₁₋₆ alkoxy, C₁₋₆ haloalkyl, C₁₋₆ haloalkoxy, C₁₋₆ deuterated alkyl and C₁₋₆ deuterated alkoxy;
wherein, R⁴ and R⁵ are not H simultaneously;
or, R⁴ and R⁵, together with the atoms to which they are attached, form a 5 to 6 membered heteroaryl, 5 to 6 membered heterocyclyl, phenyl or 5 to 6 membered cycloalkyl, and the 5 to 6 membered heteroaryl, 5 to 6 membered heterocyclyl, phenyl or 5 to 6 membered cycloalkyl is optionally further substituted by one or more groups selected from the group consisting of halogen, amino, nitro, cyano, hydroxy, C₁₋₆ alkyl, C₁₋₆ alkoxy, C₁₋₆ haloalkyl, C₁₋₆ haloalkoxy, C₂₋₆ alkenyl and C₂₋₆ alkynyl;
R⁶ is selected from the group consisting of hydrogen, halogen, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and C₃₋₆ cycloalkyl;
R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, oxo, -NR^{a}R^{b}, -(CH2)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
R^{a} and R^{b} are each independently selected from the group consisting of hydrogen, halogen, C₁₋₆ alkyl, C₂₋₆ alkenyl, C₂₋₆ alkynyl and C₃₋₆ cycloalkyl, wherein the C₁₋₆ alkyl, C₂₋₆ alkenyl, C₂₋₆ alkynyl and C₃₋₆ cycloalkyl are optionally further substituted by one or more groups selected from the group consisting of halogen, amino, nitro, cyano, hydroxy, -NR^{c}R^{d}, C₁₋₆ alkyl, C₁₋₆ alkoxy, C₁₋₆ haloalkyl, C₁₋₆ haloalkoxy, C₂₋₆ alkenyl and C₂₋₆ alkynyl;
or R^{a} and R^{b}, together with the atoms to which they are attached, form a 4 to 6 membered heterocyclyl, and the 4 to 6 membered heterocyclyl is optionally substituted by halogen, oxo or C₁₋₆ alkyl;
R^{c} and R^{d} are each independently selected from the group consisting of hydrogen, halogen, hydroxy, C₁₋₆ alkyl, C₁₋₆ alkoxy, C₁₋₆ haloalkyl, C₁₋₆ haloalkoxy, C₂₋₆ alkenyl and C₂₋₆ alkynyl;
p is an integer from 1 to 6;
q is an integer from 1 to 6.

In a preferred embodiment, provided is the compound of general formula (I) or a tautomer, mesomer, racemate, enantiomer, diastereomer thereof, or a mixture thereof, or an oxynitride thereof, or a pharmaceutically acceptable salt thereof according to the present invention, wherein,
X is CR⁶;
R¹ is selected from the group consisting of hydrogen, C₁₋₆ alkyl and C₃₋₆ cycloalkyl, wherein the C₁₋₆ alkyl, C₂₋₆ alkenyl, C₂₋₆ alkynyl, C₃₋₆ cycloalkyl and 4 to 6 membered heterocyclyl are optionally further substituted by one or more groups selected from the group consisting of deuterium, halogen, C₁₋₆ alkoxy and C₁₋₆ haloalkoxy;
R² is selected from the group consisting of C₆₋₁₀ aryl and 5 to 10 membered heteroaryl, wherein the C₁₋₆ alkyl, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, -NR^{a}R^{b}, -S(=O)₂R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R³ is -(CH₂)ₚ-R⁷, wherein R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, oxo, -NR^{a}R^{b}, -(CH2)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
or, R¹ and R², together with the nitrogen atom to which they are attached, form a 5 to 10 membered nitrogen-containing heterocyclyl or 5 to 10 membered nitrogen-containing heteroaryl, and the 5 to 10 membered nitrogen-containing heterocyclyl or 5 to 10 membered nitrogen-containing heteroaryl is optionally further substituted by one or more groups selected from the group consisting of halogen, amino, nitro, cyano, hydroxy, thiol, carboxyl, oxo, C₁₋₆ alkyl, C₁₋₆ alkoxy, C₁₋₆ haloalkyl and C₁₋₆ haloalkoxy;
or, R¹ and R³, together with the nitrogen atom to which they are attached, form a 5 to 10 membered nitrogen-containing heterocyclyl, and the 5 to 10 membered nitrogen-containing heterocyclyl is optionally substituted by one or more groups selected from the group consisting of halogen, hydroxy, nitro, cyano, hydroxy, thiol, oxo, methylene, -(CH2)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and C₆₋₁₀ aryl;
R⁴ is hydrogen;
R⁵ is C₁₋₆ alkyl;
or, R⁴ and R⁵, together with the atoms to which they are attached, form a 5 to 6 membered heteroaryl, 5 to 6 membered heterocyclyl, phenyl or 5 to 6 membered cycloalkyl, and the 5 to 6 membered heteroaryl, 5 to 6 membered heterocyclyl, phenyl or 5 to 6 membered cycloalkyl is optionally further substituted by one or more groups selected from the group consisting of halogen and C₁₋₆ alkyl;
R⁶ is selected from the group consisting of hydrogen, halogen and C₁₋₆ alkyl;
R^{a} and R^{b} are each independently selected from the group consisting of hydrogen, halogen, C₁₋₆ alkyl and C₃₋₆ cycloalkyl, wherein the C₁₋₆ alkyl and C₃₋₆ cycloalkyl are optionally further substituted by one or more groups selected from the group consisting of halogen, amino, -NR^{c}R^{d}, C₁₋₆ alkyl, C₁₋₆ alkoxy, C₁₋₆ haloalkyl, C₁₋₆ haloalkoxy, C₂₋₆ alkenyl and C₂₋₆ alkynyl;
or R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen, halogen, hydroxy, C₁₋₆ alkyl, C₁₋₆ alkoxy, C₁₋₆ haloalkyl, C₁₋₆ haloalkoxy, C₂₋₆ alkenyl and C₂₋₆ alkynyl;
p is an integer from 1 to 6;
q is an integer from 1 to 6.

In a preferred embodiment, provided is the compound of general formula (I) or a tautomer, mesomer, racemate, enantiomer, diastereomer thereof, or a mixture thereof, or an oxynitride thereof, or a pharmaceutically acceptable salt thereof according to the present invention, which is a compound of general formula (II) or a tautomer, mesomer, racemate, enantiomer, diastereomer thereof, or a mixture thereof, or an oxynitride thereof, or a pharmaceutically acceptable salt thereof, 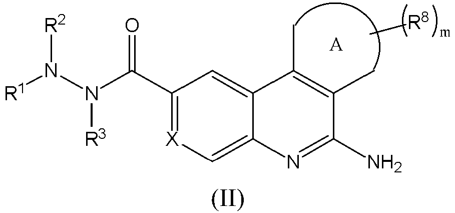
wherein, ring A is selected from the group consisting of heteroaryl, heterocyclyl, aryl and cycloalkyl;
each R⁸ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
m is an integer from 0 to 4;
X, R¹ to R³, R^{a} and R^{b} are as defined in general formula (I).

In another preferred embodiment, provided is the compound of general formula (I) or a tautomer, mesomer, racemate, enantiomer, diastereomer thereof, or a mixture thereof, or an oxynitride thereof, or a pharmaceutically acceptable salt thereof according to the present invention, wherein R⁴ is selected from the group consisting of hydrogen, C₁₋₆ alkyl and C₃₋₆ cycloalkyl.

In another preferred embodiment, provided is the compound of general formula (I) or a tautomer, mesomer, racemate, enantiomer, diastereomer thereof, or a mixture thereof, or an oxynitride thereof, or a pharmaceutically acceptable salt thereof according to the present invention, wherein R⁵ is selected from the group consisting of halogen, cyano, C₁₋₆ alkyl, C₁₋₆ alkoxy, C₃₋₆ cycloalkyl and C₂₋₆ alkenyl, and the C₁₋₆ alkyl is optionally substituted by deuterium, halogen, hydroxy or C₁₋₆ alkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R¹ is selected from the group consisting of hydrogen, C₁₋₆ alkyl, C₃₋₆ cycloalkyl and 4 to 6 membered heterocyclyl, wherein the C₁₋₆ alkyl, C₃₋₆ cycloalkyl and 4 to 6 membered heterocyclyl are optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R¹ is selected from the group consisting of C₁₋₆ alkyl, C₁₋₆ deuterated alkyl, C₃₋₆ cycloalkyl and 4 to 6 membered heterocyclyl, wherein the C₁₋₆ alkyl is optionally substituted by C₁₋₆ alkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R² is selected from the group consisting of C₁₋₆ alkyl, C₃₋₆ cycloalkyl, 4 to 6 membered heterocyclyl, C₆₋₁₀ aryl and 5 to 10 membered heteroaryl, wherein the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, ester, oxo, -S(=O)₂R^{a}, -S(=O)R^{a}, -C(=O)R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl and C₂₋₆ alkynyl;
R^{a} and R^{b} are each independently selected from the group consisting of hydroxy and C₁₋₆ alkyl.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R² is selected from the group consisting of C₆₋₁₀ aryl and 5 to 10 membered heteroaryl, wherein the C₆₋₁₀ aryl or 5 to 10 membered heteroaryl is optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R² is selected from the group consisting of C₃₋₆ cycloalkyl and 4 to 6 membered heterocyclyl, wherein the C₃₋₆ cycloalkyl or 4 to 6 membered heterocyclyl is optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R² is selected from the group consisting of C₆₋₁₀ aryl and 5 to 10 membered heteroaryl, wherein the C₆₋₁₀ aryl or 5 to 10 membered heteroaryl is optionally further substituted by one or more groups selected from the group consisting of deuterium, halogen, cyano, carboxyl, -S(=O)₂R^{a}, -S(=O)R^{a}, -C(=O)R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl or 5 to 10 membered heteroaryl is optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein R² is selected from the group consisting of phenyl, pyridyl, pyrimidinyl, pyrazinyl and pyridazinyl, which is optionally further substituted by one or more groups selected from the group consisting of halogen.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R¹ and R², together with the nitrogen atom to which they are attached, form a 5 to 10 membered nitrogen-containing heterocyclyl or 5 to 10 membered nitrogen-containing heteroaryl, and the 5 to 10 membered nitrogen-containing heterocyclyl or 5 to 10 membered nitrogen-containing heteroaryl is optionally further substituted by one or more groups selected from the group consisting of halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R¹ and R², together with the nitrogen atom to which they are attached, form pyrrolidinyl, piperidyl, piperazinyl, morpholinyl, thiomorpholinyl, pyridopyrrolyl, pyridopyrrolidinyl, pyridopyrrolidonyl, pyridoimidazolyl, pyridoimidazolonyl, pyridopiperidyl, pyridomorpholinyl, pyridopiperazinyl, pyridopiperidonyl, pyridomorpholinonyl, pyrimidopyrrolidonyl, pyrimidoimidazolyl, pyrimidoimidazolonyl, pyrimidopiperidyl, pyrimidomorpholinyl, pyrimidopiperazinyl, pyrimidopiperidonyl, pyrimidomorpholinonyl, pyridazinopyrrolidonyl, pyridazinoimidazolyl, pyridazinoimidazolonyl, pyridazinopiperidyl, pyridazinomorpholinyl, pyridazinopiperazinyl, pyridazinopiperidonyl, pyridazinomorpholinonyl and benzopyrrolyl, which is optionally further substituted by one or more groups selected from the group consisting of halogen, amino, nitro, cyano, hydroxy, thiol, carboxyl, ester, oxo, C₃₋₆ cycloalkyl, 4 to 6 membered heterocyclyl, C₆₋₁₀ aryl, 5 to 10 membered heteroaryl, C₁₋₆ alkyl, C₁₋₆ alkoxy, C₁₋₆ haloalkyl and C₁₋₆ haloalkoxy.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R³ is selected from the group consisting of hydrogen, -(CH₂)ₚ-R⁷, -NR^{a}R^{b}, -C(=O)R^{a}, -S(=O)₂R^{a}, C₁₋₆ alkyl, C₃₋₆ cycloalkyl, 4 to 10 membered heterocyclyl, C₆₋₁₀ aryl and 5 to 10 membered heteroaryl, wherein the C₁₋₆ alkyl, C₃₋₆ cycloalkyl, 4 to 10 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, C₁₋₆ alkyl, -OR^{a}, C₁₋₆ haloalkyl and C₁₋₆ haloalkoxy;
R⁷ is selected from the group consisting of 5 to 6 membered heteroaryl and C₆₋₁₀ aryl, and the 5 to 6 membered heteroaryl and C₆₋₁₀ aryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, C₁₋₆ alkyl, C₁₋₆ alkoxy, C₁₋₆ haloalkyl and C₁₋₆ haloalkoxy;
p is an integer from 1 to 6;
R^{a} and R^{b} are as defined in general formula (I).

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R³ is -CH₂-R⁷, wherein R⁷ is selected from the group consisting of 5 to 6 membered heteroaryl and C₆₋₁₀ aryl, and the 5 to 6 membered heteroaryl or C₆₋₁₀ aryl is optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy; preferably, R⁷ is phenyl or pyridyl, which is optionally substituted by C₁₋₆ alkyl or C₁₋₆ haloalkyl.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R³ is -(CH₂)ₚ-R⁷; R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, oxo, -NR^{a}R^{b}, -(CH2)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen, C₁₋₆ alkyl and hydroxy substituted C₁₋₆ alkyl;
p is 1;
q is 1 or 2.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein, R¹ and R³, together with the nitrogen atom to which they are attached, form a 5 to 10 membered nitrogen-containing heterocyclyl, and the 5 to 10 membered nitrogen-containing heterocyclyl is optionally substituted by one or more groups selected from the group consisting of halogen, hydroxy, thiol, oxo, methylene, -(CH2)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, C₆₋₁₀ aryl and 5 to 10 membered heteroaryl;
R^{a} is hydrogen or C₁₋₆ alkyl;
q is 1 or 2.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein,
R¹ is selected from the group consisting of hydrogen, C₁₋₆ alkyl and C₃₋₆ cycloalkyl;
R² is phenyl or 5 to 6 membered heterocyclyl, preferably phenyl or 6 membered heterocyclyl, and optionally further substituted by one or more groups selected from the group consisting of deuterium, halogen, C₁₋₆ alkyl, C₁₋₆ alkoxy, C₁₋₆ haloalkyl and C₁₋₆ haloalkoxy;
R³ is selected from the group consisting of hydrogen, C₁₋₆ alkyl, C₁₋₆ haloalkyl, C₃₋₆ cycloalkyl and 4 to 6 membered heterocyclyl.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, wherein,
R¹ is selected from the group consisting of hydrogen, C₁₋₆ alkyl and C₃₋₆ cycloalkyl;
R² is selected from the group consisting of hydrogen, C₁₋₆ alkyl and C₃₋₆ cycloalkyl; and the C₁₋₆ alkyl is optionally substituted by hydroxy or C₁₋₆ alkoxy;
R³ is -(CH₂)ₚ-R⁷;
R⁷ is a 5 to 6 membered heteroaryl or phenyl, preferably 6 membered heteroaryl or phenyl, which is optionally further substituted by one or more groups selected from the group consisting of deuterium, halogen, C₁₋₆ alkyl, C₁₋₆ alkoxy, C₁₋₆ haloalkyl and C₁₋₆ haloalkoxy;
p is 1.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, which is a compound of general formula (IA) or (IIA) or a tautomer, mesomer, racemate, enantiomer, diastereomer thereof, or a mixture thereof, or an oxynitride thereof, or a pharmaceutically acceptable salt thereof, 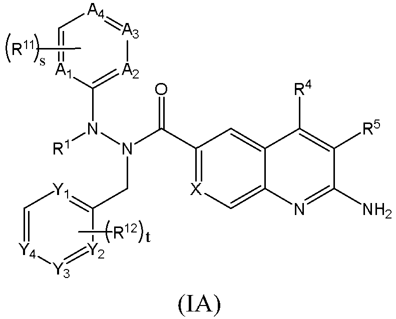 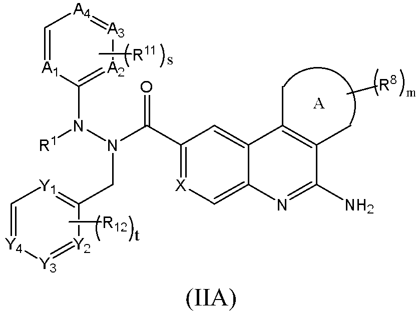 wherein,
A₁, A₂, A₃ and A₄ are each independently selected from the group consisting of CH and N, and preferably, one of them is N and the others are CH, or two of them are N and the others are CH;
Y₁, Y₂, Y₃ and Y₄ are each independently selected from the group consisting of CH and N, and preferably, one of them is N and the others are CH, or two of them are N and the others are CH;
ring A is selected from the group consisting of 5 to 6 membered heteroaryl, 5 to 6 membered heterocyclyl, phenyl and 5 to 6 membered cycloalkyl;
R¹ is selected from the group consisting of hydrogen, C₁₋₆ alkyl, C₃₋₆ cycloalkyl and 4 to 6 membered heterocyclyl, wherein the C₁₋₆ alkyl, C₃₋₆ cycloalkyl or 4 to 6 membered heterocyclyl is optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy;
R⁴ is selected from the group consisting of hydrogen, C₁₋₆ alkyl and C₃₋₆ cycloalkyl;
R⁵ is selected from the group consisting of halogen, cyano, C₁₋₆ alkyl, C₁₋₆ alkoxy, C₃₋₆ cycloalkyl and C₂₋₆ alkenyl, and the C₁₋₆ alkyl is optionally substituted by deuterium, halogen, hydroxy or C₁₋₆ alkoxy;
each R¹¹ is independently selected from the group consisting of hydrogen, deuterium, halogen, cyano, C₁₋₆ alkyl, C₁₋₆ alkoxy, C₁₋₆ haloalkyl, C₁₋₆ haloalkoxy, C₂₋₆ alkenyl, C2-6 alkynyl, -S(=O)₂R^{a}, -S(=O)R^{a}, -C(=O)R^{a}, -P(=O)R^{a}R^{b}, -B(OH)₂, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy; or,
any two adjacent R¹¹, together with the atoms to which they are attached, form a phenyl, 5 to 6 membered heteroaryl, 5 to 6 membered heterocyclyl or 4 to 6 membered cycloalkyl, and the phenyl, 5 to 6 membered heteroaryl, 5 to 6 membered heterocyclyl or 4 to 6 membered cycloalkyl is optionally substituted by a group(s) selected from the group consisting of halogen and C₁₋₆ alkyl;
each R¹² is independently selected from the group consisting of hydrogen, deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, amino, oxo, -NR^{a}R^{b}, -(CH2)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl; or
any two adjacent R¹², together with the atoms to which they are attached, form a phenyl, 5 to 6 membered heteroaryl, 5 to 6 membered heterocyclyl or 4 to 6 membered cycloalkyl, and the phenyl, 5 to 6 membered heteroaryl, 5 to 6 membered heterocyclyl or 4 to 6 membered cycloalkyl is optionally substituted by a group(s) selected from the group consisting of halogen, C₁₋₆ alkyl and 4 to 6 membered heterocyclyl, and the 4 to 6 membered heterocyclyl is optionally substituted by C₁₋₆ alkyl;
each R⁸ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by one or more groups selected from the group consisting of halogen, oxo and C₁₋₆ alkyl;
R^{c} and R^{d} are each independently selected from the group consisting of hydrogen, C₁₋₆ alkyl and hydroxy substituted C₁₋₆ alkyl;
m is an integer from 0 to 4;
q is 1 or 2.
s is an integer from 1 to 4; and preferably 1 or 2;
t is an integer from 1 to 4, and preferably 1, 2 or 3;
X is as defined in general formula (I).

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein, 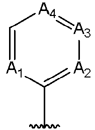 is selected from the group consisting of pyrimidinyl, pyridyl, phenyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl, preferably, is selected from the group consisting of 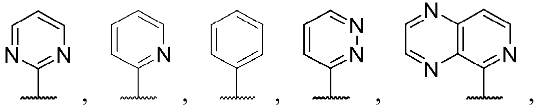 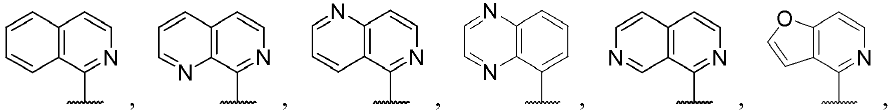 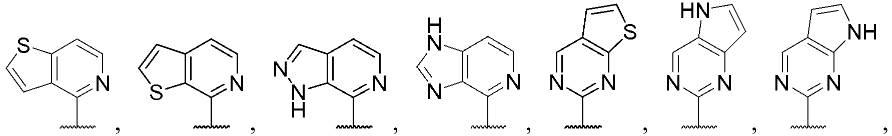 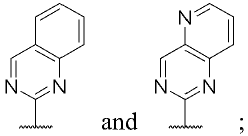 and more preferably, is

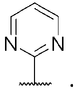

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein, 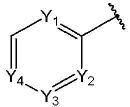 is selected from the group consisting of pyridyl, phenyl, pyrimidinyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl, preferably, is selected from the group consisting of 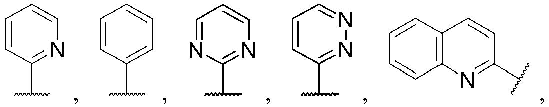 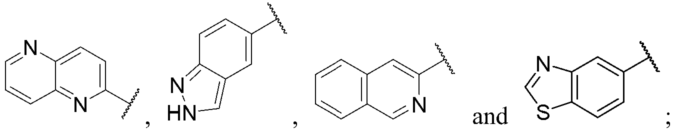 and more preferably, is selected from the group consisting of

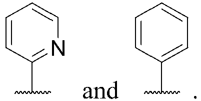

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein, R¹ is selected from the group consisting of hydrogen, C₁₋₆ alkyl and C₃₋₆ cycloalkyl, wherein the C₁₋₆ alkyl is optionally substituted by one or more groups selected from the group consisting of deuterium, halogen and C₁₋₆ alkoxy.

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein, each R¹¹ is each independently selected from the group consisting of hydrogen, deuterium, halogen, cyano, carboxyl, C₁₋₆ alkyl, C₁₋₆ alkoxy, C₁₋₆ haloalkyl, C₁₋₆ haloalkoxy, C₂₋₆ alkenyl, C₂₋₆ alkynyl, -P(=O)R^{a}R^{b} and -B(OH)₂;
R^{a} and R^{b} are each independently selected from the group consisting of hydrogen and C₁₋₆ alkyl;
s is 1 or 2.

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein, each R¹² is independently selected from the group consisting of hydrogen, deuterium, halogen, -NR^{a}R^{b}, cyano, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl and 4 to 6 membered heterocyclyl are optionally further substituted by one or more groups selected from the group consisting of deuterium, halogen, hydroxy and C₁₋₆ alkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by halogen;
t is 1, 2 or 3;
preferably, R¹² is C₁₋₆ haloalkyl, and t is 1.

In another preferred embodiment, provided is the compound of general formula (IA) or general formula (IIA) or a tautomer, mesomer, racemate, enantiomer, diastereomer thereof, or a mixture thereof, or an oxynitride thereof, or a pharmaceutically acceptable salt thereof according to the present invention, wherein 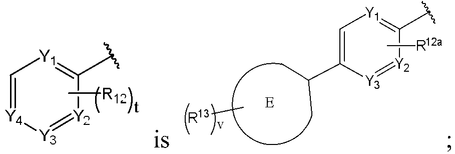 wherein,
Y₁, Y₂ and Y₃ are each independently CH or N;
ring E is selected from the group consisting of 5 to 10 membered heteroaryl and 5 to 10 membered heterocyclyl; preferably selected from the group consisting of pyridyl, dihydropyridyl, tetrahydropyridyl, phenyl, pyrazinyl, pyrimidinyl, furanyl, dihydrofuranyl, tetrahydrofuranyl, oxazolyl, isoxazolyl, pyrrolyl, pyrazolyl, imidazolyl, triazolyl, tetrazolyl, thiazolyl, thiadiazolyl, thienyl, pyranyl, dihydropyranyl, tetrahydropyranyl, piperidyl, morpholinyl, pyridopyrazolyl and quinolyl;
R^{12a} is selected from the group consisting of hydrogen and halogen, preferably hydrogen;
each R¹³ is independently selected from the group consisting of hydrogen, deuterium, halogen, cyano, hydroxy, oxo, -NR^{a}R^{b}, -(CH2)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}, or
R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen and C₁₋₆ alkyl;
q is 1 or 2, preferably 1;
t is 1, 2 or 3;
v is 1 or 2.

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, which is a compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof, 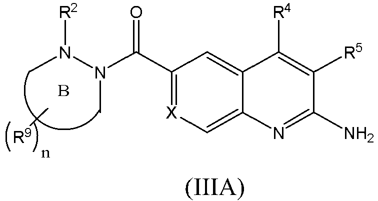 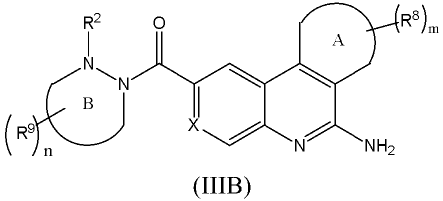 wherein,
ring B is selected from the group consisting of 5 to 10 membered nitrogen-containing heterocyclyl and 5 to 10 membered nitrogen-containing heteroaryl;
each R⁹ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -(CH2)_{q}-OR^{a}, oxo, methylene, alkyl, alkoxy, haloalkyl, haloalkoxy, deuterated alkyl, deuterated alkoxy, alkenyl, alkynyl, cycloalkyl, heterocyclyl, aryl and heteroaryl;
n is an integer from 0 to 4;
q is 1 or 2;
R² is selected from the group consisting of hydrogen, -(CH₂)ₚ-R⁷, -NR^{a}R^{b}, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, -B(OH)₂, alkyl, -OR^{a}, haloalkyl, haloalkoxy, alkenyl, alkynyl, cycloalkyl, heterocyclyl, aryl and heteroaryl, and the alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
X, R⁴, R⁵, R⁷, R^{a}, R^{b} and p are as defined in general formula (I);
ring A, R⁸ and m are as defined in general formula (II).

In another preferred embodiment, provided is the compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof according to the present invention, wherein, ring B is a 5 to 10 membered nitrogen-containing heterocyclyl;
each R⁹ is independently selected from the group consisting of halogen, hydroxy, thiol, oxo, methylene, -(CH2)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, and phenyl;
n is an integer from 0 to 4;
q is 1 or 2;
R² is selected from the group consisting of C₆₋₁₀ aryl and 5 to 10 membered heteroaryl, wherein the C₆₋₁₀ aryl or 5 to 10 membered heteroaryl is optionally further substituted by one or more groups selected from the group consisting of deuterium, halogen, amino, nitro, cyano, hydroxy, thiol, carboxyl, -NR^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl or 5 to 10 membered heteroaryl is optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl;
X, R⁴ and R⁵ are as defined in general formula (I) or (II) above;
ring A, R⁸ and m are as defined in general formula (I) or (II) above.

In another preferred embodiment, provided is the compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof according to the present invention, wherein, ring B is a 5 to 10 membered nitrogen-containing heterocyclyl, and preferably 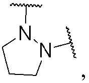 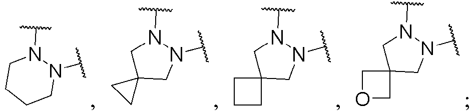
each R⁹ is independently selected from the group consisting of halogen, hydroxy, thiol, oxo, methylene, -(CH2)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and phenyl;
R^{a} is hydrogen or C₁₋₆ alkyl;
q is 1 or 2; and preferably 1;
n is 1 or 2.

In another preferred embodiment, provided is the compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof according to the present invention, wherein, R² is selected from the group consisting of C₆₋₁₀ aryl and 5 to 10 membered heteroaryl, preferably is selected from the group consisting of phenyl, pyridyl, pyrimidinyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl; and more preferably, is selected from the group consisting of phenyl and pyridyl;
which is optionally further substituted by one or more groups selected from the group consisting of deuterium, halogen, cyano, -NR^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl or 5 to 10 membered heteroaryl is optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl.

In another preferred embodiment, provided is the compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof according to the present invention, which is a compound of general formula (IIIAa) or general formula (IIIBa) or a tautomer, mesomer, racemate, enantiomer, diastereomer thereof, or a mixture thereof, or an oxynitride thereof, or a pharmaceutically acceptable salt thereof, 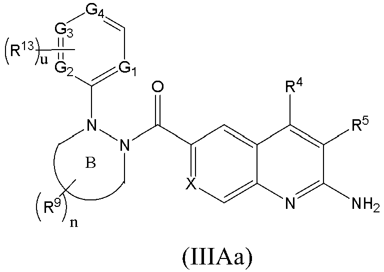 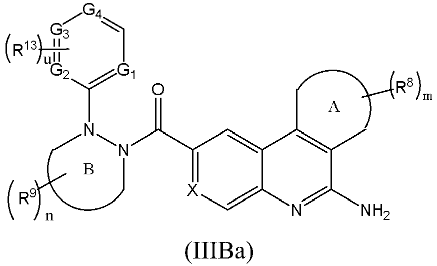 wherein,
G₁, G₂, G₃ and G₄ are each independently selected from the group consisting of CH and N; preferably, G₁, G₂, G₃ and G₄ are all CH; or one of G₁, G₂, G₃ and G₄ is N and the others are CH, or two of G₁, G₂, G₃ and G₄ are N and the others are CH;
each R¹³ is independently selected from the group consisting of hydrogen, deuterium, halogen, cyano, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl;
u is 1 or 2;
ring B, ring A, X, R⁴, R⁵, R⁸, R⁹, n and m are as defined in general formula (IIIA) or general formula (IIIB).

In another preferred embodiment, provided is the compound of general formula (I) or general formula (II) or a tautomer, mesomer, racemate, enantiomer, diastereomer thereof, or a mixture thereof, or an oxynitride thereof, or a pharmaceutically acceptable salt thereof according to the present invention, which is a compound of general formula (IVA) or general formula (IVB) or a tautomer, mesomer, racemate, enantiomer, diastereomer thereof, or a mixture thereof, or an oxynitride thereof, or a pharmaceutically acceptable salt thereof, 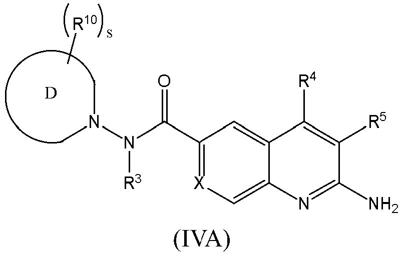 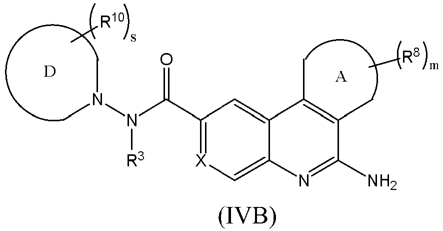 wherein,
ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, oxo, alkyl, alkoxy, haloalkyl, haloalkoxy, alkenyl, alkynyl, cycloalkyl, heterocyclyl, aryl and heteroaryl;
s is an integer from 0 to 4;
R³ is selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
X, R⁴, R⁵, R⁷, R^{a}, R^{b} and p are as defined in general formula (I);
ring A, R⁸ and m are as defined in general formula (II).

In another preferred embodiment, provided is the compound of general formula (IVA) or general formula (IVB) or a tautomer, mesomer, racemate, enantiomer, diastereomer thereof, or a mixture thereof, or an oxynitride thereof, or a pharmaceutically acceptable salt thereof according to the present invention, wherein ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, oxo, C₁₋₆ alkyl, C₁₋₆ alkoxy, C₁₋₆ haloalkyl and C₁₋₆ haloalkoxy;
s is an integer from 0 to 4;
R³ is selected from the group consisting of hydrogen, -(CH₂)ₚ-R⁷, C₁₋₆ alkyl, C₃₋₆ cycloalkyl and 4 to 10 membered heterocyclyl; R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, and the C₁₋₆ alkyl is optionally further substituted by one or more groups selected from the group consisting of deuterium, halogen, cyano, hydroxy, -NR^{a}R^{b}, -(CH2)_{q}-OR^{a}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen, C₁₋₆ alkyl and hydroxy substituted C₁₋₆ alkyl;
p is 1;
q is 1 or 2;
X, R⁴ and R⁵ are as defined in general formula (I) or general formula (II) above;
ring A, R⁸ and m are as defined in general formula (I) or general formula (II) above.

In another preferred embodiment, provided is the compound of general formula (IVA) or general formula (IVB) or a tautomer, mesomer, racemate, enantiomer, diastereomer thereof, or a mixture thereof, or an oxynitride thereof, or a pharmaceutically acceptable salt thereof according to the present invention, wherein ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, oxo, C₁₋₆ alkyl, C₁₋₆ alkoxy, C₁₋₆ haloalkyl and C₁₋₆ haloalkoxy;
s is an integer from 0 to 4;
R³ is selected from the group consisting of hydrogen, -(CH₂)ₚ-R⁷ and C₁₋₆ alkyl; R⁷ is selected from the group consisting of C₆₋₁₀ aryl and 5 to 10 membered heteroaryl, and the C₆₋₁₀ aryl or 5 to 10 membered heteroaryl is optionally further substituted by one or more groups selected from the group consisting of deuterium, halogen, cyano, hydroxy, oxo, -C₁₋₆ alkyl, C₁₋₆ alkoxy, C₁₋₆ haloalkyl, C₁₋₆ haloalkoxy, C₂₋₆ alkenyl and C₂₋₆ alkynyl;
p is 1;
X, R⁴ and R⁵ are as defined in general formula (I) or general formula (II) above;
ring A, R⁸ and m are as defined in general formula (I) or general formula (II) above.

In another preferred embodiment, provided is the compound of general formula (IVA) or general formula (IVB) or a tautomer, mesomer, racemate, enantiomer, diastereomer thereof, or a mixture thereof, or an oxynitride thereof, or a pharmaceutically acceptable salt thereof according to the present invention,
wherein, ring D is selected from the group consisting of 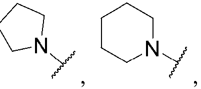 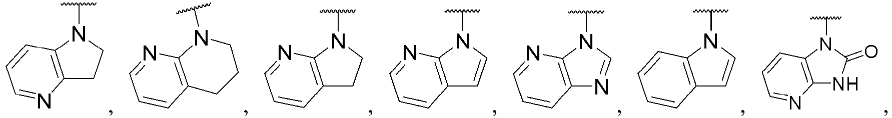 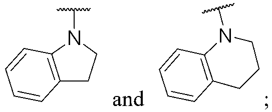
each R¹⁰ is independently selected from the group consisting of hydrogen, halogen, C₁₋₆ alkyl and C₁₋₆ haloalkyl;
s is 0 or 1.

In another preferred embodiment, provided is the compound of general formula (IVA) or general formula (IVB) or a tautomer, mesomer, racemate, enantiomer, diastereomer thereof, or a mixture thereof, or an oxynitride thereof, or a pharmaceutically acceptable salt thereof according to the present invention, wherein,
R³ is selected from the group consisting of hydrogen, -(CH₂)ₚ-R⁷ and C₁₋₆ alkyl;
R⁷ is selected from the group consisting of 5 to 6 membered heteroaryl and phenyl, preferably selected from the group consisting of 6 membered heteroaryl and phenyl, which is optionally further substituted by one or more groups selected from the group consisting of deuterium, halogen, C₁₋₆ alkyl, C₁₋₆ alkoxy, C₁₋₆ haloalkyl and C₁₋₆ haloalkoxy.

In another preferred embodiment, provided are the compounds of general formula (II), general formula (IIA), general formula (IIIB), general formula (IIIBa) and general formula (IVB) or a tautomer, mesomer, racemate, enantiomer, diastereomer thereof, or a mixture thereof, or an oxynitride thereof, or a pharmaceutically acceptable salt thereof according to the present invention, wherein, ring A is selected from the group consisting of 5 to 6 membered heteroaryl and 5 to 6 membered heterocyclyl; each R⁸ is independently selected from the group consisting of hydrogen and C₁₋₆ alkyl; m is 1 or 2, preferably 1.

In another preferred embodiment, provided are the compounds of general formula (II), general formula (IIA), general formula (IIIB), general formula (IIIBa) and general formula (IVB) or a tautomer, mesomer, racemate, enantiomer, diastereomer thereof, or a mixture thereof, or an oxynitride thereof, or a pharmaceutically acceptable salt thereof according to the present invention, wherein, ring A is selected from the group consisting of 5 to 6 membered heteroaryl and 5 to 6 membered heterocyclyl; preferably, is pyrazolyl or tetrahydrofuranyl; and especially, is 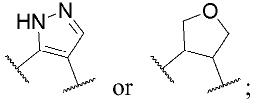
each R⁸ is independently selected from the group consisting of hydrogen and C₁₋₆ alkyl;
m is 1 or 2, preferably 1.

In a preferred embodiment, ring A is a pyrazole ring.

In another preferred embodiment, ring A is substituted by C₁₋₆ alkyl.

In another preferred embodiment, X is CR⁶ or N, and R⁶ is selected from the group consisting of hydrogen, halogen and C₁₋₆ alkyl.

In another preferred embodiment, provided are the compounds of general formula (II), general formula (IIA), general formula (IIIB), general formula (IIIBa) and general formula (IVB) or a tautomer, mesomer, racemate, enantiomer, diastereomer thereof, or a mixture thereof, or an oxynitride thereof, or a pharmaceutically acceptable salt thereof according to the present invention, wherein, X is CR⁶ or N, and R⁶ is selected from the group consisting of hydrogen, halogen, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and C₃₋₆ cycloalkyl.

In another preferred embodiment, provided are the compounds of general formula (I), general formula (IA), general formula (IIIA), general formula (IIIAa) and general formula (IVA) or a tautomer, mesomer, racemate, enantiomer, diastereomer thereof, or a mixture thereof, or an oxynitride thereof, or a pharmaceutically acceptable salt thereof according to the present invention, wherein R⁴ is selected from the group consisting of hydrogen, C₁₋₆ alkyl and C₃₋₆ cycloalkyl.

In another preferred embodiment, provided are the compounds of general formula (I), general formula (IA), general formula (IIIA), general formula (IIIAa) and general formula (IVA) or a tautomer, mesomer, racemate, enantiomer, diastereomer thereof, or a mixture thereof, or an oxynitride thereof, or a pharmaceutically acceptable salt thereof according to the present invention, wherein R⁵ is selected from the group consisting of halogen, cyano, C₁₋₆ alkyl, C₁₋₆ alkoxy, C₃₋₆cycloalkyl and C₂₋₆ alkenyl, and the C₁₋₆ alkyl is optionally substituted by deuterium, halogen, hydroxy or C₁₋₆ alkoxy.

In another preferred embodiment, provided are the compounds of general formula (I), general formula (IA), general formula (IIIA), general formula (IIIAa) and general formula (IVA) or a tautomer, mesomer, racemate, enantiomer, diastereomer thereof, or a mixture thereof, or an oxynitride thereof, or a pharmaceutically acceptable salt thereof according to the present invention, wherein, X is CR⁶, and R⁶ is selected from the group consisting of hydrogen, halogen, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and C₃₋₆ cycloalkyl;

In another preferred embodiment, X, R¹, R², R³, R⁴ and R⁵ are each independently corresponding groups in Example Compounds 1 to 512.

In a preferred embodiment, the present invention provides a compound of general formula (I) or a tautomer, mesomer, racemate, enantiomer, diastereomer thereof, or a mixture thereof, or a pharmaceutically acceptable salt thereof, 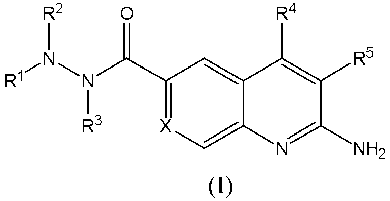 wherein:
X is CR⁶ or N;
R¹ and R² are each independently selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R³ is selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
or, R¹ and R², or R¹ and R³, together with the nitrogen atom to which they are attached, form a nitrogen-containing heterocyclyl or nitrogen-containing heteroaryl, and the nitrogen-containing heterocyclyl or nitrogen-containing heteroaryl is optionally further substituted by one or more groups selected from the group consisting of halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R⁴ and R⁵ are each independently selected from the group consisting of hydrogen, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)ₐR^{b}, -CH₂-R⁷, -NR^{a}R^{b}, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxyl, ester, amido, oxo, alkyl, alkoxy, haloalkyl, haloalkoxy, alkenyl, alkynyl, cycloalkyl, heterocyclyl, aryl and heteroaryl;
or, R⁴ and R⁵, together with the atoms to which they are attached, form a heteroaryl, heterocyclyl, aryl or cycloalkyl, and the heteroaryl, heterocyclyl, aryl or cycloalkyl is optionally further substituted by one or more groups selected from the group consisting of halogen, amino, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R⁶ is selected from the group consisting of hydrogen, halogen, amino, nitro, cyano, hydroxy, thiol, carboxyl, ester, oxo, -NR^{a}R^{b}, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more groups selected from the group consisting of deuterium, halogen, amino, nitro, cyano, hydroxy, thiol, carboxyl, ester, oxo, alkyl, alkoxy, haloalkyl, haloalkoxy, alkenyl, alkynyl, cycloalkyl, heterocyclyl, aryl and heteroaryl;
R⁷ is selected from the group consisting of heteroaryl, aryl, heterocyclyl and cycloalkyl, and the heteroaryl, aryl, heterocyclyl or cycloalkyl is optionally further substituted by one or more groups selected from the group consisting of deuterium, halogen, amino, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more groups selected from the group consisting of halogen, amino, nitro, cyano, hydroxy, thiol, carboxyl, -C(=O)OR^{c}, -C(=O)R^{c}, -C(=O)NR^{c}R^{d}, -OC(=O)R^{c}, -NR^{c}C(=O)R^{d}, -S(=O)₂R^{c}, -NR^{c}S(=O)₂R^{d}, -S(=O)₂NR^{c}R^{d}, -S(=O)R^{c}, -P(=O)R^{c}R^{d}, -NR^{c}S(=O)₂R^{d}, alkyl, alkoxy, alkenyl, alkynyl, cycloalkyl, heterocyclyl, aryl and heteroaryl;
or R^{a} and R^{b}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
R^{c} and R^{d} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl or heteroaryl is optionally further substituted by one or more groups selected from the group consisting of halogen, amino, nitro, cyano, hydroxy, thiol, carboxyl, alkyl, alkoxy, alkenyl, alkynyl, cycloalkyl, heterocyclyl, aryl and heteroaryl;
or R^{c} and R^{d}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
p is an integer from 1 to 6.

Typical compounds of the present invention include, but are not limited to:

| Compound No. | Structure | Name |
|---|---|---|
| 1 | 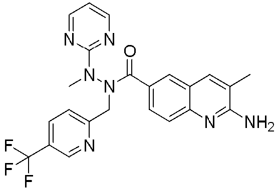 | 2-amino-N',3-dimethyl-N'-(pyrimidin-2-yl)-N-((5-(trifluoromethyl)pyridin-2-yl)me thyl)quinoline-6-carbohydrazide |
| 2 | 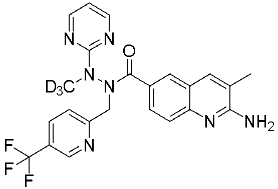 | 2-amino-3-methyl-N'-(methyl-d3)-N'-(py rimidin-2-yl)-N-((5-(trifluoromethyl)pyri din-2-yl)methyl)quinoline-6-carbohydrazide |
| 3 | 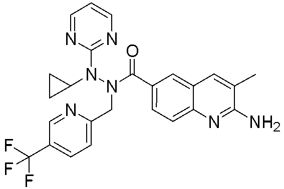 | 2-amino-N'-cyclopropyl-3-methyl-N'-( pyrimidin-2-yl)-N-((5-(trifluoromethyl)pyri din-2-yl)methyl)quinoline-6-carbohydrazide |
| 4 | 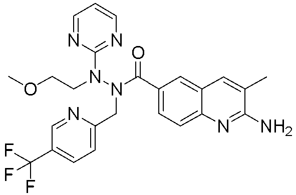 | 2-amino-N'-(2-methoxyethyl)-3-methyl-N'-(pyrimidin-2-yl)-N-((5-(trifluoromethyl )pyridin-2-yl)methyl)quinoline-6-carbohydrazide |
| 5 | 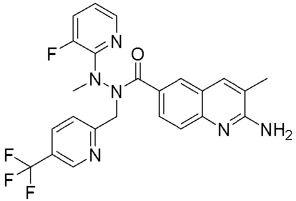 | 2-amino-N'-(3-fluoropyridin-2-yl)-N',3-di methyl-N-((5-(trifluoromethyl)pyridin-2-yl)methyl)quinoline-6-carbohydrazide |
| 6 | 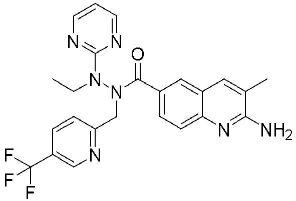 | 2-amino-N'-ethyl-3-methyl-N'-(pyrimidin -2-yl)-N-((5-(trifluoromethyl)pyridin-2-yl )methyl)quinoline-6-carbohydrazide |
| 7 | 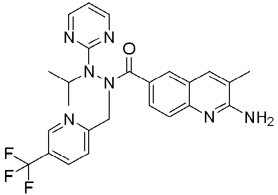 | 2-amino-N'-isopropyl-3-methyl-N'-(pyri midin-2-yl)-N-((5-(trifluoromethyl)pyridi n-2-yl)methyl)quinoline-6-carbohydrazide |
| 8 | 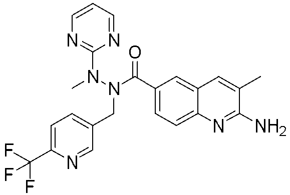 | 2-amino-N',3-dimethyl-N'-(pyrimidin-2-yl)-N-((6-(trifluoromethyl)pyridin-3-yl)me thyl)quinoline-6-carbohydrazide |
| 9 | 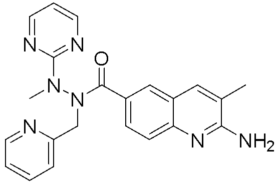 | 2-amino-N',3-dimethyl-N-(pyridin-2-ylm ethyl)-N'-(pyrimidin-2-yl)quinoline-6-carbohydrazide |
| 10 | 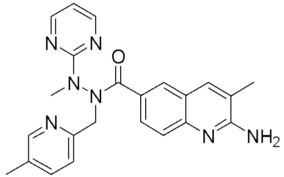 | 2-amino-N',3-dimethyl-N-((5-methylpyri din-2-yl)methyl)-N'-(pyrimidin-2-yl)quin oline-6-carbohydrazide |
| 11 | 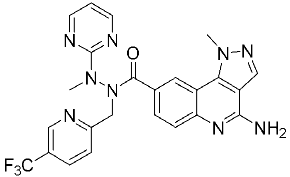 | 4-amino-N',1-dimethyl-N'-(pyrimidin-2-y 1)-N-((5-(trifluoromethyl)pyridin-2-yl)me thyl)-1H-pyrazolo[4,3-c]quinoline-8-carb ohydrazide |
| 12 | 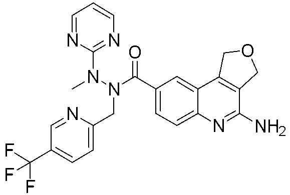 | 4-amino-N'-methyl-N'-(pyrimidin-2-yl)-N-((5-(trifluoromethyl)pyridin-2-yl)meth yl)-1,3-dihydrofuro[3,4-c]quinoline-8-car bohydrazide |
| 13 | 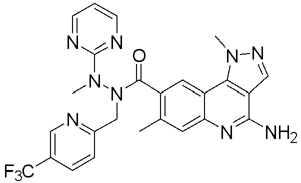 | 4-amino-N',1,7-trimethyl-N'-(pyrimidin-2 -yl)-N-((5-(trifluoromethyl)pyridin-2-yl) methyl)-1H-pyrazolo[4,3-c]quinoline-8-c arbohydrazide |
| 14 | 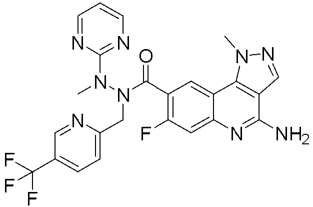 | 4-amino-7-fluoro-N',1-dimethyl-N'-(pyri midin-2-yl)-N-((5-(trifluoromethyl)pyridin -2-yl)methyl)-1H-pyrazolo[4,3-c]quinoli ne-8-carbohydrazide |
| 15 | 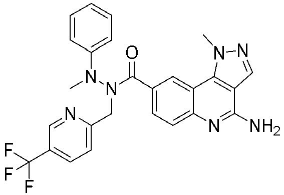 | 4-amino-N',1-dimethyl-N'-phenyl-N-((5-( trifluoromethyl)pyridin-2-yl)methyl)-1H-pyrazolo[4,3-c]quinoline-8-carbohydrazide |
| 16 | 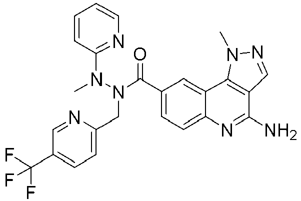 | 4-amino-N',1-dimethyl-N'-(pyridin-2-yl)-N-((5-(trifluoromethyl)pyridin-2-yl)meth yl)-1H-pyrazolo[4,3-c]quinoline-8-carbohydrazide |
| 17 | 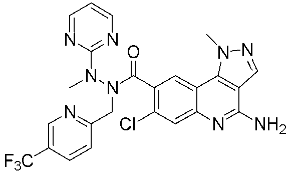 | 4-amino-7-chloro-N',1-dimethyl-N'-(pyri midin-2-yl)-N-((5-(trifluoromethyl)pyridi n-2-yl)methyl)-1H-pyrazolo[4,3-c]quinoli ne-8-carbohydrazide |
| 18 | 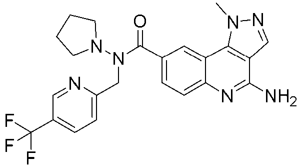 | 4-amino-1-methyl-N-(pyrrolidin-1-yl)-N-((5-(trifluoromethyl)pyridin-2-yl)methyl) -1H-pyrazolo[4,3-c]quinoline-8-carboxa mide |
| 19 | 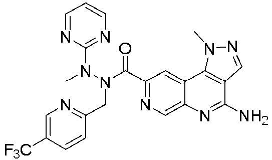 | 4-amino-N',1-dimethyl-N'-(pyrimidin-2-yl)-N-((5-(trifluoromethyl)pyridin-2-yl)me thyl)-1H-pyrazolo[4,3-c][1,7]naphthyridi ne-8-carbohydrazide |
| 20 | 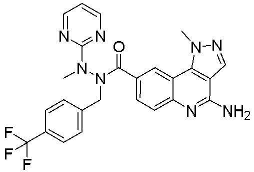 | 4-amino-N',1-dimethyl-N'-(pyrimidin-2-yl)-N-(4-(trifluoromethyl)benzyl)-1H-pyra zolo[4,3-c]quinoline-8-carbohydrazide |
| 21 | 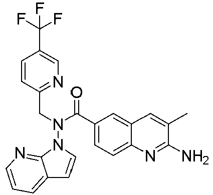 | 2-amino-3-methyl-N-(1H-pyrrolo[2,3-b] pyridin-1-yl)-N-((5-(trifluoromethyl)pyridin -2-yl)methyl)quinoline-6-carboxamide |
| 22 | 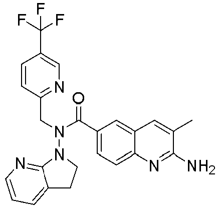 | 2-amino-N-(2,3-dihydro-1H-pyrrolo[2,3-b]pyridin-1-yl)-3-methyl-N-((5-(trifluoro methyl)pyridin-2-yl)methyl)quinoline-6-carboxamide |
| 23 | 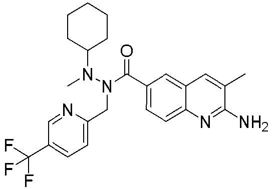 | 2-amino-N'-cyclohexyl-N',3-dimethyl-N-((5-(trifluoromethyl)pyridin-2-yl)methyl) quinoline-6-carbohydrazide |
| 24 | 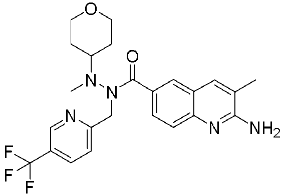 | 2-amino-N',3-dimethyl-N'-(tetrahydro-2H -pyran-4-yl)-N-((5-(trifluoromethyl)pyrid in-2-yl)methyl)quinoline-6-carbohydrazide |
| 25 | 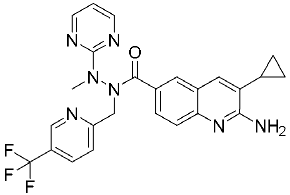 | 2-amino-3-cyclopropyl-N'-methyl-N'-(py rimidin-2-yl)-N-((5-(trifluoromethyl)pyri din-2-yl)methyl)quinoline-6-carbohydrazide |
| 26 | 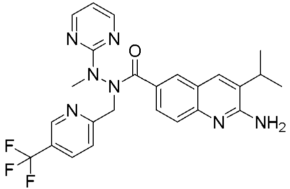 | 2-amino-3-isopropyl-N'-methyl-N'-(pyri midin-2-yl)-N-((5-(trifluoromethyl)pyridi n-2-yl)methyl)quinoline-6-carbohydrazide |
| 27 | 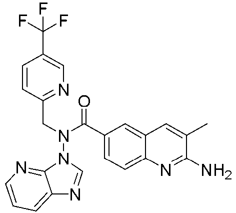 | 2-amino-N-(3H-imidazo[4,5-b]pyridin-3-yl)-3-methyl-N-((5-(trifluoromethyl)pyri din-2-yl)methyl)quinoline-6-carboxamide |
| 28 | 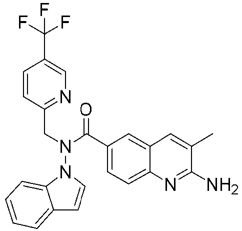 | 2-amino-N-(1H-indol-1-yl)-3-methyl-N-( (5-(trifluoromethyl)pyridin-2-yl)methyl)q uinoline-6-carboxamide |
| 29 | 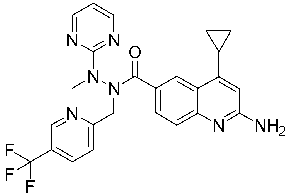 | 2-amino-4-cyclopropyl-N'-methyl-N'-(py rimidin-2-yl)-N-((5-(trifluoromethyl)pyri din-2-yl)methyl)quinoline-6-carbohydrazide |
| 30 | 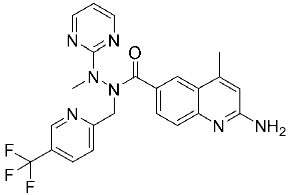 | 2-amino-N',4-dimethyl-N'-(pyrimidin-2-y 1)-N-((5-(trifluoromethyl)pyridin-2-yl)me thyl)quinoline-6-carbohydrazide |
| 31 | 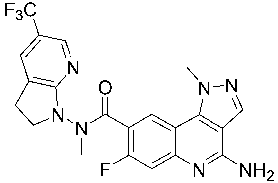 | 4-amino-7-fluoro-N,1-dimethyl-N-(5-(trif luoromethyl)-2,3-dihydro-1H-pyrrolo[2,3 -b]pyridin-1-yl)-1H-pyrazolo[4,3-c]quino line-8-carboxamide |
| 32 | 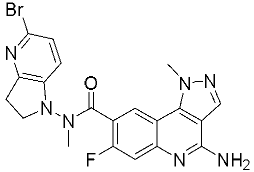 | 4-amino-N-(5-bromo-2,3-dihydro-1H-pyr rolo[3,2-b]pyridin-1-yl)-7-fluoro-N,1-di methyl-1H-pyrazolo[4,3-c]quinoline-8-ca rboxamide |
| 33 | 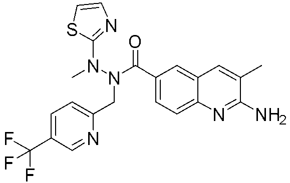 | 2-amino-N',3-dimethyl-N'-(thiazol-2-yl)-N-((5-(trifluoromethyl)pyridin-2-yl)meth yl)quinoline-6-carbohydrazide |
| 34 | 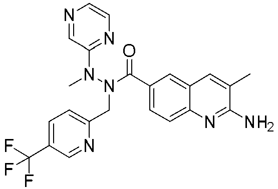 | 2-amino-N',3-dimethyl-N'-(pyrazin-2-yl) -N-((5-(trifluoromethyl)pyridin-2-yl)met hyl)quinoline-6-carbohydrazide |
| 35 | 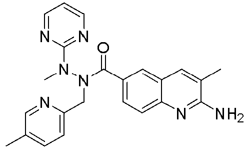 | 2-amino-N',3-dimethyl-N-((5-methylpyri din-2-yl)methyl)-N'-(pyrimidin-2-yl)quin oline-6-carbohydrazide |
| 36 | 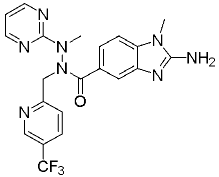 | 2-amino-N',1-dimethyl-N'-(pyrimidin-2-yl)-N-((5-(trifluoromethyl)pyridin-2-yl)m ethyl)-1H-benzo[d]imidazole-5-carbohydrazide |
| 37 | 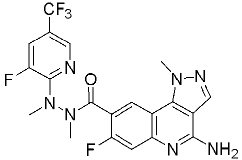 | 4-amino-7-fluoro-N'-(3-fluoro-5-(trifluor omethyl)pyridin-2-yl)-N,N',1-trimethyl-1 H-pyrazolo[4,3-c]quinoline-8-carbohydrazide |
| 38 | 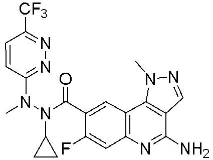 | 4-amino-N-cyclopropyl-7-fluoro-N',1-di methyl-N'-(6-(trifluoromethyl)pyridazin-3-yl)-1H-pyrazolo[4,3-c]quinoline-8-carb ohydrazide |
| 39 | 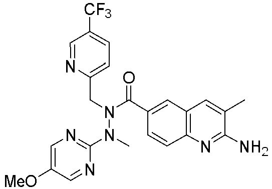 | 2-amino-N'-(5-methoxypyrimidin-2-yl)-N',3-dimethyl-N-((5-(trifluoromethyl)pyr idin-2-yl)methyl)quinoline-6-carbohydrazide |
| 40 | 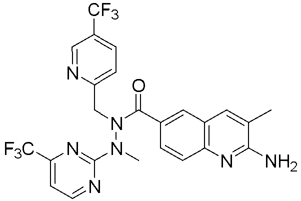 | 2-amino-N',3-dimethyl-N-((5-(trifluorom ethyl)pyridin-2-yl)methyl)-N'-(4-(trifluor omethyl)pyrimidin-2-yl)quinoline-6-carb ohydrazide |
| 41 | 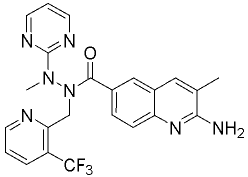 | 2-amino-N',3-dimethyl-N'-(pyrimidin-2-yl)-N-((3-(trifluoromethyl)pyridin-2-yl)me thyl)quinoline-6-carbohydrazide |
| 42 | 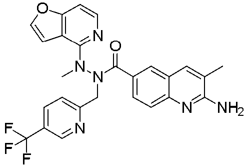 | 2-amino-N'-(furo[3,2-c]pyridin-4-yl)-N', 3-dimethyl-N-((5-(trifluoromethyl)pyridi n-2-yl)methyl)quinoline-6-carbohydrazide |
| 43 | 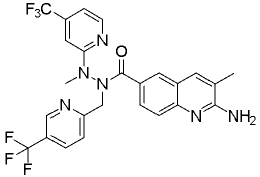 | 2-amino-N',3-dimethyl-N'-(4-(trifluorom ethyl)pyridin-2-yl)-N-((5-(trifluoromethy l)pyridin-2-yl)methyl)quinoline-6-carbohydrazide |
| 44 | 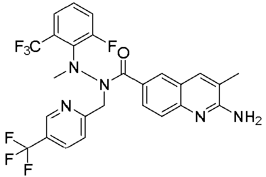 | 2-amino-N'-(2-fluoro-6-(trifluoromethyl) phenyl)-N',3-dimethyl-N-((5-(trifluorome thyl)pyridin-2-yl)methyl)quinoline-6-car bohydrazide |
| 45 | 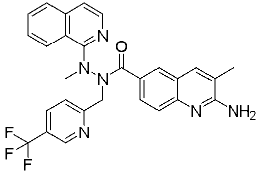 | 2-amino-*N*'-(isoquinol-1-yl)-*N*',3-dimethy l-*N*-((5-(trifluoromethyl)pyridin-2-yl)met hyl)quinoline-6-carbohydrazide |
| 46 | 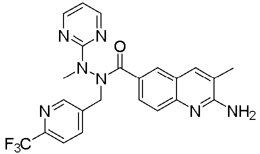 | 2-amino-N',3-dimethyl-N'-(pyrimidin-2-yl)-N-((6-(trifluoromethyl)pyridin-3-yl)me thyl)quinoline-6-carbohydrazide |
| 47 | 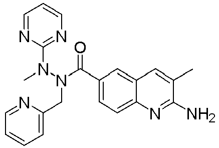 | 2-amino-N',3-dimethyl-N-(pyridin-2-ylm ethyl)-N'-(pyrimidin-2-yl)quinoline-6-car bohydrazide |
| 48 | 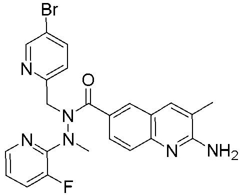 | 2-amino-N-((5-bromopyridin-2-yl)methyl )-N'-(3-fluoropyridin-2-yl)-N',3-dimethyl quinoline-6-carbohydrazide |
| 49 | 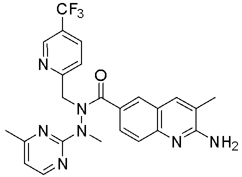 | 2-amino-N',3-dimethyl-N'-(4-methylpyri midin-2-yl)-N-((5-(trifluoromethyl)pyridi n-2-yl)methyl)quinoline-6-carbohydrazide |
| 50 | 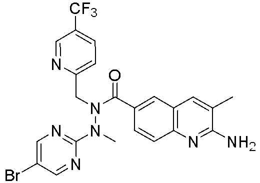 | 2-amino-N'-(5-bromopyrimidin-2-yl)-N', 3-dimethyl-N-((5-(trifluoromethyl)pyridi n-2-yl)methyl)quinoline-6-carbohydrazide |
| 51 | 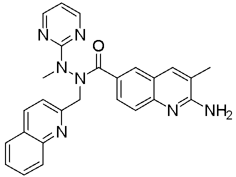 | 2-amino-N',3-dimethyl-N'-(pyrimidin-2-yl)-N-(quinolin-2-ylmethyl)quinoline-6-carbohydrazide |
| 52 | 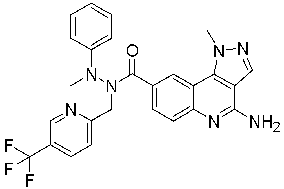 | 4-amino-N',1-dimethyl-N'-phenyl-N-((5-( trifluoromethyl)pyridin-2-yl)methyl)-1H-pyrazolo[4,3-c]quinoline-8-carbohydrazide |
| 53 | 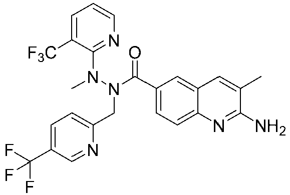 | 2-amino-N',3-dimethyl-N'-(3-(trifluorom ethyl)pyridin-2-yl)-N-((5-(trifluoromethy 1)pyridin-2-yl)methyl)quinoline-6-carbohydrazide |
| 54 | 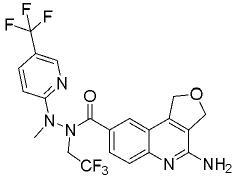 | 4-amino-N'-methyl-N-(2,2,2-trifluoroethy l)-N'-(5-(trifluoromethyl)pyridin-2-yl)-1, 3-dihydrofuro[3,4-c]quinoline-8-carbohydrazide |
| 55 | 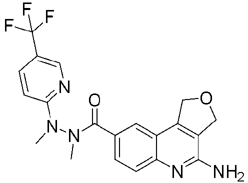 | 4-amino-N,N'-dimethyl-N'-(5-(trifluorom ethyl)pyridin-2-yl)-1,3-dihydrofuro[3,4-c ]quinoline-8-carbohydrazide |
| 56 | 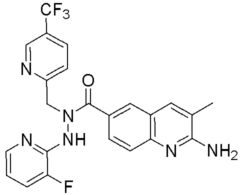 | 2-amino-N'-(3-fluoropyridin-2-yl)-3-met hyl-N-((5-(trifluoromethyl)pyridin-2-yl) methyl)quinoline-6-carbohydrazide |
| 57 | 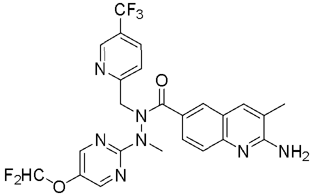 | 2-amino-N'-(5-(difluoromethoxy)pyrimid in-2-yl)-N',3-dimethyl-N-((5-(trifluorome thyl)pyridin-2-yl)methyl)quinoline-6-carbohydrazide |
| 58 | 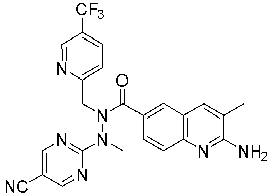 | 2-amino-N'-(5-cyanopyrimidin-2-yl)-N',3 -dimethyl-N-((5-(trifluoromethyl)pyridin-2-yl)methyl)quinoline-6-carbohydrazide |
| 59 | 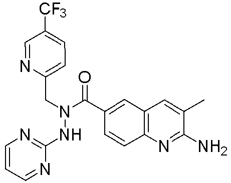 | 2-amino-3-methyl-N'-(pyrimidin-2-yl)-N-((5-(trifluoromethyl)pyridin-2-yl)methyl) quinoline-6-carbohydrazide |
| 60 | 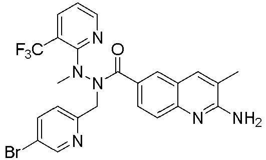 | 2-amino-N-((5-bromopyridin-2-yl)methyl )-N',3-dimethyl-N'-(3-(trifluoromethyl)py ridin-2-yl)quinoline-6-carbohydrazide |
| 61 | 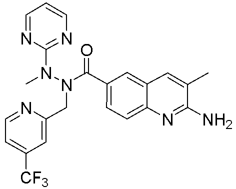 | 2-amino-N',3-dimethyl-N'-(pyrimidin-2-yl)-N-((4-(trifluoromethyl)pyridin-2-yl)me thyl)quinoline-6-carbohydrazide |
| 62 | 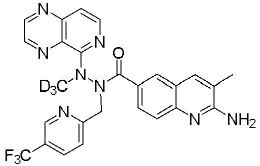 | 2-amino-3-methyl-N'-(methyl-d3)-N'-(py rido[3,4-b]pyrazin-5-yl)-N-((5-(trifluoro methyl)pyridin-2-yl) )methyl)quinoline-6 -carbohydrazide |
| 63 | 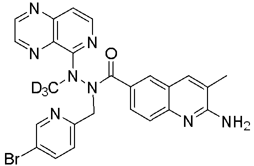 | 2-amino-N-((5-bromopyridin-2-yl)methyl )-3-methyl-N'-(methyl-d3)-N'-(pyrido[3,4 -b]pyrazin-5-yl)quinoline-6-carbohydrazide |
| 64 | 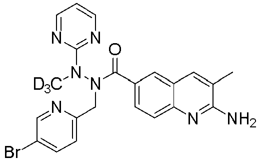 | 2-amino-N-((5-bromopyridin-2-yl)methyl )-3-methyl-N'-(methyl-d3)-N'-(pyrimidin-2-yl)quinoline-6-carbohydrazide |
| 65 | 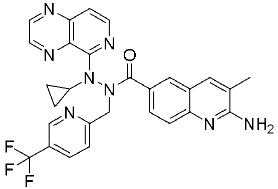 | 2-amino-N'-cyclopropyl-3-methyl-N'-(py rido[3,4-b]pyrazin-5-yl)-N-((5-(trifluoro methyl)pyridin-2-yl)methyl)quinoline-6-carbohydrazide |
| 66 | 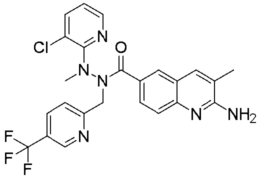 | 2-amino-N'-(3-chloropyridin-2-yl)-N',3-d imethyl-N-((5-(trifluoromethyl)pyridin-2-yl)methyl)quinoline-6-carbohydrazide |
| 67 | 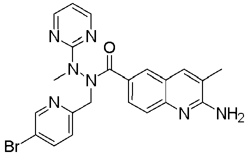 | 2-amino-N-((5-bromopyridin-2-yl)methyl )-N',3-dimethyl-N'-(pyrimidin-2-yl)quin oline-6-carbohydrazide |
| 68 | 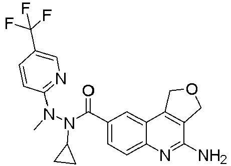 | 4-amino-N-cyclopropyl-N'-methyl-N'-(5-(trifluoromethyl)pyridin-2-yl)-1,3-d ihydrofuro[3,4-c]quinoline-8-carbohydrazide |
| 69 | 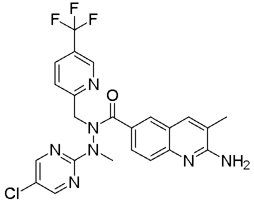 | 2-amino-N'-(5-chloropyrimidin-2-yl)-N', 3-dimethyl-N-((5-(trifluoromethyl)pyridi n-2-yl)methyl)quinoline-6-carbohydrazide |
| 70 | 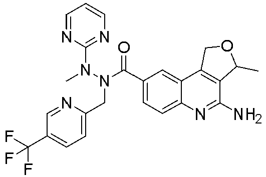 | 4-amino-N',3-dimethyl-N'-(pyrimidi n-2-yl)-N-((5-(trifluoromethyl)pyridin-2-yl)methyl)-1,3-dihydrofuro[3,4-c]quinoli ne-8-carbohydrazide |
| 71 | 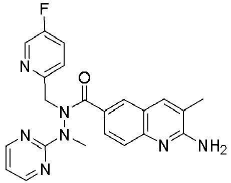 | 2-amino-N-((5-fluoropyridin-2-yl)methyl )-N',3-dimethyl-N'-(pyrimidin-2-yl)quin oline-6-carbohydrazide |
| 72 | 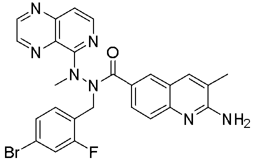 | 2-amino-N-(4-bromo-2-fluorobenzyl)-N', 3-dimethyl-N'-(pyrido[3,4-b]pyrazin-5-yl )quinoline-6-carbohydrazide |
| 73 | 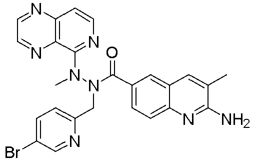 | 2-amino-N-((5-bromopyridin-2-yl)methyl )-N',3-dimethyl-N'-(pyrido[3,4-b]pyrazin -5-yl)quinoline-6-carbohydrazide |
| 74 | 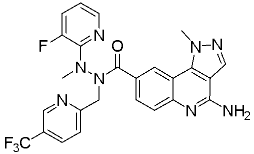 | 4-amino-N'-(3-fluoropyridin-2-yl)-N ',1-dimethyl-N-((5-(trifluoromethyl)pyrid in-2-yl)methyl)-1H-pyrazolo[4,3-c]quinol ine-8-carbohydrazide |
| 75 | 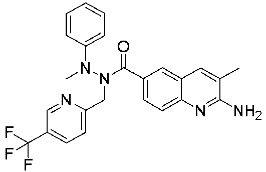 | 2-amino-N',3-dimethyl-N'-phenyl-N-((5-( trifluoromethyl)pyridin-2-yl)methyl)quinoline -6-carbohydrazide |
| 76 | 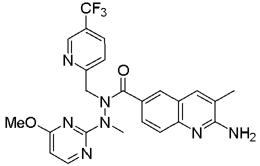 | 2-amino-N'-(4-methoxypyrimidin-2-yl)-N',3-dimethyl-N-((5-(trifluoromethyl)pyri din-2-yl)methyl)quinoline-6-carbohydrazide |
| 77 | 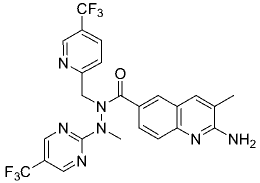 | 2-amino-N',3-dimethyl-N-((5-(trifluorom ethyl)pyridin-2-yl)methyl)-N'-(5-(trifluor omethyl)pyrimidin-2-yl)quinoline-6-carbohydrazide |
| 78 | 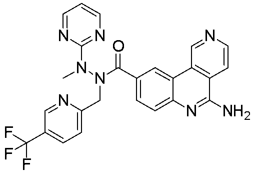 | 5-amino-N'-methyl-N'-(pyrimidin-2-yl)-N-((5-(trifluoromethyl)pyridin-2-yl)meth yl)benzo[c][2,6]naphthyridine-9-carbohydrazide |
| 79 | 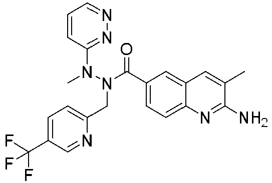 | 2-amino-N',3-dimethyl-N'-(pyridazin-3-yl)-N-((5-(trifluoromethyl)pyridin-2-yl)me thyl)quinoline-6-carbohydrazide |
| 80 | 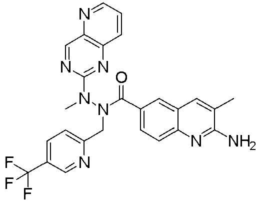 | 2-amino-N',3-dimethyl-N'-(pyrido[3,2-d] pyrimidin-2-yl)-N-((5-(trifluoromethyl)p yridin-2-yl)methyl)quinoline-6-carbohydrazide |
| 81 | 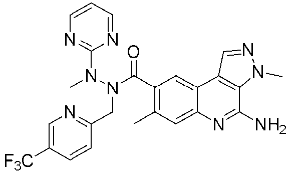 | 4-amino-N',3,7-trimethyl-N'-(pyrimidin-2 -yl)-N-((5-(trifluoromethyl)pyridin-2-yl) methyl)-3H-pyrazolo[3,4-c]quinoline-8-carbohydrazide |
| 82 | 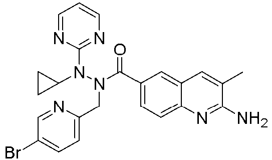 | 2-amino-N-((5-bromopyridin-2-yl)methyl )-N'-cyclopropyl-3-methyl-N'-(pyrimidin-2-yl)quinoline-6-carbohydrazide |
| 83 | 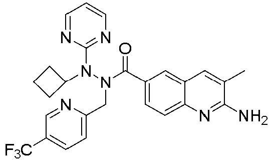 | 2-amino-N'-cyclobutyl-3-methyl-N'-(pyri midin-2-yl)-N-((5-(trifluoromethyl)pyridi n-2-yl)methyl)quinoline-6-carbohydrazide |
| 84 | 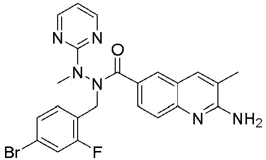 | 2-amino-N-(4-bromo-2-fluorobenzyl)-N', 3-dimethyl-N'-(pyrimidin-2-yl)quinoline-6-carbohydrazide |
| 85 | 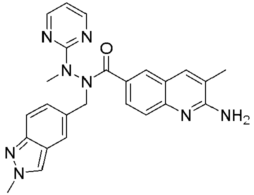 | 2-amino-N',3-dimethyl-N-((2-methyl-2H-indazol-5-yl)methyl)-N'-(pyrimidin-2-yl) quinoline-6-carbohydrazide |
| 86 | 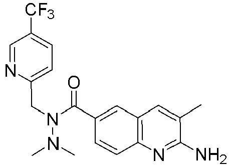 | 2-amino-N',N',3-trimethyl-N-((5-(trifluor omethyl)pyridin-2-yl)methyl)quinoline-6 -carbohydrazide |
| 87 | 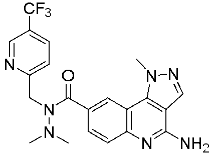 | 4-amino-N',N',1-trimethyl-N-((5-(trifluor omethyl)pyridin-2-yl)methyl)-1H-pyrazol o[4,3-c]quinoline-8-carbohydrazide |
| 88 | 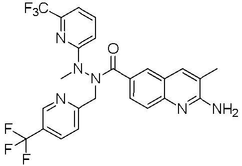 | 2-amino-N',3-dimethyl-N'-(6-(trifluorom ethyl)pyridin-2-yl)-N-((5-(trifluoromethyl )pyridin-2-yl)methyl)quinoline-6-carbohydrazide |
| 89 | 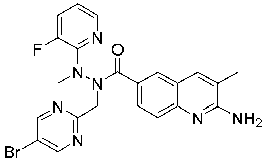 | 2-amino-N-((5-bromopyrimidin-2-yl)met hyl)-N'-(3-fluoropyridin-2-yl)-N',3-dimet hylquinoline-6-carbohydrazide |
| 90 | 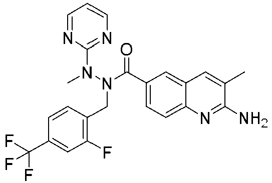 | 2-amino-N-(2-fluoro-4-(trifluoromethyl) benzyl)-N',3-dimethyl-N'-(pyrimidin-2-yl) quinoline-6-carbohydrazide |
| 91 | 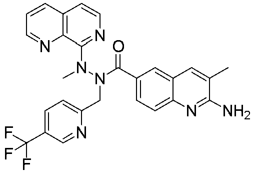 | 2-amino-N',3-dimethyl-N'-(1,7-naphthyri din-8-yl)-N-((5-(trifluoromethyl)pyridin-2-yl)methyl)quinoline-6-carbohydrazide |
| 92 | 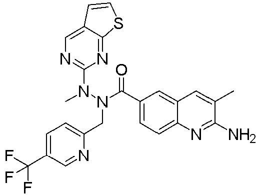 | 2-amino-N',3-dimethyl-N'-(thieno[2,3-d] pyrimidin-2-yl)-N-((5-(trifluoromethyl)p yridin-2-yl)methyl)quinoline-6-carbohydrazide |
| 93 | 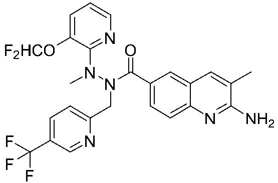 | 2-amino-N'-(3-(difluoromethoxy)pyridin-2-yl)-N',3-dimethyl-N-((5-(trifluorometh yl)pyridin-2-yl)methyl)quinoline-6-carbohydrazide |
| 94 | 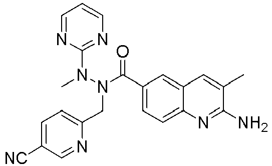 | 2-amino-N-((5-cyanopyridin-2-yl)methyl )-N',3-dimethyl-N'-(pyrimidin-2-yl) quinoline-6-carbohydrazide |
| 95 | 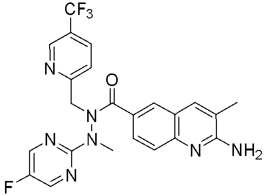 | 2-amino-N'-(5-fluoropyrimidin-2-yl)-N',3 -dimethyl-N-((5-(trifluoromethyl)pyridin-2-yl)methyl)quinoline-6-carbohydrazide |
| 96 | 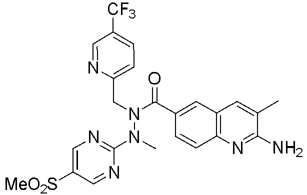 | 2-amino-N',3-dimethyl-N'-(5-(methylsulf onyl)pyrimidin-2-yl)-N-((5-(trifluoromet hyl)pyridin-2-yl)methyl)quinoline-6-carbohydrazide |
| 97 | 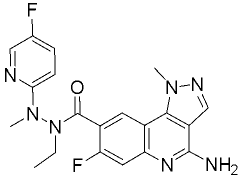 | 4-amino-N-ethyl-7-fluoro-N'-(5-fluoropy ridin-2-yl)-N',1-dimethyl-1H-pyrazolo[4, 3-c]quinoline-8-carbohydrazide |
| 98 | 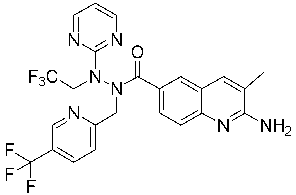 | 2-amino-3-methyl-N'-(pyrimidin-2-yl)-N' -(2,2,2-trifluoroethyl)-N-((5-(trifluoromet hyl)pyridin-2-yl)methyl)quinoline-6-carbohydrazide |
| 99 | 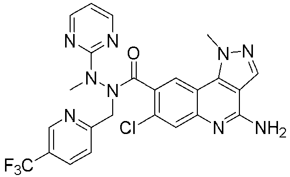 | 4-amino-7-chloro-N',1-dimethyl-N'-(pyri midin-2-yl)-N-((5-(trifluoromethyl)pyridi n-2-yl)methyl)-1H-pyrazolo[4,3-c]quinoli ne-8-carbohydrazide |
| 100 | 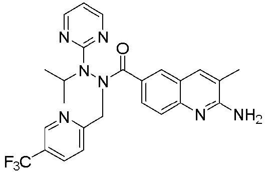 | 2-amino-N'-isopropyl-3-methyl-N'-(pyri midin-2-yl)-N-((5-(trifluoromethyl)pyridi n-2-yl)methyl)quinoline-6-carbohydrazide |
| 101 | 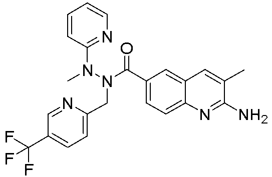 | 2-amino-N',3-dimethyl-N'-(pyridin-2-yl)-N-((5-(trifluoromethyl)pyridin-2-yl)meth yl)quinoline-6-carbohydrazide |
| 102 | 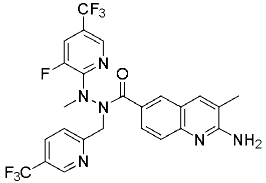 | 2-amino-N'-(3-fluoro-5-(trifluoromethyl) pyridin-2-yl)-N',3-dimethyl-N-((5-(trifluo romethyl)pyridin-2-yl)methyl)quinoline-6-carbohydrazide |
| 103 | 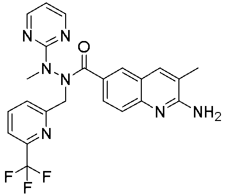 | 2-amino-N',3-dimethyl-N'-(pyrimidin-2-yl)-N-((6-(trifluoromethyl)pyridin-2-yl)me thyl)quinoline-6-carbohydrazide |
| 104 | 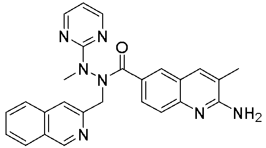 | 2-amino-N-(isoquinolin-3-ylmethyl)-N',3 -dimethyl-N'-(pyrimidin-2-yl)quinoline-6 -carbohydrazide |
| 105 | 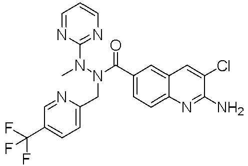 | 2-amino-3-chloro-N'-methyl-N'-(pyrimidi n-2-yl)-N-((5-(trifluoromethyl)pyridin-2-yl)methyl)quinoline-6-carbohydrazide |
| 106 | 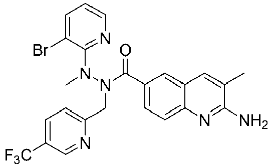 | 2-amino-*N*'-(3-bromopyridin-2-yl)-N',3-d imethyl-*N*-((5-(trifluoromethyl)pyridin-2-yl)methyl)quinoline-6-carbohydrazide |
| 107 | 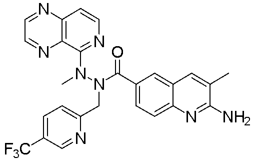 | 2-amino-*N*',3-dimethyl-*N*'-(pyrido[3,4-*b*] pyrazin-5-yl)-*N*-((5-(trifluoromethyl)pyri din-2-yl)methyl)quinoline-6-carbohydrazide |
| 108 | 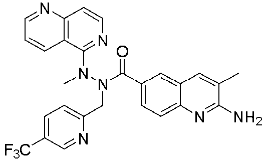 | 2-amino-N',3-dimethyl-N'-(1,6-naphthyri din-5-yl)-N-((5-(trifluoromethyl)pyridin-2-yl)methyl)quinoline-6-carbohydrazide |
| 109 | 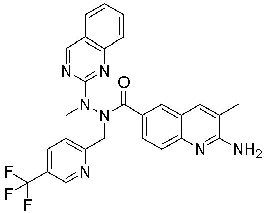 | 2-amino-N',3-dimethyl-N'-(quinazolin-2-yl)-N-((5-(trifluoromethyl)pyridin-2-yl)m ethyl)quinoline-6-carbohydrazide |
| 110 | 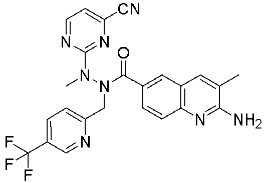 | 2-amino-N'-(4-cyanopyrimidin-2-yl)-N',3 -dimethyl-N-((5-(trifluoromethyl)pyridin-2-yl)methyl)quinoline-6-carbohydrazide |
| 111 | 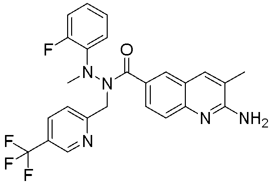 | 2-amino-N'-(2-fluorophenyl)-N',3-dimeth yl-N-((5-(trifluoromethyl)pyridin-2-yl) methyl)quinoline-6-carbohydrazide |
| 112 | 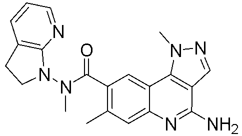 | 4-amino-N-(2,3-dihydro-1H-pyrrolo[2,3-b]pyridin-1-yl)-N,1,7-trimethyl-1H-pyrazolo[4,3-c]quinoline-8-carboxamide |
| 113 | 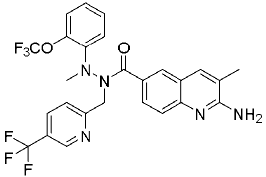 | 2-amino-N',3-dimethyl-N'-(2-(trifluorom ethoxy)phenyl)-N-((5-(trifluoromethyl)py ridin-2-yl)methyl)quinoline-6-carbohydrazide |
| 114 | 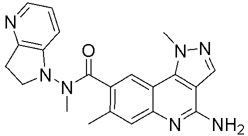 | 4-amino-N-(2,3-dihydro-1H-pyrrolo[3,2-b]pyridin-1-yl)-N,1,7-trimethyl-1H-pyraz olo[4,3-c]quinoline-8-carboxamide |
| 115 | 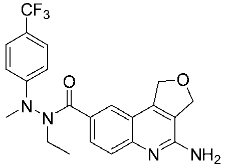 | 4-amino-N-ethyl-N-methyl-N-(4-(trifluor omethyl)phenyl)-1,3-dihydrofuro[3,4-c]q uinoline-8-carbohydrazide |
| 116 | 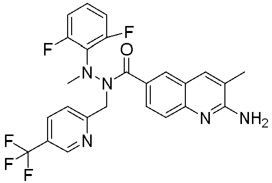 | 2-amino-N'-(2,6-difluorophenyl)-N',3-di methyl-N-((5-(trifluoromethyl)pyridin-2-yl)methyl)quinoline-6-carbohydrazide |
| 117 | 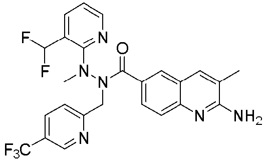 | 2-amino-*N*'-(3-(difluoromethyl)pyridin-2-yl)-*N*',3-dimethyl-*N*-((5-(trifluoromethyl) pyridin-2-yl)methyl)quinoline-6-carbohydrazide |
| 118 | 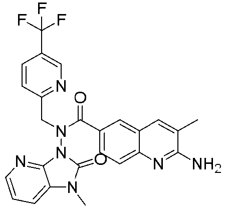 | 2-amino-3-methyl-*N*-(1-methyl-2-oxo-1,2 -dihydro-3*H*-imidazo[4,5-*b*]pyridin-3-yl)-*N*-((5-(trifluoromethyl)pyridin-2-yl)meth yl)quinoline-6-carboxamide |
| 119 | 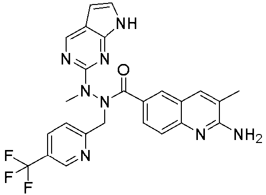 | 2-amino-N',3-dimethyl-N'-(7H-pyrrolo[2, 3-d]pyrimidin-2-yl)-N-((5-(trifluorometh yl)pyridin-2-yl)methyl)quinoline-6-carbo hydrazide |
| 120 | 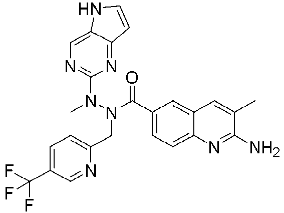 | 2-amino-*N*',3-dimethyl-*N*'-(5*H*-pyrrolo[3, 2-*d*]pyrimidin-2-yl)-*N*-((5-(trifluorometh yl)pyridin-2-yl)methyl)quinoline-6-carbo hydrazide |
| 121 | 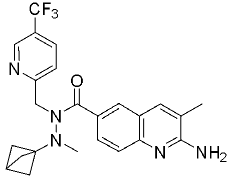 | 2-amino-N'-(bicyclo[1.1.1]pentan-1-yl)-N',3-dimethyl-N-((5-(trifluoromethyl)pyri din-2-yl)methyl)quinoline-6-carbohydrazide |
| 122 | 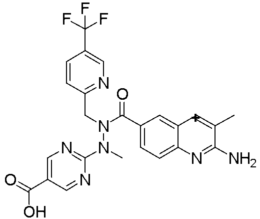 | 2-amino-6-(2-(5-carboxylpyrimidin-2-yl) -2-methyl-1-((5-(trifluoromethyl)pyridin-2-yl)methyl)hydrazine-1-carbonyl)-3-met hylquinolin-4-ylium |
| 123 | 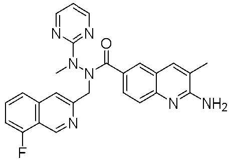 | 2-amino-N-((8-fluoroisoquinolin-3-yl)me thyl)-N',3-dimethyl-N'-(pyrimidin-2-yl)q uinoline-6-carbohydrazide |
| 124 | 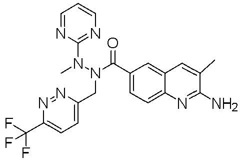 | 2-amino-N',3-dimethyl-N'-(pyrimidin-2-y 1)-N-((6-(trifluoromethyl)pyridazin-3-yl) methyl)quinoline-6-carbohydrazide |
| 125 | 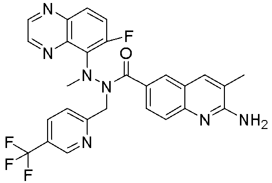 | 2-amino-N'-(6-fluoroquinoxalin-5-yl)-N', 3-dimethyl-N-((5-(trifluoromethyl)pyridi n-2-yl)methyl)quinoline-6-carbohydrazide |
| 126 | 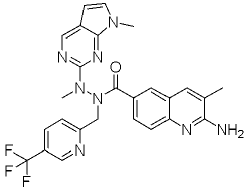 | 2-amino-N',3-dimethyl-N'-(7-methyl-7H-pyrrolo[2,3-d]pyrimidin-2-yl)-N-((5-(trifl uoromethyl)pyridin-2-yl)methyl)quinolin e-6-carbohydrazide |
| 127 | 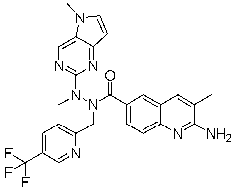 | 2-amino-*N*',3-dimethyl-*N*'-(5-methyl-5*H-*pyrrolo[3,2-*d*]pyrimidin-2-yl)-*N*-((5-(trifl uoromethyl)pyridin-2-yl)methyl)quinolin e-6-carbohydrazide |
| 128 | 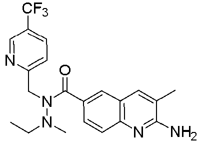 | 2-amino-*N*'-ethyl-*N*',3-dimethyl-*N*-((5-(tri fluoromethyl)pyridin-2-yl)methyl)quinoli ne-6-carbohydrazide |
| 129 | 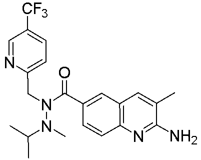 | 2-amino-*N*'-isopropyl-*N*',3-dimethyl-*N*-(( 5-(trifluoromethyl)pyridin-2-yl)methyl)q uinoline-6-carbohydrazide |
| 130 | 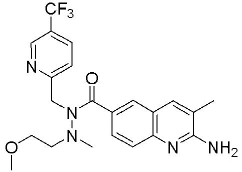 | 2-amino-N'-(2-methoxyethyl)-N',3-dimet hyl-N-((5-(trifluoromethyl)pyridin-2-yl) methyl)quinoline-6-carbohydrazide |
| 131 | 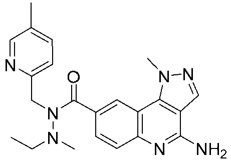 | 4-amino-*N*'-ethyl-*N',1*-dimethyl-*N*-((5-m ethylpyridin-2-yl)methyl)-1*H*-pyrazolo[4, 3-*c*]quinoline-8-carbohydrazide |
| 132 | 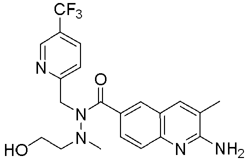 | 2-amino-*N*'-(2-hydroxyethyl)-*N*',3-dimeth yl-N-((5-(trifluoromethyl)pyridin-2-yl)me thyl)quinoline-6-carbohydrazide |
| 133 | 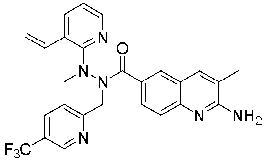 | 2-amino-N',3-dimethyl-N-((5-(trifluorom ethyl)pyridin-2-yl)methyl)-N'-(3-vinylpy ridin-2-yl)quinoline-6-carbohydrazide |
| 134 | 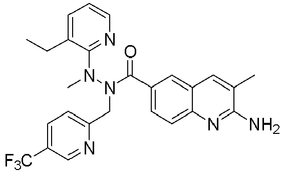 | 2-amino-*N*'-(3-ethylpyridin-2-yl)-*N*',3-di methyl-*N*-((5-(trifluoromethyl)pyridin-2-yl)methyl)quinoline-6-carbohydrazide |
| 135 | 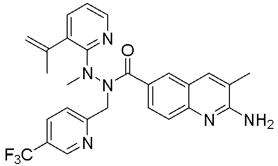 | 2-amino-N',3-dimethyl-N'-(3-(prop-1-en-2-yl)pyridin-2-yl)-N-((5-(trifluoromethyl) pyridin-2-yl)methyl)quinoline-6-carbohy drazide |
| 136 | 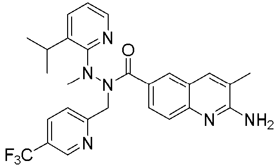 | 2-amino-*N*'-(3-isopropylpyridin-2-yl)-*N*', 3-dimethyl-*N*-((5-(trifluoromethyl)pyridi n-2-yl)methyl)quinoline-6-carbohydrazide |
| 137 | 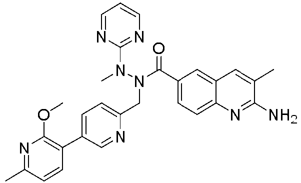 | 2-amino-N-((2'-methoxy-6'-methyl-[3,3'-bipyridine]-6-yl)methyl)-N',3-dimethyl-N '-(pyrimidin-2-yl)quinoline-6-carbohydrazide |
| 138 | 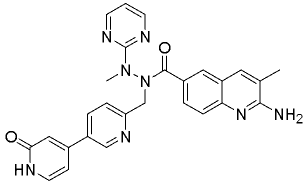 | 2-amino-N',3-dimethyl-N-((2'-oxo-1',2'-d ihydro-[3,4'-bipyridine]-6-yl)methyl)-N'-( pyrimidin-2-yl)quinoline-6-carbohydrazide |
| 139 | 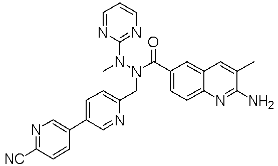 | 2-amino-N-((6'-cyano-[3,3'-bipyridine]-6 -yl)methyl)-N',3-dimethyl-N'-(pyrimidin-2-yl)quinoline-6-carbohydrazide |
| 140 | 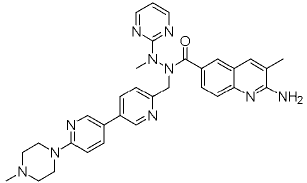 | 2-amino-N',3-dimethyl-N-((6'-(4-methylp iperazin-1-yl)-[3,3'-bipyridine]-6-yl)meth yl)-N'-(pyrimidin-2-yl)quinoline-6-carbohydrazide |
| 141 | 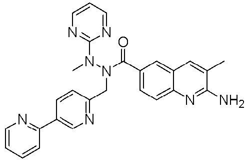 | N-([2,3'-bipyridine]-6'-ylmethyl)-2-amino -N',3-dimethyl-N'-(pyrimidin-2-yl)quino line-6-carbohydrazide |
| 142 | 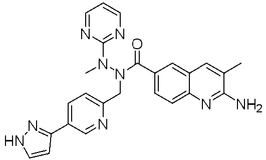 | N-((5-(1H-pyrazol-3-yl)pyridin-2-yl)met hyl)-2-amino-N',3-dimethyl-N'-(pyrimidi n-2-yl)quinoline-6-carbohydrazide |
| 143 | 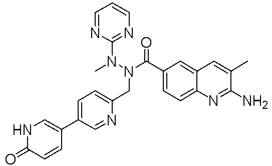 | 2-amino-N',3-dimethyl-N-((6'-oxo-1',6'-d ihydro-[3,3'-bipyridine]-6-yl)methyl)-N'-( pyrimidin-2-yl)quinoline-6-carbohydrazide |
| 144 | 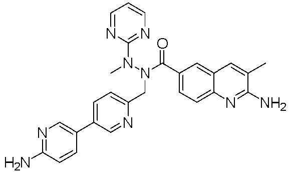 | 2-amino-N-((6'-amino-[3,3'-bipyridine]-6 -yl)methyl)-N',3-dimethyl-N'-(pyrimidin-2-yl)quinoline-6-carbohydrazide |
| 145 | 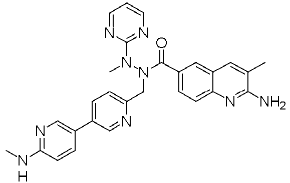 | 2-amino-N',3-dimethyl-N-((6'-(methylam ino)-[3,3'-bipyridine]-6-yl)methyl)-N'-(py rimidin-2-yl)quinoline-6-carbohydrazide |
| 146 | 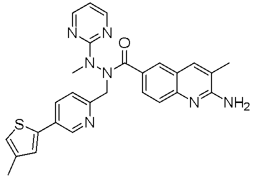 | 2-amino-*N*',3-dimethyl-*N*-((5-(4-methylth ien-2-yl)pyridin-2-yl)methyl)-*N*'-(pyrimid in-2-yl)quinoline-6-carbohydrazide |
| 147 | 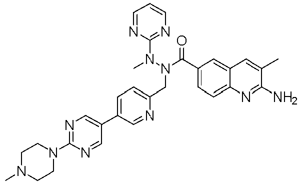 | 2-amino-N',3-dimethyl-N-((5-(2-(4-meth ylpiperazin-1-yl)pyrimidin-5-yl)pyridin-2 -yl)methyl)-N'-(pyrimidin-2-yl)quinoline-6-carbohydrazide |
| 148 | 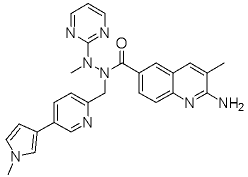 | 2-amino-N',3-dimethyl-N-((5-(1-methyl-1H-pyrrol-3-yl)pyridin-2-yl)methyl)-N'-( pyrimidin-2-yl)quinoline-6-carbohydrazide |
| 149 | 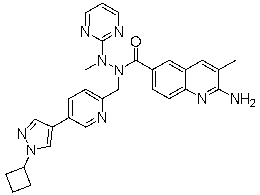 | 2-amino-N-((5-(1-cyclobutyl-1H-pyrazol -4-yl)pyridin-2-yl)methyl)-N',3-dimethyl-N'-(pyrimidin-2-yl)quinoline-6-carbohydr azide |
| 150 | 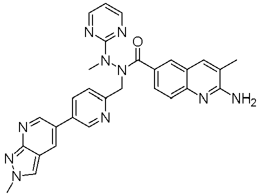 | 2-amino-N',3-dimethyl-N-((5-(2-methyl-2H-pyrazolo[3,4-b]pyridin-5-yl)pyridin-2 -yl)methyl)-N'-(pyrimidin-2-yl)quinoline-6-carbohydrazide |
| 151 | 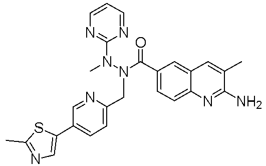 | 2-amino-N',3-dimethyl-N-((5-(2-methylth iazol-5-yl)pyridin-2-yl)methyl)-N'-(pyrim idin-2-yl)quinoline-6-carbohydrazide |
| 152 | 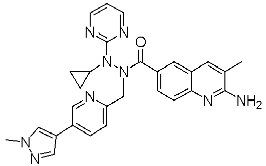 | 2-amino-N'-cyclopropyl-3-methyl-N-((5-(1-methyl-1H-pyrazol-4-yl)pyridin-2-yl) methyl)-N'-(pyrimidin-2-yl)quinoline-6-c arbohydrazide |
| 153 | 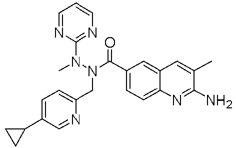 | 2-amino-N-((5-cyclopropylpyridin-2-yl) methyl)-N',3-dimethyl-N'-(pyrimidin-2-yl)quinoline-6-carbohydrazide |
| 154 | 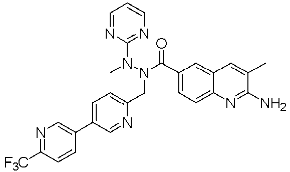 | 2-amino-N',3-dimethyl-N'-(pyrimidin-2-y 1)-N-((6'-(trifluoromethyl)-[3,3'-bipyridin e]-6-yl)methyl)quinoline-6-carbohydrazide |
| 155 | 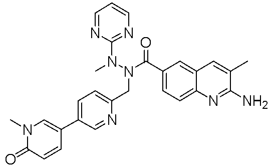 | 2-amino-N',3-dimethyl-N-((1'-methyl-6'-oxo-1',6'-dihydro-[3,3'-bipyridine]-6-yl)m ethyl)-N'-(pyrimidin-2-yl)quinoline-6-car bohydrazide |
| 156 | 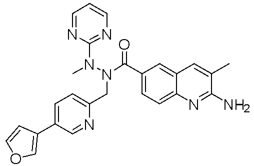 | 2-amino-N-((5-(furan-3-yl)pyridin-2-yl) methyl)-N',3-dimethyl-N'-(pyrimidin-2-yl )quinoline-6-carbohydrazide |
| 157 | 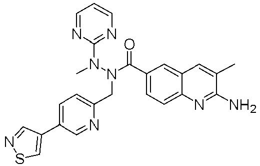 | 2-amino-N-((5-(isothiazol-4-yl)pyridin-2-yl)methyl)-N',3-dimethyl-N'-(pyrimidin-2 -yl)quinoline-6-carbohydrazide |
| 158 | 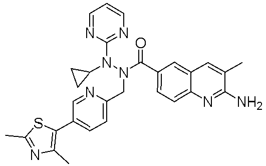 | 2-amino-N'-cyclopropyl-N-((5-(2,4-dimet hylthiazol-5-yl)pyridin-2-yl)methyl)-3-m ethyl-N'-(pyrimidin-2-yl)quinoline-6-carb ohydrazide |
| 159 | 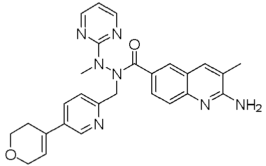 | 2-amino-N-((5-(3,6-dihydro-2H-pyran-4-yl)pyridin-2-yl)methyl)-N',3-dimethyl-N' -(pyrimidin-2-yl)quinoline-6-carbohydrazide |
| 160 | 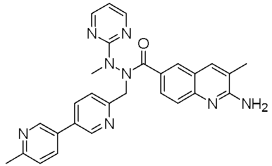 | 2-amino-N',3-dimethyl-N-((6'-methyl-[3, 3'-bipyridine]-6-yl)methyl)-N'-(pyrimidin -2-yl)quinoline-6-carbohydrazide |
| 161 | 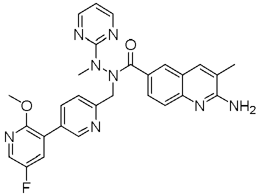 | 2-amino-N-((5'-fluoro-2'-methoxy-[3,3'-b ipyridine]-6-yl)methyl)-N',3-dimethyl-N'-(pyrimidin-2-yl)quinoline-6-carbohydrazide |
| 162 | 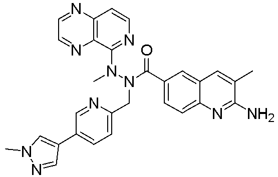 | 2-amino-N',3-dimethyl-N-((5-(1-methyl-1H-pyrazol-4-yl)pyridin-2-yl)methyl)-N'-(pyrido[3,4-b]pyrazin-5-yl)quinoline-6-carbohydrazide |
| 163 | 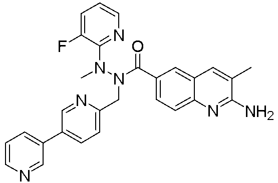 | N-([3,3'-bipyridine]-6-ylmethyl)-2-amino -N'-(3-fluoropyridin-2-yl)-N',3-dimethylq uinoline-6-carbohydrazide |
| 164 | 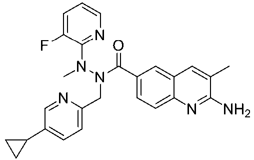 | 2-amino-N-((5-cyclopropylpyridin-2-yl) methyl)-N'-(3-fluoropyridin-2-yl)-N',3-di methylquinoline-6-carbohydrazide |
| 165 | 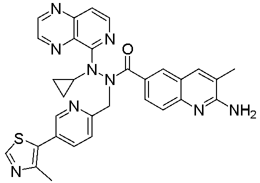 | 2-amino-N'-cyclopropyl-3-methyl-N-((5-(4-methylthiazol-5-yl)pyridin-2-yl)methy 1)-N'-(pyrido[3,4-b]pyrazin-5-yl)quinoline -6-carbohydrazide |
| 166 | 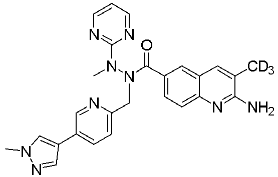 | 2-amino-N'-methyl-3-(methyl-D3)-N-((5-(1-methyl-1H-pyrazol-4-yl)pyridin-2-yl) methyl)-N'-(pyrimidin-2-yl)quinoline-6-carbohydrazide |
| 167 | 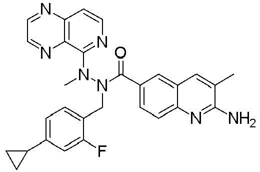 | 2-amino-N-(4-cyclopropyl-2-fluorobenzy 1)-N',3-dimethyl-N'-(pyrido[3,4-b]pyrazi n-5-yl)quinoline-6-carbohydrazide |
| 168 | 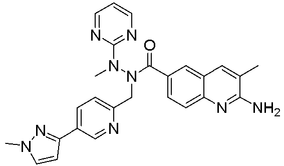 | 2-amino-N',3-dimethyl-N-((5-(1-methyl-1H-pyrazol-3-yl)pyridin-2-yl)methyl)-N'-(pyrimidin-2-yl)quinoline-6-carbohydrazide |
| 169 | 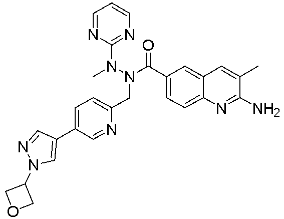 | 2-amino-N',3-dimethyl-N-((5-(1-(oxetan-3-yl)-1H-pyrazol-4-yl)pyridin-2-yl)methy l)-N'-(pyrimidin-2-yl)quinoline-6-carbohydrazide |
| 170 | 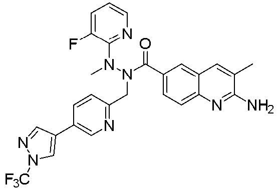 | 2-amino-N'-(3-fluoropyridin-2-yl)-N ',3-dimethyl-N-((5-(1-(trifluoromethyl)-1 H-pyrazol-4-yl)pyridin-2-yl)methyl)quin oline-6-carbohydrazide |
| 171 | 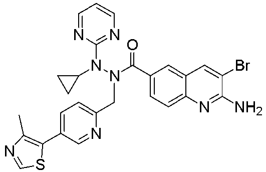 | 2-amino-3-bromo-N'-cyclopropyl-N-((5-( 4-methylthiazol-5-yl)pyridin-2-yl)methyl )-N'-(pyrimidin-2-yl)quinoline-6-carbohydrazide |
| 172 | 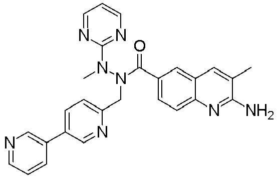 | N-([3,3'-bipyridine]-6-ylmethyl)-2-amino -N',3-dimethyl-N'-(pyrimidin-2-yl)quinol ine-6-carbohydrazide |
| 173 | 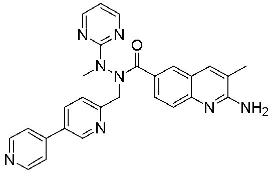 | N-([3,4'-bipyridine]-6-ylmethyl)-2-amino -N',3-dimethyl-N'-(pyrimidin-2-yl)quinol ine-6-carbohydrazide |
| 174 | 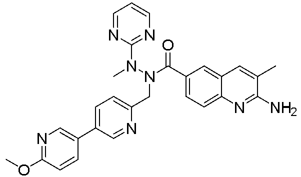 | 2-amino-N-((6'-methoxy-[3,3'-bipyridine] -6-yl)methyl)-N',3-dimethyl-N'-(pyrimidi n-2-yl)quinoline-6-carbohydrazide |
| 175 | 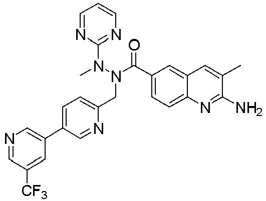 | 2-amino-N',3-dimethyl-N'-(pyrimidin-2-yl)-N-((5'-(trifluoromethyl)-[3,3'-bipyridin e]-6-yl)methyl)quinoline-6-carbohydrazide |
| 176 | 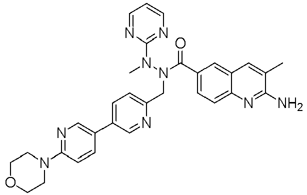 | 2-amino-N',3-dimethyl-N-((6'-morpholin yl-[3,3'-bipyridine]-6-yl)methyl)-N'-(pyri midin-2-yl)quinoline-6-carbohydrazide |
| 177 | 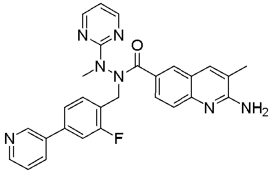 | 2-amino-N-(2-fluoro-4-(pyridin-3-yl)ben zyl)-N',3-dimethyl-N'-(pyrimidin-2-yl)qu inoline-6-carbohydrazide |
| 178 | 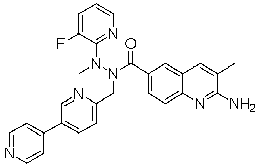 | N-([3,4'-bipyridine]-6-ylmethyl)-2-amino -N'-(3-fluoropyridin-2-yl)-N',3-dimethylq uinoline-6-carbohydrazide |
| 179 | 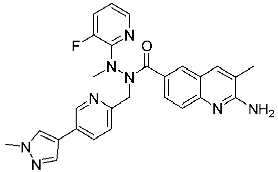 | 2-amino-N'-(3-fluoropyridin-2-yl)-N',3-di methyl-N-((5-(1-methyl-1H-pyrazol-4-yl) pyridin-2-yl)methyl)quinoline-6-carbohy drazide |
| 180 | 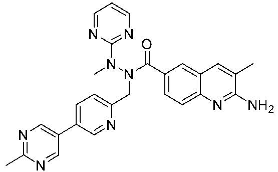 | 2-amino-N',3-dimethyl-N-((5-(2-methylp yrimidin-5-yl)pyridin-2-yl)methyl)-N'-(p yrimidin-2-yl)quinoline-6-carbohydrazide |
| 181 | 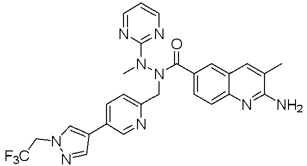 | 2-amino-N',3-dimethyl-N'-(pyrimidin-2-y l)-N-((5-(1-(2,2,2-trifluoroethyl)-1H-pyra zol-4-yl)pyridin-2-yl)methyl)quinoline-6-carbohydrazide |
| 182 | 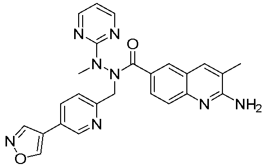 | 2-amino-N-((5-(isoxazol-4-yl)pyridin-2-y l)methyl)-N',3-dimethyl-N'-(pyrimidin-2-yl)quinoline-6-carbohydrazide |
| 183 | 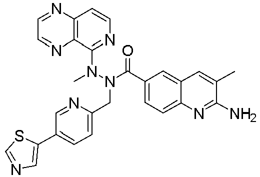 | 2-amino-N',3-dimethyl-N'-(pyrido[3,4-b] pyrazin-5-yl)-N-((5-(thiazol-5-yl)pyridin-2-yl)methyl)quinoline-6-carbohydrazide |
| 184 | 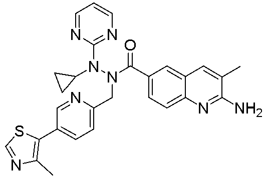 | 2-amino-N'-cyclopropyl-3-methyl-N-((5-(4-methylthiazol-5-yl)pyridin-2-yl)methy l)-N'-(pyrimidin-2-yl)quinoline-6-carbohydrazide |
| 185 | 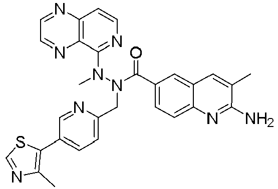 | 2-amino-N',3-dimethyl-N-((5-(4-methylth iazol-5-yl)pyridin-2-yl)methyl)-N'-(pyrid o[3,4-b]pyrazin-5-yl)quinoline-6-carbohydrazide |
| 186 | 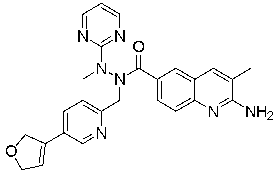 | 2-amino-N-((5-(2,5-dihydrofuran-3-yl)py ridin-2-yl)methyl)-N',3-dimethyl-N'-(pyri midin-2-yl)quinoline-6-carbohydrazide |
| 187 | 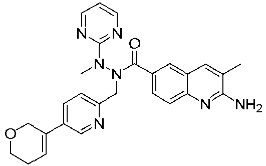 | 2-amino-N-((5-(5,6-dihydro-2H-pyran-3-yl)pyridin-2-yl)methyl)-N',3-dimethyl-N' -(pyrimidin-2-yl)quinoline-6-carbohydrazide |
| 188 | 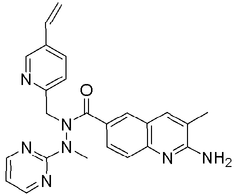 | 2-amino-N',3-dimethyl-N'-(pyrimidin-2-yl)-N-((5-vinylpyridin-2-yl)methyl)quinoli ne-6-carbohydrazide |
| 189 | 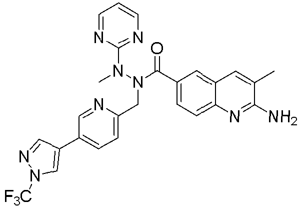 | 2-amino-N',3-dimethyl-N'-(pyrimidin-2-yl)-N-((5-(1-(trifluoromethyl)-1H-pyrazol-4-yl)pyridin-2-yl)methyl)quinoline-6-carbohydrazide |
| 190 | 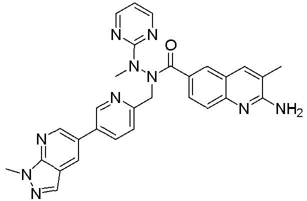 | 2-amino-N',3-dimethyl-N-((5-(1-methyl-1H-pyrazolo[3,4-b]pyridin-5-yl)pyridin-2 -yl)methyl)-N'-(pyrimidin-2-yl)quinoline-6-carbohydrazide |
| 191 | 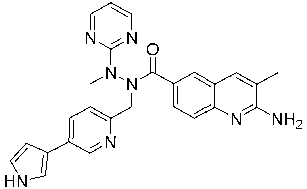 | N-((5-(1H-pyrrol-3-yl)pyridin-2-yl)meth yl)-2-amino-N',3-dimethyl-N'-(pyrimidin -2-yl)quinoline-6-carbohydrazide |
| 192 | 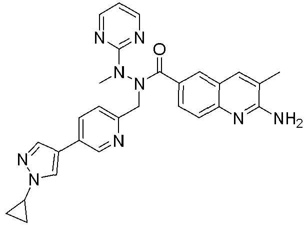 | 2-amino-N-((5-(1-cyclopropyl-1H-pyrazol -4-yl)pyridin-2-yl)methyl)-N',3-dimethyl -N'-(pyrimidin-2-yl)quinoline-6-carbohydrazide |
| 193 | 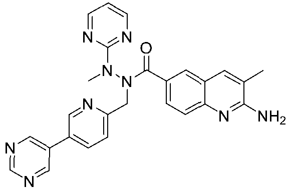 | 2-amino-N',3-dimethyl-N'-(pyrimidin-2-yl)-N-((5-(pyrimidin-5-yl)pyridin-2-yl) methyl)quinoline-6-carbohydrazide |
| 194 | 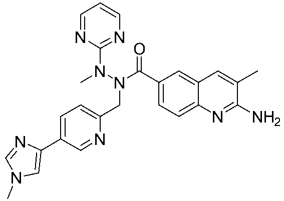 | 2-amino-N',3-dimethyl-N-((5-(1-methyl-1H-imidazol-4-yl)pyridin-2-yl)methyl)-N '-(pyrimidin-2-yl)quinoline-6-carbohydrazide |
| 195 | 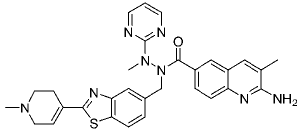 | 2-amino-N',3-dimethyl-N-((2-(1-methyl-1,2,3,6-tetrahydropyridin-4-yl)benzo[d]th iazol-5-yl)methyl)-N'-(pyrimidin-2-yl)qui noline-6-carbohydrazide |
| 196 | 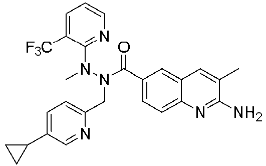 | 2-amino-N-((5-cyclopropylpyridin-2-yl) methyl)-N',3-dimethyl-N'-(3-(trifluorome thyl)pyridin-2-yl)quinoline-6-carbohydrazide |
| 197 | 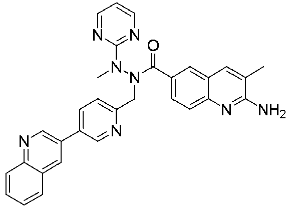 | 2-amino-N',3-dimethyl-N'-(pyrimidin-2-yl)-N-((5-(quinolin-3-yl)pyridin-2-yl)meth yl)quinoline-6-carbohydrazide |
| 198 | 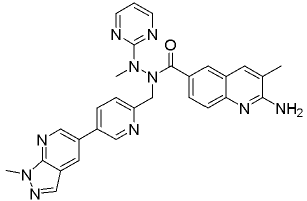 | 2-amino-N',3-dimethyl-N-((5-(1-methyl-1H-pyrazolo[3,4-b]pyridin-5-yl)pyridin-2 -yl)methyl)-N'-(pyrimidin-2-yl)quinoline-6-carbohydrazide |
| 199 | 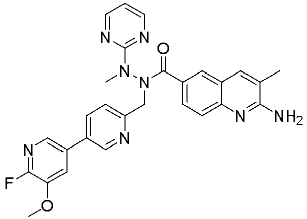 | 2-amino-N-((6'-fluoro-5'-methoxy-[3,3'-bipyridine]-6-yl)methyl)-N',3-dimethyl-N'-(pyrimidin-2-yl)quinoline-6-carbohydrazide |
| 200 | 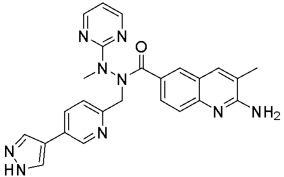 | N-((5-(1H-pyrazol-4-yl)pyridin-2-yl)met hyl)-2-amino-N',3-dimethyl-N'-(pyrimidin -2-yl)quinoline-6-carbohydrazide |
| 201 | 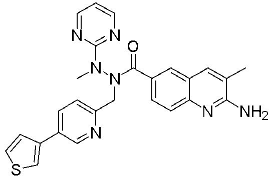 | 2-amino-N',3-dimethyl-N'-(pyrimidin-2-y 1)-N-((5-(thien-3-yl)pyridin-2-yl)methyl) quinoline-6-carbohydrazide |
| 202 | 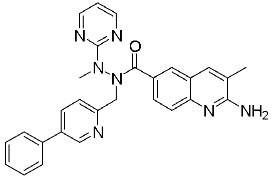 | 2-amino-N',3-dimethyl-N-((5-phenylpyri din-2-yl)methyl)-N'-(pyrimidin-2-yl)quin oline-6-carbohydrazide |
| 203 | 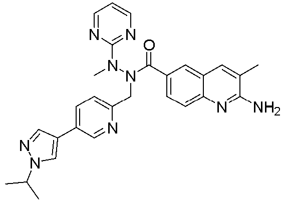 | 2-amino-N-((5-(1-isopropyl-1H-pyrazol-4-yl)pyridin-2-yl)methyl)-N',3-dimethyl-N'-(pyrimidin-2-yl)quinoline-6-carbohydrazide |
| 204 | 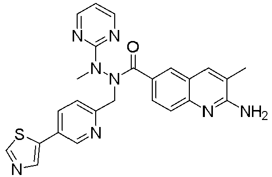 | 2-amino-N',3-dimethyl-N'-(pyrimidin-2-y l)-N-((5-(thiazol-5-yl)pyridin-2-yl)methyl )quinoline-6-carbohydrazide |
| 205 | 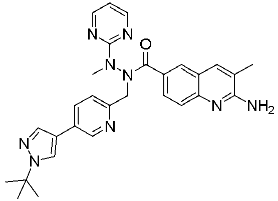 | 2-amino-N-((5-(1-(tert-butyl)-1H-pyrazol -4-yl)pyridin-2-yl)methyl)-N',3-dimethyl-N'-(pyrimidin-2-yl)quinoline-6-carbohydr azide |
| 206 | 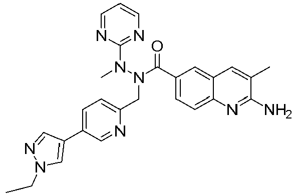 | 2-amino-N-((5-(1-ethyl-1H-pyrazol-4-yl) pyridin-2-yl)methyl)-N',3-dimethyl-N'-(p yrimidin-2-yl)quinoline-6-carbohydrazide |
| 207 | 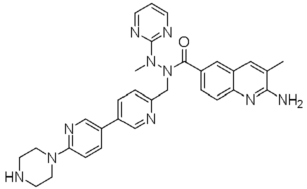 | 2-amino-N',3-dimethyl-N-((6'-(piperazin-1-yl)-[3,3'-bipyridine]-6-yl)methyl)-N'-(p yrimidin-2-yl)quinoline-6-carbohydrazide |
| 208 | 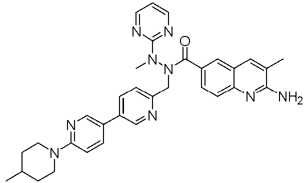 | 2-amino-N',3-dimethyl-N-((6'-(4-methylp iperid-1-yl)-[3,3'-bipyridine]-6-yl)methyl )-N'-(pyrimidin-2-yl)quinoline-6-carbohy drazide |
| 209 | 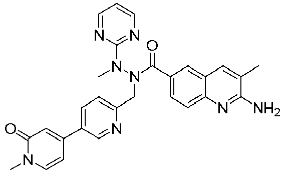 | 2-amino-N',3-dimethyl-N-((1'-methyl-2'-oxo-1',2'-dihydro-[3,4'-bipyridine]-6-yl)m ethyl)-N'-(pyrimidin-2-yl)quinoline-6-car bohydrazide |
| 210 | 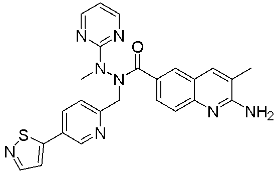 | 2-amino-N-((5-(isothiazol-5-yl)pyridin-2-yl)methyl)-N',3-dimethyl-N'-(pyrimidin-2 -yl)quinoline-6-carbohydrazide |
| 211 | 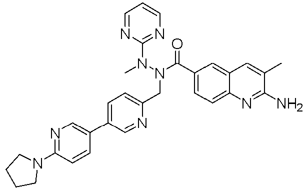 | 2-amino-*N*',3-dimethyl-*N*'-(pyrimidin-2-yl )-*N*-((6'-(pyrrolidin-1-yl)-[3,3'-bipyridine ]-6-yl)methyl)quinoline-6-carbohydrazide |
| 212 | 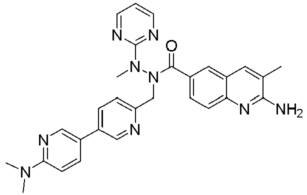 | 2-amino-*N*-((6'-(dimethylamino)-[3,3'-bip yridine]-6-yl)methyl)-*N*',3-dimethyl-*N*'-( pyrimidin-2-yl)quinoline-6-carbohydrazide |
| 213 | 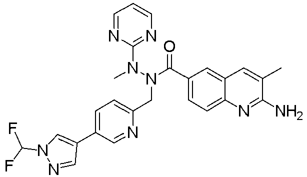 | 2-amino-*N*-((5-(1-(difluoromethyl)-1*H*-pyrazol-4-yl)pyridin-2-yl)methyl)-*N*',3-di methyl-*N*'-(pyrimidin-2-yl)quinoline-6-c arbohydrazide |
| 214 | 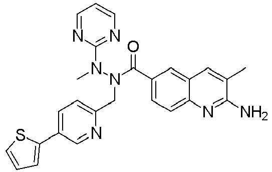 | 2-amino-*N*',3-dimethyl-*N*'-(pyrimidin-2-yl)-*N*-((5-(thien-2-yl)pyridin-2-yl)methyl) quinoline-6-carbohydrazide |
| 215 | 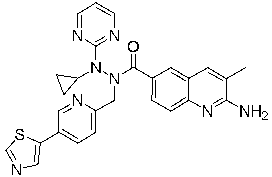 | 2-amino-N'-cyclopropyl-3-methyl-N'-(py rimidin-2-yl)-N-((5-(thiazol-5-yl)pyridin-2-yl)methyl)quinoline-6-carbohydrazide |
| 216 | 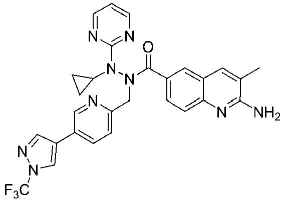 | 2-amino-N'-cyclopropyl-3-methyl-N'-(py rimidin-2-yl)-N-((5-(1-(trifluoromethyl)-1H-pyrazol-4-yl)pyridin-2-yl)methyl)qui noline-6-carbohydrazide |
| 217 | 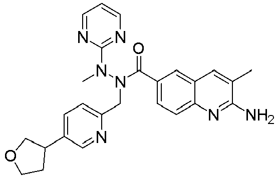 | 2-amino-N',3-dimethyl-N'-(pyrimidin-2-yl )-N-((5-(tetrahydrofuran-3-yl)pyridin-2-yl)methyl)quinoline-6-carbohydrazide |
| 218 | 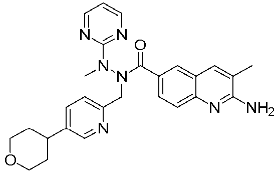 | 2-amino-N',3-dimethyl-N'-(pyrimidin-2-yl )-N-((5-(tetrahydro-2H-pyran-4-yl)pyridi n-2-yl)methyl)quinoline-6-carbohydrazide |
| 219 | 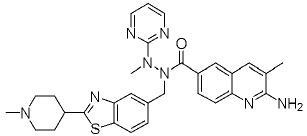 | 2-amino-N',3-dimethyl-N-((2-(1-methyl-1,2,3,6-tetrahydropyridin-4-yl)benzo[d] thiazol-5-yl)methyl)-N'-(pyrimidin-2-yl)qui noline-6-carbohydrazide |
| 220 | 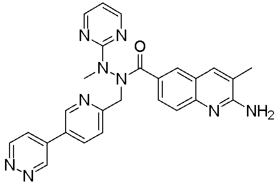 | 2-amino-N',3-dimethyl-N-((5-(pyridazin-4-yl)pyridin-2-yl)methyl)-N'-(pyrimidin-2-yl)quinoline-6-carbohydrazide |
| 221 | 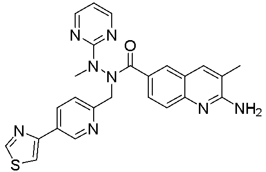 | 2-amino-N',3-dimethyl-N'-(pyrimidin-2-yl)-N-((5-(thiazol-4-yl)pyridin-2-yl)methyl )quinoline-6-carbohydrazide |
| 222 | 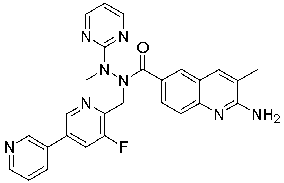 | 2-amino-N-((5-fluoro-[3,3'-bipyridine]-6-yl)methyl)-N',3-dimethyl-N'-(pyrimidin-2 -yl)quinoline-6-carbohydrazide |
| 223 | 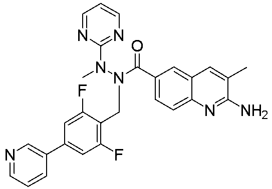 | 2-amino-N-(2,6-difluoro-4-(pyridin-3-yl) benzyl)-N',3-dimethyl-N'-(pyrimidin-2-yl )quinoline-6-carbohydrazide |
| 224 | 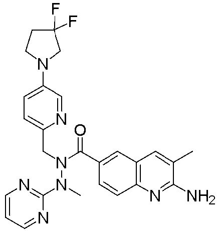 | 2-amino-N-((5-(3,3-difluoropyrrolidin-1-yl)pyridin-2-yl)methyl)-N',3-dimethyl-N' -(pyrimidin-2-yl)quinoline-6-carbohydrazide |
| 225 | 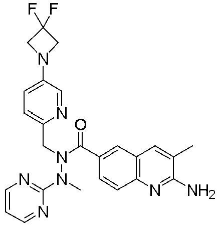 | 2-amino-N-((5-(3,3-difluoroazetidin-1-yl) pyridin-2-yl)methyl)-N',3-dimethyl-N'-( pyrimidin-2-yl)quinoline-6-carbohydrazide |
| 226 | 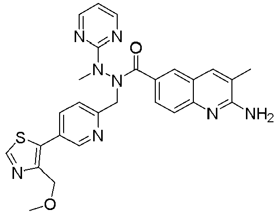 | 2-amino-N-((5-(4-(methoxymethyl)thiazo 1-5-yl)pyridin-2-yl)methyl)-N',3-dimethyl -N'-(pyrimidin-2-yl)quinoline-6-carbohydrazide |
| 227 | 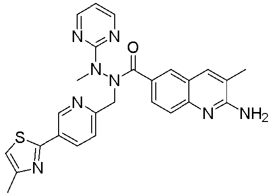 | 2-amino-N',3-dimethyl-N-((5-(4-methylth iazol-2-yl)pyridin-2-yl)methyl)-N'-(pyrim idin-2-yl)quinoline-6-carbohydrazide |
| 228 | 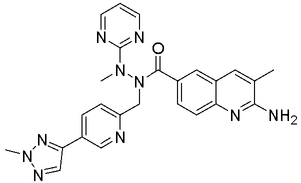 | 2-amino-N',3-dimethyl-N-((5-(2-methyl-2H-1,2,3-triazol-4-yl)pyridin-2-yl)methyl )-N'-(pyrimidin-2-yl)quinoline-6-carbohy drazide |
| 229 | 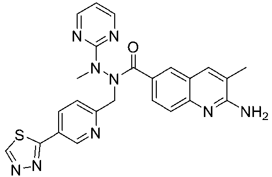 | N-((5-(1,3,4-thiadiazol-2-yl)pyridin-2-yl) methyl)-2-amino-N',3-dimethyl-N'-(pyri midin-2-yl)quinoline-6-carbohydrazide |
| 230 | 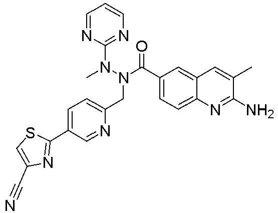 | 2-amino-N-((5-(4-cyanothiazol-2-yl)pyri din-2-yl)methyl)-N',3-dimethyl-N'-(pyri midin-2-yl)quinoline-6-carbohydrazide |
| 231 | 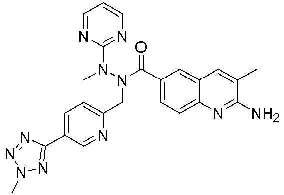 | 2-amino-N',3-dimethyl-N-((5-(2-methyl-2H-tetrazol-5-yl)pyridin-2-yl)methyl)-N'-(pyrimidin-2-yl)quinoline-6-carbohydrazide |
| 232 | 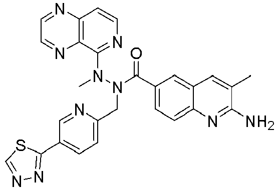 | N-((5-(1,3,4-thiadiazol-2-yl)pyridin-2-yl) methyl)-2-amino-N',3-dimethyl-N'-(pyrid o[3,4-b]pyrazin-5-yl)quinoline-6-carbohy drazide |
| 233 | 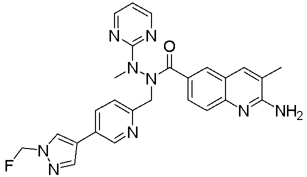 | 2-amino-N-((5-(1-(fluoromethyl)-1H-pyr azol-4-yl)pyridin-2-yl)methyl)-N',3-dimet hyl-N'-(pyrimidin-2-yl)quinoline-6-carbo hydrazide |
| 234 | 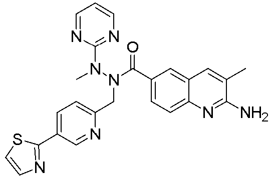 | 2-amino-N',3-dimethyl-N'-(pyrimidin-2-yl)-N-((5-(thiazol-2-yl)pyridin-2-yl)methyl )quinoline-6-carbohydrazide |
| 235 | 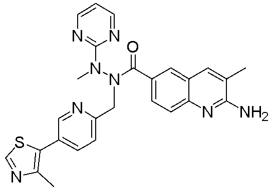 | 2-amino-N',3-dimethyl-N-((5-(4-methylth iazol-5-yl)pyridin-2-yl)methyl)-N'-(pyrim idin-2-yl)quinoline-6-carbohydrazide |
| 236 | 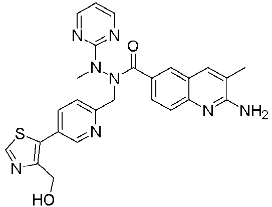 | 2-amino-N-((5-(4-(hydroxymethyl)thiazo 1-5-yl)pyridin-2-yl)methyl)-N',3-dimethyl -N'-(pyrimidin-2-yl)quinoline-6-carbohyd razide |
| 237 | 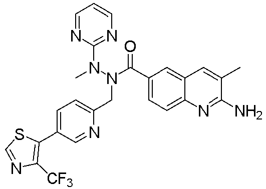 | 2-amino-N',3-dimethyl-N'-(pyrimidin-2-y 1)-N-((5-(4-(trifluoromethyl)thiazol-5-yl) pyridin-2-yl)methyl)quinoline-6-carbohy drazide |
| 238 | 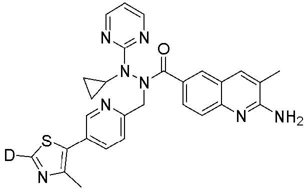 | 2-amino-N'-cyclopropyl-3-methyl-N-((5-(4-methylthiazol-5-yl-2d)pyridin-2-yl)me thyl)-N'-(pyrimidin-2-yl)quinoline-6-carb ohydrazide |
| 239 | 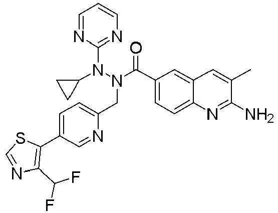 | 2-amino-N'-cyclopropyl-N-((5-(4-(difluor omethyl)thiazol-5-yl)pyridin-2-yl)methyl )-3-methyl-N'-(pyrimidin-2-yl)quinoline-6-carbohydrazide |
| 240 | 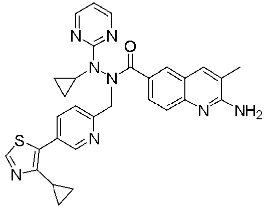 | 2-amino-N'-cyclopropyl-N-((5-(4-cyclopr opylthiazol-5-yl)pyridin-2-yl)methyl)-3-methyl-N'-(pyrimidin-2-yl)quinoline-6-carbohydrazide |
| 241 | 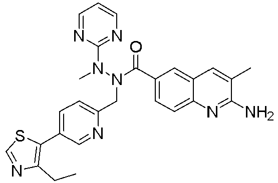 | 2-amino-N-((5-(4-ethylthiazol-5-yl)pyridin -2-yl)methyl)-N',3-dimethyl-N'-(pyrimi din-2-yl)quinoline-6-carbohydrazide |
| 242 | 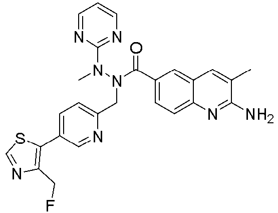 | 2-amino-N-((5-(4-(fluoromethyl)thiazol-5-yl)pyridin-2-yl)methyl)-N',3-dimethyl-N'-(pyrimidin-2-yl)quinoline-6-carbohydrazide |
| 243 | 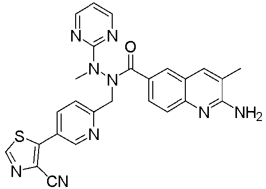 | 2-amino-N-((5-(4-cyanothiazol-5-yl)pyri din-2-yl)methyl)-N',3-dimethyl-N'-(pyri midin-2-yl)quinoline-6-carbohydrazide |
| 244 | 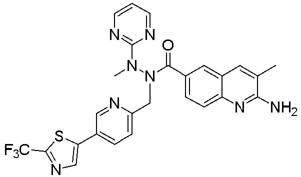 | 2-amino-N',3-dimethyl-N'-(pyrimidin-2-yl)-N-((5-(2-(trifluoromethyl)thiazol-5-yl) pyridin-2-yl)methyl)quinoline-6-carbohydrazide |
| 245 | 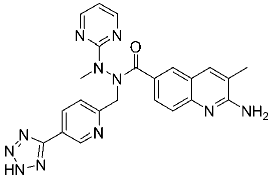 | N-((5-(2H-tetrazol-5-yl)pyridin-2-yl)met hyl)-2-amino-N',3-dimethyl-N'-(pyrimidi n-2-yl)quinoline-6-carbohydrazide |
| 246 | 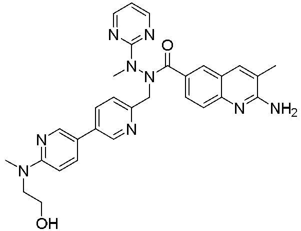 | 2-amino-N-((6'-((2-hydroxyethyl)(methyl )amino)-[3,3'-bipyridine]-6-yl)methyl)-N' ,3-dimethyl-N'-(pyrimidin-2-yl)quinoline -6-carbohydrazide |
| 247 | 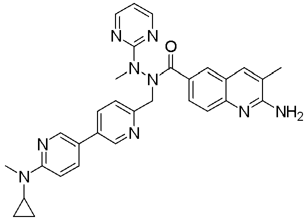 | 2-amino-N-((6'-(cyclopropyl(methyl)ami no)-[3,3'-bipyridine]-6-yl)methyl)-N',3-di methyl-N'-(pyrimidin-2-yl)quinoline-6-carbohydrazide |
| 248 | 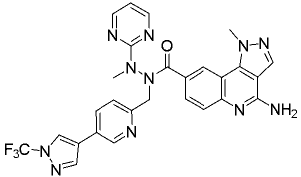 | 4-amino-N',1-dimethyl-N'-(pyrimidin-2-yl)-N-((5-(1-(trifluoromethyl)-1H-pyrazo l-4-yl)pyridin-2-yl)methyl)-1Hpyrazolo[4 ,3-c]quinoline-8-carbohydrazide |
| 249 | 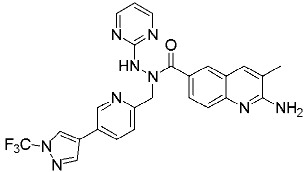 | 2-amino-3-methyl-N'-(pyrimidin-2-yl)-N-((5-(1-(trifluoromethyl)-1H-pyrazol-4-yl) pyridin-2-yl)methyl)quinoline-6-carbohy drazide |
| 250 | 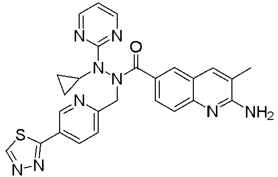 | N-((5-(1,3,4-thiadiazol-2-yl)pyridin-2-yl) methyl)-2-amino-N'-cyclopropyl-3-methy l-N'-(pyrimidin-2-yl)quinoline-6-carbohy drazide |
| 251 | 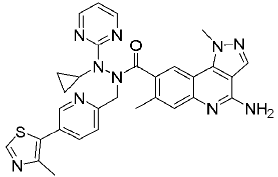 | 4-amino-N'-cyclopropyl-1,7-dimethyl-N-((5-(4-methylthiazol-5-yl)pyridin-2-yl)m ethyl)-N'-(pyrimidin-2-yl)-1H-pyrazolo[4 ,3-c]quinoline-8-carbohydrazide |
| 252 | 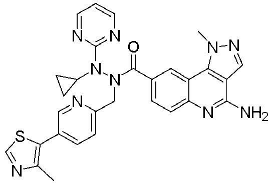 | 4-amino-N'-cyclopropyl-1-methyl-N-((5-(4-methylthiazol-5-yl)pyridin-2-yl)methy l)-N'-(pyrimidin-2-yl)-1H-pyrazolo[4,3-c] quinoline-8-carbohydrazide |
| 253 | 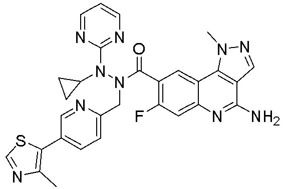 | 4-amino-N'-cyclopropyl-7-fluoro-1-meth yl-N-((5-(4-methylthiazol-5-yl)pyridin-2-yl)methyl)-N'-(pyrimidin-2-yl)-1H-pyraz olo[4,3-c]quinoline-8-carbohydrazide |
| 254 | 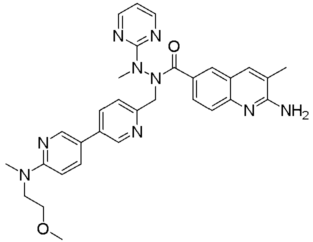 | 2-amino-N-((6'-((2-methoxyethyl)(methy l)amino)-[3,3'-bipyridin]-6-yl)methyl)-N', 3-dimethyl-N'-(pyrimidin-2-yl)quinoline-6-carbohydrazide |
| 255 | 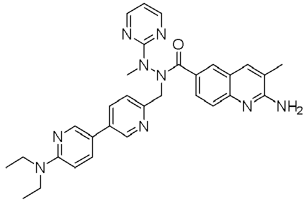 | 2-amino-N-((6'-(diethylamino)-[3,3'-bipy ridin]-6-yl)methyl)-N',3-dimethyl-N'-(pyr imidin-2-yl)quinoline-6-carbohydrazide |
| 256 | 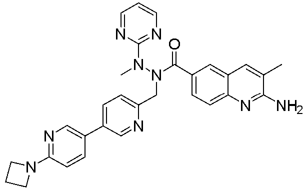 | 2-amino-N-((6'-(azetidin-1-yl)-[3,3'-bipyr idin]-6-yl)methyl)-N',3-dimethyl-N'-(pyri midin-2-yl)quinoline-6-carbohydrazide |
| 257 | 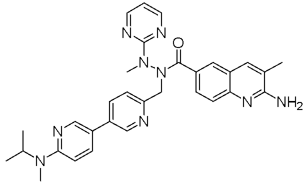 | 2-amino-N-((6'-(isopropyl(methyl)amino) -[3,3'-bipyridin]-6-yl)methyl)-N',3-dimet hyl-N'-(pyrimidin-2-yl)quinoline-6-carbo hydrazide |
| 258 | 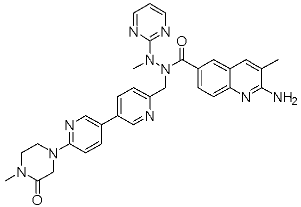 | 2-amino-N',3-dimethyl-N-((6'-(4-methyl-3-oxopiperazin-1-yl)-[3,3'-bipyridin]-6-yl )methyl)-N'-(pyrimidin-2-yl)quinoline-6-carbohydrazide |
| 259 | 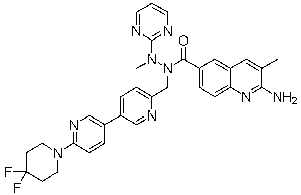 | 2-amino-N-((6'-(4,4-difluoropiperid-1-yl) -[3,3'-bipyridin]-6-yl)methyl)-N',3-dimet hyl-N'-(pyrimidin-2-yl)quinoline-6-carbo hydrazide |
| 260 | 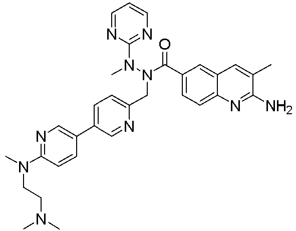 | 2-amino-*N*-((6'-((2-(dimethylamino)ethyl )(methyl)amino)-[3,3'-bipyridin]-6-yl)met hyl)-*N*',3-dimethyl-*N*'-(pyrimidin-2-yl)qui noline-6-carbohydrazide |
| 261 | 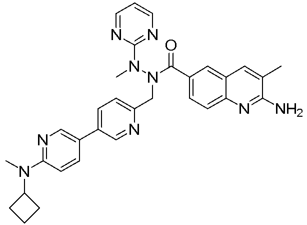 | 2-amino-*N*-((6'-(cyclobutyl(methyl)amin o)-[3,3'-bipyridin]-6-yl)methyl)-*N*',3-dim ethyl-*N*'-(pyrimidin-2-yl)quinoline-6-carb ohydrazide |
| 262 | 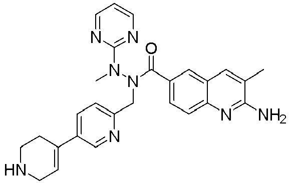 | 2-amino-N',3-dimethyl-N'-(pyrimidin-2-y l)-N-((1',2',3',6'-tetrahydro-[3,4'-bipyridin ]-6-yl)methyl)quinoline-6-carbohydrazide |
| 263 | 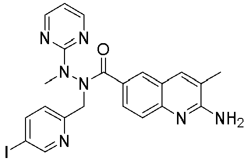 | 2-amino-N-((5-iodopyridin-2-yl)methyl)-N',3-dimethyl-N'-(pyrimidin-2-yl)quinoli ne-6-carbohydrazide |
| 264 | 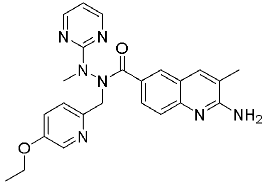 | 2-amino-N-((5-ethoxypyridin-2-yl)methy l)-N',3-dimethyl-N'-(pyrimidin-2-yl)quin oline-6-carbohydrazide |
| 265 | 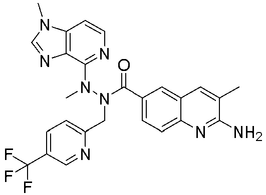 | 2-amino-N',3-dimethyl-N'-(1-methyl-1H-imidazo[4,5-c]pyridin-4-yl)-N-((5-(trifluo romethyl)pyridin-2-yl)methyl)quinoline-6-carbohydrazide |
| 266 | 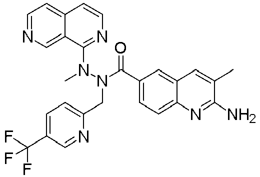 | 2-amino-N',3-dimethyl-N'-(2,7-naphthyri din-1-yl)-N-((5-(trifluoromethyl)pyridin-2-yl)methyl)quinoline-6-carbohydrazide |
| 267 | 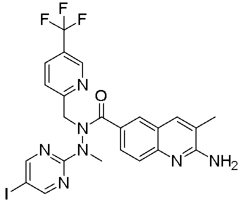 | 2-amino-N'-(5-iodopyrimidin-2-yl)-N',3-dimethyl-N-((5-(trifluoromethyl)pyridin-2-yl)methyl)quinoline-6-carbohydrazide |
| 268 | 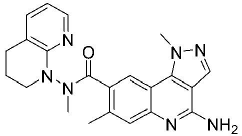 | 4-amino-*N*-(3,4-dihydro-1,8-naphthyridin -1(2*H*)-yl)-*N*,1,7-trimethyl-1*H*-pyrazolo[ 4,3-c]quinoline-8-carboxamide |
| 269 | 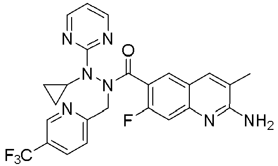 | 2-amino-N'-cyclopropyl-7-fluoro-3-meth yl-N'-(pyrimidin-2-yl)-N-((5-(trifluorome thyl)pyridin-2-yl)methyl)quinoline-6-car bohydrazide |
| 270 | 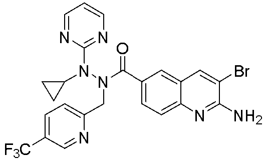 | 2-amino-3-bromo-N'-cyclopropyl-N'-(pyr imidin-2-yl)-N-((5-(trifluoromethyl)pyrid in-2-yl)methyl)quinoline-6-carbohydrazide |
| 271 | 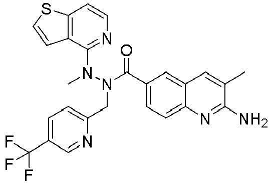 | 2-amino-N',3-dimethyl-N'-(thieno[3,2-c]p yridin-4-yl)-N-((5-(trifluoromethyl)pyridi n-2-yl)methyl)quinoline-6-carbohydrazide |
| 272 | 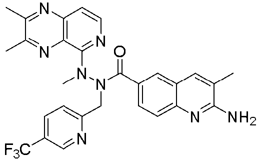 | 2-amino-N'-(2,3-dimethylpyrido[3,4-b]py razin-5-yl)-N',3-dimethyl-N-((5-(trifluoro methyl)pyridin-2-yl)methyl)quinoline-6-carbohydrazide |
| 273 | 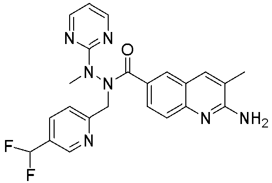 | 2-amino-N-((5-(difluoromethyl)pyridin-2 -yl)methyl)-N',3-dimethyl-N'-(pyrimidin-2-yl)quinoline-6-carbohydrazide |
| 274 | 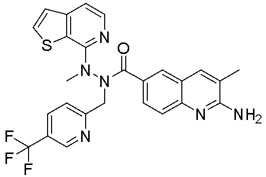 | 2-amino-N',3-dimethyl-N'-(thieno[2,3-c]p yridin-7-yl)-N-((5-(trifluoromethyl)pyridi n-2-yl)methyl)quinoline-6-carbohydrazide |
| 275 | 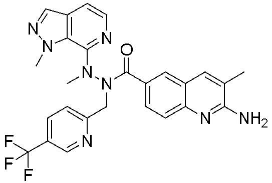 | 2-amino-N',3-dimethyl-N'-(1-methyl-1H-pyrazolo[3,4-c]pyridin-7-yl)-N-((5-(triflu oromethyl)pyridin-2-yl)methyl)quinoline -6-carbohydrazide |
| 276 | 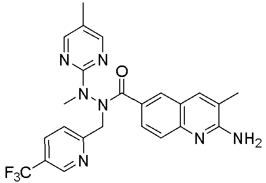 | 2-amino-N',3-dimethyl-N'-(5-methylpyri midin-2-yl)-N-((5-(trifluoromethyl)pyridi n-2-yl)methyl)quinoline-6-carbohydrazide |
| 277 | 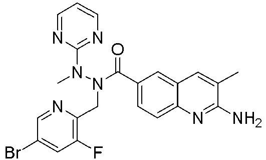 | 2-amino-N-((5-bromo-3-fluoropyridin-2-yl)methyl)-N',3-dimethyl-N'-(pyrimidin-2 -yl)quinoline-6-carbohydrazide |
| 278 | 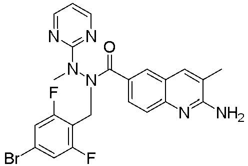 | 2-amino-N-(4-bromo-2,6-difluorobenzyl) -N',3-dimethyl-N'-(pyrimidin-2-yl)quinol ine-6-carbohydrazide |
| 279 | 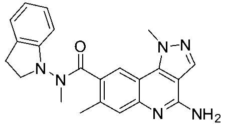 | 4-amino-*N*-(indol-1-yl)-*N*,1,7-trimethyl-1 *H*-pyrazolo[4,3-*c*]quinoline-8-carboxami de |
| 280 | 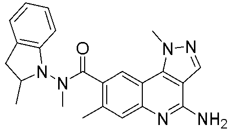 | 4-amino-*N*,1,7-trimethyl-*N*-(2-methylind ol-1-yl)-1*H*-pyrazolo[4,3-*c*]quinoline-8-carboxamide |
| 281 | 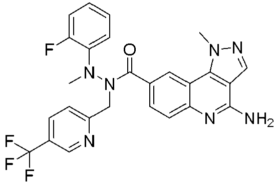 | 4-amino-*N*'-(2-fluorophenyl)-*N',1*-dimeth yl-*N*-((5-(trifluoromethyl)pyridin-2-yl)me thyl)-1*H*-pyrazolo[4,3-*c*]quinoline-8-carb ohydrazide |
| 282 | 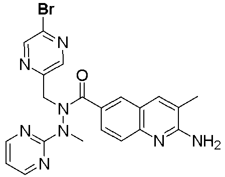 | 2-amino-N-((5-bromopyrazin-2-yl)methy l)-N',3-dimethyl-N'-(pyrimidin-2-yl)quin oline-6-carbohydrazide |
| 283 | 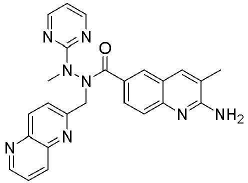 | N-((1,5-naphthyridin-2-yl)methyl)-2-ami no-N',3-dimethyl-N'-(pyrimidin-2-yl)qui noline-6-carbohydrazide |
| 284 | 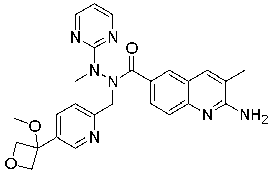 | 2-amino-N-((5-(3-methoxyoxetan-3-yl)p yridin-2-yl)methyl)-N',3-dimethyl-N'-(py rimidin-2-yl)quinoline-6-carbohydrazide |
| 285 | 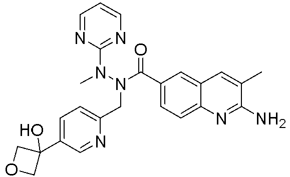 | 2-amino-N-((5-(3-hydroxyoxetan-3-yl)py ridin-2-yl)methyl)-N',3-dimethyl-N'-(pyri midin-2-yl)quinoline-6-carbohydrazide |
| 286 | 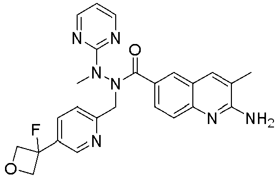 | 2-amino-N-((5-(3-fluorooxetan-3-yl)pyri din-2-yl)methyl)-N',3-dimethyl-N'-(pyri midin-2-yl)quinoline-6-carbohydrazide |
| 287 | 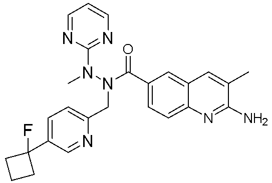 | 2-amino-N-((5-(1-fluorocyclobutyl)pyridi n-2-yl)methyl)-N',3-dimethyl-N'-(pyrimi din-2-yl)quinoline-6-carbohydrazide |
| 288 | 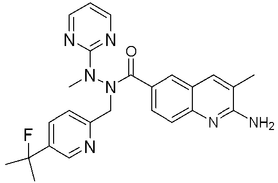 | 2-amino-N-((5-(2-fluoroprop-2-yl)pyridi n-2-yl)methyl)-N',3-dimethyl-N'-(pyrimi din-2-yl)quinoline-6-carbohydrazide |
| 289 | 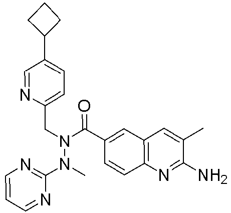 | 2-amino-N-((5-cyclobutylpyridin-2-yl)m ethyl)-N',3-dimethyl-N'-(pyrimidin-2-yl) quinoline-6-carbohydrazide |
| 290 | 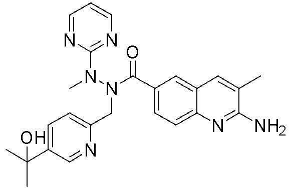 | 2-amino-N-((5-(2-hydroxyprop-2-yl)pyri din-2-yl)methyl)-N',3-dimethyl-N'-(pyri midin-2-yl)quinoline-6-carbohydrazide |
| 291 | 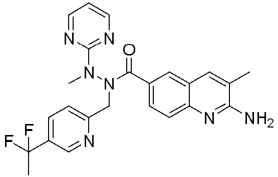 | 2-amino-N-((5-(1,1-difluoroethyl)pyridin -2-yl)methyl)-N',3-dimethyl-N'-(pyrimidi n-2-yl)quinoline-6-carbohydrazide |
| 292 | 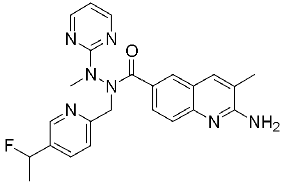 | 2-amino-N-((5-(1-fluoroethyl)pyridin-2-y l)methyl)-N',3-dimethyl-N'-(pyrimidin-2-yl)quinoline-6-carbohydrazide |
| 293 | 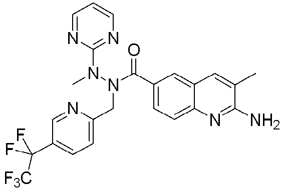 | 2-amino-N',3-dimethyl-N-((5-(perfluoroe thyl)pyridin-2-yl)methyl)-N'-(pyrimidin-2-yl)quinoline-6-carbohydrazide |
| 294 | 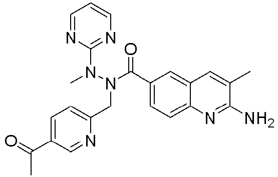 | N-((5-acetylpyridin-2-yl)methyl)-2-amin o-N',3-dimethyl-N'-(pyrimidin-2-yl)quino line-6-carbohydrazide |
| 295 | 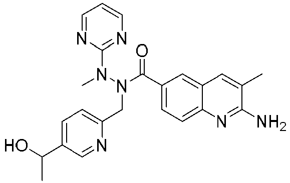 | 2-amino-N-((5-(1-hydroxyethyl)pyridin-2 -yl)methyl)-N',3-dimethyl-N'-(pyrimidin-2-yl)quinoline-6-carbohydrazide |
| 296 | 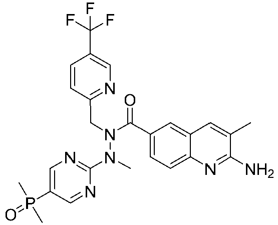 | 2-amino-N'-(5-(dimethylphosphoryl)pyri midin-2-yl)-N',3-dimethyl-N-((5-(trifluor omethyl)pyridin-2-yl)methyl)quinoline-6 -carbohydrazide |
| 297 | 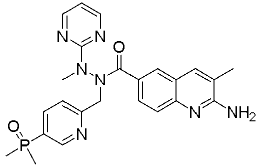 | 2-amino-N-((5-(dimethylphosphoryl)pyri din-2-yl)methyl)-N',3-dimethyl-N'-(pyri midin-2-yl)quinoline-6-carbohydrazide |
| 298 | 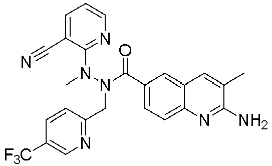 | 2-amino-N'-(3-cyanopyridin-2-yl)-N',3-di methyl-N-((5-(trifluoromethyl)pyridin-2-yl)methyl)quinoline-6-carbohydrazide |
| 299 | 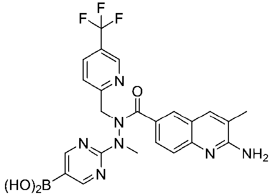 | (2-(2-(-2-amino-3-methylquinoline-6-car bonyl)-1-methyl-2-((5-(trifluoromethyl)p yridin-2-yl)methyl)hydrazino)pyrimidin-5-yl)boronic acid |
| 300 | 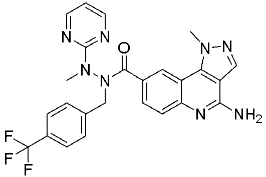 | 4-amino-N',1-dimethyl-N'-(pyrimidin-2-y l)-N-(4-(trifluoromethyl)benzyl)-1H-pyra zolo[4,3-c]quinoline-8-carbohydrazide |
| 301 | 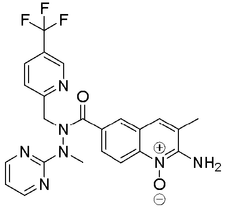 | 2-amino-3-methyl-6-(2-methyl-2-(pyrimi din-2-yl)-1-((5-(trifluoromethyl)pyridin-2 -yl)methyl)hydrazine-1-carbonyl)quinoli ne-1-oxide |
| 302 | 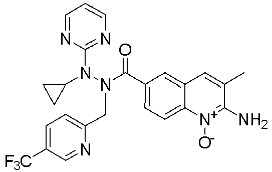 | 2-amino-6-(2-cyclopropyl-2-(pyrimidin-2 -yl)-1-((5-(trifluoromethyl)pyridin-2-yl) methyl)hydrazine-1-carbonyl)-3-methylq uinoline-1-oxide |
| 303 | 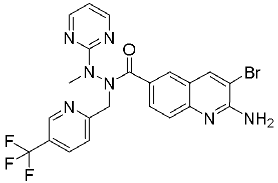 | 2-amino-3-bromo-N'-methyl-N'-(pyrimidi n-2-yl)-N-((5-(trifluoromethyl)pyridin-2-yl)methyl)quinoline-6-carbohydrazide |
| 304 | 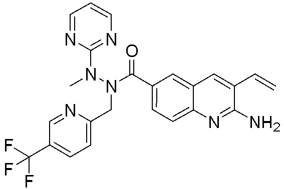 | 2-amino-N'-methyl-N'-(pyrimidin-2-yl)-N-((5-(trifluoromethyl)pyridin-2-yl)meth yl)-3-vinylquinoline-6-carbohydrazide |
| 305 | 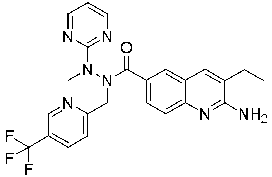 | 2-amino-3-ethyl-N'-methyl-N'-(pyrimidin -2-yl)-N-((5-(trifluoromethyl)pyridin-2-yl )methyl)quinoline-6-carbohydrazide |
| 306 | 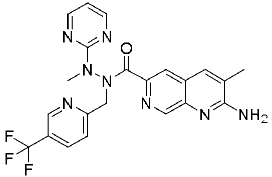 | 2-amino-N',3-dimethyl-N'-(pyrimidin-2-y l)-N-((5-(trifluoromethyl)pyridin-2-yl)me thyl)-1,7-naphthyridine-6-carbohydrazide |
| 307 | 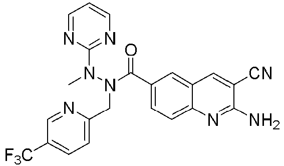 | 2-amino-3-cyano-N'-methyl-N'-(pyrimidi n-2-yl)-N-((5-(trifluoromethyl)pyridin-2-yl)methyl)quinoline-6-carbohydrazide |
| 308 | 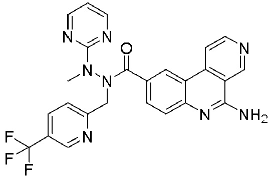 | 5-amino-*N*'-methyl-*N*'-(pyrimidin-2-yl)-*N* -((5-(trifluoromethyl)pyridin-2-yl)methyl )benzo[c] [2,7]naphthyridine-9-carbohydr azide |
| 309 | 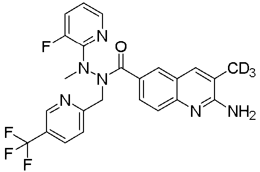 | 2-amino-N'-(3-fluoropyridin-2-yl)-N'-met hyl-3-(methyl-D3)-N-((5-(trifluoromethyl )pyridin-2-yl)methyl)quinoline-6-carbohy drazide |
| 310 | 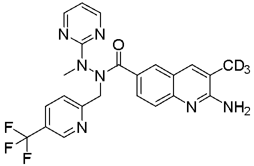 | 2-amino-N'-methyl-3-(methyl-d3)-N'-(py rimidin-2-yl)-N-((5-(trifluoromethyl)pyri din-2-yl)methyl)quinoline-6-carbohydraz ide |
| 311 | 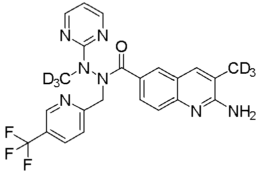 | 2-amino-N',3-bis(methyl-D3)-N'-(pyrimi din-2-yl)-N-((5-(trifluoromethyl)pyridin-2-yl)methyl)quinoline-6-carbohydrazide |
| 312 | 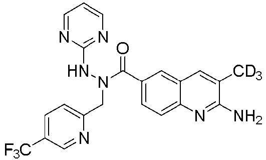 | 2-amino-3-(methyl-D3)-N'-(pyrimidin-2-yl)-N-((5-(trifluoromethyl)pyridin-2-yl)m ethyl)quinoline-6-carbohydrazide |
| 313 | 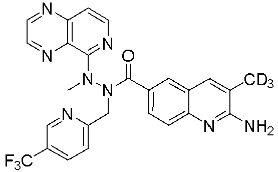 | 2-amino-N'-methyl-3-(methyl-d3)-N'-(py rido[3,4-b]pyrazin-5-yl)-N-((5-(trifluoro methyl)pyridin-2-yl)methyl)quinoline-6-c arbohydrazide |
| 314 | 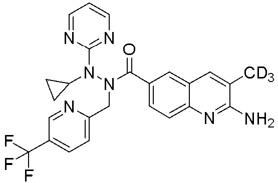 | 2-amino-N'-cyclopropyl-3-(methyl-D3)-N'-(pyrimidin-2-yl)-N-((5-(trifluorometh yl)pyridin-2-yl)methyl)quinoline-6-carbo hydrazide |
| 315 | 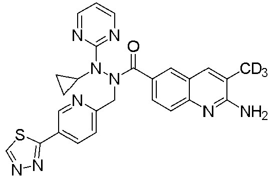 | N-((5-(1,3,4-thiadiazol-2-yl)pyridin-2-yl) methyl)-2-amino-N'-cyclopropyl-3-(meth yl-d3)-N'-(pyrimidin-2-yl)quinoline-6-car bohydrazide |
| 316 | 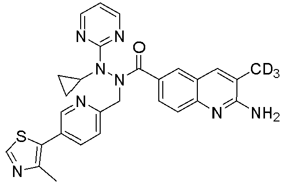 | 2-amino-N'-cyclopropyl-3-(methyl-D3)-N-((5-(4-methylthiazol-5-yl)pyridin-2-yl) methyl)-N'-(pyrimidin-2-yl)quinoline-6-c arbohydrazide |
| 317 | 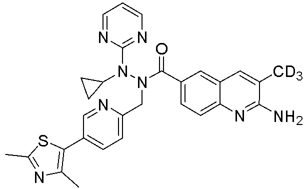 | 2-amino-N'-cyclopropyl-N-((5-(2,4-dimet hylthiazol-5-yl)pyridin-2-yl)methyl)-3-( methyl-D3)-N'-(pyrimidin-2-yl)quinoline -6-carbohydrazide |
| 318 | 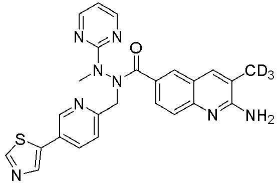 | 2-amino-N'-methyl-3-(methyl-D3)-N'-(py rimidin-2-yl)-N-((5-(thiazol-5-yl)pyridin-2-yl)methyl)quinoline-6-carbohydrazide |
| 319 | 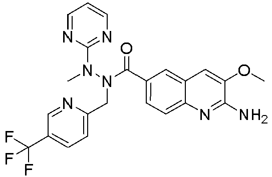 | 2-amino-3-methoxy-N'-methyl-N'-(pyrim idin-2-yl)-N-((5-(trifluoromethyl)pyridin-2-yl)methyl)quinoline-6-carbohydrazide |
| 320 | 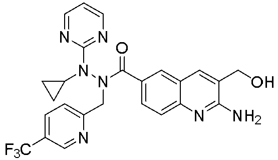 | 2-amino-N'-cyclopropyl-3-(hydroxymeth yl)-N'-(pyrimidin-2-yl)-N-((5-(trifluorom ethyl)pyridin-2-yl)methyl)quinoline-6-car bohydrazide |
| 321 | 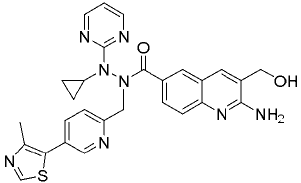 | 2-amino-N'-cyclopropyl-3-hydroxymethy l-N-((5-(4-methylthiazol-5-yl)pyridin-2-y l)methyl)-N'-(pyrimidin-2-yl)quinoline-6-carbohydrazide |
| 322 | 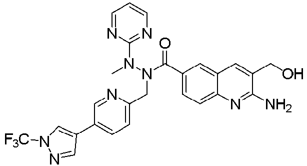 | 2-amino-3-(hydroxymethyl)-N'-methyl-N '-(pyrimidin-2-yl)-N-((5-(1-(trifluorometh yl)-1H-pyrazol-4-yl)pyridin-2-yl)methyl) quinoline-6-carbohydrazide |
| 323 | 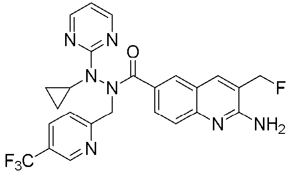 | 2-amino-N'-cyclopropyl-3-(fluoromethyl) -N'-(pyrimidin-2-yl)-N-((5-(trifluorometh yl)pyridin-2-yl)methyl)quinoline-6-carbo hydrazide |
| 324 | 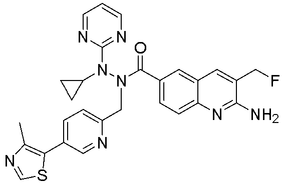 | 2-amino-N'-cyclopropyl-3-(fluoromethyl) -N-((5-(4-methylthiazol-5-yl)pyridin-2-yl )methyl)-N'-(pyrimidin-2-yl)quinoline-6-carbohydrazide |
| 325 | 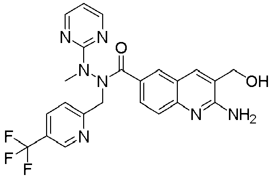 | 2-amino-3-hydroxymethyl-N'-methyl-N'-(pyrimidin-2-yl)-N-((5-(trifluoromethyl)p yridin-2-yl)methyl)quinoline-6-carbohydr azide |
| 326 | 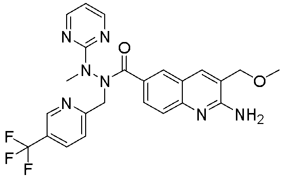 | 2-amino-3-(methoxymethyl)-N'-methyl-N'-(pyrimidin-2-yl)-N-((5-(trifluorometh yl)pyridin-2-yl)methyl)quinoline-6-carbo hydrazide |
| 327 | 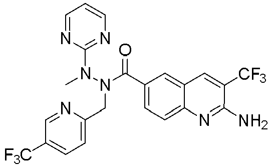 | 2-amino-N'-methyl-N'-(pyrimidin-2-yl)-3 -(trifluoromethyl)-N-((5-(trifluoromethyl) pyridin-2-yl)methyl)quinoline-6-carbohy drazide |
| 328 | 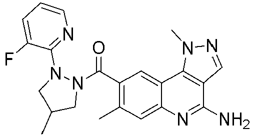 | (4-amino-1,7-dimethyl-1H-pyrazolo[4,3-c]quinolin-8-yl)(2-(3-fluoropyridin-2-yl)-4-methylpyrazolidin-1-yl)methanone |
| 329 | 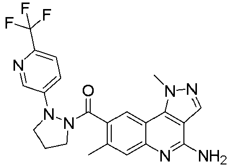 | (4-amino-1,7-dimethyl-1H-pyrazolo[4,3-c]quinolin-8-yl)(2-(6-(trifluoromethyl)py ridin-3-yl)pyrazolidin-1-yl)methanone |
| 330 | 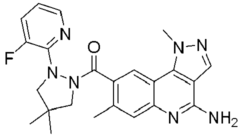 | (4-amino-1,7-dimethyl-1H-pyrazolo[4,3-c]quinolin-8-yl)(2-(3-fluoropyridin-2-yl)-4,4-dimethylpyrazolidin-1-yl)methanone |
| 331 | 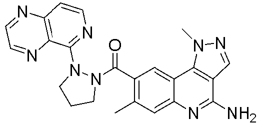 | (4-amino-1,7-dimethyl-1H-pyrazolo[4,3-c]quinolin-8-yl)(2-(pyrido[3,4-b]pyrazin-5-yl)pyrazolidin-1-yl)methanone |
| 332 | 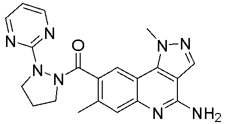 | (4-amino-1,7-dimethyl-1H-pyrazolo[4,3-c]quinolin-8-yl)(2-(pyrimidin-2-yl)pyraz olidin-1-yl)methanone |
| 333 | 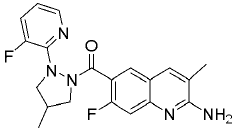 | (2-amino-7-fluoro-3-methylquinolin-6-yl )(2-(3-fluoropyridin-2-yl)-4-methylpyraz olidin-1-yl)methanone |
| 334 | 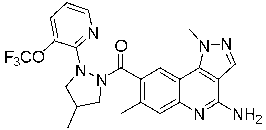 | (4-amino-1,7-dimethyl-1H-pyrazolo[4,3-c]quinolin-8-yl)(4-methyl-2-(3-(trifluoro methoxy)pyridin-2-yl)pyrazolidin-1-yl)m ethanone |
| 335 | 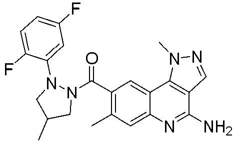 | (4-amino-1,7-dimethyl-1H-pyrazolo[4,3-c]quinolin-8-yl)(2-(2,5-difluorophenyl)-4 -methylpyrazolidin-1-yl)methanone |
| 336 | 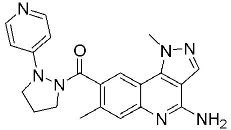 | (4-amino-1,7-dimethyl-1H-pyrazolo[4,3-c]quinolin-8-yl)(2-(pyridin-4-yl)pyrazoli din-1-yl)methanone |
| 337 | 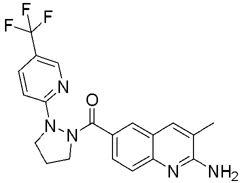 | (2-amino-3-methylquinolin-6-yl)(2-(5-(tri fluoromethyl)pyridin-2-yl)pyrazolidin-1-yl)methanone |
| 338 | 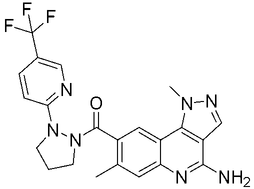 | (4-amino-1,7-dimethyl-1H-pyrazolo[4,3-c]**quinolin**-8-yl)(2-(5-(trifluoromethyl)py ridin-2-yl)pyrazolidin-1-yl)methanone |
| 339 | 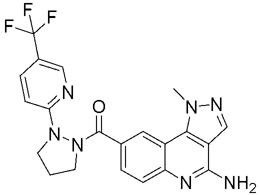 | (4-amino-1-methyl-1H-pyrazolo[4,3-c]qu inolin-8-yl)(2-(5-(trifluoromethyl)pyridin -2-yl)pyrazolidin-1-yl)methanone |
| 340 | 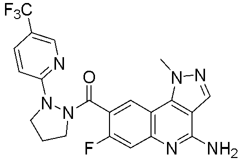 | (4-amino-7-fluoro-1-methyl-1H-pyrazolo [4,3-c]quinolin-8-yl)(2-(5-(trifluoromethy l)pyridin-2-yl)pyrazolidin-1-yl)methanone |
| 341 | 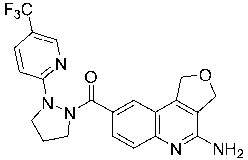 | (4-amino-1,3-dihydrofuro[3,4-c]quinolin-8-yl)(2-(5-(trifluoromethyl)pyridin-2-yl)p yrazolidin-1-yl)methanone |
| 342 | 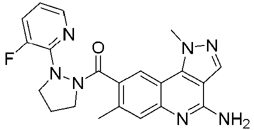 | (4-amino-1,7-dimethyl-1H-pyrazolo[4,3-c]quinolin-8-yl)(2-(3-fluoropyridin-2-yl) pyrazolidin-1-yl)methanone |
| 343 | 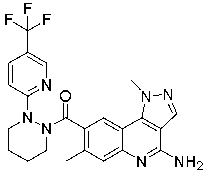 | (4-amino-1,7-dimethyl-1H-pyrazolo[4,3-c]quinolin-8-yl)(2-(5-(trifluoromethyl)py ridin-2-yl)tetrahydropyridazin-1 (2H)-yl) methanone |
| 344 | 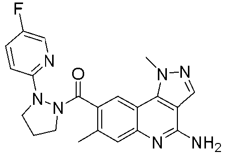 | (4-amino-1,7-dimethyl-1H-pyrazolo[4,3-c]quinolin-8-yl)(2-(5-fluoropyridin-2-yl) pyrazolidin-1-yl)methanone |
| 345 | 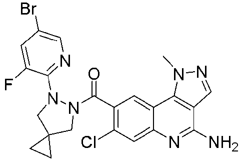 | (4-amino-7-chloro-1-methyl-1H-pyrazolo [4,3-c]quinolin-8-yl)(6-(5-bromo-3-fluor opyridin-2-yl)-5,6-diazaspiro[2.4]heptan-5-yl)methanone |
| 346 | 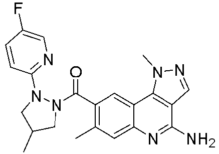 | (4-amino-1,7-dimethyl-1H-pyrazolo[4,3-c]quinolin-8-yl)(2-(5-fluoropyridin-2-yl)-4-methylpyrazolidin-1-yl)methanone |
| 347 | 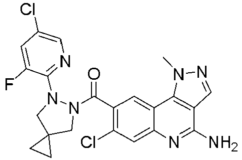 | (4-amino-7-chloro-1-methyl-1H-pyrazolo [4,3-c]quinolin-8-yl)(6-(5-chloro-3-fluoro pyridin-2-yl)-5,6-diazaspiro[2.4]heptan-5 -yl)methanone |
| 348 | 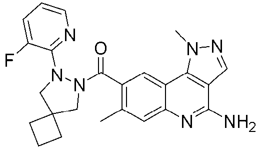 | (4-amino-1,7-dimethyl-1H-pyrazolo[4,3-c]quinolin-8-yl)(7-(3-fluoropyridin-2-yl)-6,7-diazaspiro[3.4]oct-6-yl)methanone |
| 349 | 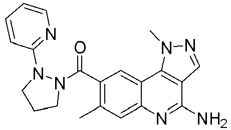 | (4-amino-1,7-dimethyl-1H-pyrazolo[4,3-c]quinolin-8-yl)(2-(pyridin-2-yl)pyrazoli din-1-yl)methanone |
| 350 | 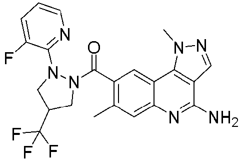 | (4-amino-1,7-dimethyl-1H-pyrazolo[4,3-c]quinolin-8-yl)(2-(3-fluoropyridin-2-yl)-4-(trifluoromethyl)pyrazolidin-1-yl)meth anone |
| 351 | 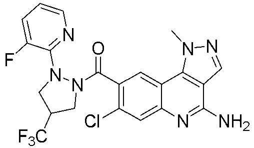 | (4-amino-7-chloro-1-methyl-1H-pyrazolo [4,3-c]quinolin-8-yl)(2-(3-fluoropyridin-2 -yl)-4-(trifluoromethyl)pyrazolidin-1-yl) methanone |
| 352 | 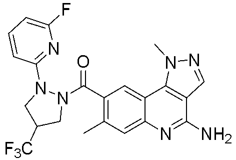 | (4-amino-1,7-dimethyl-1H-pyrazolo[4,3-c]quinolin-8-yl)(2-(6-fluoropyridin-2-yl)-4-(trifluoromethyl)pyrazolidin-1-yl)meth anone |
| 353 | 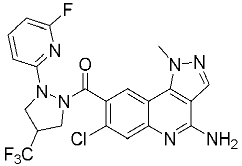 | (4-amino-7-chloro-1-methyl-1H-pyrazolo [4,3-c]quinolin-8-yl)(2-(6-fluoropyridin-2 -yl)-4-(trifluoromethyl)pyrazolidin-1-yl) methanone |
| 354 | 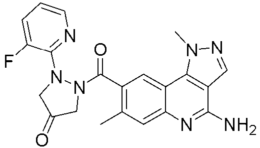 | 1-(4-amino-1,7-dimethyl-1H-pyrazolo[4, 3-c]quinoline-8-formyl)-2-(3-fluoropyridi n-2-yl)pyrazolidin-4-one |
| 355 | 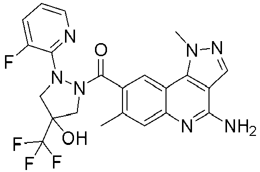 | (4-amino-1,7-dimethyl-1H-pyrazolo[4,3-c]quinolin-8-yl)(2-(3-fluoropyridin-2-yl)-4-hydroxy-4-(trifluoromethyl)pyrazolidin -1-yl)methanone |
| 356 | 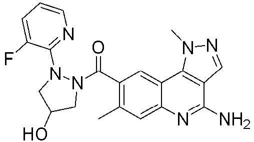 | (4-amino-1,7-dimethyl-1H-pyrazolo[4,3-c]quinolin-8-yl)(2-(3-fluoropyridin-2-yl)-4-hydroxypyrazolidin-1-yl)methanone |
| 357 | 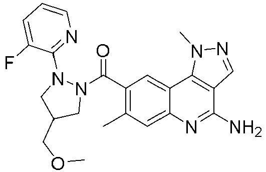 | (4-amino-1,7-dimethyl-1H-pyrazolo[4,3-c]quinolin-8-yl)(2-(3-fluoropyridin-2-yl)-4-(methoxymethyl)pyrazolidin-1-yl)meth anone |
| 358 | 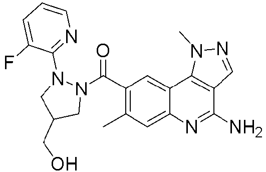 | (4-amino-1,7-dimethyl-1H-pyrazolo[4,3-c]quinolin-8-yl)(2-(3-fluoropyridin-2-yl)-4-(hydroxymethyl)pyrazolidin-1-yl)meth anone |
| 359 | 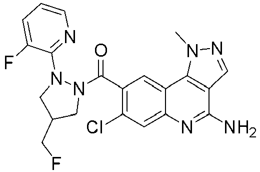 | (4-amino-7-chloro-1-methyl-1H-pyrazolo [4,3-c]quinolin-8-yl)(4-(fluoromethyl)-2-(3 -fluoropyridin-2-yl)pyrazolidin-1-yl)m ethanone |
| 360 | 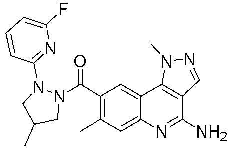 | (4-amino-1,7-dimethyl-1H-pyrazolo[4,3-c]quinolin-8-yl)(2-(6-fluoropyridin-2-yl)-4-methylpyrazolidin-1-yl)methanone |
| 361 | 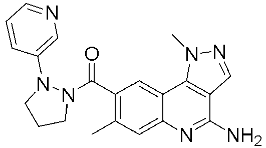 | (4-amino-1,7-dimethyl-1H-pyrazolo[4,3-c]quinolin-8-yl)(2-(pyridin-3-yl)pyrazoli din-1-yl)methanone |
| 362 | 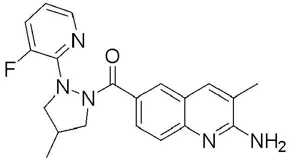 | (2-amino-3-methylquinolin-6-yl)(2-(3-flu oropyridin-2-yl)-4-methylpyrazolidin-1-y 1)methanone |
| 363 | 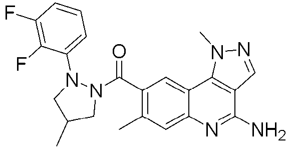 | (4-amino-1,7-dimethyl-1H-pyrazolo[4,3-c]quinolin-8-yl)(2-(2,3-difluorophenyl)-4 -methylpyrazolidin-1-yl)methanone |
| 364 | 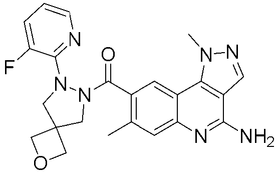 | (4-amino-1,7-dimethyl-1H-pyrazolo[4,3-c]quinolin-8-yl)(7-(3-fluoropyridin-2-yl)-2-oxa-6,7-diazaspiro[3.4]oct-6-yl)methan one |
| 365 | 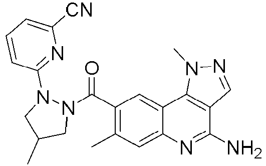 | 6-(2-(4-amino-1,7-dimethyl-1H-pyrazolo [4,3-c]quinoline-8-formyl)-4-methylpyra zolidin-1-yl)pyridinecarbonitrile |
| 366 | 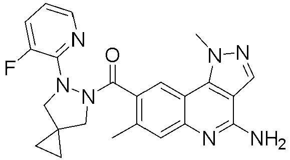 | (4-amino-1,7-dimethyl-1H-pyrazolo[4,3-c]quinolin-8-yl)(6-(3-fluoropyridin-2-yl)-5,6-diazaspiro[2.4]heptan-5-yl)methanone |
| 367 | 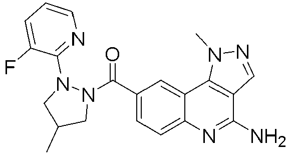 | (4-amino-1-methyl-1H-pyrazolo[4,3-c]qu inolin-8-yl)(2-(3-fluoropyridin-2-yl)-4-m ethylpyrazolidin-1-yl)methanone |
| 368 | 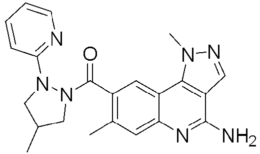 | (4-amino-1,7-dimethyl-1H-pyrazolo[4,3-c]quinolin-8-yl)(4-methyl-2-(pyridin-2-yl )pyrazolidin-1-yl)methanone |
| 369 | 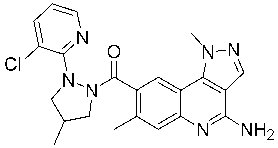 | (4-amino-1,7-dimethyl-1H-pyrazolo[4,3-c]quinolin-8-yl)(2-(3-chloropyridin-2-yl)-4-methylpyrazolidin-1-yl)methanone |
| 370 | 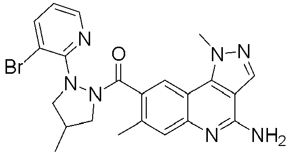 | (4-amino-1,7-dimethyl-1H-pyrazolo[4,3-c]quinolin-8-yl)(2-(3-bromopyridin-2-yl) -4-methylpyrazolidin-1-yl)methanone |
| 371 | 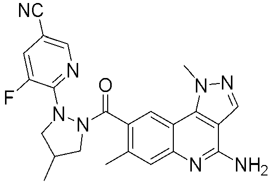 | 6-(2-(4-amino-1,7-dimethyl-1H-pyrazolo [4,3-c]quinoline-8-formyl)-4-methylpyra zolidin-1-yl)-5-fluoronicotinonitrile |
| 372 | 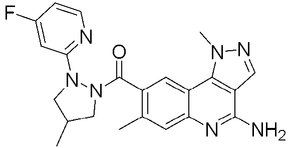 | (4-amino-1,7-dimethyl-1H-pyrazolo[4,3-c]quinolin-8-yl)(2-(4-fluoropyridin-2-yl)-4-methylpyrazolidin-1-yl)methanone |
| 373 | 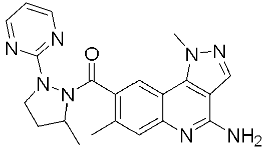 | (4-amino-1,7-dimethyl-1H-pyrazolo[4,3-c]quinolin-8-yl)(5-methyl-2-(pyrimidin-2 -yl)pyrazolidin-1-yl)methanone |
| 374 | 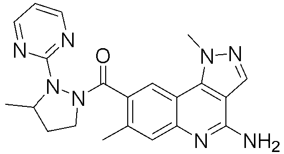 | (4-amino-1,7-dimethyl-1H-pyrazolo[4,3-c]quinolin-8-yl)(3-methyl-2-(pyrimidin-2 -yl)pyrazolidin-1-yl)methanone |
| 375 | 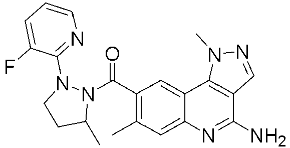 | (4-amino-1,7-dimethyl-1H-pyrazolo[4,3-c]quinolin-8-yl)(2-(3-fluoropyridin-2-yl)-5-methylpyrazolidin-1-yl)methanone |
| 376 | 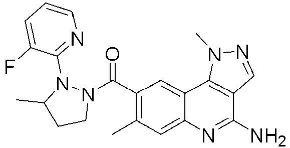 | (4-amino-1,7-dimethyl-1H-pyrazolo[4,3-c]quinolin-8-yl)(2-(3-fluoropyridin-2-yl)-5-methylpyrazolidin-1-yl)methanone |
| 377 | 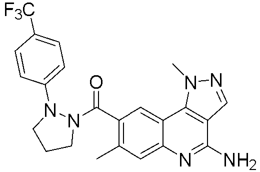 | (4-amino-1,7-dimethyl-1H-pyrazolo[4,3-c]quinolin-8-yl)(2-(4-(trifluoromethyl)ph enyl)pyrazolidin-1-yl)methanone |
| 378 | 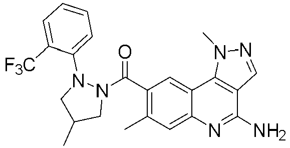 | (4-amino-1,7-dimethyl-1H-pyrazolo[4,3-c]quinolin-8-yl)(4-methyl-2-(2-(trifluoro methyl)phenyl)pyrazolidin-1-yl)methanone |
| 379 | 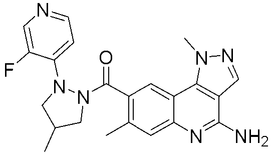 | (4-amino-1,7-dimethyl-1H-pyrazolo[4,3-c]quinolin-8-yl)(2-(3-fluoropyridin-4-yl)-4-methylpyrazolidin-1-yl)methanone |
| 380 | 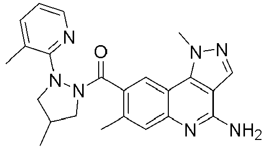 | (4-amino-1,7-dimethyl-1H-pyrazolo[4,3-c]quinolin-8-yl)(4-methyl-2-(3-methylpyr idin-2-yl)pyrazolidin-1-yl)methanone |
| 381 | 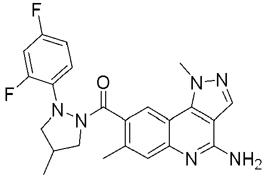 | (4-amino-1,7-dimethyl-1H-pyrazolo[4,3-c]quinolin-8-yl)(2-(2,4-difluorophenyl)-4 -methylpyrazolidin-1-yl)methanone |
| 382 | 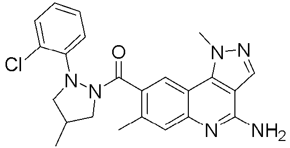 | (4-amino-1,7-dimethyl-1H-pyrazolo[4,3-c]quinolin-8-yl)(2-(2-chlorophenyl)-4-me thylpyrazolidin-1-yl)methanone |
| 383 | 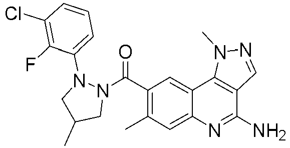 | (4-amino-1,7-dimethyl-1H-pyrazolo[4,3-c]quinolin-8-yl)(2-(3-chloro-2-fluorophe nyl)-4-methylpyrazolidin-1-yl)methanone |
| 384 | 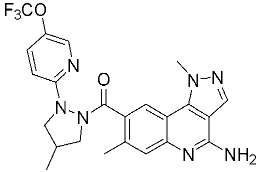 | (4-amino-1,7-dimethyl-1H-pyrazolo[4,3-c]quinolin-8-yl)(4-methyl-2-(5-(trifluoro methoxy)pyridin-2-yl)pyrazolidin-1-yl)m ethanone |
| 385 | 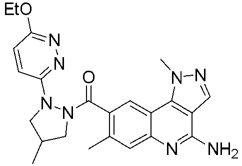 | (4-amino-1,7-dimethyl-1H-pyrazolo[4,3-c]quinolin-8-yl)(2-(6-ethoxypyridazin-3-yl)-4-methylpyrazolidin-1-yl)methanone |
| 386 | 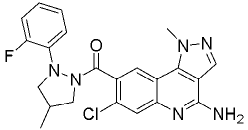 | (4-amino-7-chloro-1-methyl-1H-pyrazolo [4,3-c]quinolin-8-yl)(2-(2-fluorophenyl)-4-methylpyrazolidin-1-yl)methanone |
| 387 | 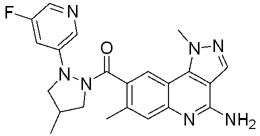 | (4-amino-1,7-dimethyl-1H-pyrazolo[4,3-c]quinolin-8-yl)(2-(5-fluoropyridin-3-yl)-4-methylpyrazolidin-1-yl)methanone |
| 388 | 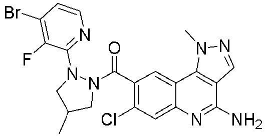 | (4-amino-7-chloro-1-methyl-1H-pyrazolo [4,3-c]quinolin-8-yl)(2-(4-bromo-3-fluor opyridin-2-yl)-4-methylpyrazolidin-1-yl) methanone |
| 389 | 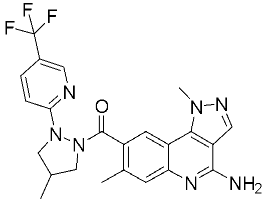 | (4-amino-1,7-dimethyl-1H-pyrazolo[4,3-c]quinolin-8-yl)(4-methyl-2-(5-(trifluoro methyl)pyridin-2-yl)pyrazolidin-1-yl)met hanone |
| 390 | 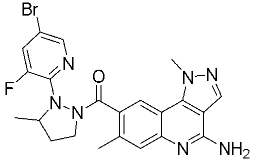 | (4-amino-1,7-dimethyl-1H-pyrazolo[4,3-c]quinolin-8-yl)(2-(5-bromo-3-fluoropyri din-2-yl)-3-methylpyrazolidin-1-yl)metha none |
| 391 | 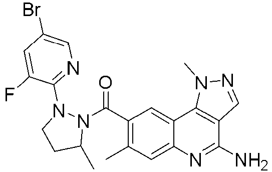 | (4-amino-1,7-dimethyl-1H-pyrazolo[4,3-c]quinolin-8-yl)(2-(5-bromo-3-fluoropyri din-2-yl)-5-methylpyrazolidin-1-yl)metha none |
| 392 | 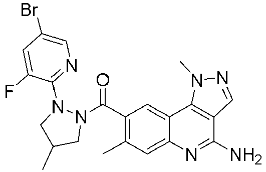 | (4-amino-1,7-dimethyl-1H-pyrazolo[4,3-c]quinolin-8-yl)(2-(5-bromo-3-fluoropyri din-2-yl)-4-methylpyrazolidin-1-yl)metha none |
| 393 | 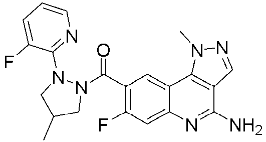 | (4-amino-7-fluoro-1-methyl-1H-pyrazolo [4,3-c]quinolin-8-yl)(2-(3-fluoropyridin-2 -yl)-4-methylpyrazolidin-1-yl)methanone |
| 394 | 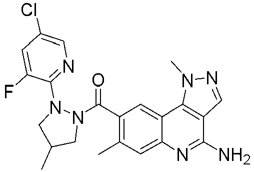 | (4-amino-1,7-dimethyl-1H-pyrazolo[4,3-c]quinolin-8-yl)(2-(5-chloro-3-fluoropyri din-2-yl)-4-methylpyrazolidin-1-yl)metha none |
| 395 | 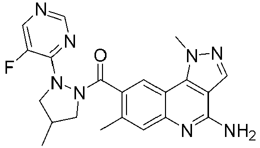 | (4-amino-1,7-dimethyl-1H-pyrazolo[4,3-c]quinolin-8-yl)(2-(5-fluoropyrimidin-4-y l)-4-methylpyrazolidin-1-yl)methanone |
| 396 | 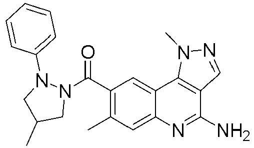 | (4-amino-1,7-dimethyl-1H-pyrazolo[4,3-c]quinolin-8-yl)(4-methyl-2-phenylpyraz olidin-1-yl)methanone |
| 397 | 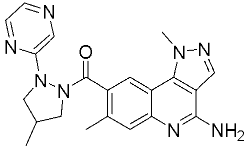 | (4-amino-1,7-dimethyl-1H-pyrazolo[4,3-c]quinolin-8-yl)(4-methyl-2-(pyrazin-2-yl )pyrazolidin-1-yl)methanone |
| 398 | 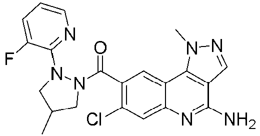 | (4-amino-7-chloro-1-methyl-1H-pyrazolo [4,3-c]quinolin-8-yl)(2-(3-fluoropyridin-2 -yl)-4-methylpyrazolidin-1-yl)methanone |
| 399 | 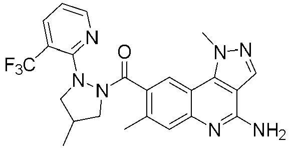 | (4-amino-1,7-dimethyl-1H-pyrazolo[4,3-c]quinolin-8-yl)(4-methyl-2-(3-(trifluoro methyl)pyridin-2-yl)pyrazolidin-1-yl)met hanone |
| 400 | 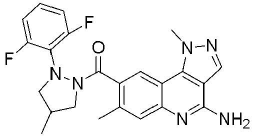 | (4-amino-1,7-dimethyl-1H-pyrazolo[4,3-c]quinolin-8-yl)(2-(2,6-difluorophenyl)-4 -methylpyrazolidin-1-yl)methanone |
| 401 | 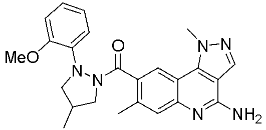 | (4-amino-1,7-dimethyl-1H-pyrazolo[4,3-c]quinolin-8-yl)(2-(2-methoxyphenyl)-4-methylpyrazolidin-1-yl)methanone |
| 402 | 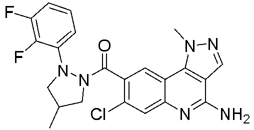 | (4-amino-7-chloro-1-methyl-1H-pyrazolo [4,3-c]quinolin-8-yl)(2-(2,3-difluorophen yl)-4-methylpyrazolidin-1-yl)methanone |
| 403 | 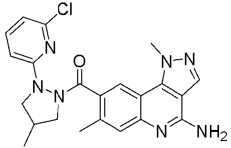 | (4-amino-1,7-dimethyl-1H-pyrazolo[4,3-c]quinolin-8-yl)(2-(6-chloropyridin-2-yl)-4-methylpyrazolidin-1-yl)methanone |
| 404 | 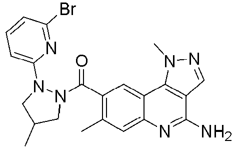 | (4-amino-1,7-dimethyl-1H-pyrazolo[4,3-c]quinolin-8-yl)(2-(6-bromopyridin-2-yl) -4-methylpyrazolidin-1-yl)methanone |
| 405 | 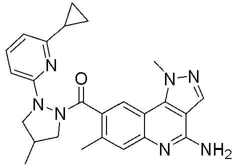 | (4-amino-1,7-dimethyl-1H-pyrazolo[4,3-c]quinolin-8-yl)(2-(6-cyclopropylpyridin-2-yl)-4-methylpyrazolidin-1-yl)methanone |
| 406 | 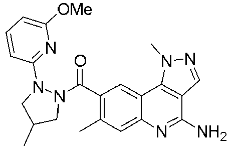 | (4-amino-1,7-dimethyl-1H-pyrazolo[4,3-c]quinolin-8-yl)(2-(6-methoxypyridin-2-y l)-4-methylpyrazolidin-1-yl)methanone |
| 407 | 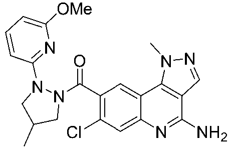 | (4-amino-7-chloro-1-methyl-1H-pyrazolo [4,3-c]quinolin-8-yl)(2-(6-methoxypyridi n-2-yl)-4-methylpyrazolidin-1-yl)methan one |
| 408 | 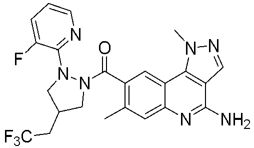 | (4-amino-1,7-dimethyl-1H-pyrazolo[4,3-c]quinolin-8-yl)(2-(3-fluoropyridin-2-yl)-4-(2,2,2-trifluoroethyl)pyrazolidin-1-yl)m ethanone |
| 409 | 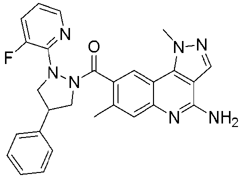 | (4-amino-1,7-dimethyl-1H-pyrazolo[4,3-c]quinolin-8-yl)(2-(3-fluoropyridin-2-yl)-4-phenylpyrazolidin-1-yl)methanone |
| 410 | 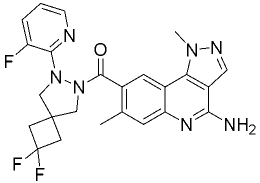 | (4-amino-1,7-dimethyl-1H-pyrazolo[4,3-c]quinolin-8-yl)(2,2-difluoro-7-(3-fluorop yridin-2-yl)-6,7-diazaspiro[3.4]octan-6-yl )methanone |
| 411 | 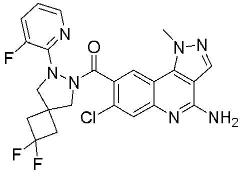 | (4-amino-7-chloro-1-methyl-1H-pyrazolo [4,3-c]quinolin-8-yl)(2,2-difluoro-7-(3-fl uoropyridin-2-yl)-6,7-diazaspiro[3.4]octa n-6-yl)methanone |
| 412 | 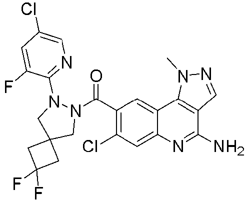 | (4-amino-7-chloro-1-methyl-1H-pyrazolo [4,3-c]quinolin-8-yl)(7-(5-chloro-3-fluoro pyridin-2-yl)-2,2-difluoro-6,7-diazaspiro[ 3.4]octan-6-yl)methanone |
| 413 | 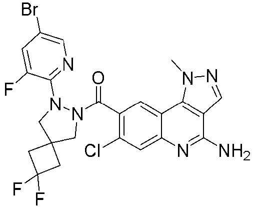 | (4-amino-7-chloro-1-methyl-1H-pyrazolo [4,3-c]quinolin-8-yl)(7-(5-bromo-3-fluor opyridin-2-yl)-2,2-difluoro-6,7-diazaspir o[3.4]octan-6-yl)methanone |
| 414 | 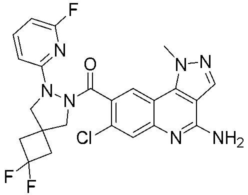 | (4-amino-7-chloro-1-methyl-1H-pyrazolo [4,3-c]quinolin-8-yl)(2,2-difluoro-7-(6-fl uoropyridin-2-yl)-6,7-diazaspiro[3.4]octa n-6-yl)methanone |
| 415 | 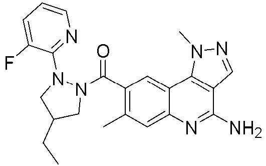 | (4-amino-1,7-dimethyl-1H-pyrazolo[4,3-c]quinolin-8-yl)(4-ethyl-2-(3-fluoropyridi n-2-yl)pyrazolidin-1-yl)methanone |
| 416 | 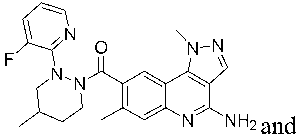 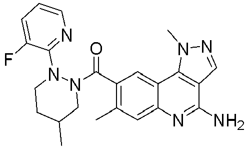 | (4-amino-1,7-dimethyl-1H-pyrazolo[4,3-c]quinolin-8-yl)(2-(3-fluoropyridin-2-yl)-4-methyltetrahydropyridazin-1 (2H)-yl)m ethanone and (4-amino-1,7-dimethyl-1H-pyrazolo[4,3-c]quinolin-8-yl)(2-(3-fluoropyridin-2-yl)-5-methyltetrahydropyridazin-1 (2H)-yl)m ethanone |
| 417 | 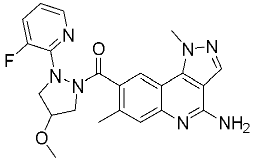 | (4-amino-1,7-dimethyl-1H-pyrazolo[4,3-c]quinolin-8-yl)(2-(3-fluoropyridin-2-yl)-4-methoxypyrazolidin-1-yl)methanone |
| 418 | 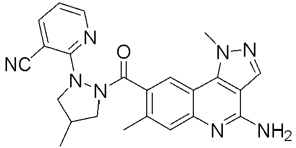 | 2-(2-(4-amino-1,7-dimethyl-1H-pyrazolo [4,3-c]quinoline-8-formyl)-4-methylpyra zolidin-1-yl)nicotinonitrile |
| 419 | 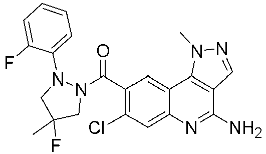 | (4-amino-7-chloro-1-methyl-1H-pyrazolo [4,3-c]quinolin-8-yl)(4-fluoro-2-(2-fluoro phenyl)-4-methylpyrazolidin-1-yl)methan one |
| 420 | 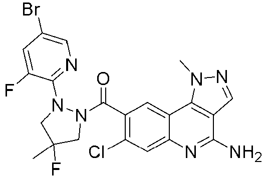 | (4-amino-7-chloro-1-methyl-1H-pyrazolo [4,3-c]quinolin-8-yl)(2-(5-bromo-3-fluor opyridin-2-yl)-4-fluoro-4-methylpyrazoli din-1-yl)methanone |
| 421 | 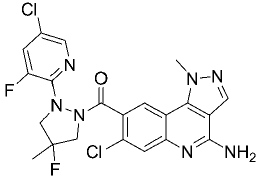 | (4-amino-7-chloro-1-methyl-1H-pyrazolo [4,3-c]quinolin-8-yl)(2-(5-chloro-3-fluoro pyridin-2-yl)-4-fluoro-4-methylpyrazolidi n-1-yl)methanone |
| 422 | 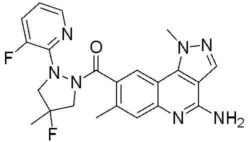 | (4-amino-1,7-dimethyl-1H-pyrazolo[4,3-c]quinolin-8-yl)(4-fluoro-2-(3-fluoropyri din-2-yl)-4-methylpyrazolidin-1-yl)metha none |
| 423 | 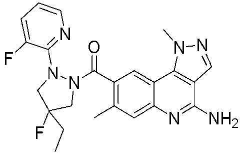 | (4-amino-1,7-dimethyl-1H-pyrazolo[4,3-c]**quinolin**-8-yl)(4-ethyl-4-fluoro-2-(3-fl uoropyridin-2-yl)pyrazolidin-1-yl)methan one |
| 424 | 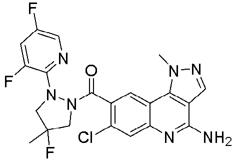 | (4-amino-7-chloro-1-methyl-1H-pyrazolo [4,3-c]quinolin-8-yl)(2-(3,5-2 fluoropyridin-2-yl)-4-fluoro-4-methylpyr azolidin-1-yl)methanone |
| 425 | 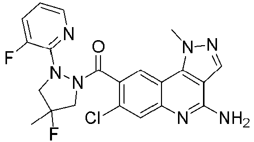 | (4-amino-7-chloro-1-methyl-1H-pyrazolo [4,3-c]quinolin-8-yl)(4-fluoro-2-(3-fluoro pyridin-2-yl)-4-methylpyrazolidin-1-yl)m ethanone |
| 426 | 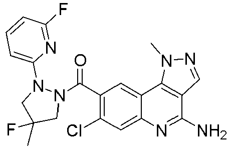 | (4-amino-7-chloro-1-methyl-1H-pyrazolo [4,3-c]quinolin-8-yl)(4-fluoro-2-(6-fluoro pyridin-2-yl)-4-methylpyrazolidin-1-yl)m ethanone |
| 427 | 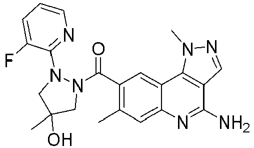 | (4-amino-1,7-dimethyl-1H-pyrazolo[4,3-c]quinolin-8-yl)(2-(3-fluoropyridin-2-yl)-4-hydroxy-4-methylpyrazolidin-1-yl)met hanone |
| 428 | 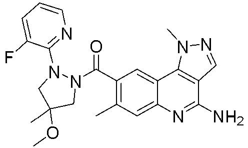 | (4-amino-1,7-dimethyl-1H-pyrazolo[4,3-c]quinolin-8-yl)(2-(3-fluoropyridin-2-yl)-4-methoxy-4-methylpyrazolidin-1-yl)met hanone |
| 429 | 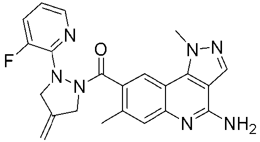 | (4-amino-1,7-dimethyl-1H-pyrazolo[4,3-c]quinolin-8-yl)(2-(3-fluoropyridin-2-yl)-4-methylenepyrazolidin-1-yl)methanone |
| 430 | 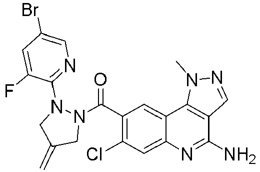 | (4-amino-7-chloro-1-methyl-1H-pyrazolo [4,3-c]quinolin-8-yl)(2-(5-bromo-3-fluor opyridin-2-yl)-4-methylenepyrazolidin-1-yl)methanone |
| 431 | 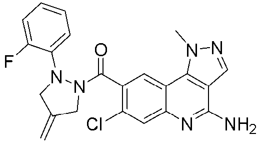 | (4-amino-7-chloro-1-methyl-1H-pyrazolo [4,3-c]quinolin-8-yl)(2-(2-fluorophenyl)-4-methylenepyrazolidin-1-yl)methanone |
| 432 | 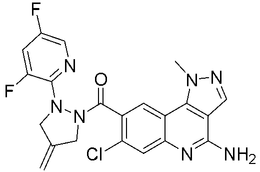 | (4-amino-7-chloro-1-methyl-1H-pyrazolo [4,3-c]quinolin-8-yl)(2-(3,5-2 fluoropyridin-2-yl)-4-methylenepyrazolid in-1-yl)methanone |
| 433 | 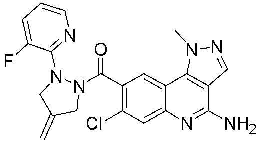 | (4-amino-7-chloro-1-methyl-1H-pyrazolo [4,3-c]quinolin-8-yl)(2-(3-fluoropyridin-2 -yl)-4-methylenepyrazolidin-1-yl)methan one |
| 434 | 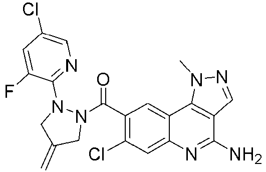 | (4-amino-7-chloro-1-methyl-1H-pyrazolo [4,3-c]quinolin-8-yl)(2-(5-chloro-3-fluoro pyridin-2-yl)-4-methylenepyrazolidin-1-y 1)methanone |
| 435 | 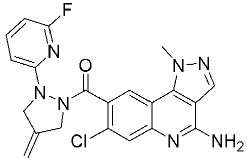 | (4-amino-7-chloro-1-methyl-1H-pyrazolo [4,3-c]quinolin-8-yl)(2-(6-fluoropyridin-2 -yl)-4-methylenepyrazolidin-1-yl)methan one |
| 436 | 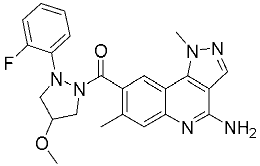 | (4-amino-1,7-dimethyl-1H-pyrazolo[4,3-c]quinolin-8-yl)(2-(2-fluorophenyl)-4-me thoxypyrazolidin-1-yl)methanone |
| 437 | 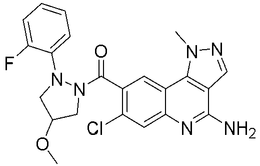 | (4-amino-7-chloro-1-methyl-1H-pyrazolo [4,3-c]quinolin-8-yl)(2-(2-fluorophenyl)-4-methoxypyrazolidin-1-yl)methanone |
| 438 | 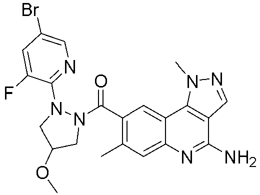 | (4-amino-1,7-dimethyl-1H-pyrazolo[4,3-c]quinolin-8-yl)(2-(5-bromo-3-fluoropyri din-2-yl)-4-methoxypyrazolidin-1-yl)met hanone |
| 439 | 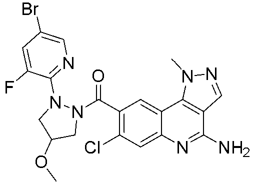 | (4-amino-7-chloro-1-methyl-1H-pyrazolo [4,3-c]quinolin-8-yl)(2-(5-bromo-3-fluor opyridin-2-yl)-4-methoxypyrazolidin-1-y l)methanone |
| 440 | 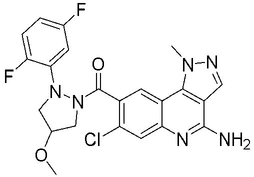 | (4-amino-7-chloro-1-methyl-1H-pyrazolo [4,3-c]quinolin-8-yl)(2-(2,5-difluorophen yl)-4-methoxypyrazolidin-1-yl)methanon e |
| 441 | 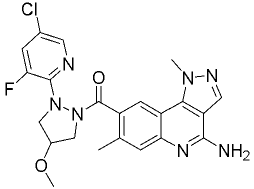 | (4-amino-1,7-dimethyl-1H-pyrazolo[4,3- c]quinolin-8-y1)(2-(5-chloro-3-fluoropyri din-2-yl)-4-methoxypyrazolidin-1-yl)met hanone |
| 442 | 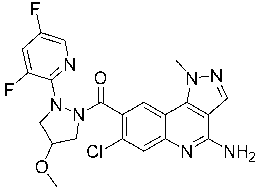 | (4-amino-7-chloro-1-methyl-1H-pyrazolo [4,3-c]quinolin-8-yl)(2-(3,5-2 fluoropyridin-2-yl)-4-methoxypyrazolidi n-1-yl)methanone |
| 443 | 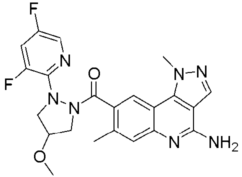 | (4-amino-1,7-dimethyl-1H-pyrazolo[4,3- c]quinolin-8-yl)(2-(3,5- 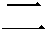 fluoropyridin-2-yl)-4-methoxypyrazolidi n-1-yl)methanone |
| 444 | 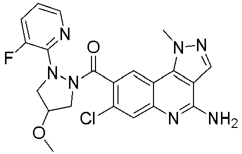 | (4-amino-7-chloro-1-methyl-1H-pyrazolo [4,3-c]quinolin-8-yl)(2-(3-fluoropyridin-2 -yl)-4-methoxypyrazolidin-1-yl)methano ne |
| 445 | 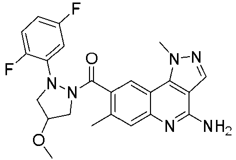 | (4-amino-1,7-dimethyl-1H-pyrazolo[4,3- c]quinolin-8-yl)(2-(2,5-difluorophenyl)-4 -methoxypyrazolidin-1-yl)methanone |
| 446 | 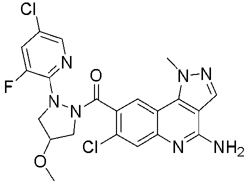 | (4-amino-7-chloro-1-methyl-1H-pyrazolo [4,3-c]quinolin-8-yl)(2-(5-chloro-3-fluoro pyridin-2-yl)-4-methoxypyrazolidin-1-yl) methanone |
| 447 | 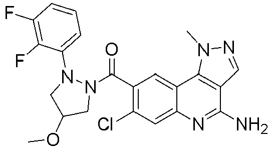 | (4-amino-7-chloro-1-methyl-1H-pyrazolo [4,3-c]quinolin-8-yl)(2-(2,3-difluorophen yl)-4-methoxypyrazolidin-1-yl)methanone |
| 448 | 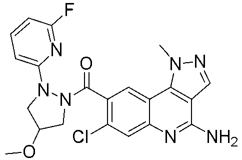 | (4-amino-7-chloro-1-methyl-1H-pyrazolo [4,3-c]quinolin-8-yl)(2-(6-fluoropyridin-2 -yl)-4-methoxypyrazolidin-1-yl)methano ne |
| 449 | 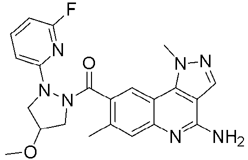 | (4-amino-1,7-dimethyl-1H-pyrazolo[4,3-c]quinolin-8-yl)(2-(6-fluoropyridin-2-yl) -4-methoxypyrazolidin-1-yl)methanone |
| 450 | 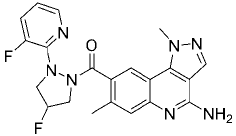 | (4-amino-1,7-dimethyl-1H-pyrazolo[4,3-c]quinolin-8-yl)(4-fluoro-2-(3-fluoropyri din-2-yl)pyrazolidin-1-yl)methanone |
| 451 | 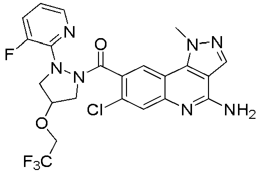 | (4-amino-7-chloro-1-methyl-1H-pyrazolo [4,3-c]quinolin-8-yl)(2-(3-fluoropyridin-2 -yl)-4-(2,2,2-trifluoroethoxy)pyrazolidin-1-yl)methanone |
| 452 | 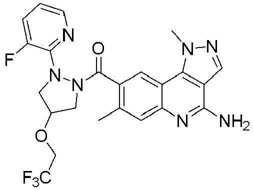 | (4-amino-1,7-dimethyl-1H-pyrazolo[4,3-c]quinolin-8-yl)(2-(3-fluoropyridin-2-yl)-4-(2,2,2-trifluoroethoxy)pyrazolidin-1-yl) methanone |
| 453 | 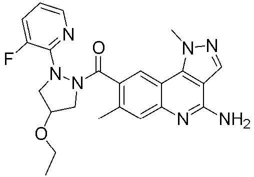 | (4-amino-1,7-dimethyl-1H-pyrazolo[4,3-c]quinolin-8-yl)(4-ethoxy-2-(3-fluoropyri din-2-yl)pyrazolidin-1-yl)methanone |
| 454 | 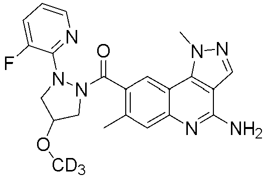 | (4-amino-1,7-dimethyl-1H-pyrazolo[4,3-c]quinolin-8-yl)(2-(3-fluoropyridin-2-yl)-4-(methoxy-D3)pyrazolidin-1-yl)methan one |
| 455 | 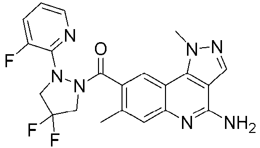 | (4-amino-1,7-dimethyl-1H-pyrazolo[4,3-c]quinolin-8-yl)(4,4-difluoro-2-(3-fluorop yridin-2-yl)pyrazolidin-1-yl)methanone |
| 456 | 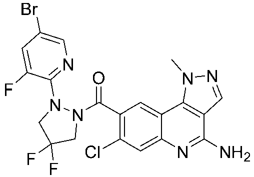 | (4-amino-7-chloro-1-methyl-1H-pyrazolo [4,3-c]quinolin-8-yl)(2-(5-bromo-3-fluor opyridin-2-yl)-4,4-difluoropyrazolidin-1-yl)methanone |
| 457 | 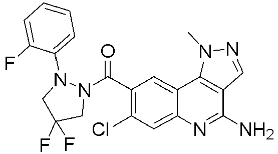 | (4-amino-7-chloro-1-methyl-1H-pyrazolo [4,3-c]quinolin-8-yl)(4,4-difluoro-2-(2-fl uorophenyl)pyrazolidin-1-yl)methanone |
| 458 | 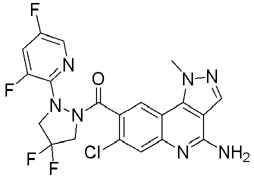 | (4-amino-7-chloro-1-methyl-1H-pyrazolo [4,3-c]quinolin-8-yl)(2-(3,5-difluoropyrid in-2-yl)-4,4-difluoropyrazolidin-1-yl)met hanone |
| 459 | 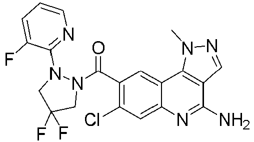 | (4-amino-7-chloro-1-methyl-1H-pyrazolo [4,3-c]quinolin-8-yl)(4,4-difluoro-2-(3-fl uoropyridin-2-yl)pyrazolidin-1-yl)methan one |
| 460 | 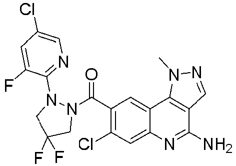 | (4-amino-7-chloro-1-methyl-1H-pyrazolo [4,3-c]quinolin-8-yl)(2-(5-chloro-3-fluoro pyridin-2-yl)-4,4-difluoropyrazolidin-1-yl )methanone |
| 461 | 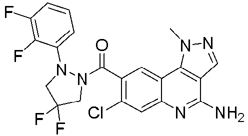 | (4-amino-7-chloro-1-methyl-1H-pyrazolo [4,3-c]quinolin-8-yl)(2-(2,3-difluorophen yl)-4,4-difluoropyrazolidin-1-yl)methano ne |
| 462 | 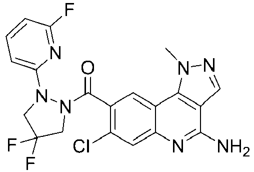 | (4-amino-7-chloro-1-methyl-1H-pyrazolo [4,3-c]quinolin-8-yl)(4,4-difluoro-2-(6-fl uoropyridin-2-yl)pyrazolidin-1-yl)methan one |
| 463 | 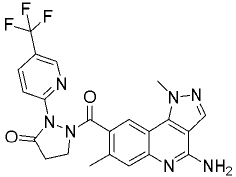 | 1-(4-amino-1,7-dimethyl-1H-pyrazolo[4, 3-c]quinoline-8-formyl)-2-(5-(trifluorom ethyl)pyridin-2-yl)pyrazolidin-3-one |
| 464 | 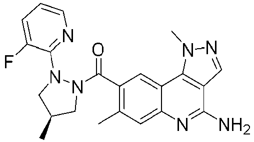 | rel-(R)-(4-amino-1,7-dimethyl-1H-pyrazo lo[4,3-c]quinolin-8-yl)(2-(3-fluoropyridin -2-yl)-4-methylpyrazolidin-1-yl)methanone |
| 465 | 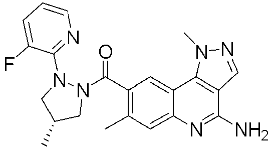 | rel-(S)-(4-amino-1,7-dimethyl-1H-pyrazo lo[4,3-c]quinolin-8-yl)(2-(3-fluoropyridin -2-yl)-4-methylpyrazolidin-1-yl)methano ne |
| 466 | 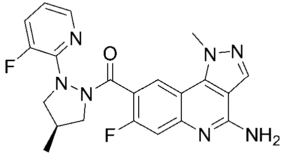 | rel-(S)-(4-amino-7-fluoro-1-methyl-1H-p yrazolo[4,3-c]quinolin-8-yl)(2-(3-fluorop yridin-2-yl)-4-methylpyrazolidin-1-yl)me thanone |
| 467 | 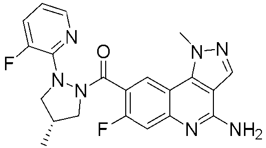 | rel-(R)-(4-amino-7-fluoro-1-methyl-1H-p yrazolo[4,3-c]quinolin-8-yl)(2-(3-fluorop yridin-2-yl)-4-methylpyrazolidin-1-yl)me thanone |
| 468 | 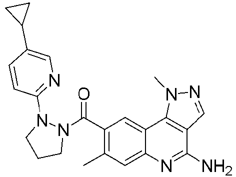 | (4-amino-1,7-dimethyl-1H-pyrazolo[4,3-c]quinolin-8-yl)(2-(5-cyclopropylpyridin-2-yl)pyrazolidin-1-yl)methanone |
| 469 | 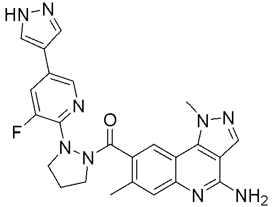 | (4-amino-1,7-dimethyl-1H-pyrazolo[4,3-c]**quinolin**-8-y1)(2-(3-fluoro-5-(1H-pyraz ol-4-yl)pyridin-2-yl)pyrazolidin-1-yl)met hanone |
| 470 | 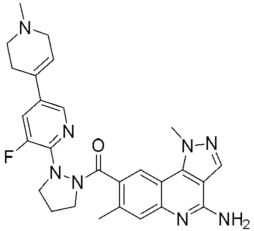 | (4-amino-1,7-dimethyl-1H-pyrazolo[4,3-c]quinolin-8-yl)(2-(5-fluoro-1'-methyl-1', 2',3',6'-tetrahydro-[3,4'-bipyridine]-6-yl)p yrazolidin-1-yl)methanone |
| 471 | 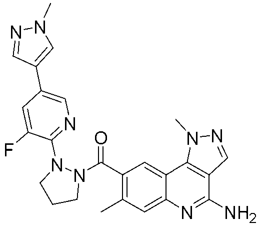 | (4-amino-1,7-dimethyl-1H-pyrazolo[4,3-c]quinolin-8-yl)(2-(3-fluoro-5-(1-methyl-1H-pyrazol-4-yl)pyridin-2-yl)pyrazolidin -1-yl)methanone |
| 472 | 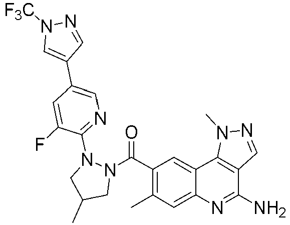 | (4-amino-1,7-dimethyl-1H-pyrazolo[4,3-c]quinolin-8-y1)(2-(3-fluoro-5-(1-(trifluor omethyl)- 1H-pyrazol-4-yl)pyridin-2-yl)-4 -methylpyrazolidin-1-yl)methanone |
| 473 | 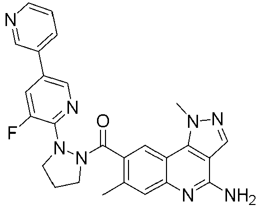 | (4-amino-1,7-dimethyl-1H-pyrazolo[4,3-c]**quinolin**-8-yl)(2-(5-fluoro-[3,3'-bipyrid ine]-6-yl)pyrazolidin-1-yl)methanone |
| 474 | 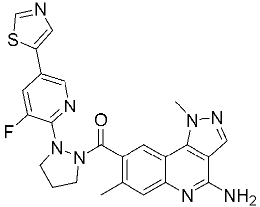 | (4-amino-1,7-dimethyl-1H-pyrazolo[4,3-c]quinolin-8-yl)(2-(3-fluoro-5-(thiazol-5-yl)pyridin-2-yl)pyrazolidin-1-yl)methano ne |
| 475 | 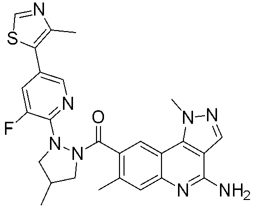 | (4-amino-1,7-dimethyl-1H-pyrazolo[4,3-c]quinolin-8-yl)(2-(3-fluoro-5-(4-methylt hiazol-5-yl)pyridin-2-yl)-4-methylpyrazo lidin-1-yl)methanone |
| 476 | 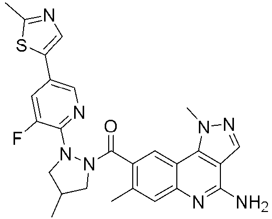 | (4-amino-1,7-dimethyl-1H-pyrazolo[4,3-c]quinolin-8-yl)(2-(3-fluoro-5-(2-methylt hiazol-5-yl)pyridin-2-yl)-4-methylpyrazo lidin-1-yl)methanone |
| 477 | 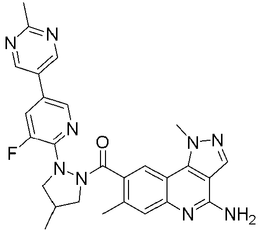 | (4-amino-1,7-dimethyl-1H-pyrazolo[4,3-c]quinolin-8-yl)(2-(3-fluoro-5-(2-methylp yrimidin-5-yl)pyridin-2-yl)-4-methylpyra zolidin-1-yl)methanone |
| 478 | 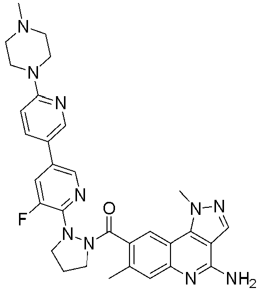 | (4-amino-1,7-dimethyl-1H-pyrazolo[4,3-c]quinolin-8-yl)(2-(5-fluoro-6-(4-methylp iperazin-1-yl)-[3,3'-bipyridine]-6-yl)pyra zolidin-1-yl)methanone |
| 479 | 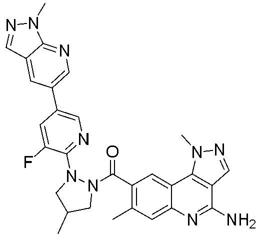 | (4-amino-1,7-dimethyl-1H-pyrazolo[4,3-c]quinolin-8-yl)(2-(3-fluoro-5-(1-methyl-1H-pyrazolo[3,4-b]pyridin-5-yl)pyridin-2 -yl)-4-methylpyrazolidin-1-yl)methanone |
| 480 | 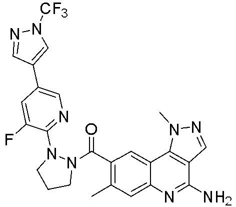 | (4-amino-1,7-dimethyl-1H-pyrazolo[4,3-c]quinolin-8-yl)(2-(3-fluoro-5-(1-(trifluor omethyl)-1H-pyrazol-4-yl)pyridin-2-yl)p yrazolidin-1-yl)methanone |
| 481 | 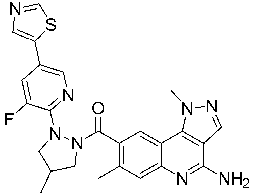 | (4-amino-1,7-dimethyl-1H-pyrazolo[4,3-c]quinolin-8-yl)(2-(3-fluoro-5-(thiazol-5-yl)pyridin-2-yl)-4-methylpyrazolidin-1-yl )methanone |
| 482 | 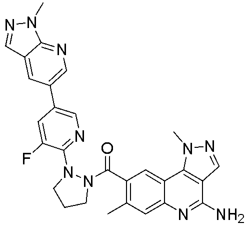 | (4-amino-1,7-dimethyl-1H-pyrazolo[4,3-c]quinolin-8-yl)(2-(3-fluoro-5-(1-methyl-1H-pyrazolo[3,4-b]pyridin-5-yl)pyridin-2 -yl)pyrazolidin-1-yl)methanone |
| 483 | 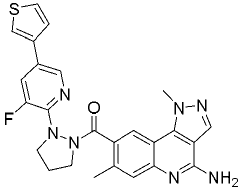 | (4-amino-1,7-dimethyl-1H-pyrazolo[4,3-c]quinolin-8-yl)(2-(3-fluoro-5-(thien-3-yl )pyridin-2-yl)pyrazolidin-1-yl)methanone |
| 484 | 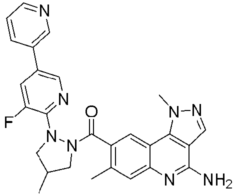 | (4-amino-1,7-dimethyl-1H-pyrazolo[4,3-c]quinolin-8-yl)(2-(5-fluoro-[3,3'-bipyridi ne]-6-yl)-4-methylpyrazolidin-1-yl)metha none |
| 485 | 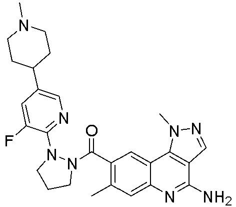 | (4-amino-1,7-dimethyl-1H-pyrazolo[4,3-c]quinolin-8-yl)(2-(3-fluoro-5-(1-methylp iperid-4-yl)pyridin-2-yl)pyrazolidin-1-yl) methanone |
| 486 | 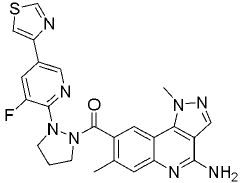 | (4-amino-1,7-dimethyl-1H-pyrazolo[4,3-c]quinolin-8-yl)(2-(3-fluoro-5-(thiazol-4-yl)pyridin-2-yl)pyrazolidin-1-yl)methano ne |
| 487 | 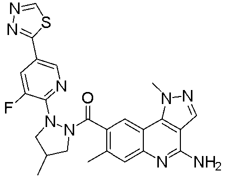 | (4-amino-1,7-dimethyl-1H-pyrazolo[4,3-c]quinolin-8-yl)(2-(3-fluoro-5-(1,3,4-thia diazol-2-yl)pyridin-2-yl)-4-methylpyrazo lidin-1-yl)methanone |
| 488 | 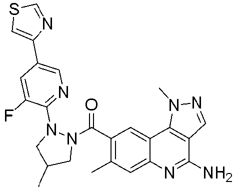 | (4-amino-1,7-dimethyl-1H-pyrazolo[4,3-c]quinolin-8-yl)(2-(3-fluoro-5-(thiazol-4-yl)pyridin-2-yl)-4-methylpyrazolidin-1-yl )methanone |
| 489 | 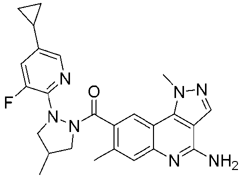 | (4-amino-1,7-dimethyl-1H-pyrazolo[4,3-c]quinolin-8-yl)(2-(5-cyclopropyl-3-fluor opyridin-2-yl)-4-methylpyrazolidin-1-yl) methanone |
| 490 | 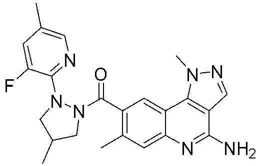 | (4-amino-1,7-dimethyl-1H-pyrazolo[4,3-c]quinolin-8-yl)(2-(3-fluoro-5-methylpyri din-2-yl)-4-methylpyrazolidin-1-yl)metha none |
| 491 | 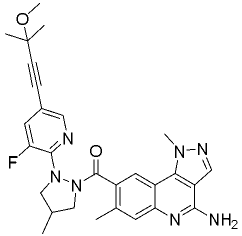 | (4-amino-1,7-dimethyl-1H-pyrazolo[4,3-c]quinolin-8-yl)(2-(3-fluoro-5-(3-methox y-3-methylbut-1-yn-1-yl)pyridin-2-yl)-4-methylpyrazolidin-1-yl)methanone |
| 492 | 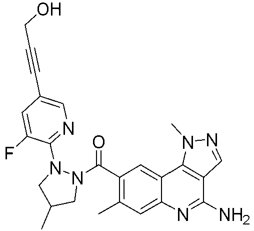 | (4-amino-1,7-dimethyl-1H-pyrazolo[4,3-c]quinolin-8-yl)(2-(3-fluoro-5-(3-hydrox yprop-1-yn-1-yl)pyridin-2-yl)-4-methylp yrazolidin-1-yl)methanone |
| 493 | 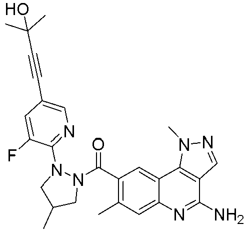 | (4-amino-1,7-dimethyl-1H-pyrazolo[4,3-c]quinolin-8-yl)(2-(3-fluoro-5-(3-hydrox y-3-methyl-but-1-yn-1-yl)pyridin-2-yl)-4 -methylpyrazolidin-1-yl)methanone |
| 494 | 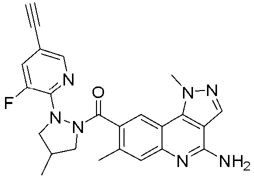 | (4-amino-1,7-dimethyl-1H-pyrazolo[4,3-c]quinolin-8-yl)(2-(5-ethynyl-3-fluoropyr idin-2-yl)-4-methylpyrazolidin-1-yl)meth anone |
| 495 | 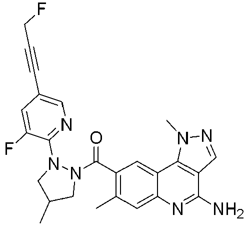 | (4-amino-1,7-dimethyl-1H-pyrazolo[4,3-c]quinolin-8-yl)(2-(3-fluoro-5-(3-fluoropr op-1-yn-1-yl)pyridin-2-yl)-4-methylpyraz olidin-1-yl)methanone |
| 496 | 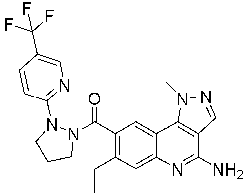 | (4-amino-7-ethyl-1-methyl-1H-pyrazolo[ 4,3-c]quinolin-8-yl)(2-(5-(trifluoromethyl )pyridin-2-yl)pyrazolidin-1-yl)methanone |
| 497 | 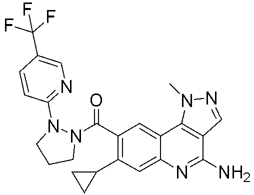 | (4-amino-7-cyclopropyl-1-methyl-1H-pyr azolo[4,3-c]quinolin-8-yl)(2-(5-(trifluoro methyl)pyridin-2-yl)pyrazolidin-1-yl)met hanone |
| 498 | 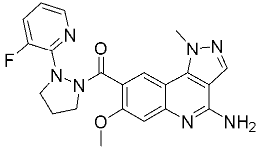 | (4-amino-7-methoxy-1-methyl-1H-pyraz olo[4,3-c]quinolin-8-yl)(2-(3-fluoropyridi n-2-yl)pyrazolidin-1-yl)methanone |
| 499 | 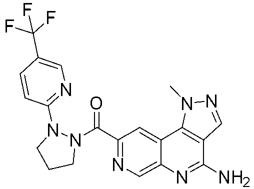 | (4-amino-1-methyl-1H-pyrazolo[4,3-c][1, 7]naphthyridin-8-yl)(2-(5-(trifluoromethy l)pyridin-2-yl)pyrazolidin-1-yl)methanone |
| 500 | 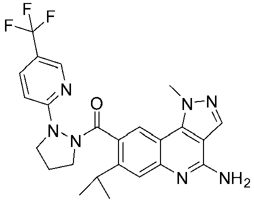 | (4-amino-7-isopropyl-1-methyl-1H-pyraz olo[4,3-c]quinolin-8-yl)(2-(5-(trifluorome thyl)pyridin-2-yl)pyrazolidin-1-yl)metha none |
| 501 | 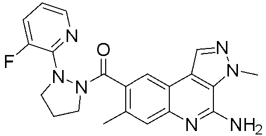 | (4-amino-3,7-dimethyl-3H-pyrazolo[3,4-c]quinolin-8-yl)(2-(3-fluoropyridin-2-yl) pyrazolidin-1-yl)methanone |
| 502 | 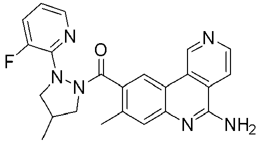 | (5-amino-8-methylbenzo[C] [2,6]naphthyr idin-9-yl)(2-(3-fluoropyridin-2-yl)-4-met hylpyrazolidin-1-yl)methanone |
| 503 | 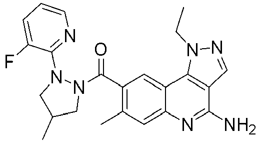 | (4-amino-1-ethyl-7-methyl-1H-pyrazolo[ 4,3-c]quinolin-8-yl)(2-(3-fluoropyridin-2-yl)-4-methylpyrazolidin-1-yl)methanone |
| 504 | 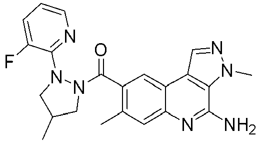 | (4-amino-3,7-dimethyl-3H-pyrazolo[3,4-c]quinolin-8-yl)(2-(3-fluoropyridin-2-yl)-4-methylpyrazolidin-1-yl)methanone |
| 505 | 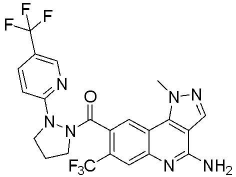 | (4-amino-1-methyl-7-trifluoromethyl-1H-pyrazolo[4,3-c]quinolin-8-yl)(2-(5-(triflu oromethyl)pyridin-2-yl)pyrazolidin-1-yl) methanone |
| 506 | 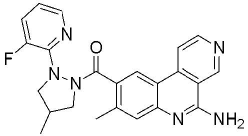 | (5-amino-8-methylbenzo[C] [2,7]naphthyr idin-9-yl)(2-(3-fluoropyridin-2-yl)-4-met hylpyrazolidin-1-yl)methanone |
| 507 | 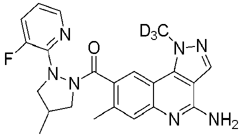 | (4-amino-7-methyl-1-(methyl-D3 )- H-pyrazolo [4,3-c]quinolin-8-yl)(2-(3-f luoropyridin-2-yl)-4-methylpyrazolidin-1 -yl)methanone |
| 508 | 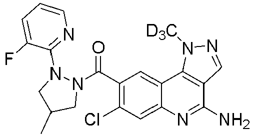 | (4-amino-7-chloro-1-(methyl-D3)-1H-pyr azolo[4,3-c]quinolin-8-yl)(2-(3-fluoropyr idin-2-yl)-4-methylpyrazolidin-1-yl)meth anone |
| 509 | 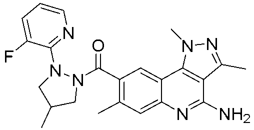 | (4-amino-1,3,7-trimethyl-1H-pyrazolo[4, 3-c]quinolin-8-yl)(2-(3-fluoropyridin-2-y l)-4-methylpyrazolidin-1-yl)methanone |
| 510 | 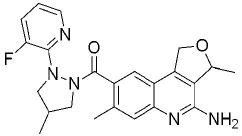 | (4-amino-3,7-dimethyl-1,3-dihydrofuro[3 ,4-c]quinolin-8-yl)(2-(3-fluoropyridin-2-y l)-4-methylpyrazolidin-1-yl)methanone |
| 511 | 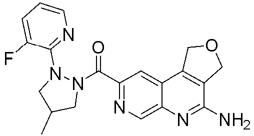 | (4-amino-1,3-dihydrofuro[3,4-c][1,7]nap hthyridin-8-yl)(2-(3-fluoropyridin-2-yl)-4 -methylpyrazolidin-1-yl)methanone |
| 512 | 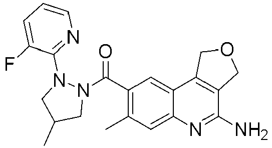 | (4-amino-7-methyl-1,3-dihydrofuro[3,4-c]quinolin-8-yl)(2-(3-fluoropyridin-2-yl)-4 -methylpyrazolidin-1-yl)methanone |

or a mesomer, racemate, enantiomer, diastereomer thereof, or a mixture thereof, or a pharmaceutically acceptable salt thereof.

The present invention further provides a method for preparing the compound of general formula (I) according to the present invention or a mesomer, racemate, enantiomer, diastereomer thereof, or a mixture thereof, or a pharmaceutically acceptable salt thereof.

The method can be implemented by using the process shown in Scheme 1. For example, adding A, B, and a condensing agent to a reaction solvent under nitrogen atmosphere, and subjecting them to a condensation reaction to obtain C; and then adding C, D, and a base to a reaction solvent under nitrogen atmosphere, and subjecting them to a substitution reaction to obtain a final product E. Scheme 1 is as follows:

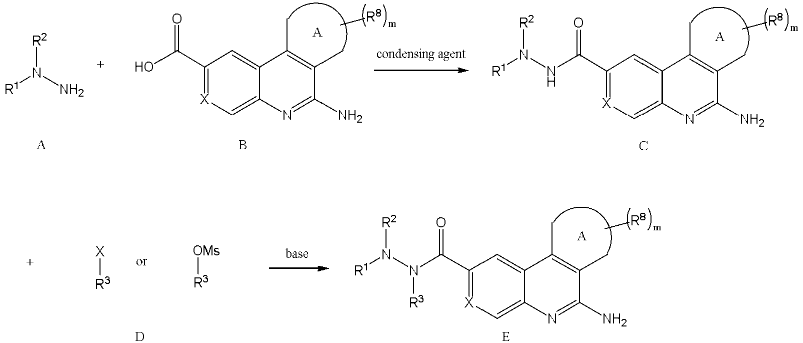

The method can be implemented by using the process shown in Scheme 2. For example, adding F, G, and a base to a reaction solvent under nitrogen atmosphere, and subjecting them to a condensation reaction to obtain a product H. Scheme 2 is as follows:

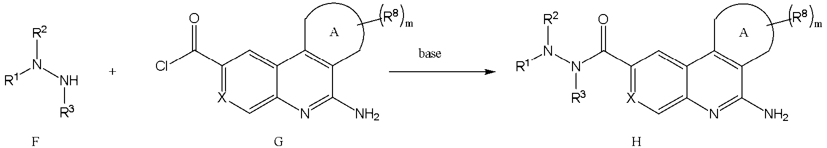

In the compounds presented in the above preparation methods, each of the substituents is as defined above.

The present invention further provides a pharmaceutical composition comprising the compound according to the present invention or a mesomer, racemate, enantiomer, diastereomer thereof, or a mixture thereof, or a pharmaceutically acceptable salt thereof, and a pharmaceutically acceptable carrier or excipient.

The present invention further relates to a use of the compound according to the present invention or a mesomer, racemate, enantiomer, diastereomer thereof, or a mixture thereof, or a pharmaceutically acceptable salt thereof or a pharmaceutical composition comprising the same in the preparation of a PRMT5 inhibitor.

The present invention further relates to a use of the compound according to the present invention or a mesomer, racemate, enantiomer, diastereomer thereof, or a mixture thereof, or a pharmaceutically acceptable salt thereof or a pharmaceutical composition comprising the same in the preparation of a medicament for the prevention and/or treatment of diseases related to PRMT5 activity, preferably the disease is a disease related to cancer or tumor.

The present invention further relates to the compound according to the present invention or a mesomer, racemate, enantiomer, diastereomer thereof, or a mixture thereof, or a pharmaceutically acceptable salt thereof or a pharmaceutical composition comprising the same for use as a PRMT5 inhibitor.

The present invention further relates to the compound according to the present invention or a mesomer, racemate, enantiomer, diastereomer thereof, or a mixture thereof, or a pharmaceutically acceptable salt thereof or a pharmaceutical composition comprising the same for use in a medicament for preventing and/or treating a disease related to PRMT5 activity, preferably the disease is a disease related to cancer or tumor.

The present invention further relates to a method for inhibiting PRMT5, comprising administering to a patient in need thereof an effective amount of the compound according to the present invention or a mesomer, racemate, enantiomer, diastereomer thereof, or a mixture thereof, or a pharmaceutically acceptable salt thereof or a pharmaceutical composition comprising the same.

The present invention further relates to a method for preventing and/or treating a disease related to PRMT5 activity, comprising administering to a patient in need thereof a prophylactically or therapeutically effective amount of the compound according to the present invention or a mesomer, racemate, enantiomer, diastereomer thereof, or a mixture thereof, or a pharmaceutically acceptable salt thereof or a pharmaceutical composition comprising the same,.

In a preferred embodiment, according to the present invention, the disease related to cancer and tumor is bladder cancer.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art known for the preparation of a pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic and pharmaceutically acceptable excipients suitable for the preparation of a tablet. These excipients can be inert excipients, such as calcium carbonate, sodium carbonate, lactose, calcium phosphate or sodium phosphate; granulating and disintegrating agents, such as microcrystalline cellulose, cross-linked sodium carboxymethyl cellulose, corn starch or alginic acid; binders, such as starch, gelatin, polyvinylpyrrolidone or gum arabic; and lubricants, such as magnesium stearate, stearic acid or talc. These tablets can be uncoated or coated by means of known techniques which can mask drug taste or delay the disintegration and absorption thereof in the gastrointestinal tract, thereby providing sustained-release effect over an extended period. For example, a water-soluble taste masking material can be used, such as hydroxypropyl methylcellulose or hydroxypropyl cellulose, or an extended release material can be used, such as ethyl cellulose, cellulose acetate butyrate.

An oral formulation can also be provided as a hard gelatin capsule in which the active ingredient is mixed with an inert solid diluent such as calcium carbonate, calcium phosphate or kaolin, or as a soft gelatin capsule in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of an aqueous suspension. Such an excipient is a suspending agent, such as sodium carboxymethylcellulose, methylcellulose, hydroxypropyl methylcellulose, sodium alginate, polyvinylpyrrolidone and gum arabic; or a dispersant or wetting agent. The aqueous suspension can also contain one or more preservatives such as ethylparaben or n-propylparaben, one or more colorants, one or more flavoring agents, and one or more sweeteners such as sucrose, saccharin or aspartame.

An oily suspension can be formulated by suspending the active ingredient in a vegetable oil such as peanut oil, olive oil, sesame oil or coconut oil, or in a mineral oil such as liquid paraffin. The oily suspension can contain a thickener, such as beeswax, hard paraffin or cetyl alcohol. The above sweetener and flavoring agent can be added to provide a palatable formulation. These compositions can be preserved by addition of an antioxidant such as butylated hydroxyanisole or α-tocopherol.

The pharmaceutical composition of the present invention can also be in the form of an oil-in-water emulsion. The oil phase can be a vegetable oil such as olive oil or peanut oil, or a mineral oil such as liquid paraffin or a mixture thereof. Suitable emulsifying agents can be naturally occurring phosphatides such as soybean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, preservative, colorant and antioxidant.

The pharmaceutical composition of the present invention can be in the form of a sterile injectable aqueous solution. The acceptable vehicles and solvents that can be employed include water, Ringer's solution and isotonic sodium chloride solution. The sterile injectable formulation can be a sterile injectable oil-in-water microemulsion in which the active ingredient is dissolved in the oil phase. For example, the active ingredient is dissolved in a mixture of soybean oil and lecithin. Then, the oily solution is introduced into a mixture of water and glycerol and processed to form a microemulsion. The injectable solution or microemulsion can be injected into a patient's bloodstream by local bolus injection. Alternatively, it can be advantageous to administrate the solution or microemulsion in such a way as to maintain a constant circulating concentration of the compound of the present invention. In order to maintain such a constant concentration, a continuous intravenous delivery device can be utilized.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic and parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

The compound of the present invention can be administered in the form of a suppository for rectal administration. These pharmaceutical composition can be prepared by mixing the active ingredient with a suitable non-irritating excipient which is solid at normal temperature but liquid in the rectum and thus can dissolve to release the active ingredient in the rectum. Such a material includes cocoa butter, glycerol gelatin, hydrogenated vegetable oil, mixture of polyethylene glycol of various molecular weights and fatty acid esters of polyethylene glycol.

It is well known to those skilled in the art that the dosage of a drug depends on a variety of factors including, but not limited to the following factors of activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound with the general formula or the type of the pharmaceutically acceptable salt thereof can be verified according to the traditional therapeutic regimens.

In the present invention, the compound and a pharmaceutically acceptable salt, hydrate, or solvate thereof can be contained as the active ingredient, and mixed with a pharmaceutically acceptable carrier or excipient to prepare a composition and form into a clinically acceptable dosage form. The derivatives of the present invention can be used in combination with other active ingredients, as long as they do not produce other adverse effects, such as allergic responses. The compound of the present invention can be used as the only active ingredient or in combination with other anticancer agents or immune checkpoint inhibitors. Combination therapy is implemented by administering each therapeutic component simultaneously, separately, or sequentially.

### Explanation of terms

Unless stated to the contrary, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl is a lower alkyl having 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent(s) may be substituted at any available connection point. The substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy and carboxylic ester group.

The term "alkenyl" refers to an alkyl as defined above consisting of at least two carbon atoms and at least one carbon-carbon double bond, such as vinyl, 1-propenyl, 2-propenyl, 1-, 2- or 3-butenyl and the like. The alkenyl may be substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio and heterocycloalkylthio.

The term "alkynyl" refers to an alkyl as defined above consisting of at least two carbon atoms and at least one carbon-carbon triple bond, such as ethynyl, propynyl, butynyl and the like. The alkynyl may be substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon group, in which the cycloalkyl ring has 3 to 20 carbon atoms, preferably 3 to 12 carbon atoms, and more preferably 3 to 6 carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. The polycyclic cycloalkyl includes spiro cycloalkyl, fused cycloalkyl and bridged cycloalkyl.

The term "spiro cycloalkyl" refers to a 5 to 20 membered polycyclic group with individual rings sharing one common carbon atom (called a spiro atom), which may contain one or more double bonds, but none of the rings has a completely conjugated π-electron system. The spiro cycloalkyl is preferably a 6 to 14 membered spiro cycloalkyl, and more preferably a 6 to 10 membered spiro cycloalkyl. According to the number of the spiro atoms shared between the rings, the spiro cycloalkyl is divided into a mono-spiro cycloalkyl, di-spiro cycloalkyl or poly-spiro cycloalkyl, and is preferably a mono-spiro cycloalkyl and di-spiro cycloalkyl, and more preferably a 4-membered/4-membered, 4-membered/5-membered, 4-membered/6-membered, 5-membered/5-membered or 5-membered/6-membered mono-spiro cycloalkyl. Non-limiting examples of the spiro cycloalkyl include:

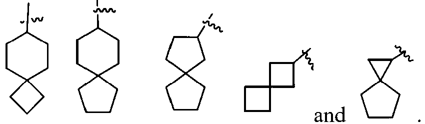

The term "fused cycloalkyl" refers to a 5 to 20 membered full-carbon polycyclic group, in which each ring shares a pair of adjacent carbon atoms with another ring in the system, and one or more rings may contain one or more double bonds, but none of the rings has a completely conjugated π-electron system. The fused cycloalkyl is preferably a 6 to 14 membered fused cycloalkyl, and more preferably a 6 to 10 membered fused cycloalkyl. According to the number of membered rings, the fused cycloalkyl may be divided into a bicyclic, tricyclic, tetracyclic or polycyclic fused cycloalkyl, and is preferably a bicyclic or tricyclic fused cycloalkyl, and more preferably a 5-membered/5-membered or 5-membered/6-membered bicyclic cycloalkyl. Non-limiting examples of the fused cycloalkyl include:

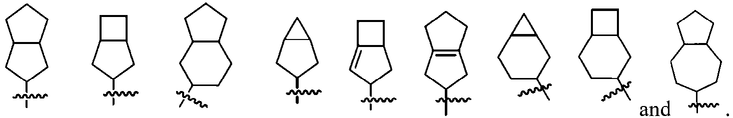

The term "bridged cycloalkyl" refers to a 5 to 20 membered full-carbon polycyclic group with any two of rings thereof sharing two carbon atoms that are not directly connected to each other, which may have one or more double bonds, but none of the rings has a completely conjugated π-electron system. The bridged cycloalkyl is preferably a 6 to 14 membered bridged cycloalkyl, and more preferably a 6 to 10 membered bridged cycloalkyl. According to the number of membered rings, the bridged cycloalkyl may be divided into a bicyclic, tricyclic, tetracyclic or polycyclic bridged cycloalkyl, and is preferably a bicyclic, tricyclic or tetracyclic bridged cycloalkyl, and more preferably a bicyclic or tricyclic bridged cycloalkyl. Non-limiting examples of the bridged cycloalkyl include:

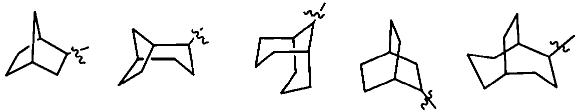

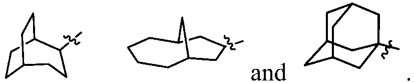

The cycloalkyl ring may be fused to an aryl, heteroaryl or heterocycloalkyl ring, in which the ring linking to the parent structure is cycloalkyl. Non-limiting examples include indanyl, tetrahydronaphthyl, benzocycloheptyl and the like. The cycloalkyl may be optionally substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy and carboxylic ester group.

The term "heterocyclyl" refers to a saturated or partially unsaturated monocyclic or polycyclic hydrocarbon group comprising 3 to 20 ring atoms, in which one or more ring atoms are heteroatoms selected from the group consisting of N, O and S(O)ₘ (wherein m is an integer from 0 to 2), but excluding -O-O-, -O-S- or -S-S- in the ring, the remaining ring atoms being carbon atoms. Preferably, the heterocyclyl comprises 3 to 12 ring atoms, 1 to 4 atoms of which are heteroatoms; most preferably 3 to 8 ring atoms, 1 to 3 atoms of which are heteroatoms; and most preferably 5 to 6 ring atoms, 1 to 2 atoms or 1 to 3 atoms of which are heteroatoms. Non-limiting examples of the monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. The polycyclic heterocyclyl includes spiro heterocyclyl, fused heterocyclyl and bridged heterocyclyl.

The term "spiro heterocyclyl" refers to a 5 to 20 membered polycyclic heterocyclyl group with individual rings sharing one common atom (called a spiro atom), in which one or more ring atoms are heteroatoms selected from the group consisting of N, O and S(O)ₘ (wherein m is an integer from 0 to 2), and the remaining ring atoms are carbon atoms. The spiro heterocyclyl may contain one or more double bonds, but none of the rings thereof has a completely conjugated π-electron system. The spiro heterocyclyl is preferably a 6 to 14 membered spiro heterocyclyl, and more preferably a 6 to 10 membered spiro heterocyclyl. According to the number of the spiro atoms shared between the rings, the spiro heterocyclyl is divided into mono-spiro heterocyclyl, di-spiro heterocyclyl or poly-spiro heterocyclyl, and is preferably a mono-spiro heterocyclyl and di-spiro heterocyclyl, and more preferably a 4-membered/4-membered, 4-membered/5-membered, 4-membered/6-membered, 5-membered/5-membered or 5-membered/6-membered mono-spiro heterocyclyl. Non-limiting examples of the spiro heterocyclyl include:

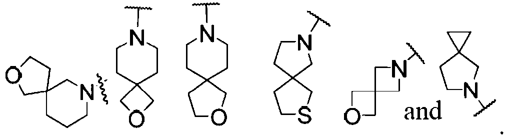

The term "fused heterocyclyl" refers to a 5 to 20 membered polycyclic heterocyclyl group, in which each ring shares a pair of adjacent atoms with another ring in the system, one or more rings may contain one or more double bonds, but none of the rings has a completely conjugated π-electron system, and one or more ring atoms are heteroatoms selected from the group consisting of N, O and S(O)ₘ (wherein m is an integer from 0 to 2), with the remaining ring atoms being carbon atoms. The fused heterocyclyl is preferably a 6 to 14 membered fused heterocyclyl, and more preferably a 8 to 10 membered fused heterocyclyl. According to the number of membered rings, the fused heterocyclyl may be divided into a bicyclic, tricyclic, tetracyclic or polycyclic fused heterocyclyl, and is preferably a bicyclic or tricyclic fused heterocyclyl, and more preferably a 5-membered/5-membered or 5-membered/6-membered bicyclic fused heterocyclyl. Non-limiting examples of the fused heterocyclyl include: 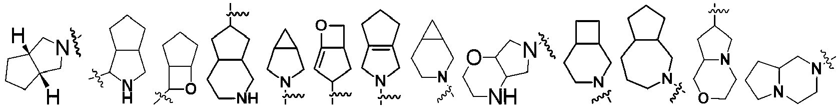 and

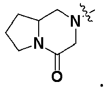

The term "bridged heterocyclyl" refers to a 5 to 14 membered polycyclic heterocyclyl group with any two of rings thereof sharing two atoms that are not directly connected to each other, which may have one or more double bonds, but none of the rings has a completely conjugated π-electron system, and in which one or more ring atoms are heteroatoms selected from the group consisting of N, O and S(O)ₘ (wherein m is an integer from 0 to 2), with the remaining ring atoms being carbon atoms. The bridged heterocyclyl is preferably a 6 to 14 membered bridged heterocyclyl, and more preferably a 8 to 10 membered bridged heterocyclyl. According to the number of membered rings, the bridged heterocyclyl may be divided into a bicyclic, tricyclic, tetracyclic or polycyclic bridged heterocyclyl, and is preferably a bicyclic, tricyclic or tetracyclic bridged heterocyclyl, and more preferably a bicyclic or tricyclic bridged heterocyclyl. Non-limiting examples of the bridged heterocyclyl include:

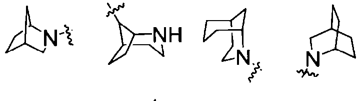

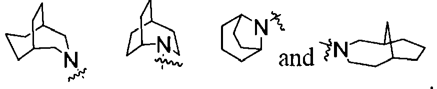

The heterocyclyl ring may be fused to an aryl, heteroaryl or cycloalkyl ring, in which the ring linking to the parent structure is heterocyclyl. Non-limiting examples thereof include: 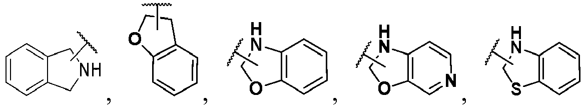 and the like.

The heterocyclyl may be optionally substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy and carboxylic ester group.

The term "aryl" refers to a 6 to 14 membered full-carbon monocyclic or fused polycyclic (i.e., rings sharing a pair of adjacent carbon atoms) group having a conjugated π-electron system, and preferably a 6 to 10 membered aryl, for example, phenyl and naphthyl. The aryl is more preferably phenyl. The aryl ring may be fused to a heteroaryl, heterocyclyl or cycloalkyl ring, i.e., as a fused aryl, in which the ring linking to the parent structure is an aryl ring. Non-limiting examples thereof include:

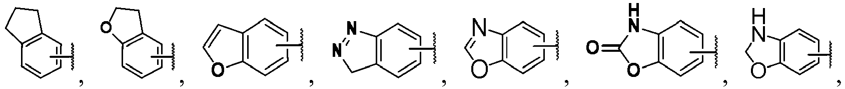

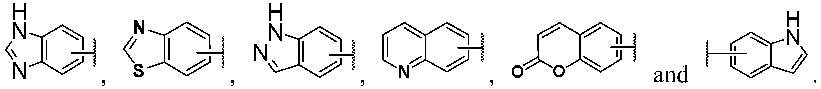

The aryl may be substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, carboxy and carboxylic ester group.

The term "heteroaryl" refers to a heteroaromatic system having 5 to 14 ring atoms, which comprises 1 to 4 heteroatoms selected from the group consisting of O, S and N. The heteroaryl is preferably a 5 to 10 membered heteroaryl having 1 to 3 heteroatoms, and more preferably a 5 or 6 membered heteroaryl having 1 to 2 heteroatoms, for example imidazolyl, furanyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl can be oxidized, for example, a C atom being oxidized to C=O, a S atom being oxidized to S=O or SO₂, and a N atom being oxidized to N⁺-O⁻. The heteroaryl ring may be fused to an aryl, heterocyclyl or cycloalkyl ring, i.e., as a fused heteroaryl, in which the ring linking to the parent structure is a heteroaryl ring. Non-limiting examples thereof include:

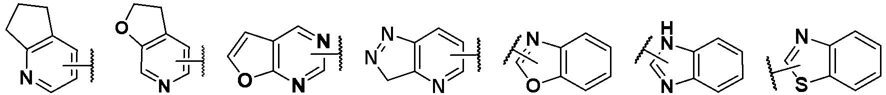

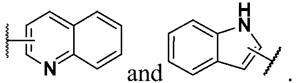

The heteroaryl may be optionally substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, carboxy and carboxylic ester group.

The term "alkoxy" refers to -O-(alkyl) and -O-(unsubstituted cycloalkyl), in which the alkyl and cycloalkyl are as defined above. Non-limiting examples of the alkoxy include methoxy, ethoxy, propoxy, butoxy, cyclopropyloxy, cyclobutyloxy, cyclopentyloxy and cyclohexyloxy. The alkoxy may be optionally substituted or unsubstituted. When it is substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, carboxy and carboxylic ester group.

In the chemical structure of the compound of the present disclosure, the bond " 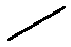 " represents an unspecified configuration, and that is to say, if chiral isomers exist in the chemical structure, the bond " 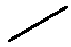 " may be " 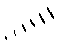 " or " 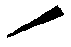 ", or contains both the configurations of " 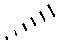 " and " 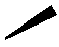 ".

The term "haloalkyl" refers to an alkyl group substituted by one or more halogens, in which the alkyl is as defined above.

The term "haloalkoxy" refers to an alkoxy group substituted by one or more halogens, in which the alkoxy is as defined above.

The term "deuterated alkyl" refers to an alkyl group substituted by one or more deuterium atoms, in which the alkyl is as defined above.

The term "deuterated alkoxy" refers to an alkoxy substituted by one or more deuterium atoms, in which the alkoxy is as defined above.

The term "hydroxyalkyl" refers to an alkyl group substituted by one or more hydroxy groups, in which the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to an =O group.

The term "thioxo" refers to a =S group.

The term "carboxy" refers to a -C(O)OH group.

The term "thiol" refers to a -SH group.

The term "ester" refers to a -C(O)O(alkyl) or -C(O)O(cycloalkyl) group, in which the alkyl and cycloalkyl are as defined above.

The compound of the present invention can be in a deuterated form. Each available hydrogen atom connected to a carbon atom may be independently replaced by a deuterium atom. Those skilled in the art are able to synthesize the deuterated forms of the compound with reference to relevant literatures. In preparing the deuterated forms of the compound, commercially available deuterated starting materials can be used, or they can be synthesized by conventional techniques with deuterated reagents.

The term "optional" or "optionally" means that the event or circumstance described subsequently can, but need not, occur, and such a description includes the situation in which the event or circumstance does or does not occur. For example, the expression "heterocyclyl optionally substituted by an alkyl group" means that an alkyl group can be, but need not be, present, and such a description includes the situation of the heterocyclyl being substituted by an alkyl group and the heterocyclyl being not substituted by an alkyl group.

The term "substituted" means that one or more hydrogen atoms in a group, preferably up to 5, and more preferably 1 to 3 hydrogen atoms in a group, are independently replaced by a corresponding number of substituents. It goes without saying that the substituents only exist in their possible chemical position. Those skilled in the art are able to determine whether the substitution is possible or impossible by experiments or theory without excessive efforts. For example, the combination of amino or hydroxy having free hydrogen and carbon atoms having unsaturated bond(s) (such as olefinic) may be unstable.

The term "pharmaceutical composition" represents a mixture containing one or more compounds described herein or physiologically/pharmaceutically acceptable salts or prodrugs thereof and other components such as physiologically/pharmaceutically acceptable carriers and excipients. The purpose of the pharmaceutical composition is to facilitate administration of a drug to an organism, which is conducive to absorption of an active ingredient(s) and thus exhibit biological activity.

The term "pharmaceutically acceptable salt" refers to a salt of the compound of the present invention, which is safe and effective in mammals and has desired biological activity.

### DETAILED DESCRIPTION OF THE INVENTION

The compounds of the present invention and the preparation thereof will be further understood through the examples below, which illustrate certain methods for preparing or using the compounds. However, it should be understood that the scope of the present invention is not limited by these examples. All currently known or further developed variations of the present invention are considered as falling within the scope of the present invention described and claimed herein.

The compounds of the present invention are prepared by using convenient starting materials and general preparation procedures. The present invention provides typical or preferential reaction conditions, such as reaction temperature, time, solvent, pressure and molar ratio of reactants. However, unless specially specified, other reaction conditions can also be adopted. Optimal conditions may vary with the use of specific reactants or solvents, but under normal circumstances, the steps and conditions optimized for reaction can be determined.

In addition, some protecting groups may be used in the present invention to protect certain functional groups from unnecessary reactions. The protecting groups suitable for various functional groups and their protection or deprotection conditions are already well known to those skilled in the art. For example, "Protective Groups in Organic Synthesis" by T. W. Greene and G. M. Wuts (3rd edition, Wiley, New York, 1999) and the citations in the book describe the protection or deprotection of a large number of protecting groups in detail.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific methods used may be referred to the examples described in the present invention. Of course, other similar isolation and purification methods can also be used. They can be characterized using conventional methods (including physical constants and spectral data).

The structures of the compounds are identified by nuclear magnetic resonance (NMR) and/or mass spectrometry (MS). NMR shift is given in units of 10⁻⁶ (ppm). NMR is determined on a WNMR-I-400 MHz nuclear magnetic spectrometer from Zhongke-Niujin. The solvents for determination are deuterated dimethyl sulfoxide (DMSO-*d₆*), deuterated chloroform (CDCl₃), and deuterated methanol (CD₃OD), and the internal standard is tetramethylsilane (TMS).

MS is determined on a 1260 Infinity II 6125B single quadrupole LC-MS (manufacturer: Agilent), for which the chromatographic column is kinetex XB-C18 100A 1.7µm (30×3mm) (manufacturer: Phenomenex) and the mobile phase is acetonitrile/water (0.1% FA). Preparative liquid chromatography is determined using a 1260 Infinity II liquid chromatograph (manufacturer: Agilent), for which the chromatographic column is Xtimate C18 5µm (21.2×250mm) (manufacturer: Welch Materials) and the mobile phase is acetonitrile/water. GF254 silica gel plates from Qingdao Haiyang Chemical are used for thin-layer chromatography (TLC). For the silica gel plates used, the specification is 0.20 mm to 0.25 mm for the thin-layer chromatography for monitoring of reactions, and 0.5 mm for the thin-layer chromatography for separation and purification.

Silica gels of 100 to 200 mesh, 200 to 300 mesh and 300 to 400 mesh from Qingdao Haiyang Chemical are used as a carrier for silica gel column chromatography.

The known starting materials of the present invention can be prepared by the known methods in the art, or can be purchased from Wanghua Mall, Tansoole, ChemicalBook, LabNetwork, Beijing Ouhe Technology, Sigma, J&K Scientific, Yishiming, Shanghai Shuya Chemical, Shanghai Innochem Science & Technology, Energy Chemical, Shanghai Bide Pharmatech, Shanghai Leyan, Nanjing PharmaBlock and the like.

Unless specifically stated in the examples, all reactions can be carried out under nitrogen atmosphere.

Argon, nitrogen or hydrogen atmosphere means that a reaction flask is connected to an argon, nitrogen or hydrogen balloon with a volume of about 1 L.

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane (DCM), ether, methanol (MeOH), ethanol (EtOH), dimethyl sulfoxide (DMSO), 1,4-dioxane, N-methylpyrrolidone (NMP), pyridine, water and the like. Unless specifically stated in the examples, a solution refers to an aqueous solution.

The chemical reactions described in the present invention are generally carried out under normal pressure. The reaction time and conditions are, for example, between -78°C and 200°C at one atmosphere, and completed within about 1 to 24 hours. If the reaction is carried out overnight, the reaction time is generally 16 hours. Unless specifically stated in the examples, the reaction temperature is room temperature, which is 20°C to 30°C.

Reaction processes in the examples are monitored by thin-layer chromatography (TLC). The developing systems used for the reactions include: A: dichloromethane-methanol system, B: petroleum ether-ethyl acetate system, and C: acetone, and the volume ratios of the solvents are adjusted depending on the polarity of the compounds.

The eluent systems of the column chromatography and the developing systems of the thin-layer chromatography used for purifying the compounds include: A: dichloromethane-methanol system, and B: petroleum ether-ethyl acetate system, and the volume ratios of the solvents are adjusted depending on the polarity of the compounds. A small amount of alkaline or acidic reagents such as triethylamine and trifluoroacetic acid can also be added for adjustment.

Unless otherwise defined, all professional and scientific terms used herein have the same meaning as those familiar to those skilled in the art. In addition, any methods and materials similar or equivalent to the content described herein can be applied to the methods of the present invention.

### Example 1: Preparation of 2-amino-N',3-dimethyl-N'-(pyrimidin-2-yl)-N-((5-(trifluoromethyl)pyridin-2-yl)methyl) quinoline-6-carbohydrazide (1)

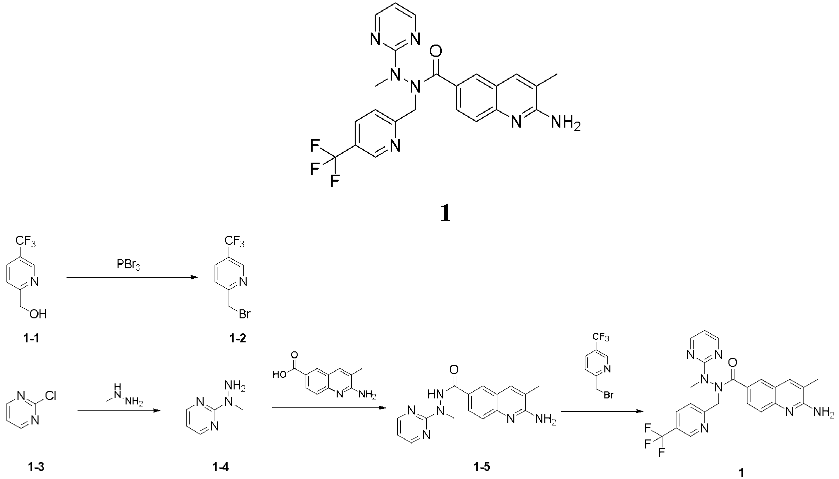

### Step 1: Preparation of 2-(bromomethyl)-5-(trifluoromethyl)pyridine (1-2)

(5-(trifluoromethyl)pyridin-2-yl)methanol (1-1) (500 mg, 2.82 mmol) was dissolved in DCM (5 mL), and cooled to 0°C in an ice bath under nitrogen atmosphere, and then phosphorus tribromide (0.76 g, 2.82 mmol) was added. After the addition was completed, the resulting mixture was naturally warmed up to room temperature and stirred for 2 hours. The reaction solution was cooled to 0°C, followed by the addition of ice water (10 mL), and extracted with DCM (10 mL × 3). The organic phases were combined, dried over anhydrous sodium sulfate, and filtered, and the filtrate was concentrated under reduced pressure. The residues were purified by silica gel column chromatography (DCM/MeOH = 20/1) to obtain Compound 1-2 (0.32 g, yield 47%, purity 90%, a brown liquid).

LC-MS (ESI+): 240.1 m/z [M+H]⁺.

### Step 2: Preparation of 2-(1-methylhydrazino)pyrimidine (1-4)

2-Chloropyrimidine (1-3, 500 mg, 4.36 mmol) was dissolved in EtOH (10 mL), followed by the addition of methylhydrazine (350 mg, 7.68 mmol), and the resulting mixture was stirred at 65°C overnight. Water (20 mL) was added to the reaction solution, and the reaction solution was extracted with EtOAc (20 mL × 3). The organic phases were combined, dried over anhydrous sodium sulfate, and filtered, and the filtrate was concentrated under reduced pressure. The residues were purified by Prep-TLC (petroleum ether/ethyl acetate = 1/1) to obtain Compound 1-4 (0.2 g, yield 37%, purity 95%, a white solid).

LC-MS (ESI+): 125.1 m/z [M+H]⁺.

### Step 3: Preparation of 2-amino-N',3-dimethyl-N'-(pyrimidin-2-yl)quinoline-6-carbohydrazide (1-5)

2-Amino-3-methylquinoline-6-carboxylic acid (922 mg, 4.56 mmol) was dissolved in DMF (5 mL), followed by the addition of Compound 1-4 (566 mg, 4.56 mmol), HATU (2.08 g, 5.47 mmol) and DIEA (1.77 g, 13.68 mmol) successively under nitrogen atmosphere, and the resulting mixture was stirred at room temperature for 18 hours. Water (10 mL) was added to the reaction solution, and the reaction solution was extracted with EtOAc (20 mL × 3). The organic phases were combined, dried over anhydrous sodium sulfate, and filtered, and the filtrate was concentrated under reduced pressure. The residues were purified by silica gel column chromatography (DCM/MeOH = 15/1) to obtain Compound 1-5 (760 mg, yield 54%, purity 90%, a light yellow solid).

LC-MS (ESI+): 309. 0 m/z [M+H]⁺.

### Step 4: Preparation of 2-amino-N',3-dimethyl-N'-(pyrimidin-2-yl)-N-((5-(trifluoromethyl)pyridin-2-yl)methyl) quinoline-6-carbohydrazide (1)

Compound 1-5 (231 mg, 0.75 mmol) was dissolved in DMF (6 mL), followed by the addition of 2-(bromomethyl)-5-(trifluoromethyl)pyridine (1-2) (180 mg, 0.75 mmol) and K₂CO₃ (312 mg, 2.26 mmol) successively, and the resulting mixture was stirred at room temperature overnight. Water (20 mL) was added to the reaction solution, and the reaction solution was extracted with EtOAc (20 mL × 3). The organic phases were combined, dried over anhydrous sodium sulfate, and filtered, and the filtrate was concentrated under reduced pressure. The residues were purified by preparative high performance liquid chromatography (water/acetonitrile=95%/5% - 10%/90%), and put to lyophilization to obtain Compound **1** (35.4 mg, yield 10%, purity 99%, a white solid).

LC-MS (ESI+): 468.0 m/z [M+H]⁺.

¹H NMR (400 MHz, DMSO-*d₆*) δ 8.86 (s, 1H), 8.42 - 8.31 (m, 2H), 8.23 - 8.10 (m, 1H), 7.82 (d, J = 8.2 Hz, 1H), 7.75 - 7.61 (m, 2H), 7.44 (d, J = 8.3 Hz, 1H), 7.26 (d, J = 8.6 Hz, 1H), 6.79 - 6.69 (m, 1H), 6.47 (s, 2H), 5.36 (d, J = 15.6 Hz, 1H), 4.56 (d, J = 15.6 Hz, 1H), 3.25 (s, 3H), 2.13 (s, 3H).

### Example 2: Preparation of 2-amino-3-methyl-N'-(methyl-d3)-N'-(pyrimidin-2-yl)-N-((5-(trifluoromethyl)pyridin-2 -yl)methyl)quinoline-6-carbohydrazide (2)

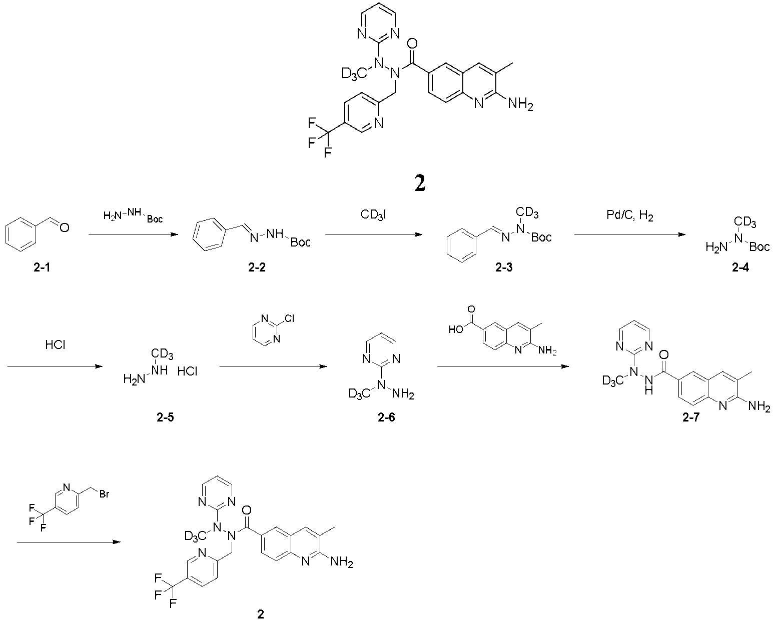

### Step 1: Preparation of tert-butyl (E)-2-benzylidenehydrazine-1-carboxylate (2-2)

Benzaldehyde (2-1, 1.00 g, 9.42 mmol) was dissolved in THF (10 mL), followed by the addition of BocN₂H₃ (1.25 g, 9.42 mmol), and the resulting mixture was stirred at room temperature for 4 hours. The reaction solution was concentrated under reduced pressure to obtain a crude product Compound 2-2 (2.1 g, yield 101%, a white solid).

### Step 2: Preparation of tert-butyl (E)-2-benzylidene-1-(methyl-d₃)hydrazine-1-carboxylate (2-3)

Compound 2-2 (2.10 g, 9.56 mmol) was dissolved in THF (10 mL), followed by the addition of t-BuOK (1.29 g, 11.46 mmol), and purged with nitrogen, followed by the dropwise addition of CD₃I (1.6 g, 11.46 mmol), and the resulting mixture was stirred at room temperature for 16 hours. The reaction solution was concentrated under reduced pressure to obtain Compound 2-3 (2.0 g, yield 22%, a yellow oil).

¹H NMR (400 MHz, DMSO-d6): δ 7.80 (s, 1H), 7.69 (dt, *J* = 6.1, 1.4 Hz, 2H), 7.45 - 7.39 (m, 2H), 7.39 - 7.33 (m, 1H), 1.51 - 1.48 (m, 9H).

### Step 3: Preparation of tert-butyl 1-(methyl-d₃)hydrazine-1-carboxylate (2-4)

Compound 2-3 (500 mg, 2.11 mmol) was dissolved in MeOH (5 mL), followed by the addition of Pd/C (250 mg, 50% wt) under nitrogen atmosphere, and the resulting mixture was purged with hydrogen and then stirred at room temperature for 16 hours. The reaction solution was filtered through Celite, and the filtrate was concentrated under reduced pressure to obtain Compound 2-4 (260 mg, yield 82%, a colourless oil).

¹H NMR (400 MHz, DMSO-*d6*): δ 4.48 (s, 2H), 1.40 (s, 9H).

### Step 4: Preparation of (methyl-d₃)hydrazine hydrochloride (2-5)

Compound 2-4 (260 mg, 1.74 mmol) was dissolved in EtOAc/HCl (4 *M,* 5 mL), and the resulting mixture was stirred at room temperature for 16 hours. The reaction solution was directly filtered, and the filter cake was dried under vacuum to obtain Compound 2-5 (167 mg, yield 112%, a white solid).

¹H NMR (400 MHz, DMSO-*d₆*): δ 4.23 (s, 2H), 3.80 (s, 1H).

### Step 5: Preparation of 2-(1-(methyl-d₃)hydrazino)pyrimidine (2-6)

Compound 2-5 (167 mg,3.66 mmol) was dissolved in EtOH (10 mL), followed by the addition of 2-chloropyrimidine (420 mg,3.66 mmol) and Na₂CO₃ (1165 mg, 10.99 mmol) successively, and the resulting mixture was heated to 75°C and stirred for 16 hours. The reaction solution was cooled to room temperature, followed by the addition of ethyl acetate (10 mL) and water (10 mL), and the organic phase was washed with saturated brine, dried over anhydrous sodium sulfate and filtered. The filtrate was concentrated under reduced pressure, and the residues were purified by silica gel column chromatography (petroleum ether/ethyl acetate = 2/1) to obtain Compound 2-6 (50 mg, yield 11%, a yellow solid).

LC-MS (ESI+): m/z 128.1 [M+1]⁺.

### Step 6: Preparation of 2-amino-3-methyl-N-methyl-d₃-pyrimidin-2-ylquinoline-6-carbohydrazide (2-7)

Compound 2-6 (93 mg, 0.46 mmol) was dissolved in DMF (2 mL), followed by the addition of HATU(227 mg, 0.60 mmol), 2-(1-(methyl-*d*₃)hydrazino)pyrimidine (57 mg, 0.46 mmol) and DIEA (178 mg, 1.38 mmol) successively under nitrogen atmosphere, and the resulting mixture was stirred at room temperature for 16 hours. Ethyl acetate (10 mL) and water (10 mL) were added to the reaction solution, and the organic phase was washed with saturated brine, dried over anhydrous sodium sulfate and filtered. The filtrate was concentrated under reduced pressure. The residues were purified by Prep-TLC (DCM/MeOH = 10/1) to obtain Compound 2-7 (28 mg, yield 20%, a yellow solid).

LC-MS (ESI+): m/z 312.2 [M+1]⁺.

### Step 7: Preparation of 2-amino-3-methyl-N'-(methyl-d3)-N'-(pyrimidin-2-yl)-N-((5-(trifluoromethyl)pyridin-2 -yl)methyl)quinoline-6-carbohydrazide (2)

Compound 2-7 (28 mg, 0.09 mmol) was dissolved in DMF (2 mL), followed by the addition of K₂CO₃ (37 mg, 0.27 mmol) and 2-bromomethyl-5-trifluoromethylpyridine (22 mg, 0.09 mmol) successively, and the resulting mixture was stirred at room temperature for 16 hours. Ethyl acetate (10 mL) and water (10 mL) were added to the reaction solution, and the organic phase was washed with saturated brine, dried over anhydrous sodium sulfate and filtered. The filtrate was concentrated under reduced pressure. The residues were purified by Prep-TLC (DCM/MeOH = 10/1) to obtain Compound 2 (12.2 mg, yield 28%, a white solid).

LC-MS (ESI+): m/z 471.2 [M+1]⁺.

¹H NMR (400 MHz, DMSO-*d6*): δ 8.90 (s, 1H), 8.43 - 8.35 (m, 2H), 8.21 (d, J = 8.2 Hz, 1H), 7.85 (d, J = 8.4 Hz, 1H), 7.71 (d, J = 19.5 Hz, 2H), 7.47 (d, J = 8.6 Hz, 1H), 7.30 (d, J = 8.7 Hz, 1H), 6.78 (s, 1H), 6.48 (s, 2H), 5.40 (d, J = 15.5 Hz, 1H), 4.59 (d, J = 15.8 Hz, 1H), 2.17 (s, 3H).

The following compounds were obtained according to the synthetic method in Example 2, using corresponding raw materials.

| Example | Structure | Mass spectrometry data (ESI+): m/z | Nuclear magnetic data |
|---|---|---|---|
| 3 | 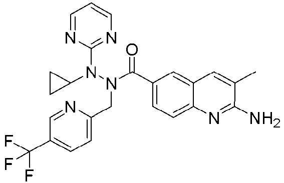 | 494.2 | ¹H NMR (400 MHz, DMSO-d6): δ 8.84 (s, 1H), 8.58 (d, J = 4.8 Hz, 2H), 8.22 (d, J = 8.3 Hz, 1H), 7.92 (d, J = 8.3 Hz, 1H), 7.69 (d, J = 1.9 Hz, 1H), 7.59 (s, 1H), 7.43 - 7.38 (m, 1H), 7.26 (d, J = 8.7 Hz, 1H), 7.01 (t, J = 4.8 Hz, 1H), 6.50 (s, 2H), 5.34 (d, J = 15.7 Hz, 1H), 4.67 (d, J = 15.7 Hz, 1H), 2.76 (s, 1H), 2.16 (s, 3H), 0.76 - 0.74 (m, 1H), 0.54 - 0.38 (m, 2H), 0.14 (s, 1H). |
| 4 | 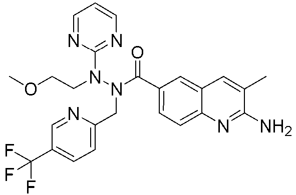 | 512.2 | ¹H NMR (400 MHz, DMSO-d6): δ 8.88 (s, 1H), 8.39 (d, J = 4.8 Hz, 2H), 8.18 (d, J = 8.4 Hz, 1H), 7.86 (d, J = 8.3 Hz, 1H), 7.68 (s, 1H), 7.61 (s, 1H), 7.41 - 7.35 (m, 1H), 7.24 (d, J = 8.6 Hz, 1H), 6.78 (t, J = 4.8 Hz, 1H), 6.45 (s, 2H), 5.38 (d, J = 16.3 Hz, 1H), 4.58 (d, J = 16.3 Hz, 1H), 4.05 - 3.92 (m, 1H), 3.90 - 3.82 (m, 1H), 3.36 - 3.30 (m, 2H), 2.96 (s, 3H), 2.13 (s, 3H). |
| 5 | 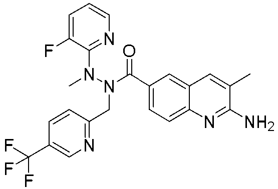 | 485.5 | ¹HNMR (400 MHz, DMSO-d6) δ 8.91 (s,1H), 8.21 (s,1H), 8.03 (s,1H), 7.87 (s,1H), 7.83 - 7.74 (m,2H), 7.62 (d, *J* = 8.1 Hz,1H), 7.50 (s,1H), 7.40 (s,1H), 6.95 - 6.90 (m,1H), 6.52 (s,2H), 5.33 (d, J = 16.3 Hz,1H), 4.70 (d, J = 14.0 Hz, 1H), 3.25 (s,3H), 2.21 (s, 3H). |
| 6 | 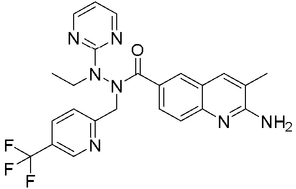 | 482.2 | ¹HNMR (400 MHz, DMSO-d6) δ 8.95 (s,1H), 8.49 (d, J = 4.6 Hz,2H), 8.26 (d, J = 9.1 Hz, 1H), 7.93 (d, J = 7.8 Hz, 1H), 7.71 (s, 1H), 7.66 (s,1H), 7.44 (d, J = 9.1 Hz,1H), 7.31 (d, J = 7.7 Hz,1H), 6.87 (s,1H), 6.49 (s,1H), 5.48 (d, J = 16.8 Hz,1H), 4.58 (d, J = 16.4 Hz,1H), 3.78 (q, J = 6.4 Hz,2H), 2.19 (s,3H), 0.92 (t,J=6.4 Hz,3H). |
| 7 | 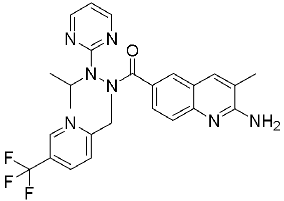 | 496.2 | |
| 8 | 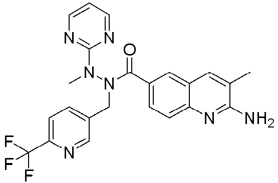 | 468.2 | |
| 9 | 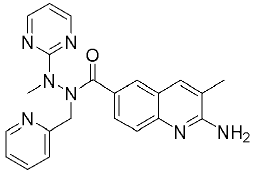 | 400.2 | ¹H NMR (400 MHz, DMSO-d6) δ 8.58 - 8.35 (m, 3H), 7.97 - 7.79 (m, 2H), 7.73 - 7.57 (m, 2H), 7.48 -7.43 (m, 1H), 7.35 - 7.25 (m, 2H), 6.80 (t, J = 5.6 Hz, 1H), 5.44 (d, J = 14.8 Hz, 1H), 4.41 (d, J = 15.2 Hz, 1H), 3.16 (s, 3H), 2.30 (s, 0.9H), 2.19 (s, 2.1H). |
| 10 | 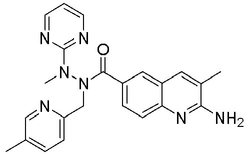 | 414.4 | ¹H NMR (400 MHz, DMSO-d6) δ 8.41 (s, 1H), 8.32 (s, 1H), 8.17 (s, 1H), 7.96 (dd, J = 8.0, 1Hz, 1H), 7.90 (s, 1H), 7.66 (d, J = 8.0 Hz, 1H), 7.49 (d, J = 8.0 Hz, 1H), 7.43 (d, J = 8.0 Hz, 1H), 6.72 (s, 2H), 5.37 (s, 2H), 4.12 (s, 2H), 3.17 (s, 3H), 2.30 (s, 3H), 2.21 (s, 3H). |
| 11 | 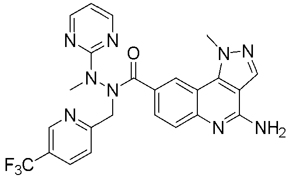 | 508.2 | ¹HNMR (400 MHz, DMSO-d6) δ 9.64 (s, 1H), 8.92 (s,1H), 8.61 (s,1H), 8.47 (d, J = 6.4 Hz,1H), 8.38 (s, 1H),8.27 - 8.24 (m, 1H), 7.94 - 7.91 (m, 2H), 7.75 ( d, J = 8.4 Hz,1H), 6.88 (t, J = 4.8 Hz,1H), 5.40 (d, J = 15.6 Hz, 1H), 4.76 (d, J = 15.6 Hz,1H), 4.29 (s,3H), 3.32 (s, 3H). |
| 12 | 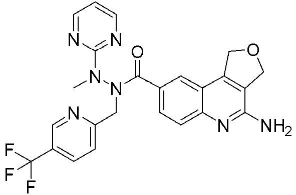 | 496.2 | |
| 13 | 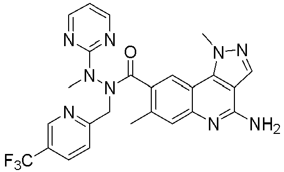 | 522.2 | ¹HNMR (400 MHz, DMSO-d6) δ 8.94(s, 1H), 8.55 (s,1H), 8.51(d, J = 4.8 Hz,2H), 8.27 (dd, J = 8.0, 1H), 8.07 (s, 1H), 7.87 ( d, J = 8.4 Hz,1H), 7.59 (s, 1H), 6.93 (t, J = 4.4 Hz,1H), 5.49 (d, J = 16.0 Hz,1H), 4.76 (d, J = 16.0 Hz,1H), 4.13 (s,3H), 3.20 (s,3H), 2.56 (s, 3H). |
| 14 | 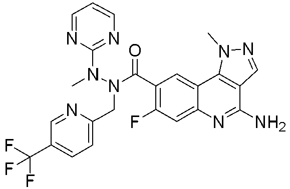 | 526.3 | |
| 15 | 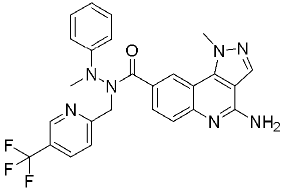 | 506.3 | ¹H NMR (400 MHz, DMSO-d6) δ 8.94 (s, 1H), 8.46 (s, 1H), 8.22 - 8.19 (m, 2H), 7.81 - 7.77 (m, 2H), 7.54 (d, J = 8.6 Hz, 1H), 7.31 (t, J = 8.0 Hz, 2H), 7.20 (s, 2H), 6.95 (d, J = 7.8 Hz, 2H), 6.89 (t, J = 7.2 Hz,1H), 5.27 (d, J = 15.8 Hz, 1H), 4.60 (d, J = 14.8 Hz, 1H), 3.89 (s, 3H), 3.08 (s, 3H). |
| 16 | 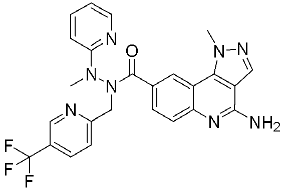 | 508.2 | ¹H NMR (400 MHz, DMSO-d6) δ 8.95 (s, 1H), 8.38 (s, 1H), 8.22 (d, J = 5.4 Hz, 3H), 7.86 - 7.84 (m, 1H), 7.73 (d, J = 8.4 Hz, 1H), 7.65 (t, J = 8.0 Hz, 1H), 7.53 (d, J = 8.8 Hz, 1H), 7.19 (s, 2H), 6.97 (d, J *=* 8.4 Hz, 1H), 6.83 (t, J = 6.0 Hz, 1H), 5.33 (d, J = 15.8 Hz, 1H), 4.70 (d, J = 15.6 Hz, 1H), 4.00 (s, 3H), 3.20 (s, 3H). |
| 17 | 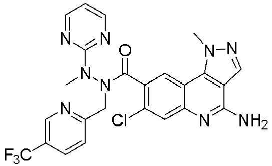 | 541.1 | |
| 18 | 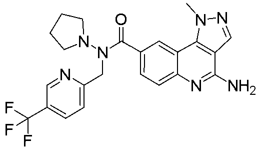 | 470.3 | ¹HNMR (400 MHz, DMSO-d6) δ 9.02 (s, 1H), 8.64 (d, J = 4.0 Hz, 1H), 8.22 (s, 1H), 8.22 - 8.17 (m, 1H), 7.82 (dd, J = 8.0 Hz, 1H), 7.64 (d, J = 12.0 Hz, 1H), 7.41 (d, J = 8.0 Hz, 1H), 6.99 (s, 2H), 5.37 (s, 2H), 4.34 (s, 3H), 4.13 - 4.10 (m, 2H), 3.72 - 3.68 (m, 2H), 2.08 - 2.04 (m, 4H). |
| 19 | 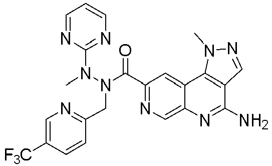 | 509.2 | |
| 33 | 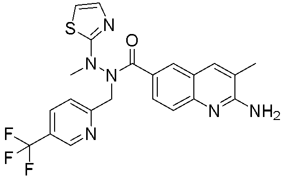 | 473.1 | |
| 34 | 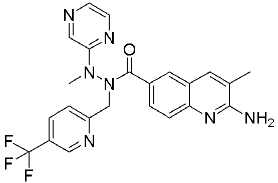 | 468.1 | |
| 35 | 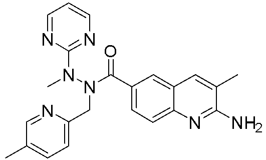 | 414.2 | ¹H NMR (400 MHz, DMSO-*d₆*) δ 8.41 (s, 1H), 8.32 (d, *J =* 1.8 Hz, 1 H),, 8.17 (s, 1H), 7.96 (dd, *J* = 8.0, 2.0Hz, 1H), 7.90 (s, 1H), 7.66 (d, *J =* 8.0 Hz, 1H), 7.49 (d, *J* = 8.0 Hz, 1H), 7.43 (d, *J =* 8.0 Hz, 1H), 6.72 (s, 2H), 5.37 (s, 2H), 3.17 (s, 3H), 2.30 (s, 3H), 2.21 (s, 3H). |
| 36 | 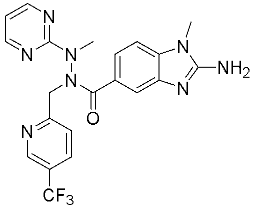 | 457.1 | ¹H NMR (400 MHz, DMSO-*d₆*) δ 9.07 (s, 2H), 8.89 (s, 1H), 8.40 (d, *J* = 4.0 Hz, 2H), 8.31 (d, *J =* 8.0 Hz, 1H), 7.98 - 7.94 (m, 2H), 7.77 - 7.73 (m, 2H), 6.78 (t, *J =* 4.0 Hz, 1H), 5.75 (s, 2H), 3.76 (s, 3H), 3.20 (s, 3H). |
| 37 | 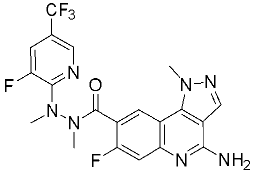 | 466.1 | |
| 38 | 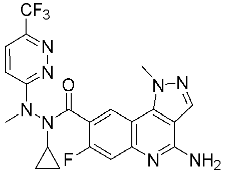 | 475.1 | |
| 39 | 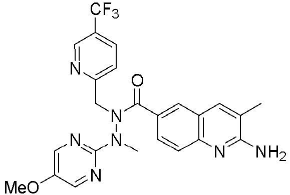 | 498.2 | ¹H NMR (400 MHz, DMSO-*d₆*) δ 8.88 (s, 1H), 8.22-8.18 (m, 3H), 7.83 (d,*J* = 8.0 Hz, 1H), 7.77 (s, 1H), 7.70 (s, 1H), 7.51 (d, *J =* 8.0 Hz, 1H), 7.31 (d, *J* = 8.0 Hz, 1H), 6.44 (s, 2H), 5.35 (d, *J* = 16.0 Hz, 1H), 4.54 (d, *J* = 16.0 Hz, 1H), 3.75 (s, 3H), 3.24 (s, 3H), 2.18 (s, 3H). |
| 40 | 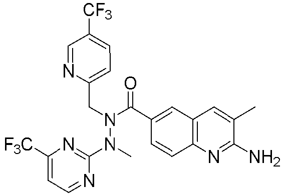 | 536.1 | ¹H NMR (400 MHz, DMSO-*d₆*) δ 8.81 (s, 1H), 8.67 (s, 1H), 8.18 (d, *J* = 8.0 Hz, 1H), 7.86 (s, 1H), 7.70 (s, 2H), 7.45 (s, 1H), 7.33 (s, 1H), 7.15 (s, 1H), 6.53 (s, 2H), 5.14 (s, 1H), 4.92 (s, 1H), 3.35 (s, 3H), 2.17 (s, 3H). |
| 41 | 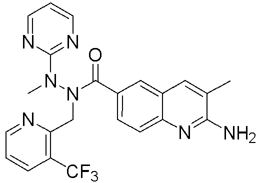 | 468.2 | |
| 42 | 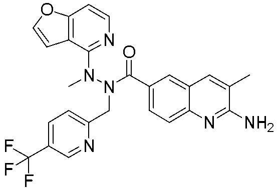 | 507.2 | ¹H NMR (400 MHz, DMSO-*d₆*) δ 8.92 (s, 1H), 8.30 (s, 1H), 8.20 (d, *J* = 8.0 Hz, 1H), 8.00 (s, 1H), 7.79 (d, *J* = 8.0 Hz, 2H), 7.67 (s, 1H), 7.51 (d, *J* = 8.0 Hz, 1H), 7.30 (d, *J* = 8.0 Hz, 1H), 7.13 (d, *J =* 8.0 Hz, 1H), 6.83 (s, 1H), 6.48 (s, 2H), 5.52 (d, *J* = 16.0 Hz, 1H), 4.55 (d, *J =* 16.0 Hz, 1H), 3.34 (s, 3H), 2.16 (s, 3H). |
| 43 | 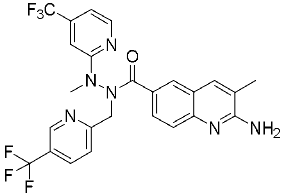 | 535.2 | |
| 44 | 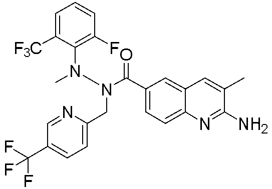 | 552.2 | |
| 45 | 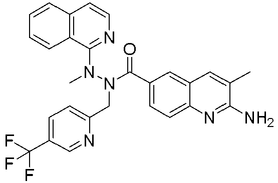 | 517.2 | |
| 46 | 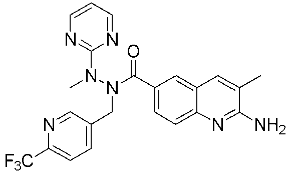 | 468.2 | ¹H NMR (400 MHz, DMSO-*d₆*) δ 8.88 (d, *J* = 2.4 Hz, 1H), 8.45 (d, *J =* 4.8 Hz, 2H), 8.30 (dd, *J* = 8.3, 2.3 Hz, 2H), 8.14 - 8.00 (m, 2H), 7.72 (d, *J* = 8.2 Hz, 1H), 7.59 (d, *J =* 9.0 Hz, 1H), 6.82 (t, *J =* 4.8 Hz, 1H), 6.00 (s, 1H), 4.82 (dd, *J* = 107.4, 5.9 Hz, -1H), 3.42 (s, 3H), 2.36 (s, 3H). |
| 47 | 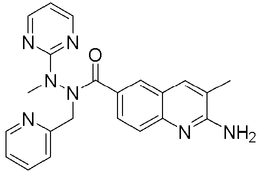 | 400.2 | ¹H NMR (400 MHz, DMSO-*d₆*) δ 8.42 (dd, *J =* 8.9, 4.7 Hz, 3H), 8.24 (s, 1H), 8.00 (d, *J* = 8.7 Hz, 1H), 7.81 (t, *J =* 8.1 Hz, 2H), 7.72 (d, *J =* 10.6 Hz, 2H), 7.32 (t, *J* = 6.4 Hz, 3H), 6.80 (q, *J =* 4.5 Hz, 1H), 6.49(s, 2H), 3.16 (s, 4H), 2.19 (s, 4H). |
| 48 | 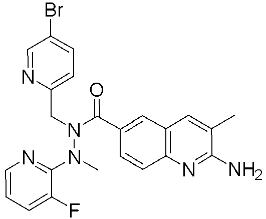 | 495.1 | |
| 49 | 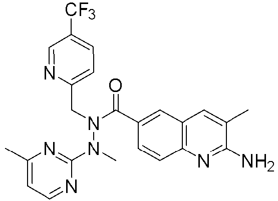 | 482.2 | ¹H NMR (400 MHz, DMSO-*d₆*) δ 8.23 (d, *J =* 8.1 Hz, 2H), 8.16 (s, 1H), 7.90 (d, *J* = 8.4 Hz, 1H), 7.74 (d, *J* = 16.2 Hz, 2H), 7.49 (d, *J =* 8.8 Hz, 1H), 7.32 (d, *J* = 8.6 Hz, 1H), 6.66 (s, 1H), 6.50 (s, 3H), 5.32 (d, *J =* 15.7 Hz, 1H), 4.68 (d, *J =* 15.7 Hz, 1H), 2.26 (s, 3H), 2.20 (s, 3H). |
| 50 | 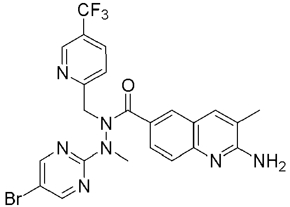 | 546.1 | ¹H NMR (400 MHz, DMSO-*d₆*) δ 8.53 (s, 2H), 8.26 - 8.13 (m, 2H), 7.81 (d, *J* = 36.3 Hz, 4H), 7.50 (s, 1H), 7.35 (d, *J* = 8.7 Hz, 1H), 6.58 (s, 2H), 5.38 (d, *J* = 16.0 Hz, 1H), 4.69 (d, *J* = 15.7 Hz, 1H), 3.29 (s, 3H), 2.21 (s, 3H). |
| 51 | 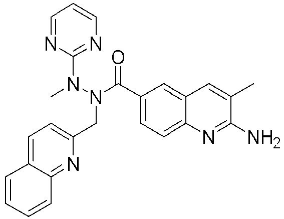 | 450.2 | |
| 52 | 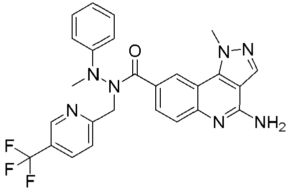 | 505.2 | ¹H NMR (400 MHz, DMSO-*d₆*) δ 8.93 (s, 1H), 8.46 (s, 1H), 8.24 - 8.18 (m, 2H), 7.83 - 7.75 (m, 2H), 7.54 (d, J = 8.8 Hz, 1H), 7.31 (t, J = 7.8 Hz, 2H), 7.19 (s, 2H), 6.95 (d, J = 8.0 Hz, 2H), 6.89 (t, J = 7.2 Hz, 1H), 5.27 (d, J = 16.0 Hz, 1H), 4.60 (d, J = 16.0 Hz, 1H), 3.89 (s, 3H), 3.08 (s, 3H). |
| 53 | 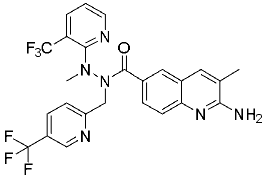 | 534.2 | |
| 54 | 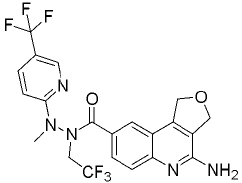 | 486.2 | |
| 55 | 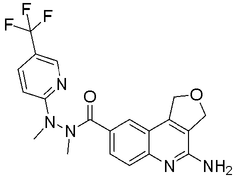 | 418.2 | |
| 56 | 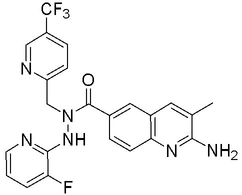 | 471.2 | |
| 57 | 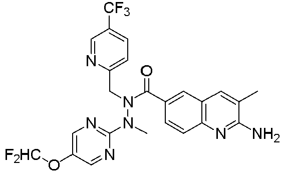 | 534.2 | |
| 58 | 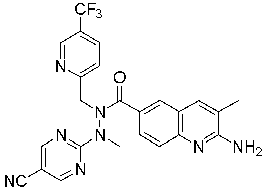 | 493.2 | |
| 59 | 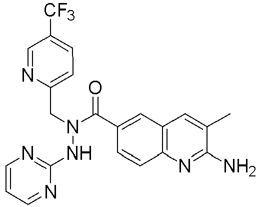 | 454.2 | |
| 60 | 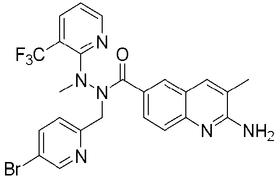 | 545.2 | |
| 61 | 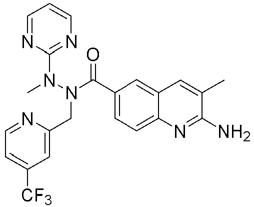 | 468.2 | |
| 62 | 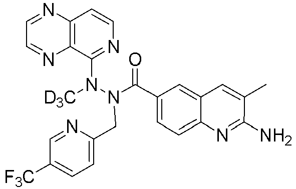 | 522.2 | |
| 63 | 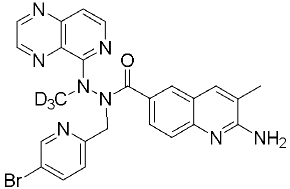 | 532.2 | |
| 64 | 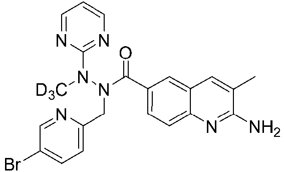 | 481.2 | |
| 65 | 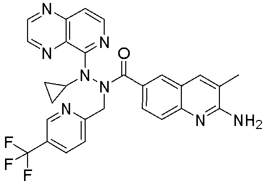 | 545.2 | |
| 66 | 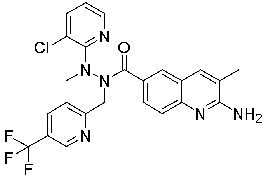 | 501.2 | |
| 67 | 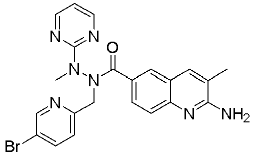 | 478.2 | |
| 68 | 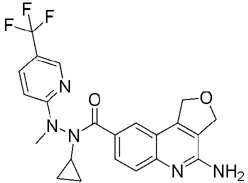 | 444.2 | |
| 69 | 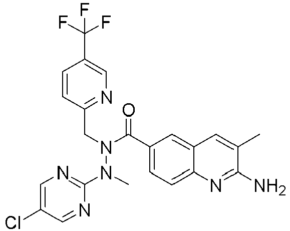 | 502.2 | |
| 70 | 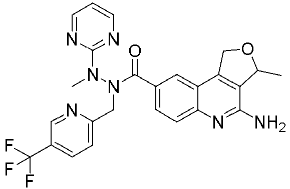 | 510.2 | |
| 71 | 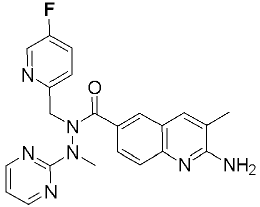 | 418.2 | |
| 72 | 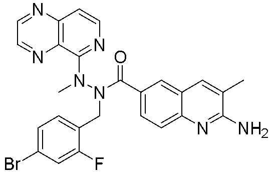 | 546.2 | ¹H NMR (400 MHz, DMSO-*d*₆) δ 8.96 (s, 1H), 8.82 (s, 1H), 8.49 (s, 1H), 8.24 (d, *J* = 5.7 Hz, 1H), 7.80 (s, 1H), 7.64 (s, 1H), 7.54 (d, *J* = 8.8 Hz, 1H), 7.40 - 7.20 (m, 4H), 6.40 (s, 2H), 5.19 (d, *J* = 15.2 Hz, 1H), 4.62 (d, *J* = 15.2 Hz, 1H), 3.45 (s, 3H), 2.14 (s, 3H). |
| 73 | 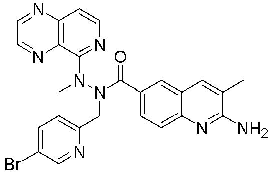 | 529.2 | |
| 74 | 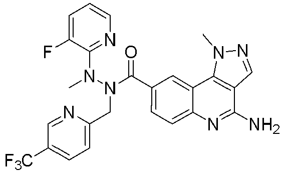 | 525.2 | ¹H NMR (400 MHz, DMSO-*d*₆)δ 8.88 (s, 1H), 8.45 (d, *J=* 13.6 Hz, 2H), 8.24 (s, 1H), 8.19 (dd, *J*= 8.4, 2.4 Hz, 1H), 8.03 (d, *J* = 4.8 Hz, 1H), 7.73 (d, *J =* 8.8 Hz, 1H), 7.58 - 7.44 (m, 2H), 7.18 (s, 2H), 6.95-6.86 (m, 1H), 5.31 (d, *J =* 15.6 Hz, 1H), 4.76 (d, *J* = 16.0 Hz, 1H), 4.20 (s, 3H), 3.34 (s, 3H). |
| 75 | 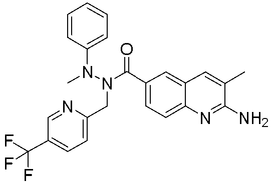 | 466.6 | ¹H NMR (400 MHz, DMSO-*d*₆) δ 8.95 (s, 1H), 8.21 (d, *J* = 8.2 Hz, 1H), 7.93 (s, 1H), 7.79 - 7.73 (m, 2H), 7.66 (d, *J* = 8.6 Hz, 1H), 7.37 (d, *J* = 8.9 Hz, 1H), 7.30-7.24 (m, 2H), 6.82-6.85 (m, 3H), 6.56 (s, 2H), 5.30 (d, *J* = 15.9 Hz, 1H), 4.49 (d, *J* = 15.8 Hz, 1H), 3.14 (s, 3H), 2.20 (s, 3H). |
| 76 | 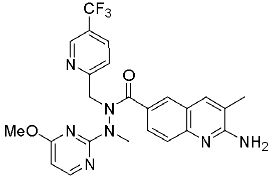 | 498.4 | ¹H NMR (400 MHz, DMSO-*d*₆) δ 8.88 (s, 1H), 8.25-8.15 (m, 1H), 8.06 (s, 1H), 7.85 (d, *J* = 8.3 Hz, 1H), 7.78 (s, 1H), 7.73 (s, 1H), 7.52 (d, *J* = 8.7 Hz, 1H), 7.34 (d, *J* = 8.7 Hz, 1H), 6.52 (s, 2H), 6.17 (d, *J=* 5.6 Hz, 1H), 5.38 (d, *J* =15.6 Hz, 1H), 4.62 (d, *J* = 15.6 Hz, 1H), 3.81 (s, 3H), 3.30 (s, 3H), 2.18 (s, 3H). |
| 77 | 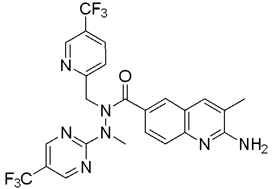 | 536.5 | ¹H NMR (400 MHz, DMSO-*d*₆) δ 8.88 (s, 1H), 8.76 (s, 1H), 8.25 - 8.20 (m, 2H), 7.88 (d, *J* = 7.7 Hz, 1H), 7.80 - 7.72 (m, 2H), 7.49 (s, 1H), 7.35 (s, 1H), 6.52 (s, 2H), 5.36 (d, *J* = 15.6 Hz, 1H), 4.82 (d, *J* = 15.5 Hz, 1H), 3.40 (s, 3H), 2.20 (s, 3H). |
| 78 | 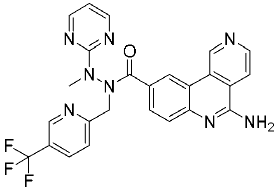 | 505.5 | ¹H NMR (400 MHz, DMSO-*d*₆) δ 9.84 (s, 1H), 8.92 (s, 1H), 8.82 (d, *J* = 5.5 Hz, 1H), 8.69 (s, 1H), 8.43 (s, 2H), 8.20-8.24 (m, 2H), 7.96 (d, *J* = 8.3 Hz, 1H), 7.61 (d, *J* = 8.0 Hz, 1H), 7.50 (s, 2H), 7.44 (d, *J* = 8.3 Hz, 1H), 6.81 (s, 1H), 5.44 (d, *J* = 15.7 Hz, 1H), 4.67 (d, *J= 15.9* Hz, 1H), 3.30 (s, 3H). |
| 79 | 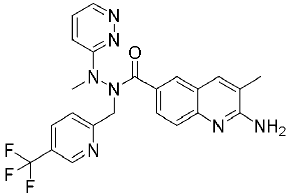 | 468.6 | |
| 80 | 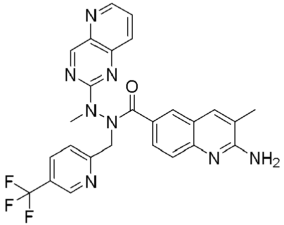 | 519.6 | ¹H NMR (400 MHz, DMSO-*d*₆) δ 9.30 (s, 1H), 8.86 (s, 1H), 8.77 (s, 1H), 8.23 (d, *J* = 8.0 Hz, 2H), 8.05-7.90 (m, 2H), 7.69 (s, 1H), 7.53 (d, *J* = 8.7 Hz, 1H),7.28 (d, *J* = 8.7 Hz, 1H), 6.49 (s, 2H), 5.40 (d, *J* = 16.1 Hz, 1H), 4.85 (s, 1H), 3.35(s, 3H), 2.15 (s, 3H). |
| 81 | 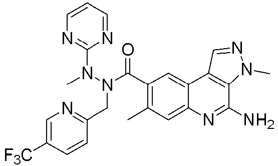 | 522.6 | ¹H NMR (400 MHz, DMSO-*d*₆) δ 8.97 (s, 1H), 8.47 (s, 2H), 8.28 (d, *J* = 8.2 Hz, 1H), 8.15 (s, 1H), 7.91 (d, *J* = 8.2 Hz, 1H), 7.83 (s, 1H), 7.33 (s, 1H), 6.85 (s, 1H), 6.77 (s, 2H), 5.54 (d, *J* = 15.6 Hz, 1H), 4.68 (d, *J* = 15.7 Hz, 1H), 4.31 (s, 3H), 3.15 (s, 3H), 2.44 (s, 3H). |
| 82 | 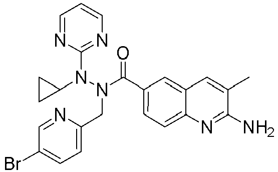 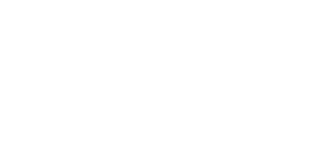 | 504.2 | ¹H NMR (400 MHz, DMSO-*d*₆) δ 8.60 (d, *J=* 4.8 Hz, 1H), 8.58 (d, *J =*2.7 Hz, 1H), 8.32 (s, 1H), 8.07 (dd, *J=* 8.4, 2.5 Hz, 1H), 7.70 - 7.64 (m, 2H), 7.60 (s, 1H), 7.40 (dd, *J* = 8.7, 2.0 Hz, 1H), 7.27 (d, *J* = 8.7 Hz, 1H), 7.04 (t, *J =* 4.8 Hz, 1H), 6.52 (s, 2H), 5.31 (d, *J =* 15.2 Hz, 1H), 4.55 - 4.49 (m, 1H), 2.75 - 2.65 (m, 1H), 2.18 (s, 3H), 0.75 - 0.65 (m, 1H), 0.53 - 0.34 (m, 2H), 0.11- 0.03 (m, 1H). |
| 83 | 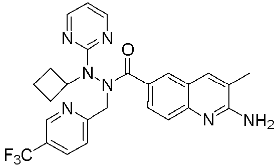 | 508.5 | ¹H NMR (400 MHz, DMSO-*d*₆) δ 8.91 (d, *J=* 2.4 Hz, 1H), 8.50 (d, *J* = 4.8 Hz, 2H), 8.24-8.26 (m, 1H), 7.96 (d, *J =* 8.3 Hz, 1H), 7.72 (d, *J* = 2.0 Hz, 1H), 7.64 (s, 1H), 7.43-7.46 (m, 1H), 7.30 (d, *J* = 8.7 Hz, 1H), 6.89-6.91 (m, 1H), 6.52 (s, 2H), 5.35 (d, *J* = 15.4 Hz, 1H), 4.65 (d, J=15.4Hz, 1H), 4.42-4.46 (m, 1H), 2.18 (s, 3H), 2.09 - 1.87 (m, 4H), 1.41-1.54 (m, 2H). |
| 84 | 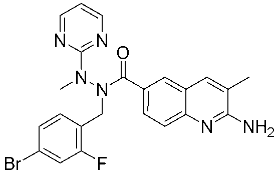 | 495.2 | ¹H NMR (400 MHz, DMSO-*d*₆) δ 8.38 (s, 2H), 7.69 (s, 1H), 7.67 (s, 1H), 7.48-7.53 (m, 2H), 7.39-7.42 (m, 2H), 7.28 (d, *J* = 8.7 Hz, 1H), 6.81 - 6.75 (m, 1H), 6.45 (s, 2H), 5.23 (d, *J* = 14.7 Hz, 1H), 4.50 (d, *J* = 14.8 Hz, 1H), 3.10 (s, 3H), 2.17 (s, 3H). |
| 85 | 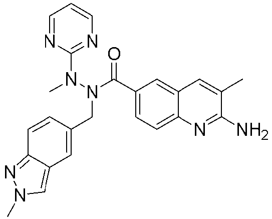 | 453.6 | ¹H NMR (400 MHz, DMSO-*d*₆) δ 8.41 (s, 1H), 8.29 (s, 1H), 8.26 (s, 1H), 7.67 (d, *J* = 10.0 Hz, 2H), 7.59 - 7.56 (m, 2H), 7.41 (d, *J* = 8.8 Hz, 1H), 7.26-7.32 (m, 2H), 6.78 (s, 1H), 6.43 (s, 2H), 5.48 (d, *J=* 14.4 Hz, 1H), 4.36 (d, *J* = 14.5 Hz, 1H), 4.14 (s, 3H), 2.92 (s, 3H), 2.16 (s, 3H). |
| 86 | 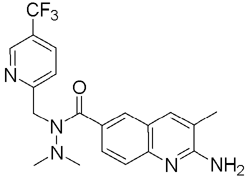 | 404.3 | ¹H NMR (400 MHz, DMSO-*d*₆) δ 9.06 (s, 1H), 8.32 - 8.24 (m, 1H), 8.05 - 7.97 (m, 1H), 7.88 - 7.86 (m, 1H), 7.86 - 7.83 (m, 1H), 7.74 (s, 1H), 7.33 (d, *J* = 8.8 Hz, 1H), 6.31 (s, 2H), 5.21 (s, 2H), 3.48 (s, 6H), 2.22 (s, 3H). |
| 87 | 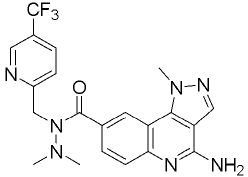 | 444.2 | ¹H NMR (400 MHz, DMSO-*d*₆) δ 9.07 (s, 1H), 8.74 - 8.65 (m, 1H), 8.32 - 8.28 (m, 2H), 7.94 - 7.91 (m, 1H), 7.91 - 7.89 (m, 1H), 7.45 (d, *J* = 8.4 Hz, 1H), 7.06 (s, 2H), 5.18 (s, 2H), 4.39 (s, 3H), 3.52 (s, 6H). |
| 88 | 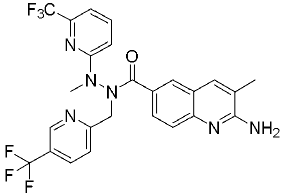 | 535.2 | ¹H NMR (400 MHz, DMSO-*d*₆) δ 8.92 (s, 1H), 8.23 (s, 1H), 8.21 - 8.16 (m, 1H), 7.86 - 7.77 (m, 2H), 7.73 - 7.67 (m, 1H), 7.59 - 7.43 (m, 1H), 7.41 - 7.28 (m, 1H), 7.18 (d, *J* = 7.2 Hz, 1H), 7.13 - 7.03 (m, 1H), 6.52 (s, 2H), 5.24 (d, *J* = 16.0 Hz, 1H), 4.99 - 4.70 (m, 1H), 3.30 (s, 3H), 2.20 (s, 3H). |
| 89 | 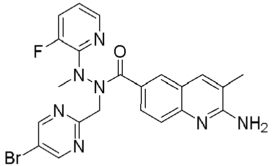 | 498.2 | |
| 90 | 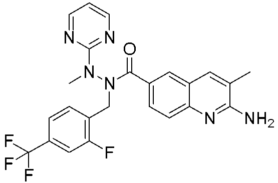 | 485.0 | ¹H NMR (400 MHz, DMSO-*d*₆) δ 8.50 (s, 1H), 8.42 - 8.38 (m, 1H), 7.89 - 7.81 (m, 1H), 7.78 -7.72 (m, 1H), 7.71 - 7.67 (m, 1H), 7.67 - 7.62 (m, 1H), 7.63 - 7.58 (m, 1H), 7.52 - 7.43 (m, 1H), 7.38 - 7.26 (m, 1H), 6.86 - 6.75 (m, 1H), 6.49 (s, 2H), 5.32 (d, *J* = 14.8 Hz, 1H), 4.64 (d, *J* = 15.2 Hz, 1H), 3.21 (s, 3H), 2.19 (s, 3H). |
| 91 | 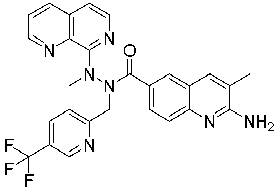 | 518.0 | |
| 92 | 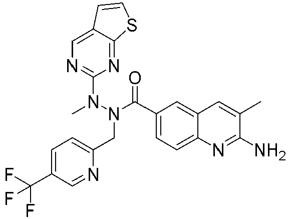 | 524.2 | ¹H NMR (400 MHz, DMSO-*d*₆) δ 8.95 - 8.90 (m, 1H), 8.90 - 8.84 (m, 1H), 8.26 - 8.19 (m, 1H), 7.96 - 7.85 (m, 1H), 7.80 (s, 1H), 7.70 (s, 1H), 7.57 - 7.50 (m, 1H), 7.50 - 7.45 (m, 1H), 7.37 - 7.30 (m, 1H), 7.30 - 7.23 (m, 1H), 6.49 (s, 2H), 5.39 (d, *J* = 15.6 Hz, 1H), 4.72 (d, *J* = 15.6 Hz, 1H), 3.33 (s, 3H), 2.20 (s, 3H). |
| 93 | 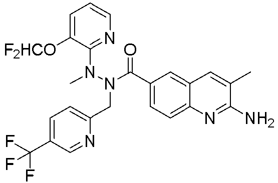 | 533.2 | ¹H NMR (400 MHz, DMSO-*d*₆) δ 8.93 (s, 1H), 8.25 - 8.19 (m, 1H), 8.07 (d, J = 4.8 Hz, 1H), 7.87 (d, J = 2.0 Hz, 1H), 7.80 (d, J = 8.4 Hz, 1H), 7.73 (s, 1H), 7.62 (dd, J = 8.8, 2.0 Hz, 1H), 7.44 (d, J =8.0 Hz, 1H), 7.36 (d, J = 8.8 Hz, 1H), 6.98 (dd, J = 8.0, 4.4 Hz, 1H), 6.93 (t, J = 73.2 Hz, 1H), 6.52 (s, 2H), 5.41 (d, J = 16.4 Hz, 1H), 4.60 (d, J = 16.4 Hz, 1H), 3.34 (s, 3H), 2.20 (s, 3H). |
| 94 | 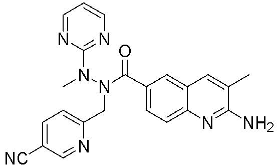 | 425.2 | ¹H NMR (400 MHz, DMSO-*d*₆) δ 8.99 (s, 1H), 8.46 - 8.36 (m, 2H), 8.34 (dd, J = 8.0, 2.0 Hz, 1H), 7.87 (d, J = 8.0 Hz, 1H), 7.79 - 7.70 (m, 2H), 7.54 - 7.47 (m, 1H), 7.33 (d, J = 8.8 Hz, 1H), 6.82 (s, 1H), 6.49 (s, 2H), 5.41 (d, J = 16.0 Hz, 1H), 4.61 (d, J = 16.0 Hz, 1H), 3.32 (s, 3H), 2.21 (s, 3H). |
| 95 | 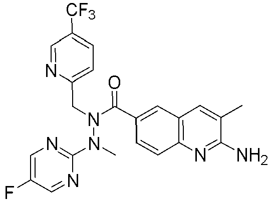 | 486.2 | |
| 96 | 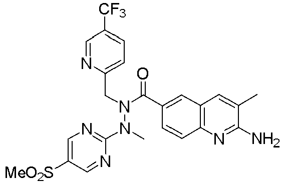 | 546.2 | |
| 97 | 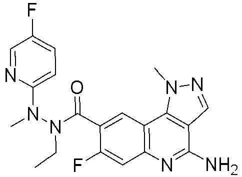 | 412.2 | |
| 98 | 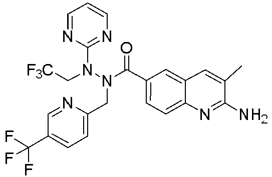 | 536.2 | |
| 99 | 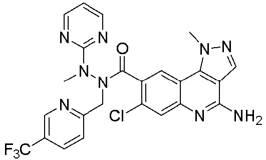 | 542.0 | |
| 100 | 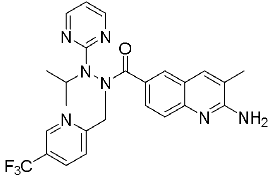 | 496.0 | |
| 101 | 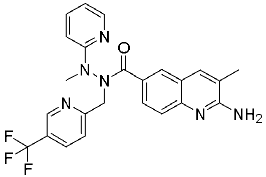 | 467.0 | |
| 102 | 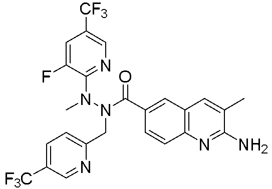 | 553.0 | ¹H NMR (400 MHz, DMSO-*d*₆) δ 8.88 (s, 1H), 8.35 (s, 1H), 8.27 (s, 1H), 8.25 - 8.16 (m, 1H), 8.07 - 7.90 (m,1H), 7.86 - 7.78 (m, 1H), 7.77 (s, 1H), 7.60 - 7.50 (m, 1H), 7.42 - 7.35 (m, 1H), 6.52 (s, 2H), 5.31 (d, J = 14.4 Hz, 1H), 4.89 (d, J = 14.4 Hz, 1H), 3.25 (s, 3H), 2.20 (s, 3H). |
| 103 | 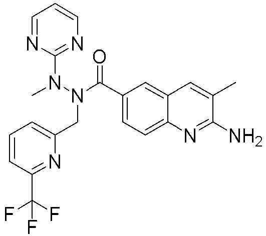 | 468.0 | |
| 104 | 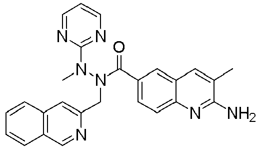 | 450.0 | ¹H NMR (400 MHz, DMSO-*d*₆) δ 9.32 (s, 1H), 8.43 (s, 2H), 8.19 - 8.12 (m, 1H), 8.01 (d, *J* = 8.2 Hz, 1H), 7.95 (s, 1H), 7.83 - 7.79 (m, 1H), 7.75 (s, 1H), 7.72 - 7.67 (m, 2H), 7.49 (d, *J* = 7.9 Hz, 1H), 7.31 (d, *J* = 8.5 Hz, 1H), 6.83 - 6.76 (m, 1H), 6.48 (s, 2H), 5.65 (d, *J* = 15.1 Hz, 1H), 4.55 (d, *J* = 15.0 Hz, 1H), 3.16 (s, 3H), 2.19 (s, 3H). |
| 105 | 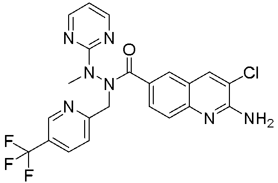 | 488.0 | |
| 106 | 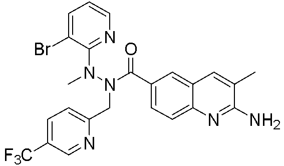 | 545.2 | ¹H NMR (400 MHz, DMSO-*d*₆) δ 8.94 (s, 1H), 8.29 - 8.18 (m, 2H), 7.94 (d, *J* = 9.6 Hz, 2H), 7.77 (d, *J* = 16.0 Hz, 2H), 7.70 - 7.66 (m, 1H), 7.38 (d, *J* = 8.8 Hz, 1H), 6.93-6.90 (m, 1H), 6.49 (s, 2H), 5.52 (d, *J=* 16.4 Hz, 1H), 4.67 (d, *J* = 16.4 Hz, 1H), 3.31 (s, 3H), 2.21 (s, 3H). |
| 107 | 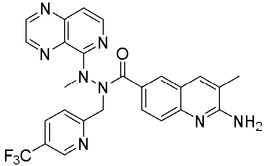 | 519.2 | ¹H NMR (400 MHz, DMSO-*d*₆) δ 8.99 (d, *J* = 2.0 Hz, 1H), 8.87-8.82 (m, 2H), 8.28 (d, *J* = 5.6 Hz, 1H), 8.19 - 8.13 (m, 1H), 7.86 - 7.77 (m, 2H), 7.68 (s, 1H), 7.61 - 7.55 (m, 1H), 7.32-7.26 (m, 2H), 6.46 (s, 2H), 5.40 (d, *J* = 15.6 Hz, 1H), 4.71 (d, *J* = 15.6 Hz, 1H), 3.67 (s, 3H), 2.16 (s, 3H). |
| 108 | 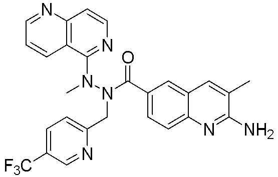 | 518.1 | ¹H NMR (400 MHz, DMSO-*d*₆) δ 8.96 (s, 1H), 8.85 (s, 1H), 8.42 - 8.25 (m, 2H), 8.16 (s, 1H), 8.00 (s, 1H), 7.81 - 7.71 (m, 3H), 7.48 (s, 1H), 7.39 (t, *J*= 8.2 Hz, 2H), 6.54 (s, 2H), 5.60 - 5.40 (m, 1H), 5.05 - 4.85 (m, 1H),3.50 (s, 3H), 2.19 (s, 3H). |
| 109 | 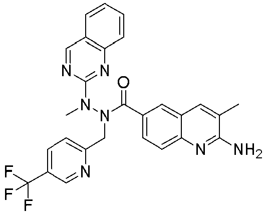 | 518.2 | |
| 110 | 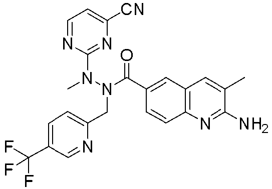 | 493.0 | |
| 111 | 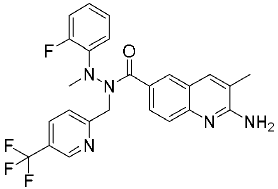 | 484.2 | |

### Example 20: Preparation of 4-amino-N',1-dimethyl-N'-(pyrimidin-2-yl)-N-(4-(trifluoromethyl)benzyl)-1H-pyrazolo[ 4,3-c]quinoline-8-carbohydrazide (20)

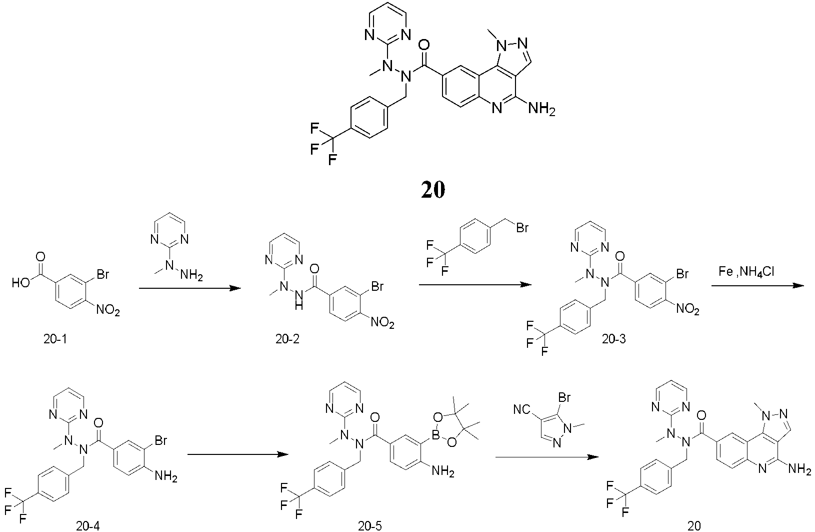

### Step 1: Preparation of 3-bromo-N'-methyl-4-nitro-N-(pyrimidin-2-yl)benzoylhydrazine (20-2)

3-Bromo-4-nitrobenzoic acid (20-1, 400 mg, 1.6 mmol) was dissolved in 6 mL of DMF at room temperature, followed by the addition of PyBroP (1.1 g, 2.4 mmol), 2-(1-methylhydrazino)pyrimidine (201 mg, 1.6 mmol) and DIEA (629 mg, 4.8 mmol). The reaction solution was stirred at room temperature overnight. The reaction mixture was poured into water (30.0 mL), stirred for 10 minutes, and then extracted with ethyl acetate (50.0 mL × 2). The organic phases were combined, washed with 5% aqueous sodium chloride solution (50.0 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 20-2 (300 mg, yield 52%).

### Step 2: Preparation of 3-bromo-N'-methyl-4-nitro-N-pyrimidin-2-yl-N-(4-trifluoromethylbenzyl)benzoylhydra zine (20-3)

Compound 20-2 (300 mg, 0.8 mmol) was dissolved in 3 mL of DMF at room temperature, followed by the addition of 1-bromomethyl-4-trifluoromethylbenzene (407 mg, 1.6 mmol) and cesium carbonate (830 mg, 2.5 mmol), and reacted at 60oC°C overnight. The reaction mixture was cooled to room temperature, poured into water (30.0 mL), stirred for 10 minutes, and then extracted with ethyl acetate (50.0 mL × 2). The organic phases were combined, washed with 5% aqueous sodium chloride solution (50.0 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 20-3 (230 mg, yield 53%).

### Step 3: Preparation of 4-amino-3-bromo-N'-methyl-N-pyrimidin-2-yl-N-(4-trifluoromethyl)benzylbenzoylhydr azine (20-4)

Compound 20-3 (230 mg, 0.4 mmol) was dissolved in 3 mL of ethanol and 3 mL of saturated ammonium chloride solution at room temperature,, followed by the addition of iron powder (109 mg, 2 mmol). oCThe reaction was carried out for 2 hours at 80°C. The reaction mixture was cooled to room temperature,and filtered under reduced pressure. The filtrate was poured into water (30.0 mL) and stirred for 10 minutes, and then extracted with ethyl acetate (50.0 mL × 2). The organic phases were combined, washed with saturated aqueous sodium chloride solution (50.0 mL), dried over anhydrous sodium sulfate and filtered. The filtrate was concentrated under reduced pressure, and the resulting residues were purified by silica gel column chromatography (dichloromethane/methanol=0-5%) to obtain Compound 20-4 (140 mg, yield 74%).

### Step 4: Preparation of 4-amino-N'-methyl-N'-(pyrimidin-2-yl)-3-(4,4,5,5-tetramethyl-1,3,2-dioxaborolan-2-yl) -N-(4-(trifluoromethyl)benzyl)benzoylhydrazine (20-5)

Compound 20-4 (140 mg, 0.3 mmol) was dissolved in 3 mL of dioxane at room temperature, followed by the addition of 4,4,4,5,5,5,5-octamethyl-2,2-bis(1,3,2-dioxaborolane) (370 mg, 1.5 mmol), potassium acetate (85 mg, 0.9 mmol) and Pd(dppf)Cl₂ (21 mg, 0.03 mmol). The reaction was carried out under nitrogen atmosphere at 90°Covernight. The reaction solution was directly used for the next step.

### Step 5: Preparation of 4-amino-N',1-dimethyl-N'-(pyrimidin-2-yl)-N-(4-(trifluoromethyl)benzyl)-1H-pyrazolo[ 4,3-c]quinoline-8-carbohydrazide (20)

0.5 mL of water was added to the reaction solution from the previous step at room temperature,, followed by the addition of 5-bromo-1-methylpyrazole-4-carbonitrile (60 mg, 0.3 mmol) and potassium carbonate (120 mg, 0.9 mmol, and the reaction was carried out at 90°C overnight under nitrogen atmosphere. The reaction solution was cooled to room temperature, and concentrated under reduced pressure, and the residues were purified by preparative high performance liquid chromatography (water/acetonitrile=95%/5% - 10%/90%) to obtain Compound **20** (1.7 mg, 0.003 mmol, yield 1%).

LC-MS (ESI+): m/z 507.2 [M+1]⁺.

### Example 21: Preparation of 2-amino-3-methyl-N-(1H-pyrrolo[2,3-b]pyridin-1-yl)-N-((5-(trifluoromethyl)pyridin-2-yl)methyl)quinoline-6-carboxamide (21)

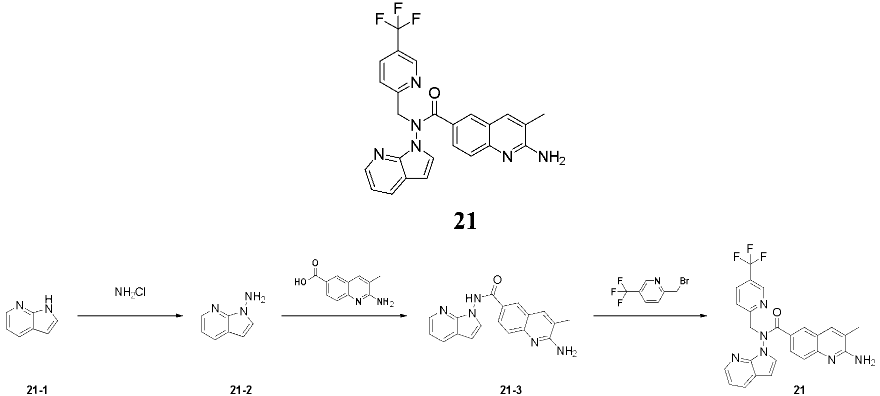

### Step 1: Preparation of 1H pyrrolo[2,3-b]pyridine-1-amine (21-2)

NH₄Cl (300 mg) was added to ether (10 mL) and cooled to -5°C in an ice salt bath, followed by the addition of ammonia water (0.5 mL). The mixture was stirred for 5 minutes, followed by the addition of NaClO aqueous solution (10%, 7.5 mL), and the resulting mixture was further stirred at 0°C for 1 hour. The reaction solution was left to separate a ether layer, which was dried over CaCl₂ to obtain a solution of NH₂Cl in ether. 1*H*-pyrrolo[2,3-*b*]pyridine (21-1, 750 mg, 6.35 mmol) was dissolved in DMF (20 mL), followed by the addition of tBuOK (750 mg, 12.70 mmol), and the resulting mixture was stirred at room temperature for 2 hours. NH₂Cl was added to the above mixture at 0°C, and the resulting mixture was stirred at room temperature overnight. Saturated Na₂SO₃ aqueous solution (20 mL) was added to the reaction solution, and the reaction solution was extracted with ether (25 mL × 3). The organic phases were combined, washed with saturated brine, dried over anhydrous sodium sulfate and filtered. The filtrate was concentrated under reduced pressure, and the residues were purified by silica gel column chromatography (DCM/EtOAc = 1/1) to obtain Compound 21-2 (500 mg, yield 59%, a yellow solid).

LC-MS (ESI+): 134.1 m/z [M+H]⁺.

### Step 2: Preparation of 2-amino-3-methyl-N-(1H-pyrrolo[2,3-b]pyridin-1-yl)quinoline-6-carboxamide (21-3)

Compound 21-2 (500 mg, 3.75 mmol) was dissolved in DMF (10 mL), followed by the addition of 2-amino-3-methylquinoline-6-carboxylic acid (prepared according to the synthetic method of Intermediate 1 in the patent WO2021163344A1 (pages 58/311)) (760 mg, 3.75 mmol) and triethylamine (1.14 g, 11.2 mmol). The resulting mixture was cooled to 0°C, followed by the addition of BOPCl (1.14 g, 4.5 mmol). The mixture was naturally warmed up to room temperature and stirred for 2 hours. The reaction solution was concentrated under reduced pressure, and the residues were purified by Prep-TLC (DCM/MeOH = 15/1) to obtain Compound 21-3 (20 mg, yield 5%, a white solid).

LC-MS (ESI+): 318.1 m/z [M+H]⁺.

### Step 3: Preparation of 2-amino-3-methyl-N-(1H-pyrrolo[2,3-b]pyridin-1-yl)-N-((5-(trifluoromethyl)pyridin-2-yl)methyl)quinoline-6-carboxamide (21)

Compound 21-3 (20 mg, 0.06 mmol) was dissolved in DMF (4 mL), followed by the addition of 2-(bromomethyl)-5-(trifluoromethyl)pyridine (15 mg, 0.06 mmol) and K₂CO₃ (26 mg, 0.18 mmol) successively, and the resulting mixture was heated to 40°C and stirred overnight. The reaction solution was cooled to room temperature, followed by the addition of H₂O (25 mL), and extracted with EtOAc (25 mL × 3). The organic phases were combined, washed with saturated brine, dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound **21** (4.2 mg, yield 13%, purity 99%, a yellow solid).

LC-MS (ESI+): 477.2 m/z [M+H]⁺.

¹H NMR (400 MHz, CHCl₃-*d*) δ 10.98 (s, 1H), 8.83 (s, 1H), 8.36 (s, 1H), 8.00 - 7.80 (m, 3H), 7.79 - 7.68 (m, 1H), 7.65 - 7.35 (m, 3H), 7.21- 7.16 (m, 2H), 6.30 (s, 1H), 6.05 (s, 1H),5.85 - 5.68 (m, 1H), 5.01 - 4.85 (m, 1H), 2.29 (s, 3H).

### Example 22: Preparation of 2-amino-N-(2,3-dihydro-1H-pyrrolo[2,3-b]pyridin-1-yl)-3-methyl-N-((5-(trifluoromethy 1)pyridin-2-yl)methyl)quinoline-6-carboxamide (22)

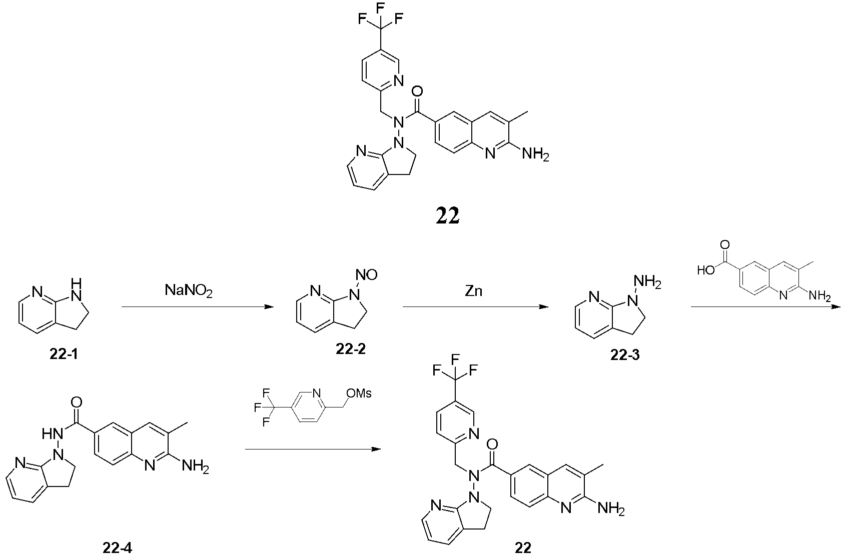

### Step 1: Preparation of 1-nitroso-2,3-dihydro-1H-pyrrolo[2,3-b]pyridine (22-2)

2,3-Dihydro-1*H*-pyrrolo[2,3-*b*]pyridine (22-1, 550 mg, 4.58 mmol) was dissolved in AcOH/H₂O (10 mL/2 mL), followed by the slow addition of a solution of NaNO₂ (230 mg, 5.04 mmol) in water (2 mL), and the resulting mixture was stirred at room temperature for 3 hours. The reaction solution was extracted with EtOAc (20 mL × 3). The organic phases were combined, washed with saturated brine, dried over anhydrous sodium sulfate and filtered. The filtrate was concentrated under reduced pressure. The residues were purified by silica gel column chromatography (DCM/EtOAc = 1/1) to obtain Compound 22-2 (560 mg, yield 82%, a colourless oil).

LC-MS (ESI+): 150.1 m/z [M+H]⁺.

### Step 2: Preparation of 2,3-dihydro-1H-pyrrolo[2,3-b]pyridine-1-amine (22-3)

Compound 22-2 (560 mg, 3.30 mmol) was dissolved in MeOH (10 mL), cooled to 0°C in an ice bath, followed by the addition of zinc powder (882 mg, 13.5 mmol) and AcOH (2 mL), and the resulting mixture was stirred at room temperature for 4 hours. Water (20 mL) was added to the reaction solution, and the reaction solution was extracted with EtOAc (25 mL × 3). The organic phases were combined, washed with saturated brine, dried over anhydrous sodium sulfate and filtered. The filtrate was concentrated under reduced pressure. The residues were purified by preparative high performance liquid chromatography (water/acetonitrile=95%/5% - 10%/90%), and subjected to lyophilization to obtain Compound 22-3 (350 mg, yield 76%, a white solid).

LC-MS (ESI+): 136.1 m/z [M+H]⁺.

### Step 3: Preparation of 2-amino-N-(2,3-dihydro-1H-pyrrolo[2,3-b]pyridin-1-yl)-3-methylquinoline-6-carboxam ide (22-4)

Compound 22-3 (350 mg, 2.59 mmol) was dissolved in DMF (4 mL), followed by the addition of 2-amino-3-methylquinoline-6-carboxylic acid (523 mg, 2.59 mmol) and triethylamine (785 mg, 7.77 mmol) successively. The resulting mixture was cooled to 0°C, followed by the addition of BOPCl (790 mg, 3.10 mmol). The mixture was naturally warmed up to room temperature and stirred for 2 hours. H₂O (25 mL) was added to the reaction solution, and the reaction solution was extracted with EtOAc (25 mL × 3). The organic phases were combined, washed with saturated brine, dried over anhydrous sodium sulfate and filtered. The filtrate was concentrated under reduced pressure. The residues were purified by preparative high performance liquid chromatography (water/acetonitrile=95%/5% - 10%/90%), and subjected to lyophilization to obtain Compound 22-4 (130 mg, yield 15%, a white solid).

LC-MS (ESI⁺): 320.2 m/z [M+H]⁺.

### Step 4: Preparation of 2-amino-N-(2,3-dihydro-1H-pyrrolo[2,3-b]pyridin-1-yl)-3-methyl-N-((5-(trifluoromethy 1)pyridin-2-yl)methyl)quinoline-6-carboxamide(22)

Compound 22-4 (50 mg, 0.15 mmol) was dissolved in DMF (4 mL), followed by the addition of (5-(trifluoromethyl)pyridin-2-yl)methyl methanesulfonate (50 mg, 0.19 mmol) and K₂CO₃ (65 mg, 0.47 mmol) successively, and the resulting mixture was heated to 40°C and stirred overnight. The reaction solution was cooled to room temperature, followed by the addition of H₂O (25 mL), and extracted with EtOAc (25 mL × 3). The organic phases were combined, washed with saturated brine, dried over anhydrous sodium sulfate and filtered. The filtrate was concentrated under reduced pressure, and the residues were purified by preparative high performance liquid chromatography (water/acetonitrile=95%/5% - 10%/90%), and subjected to lyophilization to obtain Compound **22** (28.3 mg, yield 37%, a white solid).

LC-MS (ESI+): 479.2 m/z [M+H]⁺.

¹H NMR (400 MHz, CHCl₃-*d*) δ8.84 - 8.66 (m, 1H), 8.61 (s, 1H), 8.05 - 7.98 (m, 2H), 7.96 - 7.90 (m, 1H), 7.89 - 7.84 (m, 2H), 7.73 (s, 1H), 7.63 (d, *J* = 8.9 Hz, 1H), 7.34 (d, *J* = 8.2 Hz, 1H), 7.29 (d, *J* = 7.0 Hz, 1H), 6.75 - 6.66 (m, 1H), 5.32 (d, *J=* 16.4 Hz, 1H), 4.67 (d, *J* = 15.6 Hz, 1H), 3.70 - 3.41 (m, 2H), 2.98 - 2.80 (m, 1H), 2.80 - 2.62 (m, 1H), 2.28 (s, 3H).

The following compounds were obtained according to the synthetic method in Example 22, using corresponding raw materials.

| Example | Structure | Mass spectrometry data (ESI+): m/z | Nuclear magnetic data |
|---|---|---|---|
| 112 | 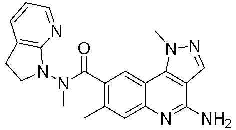 | 388.6 | |
| 113 | 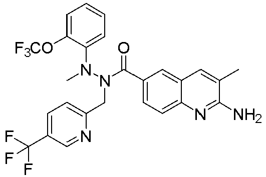 | 550.3 | ¹H NMR (400 MHz, DMSO-*d*₆) δ 9.01 (s, 1H), 8.29 - 8.24 (m, 1H), 8.14 - 8.10 (m, 1H), 7.88 - 7.85 (m, 1H), 7.81 - 7.77 (m, 1H), 7.71 (s, 1H), 7.44 - 7.39 (m, 1H), 7.26 (d, *J* = 8.0 Hz, 1H), 7.24 - 7.20 (m, 1H), 7.19 - 7.13 (m, 1H), 7.06 - 6.99 (m, 1H), 6.57 (s, 2H), 5.57 (s, 2H), 2.71 (s, 3H), 2.21 (s, 3H). |

### Example 23: Preparation of 2-amino-N'-cyclohexyl-N',3-dimethyl-N-((5-(trifluoromethyl)pyridin-2-yl)methyl)quino line-6-carbohydrazide (23)

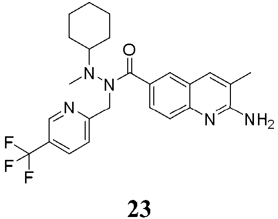

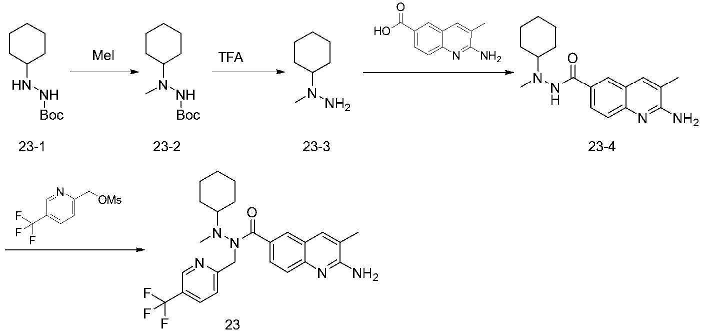

### Step 1: Preparation of tert-butyl 2-cyclohexyl-2-methylhydrazine-1-carboxylate (23-2)

Tert-butyl 2-cyclohexylhydrazine-1-carboxylate (23-1, 300 mg, 1.4 mmol) and potassium carbonate (387 mg, 2.8 mmol) were dissolved in dry DMF (3 mL) at room temperature, followed by the addition of iodomethane (0.1 mL). The reaction solution was stirred at 25°C for 4 hours. After the reaction was completed, the reaction solution was quenched with water (20 mL), and then extracted with ethyl acetate (20 mL ×3). The organic phases were combined, dried over anhydrous sodium sulfate and filtered. The filtrate was concentrated under reduced pressure. The crude product was purified by silica gel column chromatography (ethyl acetate/petroleum ether=1/1) to obtain Compound 23-2 (250 mg, yield 78%).

LC-MS (ESI+): 229.0 m/z [M+H]⁺.

### Step 2: Preparation of 1-cyclohexyl-1-methylhydrazine trifluoroacetate (23-3)

Compound 23-2 (200 mg, 0.87 mmol) was dissolved in dry dichloromethane (1 mL) at room temperature, followed by the addition of trifluoroacetic acid (1 mL). The reaction solution was stirred at 25°C for 1 hour until raw materials disappeared. The reaction solution was concentrated under reduced pressure to obtain Compound 23-3 (100 mg, yield 89%).

LC-MS (ESI+): 128.8 m/z [M+H]⁺.

### Step 3: Preparation of 2-amino-N'-cyclohexyl-N',3-dimethylquinoline-6-carbohydrazide (23-4)

Compound 23-3 (80 mg, 0.62 mmol) and 2-amino-3-methylquinoline-6-carboxylic acid (126 mg, 0.62 mmol) were dissolved in dry DMF (3 mL) at room temperature, followed by the addition of BOP reagent (552 mg, 1.25 mmol) and DIEA (0.5 mL) successively. The reaction solution was stirred at 25°C for 12 hours. The reaction solution was quenched with water (20 mL), and then extracted with ethyl acetate (20 mL ×3). The organic phases were combined, dried over anhydrous sodium sulfate and filtered. The filtrate was concentrated under reduced pressure, and the residues were purified by silica gel column chromatography (methanol/dichloromethane=1/10) to obtain Compound 23-4 (60 mg, yield 31%).

LC-MS (ESI+): 313.2 m/z [M+H]⁺.

### Step 4: Preparation of 2-amino-N'-cyclohexyl-N',3-dimethyl-N-((5-(trifluoromethyl)pyridin-2-yl)methyl)quino line-6-carbohydrazide (23)

Compound 23-4 (55 mg, 0.17 mmol) and (5-(trifluoromethyl)pyridin-2-yl)methyl methanesulfonate (135 mg, 0.53 mmol) were dissolved in dry DMF (2 mL) at room temperature, followed by the addition of potassium carbonate (73 mg, 0.53 mmol). The reaction solution was stirred at 50 °C for 12 hours. The reaction solution was quenched with water (20 mL), and then extracted with ethyl acetate (20 mL ×3). The organic phases were combined, dried over anhydrous sodium sulfate and filtered. The filtrate was concentrated under reduced pressure. The residues were purified by preparative high performance liquid chromatography (water/acetonitrile=95%/5% - 10%/90%) to obtain Compound **23** (1.4 mg, yield 1.7%).

LCMS found: LC-MS (ESI+): 472.2 m/z[M+H]⁺.

The following compound was obtained according to the synthetic method in Example 23, using corresponding raw materials.

| Example | Structure | Mass spectrometry data (ESI+): m/z | Nuclear magnetic data |
|---|---|---|---|
| 24 | 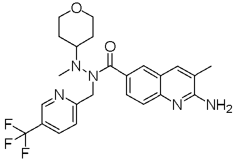 | 474.0 | |

### Example 25: Preparation of 2-amino-3-cyclopropyl-N'-methyl-N'-(pyrimidin-2-yl)-N-((5-(trifluoromethyl)pyridin-2 -yl)methyl)quinoline-6-carbohydrazide (25)

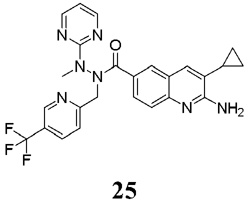

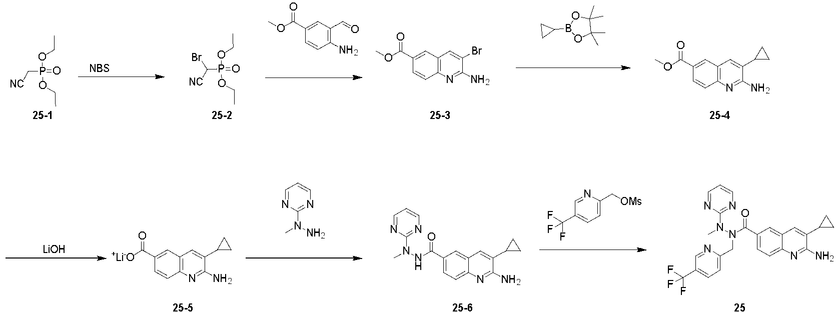

### Step 1: Preparation of diethyl (bromo(cyano)methyl)phosphate (25-2)

Diethyl cyanomethylphosphate (25-1, 4.00 g, 22.6 mmol) was dissolved in tetrahydrofuran (50.0 mL) solution at room temperature, followed by the dropwise addition of lithium bis(trimethylsilyl)amide (24.8 mmol, 2M) at 0°C, and reacted for 0.5 hour. N-bromosuccinimide (NBS) (4.42 g, 24.8 mmol) was added in batches at -78°C, and reacted at 0°C for 1 hour. The reaction was quenched by pouring into ice water (100 mL), and extracted with ethyl acetate (3×100mL). The organic phases were combined, washed with water (200 mL) five times and then washed with brine (200 mL). The organic phases were dried over anhydrous sodium sulfate and filtered. The filtrate was concentrated under reduced pressure. The resulting residues were purified by silica gel column chromatography (petroleum ether/ethyl acetate= 4/1) to obtain Compound 25-2 (4.2 g, yield 72%).

### Step 2: Preparation of methyl 2-amino-3-bromoquinoline-6-carboxylate (25-3)

Sodium hydride (1.92 g, 48.0 mmol, 60%) was dissolved in anhydrous tetrahydrofuran (20.0 mL) at room temperature. Compound 25-2 (4.1 g, 16.1 mmol) was added at 0°C under nitrogen atmosphere, and reacted for 0.5 hour. Then, methyl 4-amino-3-formylbenzoate (2.87 g, 16.1 mmol) was added, and reacted at room temperature for 4 hours. The reasction was quenched with addition of water (50 mL) at 0°C. The resulting mixture was extracted with ethyl acetate (50 mL × 3). The organic phases were combined, washed with saturated brine (60.0 mL), dried over anhydrous sodium sulfate and filtered. The filtrate was concentrated under reduced pressure. The resulting residues were purified by silica gel column chromatography (petroleum ether/ethyl acetate =1/1) to obtain Compound 25-3 (1.6 g, yield 36%).

### Step 3: Preparation of methyl 2-amino-3-cyclopropylquinoline-6-carboxylate (25-4)

Compound 25-3 (200 mg, 0.71 mmol), cyclopropylborate (144 mg, 0.85 mmol), [1,1'-bis(diphenylphosphino)ferrocene]palladium dichloride (51.7 mg, 0.07 mmol) and sodium carbonate (151 mg, 1.42 mmol) were dissolved in a solution of 1,4-dioxane (4 mL) and water (1 mL) at room temperature, and reacted at 110°C for 12 hours under nitrogen atmosphere. The resulting reaction mixture was diluted with water (15.0 mL). The resulting mixture was extracted with ethyl acetate (15.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (ethyl acetate) to obtain Compound 25-4 (90.0 mg, yield 52%).

### Step 4: Preparation of lithium 2-amino-3-cyclopropylquinoline-6-carboxylate (25-5)

Compound 25-4 (60.0 mg, 0.25 mmol) and lithium hydroxide (23.8 mg, 0.99 mmol) were dissolved in a solution of methanol (4 mL) and water (1 mL) at room temperature, and reacted at 50°C for 12 hours. The resulting reaction mixture was concentrated under reduced pressure to obtain a crude product Compound 25-5 (50.0 mg, crude yield 89%).

### Step 5: Preparation of 2-amino-3-cyclopropyl-N'-methyl-N'-(pyrimidin-2-yl)quinoline-6-carbohydrazide (25-6)

Compound 25-5 (50.0 mg, 0.22 mmol), 2-(1-methylhydrazino)pyrimidine (27.0 mg, 0.22 mmol), bromo-tris-pyrrolidino-phosphonium hexafluorophosphate (153.2 mg, 0.33 mmol) and triethylamine (44.4 mg, 0.44 mmol) were dissolved in DMF (2 mL) at room temperature, and reacted at room temperature for 12 hours. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (petroleum ether/ethyl acetate=1/1) to obtain Compound 25-6 (50.0 mg, yield 68%).

### Step 6: Preparation of 2-amino-3-cyclopropyl-N'-methyl-N'-(pyrimidin-2-yl)-N-((5-(trifluoromethyl)pyridin-2 -yl)methyl)quinoline-6-carbohydrazide (25)

Compound 25-6 (20.0 mg, 0.06 mmol), (5-(trifluoromethyl)pyridin-2-yl)methyl methanesulfonate (30.5 mg, 0.12 mmol), potassium carbonate (41.3 mg, 0.3 mmol) were dissolved in DMF (2 mL) at room temperature, and reacted at 70°C for 12 hours. The resulting reaction mixture was filtered through Celite, and the filter cake was washed with a mixed solvent of dichloromethane and methanol (dichloromethane/methanol= 20/1) (10.0 mL × 2). The filtrate was concentrated under reduced pressure, and the resulting residues were purified by silica gel column chromatography (dichloromethane/methanol=10/1) to obtain Compound **25** (10.2 mg, yield 34.5%).

LC-MS (ESI+): 494.2 m/z [M+H]⁺.

¹H NMR (400 MHz, DMSO-*d*₆) δ 8.89 (s, 1H), 8.38 (d, *J=* 4.0 Hz, 2H), 8.20 (d, *J* = 8.0 Hz, 1H), 7.84 (d, *J* = 8.0 Hz, 1H), 7.78 (s, 1H), 7.60 (s, 1H), 7.46 (d, *J* = 8.0 Hz, 1H), 7.28 (d, *J* = 8.0 Hz, 1H), 6.78 (t, *J=* 4.0 Hz, 1H), 6.58 (s, 2H), 5.40 (d, *J=* 16.0 Hz, 1H), 4.58 (d, *J=* 16.0 Hz, 1H), 3.29 (s, 3H), 1.77 (s, 1H), 0.93 (d, *J* = 8.0 Hz, 2H), 0.62 (s, 2H).

### Example 26: Preparation of 2-amino-3-isopropyl-N'-methyl-N'-(pyrimidin-2-yl)-N-((5-(trifluoromethyl)pyridin-2-yl )methyl)quinoline-6-carbohydrazide (26)

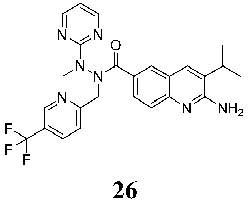

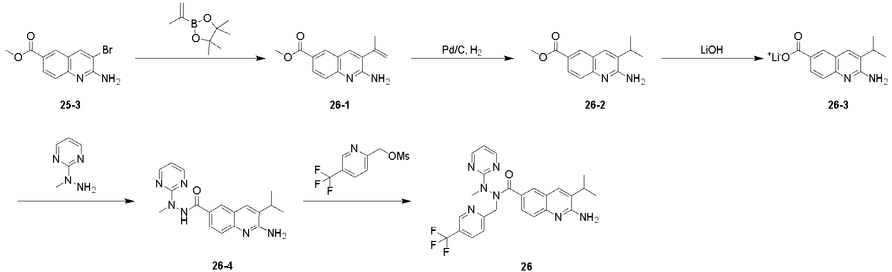

### Step 1: Preparation of methyl 2-amino-3-isopropenylquinoline-6-carboxylate (26-1)

Methyl 2-amino-3-bromoquinoline-6-carboxylate (25-3, 200 mg, 0.71 mmol), isopropenylborate (179 mg, 1.07 mmol), [1,1'-bis(diphenylphosphino)ferrocene]palladium dichloride (51.7 mg, 0.07 mmol) and sodium carbonate (151 mg, 1.42 mmol) were dissolved in 1,4-dioxane (4 mL) and water (1 mL) at room temperature, and reacted at 110°C for 12 hours under nitrogen atmosphere. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (petroleum ether/ethyl acetate =1/1) to obtain Compound 26-1 (100 mg, yield 58%).

### Step 2: Preparation of methyl 2-amino-3-isopropylquinoline-6-carboxylate (26-2)

Compound 26-1 (100 mg, 0.41 mmol), palladium on carbon (875 mg, 0.82 mmol, 10% wt%) were dissolved in ethanol (5 mL) solution at room temperature, and the reaction solution was stirred at 25°C for 2 hours under hydrogen atmosphere. The reaction solution was concentrated under reduced pressure to obtain Compound 26-2 (100 mg, yield 99%).

### Step 3: Preparation of lithium 2-amino-3-cyclopropylquinoline-6-carboxylate (26-3)

Compound 26-2 (100 mg, 0.41 mmol) and lithium hydroxide (39.3 mg, 1.64 mmol) were dissolved in methanol (4 mL) and water (1 mL) at room temperature, and reacted at 50°C for 12 hours. The resulting reaction mixture was concentrated under reduced pressure to obtain a crude product Compound 26-3 (50.0 mg, crude yield 89%).

### Step 4: Preparation of 2-amino-3-isopropyl- N'-methyl-N'-(pyrimidin-2-yl)quinoline-6-carbohydrazide (26-4)

Compound 26-3 (100 mg, 0.43 mmol), 2-(1-methylhydrazino)pyrimidine (64.7 mg, 0.52 mmol), bromo-tris-pyrrolidino-phosphonium hexafluorophosphate (304 mg, 0.65 mmol) and triethylamine (112 mg, 0.87 mmol) were dissolved in N,N-dimethylacetamide (2 mL) at room temperature, and reacted at room temperature for 12 hours. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate, filtered, and concentrated under reduced pressure. The resulting residues were purified by silica gel column chromatography (petroleum ether/ethyl acetate=1/1) to obtain Compound 26-4 (100 mg, yield 68%).

### Step 5: Preparation of 2-amino-3-isopropyl-N'-methyl-N'-(pyrimidin-2-yl)-N-((5-(trifluoromethyl)pyridin-2-yl )methyl)quinoline-6-carbohydrazide (26)

Compound 26-4 (100 mg, 0.30 mmol), (5-(trifluoromethyl)pyridin-2-yl)methyl methanesulfonate (152 mg, 0.60 mmol) and potassium carbonate (123 mg, 0.90 mmol) were dissolved in DMF (2 mL) at room temperature, and reacted at 70°C for 12 hours. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (dichloromethane/methanol=10/1) to obtain Compound **26** (11.0 mg, yield 7.5%).

LC-MS (ESI+): 496.4 m/z [M+H]⁺.

¹H NMR (400 MHz, DMSO-*d*₆) δ 8.89 (s, 1H), 8.39 (d, *J=* 4.0 Hz, 2H), 8.21 (d, *J* = 4.0 Hz, 1H), 7.86 - 7.83 (m, 2H), 7.76 (s, 1H), 7.46 (d, *J* = 8.0 Hz, 1H), 7.27 (d, *J* = 8.0 Hz, 1H), 6.79 (t, *J* = 4.0 Hz, 1H), 6.52 (s, 2H), 5.41 (d, *J* = 16.0 Hz, 1H), 4.57 (d, *J* = 16.0 Hz, 1H), 3.31 (s, 3H), 3.01 - 2.65 (m, 1H), 1.20 (d, *J=* 4.0 Hz, 6H).

### Example 27: Preparation of 2-amino-N-(3H-imidazo[4,5-b]pyridin-3-yl)-3-methyl-N-((5-(trifluoromethyl)pyridin-2-yl)methyl)quinoline-6-carboxamide (27)

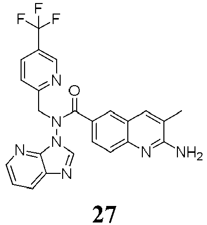

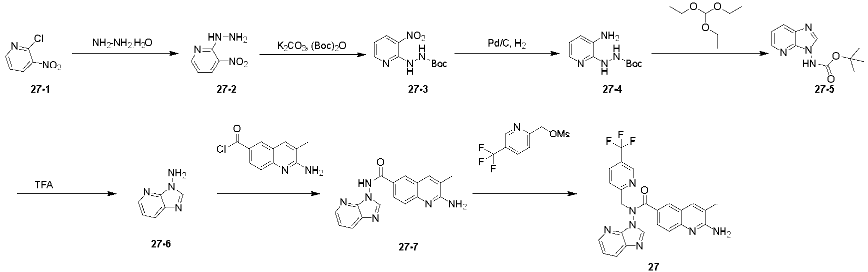

### Step 1: Preparation of 2-hydrazino-3-nitropyridine (27-2)

2-Chloro-3-nitropyridine (27-1, 1.0 g, 6.3 mmol) was dissolved in ethanol (10.0 mL) at room temperature, followed by the addition of hydrazine hydrate (742 mg, 12.6 mmol) at 0°C, and the reaction solution was reacted at 80°C for 2 hours. The reaction mixture was concentrated under reduced pressure to obtain a crude product Compound 27-2 (1.2 g, crude yield >100%).

### Step 2: Preparation of tert-butyl 2-(3-nitropyridin-2-yl)hydrazine-1-carboxylate (27-3)

Compound 27-2 (400 mg, 2.6 mmol) and potassium carbonate (1.07 g, 7.78 mmol) were dissolved in 1,4-dioxane (8 mL) at room temperature, followed by slowly dropwise addition of di-tert-butyl dicarbonate (622 mg, 2.85 mmol) at 0°C, and reacted at room temperature for 12 hours. The resulting reaction mixture was diluted with water (15.0 mL), and then extracted with ethyl acetate (20.0 mL × 2). The organic phases were combined, dried over anhydrous sodium sulfate and filtered. The filtrate was concentrated under reduced pressure to obtain Compound 27-3 (500 mg, yield 76%).

### Step 3: Preparation of tert-butyl 2-(3-aminopyridin-2-yl)hydrazine-1-carboxylate (27-4)

Compound 27-3 (500 mg, 1.97 mmol) and palladium on carbon (3.1 g, 2.95 mmol, 10% wt%) were dissolved in methanol (6 mL) at room temperature, and the reaction solution was reacted at 25°C for 2 hours under hydrogen atmosphere. The reaction solution was concentrated under reduced pressure to obtain a crude product Compound 27-4 (480 mg, crude yield >100%).

### Step 4: Preparation of tert-butyl (3H-imidazo[4,5-b]pyridin-3-yl)carbamate (27-5)

Compound 27-4 (480 mg, 2.1 mmol) was dissolved in triethoxymethane (4 mL) at room temperature, and reacted at 130°C for 3 hours. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate, and filtered. The filtrate was concentrated under reduced pressure. The resulting residues were purified by silica gel column chromatography (dichloromethane/methanol=10/1) to obtain Compound 27-5 (210 mg, yield 42%).

### Step 5: Preparation of 3H-imidazo[4,5-b]pyridin-3-amine (27-6)

Compound 27-5 (210 mg, 0.89 mmol) was dissolved in dichloromethane (2mL) and trifluoroacetic acid (0.5 mL) at room temperature, and the reaction solution was reacted at 25°C for 2 hours. The reaction solution was concentrated to dryness under reduced pressure to obtain a crude product Compound 27-6 (480 mg, crude yield 100%).

### Step 6: Preparation of 2-amino-N-(3H-imidazo[4,5-b]pyridin-3-yl)-3-methylquinoline-6-carboxamide (27-7)

Compound 27-6 (50.0 mg, 0.37 mmol), 2-amino-3-methylquinoline-6-carbonic dichloride (82.2 mg, 0.37 mmol) and triethylamine (226 mg, 2.2 mmol) were dissolved in N,N-dimethylformamide (2 mL) solution at room temperature, and reacted at room temperature for 12 hours. The resulting reaction mixture was diluted with water (20.0 mL). The resulting mixture was extracted with ethyl acetate (20.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (dichloromethane/methanol=10/1) to obtain Compound 27-7 (50.0 mg, yield 42%).

### Step 7: Preparation of 2-amino-N-(3H-imidazo[4,5-b]pyridin-3-yl)-3-methyl-N-((5-(trifluoromethyl)pyridin-2-yl)methyl)quinoline-6-carboxamide (27)

Compound 27-7 (50.0 mg, 0.16 mmol), (5-(trifluoromethyl)pyridin-2-yl)methyl methanesulfonate (60.1 mg, 0.23 mmol) and potassium carbonate (43.4 mg, 0.31 mmol) were dissolved in DMF (2 mL) at room temperature, and reacted at 50°C for 12 hours. The resulting reaction mixture was diluted with water (20.0 mL), and extracted with ethyl acetate (20.0 mL × 3). The organic phases were combined, washed with saturated brine (20.0 mL), dried over anhydrous sodium sulfate and filtered. The filtrate was concentrated under reduced pressure. The resulting residues were purified by silica gel column chromatography (dichloromethane/methanol=10/1) to obtain Compound 27 (0.5 mg, yield 0.67%).

LC-MS (ESI+): 478.2 m/z [M+H]⁺.

### Example 28: Preparation of 2-amino-N-(1H-indol-1-yl)-3-methyl-N-((5-(trifluoromethyl)pyridin-2-yl)methyl)quinol ine-6-carboxamide (28)

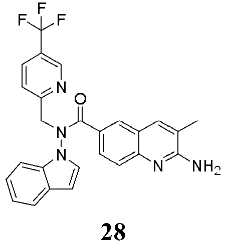

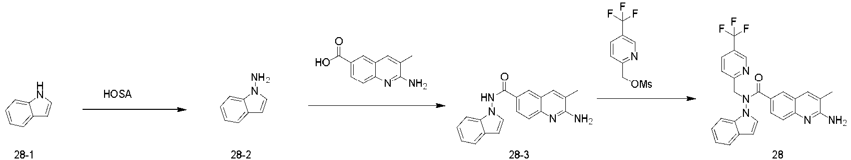

### Step 1: Preparation of 1H-indol-1-amine (28-2)

1H-indole (28-1, 2.00 g, 17 mmol) was dissolved in 40 mL of DMF at room temperature, followed by the addition of potassium hydroxide powder (9.60 g, 170 mmol), and hydroxylamine-O-sulfonic acid (3.90 g, 34 mmol) was added slowly in an ice bath, and kept below 10°C throughout the entire process. The reaction mixture was poured into water (200 mL) and stirred for 10 minutes. The reaction mixture was extracted with ethyl acetate (200 mL × 2). The organic phases were combined, washed with saturated aqueous sodium chloride solution (50.0 mL), dried over anhydrous sodium sulfate and filtered. The filtrate was concentrated under reduced pressure, and the resulting residues were purified by silica gel column chromatography (petroleum ether/dichloromethane=100%-50%) to obtain Compound 28-2 (1.00 g, yield 44%).

### Step 2: Preparation of 2-amino-N-(1H-indol-1-yl)-3-methylquinoline-6-carboxamide (28-3)

Compound 28-2 (200 mg, 1.5 mmol) was dissolved in 3mL DMF at room temperature, followed by the addition of PyBroP (297 mg, 0.6 mmol), 2-amino-3-methylquinoline-6-carboxylic acid (197 mg, 1.0 mmol) and DIEA (598 mg, 4.6 mmol). The reaction was carried out at room temperature overnight. The reaction mixture was poured into water (30.0 mL) and stirred for 10 minutes. The reaction mixture was then extracted with ethyl acetate (50.0 mL × 2). The organic phases were combined, washed with saturated aqueous sodium chloride solution (50.0 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 28-3 (320 mg, yield 67%).

### Step 3: Preparation of 2-amino-N-(1H-indol-1-yl)-3-methyl-N-((5-(trifluoromethyl)pyridin-2-yl)methyl)quinol ine-6-carboxamide (28)

Compound 28-3 (200 mg, 0.6 mmol) was dissolved in 3 mL of DMF at room temperature, followed by the addition of (5-(trifluoromethyl)pyridin-2-yl)methyl methanesulfonate (323 mg, 1.3 mmol) and potassium carbonate (610 mg, 4.4 mmol). The mixture was reacted at 60°C overnight. The reaction solution was filtered, and purified by preparative high performance liquid chromatography (water/acetonitrile=95%/5% - 10%/90%) to obtain Compound **28** (8.5 mg, 0.018 mmol, yield 3%).

LC-MS (ESI+): 476.2 m/z [M+H]⁺.

¹H NMR (400 MHz, DMSO-*d*₆) δ 8.95 (d, *J=* 2.3 Hz, 1H), 8.19 (dd, *J=* 8.3, 2.4 Hz, 1H), 7.72 (d, *J=* 7.6 Hz, 2H), 7.52 (s, 1H), 7.48 - 7.45 (m, 2H), 7.40 (d, *J* = 8.0 Hz, 2H), 7.18 -7.14 (m, 2H), 7.04 (t, *J* = 7.5 Hz, 1H), 6.51 (s, 2H), 6.36 (d, *J=* 3.4 Hz, 1H), 5.54 (d, *J=* 16.0 Hz, 1H), 5.03 (d, *J=* 15.6 Hz, 1H), 2.15 (s, 4H).

### Example 29: Preparation of 2-amino-4-cyclopropyl-N'-methyl-N'-(pyrimidin-2-yl)-N-((5-(trifluoromethyl)pyridin-2 -yl)methyl)quinoline-6-carbohydrazide (29) 5215

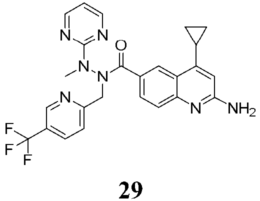

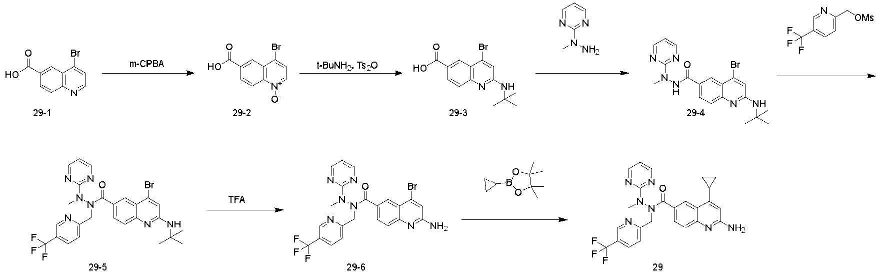

### Step 1: Preparation of 4-bromo-6-carboxylquinoline 1-oxide (29-2)

4-Bromoquinoline-6-carboxylic acid (29-1, 3.0 g, 7.9 mmol) and m-CPBA (2.7 g, 15.6 mmol) were added to dichloromethane (20.0 mL) at room temperature, and the resulting mixture was stirred at 25°C overnight. After completion of the reaction, the reaction solution was filtered, and the filter cake was dried reduced pressure to obtain a crude product Compound 29-2 (2.3 g, crude yield 75%).

### Step 2: Preparation of 4-bromo-2-tert-butylaminoquinoline-6-carboxylic acid (29-3)

The crude product Compound 29-2 (2.3 g, 8.6 mmol) was dissolved in 25 mL of dichloromethane at room temperature, followed by the addition of tert-butylamine (3.2 g, 42.9 mmol) and p-toluenesulfonic anhydride (5.6 g, 17.2 mmol) in an ice bath. The mixture was reacted at room temperature for 4 hours. The reaction mixture was poured into water (100 mL) and stirred for 10 minutes. The reaction mixture was then extracted with ethyl acetate (150 mL × 2). The organic phases were combined, washed with saturated aqueous sodium chloride solution (100 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 29-3 (750 mg, yield 27%).

### Step 3: Preparation of 4-bromo-2-(tert-butylamino)-N'-methyl-N'-(pyrimidin-2-yl)quinoline-6-carbohydrazide (29-4)

Compound 29-3 (600 mg, 1.4 mmol) was dissolved in 6 mL of DMF at room temperature, followed by the addition of PyBroP (1.60 g, 3.5 mmol), 2-(1-methylhydrazino)pyrimidine (288 mg, 2.3 mmol) and DIEA (898 mg, 6.9 mmol), and the mixture was reacted at room temperature overnight. Water (30.0 mL) was poured into the reaction mixture, and the reaction mixture was stirred for 10 minutes, and then extracted with ethyl acetate (50.0 mL × 2). The organic phases were combined, washed with sodium chloride saturated aqueous solution (50.0 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 29-4 (600 mg, yield 60%).

### Step 4: Preparation of 4-bromo-2-(tert-butylamino)-N'-methyl-N'-(pyrimidin-2-yl)-N-((5-(trifluoromethyl)pyri din-2-yl)methyl)quinoline-6-carbohydrazide (29-5)

Compound 29-4 (600 mg, 2.3 mmol) was dissolved in 7 mL of DMF at room temperature, followed by the addition of (5-(trifluoromethyl)pyridin-2-yl)methyl methanesulfonate (713 mg, 2.8 mmol) and potassium carbonate (578 mg, 4.2 mmol), and reacted at 60°C overnight. The reaction mixture was cooled to room temperature and then poured into water (30.0 mL) and stirred for 10 minutes. The mixture was then extracted with ethyl acetate (50.0 mL × 2). The organic phases were combined, washed with sodium chloride saturated aqueous solution (50.0 mL), dried over anhydrous sodium sulfate and filtered. The filtrate was concentrated under reduced pressure, and the resulting residues were purified by silica gel column chromatography (petroleum ether/ethyl acetate=100%-50%) to obtain Compound 29-5 (350 mg, yield 42 %).

### Step 5: Preparation of 2-amino-4-bromo-N'-methyl-N'-(pyrimidin-2-yl)-N-((5-(trifluoromethyl)pyridin-2-yl)m ethyl)quinoline-6-carbohydrazide (29-6)

Compound 29-5 (350 mg, 0.59 mmol) was dissolved in 4 mL of trifluoroacetic acid at room temperature, and reacted at 80°C overnight. The reaction mixture was cooled to room temperature and concentrated under reduced pressure. The residues were poured into water (30.0 mL) and stirred for 10 minutes, and then extracted with ethyl acetate (50.0 mL × 2). The organic phases were combined, washed with sodium bicarbonate saturated aqueous solution (50.0 mL), dried over anhydrous sodium sulfate and filtered. The filtrate was concentrated under reduced pressure, and the resulting residues were purified by silica gel column chromatography (dichloromethane/methanol=0-5%) to obtain Compound 29-6 (110 mg, yield 26%).

### Step 6: Preparation of 2-amino-4-cyclopropyl-N'-methyl-N'-(pyrimidin-2-yl)-N-((5-(trifluoromethyl)pyridin-2 -yl)methyl)quinoline-6-carbohydrazide (29)

Compound 29-6 (110 mg, 1.4 mmol) was dissolved in 3 mL of dioxane and 0.5 mL of water at room temperature, followed by the addition of 2-cyclopropyl-4,4,5,5-tetramethyl-1,3,2-dioxaborolane (57 mg, 0.4 mmol), potassium carbonate (70 mg, 0.5 mmol) and Pd(dppf)Cl₂ (13 mg, 0.02 mmol), and reacted at 100°C overnight. The reaction mixture was cooled to room temperature and concentrated under reduced pressure. The residues were dissolved in methanol/dichloromethane (10:1), and then filtered through Celite. The filtrate was concentrated under reduced pressure again, and the residues were purified by preparative high performance liquid chromatography (water/acetonitrile=95%/5% - 10%/90%) to obtain Compound **29** (42 mg, 0.085mmol, yield 6%).

LC-MS (ESI+): 494.2 m/z [M+H]⁺.

¹H NMR (400 MHz, DMSO-*d*₆) δ 8.92 (s, 1H), 8.46 (d, *J* = 4.7 Hz, 2H), 8.24 (dd, *J* = 8.3, 2.4 Hz, 1H), 8.16 (s, 1H), 7.90 (d, *J* = 8.1 Hz, 1H), 7.60 (d, *J* = 8.7 Hz, 1H), 7.36 (d, *J* = 8.7 Hz, 1H), 6.83 (s, 1H), 6.52 (s, 2H), 6.44 (s, 1H), 5.42 (d, *J* = 15.6 Hz, 1H), 4.68 (d, *J=* 15.6 Hz, 1H), 3.27 (s, 3H), 2.15 - 1.93 (m, 1H), 1.14 (d, *J=* 35.3 Hz, 1H), 1.02 - 0.91 (m, 2H), 0.60 - 0.52 (m, 2H).

The following compound was obtained according to the synthetic method in Example 29, using corresponding raw materials.

| Example | Structure | Mass spectrometry data (ESI+): m/z | Nuclear magnetic data |
|---|---|---|---|
| 30 | 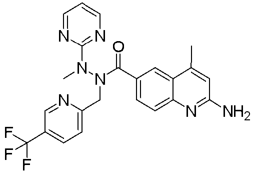 | 467.2 | ¹H NMR (400 MHz, DMSO-*d*₆) δ 8.92 (s, 1H), 8.46 (d, *J* = 4.8 Hz, 2H), 8.23 (dd, *J* = 8.3, 2.5 Hz, 1H), 8.17 (s, 1H), 7.95 - 7.82 (m, 2H), 7.57 (d, *J* = 8.7 Hz, 1H), 7.34 (d, *J* = 8.8 Hz, 1H), 6.84 (s, 1H), 6.60 (s, 1H), 6.57 (s, 1H), 5.41 (d, *J* = 15.6 Hz, 1H), 4.68 (d, *J* = 15.8 Hz, 1H), 3.26 (s, 3H), 2.31 (s, 3H). |

### Example 31: Preparation of 4-amino-7-fluoro-N,1-dimethyl-N-(5-(trifluoromethyl)-2,3-dihydro-1H-pyrrolo[2,3-b]p yridin-1-yl)-1H-pyrazolo[4,3-c]quinoline-8-carboxamide (31)

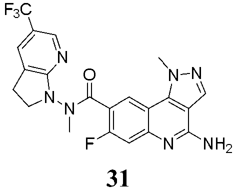

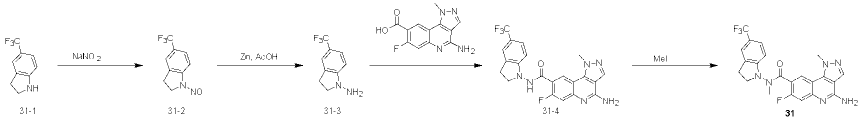

### Step 1: Preparation of 1-nitroso-5-(trifluoromethyl)indoline (31-2)

5-Trifluoromethylindoline (31-1, 400 mg, 2.10 mmol) was dissolved in 3 mL of acetic acid and 2 mL of water at room temperature, and then cooled to 0°C, followed by the addition of sodium nitrite (162 mg, 2.30 mmol). The mixture was reacted at room temperature for 3 hours. The reaction mixture was diluted by pouring into water (100 mL) to be, and then extracted with ethyl acetate (3×100 mL). The organic phases were combined, washed with water (200 mL) once, washed with brine (200 mL) once, dried over anhydrous sodium sulfate and filtered. The filtrate was concentrated under reduced pressure. The resulting residues were purified by silica gel column chromatography (petroleum ether/ethyl acetate=4/1) to obtain Compound 31-2 (496 mg, yield 87%).

### Step 2: Preparation of 5-(trifluoromethyl)indoline-1-amino (31-3)

Compound 31-2 (430 mg, 1.80 mmol) was dissolved in 3 mL of methanol at room temperature, and then cooled to 0°C, followed by the addition of zinc powder (481 mg, 7.20 mmol) and 3 mL of acetic acid. The mixture was reacted at room temperature for 4 hours, and the resulting reaction mixture was diluted with water (50 mL). The resulting mixture was extracted with ethyl acetate (50 mL × 3). The organic phases were combined, washed with saturated brine (60.0 mL), dried over anhydrous sodium sulfate and filtered. The filtrate was concentrated under reduced pressure. The resulting residues were purified by silica gel column chromatography (petroleum ether/ethyl acetate=4/1) to obtain Compound 31-3 (376 mg, yield 53%).

### Step 3: Preparation of 4-amino-7-fluoro-1-methyl-N-(5-(trifluoromethyl)indolin-1-yl)-1H-pyrazolo[4,3-c]quin oline-8-carboxamide (31-4)

Compound 31-3 (80.0 mg, 0.30 mmol) and 4-amino-7-fluoro-1-methyl-1H-pyrazolo[4,3-c]quinoline-8-carboxylic acid (60.0 mg, 0.30 mmol) were dissolved in 2 mL of DMF at room temperature, followed by the addition of bromo-tris-pyrrolidino-phosphonium hexafluorophosphate (172 mg, 0.36 mmol) and N,N-diisopropylethylamine (79.0 mg, 0.6 mmol). The reaction solution was reacted at 25°C for 12 hours, and diluted with water (40.0 mL). The resulting mixture was extracted with ethyl acetate (30.0 mL × 3). The organic phases were combined, washed with saturated brine (50.0 mL), dried over anhydrous sodium sulfate and filtered. The filtrate was concentrated under reduced pressure. The resulting residues were purified by silica gel column chromatography (dichloromethane/methanol=10/1) to obtain Compound 31-4 (30.0 mg, yield 22%).

### Step 4: Preparation of 4-amino-7-fluoro-N,1-dimethyl-N-(5-(trifluoromethyl)-2,3-dihydro-1H-pyrrolo[2,3-b]p yridin-1-yl)-1H-pyrazolo[4,3-c]quinoline-8-carboxamide (31)

Compound 31-4 (20.0 mg, 0.04 mmol) was dissolved in 2 mL of tetrahydrofuran at room temperature, followed by the addition of potassium carbonate (20.0 mg, 0.12 mmol) and iodomethane (7.60 mg, 0.05 mmol). The reaction solution was reacted at 50°C for 12 hours, and diluted with water (40.0 mL). The resulting mixture was extracted with ethyl acetate (30.0 mL × 3). The organic phases were combined, washed with saturated brine (50.0 mL), dried over anhydrous sodium sulfate and filtered. The filtrate was concentrated under reduced pressure. The resulting residues were purified by silica gel column chromatography (dichloromethane/methanol=10/1) to obtain Compound **31** (13.7 mg, yield 66%).

LC-MS (ESI+): 459.2 m/z [M+H]⁺.

¹H NMR (400 MHz, DMSO-*d*₆) δ 8.28 - 8.21 (m, 2H), 7.48 (d, *J* = 8.0 Hz, 1H), 7.39 (s, 1H), 7.23 - 7.20 (m, 3H), 6.98 (d, *J* = 8.0 Hz, 1H), 4.28 (s, 3H), 3.65 - 3.60 (m, 2H), 3.07 (s, 3H), 3.05- 2.99 (m, 2H).

### Example 32: Preparation of 4-amino-N-(5-bromo-2,3-dihydro-1H-pyrrolo[3,2-b]pyridin-1-yl)-7-fluoro-N,1-dimethy 1-1H-pyrazolo[4,3-c]quinoline-8-carboxamide (32)

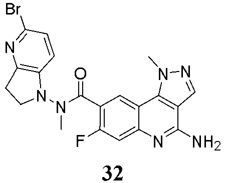

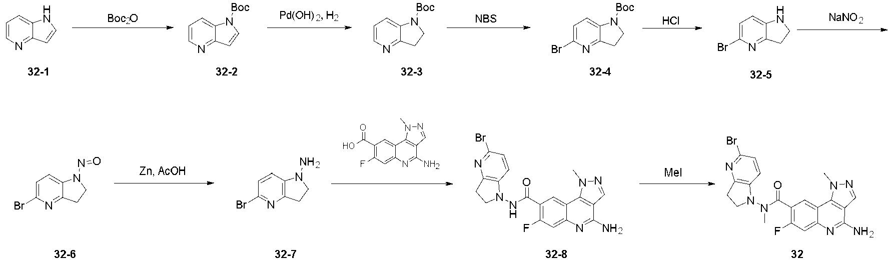

### Step 1: Preparation of tert-butyl 1H-pyrrolo[3,2-b]pyridine-1-carboxylate (32-2)

1*H*-Pyrrolo[3,2-*b*]pyridine (32-1, 4.50 g, 38 mmol) was dissolved in DCM (20 mL), followed by the addition of triethylamine (9.60 g, 95 mmol), DMAP (0.470 g, 3.8 mmol) and Boc₂O (9.90 g, 45 mmol) successively, and the resulting mixture was stirred at room temperature for 18 hours. Water (100 mL) was added to the reaction solution, and the reaction solution was extracted with DCM (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 4/1) to obtain Compound 32-2 (5.2 g, yield 99%, a white solid).

LC-MS (ESI+): 219.1 m/z [M+H]⁺.

### Step 2: Preparation of tert-butyl 2,3-dihydro-1H-pyrrolo[3,2-b]pyridine-1-carboxylate (32-3)

Compound 32-2 (0.25 g, 1.15 mmol) was dissolved in methanol (2 mL), followed by the addition of Pd(OH)₂ (0.25 g) under nitrogen atmosphere, and the resulting mixture was purged with hydrogen and then stirred at room temperature for 18 hours. The reaction solution was filtered through Celite. The filtrate was concentrated under reduced pressure, and the residues were purified by Prep-TLC (petroleum ether/ethyl acetate = 2/1) to obtain Compound 32-3 (20 mg, yield 8%, a yellow oil).

LC-MS (ESI+): 221.1 m/z [M+H]⁺.

### Step 3: Preparation of tert-butyl 5-bromo-2,3-dihydro-1H-pyrrolo[3,2-b]pyridine-1-carboxylate (32-4)

Compound 32-3 (0.98 g, 4.4 mmol) was dissolved in acetonitrile (10 mL), followed by the slow addition of NBS (1.19 g, 6.6 mmol) at 0°C, and the resulting mixture was stirred at room temperature for 18 hours. Water (20 mL) was added to the reaction solution, and the reaction solution was extracted with EtOAc (20 mL × 3). The organic phases were combined, washed with saturated brine, dried over anhydrous sodium sulfate and filtered. The filtrate was concentrated under reduced pressure, and the residues were purified by silica gel column chromatography (petroleum ether/ethyl acetate = 3/1) to obtain Compound 32-4 (0.78 g, yield 58%, a yellow solid).

LC-MS (ESI+): 299.0, 301.0 m/z [M+H]⁺.

### Step 4: Preparation of 5-bromo-2,3-dihydro-1H-pyrrolo[3,2-b]pyridine (32-5)

Compound 32-4 (0.78 g, 2.6 mmol) was dissolved in ethyl acetate (2 mL), followed by the addition of EtOAc/HCl (2 *M*, 6.5 mL, 13 mmol). The resulting mixture was stirred at room temperature for 2 hours. The reaction solution was concentrated under reduced pressure to obtain Compound 32-5 (0.55 g, yield 89%, a white solid).

LC-MS (ESI+): 199.0 m/z [M+H]⁺.

### Step 5: Preparation of 5-bromo-1-nitroso-2,3-dihydro-1H-pyrrolo[3,2-b]pyridine (32-6)

Compound 32-5 (0.1 g, 0.4 mmol) was dissolved in AcOH/H₂O (3 mL/1 mL), followed by the slow addition of NaNO₂ aqueous solution (32 mg, 0.6 mL of water), and the resulting mixture was further stirred at room temperature for 3 hours. Water (10 mL) was added to the reaction solution, and the reaction solution was extracted with EtOAc (10 mL × 3). The organic phases were combined, washed with saturated brine, dried over anhydrous sodium sulfate and filtered. The filtrate was concentrated under reduced pressure, and the residues were purified by Prep-TLC (petroleum ether/ethyl acetate = 1/1) to obtain Compound 32-6 (0.1 g, yield 99%, a white solid).

LC-MS (ESI-): 226.0, 228.0 m/z [M-H]⁻.

### Step 6: Preparation of 5-bromo-2,3-dihydro-1H-pyrrolo[3,2-b]pyridine-1-amine (32-7)

Compound 32-6 (0.100 g, 0.4 mmol) was dissolved in MeOH (1 mL), followed by the addition of zinc powder (105 mg, 1.6 mmol) and AcOH (0.5 mL), and the resulting mixture was stirred at room temperature for 4 hours. The reaction solution was filtered through Celite, and the filtrate was diluted with saturated sodium bicarbonate solution (10 mL), and extracted with EtOAc (10 mL × 3). The organic phases were combined, washed with saturated brine, dried over anhydrous sodium sulfate and filtered. The filtrate was concentrated under reduced pressure, and the residues were purified by Prep-TLC (petroleum ether/ethyl acetate = 3/1) to obtain Compound 32-7 (20 mg, yield 21%, a yellow solid).

LC-MS (ESI+): 214.0, 216.0 m/z [M+H]⁺.

### Step 7: Preparation of 4-amino-N-(5-bromo-2,3-dihydro-1H-pyrrolo[3,2-b]pyridin-1-yl)-7-fluoro-1-methyl-1H -pyrazolo[4,3-c]quinoline-8-carboxamide (32-8)

Compound 32-7 (80 mg, 0.3 mmol) was dissolved in DMF (2 mL), followed by the addition of 4-amino-7-fluoro-1-methyl-1*H*-pyrazolo[4,3-*c*]quinoline-8-carboxylic acid (97 mg, 0.3 mmol) and TEA (93 mg, 0.93 mmol) successively. The resulting mixture was cooled to 0°C, followed by the addition of BOPCl (0.11 g, 0.44 mmol). The mixture was naturally warmed up to room temperature and stirred for 18 hours. EtOAc (50 mL) was added to the reaction solution, and the reaction solution was washed with saturated brine (10 mL × 3). The organic phase was dried over anhydrous sodium sulfate and filtered, and the filtrate was concentrated under reduced pressure. The residues were purified by Prep-TLC (DCM/MeOH = 15/1) to obtain Compound 32-8 (20 mg, yield 12%, a white solid).

LC-MS (ESI+): 456.0, 458.0 m/z [M+H]⁺.

### Step 8: Preparation of 4-amino-N-(5-bromo-2,3-dihydro-1H-pyrrolo[3,2-b]pyridin-1-yl)-7-fluoro-N,1-dimethy 1-1H-pyrazolo[4,3-c]quinoline-8-carboxamide (32)

Compound 32-8 (20.0 mg, 0.04 mmol) was dissolved in DMF (2 mL) in a sealing tube, followed by the addition of K₂CO₃ (9.00 mg, 0.06 mmol) and iodomethane (5 mg, 0.03 mmol) successively, and the resulting mixed solution was stirred at normal temperature for 18 hours. EtOAc (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound **32** (10 mg, yield 50%, a white solid).

LC-MS (ESI+): 470.0, 472.0 m/z [M+H]⁺.

¹H NMR (400 MHz, CH₃OH-*d*₄) δ 8.35 (d, *J=* 7.0 Hz, 1H), 8.21 (s, 1H), 7.33 (d, *J* = 8.2 Hz, 1H), 7.26 (d, *J* = 11.4 Hz, 1H), 7.17 (d, *J* = 6.3 Hz, 1H), 4.36 (s, 3H), 3.70 - 3.59 (m, 1H), 3.52 - 3.48 (m, 1H), 3.15 (s, 3H), 3.08 - 2.95 (m, 1H), 2.88 - 2.72 (m, 1H).

The following compound was obtained according to the synthetic method in Example 32, using corresponding raw materials.

| Example | Structure | Mass spectrometry data (ESI+): m/z | Nuclear magnetic data |
|---|---|---|---|
| 114 | 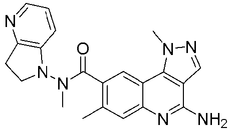 | 388.2 | |

### Example 115: Preparation of 4-amino-N-ethyl-N-methyl-N-(4-(trifluoromethyl)phenyl)-1,3-dihydrofuro[3,4-c]quinol ine-8-carbohydrazide (115)

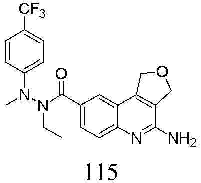

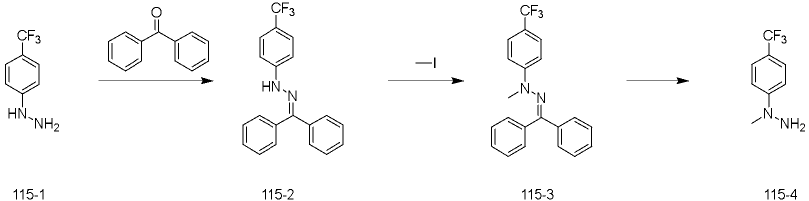

### Step 1: Preparation of (diphenylmethylene)-2-(4-(trifluoromethyl)phenyl)hydrazine (115-2)

4-(Trifluoromethyl)phenylhydrazine (1.0 g, 5.68 mmol), benzophenone (1.03 g, 5.68 mmol) and acetic acid (974 µL, 17.0 mmol) were dissolved in ethanol (30 mL) at room temperature,. The resulting mixture was heated to 80°C and further reacted for 4 hours. The reaction was cooled to room temperature. The reaction mixture was poured into water, and extracted with ethyl acetate (50 mLx3). The organic phases were combined, washed with brine (50 mL) once, dried over anhydrous sodium sulfate and filtered to collect a filtrate. The filtrate was concentrated under reduced pressure, and purified by silica gel chromatography (eluent: PE/EA= 10/1) to obtain Compound 115-2 (1.2 g, yield 62%). LC-MS (ESI+): 341.1 m/z [M+H]⁺.

### Step 2: Preparation of 2-(diphenylmethylene)-1-methyl-1-(4-(trifluoromethyl)phenyl)hydrazine (115-3)

Compound 115-2 (1.0 g, 2.94 mmol) was dissolved in N,N-dimethylformamide (20 mL) at room temperature, and cooled to 0°C in an ice water bath, followed by the slow addition of sodium hydride (60%, 141 mg, 3.52 mmol). The resulting mixture was further kept at 0°C and reacted for 15 minutes, followed by the slow addition of iodomethane (220 µL, 3.52 mmol). The mixture was warmed up to room temperature and further reacted for 16 hours. The reaction mixture was poured into ice water, and extracted with ethyl acetate (80 mLx3). The organic phases were combined, washed with saturated brine, dried over anhydrous sodium sulfate and filtered to collect a filtrate. The filtrate was concentrated under reduced pressure, and purified by silica gel chromatography (eluent: PE/EA= 5/1) to obtain Compound 115-3 (800 mg, yield 77%). LC-MS (ESI+): 355.2 m/z [M+H]⁺.

### Step 3: Preparation of 1-methyl-1-(4-(trifluoromethyl)phenyl)hydrazine (115-4)

Compound X-3 (700 mg, 1.98 mmol) was dissolved in a mixed solution of 10 mL of concentrated hydrochloric acid/1 mL of ethanol at room temperature, and reacted under stirring at room temperature for 16 hours. The reaction solution was concentrated under reduced pressure to obtain a solid, which was washed with petroleum ether (10 mLx3) to obtain Compound 115-4 (300 mg, yield 80%). LC-MS (ESI+): 191.1 m/z [M+H]⁺.

4-Amino-N-ethyl-N-methyl-N-(4-(trifluoromethyl)phenyl)-1,3-dihydrofuro[3,4-c] quinoline-8-carbohydrazide was obtained from Compound 115-4 according to the synthetic route in Example 1.

LC-MS (ESI+): 431.0 m/z [M+H]⁺.

The following compound was obtained according to the synthetic method in Example 115, using corresponding raw materials.

| Example | Structure | Mass spectrometry data (ESI+): m/z | Nuclear magnetic data |
|---|---|---|---|
| 116 | 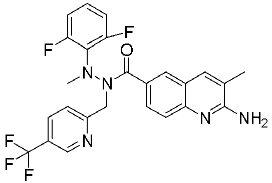 | 502.0 | |

### Example 117: Preparation of 2-amino-N'-(3-(difluoromethyl)pyridin-2-yl)-N',3-dimethyl-N-((5-(trifluoromethyl)pyri din-2-yl)methyl)quinoline-6-carbohydrazide (117)

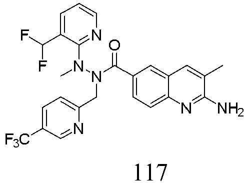

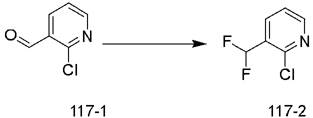

### Step 1: Preparation of 2-chloro-3-(difluoromethyl)pyridine (117-2)

Diethylaminosulphur trifluoride (2.27 g, 14.1 mmol) was added to 2-chloronicotinic acid (117-1, 2 g, 14.1 mmol) dissolved in dichloromethane (15 mL) at 0°C. The reaction solution was stirred at 25°C for 12 hours. After the reaction was completed, the reaction solution was quenched with sodium bicarbonate saturated aqueous solution (45 mL), and then extracted with ethyl acetate (60 mL x3). The organic phases were combined, washed with saturated brine (45 mL), dried over anhydrous sodium sulfate and filtered, and the filtrate was concentrated to dryness under reduced pressure. The crude product was purified by silica gel column chromatography (eluent: ethyl acetate/petroleum ether=1/4) to obtain Compound 117-2 (1.35 g, yield 58%).

LC-MS (ESI+): 164.1 m/z [M+H]⁺.

Remaining steps were the same as those in the synthetic method of Example 1 to give 2-amino-*N*'-(3-(difluoromethyl)pyridin-2-yl)-*N*',*3*-dimethyl-*N*-((5-(trifluoromethyl)pyri din-2-yl)methyl)quinoline-6-carbohydrazide which were the same, except that Compound 117-2 was used instead of Compound 1-3.

LC-MS (ESI+): 517.1 m/z [M+H]⁺.

### Example 118: Preparation of 2-amino-3-methyl-N-(1-methyl-2-oxo-1,2-dihydro-3H-imidazo[4,5-b]pyridin-3-yl)-N-(( 5-(trifluoromethyl)pyridin-2-yl)methyl)quinoline-6-carboxamide (118)

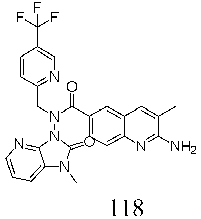

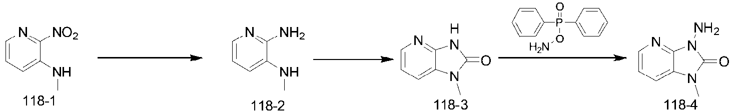

### Step 1: Preparation of N3-methylpyridine-2,3-diamine (118-2)

2-Nitro-3-methylamino-pyridine (118-1, 1.0 g, 6.53 mmol) and palladium on carbon (300 mg, 5%) were dissolved in dry methanol (15 mL) at room temperature. The reaction solution was stirred at 40°C for 12 hours under hydrogen atmosphere. The reaction solution was filtered. The filtrate was concentratedconcentrated under reduced pressure to obtain Compound 118-2 (700 mg, yield 87%).

LC-MS (ESI+): 124.1 m/z [M+H]⁺.

### Step 2: Preparation of 1-methyl-1,3-dihydro-2H-imidazo[4,5-b]pyridin-2-one (118-3)

N,N'-carbonyldiimidazole (1.18 g, 7.31 mmol) was added to compound 118-2 (600 mg, 4.88 mmol) dissolved in tetrahydrofuran (10 mL) at room temperature. The reaction solution was stirred at 25°C for 12 hours. The reaction solution was quenched with water (20 mL), and then extracted with ethyl acetate (20 mL x2). The organic phases were combined, dried over anhydrous sodium sulfate and filtered, and the filtrate was concentrated to dryness under reduced pressure. The crude product was purified by silica gel column chromatography (eluent: ethyl acetate/petroleum ether=1/1) to obtain Compound 118-3 (700 mg, yield 96.3%).

LC-MS (ESI+): 150.1 m/z [M+H]⁺.

### Step 3: Preparation of 3-amino-1-methyl-1,3-dihydro-2H-imidazo[4,5-b]pyridin-2-one (118-4)

Sodium hydride (60%, 241 mg, 6 mmol) was added to compound 118-3 (600 mg, 4..0 mmol) dissolved in tetrahydrofuran (10 mL) at 0°C. After 20 minutes, (aminooxy)diphenylphosphine oxide (1125 mg, 4.8 mmol) was added to the reaction solution, and the reaction solution was stirred at 25°C for 2 hours. The reaction solution was quenched with water (25 mL), and then extracted with ethyl acetate (25 mL x2). The organic phases were combined, washed with saturated brine (15 mL), dried over anhydrous sodium sulfate and filtered, and the filtrate was concentrated to dryness under reduced pressure. The crude product was purified by silica gel column chromatography (eluent: methanol/dichloromethane=1/10) to obtain Compound 118-4 (400 mg, yield 61%).

LC-MS (ESI+): 165.1 m/z [M+H]⁺.

Remaining steps were the same as those in the synthetic method of Example 1 to give 2-amino-3-methyl-*N*-(1-methyl-2-oxo-1,2-dihydro-3*H*-imidazo[4,5-*b*]pyridin-3-yl)-*N*-(( 5-(trifluoromethyl)pyridin-2-yl)methyl)quinoline-6-carboxamide , except that Compound 118-4 was used instead of Compound 1-3.

LC-MS (ESI+): 508.0 m/z [M+H]⁺.

### Example 119: Preparation of 2-amino-N',3-dimethyl-N'-(7H-pyrrolo[2,3-d]pyrimidin-2-yl)-N-((5-(trifluoromethyl)py ridin-2-yl)methyl)quinoline-6-carbohydrazide (119)

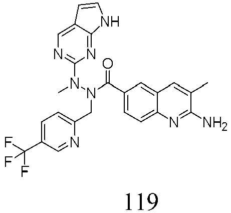

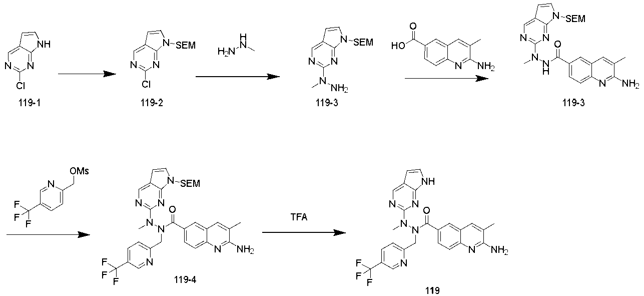

### Step 1: Preparation of 2-chloro-7-((2-(trimethylsilyl)ethoxy)methyl)-7H-pyrrolo[2,3-d]pyrimidine (119-2)

2-Chloro-7H-pyrrolo[2,3-d]pyrimidine (119-1, 1.0 g, 6.51 mmol) was dissolved in tetrahydrofuran (10 mL), followed by the addition of sodium hydride (60%, 390 mg, 9.77 mmol) at 0°C, and further stirred for 30 minutes. 2-(Trimethylsilyl)ethoxymethyl chloride (2.18 g, 13.0 mmol) was added to the reaction solution. The reaction solution was stirred at 25°C for 2 hours. The reaction solution was quenched with water (50 mL), and then extracted with ethyl acetate (40 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/2) to obtain Compound 119-2 (800 mg, yield 43%).

LC-MS (ESI+): 284.2 m/z [M+H]⁺.

### Step 2: Preparation of 2-(1-methylhydrazino)-7-((2-(trimethylsilyl)ethoxy)methyl)-7H-pyrrolo[2,3-d]pyrimidi ne (119-3)

Compound 119-2 (200 mg, 0.71 mmol), methylhydrazine (206 mg, 1.41 mmol) and potassium carbonate (292 mg, 2.11 mmol) were dissolved in dioxane (4 mL) solution, and the reaction solution was stirred at 100°C for 16 hours. After the reaction was completed, the reaction solution was quenched with water (20 mL), and then extracted with ethyl acetate (20 mL x3). The organic phases were combined, dried over anhydrous sodium sulfate and filtered, and the filtrate was concentrated to dryness under reduced pressure. The resulting crude product was purified by C18 reversed-phase column chromatography (eluent: water/acetonitrile=1.5/1) to obtain Compound 119-3 (130 mg, yield 63%). LC-MS (ESI+): 294.2 m/z [M+H]⁺.

### Step 3: Preparation of 2-amino-N',3-dimethyl-N'-(7-((2-(trimethylsilyl)ethoxy)methyl)-7H-pyrrolo[2,3-d]pyri midin-2-yl)quinoline-6-carbohydrazide (119-4)

Compound 119-3 (130 mg, 0.44 mmol), 2-amino-3-methylquinoline-6-carboxylic acid (134 mg, 0.66 mmol) and N,N-diisopropylethylamine (171 mg, 1.33 mmol) were dissolved in N,N-dimethylacetamide (2 mL) solution, and the reaction solution was stirred at 25°C for 10 minutes. Then, bromo-tris-pyrrolidino-phosphonium hexafluorophosphate (268 mg, 0.58 mmol) was added, and the reaction solution was stirred at 25°C for 2 hours. After the reaction was completed, the reaction solution was quenched with water (20 mL), and then extracted with ethyl acetate (20 mL x3). The organic phases were combined, dried over anhydrous sodium sulfate and filtered, and the filtrate was concentrated to dryness under reduced pressure. The resulting crude product was purified by silica gel column chromatography (eluent: dichloromethane/methanol=10/1) to obtain Compound 119-4 (100 mg, yield 47%).

LC-MS (ESI+): 478.2 m/z [M+H]⁺.

### Step 4: Preparation of 2-amino-N',3-dimethyl-N-((5-(trifluoromethyl)pyridin-2-yl)methyl)-N'-(7-((2-(trimethy lsilyl)ethoxy)methyl (119-5)

Compound 119-4 (90 mg, 0.18 mmol) and potassium carbonate (78 mg, 0.57 mmol) were dissolved in dimethyl sulfoxide (2 mL) solution, and the reaction solution was stirred at 25°C for 1 hour. Then, (5-(trifluoromethyl)pyridin-2-yl)methyl methanesulfonate (96 mg, 0.38 mmol) was added. The reaction solution was stirred at 25°C for 16 hours. After the reaction was completed, the reaction solution was quenched with water (20 mL), and then extracted with ethyl acetate (20 mL x3). The organic phases were combined, dried over anhydrous sodium sulfate and filtered, and the filtrate was concentrated to dryness under reduced pressure. The resulting crude product was purified by C18 reversed-phase column chromatography (eluent: water/acetonitrile=4/1) to obtain Compound 119-5 (20 mg, yield 17%).

LC-MS (ESI+): 637.2 m/z [M+H]⁺.

### Step 5: Preparation of 2-amino-N',3-dimethyl-N'-(7H-pyrrolo[2,3-d]pyrimidin-2-yl)-N-((5-(trifluoromethyl)py ridin-2-yl)methyl)quinoline-6-carbohydrazide (119)

Compound 119-5 (20 mg, 0.03 mmol) was dissolved in trifluoroacetic acid (1.5 mL) at room temperature. The reaction solution was stirred at 25°C for 2 hour until raw materials disappeared. The reaction solution was concentrated to dryness under reduced pressure. The resulting crude product was diluted with tetrahydrofuran (2 mL), followed by the addition of ammonia water (2 mL) to adjust the reaction solution to pH = 9, and the reaction solution was stirred at 25°C for 16 hours. The filtrate was concentrated to dryness under reduced pressure. The residues were purified by preparative high performance liquid chromatography (water/acetonitrile=95%/5%-10%/90%), and subjected to lyophilization to obtain Compound 119 (1.3 mg, yield 8%).

LC-MS (ESI+): 507.1 m/z [M+H]⁺.

The following compound was obtained according to the synthetic method in Example 119, using corresponding raw materials.

| Example | Structure | Mass spectrometry data (ESI+): m/z | Nuclear magnetic data |
|---|---|---|---|
| 120 | 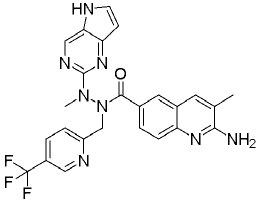 | 507.2 | |

### Example 121: Preparation of 2-amino-N'-(bicyclo[1.1.1]pent-1-yl)-N',3-dimethyl-N-((5-(trifluoromethyl)pyridin-2-yl )methyl)quinoline-6-carbohydrazide (121)

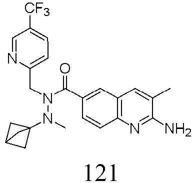

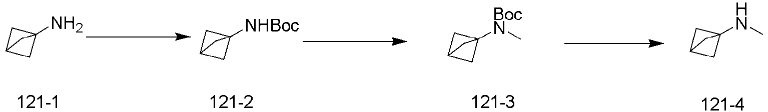

### Step 1: Preparation of tert-butyl bicyclo[1.1.l]pent-l-ylcarbamate (121-2)

Bicyclo[1.1.1]pentane-1-amine (121-1, 2.0 g, 16.8 mmol) was dissolved in THF (20 mL), followed by the addition of sodium hydride (60%, 806 mg, 33.6 mmol) and Boc₂O (4.03 g, 18.5 mmol) at 0°C, and the resulting mixture was stirred at room temperature for 24 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 20/1) to obtain Compound 121-2 (1.5 g, yield 49%, a white solid).

LC-MS (ESI+): 184.2 m/z [M+H]⁺.

### Step 2: Preparation of tert-butyl bicyclo[1.1.l]pent-l-yl (methyl)carbamate (121-3)

Compound 121-2 (1.5 g, 8.2 mmol) was dissolved in DMF (2 mL), followed by the addition of sodium hydride (60%, 393 mg, 16.3 mmol) at 0°C. The reaction solution was stirred for 5 minutes, followed by the addition of iodomethane (2.3 g, 16.4 mmol), and the resulting mixture was stirred at room temperature for 4 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure to obtain Compound 121-3 (700 mg, yield 43%, a colourless oil).

LC-MS (ESI+): 198.3 m/z [M+H]⁺.

### Step 3: Preparation of N-methylbicyclo[1.1.1]pentane-1-amine (121-4)

Compound 121-3 (1.3 g, 6.6 mmol) was dissolved in 4M HCl-ethyl acetate solution (20 mL), and the resulting mixture was stirred at room temperature for 18 hours. The reaction solution was concentrated to obtain Compound 121-4 (hydrochloride, 600 mg, yield 94%, a white solid). LC-MS (ESI+): 98.1 m/z [M+H]⁺.

Remaining steps were the same as those in the synthetic method of Example 22 to give 2-amino-N'-(bicyclo[1.1.l]pent-l-yl)-N',3-dimethyl-N-((5-(trifluoromethyl)pyridin-2-yl )methyl)quinoline-6-carbohydrazide, except that Compound 121-4 was used instead of Compound 22-3.

LC-MS (ESI+): 456.2 m/z [M+H]⁺.

### Example 122: Preparation of 2-amino-6-(2-(5-carboxylpyrimidin-2-yl)-2-methyl-1-((5-(trifluoromethyl)pyridin-2-yl) methyl)hydrazine-1-carbonyl)-3-methylquinolin-4-yl (122)

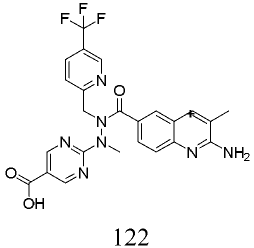

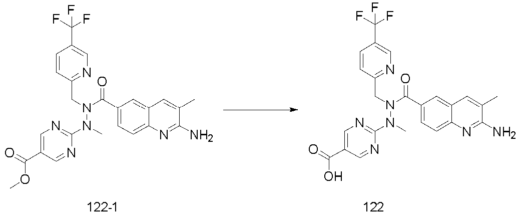

### Step 1: Preparation of 2-amino-6-(2-(5-carboxylpyrimidin-2-yl)-2-methyl-1-((5-(trifluoromethyl)pyridin-2-yl) methyl)hydrazine-1-carbonyl)-3-methylquinolin-4-yl (122)

Methyl 2-(2-(2-Amino-3-methylquinoline-6-carbonyl)-1-methyl-2-((5-(trifluoromethyl)pyridin-2-yl)methyl)hydrazino)pyrimidine-5-carboxylate (122-1, synthesized according to the method in Example 1) (100 mg, 0.19 mmol) was dissolved in THF/MeOH/H₂O (3 mL/2 mL/1 mL), followed by the addition of lithium hydroxide (23 mg, 0.57 mmol), and the resulting mixed solution was stirred at 25°C for 3 hours. The reaction solution was adjusted to pH~3 with 1N HCl, and then extracted with EtOAc (20 mL). The organic phase was washed with saturated brine (5 mL × 3), dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound **122** (37 mg, yield 38%, a white solid).

LC-MS (ESI+): 512.2 m/z [M+H]⁺.

### Example 123: Preparation of 2-amino-N-((8-fluoroisoquinolin-3-yl)methyl)-N',3-dimethyl-N'-(pyrimidin-2-yl)quinol ine-6-carbohydrazide (123)

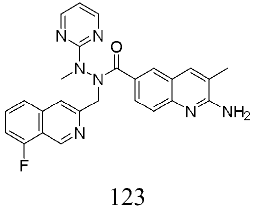

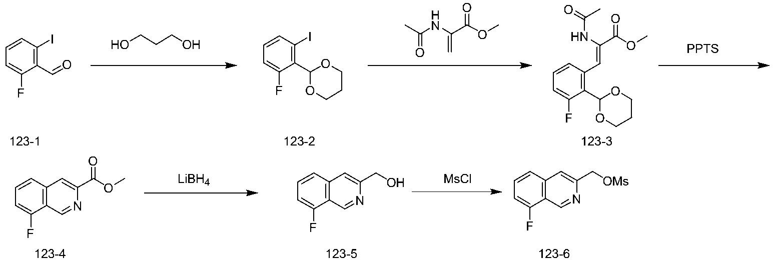

### Step 1: Preparation of 2-(2-fluoro-6-iodophenyl)-1,3-dioxane (123-2)

2-Fluoro-6-p-iodobenzaldehyde (123-1, 1.00 g, 3.99 mmol), propane-1,3-diol (456 mg, 5.99 mmol) and p-toluenesulfonic acid (69 mg, 0.40 mmol) were dissolved in toluene (10 mL) at room temperature, and reacted at 110°C for 2 hours. The resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 123-2 (1.20 g, yield 97%).

LC-MS (ESI+): 309.2 m/z [M+H]⁺.

### Step 2: Preparation of methyl (Z)-3-(2-(1,3-dioxan-2-yl)-3-fluorophenyl)-2-acetamidoacrylate (123-3)

Compound 123-2 (1.00 g, 3.25 mmol), methyl 2-acetamidoacrylate (511 mg, 3.57 mmol), sodium bicarbonate (619 mg, 8.11 mmol), palladium acetate (73.5 mg, 0.32 mmol) and 3,3-dimethylbutyryl chloride (721 mg, 2.59 mmol) were added to toluene (10 mL) at room temperature, and reacted at 90°C for 12 hours. The resulting reaction mixture was diluted with water (100 mL), and the resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 123-3 (1.00 g, yield 95%).

LC-MS (ESI+): 324.2 m/z [M+H]⁺.

### Step 3: Preparation of methyl 8-fluoroisoquinoline-3-carboxylate (123-4)

Compound 123-3 (800 mg, 2.47 mmol) and pyridinium p-toluenesulfonate (PPTS, 155 mg, 0.68 mmol) were dissolved in acetic acid (10 mL) and water (2 mL) at room temperature, and reacted at 80°C for 12 hours. The resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 123-4 (400 mg, yield 78%).

LC-MS (ESI+): 206.1 m/z [M+H]⁺.

### Step 4: Preparation of (8-fluoroisoquinolin-3-yl)methanol (123-5)

Compound 123-4 (250 mg, 1.20 mmol) was dissolved in methanol (5 mL) at room temperature, followed by the addition of sodium borohydride (53 mg, 2.43 mmol) at 0°C, and reacted at 0°C for 2 hours. The resulting reaction mixture was quenched with water (50 mL), and the resulting mixture was extracted with ethyl acetate (50 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 123-5 (230 mg, yield 100%).

LC-MS (ESI+): 178.1 m/z [M+H]⁺.

### Step 5: Preparation of (8-fluoroisoquinolin-3-yl)methyl methanesulfonate (123-6)

Compound 123-5 (230 mg, 1.29 mmol), methylsulfonyl chloride (178 mg, 1.56 mmol) and triethylamine (159 mg, 1.56 mmol) were dissolved in dichloromethane (4 mL) at room temperature, and reacted at room temperature for 12 hours. The resulting reaction mixture was diluted with water (60 mL). The resulting mixture was extracted with ethyl acetate (60 mL x 3). The organic phases were combined, washed with saturated brine (120 mL), dried over anhydrous sodium sulfate, and filtered. The filtrate was concentrated under reduced pressure. The resulting residues were purified by silica gel column chromatography (eluent: dichloromethane/methanol=10/1) to obtain Compound 123-6 (210 mg, yield 63%).

LC-MS (ESI+): 256.0 m/z [M+H]⁺.

Remaining steps were the same as those in the synthetic method of Example 1 to give 2-amino-N-((8-fluoroisoquinolin-3-yl)methyl)-N',3-dimethyl-N'-(pyrimidin-2-yl)quinol ine-6-carbohydrazide, except that Compound 123-6 was used instead of Compound 1-2.

LC-MS (ESI+): 468.2 m/z [M+H]⁺.

¹H NMR (400 MHz, DMSO-*d*₆) δ 9.44 (s, 1H), 8.40 (s, 2H), 7.87 (d, *J* = 8.3 Hz, 1H), 7.83 - 7.76 (m, 2H), 7.73 (s, 1H), 7.69 (s, 1H), 7.50 - 7.45 (m, 2H), 7.29 (d, *J* = 8.4 Hz, 1H), 6.78 (s, 1H), 6.46 (s, 2H), 5.61 (d, *J=* 15.7 Hz, 1H), 4.57 (d, *J=* 15.1 Hz, 1H), 3.19 (s, 3H), 2.16 (s, 3H).

### Example 124: Preparation of 2-amino-N',3-dimethyl-N'-(pyrimidin-2-yl)-N-((6-(trifluoromethyl)pyridazin-3-yl)meth yl)quinoline-6-carbohydrazide (124)

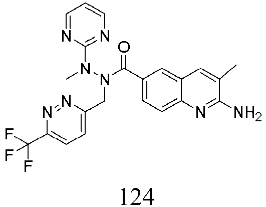

2-Amino-N',3-dimethyl-N'-(pyrimidin-2-yl)-N-((6-(trifluoromethyl)pyridazin-3-yl )methyl)quinoline-6-carbohydrazide was obtained according to the synthetic method of Example 123, except that methyl 6-(trifluoromethyl)pyridazine-3-carboxylate was used instead of methyl 8-fluoroisoquinoline-3-carboxylate (123-4).

LC-MS (ESI+): 469.2 m/z [M+H]⁺.

### Example 125: Preparation of 2-amino-N'-(6-fluoroquinoxalin-5-yl)-N',3-dimethyl-N-((5-(trifluoromethyl)pyridin-2-y l)methyl)quinoline-6-carbohydrazide (125)

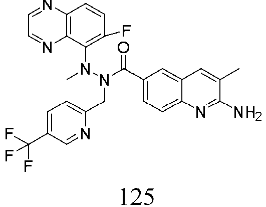

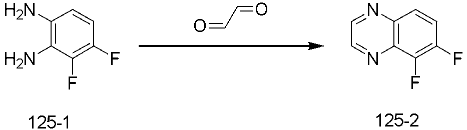

### Step 1: Preparation of 5,6-difluoroquinoxaline (125-2)

3,4-Difluorobenzene-1,2-diamino (125-1, 2.00 g, 13.9 mmol) and oxaldehyde (4.0 mg, 27.8 mmol) were dissolved in ethanol (10 mL) at room temperature, and reacted at 80°C for 12 hours. The resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 125-2 (2.00 g, yield 86%).

### LC-MS (ESI+): 167.0 m/z [M+H]⁺.

Remaining steps were the same as those in the synthetic method of Example 1 to give 2-amino-N'-(6-fluoroquinoxalin-5-yl)-N',3-dimethyl-N-((5-(trifluoromethyl)pyridin-2-y l)methyl)quinoline-6-carbohydrazide, except that 125-2 was used instead of Compound 1-3.

LC-MS (ESI+): 536.2 m/z [M+H]⁺.

### Example 126: Preparation of 2-amino-N',3-dimethyl-N'-(7-methyl-7H-pyrrolo[2,3-d]pyrimidin-2-yl)-N-((5-(trifluoro methyl)pyridin-2-yl)methyl)quinoline-6-carbohydrazide (126)

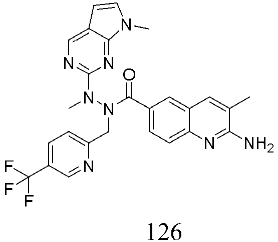

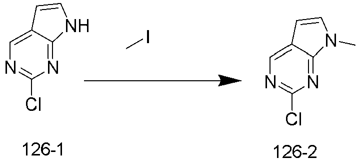

### Step 1: Preparation of 6-bromo-3-trifluoromethylquinoline (126-2)

2-Chloro-7H-pyrrolo[2,3-d]pyrimidine (126-1, 500 mg, 3.26 mmol) was dissolved in acetonitrile (6 mL) at room temperature, and sodium hydride (60%, 160 mg, 3.91 mmol) was added to the reaction solution at 0°C. The reaction solution was stirred at 25°C for 1 hour. Then, iodomethane (1020 mg, 7.16 mmol) was added to the reaction solution. The reaction solution was stirred at 25°C for 1 hours. After the reaction was completed, the reaction solution was quenched with water (30 mL), and then extracted with ethyl acetate (30 mL x3). The organic phases were combined, dried over anhydrous sodium sulfate and filtered, and the filtrate was concentrated to dryness under reduced pressure. The crude product was purified by silica gel column chromatography (eluent: ethyl acetate/petroleum ether=1/4) to obtain Compound 126-2 (300 mg, yield 55%).

LC-MS (ESI+): 168.2 m/z [M+H]⁺.

Remaining steps were the same as those in the synthetic method of Example 1 to give 2-amino-N',3-dimethyl-N'-(7-methyl-7H-pyrrolo[2,3-d]pyrimidin-2-yl)-N-((5-(trifluoro methyl)pyridin-2-yl)methyl)quinoline-6-carbohydrazide, except that 126-1 was used instead of 1-3.

LC-MS (ESI+): 521.2 m/z [M+H]⁺.

¹H NMR (400 MHz, DMSO-*d*₆) δ 8.86 (s, 1H), 8.61 (s, 1H), 8.25 - 8.19 (m, 1H), 7.93 (d, *J* = 8.0 Hz, 1H), 7.83 (s, 1H), 7.71 (s, 1H), 7.57 (d, *J=* 8.6 Hz, 1H), 7.30 (d, *J* = 8.4 Hz, 1H), 7.26 - 7.21(m, 1H), 6.46 (s, 2H), 6.42 - 6.36 (m, 1H), 5.36 (d, *J=* 15.6 Hz, 1H), 4.66 (d, *J* = 15.6 Hz, 1H), 3.63 (s, 3H), 3.44 (s, 3H), 2.18 (s, 3H).

The following compound was obtained according to the synthetic method in Example 126, using corresponding raw materials.

| Example | Structure | Mass spectrometry data (ESI+): m/z | Nuclear magnetic data |
|---|---|---|---|
| 127 | 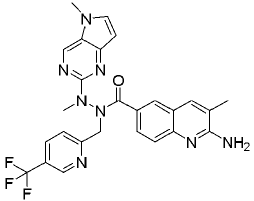 | 521.2 | ¹H NMR (400 MHz, DMSO-*d*₆) δ 8.90 (s, 1H), 8.73 (s, 1H), 8.22 (d, *J* = 8.4 Hz, 1H), 7.91 (d, *J* = 8.4 Hz, 1H), 7.84 (d, *J* = 2.0 Hz, 1H), 7.70 - 7.67 (m, 2H), 7.57 (d, *J=* 8.8 Hz, 1H), 7.29 (d, *J* = 8.8 Hz, 1H), 6.46 (s, 2H), 6.30 (d, *J* = 3.2 Hz, 1H), 5.42 (d, *J* = 15.6 Hz, 1H), 4.51 (d, *J* = 15.6 Hz, 1H), 3.81 (s, 3H), 3.30 (s, 3H), 2.18 (s, 3H). |

### Example 128: Preparation of 2-amino-N'-ethyl-N',3-dimethyl-N-((5-(trifluoromethyl)pyridin-2-yl)methyl)quinoline-6 -carbohydrazide (128)

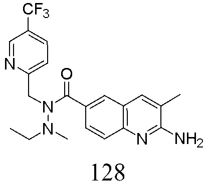

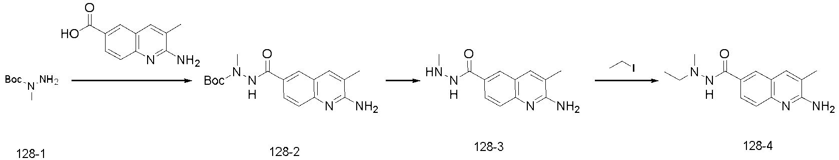

### Step 1: Preparation of tert-butyl 2-(2-amino-3-methylquinoline-6-carbonyl)-1-methylhydrazine-carboxylate (128-2)

2-Amino-3-methylquinoline-6-carboxylic acid (500 mg, 2.47 mmol) and tert-butyl 1-methylhydrazine-carboxylate (128-1, 542 mg, 3.7 mmol) were dissolved in N,N-dimethylformamide (10 mL) at room temperature,, followed by the addition of bromo-tris-pyrrolidino-phosphonium hexafluorophosphate (1729 mg, 3.7 mmol) and diisopropylethylamine (1 mL) successively. The reaction solution was stirred at 25°C for 12 hours. After the reaction was completed, the reaction solution was quenched with water (20 mL), and then extracted with ethyl acetate (20 mL x3). The organic phases were combined, dried over anhydrous sodium sulfate and filtered, and the filtrate was concentrated to dryness under reduced pressure. The crude product was purified by silica gel column chromatography (eluent: methanol/dichloromethane=10/1) to obtain Compound 128-2 (800 mg, yield 98%).

LC-MS (ESI+): 331.2 m/z [M+H]⁺.

### Step 2: Preparation of 2-amino-N',3-dimethylquinoline-6-carbohydrazide (128-3)

Compound 128-2 (300 mg, 0.91 mmol) was dissolved in dry dichloromethane (2 mL) at room temperature, followed by the addition of trifluoroacetic acid (0.4 mL). The reaction solution was stirred at 25°C for 1 hour until raw materials disappeared. The reaction solution was concentrated to dryness under reduced pressure to obtain Compound 128-3 (200 mg, yield 99%).

LC-MS (ESI+): 231.2 m/z [M+H]⁺.

### Step 3: Preparation of 2-amino-N'-ethyl-N',3-dimethylquinoline-6-carbohydrazide (128-4)

Compound 128-3 (150 mg, 0.65 mmol) and iodoethane (221 mg, 1.3 mmol) were dissolved in acetonitrile (10 mL) at room temperature, followed by the addition of potassium carbonate (269 mg, 1.9 mmol). The reaction solution was stirred at 50 °C for 2 hours. After the reaction was completed, the reaction solution was quenched with water (20 mL), and then extracted with ethyl acetate (20 mL x3). The organic phases were combined, dried over anhydrous sodium sulfate and filtered, and the filtrate was concentrated to dryness under reduced pressure. The crude product was purified by silica gel column chromatography (eluent: methanol/dichloromethane=10/1) to obtain Compound 128-4 (50 mg, yield 30%).

LC-MS (ESI+): 259.2 m/z [M+H]⁺.

Remaining steps were the same as those in the synthetic method of Example 1 to give 2-amino-*N*-ethyl-*N*', 3-dimethyl-*N*-((5-(trifluoromethyl)pyridin-2-yl)methyl) quinoline-6-carbohydrazide, except that Compound 128-4 was used instead of Compound 1-5.

LC-MS (ESI+): 418.2 m/z [M+H]⁺.

The following compounds were obtained according to the synthetic method in Example 128, using corresponding raw materials.

| Example | Structure | Mass spectrometry data (ESI+): m/z | Nuclear magnetic data |
|---|---|---|---|
| 129 | 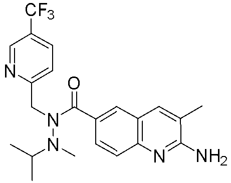 | 432.2 | |
| 130 | 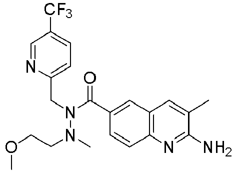 | 448.2 | |
| 131 | 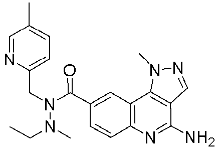 | 404.1 | ¹H NMR (400 MHz, DMSO-*d*₆) δ 9.06 (d, *J* = 2.4 Hz, 1H), 8.71 (d, *J* = 2.0 Hz, 1H), 8.31 - 8.22 (m, 2H), 7.94-7.86 (m, 1H), 7.87 (d, *J* = 8.0 Hz, 1H), 7.46 (d, *J* = 8.8 Hz, 1H), 7.01 (s, 2H), 5.32 (d, *J* = 12.0 Hz, 1H), 5.04 (d, *J* = 12.0 Hz, 1H), 4.38 (s, 3H), 4.13-4.08 (m, 1H), 3.80 - 3.75 (m, 1H), 3.60 - 3.55 (s, 3H), 3.39 (s, 3H), 1.36 - 1.32 (m, 3H). |

### Example 132: Preparation of 2-amino-N'-(2-hydroxyethyl)-N',3-dimethyl-N-((5-(trifluoromethyl)pyridin-2-yl)methyl )quinoline-6-carbohydrazide (132)

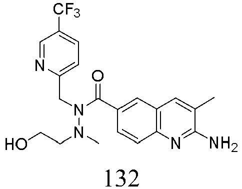

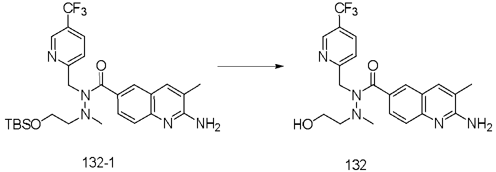

### Step 1: Preparation of 2-amino-N'-(2-hydroxyethyl)-N',3-dimethyl-N-((5-(trifluoromethyl)pyridin-2-yl)methyl )quinoline-6-carbohydrazide (132)

Compound 132-1 (synthesized according to the method in Example 128) (40 mg, 0.07 mmol) was dissolved in tetrahydrofuran (2 mL) at room temperature, followed by the addition of 1N hydrochloric acid (0.1 mL). The reaction solution was stirred at 25°C for 1 hours. After the reaction was completed, the resulting crude product was separated by reversed-phase high performance liquid chromatography (water/acetonitrile=95%/5% - 10%/90%) to obtain Compound 132 (2 mg, yield 6%).

LC-MS (ESI+): 434.3 m/z [M+H]⁺.

### Example 133: Preparation of 2-amino-N',3-dimethyl-N-((5-(trifluoromethyl)pyridin-2-yl)methyl)-N'-(3-vinylpyridin -2-yl)quinoline-6-carbohydrazide (133) and 2-amino-N'-(3-ethylpyridin-2-yl)-N',3-dimethyl-N-((5-(trifluoromethyl)pyridin-2-yl)me thyl)quinoline-6-carbohydrazide (134)

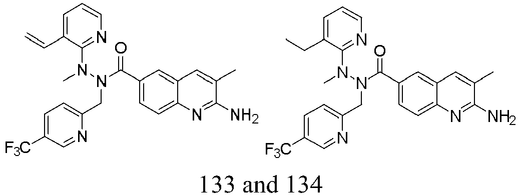

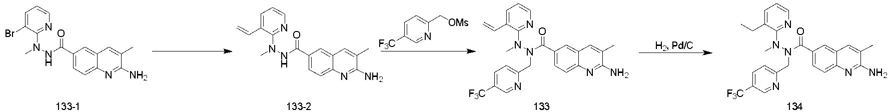

### Step 1: Preparation of 2-amino-N',3-dimethyl-N'-(3-vinylpyridin-2-yl)quinoline-6-carbohydrazide (133-2)

2-Amino-N'-(3-bromopyridin-2-yl)-N',3-dimethylquinoline-6-carbohydrazide (133-1, synthesized according to the method in Example 1) (100 mg, 0.26 mmol), potassium vinyltrifluoroborate (347 mg, 2.59 mmol), [1,1'-bis(diphenylphosphino)ferrocene]palladium dichloride (19 mg, 0.025 mmol) and cesium carbonate (422 mg, 1.3mmol) were added to 1,4-dioxane (3 mL) and water (0.4 mL) at room temperature. The reaction solution was stirred at 90°C for 12 hours under nitrogen atmosphere. After the reaction was completed, water (25 mL) was added to the reaction solution, and then the reaction solution was extracted with ethyl acetate (25 mL x2). The organic phases were combined, washed with saturated brine (20 mL), dried over anhydrous sodium sulfate and filtered, and the filtrate was concentrated to dryness under reduced pressure. The crude product was purified by silica gel column chromatography (eluent: ethyl acetate) to obtain Compound 133-2 (80 mg, yield 93%).

LC-MS (ESI+): 334.1 m/z [M+H]⁺.

### Step 2: Preparation of 2-amino-N',3-dimethyl-N-((5-(trifluoromethyl)pyridin-2-yl)methyl)-N'-(3-vinylpyridin -2-yl)quinoline-6-carbohydrazide (133)

Compound 133-2 (70 mg, 0.21 mmol), (5-(trifluoromethyl)pyridin-2-yl)methyl methanesulfonate (107 mg, 0.43 mmol) and potassium carbonate (87 mg, 0.63 mmol) were added to dimethylsulfoxide (2 mL) at room temperature. The reaction solution was stirred at 50 °C for 4 hours. After the reaction was completed, water (25 mL) was added to the reaction solution, and then the reaction solution was extracted with ethyl acetate (30 mL x 2). The organic phases were combined, washed with saturated brine (20 mL), dried over anhydrous sodium sulfate and filtered, and the filtrate was concentrated to dryness under reduced pressure. The crude product was purified by silica gel column chromatography (eluent: ethyl acetate) to obtain Compound 133 (30 mg, yield 29%).

LC-MS (ESI+): 493.1 m/z [M+H]⁺.

### Step 3: Preparation of 2-amino-N',3-dimethyl-N-((5-(trifluoromethyl)pyridin-2-yl)methyl)-N'-(3-ethylpyridin-2-yl)quinoline-6-carbohydrazide (134)

Compound 133 (20 mg, 0.04 mmol), palladium on carbon (40 mg, 10%) was added to methanol (2 mL) at room temperature. The reaction solution was stirred at 25°C for 2 hours under hydrogen atmosphere. After the reaction was completed, the reaction solution was filtered, and the filtrate was concentrated to dryness under reduced pressure. The crude product was separated by reversed-phase high performance liquid chromatography (water/acetonitrile=95%/5% - 10%/90%) to obtain Compound 134 (1.9 mg, yield 10%).

LC-MS (ESI+): 495.1 m/z [M+H]⁺.

The following compounds were obtained according to the synthetic method in Example 133, using corresponding raw materials.

| Example | Structure | Mass spectrometry data (ESI+): m/z | Nuclear magnetic data |
|---|---|---|---|
| 135 | 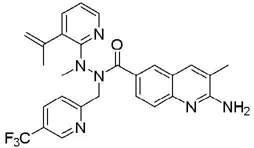 | 507.2 | |
| 136 | 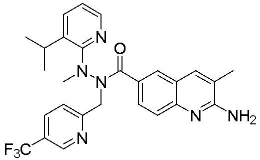 | 509.1 | |

### Example 137: Preparation of 2-amino-N-((2'-methoxy-6'-methyl-[3,3'-bipyridine]-6-yl)methyl)-N',3-dimethyl-N'-(py rimidin-2-yl)quinoline-6-carbohydrazide (137)

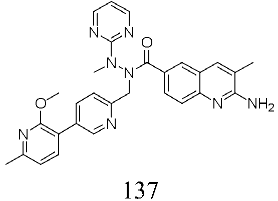

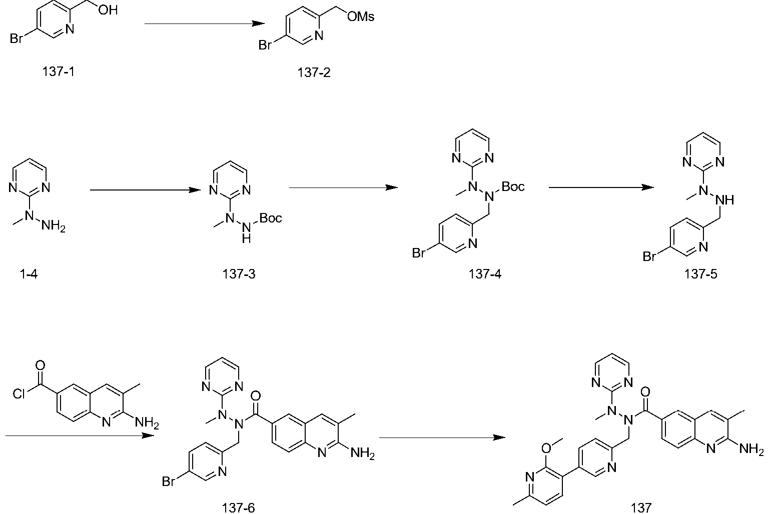

### Step 1: Preparation of methyl (5-bromopyridin-2-yl)methanesulfonate (137-2)

(5-Bromopyridin-2-yl)methanol (137-1) (20 g, 106 mmol) was dissolved in 200 mL of dichloromethane at room temperature, followed by the addition of triethylamine (32 g, 319 mmol). The mixture was stirred for 5 minutes in an ice bath, followed by the slow addition of trifluoromethanesulfonic anhydride (28 g, 159 mmol). After the addition was completed, the reaction was carried out for 1 hour in an ice bath. The reaction mixture was poured into water (300 mL) and stirred for 10 minutes. The mixture was then extracted with ethyl acetate (500 mLx 2). The organic phases were combined, dried over anhydrous sodium sulfate and filtered. The filtrate was concentrated to dryness under reduced pressure, and the resulting residues were purified by silica gel column chromatography (eluent: petroleum ether/ethyl acetate=100%-50%) to obtain Compound 137-2 (21 g, yield 74%).

LC-MS (ESI+): 266.0 m/z [M+H]⁺.

### Step 2: Preparation of tert-butyl 2-methyl-2-(pyrimidin-2-yl)hydrazine-1-carboxylate (137-3)

Compound 1-4 (20 g, 161 mmol) was dissolved in 200 mL of tetrahydrofuran and 200 mL of water at room temperature, followed by the addition of di-tert-butyl dicarbonate (53 g, 242 mmol) and sodium carbonate (51 g, 483 mmol). The reaction was carried out at room temperature under nitrogen atmosphere overnight. The reaction mixture was filtered, and concentrated under reduced pressure. The resulting mixture was extracted with ethyl acetate (200 mL x 2). The organic phases were combined, dried over anhydrous sodium sulfate and filtered. The filtrate was concentrated to dryness under reduced pressure, and the resulting residues were purified by silica gel column chromatography (eluent: petroleum ether/ethyl acetate=100%-50%) to obtain Compound 137-3 (31 g, yield 86%).

LC-MS (ESI+): 225.1 m/z [M+H]⁺.

### Step 3: Preparation of tert-butyl 1-((5-bromopyridin-2-yl)methyl)-2-methyl-2-(pyrimidin-2-yl)hydrazine-1 -carboxylate (137-4)

Compound 137-3 (15 g, 67 mmol) was dissolved in 150 mL of DMF at room temperature, followed by the addition of potassium carbonate (28 g, 201 mmol) and Compound 137-2 (20 g, 77 mmol). The reaction was carried out at 65°C under nitrogen atmosphere overnight. 200 ML of water was added to the reaction mixture, and the resulting mixture was extracted with ethyl acetate (200 mL x2). The organic phases were combined, dried over anhydrous sodium sulfate and filtered. The filtrate was concentrated to dryness under reduced pressure, and the resulting residues were purified by silica gel column chromatography (eluent: petroleum ether/ethyl acetate=100%-50%) to obtain Compound 137-4 (10 g, yield 25%).

LC-MS (ESI+): 394.1 m/z [M+H]⁺.

### Step 4: Preparation of 2-(2-((5-bromopyridin-2-yl)methyl)-1-methylhydrazino)pyrimidine (137-5)

Compound 137-4 (10 g, 25 mmol) was dissolved in 100 mL of dichloromethane at room temperature, followed by the addition of a solution (50 mL) of 4M hydrochloric acid in dioxane, and reacted at room temperature for 1 hour under nitrogen atmosphere. The reaction mixture was concentrated under reduced pressure to obtain Compound 137-5 (hydrochloride, 6.5 g, yield 87%).

LC-MS (ESI+): 294.1 m/z [M+H]⁺.

### Step 5: Preparation of 2-amino-N-((5-bromopyridin-2-yl)methyl)-N',3-dimethyl-N'-(pyrimidin-2-yl)quinoline-6-carbohydrazide (137-6)

Compound 137-5 (hydrochloride, 6.5 g, 22 mmol) was dissolved in 70 mL of dichloromethane at room temperature, followed by the addition of N,N-diisopropylethylamine (8.55 g, 66 mmol). After stirring for 5 minutes in an ice bath, 2-amino-3-methylquinoline-6-carbonyl chloride (4.88 g, 22 mmol) was added, and 1 mL of DMF was added to the reaction solution to promote the dissolution. The reaction was carried out for 1 hour under nitrogen atmosphere. The reaction mixture was directly concentrated, and the resulting residues were purified by silica gel column chromatography (eluent: dichloromethane/methanol =0-10%) to obtain Compound 137-6 (10 g, yield 95%).

LC-MS (ESI+): 478.1 m/z [M+H]⁺.

### Step 6: Preparation of 2-amino-N-((2'-methoxy-6'-methyl-[3,3'-bipyridine]-6-yl)methyl)-N',3-dimethyl-N'-(py rimidin-2-yl)quinoline-6-carbohydrazide (137)

Compound 137-6 (100 mg, 0.28 mmol) was dissolved in 3 mL of dioxane at room temperature, followed by the addition of (2-methoxy-6-methylpyridin-3-yl)boronic acid (150 mg, 0.90 mmol), potassium carbonate (298 mg, 2.16 mmol), 1,1'-bis(diphenylphosphino)ferrocene]palladium dichloridedichloromethane complex (7.31 mg, 0.02 mmol) and water (0.6 mL). The reaction was carried out at 100°C under nitrogen atmosphere overnight. The reaction mixture was filtered through Celite. The filtrate was concentrated, and the resulting residues were purified by preparative high performance liquid chromatography (water/acetonitrile=95%/5% - 10%/90%) to obtain Compound 137 (2.1 mg, yield 1.4%).

LC-MS (ESI+): 521.2 m/z [M+H]⁺.

¹H NMR (400 MHz, DMSO-*d*₆) δ 8.67 (s, 1H), 8.43 (d, *J* = 4.8 Hz, 2H), 8.28 (s, 1H), 7.98 (dd, *J* = 8.1, 2.3 Hz, 1H), 7.77 - 7.72 (m, 2H), 7.65 (d, *J* = 8.2 Hz, 1H), 7.50 (d, *J* = 8.7 Hz, 1H), 7.32 (d, *J* = 8.8 Hz, 1H), 7.00 (d, *J* = 7.5 Hz, 1H), 6.90 - 6.78 (m, 1H), 6.51 (s, 2H), 5.44 (d, *J* = 15.2 Hz, 1H), 4.46 (d, *J* = 15.3 Hz, 1H), 3.90 (s, 3H), 3.29 (s, 3H), 2.47 (s, 3H), 2.19 (s, 3H).

The following compounds were obtained according to the synthetic method in Example 137, using corresponding raw materials.

| Example | Structure | Mass spectrometry data (ESI+): m/z | Nuclear magnetic data |
|---|---|---|---|
| 138 | 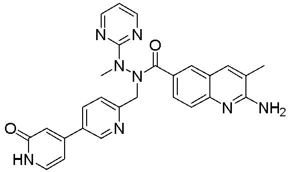 | 493.2 | |
| 139 | 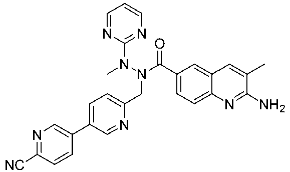 | 502.2 | |
| 140 | 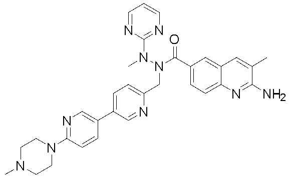 | 575.3 | |
| 141 | 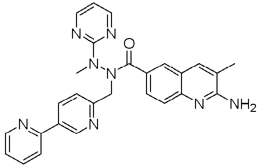 | 477.2 | ¹H NMR (400 MHz, DMSO-*d*₆) δ 9.21 (s, 1H), 8.73 (d, *J* = 4.5 Hz, 1H), 8.49 - 8.43 (m, 3H), 8.07 (d, *J* = 8.1 Hz, 1H), 7.95 (td, *J =* 7.6, 1.7 Hz, 1H), 7.76 - 7.70 (m, 3H), 7.52 - 7.47 (m, 1H), 7.45 - 7.42 (m, 1H), 7.33 (d, *J =* 8.7 Hz, 1H), 6.81 (t, *J* = 4.6 Hz, 1H), 6.55 (s, 2H), 5.50 (d, *J* = 15.1 Hz, 1H), 4.51 (d,*J* = 15.2 Hz, 1H), 3.23 (s, 3H), 2.19 (s, 3H). |
| 142 | 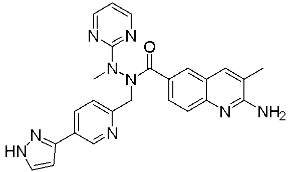 | 466.2 | ¹H NMR (400 MHz, DMSO-*d*₆) δ 8.96 (s, 1H), 8.43 (d, *J* = 4.8 Hz, 2H), 8.18 (dd, *J =* 8.1, 2.3 Hz, 1H), 7.82 (s, 2H), 7.74 (s, 1H), 7.70 (s, 1H), 7.61 (d, *J =* 8.2 Hz, 1H), 7.47 (d, *J* = 8.7 Hz, 1H), 7.31 (d, *J =* 8.7 Hz, 1H), 6.84 (d, *J =* 2.3 Hz, 1H), 6.80 (t, *J* = 4.8 Hz, 1H), 6.49 (s, 2H), 5.46 (d, *J =* 15.0 Hz, 1H), 4.44 (d, *J =* 15.0 Hz, 1H), 3.17 (s, 3H), 2.19 (s, 3H). |
| 143 | 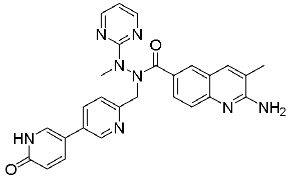 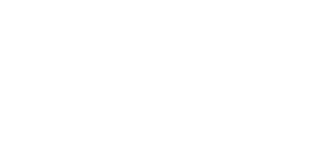 | 493.2 | ¹H NMR (400 MHz, DMSO-*d*₆) δ 8.73 (s, 1H), 8.41 (d, *J* = 4.7 Hz, 2H), 8.23 (s, 1H), 7.98 (dd, *J=* 8.2, 2.5 Hz, 1H), 7.89 (dd, *J =* 9.6, 2.7 Hz, 1H), 7.83 (s, 1H), 7.70 (d, *J =* 12.7 Hz, 2H), 7.58 (d, *J* = 8.2 Hz, 1H), 7.45 (d, *J =* 8.8 Hz, 1H), 7.30 (d, *J =* 8.7 Hz, 1H), 6.79 (t, *J =* 4.9 Hz, 1H), 6.48 - 6.45 (m, 3H), 5.43 (d, *J* = 15.1 Hz, 1H), 4.42 (d, *J =* 15.0 Hz, 1H), 3.19 (s, 3H), 2.18 (s, 3H). |
| 144 | 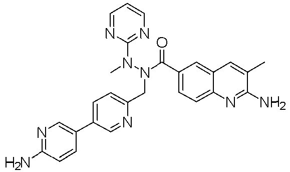 | 492.2 | ¹H NMR (400 MHz, DMSO-*d₆*) δ 8.74 (s, 1H), 8.47 - 8.30 (m, 3H), 7.99 (d, *J =* 8.0 Hz, 1H), 7.79-7.70 (m, 3H), 7.58 (d, *J =* 8.0 Hz, 1H), 7.47 (d, *J =* 8.4 Hz, 1H), 7.31 (d, *J* = 8.4 Hz, 1H), 6.80 (t, *J* = 4.4 Hz, 1H), 6.56 (d, *J* = 8.4 Hz, 1H), 6.48 (s, 2H), 6.19 (s, 2H), 5.45 (d, *J =* 14.8 Hz, 1H), 4.43 (d, *J =* 14.8 Hz, 1H), 3.20 (s, 3H), 2.19 (s, 3H). |
| 145 | 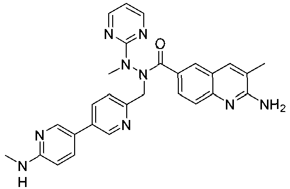 | 560.2 | ¹H NMR (400 MHz, DMSO-*d₆*) δ 8.76 (s, 1H), 8.42 (d, *J* = 9.2 Hz, 2H), 8.00 (d, *J =* 7.5 Hz, 1H), 7.86 - 7.66 (m, 3H), 7.65 - 7.26 (m, 3H), 6.87-6.81 (m, 2H), 6.66 - 6.42 (m, 3H), 5.45 (d, *J =* 15.2 Hz, 1H), 4.44 (d, *J* = 15.2 Hz, 1H), 3.21 (s, 3H), 2.84 (d, *J =* 4.0 Hz, 3H), 2.20 (s, 3H). |
| 146 | 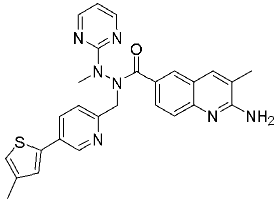 | 496.2 | |
| 147 | 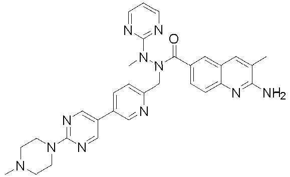 | 576.2 | |
| 148 | 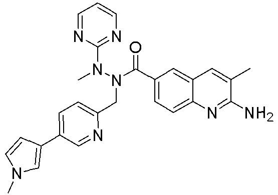 | 479.2 | |
| 149 | 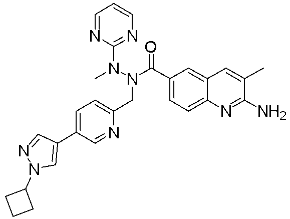 | 520.2 | |
| 150 | 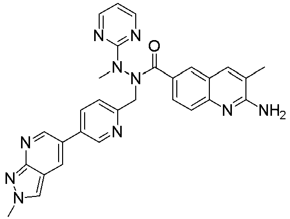 | 531.2 | |
| 151 | 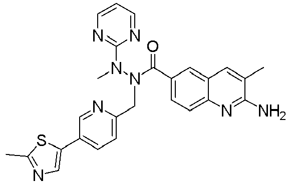 | 497.2 | |
| 152 | 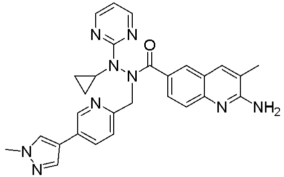 | 506.2 | |
| 153 | 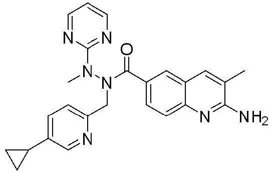 | 440.1 | |
| 154 | 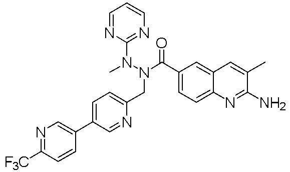 | 545.2 | |
| 155 | 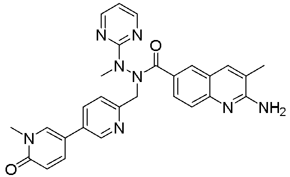 | 507.2 | ¹H NMR (400 MHz, DMSO-*d₆*) δ 8.74 (s, 1H), 8.40 (d, *J* = 4.0 Hz, 2H), 8.24 (s, 1H), 7.97 (dd, *J* = 8.0, 2.4 Hz, 1H), 7.87 (d, *J* = 12.0 Hz, 1H), 7.69 (d, *J =* 12.0 Hz, 2H), 7.59 (d, *J* = 8.0 Hz, 1H), 7.44 (d, *J =* 12.0 Hz, 1H), 7.29 (d, *J* = 8.0 Hz, 1H), 6.78 (s, 1H), 6.51 (d, *J* = 8.0 Hz, 2H), 6.47 (s, 1H), 5.42 (d, *J =* 16.0 Hz, 1H), 4.42 (d, *J =* 16.0 Hz, 1H), 3.51 (s, 3H), 3.17 (s, 3H), 2.16 (s, 3H). |
| 156 | 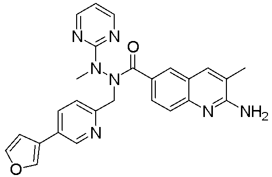 | 466.2 | ¹H NMR (400 MHz, DMSO-*d₆*) δ 8.80 (s, 1H), 8.41 (d, *J* = 4.0 Hz, 2H), 8.31 (s, 1H), 8.01 (dd, *J =* 8.0, 2.4 Hz, 1H), 7.80 (s, 1H), 7.69 (d, *J* = 12.0 Hz, 2H), 7.56 (d, *J =* 8.0 Hz, 1H), 7.44 (d, *J =* 8.0 Hz, 1H), 7.29 (d, *J* = 8.0 Hz, 1H), 7.06 (s, 1H), 6.78 (s, 1H), 6.47 (s, 2H), 5.43 (d, *J* = 16.0 Hz, 1H), 4.41 (d, *J* = 16.0 Hz, 1H), 3.16 (s, 3H), 2.17 (s, 3H). |
| 157 | 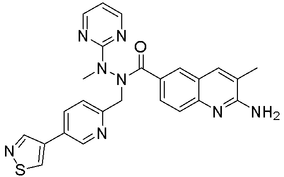 | 483.2 | ¹H NMR (400 MHz, DMSO-*d₆*) δ 9.51 (s, 1H), 9.14 (s, 1H), 8.98 (s, 1H), 8.41 (s, 2H), 8.21 (dd, *J =* 8.0, 2.4 Hz, 1H), 7.72-7.64 (m, 3H), 7.46 (d, *J =* 8.0 Hz, 1H), 7.30 (d, *J* = 8.0 Hz, 1H), 6.79 (s, 1H), 6.46 (s, 2H), 5.44 (d, *J =* 16.0 Hz, 1H), 4.45 (d, *J =* 16.0 Hz, 1H), 3.21 (s, 3H), 2.17 (s, 3H). |
| 158 | 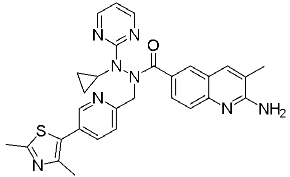 | 537.2 | ¹H NMR (400 MHz, DMSO-*d₆*) δ 8.56 (d, *J =* 4.0 Hz, 2H), 8.49 (d, *J* = 1.6 Hz, 1H), 7.87 (dd, *J* = 8.0, 2.3 Hz, 1H), 7.71 (d, *J* = 8.0 Hz, 1H), 7.68 (d, *J* = 1.6 Hz, 1H), 7.58 (s, 1H), 7.40 (dd, *J =* 8.0, 1.6 Hz, 1H), 7.25 (d, *J* = 8.0 Hz, 1H), 6.98 (t, *J =* 4.0 Hz, 1H), 6.48 (s, 2H), 5.31 (d, *J* = 16.0 Hz, 1H), 4.61 (d, *J* = 16.0 Hz, 1H), 2.75 - 2.73 (m, 1H), 2.63 (s, 3H), 2.36 (s, 3H), 2.15 (s, 3H), 0.74 - 0.65 (m, 1H), 0.48-0.47 (m, 1H), 0.42-0.40 (m, 1H), 0.09-0.08 (m, 1H). |
| 159 | 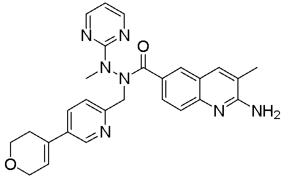 | 482.2 | |
| 160 | 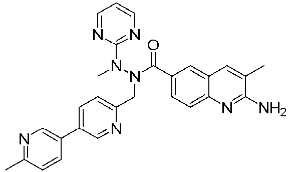 | 491.2 | ¹H NMR (400 MHz, DMSO-*d₆*) δ 8.83 (d, *J =* 16.0 Hz, 2H), 8.40 (d, *J* = 4.0 Hz, 2H), , 8.12 (dd, *J* = 8.0, 2.4Hz, 1H), 8.04 (d, *J* = 8.0 Hz, 1H), 7.73 (s, 1H), 7.69-7.66 (m, 2H), 7.46 (d, *J =* 8.0 Hz, 1H), 7.38 (d, *J =* 8.0 Hz, 1H), 7.29 (d, *J =* 8.0 Hz, 1H), 6.78 (t, *J* = 4.0 Hz, 1H), 6.49 (s, 2H), 5.43 (d, *J* = 16.0 Hz, 1H), 4.47 (d, *J =* 16.0 Hz, 1H), 3.24 (s, 3H), 2.52 (s, 3H), 2.16 (s, 3H). |
| 161 | 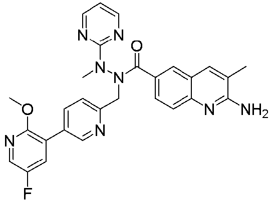 | 525.2 | ¹H NMR (400 MHz, DMSO-*d₆*) δ 8.73 (s, 1H), 8.41 (d, *J* = 4.0 Hz, 2H), 8.21 (d, *J =* 8.0 Hz, 1H), , 8.04 (dd, *J* = 8.0, 2.4 Hz, 1H), 7.95 - 7.93 (m, 1H), 7.75 (s, 1H), 7.70 - 7.66 (m, 2H), 7.48 (d, *J* = 8.0 Hz, 1H), 7.30 (d, *J =* 12.0 Hz, 1H), 6.79 (t, *J* = 4.0 Hz, 1H), 6.49 (s, 2H), 5.43 (d, *J =* 16.0 Hz, 1H), 4.46 (d, *J* = 16.0 Hz, 1H), 3.90 (s, 3H), 3.29 (s, 3H), 2.17 (s, 3H). |
| 162 | 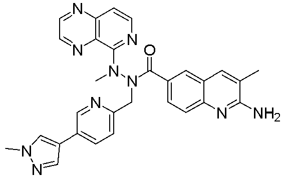 | 531.2 | |
| 163 | 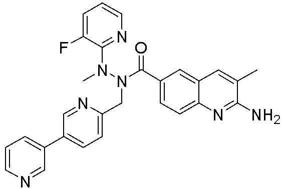 | 494.2 | ¹H NMR (400 MHz, DMSO-*d*₆) δ 8.95 (s, 1H), 8.85 (s, 1H), 8.63 (dd, *J* = 4.7, 1.6 Hz, 1H), 8.15 (d, *J* = 8.1 Hz, 2H), 8.00 (d, *J* = 4.4 Hz, 1H), 7.85 (s, 1H), 7.75 (s, 1H), 7.66 (d, *J* = 8.1 Hz, 1H), 7.60 (d, *J* = 8.6 Hz, 1H), 7.53 (dd, *J* = 8.0, 4.8 Hz, 1H), 7.48 - 7.45(m, 1H), 7.38 (d, *J* = 8.7 Hz, 1H), 6.90 - 6.86 (m, 1H), 6.50 (s, 2H), 5.33 (d, *J* = 15.5 Hz, 1H), 4.58 (d, *J* = 15.6 Hz, 1H), 3.22 (s, 3H), 2.19 (s, 3H). |
| 164 | 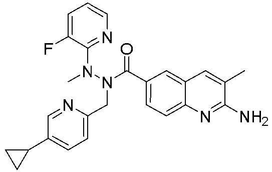 | 457.2 | ¹H NMR (400 MHz, DMSO-*d*₆) δ 8.29 (s, 1H), 8.15 (s, 1H), 7.98 (s, 1H), 7.77 (s, 1H), 7.74 (s, 1H), 7.55 (d, *J* = 8.7 Hz, 1H), 7.48 - 7.43 (m, 1H), 7.38 - 7.34 (m, 2H), 6.88 - 6.84 (m, 1H), 6.51 (s, 2H), 5.24 (d, *J* = 15.2 Hz, 1H), 4.43 (d, *J* = 15.2 Hz, 1H), 3.18 (s, 3H), 2.18 (s, 3H), 1.96 - 1.91 (m, 1H), 1.02 - 0.96 (m, 2H), 0.74 - 0.69 (m, 2H). |
| 165 | 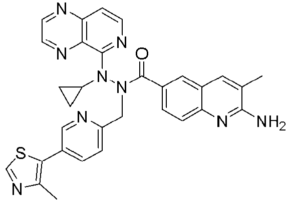 | 574.2 | |
| 166 | 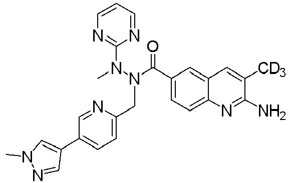 | 483.2 | |
| 167 | 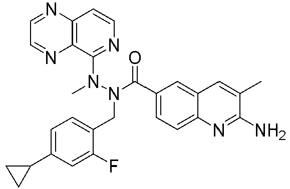 | 508.2 | ¹H NMR (400 MHz, DMSO-*d*₆) δ 8.94 (s, 1H), 8.82 (s, 1H), 8.47 (s, 1H), 8.22 (d, *J* = 5.7 Hz, 1H), 7.79 (s, 1H), 7.64 (s, 1H), 7.52 (d, *J =* 9.0 Hz, 1H), 7.24 (d, *J* = 6.8 Hz, 2H), 6.69 (dd, *J* = 23.6, 9.6 Hz, 2H), 6.38 (s, 2H), 5.17 (d, *J* = 14.8 Hz, 1H), 4.58 (d, *J* = 14.8 Hz, 1H), 3.40 (s, 3H), 2.14 (s, 3H), 1.82 (d, *J* = 9.2 Hz, 1H), 1.00 - 0.90 (m, 2H), 0.64 - 0.57 (m, 2H). |
| 168 | 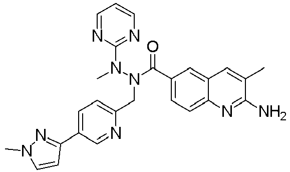 | 480.2 | ¹H NMR (400 MHz, DMSO-*d*₆) δ 8.91 (s, 1H), 8.43 - 8.32 (m, 2H), 8.20 - 8.08 (m, 1H), 7.78 (d, *J =* 2.4 Hz, 1H), 7.72 (d, *J* = 8.8 Hz, 2H), 7.57 (d, *J* = 8.0 Hz, 1H), 7.45 (s, 1H), 6.83 - 6.72 (m, 2H), 6.54 (s, 2H), 5.44 (d, *J* = 15.2 Hz, 1H), 4.43 (d, *J* = 15.2 Hz, 1H), 3.91 (s, 3H), 3.15 (s, 3H), 2.18 (s, 3H). |
| 169 | 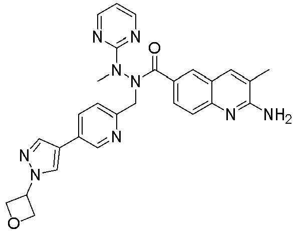 | 522.3 | ¹H NMR (400 MHz, DMSO-*d*₆) δ 8.80 (s, 1H), 8.49 (s, 1H), 8.41 (d, *J* = 4.8 Hz, 2H), 8.13 (s, 1H), 7.99 (dd, *J =* 8.0, 2.4 Hz, 1H), 7.70 (d, *J* = 14.0 Hz, 2H), 7.55 (d, *J* = 8.4 Hz, 1H), 7.46 (d, *J* = 8.8 Hz, 1H), 7.30 (d, *J* = 8.8 Hz, 1H), 6.78 (t, *J* = 4.8 Hz, 1H), 6.46 (s, 2H), 5.61 (t, *J =* 7.2 Hz, 1H), 5.43 (d, *J* = 14.8 Hz, 1H), 4.95 (p, *J* = 6.8 Hz, 4H), 4.41 (d, *J* = 14.8 Hz, 1H), 3.16 (s, 3H), 2.18 (s, 3H). |
| 170 | 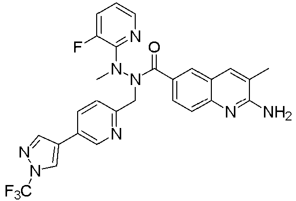 | 551.2 | |
| 171 | 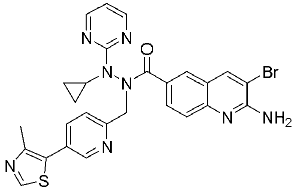 | 587.1 | |
| 172 | 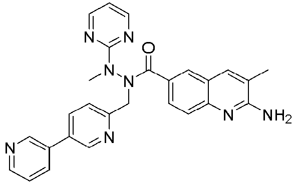 | 477.5 | ¹H NMR (400 MHz, DMSO-*d*₆) δ 8.98 (s, 1H), 8.90 (s, 1H), 8.65 (d, *J* = 4.8 Hz, 1H), 8.43 (s, 2H), 8.20 - 8.17 (m, 2H), 7.76 (s, 1H), 7.71 (s, 1H), 7.54-7.57 (m, 2H), 7.49 (d,*J* = 8.8 Hz, 1H), 7.32 (d, *J* = 8.6 Hz, 1H), 6.81 (s, 1H), 6.49 - 6.46 (m, 2H), 5.47 (d, *J* = 15.0 Hz, 1H), 4.51 (d, *J* = 15.2 Hz, 1H), 3.27 (s, 3H), 2.19 (s, 3H). |
| 173 | 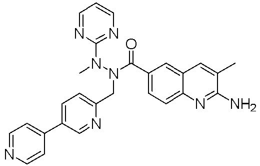 | 477.6 | ¹H NMR (400 MHz, DMSO-*d*₆) δ 8.98 (s, 1H), 8.70 (d, *J* = 2.9 Hz, 2H), 8.42 (d, *J* = 4.7 Hz, 2H), 8.25-8.26 (m, 1H), 7.82 (d, *J* = 5.3 Hz, 2H), 7.77-7.70 (m, 1H), 7.71 (s, 1H), 7.49 (d, *J* = 8.8 Hz, 1H), 7.32 (d, *J* = 8.7 Hz, 1H), 6.81 (s, 1H), 6.48 (s, 2H), 5.46 (d, *J* = 15.2 Hz, 1H), 4.52 (d, *J* = 15.2 Hz, 1H), 3.27 (s, 3H), 2.19 (s, 3H). |
| 174 | 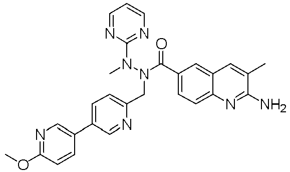 | 507.6 | ¹H NMR (400 MHz, DMSO-*d*₆) δ 9.31 (s, 1H), 9.06 - 9.02 (m, 2H), 8.62 (s, 1H), 8.44 (d, *J* = 4.8 Hz, 1H), 8.32 (m, 1H), 8.29 (s, 1H), 7.76 (s, 2H), 7.72 (s, 1H), 7.49 (d, *J* = 8.8 Hz, 1H), 7.32 (d, *J* = 8.7 Hz, 1H), 6.85 - 6.78(m, 1H), 6.50 (s, 2H), 5.48 (d, *J* = 15.2 Hz, 1H), 4.52 (d, *J* = 15.4 Hz, 1H), 3.50 (s, 3H), 3.28 (s, 3H), 2.19 (s, 3H). |
| 175 | 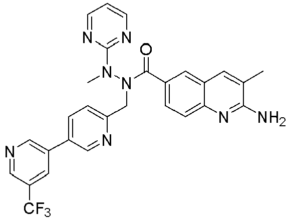 | 545.6 | ¹H NMR (400 MHz, DMSO-*d*₆) δ 9.31 (s, 1H), 9.06 - 9.02 (m, 2H), 8.62 (s, 1H), 8.44 (d, *J* = 4.8 Hz, 1H), 8.32 (m, 1H), 8.29 (s, 1H), 7.76 (s, 2H), 7.72 (s, 1H), 7.49 (d, *J* = 8.8 Hz, 1H), 7.32 (d, *J* = 8.7 Hz, 1H), 6.85 - 6.78(m, 1H),, 6.50 (s, 2H), 5.48 (d, *J* = 15.2 Hz, 1H), 4.52 (d, *J* = 15.4 Hz, 1H), 3.28 (s, 3H), 2.19 (s, 3H). |
| 176 | 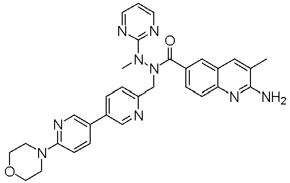 | 562.5 | ¹H NMR (400 MHz, DMSO-*d*₆) δ 8.81 (s, 1H), 8.55 (s, 1H), 8.50 (s, 1H), 8.42 (s, 1H), 8.07 (d, *J* = 5.7 Hz, 1H), 7.98 (d, *J* = 9.0 Hz, 1H), 7.73 (d, *J* = 14.3 Hz, 2H), 7.63 (d, *J* = 8.1 Hz, 1H), 7.48 (d, *J* = 9.1 Hz, 1H), 7.31 (d, *J* = 8.7 Hz, 1H), 6.98 (d, *J* = 8.9 Hz, 1H), 6.85 - 6.78(m, 1H),, 6.49 (s, 2H), 5.44 (d, *J* = 15.2 Hz, 1H), 4.45 (d, *J* = 15.1 Hz, 1H), 3.74 (s, 4H), 3.54 (d, *J* = 4.9 Hz, 4H), 3.24 (s, 3H), 2.19 (s, 3H). |
| 177 | 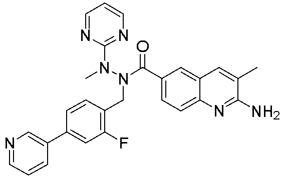 | 494.5 | ¹H NMR (400 MHz, DMSO-*d*₆) δ 8.96 (d, *J* = 2.4 Hz, 1H), 8.62 - 8.61 (m, 1H), 8.45 - 8.35 (m, 2H), 8.16 - 8.13 (m, 1H), 7.72 (d, *J =* 14.9 Hz, 2H), 7.66 - 7.61 (m, 3H), 7.52 (d, *J* = 3.3 Hz, 1H), 7.46 (d, *J* = 8.7 Hz, 1H), 7.31 (d, *J* = 8.7 Hz, 1H), 6.82 - 6.76 (m, 1H), 6.50 (s, 2H), 5.37 (d, *J* = 14.7 Hz, 1H), 4.56 (d, *J* = 14.8 Hz, 1H), 3.16 (s, 3H), 2.18 (s, 3H). |
| 178 | 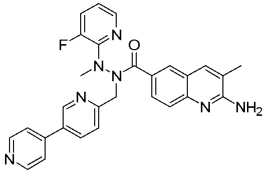 | 494.5 | ¹H NMR (400 MHz, DMSO-*d*₆) δ 8.93 (s, 1H), 8.69 - 8.67 (m, 2H), 8.20 (s, 1H), 8.00 (s, 1H), 7.84 (s, 1H), 7.79 (d, *J* = 5.2 Hz, 2H), 7.75 (s, 1H), 7.68 (d, *J* = 8.0 Hz, 1H), 7.59 (d, *J* = 8.4 Hz, 1H), 7.47 (d, *J* = 5.6 Hz, 1H), 7.37 (d, *J* = 8.8 Hz, 1H), 6.90 - 6.87 (m, 1H), 6.48 (s, |
| | | | 2H), 5.33 (d, *J* = 15.5 Hz, 1H), 4.59 (d, *J* = 15.5 Hz, 1H), 3.31(s,3H),2.19 (s, 3H). |
| 179 | 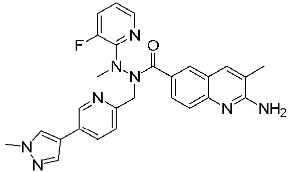 | 497.5 | ¹H NMR (400 MHz, DMSO-*d*₆) δ 8.71 (s, 1H), 8.23 (s, 1H), 7.99 (s, 1H), 7.96 - 7.90 (m, 3H), 7.80 (d, *J* = 3.7 Hz, 1H), 7.74 (s, 1H), 7.60 - 7.45 (m, 3H), 6.88 - 6.85 (m, 1H), 6.46 (s, 2H), 5.27 (d, *J* = 15.4 Hz, 1H), 4.47 (d, *J* = 15.3 Hz, 1H), 3.88 (s, 3H), 3.21 (s, 3H), 2.19 (s, 3H). |
| 180 | 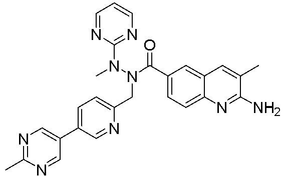 | 492.6 | ¹H NMR (400 MHz, DMSO-*d*₆) δ 9.11 (s, 2H), 8.94 (s, 1H), 8.43 (d, *J* = 4.7 Hz, 2H), 8.23 (dd, *J* = 8.2, 2.4 Hz, 1H), 7.76 - 7.71 (m, 3H), 7.49 (d, *J* = 8.9 Hz, 1H), 7.32 (d, *J* = 8.8 Hz, 1H), 6.81 (s, 1H), 6.49 (s, 2H), 5.47 (d, *J* = 15.2 Hz, 1H), 4.50 (d, *J* = 15.2 Hz, 1H), 3.28 (s, 3H), 2.70 (s, 3H), 2.19 (s, 3H). |
| 181 | 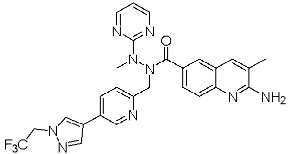 | 548.6 | ¹H NMR (400 MHz, DMSO-*d*₆) δ 8.80 (s, 1H), 8.42-8.37 (m, 2H), 8.24 (s, 1H), 8.15 (s, 1H), 8.01 (dd, *J* = 8.1, 2.3 Hz, 1H), 7.69 (d, *J =* 12.0 Hz, 2H), 7.56 (d, *J* = 8.1 Hz, 1H), 7.44 (d, *J* = 8.7 Hz, 1H), 7.29 (d, *J* = 8.7 Hz, 1H), 6.77-6.80 (m, 1H), 6.45 (s, 2H), 5.43 (d, *J* = 14.9 Hz, 1H), 5.19 (q, *J* = 9.2 Hz, 2H), 4.41 (d, *J* = 15.0 Hz, 1H), 3.15 (s, 3H), 2.17 (s, 3H). |
| 182 | 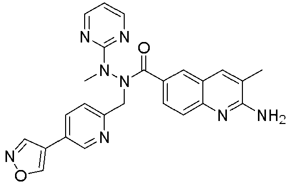 | 467.6 | ¹H NMR (400 MHz, DMSO-*d*₆) δ 8.81 (s, 1H), 8.51 (d, *J* = 22.0 Hz, 1H), 8.41 (s, 2H), 8.23 (d, *J* = 20.5 Hz, 1H), 8.04-8.07 (m, 1H), 7.81 - 7.71 (m, 3H), 7.41-7.49 (m, 2H), 7.34 (d, *J* = 8.4 Hz, 1H), 6.79 (s, 2H), 5.39 (d, *J* = 15.1 Hz, 1H), 4.38 (d, *J* = 17.6 Hz, 1H), 3.07 (s, 3H), 2.19 (s, 3H). |
| 183 | 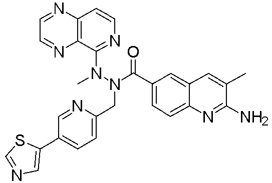 | 534.6 | ¹H NMR (400 MHz, DMSO-*d*₆) δ 9.17 (s, 1H), 9.10 - 8.99 (m, 1H), 8.97 (d, *J* = 1.8 Hz, 1H), 8.85 (d, *J* = 1.8 Hz, 1H), 8.73 (d, *J* = 2.4 Hz, 1H), 8.39 (s, 1H), 8.26 (d, *J* = 5.7 Hz, 1H), 8.00-8.02 (m, 1H), 7.81 (d, *J* = 2.1 Hz, 1H), 7.68 (d, *J =* 17.1 Hz, 1H), 7.60 - 7.54 (m, 1H), 7.49 - 7.34 (m, 1H), 7.29 - 7.24 (m, 1H), 6.42 (s, 2H), 5.35 (d, *J =* 15.3 Hz, 1H), 4.58 (d, *J* = 15.4 Hz, 1H), 3.60 (s, 3H), 2.14 (s, 3H). |
| 184 | 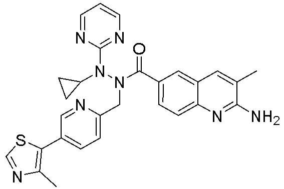 | 523.6 | ¹H NMR (400 MHz, DMSO-*d*₆) δ 9.11 (s, 1H), 8.59 (d, J = 4.8 Hz, 2H), 8.57 (d, J = 2.5 Hz, 1H), 7.94-7.97 (m,1H), 7.76 (d, J = 8.1 Hz, 1H), 7.71 (d, J = 2.0 Hz, 1H), 7.61 (s, 1H), 7.41-7.44 (m, 1H), 7.28 (d, J = 8.7 Hz, 1H), 7.00-7.03 (m, 1H), 6.54 (s, 2H), 5.35 (d, J = 15.1 Hz, 1H), 4.64 (d, J = 15.2 Hz, 1H), 2.82 - 2.72 (m, 1H), 2.48 (s, 3H), 2.18 (s, 3H), 0.77 - 0.68 (m, 1H), 0.54 - 0.40 (m, 2H), 0.16 - 0.07 (m, 1H). |
| 185 | 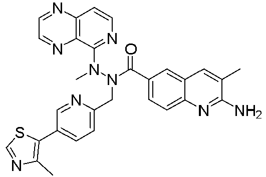 | 548.6 | ¹H NMR (400 MHz, DMSO-*d*₆) δ 9.09 (s, 1H), 8.98 (d, *J* = 1.8 Hz, 1H), 8.86 (d, *J* = 1.8 Hz, 1H), 8.50 (d, *J* = 2.3 Hz, 1H), 8.28 (s, 1H), 7.86 (d, *J* = 2.0 Hz, 1H), 7.68 (s, 1H), 7.64 - 7.58 (m, 2H), 7.37-7.49 (m, 1H), 7.26-7.30(m, 2H), 6.50 (s, 2H), 5.32 (d, *J* = 15.7 Hz, 1H), 4.69 (d, *J* = 15.4 Hz, 1H), 3.65 (s, 3H), 2.46 (s, 3H), 2.16 (s, 3H). |
| 186 | 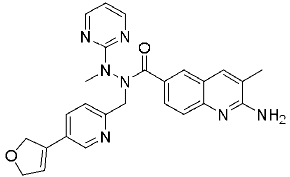 | 468.2 | ¹H NMR (400 MHz, DMSO-*d*₆) δ 8.59 (s, 1H), 8.49 - 8.37 (m, 2H), 8.30 (s, 1H), 7.91 - 7.84 (m, 1H), 7.73 - 7.69 (m, 1H), 7.63 - 7.54 (m, 1H), 7.52 - 7.40 (m, 1H), 7.37 - 7.25 (m, 1H), 6.92 - 6.77 (m, 1H), 6.67 (s, 1H), 6.48 (s, 2H), 5.43 (d, *J* = 15.2 Hz, 1H), 5.04 - 4.87 (m, 2H), 4.83 - 4.70 (m, 2H), 4.51 - 4.35 (m, 1H), 3.18 (s, 3H), 2.19 (s, 3H). |
| 187 | 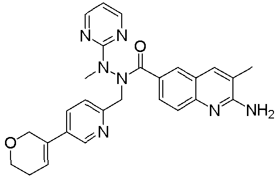 | 482.8 | |
| 188 | 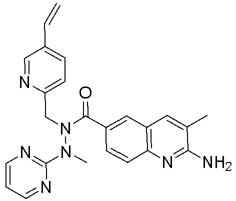 | 426.2 | |
| 189 | 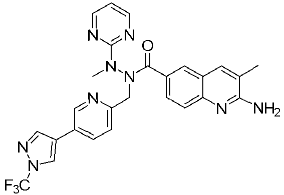 | 534.2 | ¹H NMR (400 MHz, DMSO-*d₆*) δ 9.13 (s, 1H), 8.97 - 8.92 (m, 1H), 8.58 (s, 1H), 8.43 (d, J = 4.8 Hz, 2H), 8.17 - 8.13 (m, 1H), 7.72 (d, J = 14.0 Hz, 2H), 7.64 (d, J = 8.0 Hz, 1H), 7.47 (d, J = 8.8 Hz, 1H), 7.32 (d, J = 8.8 Hz, 1H), 6.84 - 6.78 (m, 1H), 6.50 (s, 2H), 5.45 (d, J = 15.2 Hz, 1H), 4.45 (d, J = 15.2 Hz, 1H), 3.20 (s, 3H), 2.19 (s, 3H). |
| 190 | 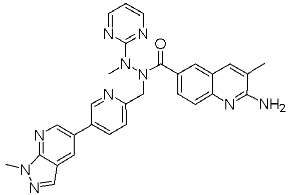 | 531.2 | ¹H NMR (400 MHz, DMSO-*d₆*) δ 8.96 - 8.89 (m, 2H), 8.58 (d, J = 2.0 Hz, 1H), 8.42 (d, J = 4.8 Hz, 2H), 8.23 (s, 1H), 8.19 (dd, J = 8.0, 2.4 Hz, 1H), 7.77 (s, 1H), 7.74 - 7.68 (m, 2H), 7.50 (d, J = 8.8 Hz, 1H), 7.33 (d, J = 8.8 Hz, 1H), 6.79 (t, J = 4.8 Hz, 1H), 6.56 (s, 2H), 5.48 (d, J = 15.2 Hz, 1H), 4.50 (d, J = 15.2 Hz, 1H), 4.11 (s, 3H), 3.27 (s, 3H), 2.19 (s, 3H). |
| 191 | 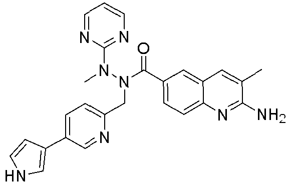 | 465.2 | ¹H NMR (400 MHz, DMSO-*d₆*) δ 11.10 (s, 1H), 8.74 (s, 1H), 8.43 (s, 2H), 7.91 (dd, J = 8.0, 2.4 Hz, 1H), 7.71 (d, J = 8.4 Hz, 2H), 7.45 (d, J = 8.4 Hz, 2H), 7.37 (s, 1H), 7.31 (d, J = 8.4 Hz, 1H), 6.89 - 6.77 (m, 2H), 6.55 - 6.47 (m, 3H), 5.44 (d, J = 14.8 Hz, 1H), 4.36 (d, J = 14.8 Hz, 1H), 3.12 (s, 3H), 2.19 (s, 3H). |
| 192 | 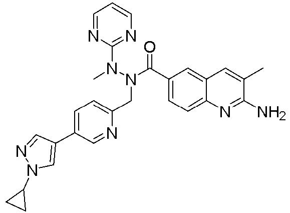 | 506.2 | |
| 193 | 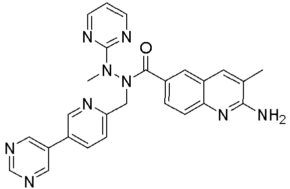 | 478.2 | ¹H NMR (400 MHz, DMSO-*d₆*) δ 9.25 (s, 1H), 9.23 (s, 2H), 8.97 (s, 1H), 8.42 (d, J = 4.8 Hz, 2H), 8.26 (dd, J = 8.0, 2.4 Hz, 1H), 7.78 - 7.68 (m, 3H), 7.48 (d, J = 8.8 Hz, 1H), 7.31 (d, J = 8.8 Hz, 1H), 6.83 - 6.76 (m, 1H), 6.49 (s, 2H), 5.46 (d, J = 15.2 Hz, 1H), 4.51 (d, J = 15.2 Hz, 1H), 3.27 (s, 3H), 2.18 (s, 3H). |
| 194 | 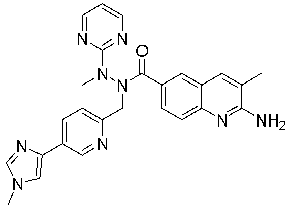 | 480.2 | |
| 195 | 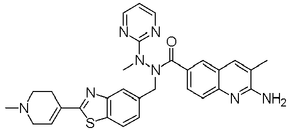 | 551.2 | |
| 196 | 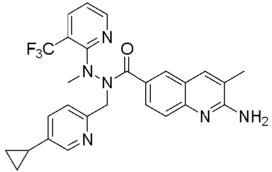 | 507.0 | ¹H NMR (400 MHz, DMSO-*d₆*) δ 8.44 (s, 1H), 8.37 (s, 1H), 8.16 (s, 1H), 8.09 - 7.83 (m, 1H), 7.79 - 7.54 (m, 2H), 7.42 (s, 2H), 7.37 - 7.14 (m, 1H), 7.10 - 6.95 (m, 1H), 6.47 (s, 2H), 5.56 (d, *J* = 15.0 Hz, 1H), 4.40 (d, *J* = 15.2 Hz, 1H), 3.35 (s, 3H), 2.19 (s, 3H), 2.00 - 1.84 (m, 1H), 1.10 - 0.89 (m, 2H), 0.84 - 0.53 (m, 2H). |
| 197 | 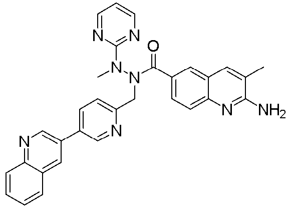 | 527.0 | |
| 198 | 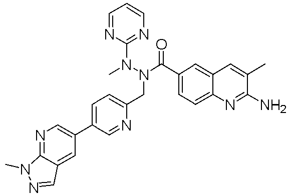 | 531.0 | |
| 199 | 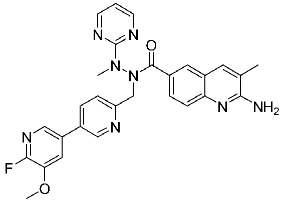 | 525.0 | |
| 200 | 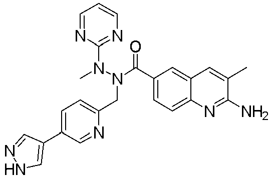 | 466.0 | |
| 201 | 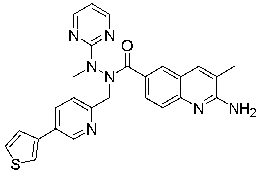 | 482.0 | |
| 202 | 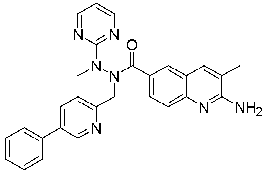 | 476.0 | |
| 203 | 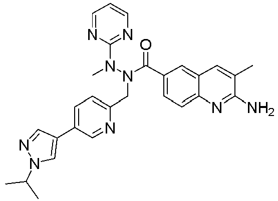 | 508.0 | |
| 204 | 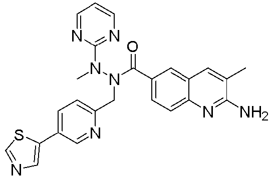 | 483.0 | |
| 205 | 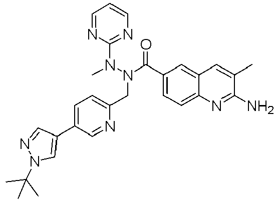 | 522.0 | |
| 206 | 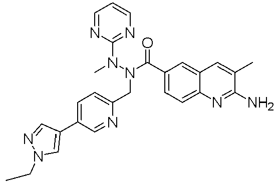 | 494.0 | |
| 207 | 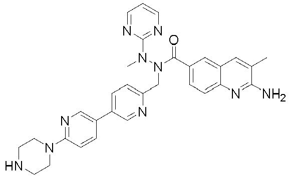 | 561.0 | |
| 208 | 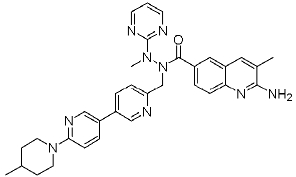 | 574.0 | |
| 209 | 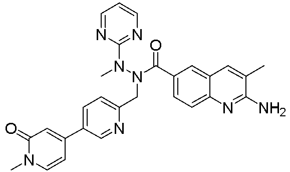 | 507.0 | |
| 210 | 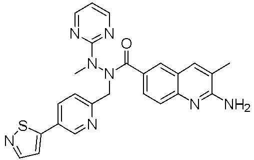 | 483.0 | |
| 211 | 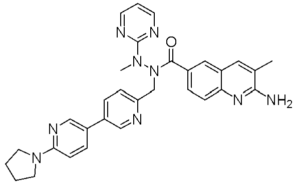 | 546.2 | ¹H NMR (400 MHz, DMSO-*d₆*) δ 8.76 (s, 1H), 8.47 (d, *J =* 2.4 Hz, 1H), 8.41 (d, *J* = 4.8 Hz, 2H), 8.00 (d, *J* = 8.0 Hz, 1H), 7.88 (d, *J* = 8.8 Hz, 1H), 7.71 (d, *J* = 13.2 Hz, 2H), 7.58 (d, *J* = 8.0 Hz, 1H), 7.46 (d, *J* = 8.8 Hz, 1H), 7.30 (d, *J* = 8.8 Hz, 1H), 6.78 (d, *J* = 6.0 Hz, 1H), 6.55 (d, *J* = 8.8 Hz, 1H), 6.47 (s, 2H), 5.43 (d, *J* = 15.2 Hz, 1H), 4.42 (d, *J* = 14.8 Hz, 1H), 3.46 - 3.41 (m, 4H), 3.20 (s, 3H), 2.17 (s, 3H), 1.98 - 1.93 (m, 4H). |
| 212 | 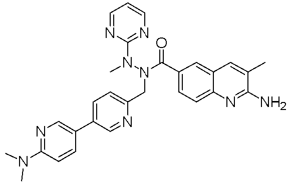 | 520.2 | ¹H NMR (400 MHz, DMSO-*d*₆) δ 8.79 (s, 1H), 8.51 (d, *J* = 2.4 Hz, 1H), 8.43 (d, *J* = 4.8 Hz, 2H), 8.04 (d, *J* = 8.0 Hz, 1H), 7.95 - 7.88 (m, 1H), 7.73 (d, *J* = 14.4 Hz, 2H), 7.61 (d, *J* = 8.0 Hz, 1H), 7.48 (d, *J* = 8.4 Hz, 1H), 7.31 (d, *J* = 8.8 Hz, 1H), 6.83 - 6.74 (m, 2H), 6.50 (s, 2H), 5.45 (d, *J* = 15.2 Hz, 1H), 4.44 (d, *J* = 15.2 Hz, 1H), 3.23 (s, 3H), 3.10 (s, 6H), 2.19 (s, 3H). |
| 213 | 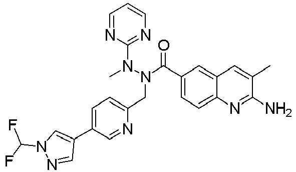 | 526.2 | ¹H NMR (400 MHz, DMSO-*d*₆) δ 8.88 (s, 1H), 8.85 (s, 1H), 8.44 - 8.36 (m, 3H), 8.10 (d, *J* = 8.0 Hz, 1H), 7.87 (s, 1H), 7.73 - 7.67 (m, 2H), 7.59 (d, *J* = 8.0 Hz, 1H), 7.45 (d, *J* = 8.8 Hz, 1H), 7.29 (d, *J* = 8.8 Hz, 1H), 6.79 (s, 1H), 6.45 (s, 2H), 5.43 (d, *J* = 15.2 Hz, 1H), 4.43 (d, *J* = 15.2 Hz, 1H), 3.18 (s, 3H), 2.17 (s, 3H). |
| 214 | 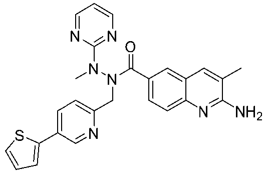 | 482.2 | ¹H NMR (400 MHz, DMSO-*d*₆) δ 8.82 (s, 1H), 8.40 (d, *J* = 4.8 Hz, 2H), 8.04 (d, *J* = 8.0 Hz, 1H), 7.72 - 7.59 (m, 5H), 7.45 (d, *J* = 8.8 Hz, 1H), 7.30 (d, *J* = 8.8 Hz, 1H), 7.19 (d, *J* = 5.2 Hz, 1H), 6.78 (t, *J* = 4.8 Hz, 1H), 6.45 (s, 2H), 5.42 (d, *J* = 15.2 Hz, 1H), 4.45 (d, *J* = 15.2 Hz, 1H), 3.19 (s, 3H), 2.17 (s, 3H). |
| 215 | 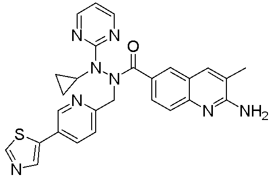 | 509.1 | ¹H NMR (400 MHz, DMSO-*d*₆) δ 9.19 (s, 1H), 8.80 (d, *J =* 2.4 Hz, 1H), 8.61 (d, *J* = 4.8 Hz, 2H), 8.44 (s, 1H), 8.16-8.10 (m, 1H), 7.74 (d, *J =* 8.0 Hz, 1H), 7.70 (d, *J* = 2.0 Hz, 1H), 7.61 (s, 1H), 7.45-7.40 (m, 1H), 7.28 (d, *J* = 8.8 Hz, 1H), 7.06-7.00 (m, 1H), 6.51 (s, 2H), 5.40 (d, *J* = 15.2 Hz, 1H), 4.55 (d, *J* = 15.2 Hz, 1H), 2.74-2.68 (m, 1H), 2.18 (s, 3H), 0.78 - 0.62 (m, 1H), |
| | | | 0.52 - 0.28 (m, 2H), 0.15 - 0.05 (m, 1H). |
| 216 | 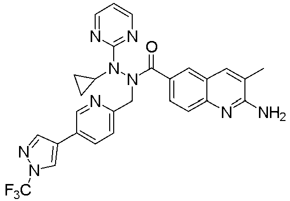 | 560.1 | ¹H NMR (400 MHz, DMSO-*d*₆) δ 9.12 (s, 1H), 8.87 (d, *J* = 2.4 Hz, 1H), 8.61 (d, *J* = 4.8 Hz, 2H), 8.57 (s, 1H), 8.19 - 8.13 (m, 1H), 7.72 - 7.68 (m, 2H), 7.61 (s, 1H), 7.43 - 7.39 (m, 1H), 7.28 (d, *J* = 8.8 Hz, 1H), 7.05 - 7.01 (m, 1H), 6.51 (s, 2H), 5.42 (d, *J* = 15.2 Hz, 1H), 4.51 (d, *J* = 15.2 Hz, 1H), 2.68-2.64 (m, 1H),2.18 (s, 3H), 0.69-0.64 (m, 1H), 0.49-0.42 (m, 1H), 0.38 - 0.29(m, 1H), 0.09-0.06 (m, 1H). |

### Example 217: Preparation of 2-amino-N',3-dimethyl-N'-(pyrimidin-2-yl)-N-((5-(tetrahydrofuran-3-yl)pyridin-2-yl)m ethyl)quinoline-6-carbohydrazide (217)

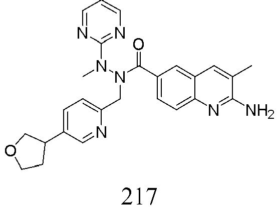

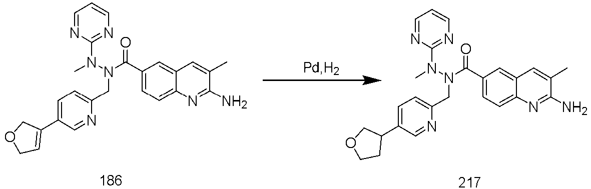

### Step 1: Preparation of 2-amino-N',3-dimethyl-N'-(pyrimidin-2-yl)-N-((5-(tetrahydrofuran-3-yl)pyridin-2-yl)m ethyl)quinoline-6-carbohydrazide (217)

Compound 186 (80 mg, 0.17 mmol) was dissolved in methanol (2 mL) at room temperature, followed by the addition of palladium on carbon (20 mg, 0.19 mmol), and the resulting mixture was purged with hydrogen and then stirred at room temperature for 4 hours. The reaction solution was filtered through Celite. The filtrate was concentrated under reduced pressure, and the residues were purified by preparative high performance liquid chromatography (water/acetonitrile=95%/5%-10%/90%), and subjected to lyophilization to obtain Compound 217 (2.1 mg, yield 2.6%).

LC-MS (ESI+): 470.3 m/z [M+H]⁺.

The following compounds were obtained according to the synthetic method in Example 217, using corresponding raw materials.

| Example | Structure | Mass spectrometry data (ESI+): m/z | Nuclear magnetic data |
|---|---|---|---|
| 218 | 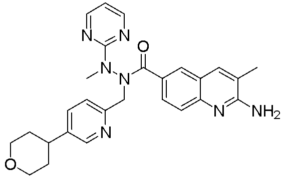 | 484.2 | |
| 219 | 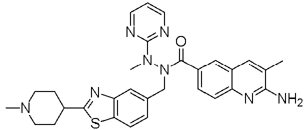 | 553.2 | |

### Example 220: Preparation of 2-amino-N',3-dimethyl-N-((5-(pyridazin-4-yl)pyridin-2-yl)methyl)-N'-(pyrimidin-2-yl) quinoline-6-carbohydrazide (220)

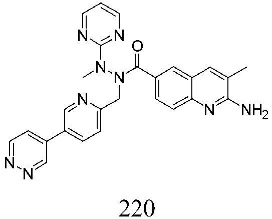

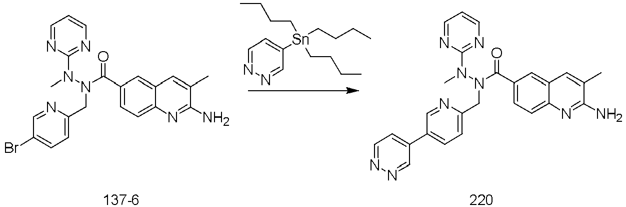

### Step 1: Preparation of 2-amino-N',3-dimethyl-N-((5-(pyridazin-4-yl)pyridin-2-yl)methyl)-N'-(pyrimidin-2-yl) quinoline-6-carbohydrazide (220)

Compound 137-6 (50 mg, 0.10 mmol) was dissolved in toluene (2 mL) in a sealing tube, followed by the addition of 4-(tributylstannyl)pyridazine (57.80 mg, 0.15 mmol), Pd(PPh₃)₄ (12 mg, 0.01 mmol) and lithium chloride (4.4 mg, 0.10 mmol) successively, and after purging with N₂, the resulting mixed solution was stirred at 100°C for 12 hours. EtOAc (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 220 (6.7 mg, yield 13%, a white solid).

LC-MS (ESI+): 478.2 m/z [M+H]⁺.

¹H NMR (400 MHz, DMSO-*d*₆) δ 9.72 (d, *J* = 2.4 Hz, 1H), 9.33 (dd, *J* = 5.6, 1.2 Hz, 1H), 9.07 (s, 1H), 8.45 - 8.23 (m, 3H), 8.11 (dd, *J* = 5.6, 2.4 Hz, 1H), 7.85 - 7.64 (m, 3H), 7.48 (d, *J* = 8.8 Hz, 1H), 7.31 (d, *J* = 8.8 Hz, 1H), 6.79 (t, *J* = 4.8 Hz, 1H), 6.46 (s, 2H), 5.46 (d, *J* = 15.2 Hz, 1H), 4.53 (d, *J* = 15.6 Hz, 1H), 3.27 (s, 3H), 2.18 (s, 3H).

The following compounds were obtained according to the synthetic method in Example 220, using corresponding raw materials.

| Example | Structure | Mass spectrometry data (ESI+): m/z | Nuclear magnetic data |
|---|---|---|---|
| 221 | 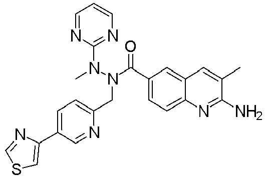 | 483.6 | ¹H NMR (400 MHz, DMSO-*d*₆) δ 9.27 (s, 1H), 9.13 (s, 1H), 8.50 (s, 1H), 8.41 (s, 1H), 8.34 (d, *J* = 6.4 Hz, 2H), 7.64-7.73 (m, 3H), 7.46 (d, *J =* 8.6 Hz, 1H), 7.29 (d, *J* = 8.6 Hz, 1H), 6.79 (s, 1H), 6.45 (s, 2H), 5.46 (d, *J* = 15.3 Hz, 1H), 4.46 (d, *J* = 15.0 Hz, 1H), 3.18 (s, 3H), 2.17 (s, 3H). |
| 222 | 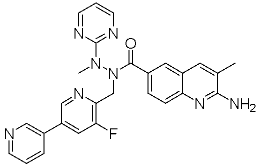 | 495.0 | ¹H NMR (400 MHz, DMSO-*d₆*) δ 9.03 (s, 1H), 8.82 (s, 1H), 8.69 - 8.64 (m, 1H), 8.42 (d, *J* = 3.4 Hz, 2H), 8.33 - 8.12 (m, 2H), 7.71 (s, 2H), 7.61 - 7.53 (m, 1H), 7.44 (d, *J* = 8.3 Hz, 1H), 7.31 (d, *J* = 8.5 Hz, 1H), 6.79 (s, 1H), 6.51 (s, 2H), 5.54 (d, *J* = 14.7 Hz, 1H), 4.67 (d, *J* = 14.8 Hz, 1H), 3.24 (s, 3H), 2.21 (s, 3H). |
| 223 | 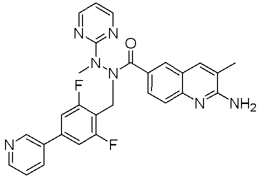 | 512.0 | |

### Example 224: Preparation of 2-amino-N-((5-(3,3-difluoropyrrolidin-1-yl)pyridin-2-yl)methyl)-N',3-dimethyl-N'-(pyr imidin-2-yl)quinoline-6-carbohydrazide (224)

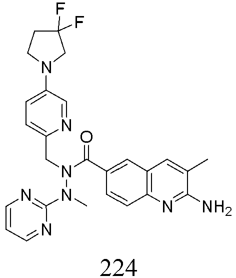

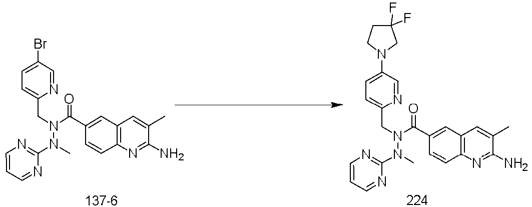

### Step 1: Preparation of 2-amino-N-((5-(3,3-difluoropyrrolidin-1-yl)pyridin-2-yl)methyl)-N',3-dimethyl-N'-(pyr imidin-2-yl)quinoline-6-carbohydrazide (224)

Compound 137-6 (100 mg, 0.21 mmol) was dissolved in 1,4-dioxane (2 mL) in a sealing tube, followed by the addition of 3,3-difluoropyrrolidine (45.00 mg, 0.31 mmol), Brettphos Pd G3 (19.0 mg, 0.02 mmol) and cesium carbonate (204 mg, 0.63 mmol) successively, and after purging with N₂, the resulting mixed solution was stirred at 100°C for 12 hours. Ethyl acetate (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 224 (0.8 mg, yield 1%, a white solid).

LC-MS (ESI+): 505.2 m/z [M+H]⁺.

The following compound was obtained according to the synthetic method in Example 224, using corresponding raw materials.

| Example | Structure | Mass spectrometry data (ESI+): m/z | Nuclear magnetic data |
|---|---|---|---|
| 225 | 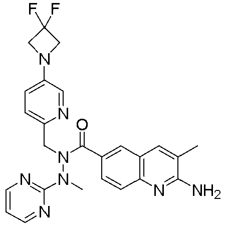 | 491.2 | |

### Example 226: Preparation of 2-amino-N-((5-(4-(methoxymethyl)thiazol-5-yl)pyridin-2-yl)methyl)-N',3-dimethyl-N'-(pyrimidin-2-yl)quinoline-6-carbohydrazide (226)

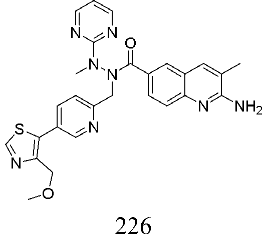

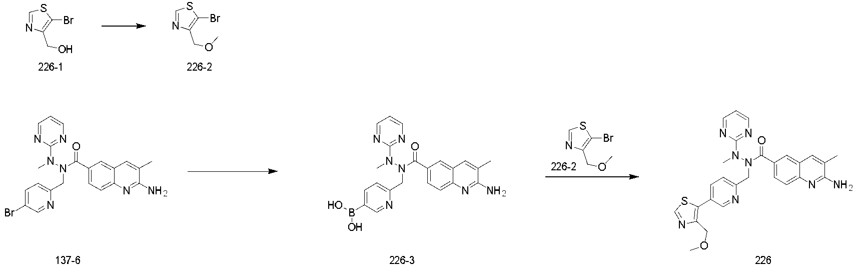

### Step 1: Preparation of 5-bromo-4-methoxymethylthiazole (226-2)

(5-Bromothiazol-4-yl)methanol (226-1) (60 mg, 0.31 mmol) was dissolved in 5 mL of tetrahydrofuran at room temperature, followed by the addition of sodium hydride (60%, 12 mg, 0.46 mmol). The mixture was reacted for 30 minutes in an ice bath, followed by slow addition of iodomethane (88 mg, 0.62 mmol), and then reacted at room temperature for 1 hour. The reaction mixture was quenched by pouring into water (20 mL), and then extracted with ethyl acetate (30 mL x2). The organic phases were combined, dried over anhydrous sodium sulfate and filtered, and the filtrate was concentrated to dryness under reduced pressure to obtain Compound 226-2 (60 mg, yield 94%), which was directly used for the next reaction step without purification.

LC-MS (ESI+): 208.2 m/z [M+H]⁺.

### Step 2: Preparation of (6-((1-(2-amino-3-methylquinoline-6-carbonyl)-2-methyl-2-(pyrimidin-2-yl)hydrazino) methyl)pyridin-3-yl)boronic acid (226-3)

Compound 137-6 (200 mg, 0.42 mmol) was dissolved in 5 mL of dioxane at room temperature, followed by the addition of bis(pinacolato)diboron (212 mg, 0.84 mmol), potassium acetate (123 mg, 1.25 mmol) and 1,1'-bis(diphenylphosphino)ferrocene]palladium dichloridedichloromethane complex (30 mg, 0.04 mmol). The reaction was carried out at 90°C overnight. After completion, the reaction solution was cooled to room temperature and then directly used for the next reaction step without treatment.

LC-MS (ESI+): 444.2 m/z [M+H]⁺.

### Step 3: Preparation of 2-amino-N-((5-(4-(methoxymethyl)thiazol-5-yl)pyridin-2-yl)methyl)-N',3-dimethyl-N'-(pyrimidin-2-yl)quinoline-6-carbohydrazide (226)

Cesium carbonate (300 mg, 0.92 mmol), Compound 226-2 (60 mg, 0.29 mmol), 1,1'-bis(diphenylphosphino)ferrocene]palladium dichloridedichloromethane complex (30 mg, 0.04 mmol) and 0.5 mL of water were added to the reaction solution from the previous step at room temperature. The reaction was carried out at 100°C under nitrogen atmosphere for 2 hours, and the reaction solution was filtered through Celite. The filtrate was concentrated under reduced pressure. The resulting residues were purified by preparative high performance liquid chromatography (water/acetonitrile=95%/5% - 10%/90%) to obtain Compound 226 (1.6 mg, yield 1.1%).

LC-MS (ESI+): 527.2 m/z [M+H]⁺.

¹H NMR (400 MHz, DMSO-d₆) δ 9.16 (s, 1H), 8.68 (s, 1H), 8.40 (d, J = 4.8Hz, 2H), 7.98 (dd, J = 9.2, 2.4 Hz, 1H), 7.78 - 7.73 (m, 3H), 7.50 (d, J = 8.8 Hz, 1H), 7.33 (d, J = 8.4Hz, 1H), 6.79 (t, J = 4.8 Hz, 1H), 6.51 (s, 2H), 5.42 (d, J = 15.2 Hz, 1H), 4.56 (d, J = 15.2 Hz, 1H), 4.49 (s, 2H), 3.32 (s, 3H), 3.28 (s, 3H), 2.19 (s, 3H).

The following compounds were obtained according to the synthetic method in Example 226 using corresponding raw materials.

| Example | Structure | Mass spectrometry data (ESI+): m/z | Nuclear magnetic data |
|---|---|---|---|
| 227 | 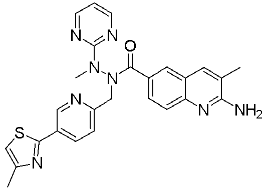 | 497.2 | |
| 228 | 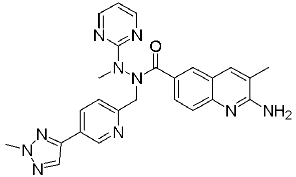 | 481.2 | |
| 229 | 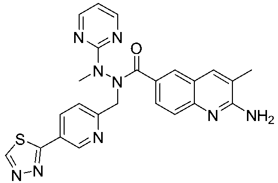 | 484.2 | ¹H NMR (400 MHz, DMSO-*d₆*) δ9.73 (s, 1H), 9.14 (s, 1H), 8.49 - 8.38 (m, 3H), 7.86 - 7.67 (m, 3H), 7.56 - 7.28 (m, 2H), 6.87 - 6.77 (m, 1H), 6.50 (s, 2H), 5.47 (d, J = 15.4 Hz, 1H), 4.56 (d, J = 15.4 Hz, 1H), 3.28 (s, 3H), 2.19 (s, 3H). |
| 230 | 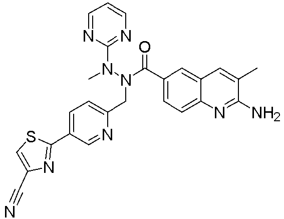 | 508.2 | ¹H NMR (400 MHz, DMSO-*d₆*) δ 9.10 (s, 1H), 8.98 (s, 1H), 8.41-8.37 (m, 3H), 7.78 (d, *J* = 8.0 Hz, 1H), 7.74 (s, 1H), 7.69 (s, 1H), 7.47 (d, *J* = 8.0 Hz, 1H), 7.30 (d, *J* = 8.0 Hz, 1H), 6.79 (s, 1H), 6.47 (s, 2H), 5.43 (d, *J =* 16.0 Hz, 1H), 4.54 (d, *J* = 16.0 Hz, 1H), 3.25 (s, 3H), 2.17 (s, 3H). |
| 231 | 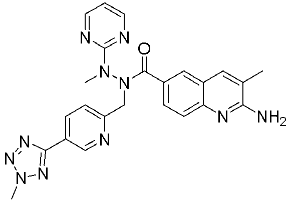 | 482.3 | ¹HNMR (400 MHz, DMSO-*d*₆) δ 9.16 (s, 1H), 8.45 - 8.35 (m, 3H), 7.86 - 7.67 (m, 3H), 7.49 (d, *J* = 8.8 Hz, 1H), 7.32 (d, *J* = 8.8 Hz, 1H), 6.80 (s, 1H), 6.49 (s, 2H), 5.47 (d, *J* = 15.2 Hz, 1H), 4.56 (d, *J* = 15.6 Hz, 1H), 4.48 (s, 3H), 3.26 (s, 3H), 2.19 (s, 3H). |
| 232 | 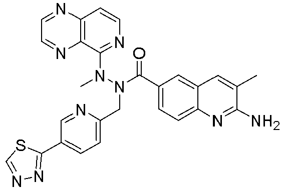 | 535.5 | ¹H NMR (400 MHz, DMSO-*d*₆) δ 9.73 (s, 1H), 9.03 - 8.98 (m, 2H), 8.88 (d, *J* = 1.8 Hz, 1H), 8.37 - 8.28 (m, 2H), 7.85 (d, *J* = 2.1 Hz, 1H), 7.75 (d, *J* = 8.2 Hz, 1H), 7.68 (s, 1H), 7.62 - 7.55 (m, 1H), 7.32-7.26 (m, 2H), 6.44 (s, 2H), 5.42 (d, *J* = 15.7 Hz, 1H), 4.66 (d, *J* = 15.6 Hz, 1H), 3.65 (s, 3H), 2.16 (s, 3H). |
| 233 | 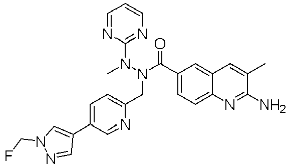 | 498.2 | ¹H NMR (400 MHz, DMSO-*d₆*) δ 8.82 (s, 1H), 8.62 (s, 1H), 8.44 - 8.38 (m, 2H), 8.25 (s, 1H), 8.06 - 8.00 (m, 1H), 7.75 - 7.70 (m, 1H), 7.70 - 7.66 (m, 1H), 7.61 - 7.55 (m, 1H), 7.47 - 7.42 (m, 1H), 7.34 - 7.26 (m, 1H), 6.83 - 6.74 (m, 1H), 6.45 (s, 2H), 6.26 (s, 1H), 6.12 (s, 1H), 5.42 (d, *J* = 14.8 Hz, 1H), 4.42 (d, *J* = 14.8 Hz, 1H), 3.16 (s, 3H), 2.17 (s, 3H). |
| 234 | 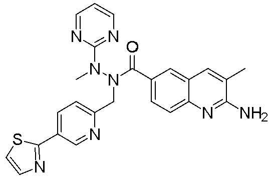 | 483.2 | |
| 235 | 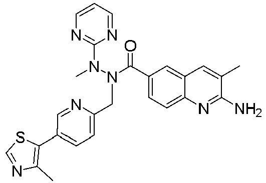 | 497.2 | ¹H NMR (400 MHz, DMSO-*d₆*) δ 9.10 (s, 1H), 8.65 (s, 1H), 8.41 (d, J = 4.8 Hz, 2H), 7.98 - 7.92 (m, 1H), 7.78 - 7.69 (m, 3H), 7.50 (d, J = 8.8 Hz, 1H), 7.32 (d, J = 8.6 Hz, 1H), 6.83 - 6.76 (m, 1H), 6.54 (s, 2H), 5.41 (d, J = 15.2 Hz, 1H), 4.55 (d, J = 15.2 Hz, 1H), 3.28 (s, 3H), 2.49 (s, 3H), 2.19 (s, 3H). |
| 236 | 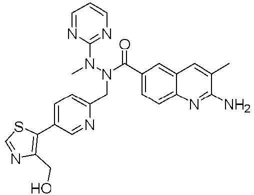 | 513.0 | |

### Example 237: Preparation of 2-amino-N',3-dimethyl-N'-(pyrimidin-2-yl)-N-((5-(4-(trifluoromethyl)thiazol-5-yl)pyrid in-2-yl)methyl)quinoline-6-carbohydrazide (237)

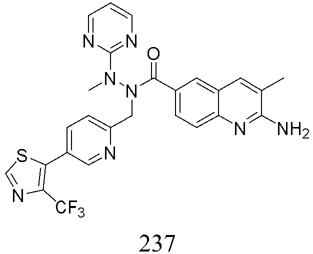

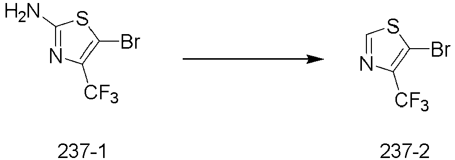

### Step 1: Preparation of 5-bromo-4-trifluoromethylthiazole (237-2)

5-Bromo-4-(trifluoromethyl)thiazol-2-amine (237-1) (60 mg, 0.24 mmol) was dissolved in 5 mL of tetrahydrofuran at room temperature, followed by the addition of tert-butyl nitrite (50 mg, 0.49 mmol). The reaction was carried out at 70°C under nitrogen atmosphere overnight. The reaction mixture was quenched by pouring with water (20.0 mL) and then extracted with ethyl acetate (30 mL x2). The organic phases were combined, dried over anhydrous sodium sulfate and filtered. The filtrate was concentrated to dryness under reduced pressure, and the resulting crude product 237-2 was directly used for the next reaction step without purification. LC-MS (ESI+): 232.2 m/z [M+H]⁺.

2-Amino-N',3-dimethyl-N'-(pyrimidin-2-yl)-N-((5-(4-(trifluoromethyl)thiazol-5-yl )pyridin-2-yl)methyl)quinoline-6-carbohydrazide was obtained according to the method in Step 3 of Example 226, except that compound 237-2 was used instead of 226-2.

LC-MS (ESI+): 551.2 m/z [M+H]⁺.

### Example 238: Preparation of 2-amino-N'-cyclopropyl-3-methyl-N-((5-(4-methylthiazol-5-yl-2d)pyridin-2-yl)methyl)-N'-(pyrimidin-2-yl)quinoline-6-carbohydrazide (238)

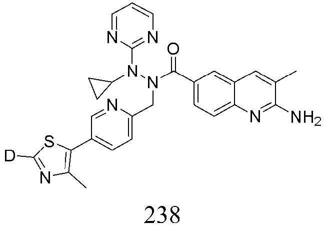

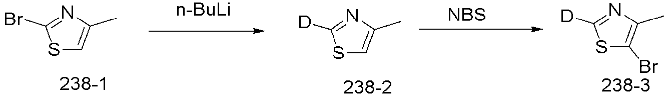

### Step 1: Preparation of 4-methylthiazole-2d (238-2)

2-Bromo-4-methylthiazole (238-1) (1500 mg, 8.42 mmol) was dissolved in THF (6 mL), followed by the addition of n-butyllithium (647 mg, 10.1 mmol) at -78°C. The mixture was reacted at -78°C for 1 hour, followed by the addition of deuterated methanol (6 mL) to the reaction solution. The mixture was slowly warmed up to room temperature and reacted for 1 hour. After the reaction was completed, water (25 mL) was added to the reaction mixture, and the reaction mixture was extracted with dichloromethane (40 mL). The organic phases were combined, washed with saturated brine (20 mL), dried over anhydrous sodium sulfate and filtered. The filtrate was directly used for the next step due to a relatively low boiling point of 238-2.

LC-MS (ESI+): 101.1 m/z [M+H]⁺.

### Step 2: Preparation of 5-bromo-4-methylthiazole-2d (238-3)

NBS (1332 mg, 7.5 mmol) and AcOH (10 mL) were added to the filtrate from the previous step, and reacted at room temperature for 12 hours. After the reaction was completed, water (25 mL) was added to the reaction mixture. The reaction mixture was extracted with ethyl acetate (30 mL x2). The organic phases were combined, washed with saturated brine (30 mL), dried over anhydrous sodium sulfate and filtered. The filtrate was concentrated to dryness under reduced pressure. The crude product was purified by silica gel column chromatography (eluent: ethyl acetate/petroleum ether=1/5) to obtain 238-3 (500 mg, yield 33%) as a yellow oil liquid. LC-MS (ESI+): 179.1 m/z [M+H]⁺.

2-Amino-N'-cyclopropyl-3-methyl-N-((5-(4-methylthiazol-5-yl-2d)pyridin-2-yl)m ethyl)-N'-(pyrimidin-2-yl)quinoline-6-carbohydrazide was obtained according to the method in Step 3 of Example 226, except that Compound 238-3 was used instead of Compound 226-2.

LC-MS (ESI+): 524.1 m/z [M+H]⁺.

¹H NMR (400 MHz, DMSO-*d*₆) δ 8.60-8.52 (m, 3H), 7.98-8.92 (m, 1H), 7.84 - 7.78 (m, 2H), 7.75 (d, *J* = 8.0 Hz, 1H), 7.56-8.50 (m, 1H), 7.39 (d, *J* = 8.8 Hz, 1H), 7.23 (s, 2H), 7.04-6.96 (m, 1H), 5.34 (d, *J* = 15.2 Hz, 1H), 4.66 (d, *J* = 15.2 Hz, 1H), 2.82-2.74 (m, 1H), 2.47 (s, 3H), 2.22 (s, 3H), 0.79-0.72 (m, 1H), 0.58 - 0.40 (m, 2H), 0.15-0.10 (m, 1H).

### Example 239: Preparation of 2-amino-N'-cyclopropyl-N-((5-(4-(difluoromethyl)thiazol-5-yl)pyridin-2-yl)methyl)-3-methyl-N'-(pyrimidin-2-yl)quinoline-6-carbohydrazide (239)

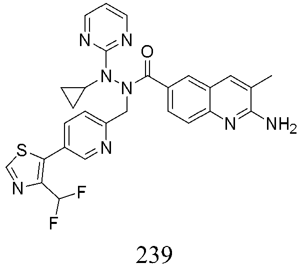

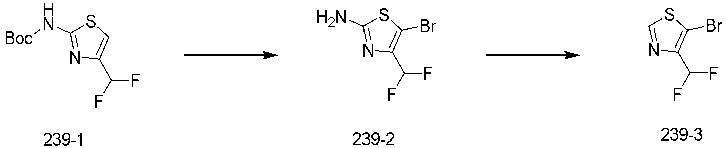

### Step 1: Preparation of 5-bromo-4-difluoromethylthiazol-2-amine (239-2)

Tert-butyl (4-(difluoromethyl)thiazol-2-yl)carbamate (239-1) (250 mg, 1.00 mmol) and N-bromosuccinimide (213 mg, 1.20 mmol) were dissolved in acetic acid (2 mL) at room temperature. The resulting mixture was heated to 80°C, and further reacted under stirring for 6 hours. The reaction mixture was poured into sodium bicarbonate saturated aqueous solution (10 mL), and extracted with dichloromethane (3x20 mL). The organic phases were combined, washed with brine (10 mL), and dried over anhydrous sodium sulfate. The filtrate was collected through filtration, concentrated under reduced pressure, and purified by silica gel column (petroleum ether: ethyl acetate= 1: 1) to obtain Compound 239-2 (200 mg, yield 61%).

LC-MS (ESI⁺): 229.0 m/z [M+H]⁺.

### Step 2: Preparation of 5-bromo-4-difluoromethylthiazol-2-amine (239-3)

Compound 239-2 (100 mg, 0.44 mmol) and isoamyl nitrite (153 mg, 1.31 mmol) were dissolved in 2 mL of 1,4-dioxane (2 mL) at room temperature. The resulting mixture was heated to 100°C under stirring and reacted for 2 h. The reaction mixture was cooled to room temperature, poured into water, and extracted with ethyl acetate (3x20 mL). The organic phases were combined, washed with brine (20 mL), and dried over anhydrous sodium sulfate. After filtration, the filtrate was collected and concentrated under reduced pressure. The residues were purified by silica gel chromatography (petroleum ether: ethyl acetate=1: 1) to obtain Compound 239-3 (14.5 mg, yield 15%).

LC-MS (ESI+): 214.0 m/z [M+H]⁺.

2-Amino-N'-cyclopropyl-N-((5-(4-(difluoromethyl)thiazol-5-yl)pyridin-2-yl)methy 1)-3-methyl-N'-(pyrimidin-2-yl)quinoline-6-carbohydrazide was obtained according to the method in Step 3 of Example 226, except that Compound 239-3 was used instead of Compound 226-2.

LC-MS (ESI+): 559.0 m/z [M+H]⁺.

¹H NMR (400 MHz, DMSO-*d₆*) δ 9.36 - 9.26 (m, 1H), 8.67 - 8.54 (m, 3H), 8.00 - 7.94 (m, 1H), 7.87 - 7.80 (m, 1H), 7.72 (d, *J* = 1.7 Hz, 1H), 7.61 (s, 1H), 7.46 - 7.40 (m, 1H), 7.34 - 7.23 (m, 1H),7.11 (t, *J* = 53.2 Hz, 1H), 7.03 (t, *J* = 4.8 Hz, 1H), 6.52 (s, 2H), 5.39 (d, *J* = 15.4 Hz, 1H), 4.63 (d, *J* = 15.4 Hz, 1H), 2.80 - 2.72 (m, 1H), 2.18 (s, 3H), 0.82 - 0.65 (m, 1H), 0.57 - 0.36 (m, 2H), 0.20 - 0.06 (m, 1H).

### Example 240: Preparation of 2-amino-N'-cyclopropyl-N-((5-(4-cyclopropylthiazol-5-yl)pyridin-2-yl)methyl)-3-meth yl-N'-(pyrimidin-2-yl)quinoline-6-carbohydrazide (240)

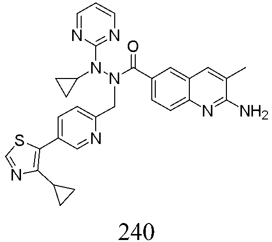

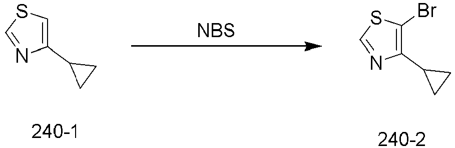

### Step 1: Preparation of 5-bromo-4-cyclopropylthiazole (240-2)

4-Cyclopropylthiazole (240-1, 200 mg, 1.59 mmol) and NBS (369 mg, 2.07 mmol) were dissolved in N,N-dimethylformamide (10 mL) at room temperature, and reacted at room temperature for 1 hour, and the resulting reaction mixture was diluted with water (50 mL). The resulting mixture was extracted with ethyl acetate (50 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 240-2 (300 mg, yield 92%). LC-MS (ESI+): 204.0 m/z [M+H]⁺.

2-Amino-N'-cyclopropyl-N-((5-(4-cyclopropylthiazol-5-yl)pyridin-2-yl)methyl)-3-methyl-N'-(pyrimidin-2-yl)quinoline-6-carbohydrazide was obtained according to the method in Step 3 of Example 226, except that Compound 240-2 was used instead of Compound 226-2.

LC-MS (ESI+): 549.2 m/z [M+H]⁺.

¹H NMR (400 MHz, DMSO-*d₆*) δ 9.01 (s, 1H), 8.59 (d, *J* = 1.6 Hz, 1H), 8.56 (d, *J* = 4.0 Hz, 2H), 7.96-7.99 (m, 1H), 7.76 (d, *J* = 8.0 Hz, 1H), 7.69 (s, 1H), 7.58 (s, 1H), 7.40 (d, *J* = 8.0 Hz, 1H), 7.25 (d, *J* = 8.0 Hz, 1H), 6.97-6.99 (m, 1H), 6.48 (s, 2H), 5.32 (d, *J* = 16.0 Hz, 1H), 4.63 (d, *J* = 16.0 Hz, 1H), 2.76 (s, 1H), 2.15 (s, 3H), 2.01-2.02 (m, 1H), 0.93-0.97 (m, 4H), 0.71 (s, 1H), 0.42-0.48 (m, 2H), 0.10 (s, 1H).

### Example 241: Preparation of 2-amino-N-((5-(4-ethylthiazol-5-yl)pyridin-2-yl)methyl)-N',3-dimethyl-N'-(pyrimidin-2 -yl)quinoline-6-carbohydrazide (241)

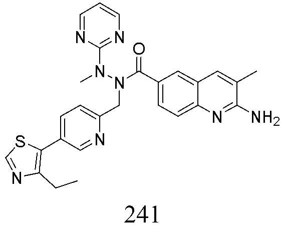

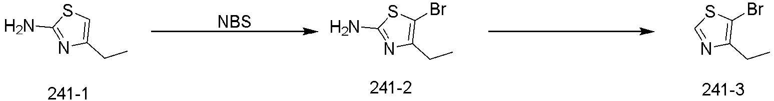

### Step 1: Preparation of 5-bromo-4-ethylthiazole-2-amino (241-2)

4-Ethylthiazol-2-amine (241-1, 500 mg, 3.90 mmol) and NBS (833 mg, 4.68 mmol) were dissolved in acetic acid (10 mL) at room temperature, and reacted at room temperature for 12 hours, and the resulting reaction mixture was diluted with water (50 mL). The resulting mixture was extracted with ethyl acetate (50 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 241-2 (800 mg, yield 99%).

LC-MS (ESI+): 207.0 m/z [M+H]⁺.

### Step 2: Preparation of 5-bromo-4-ethylthiazole (241-3)

Compound 241-2 (800 mg, 3.86 mmol) and tert-butyl nitrite (800 mg, 11.6 mmol) were dissolved in tetrahydrofuran (10 mL) at room temperature, and reacted at 60°C for 12 hours. The resulting reaction mixture was diluted with water (60 mL). The resulting mixture was extracted with ethyl acetate (60 mL x 3). The organic phases were combined, washed with saturated brine (120 mL), dried over anhydrous sodium sulfate and filtered. The filtrate was concentrated under reduced pressure. The resulting residues were purified by silica gel column chromatography (eluent: dichloromethane/methanol=10/1) to obtain Compound 241-3 (500 mg, yield 67%).

LC-MS (ESI+): 192.0 m/z [M+H]⁺.

2-Amino-N-((5-(4-ethylthiazol-5-yl)pyridin-2-yl)methyl)-N',3-dimethyl-N'-(pyrimi din-2-yl)quinoline-6-carbohydrazide was obtained according to the method in Step 3 of Example 226, except that Compound 241-3 was used instead of Compound 226-2.

LC-MS (ESI+): 511.2 m/z [M+H]⁺.

¹H NMR (400 MHz, DMSO-*d₆*) δ 9.11 (s, 1H), 8.57 (s, 1H), 8.38 (d, *J =* 4.0 Hz, 2H), 7.88 (d, *J* = 4.0 Hz, 1H), 7.74 (s, 1H), 7.69 (s, 2H), 7.47 (d, *J* = 8.0 Hz, 1H), 7.29 (d, *J* = 8.0 Hz, 1H), 6.77 (s, 1H), 6.47 (s, 2H), 5.37 (d, *J* = 16.0 Hz, 1H), 4.54 (d, *J* = 16.0 Hz, 1H), 3.27 (s, 3H), 2.74 (q, *J =* 8.0 Hz, 2H), 2.17 (s, 3H), 1.24 (t, *J =* 8.0 Hz, 3H).

### Example 242: Preparation of 2-amino-N-((5-(4-(fluoromethyl)thiazol-5-yl)pyridin-2-yl)methyl)-N',3-dimethyl-N'-(p yrimidin-2-yl)quinoline-6-carbohydrazide (242)

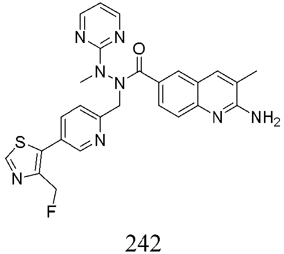

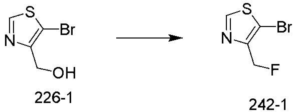

### Step 1: Preparation of 5-bromo-4-(fluoromethyl)thiazole (242-1)

(5-Bromothiazol-4-yl)methanol (226-1, 100 mg, 0.515 mmol) was dissolved in DCM (5 mL), followed by the slow addition of DAST (249 mg, 1.54 mmol) at -20°C. The mixture was reacted under stirring at room temperature overnight. After the reaction was completed, the reaction mixture was quenched with an appropriate amount of sodium bicarbonate saturated aqueous solution, and extracted with DCM (3x10 mL). The organic phases were collected, washed with saturated brine (20 mL), dried over anhydrous sodium sulfate and filtered. The filtrate was concentrated under reduced pressure to obtain a crude product 242-1 which was directly used for the next step.

LC-MS (ESI+): 196.2 m/z [M+H]⁺.

2-Amino-N-((5-(4-(fluoromethyl)thiazol-5-yl)pyridin-2-yl)methyl)-N',3-dimethyl-N'-(pyrimidin-2-yl)quinoline-6-carbohydrazide was obtained according to the method in Step 3 of Example 226, except that Compound 242-1 was used instead of Compound 226-2.

LC-MS (ESI+): 515.6 m/z [M+H]⁺.

¹H NMR (400 MHz, DMSO-*d*₆) δ 9.22 (s, 1H), 8.64 (s, 1H), 8.39 (d, *J* = 4.8 Hz, 2H), 8.17 (s, 1H), 7.96 (dd, *J=* 8.1, 2.4 Hz, 1H), 7.77 - 7.67 (m, 2H), 7.48 (d, *J=* 8.8 Hz, 1H), 7.30 (d, *J* = 8.6 Hz, 1H), 6.76-6.79 (m, 1H), 6.45 (s, 2H), 5.52 (s, 1H), 5.43 - 5.37 (m, 2H), 4.55 (d, *J* = 15.5 Hz, 1H), 3.28 (s, 3H), 2.17 (s, 3H).

### Example 243: Preparation of 2-amino-N-((5-(4-cyanothiazol-5-yl)pyridin-2-yl)methyl)-N',3-dimethyl-N'-(pyrimidin-2-yl)quinoline-6-carbohydrazide (243)

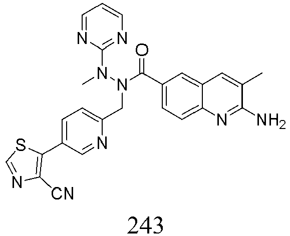

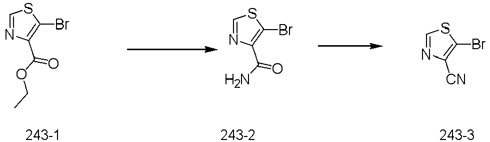

### Step 1: Preparation of 5-bromothiazole-4-carboxamide (243-2)

Ethyl 5-bromothiazole-4-carboxylate (243-1, 2 g, 8.47 mmol) was dissolved in 20 mL of ammonia water (20 mL), and reacted under stirring at room temperature overnight. After the reaction was completed, the reaction mixture was concentrated under reduced pressure to obtain a crude product 243-2 which was directly used for the next step.

LC-MS (ESI+): 207.2 m/z [M+H]⁺.

### Step 2: Preparation of 5-bromothiazole-4-carbonitrile (243-3)

Compound 243-2 (500 mg, 2.44 mmol) was dissolved in DCM (5 mL), followed by the addition of TEA (1.24 g, 12.2 mmol) at 0°C, then TFAA (2.57 g, 12.2 mmol) was added slowly. The reaction was carried out under stirring at 0°C for 2h. After the reaction was completed, the reaction mixture was diluted with an appropriate amount of water, and extracted with DCM (3x20 mL). The organic phases were collected, washed with saturated brine (20 mL), dried over anhydrous sodium sulfate, and filtered. The filtrate was concentrated under reduced pressure to obtain a crude product 243-3 which was directly used for the next step.

LC-MS (ESI+): 189.3 m/z [M+H]⁺.

2-Amino-N-((5-(4-cyanothiazol-5-yl)pyridin-2-yl)methyl)-N',3-dimethyl-N'-(pyri midin-2-yl)quinoline-6-carbohydrazide was obtained according to the method in Step 3 of Example 226, except that 243-3 was used instead of 226-2.

LC-MS (ESI+): 508.6 m/z [M+H]⁺.

¹H NMR (400 MHz, DMSO-*d*₆) δ 9.35 (s, 1H), 8.88 (s, 1H), 8.40 (d, *J* = 4.7 Hz, 2H), 8.21 (dd, *J* = 8.2, 2.5 Hz, 1H), 8.13 (s, 1H), 7.86 - 7.79 (m, 3H), 7.55 (d, *J* = 8.6 Hz, 1H), 7.37 (d, *J* = 8.6 Hz, 1H), 6.86 - 6.76 (m, 2H), 5.41 (d, *J* = 15.5 Hz, 1H), 4.57 (d, *J=* 15.7 Hz, 1H), 3.30 (s, 3H), 2.20 (s, 3H).

### Example 244: Preparation of 2-amino-N',3-dimethyl-N'-(pyrimidin-2-yl)-N-((5-(2-(trifluoromethyl)thiazol-5-yl)pyrid in-2-yl)methyl)quinoline-6-carbohydrazide (244)

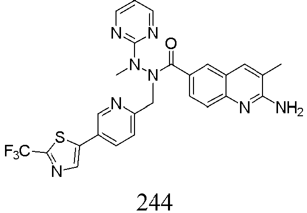

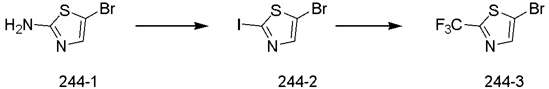

### Step 1: Preparation of 5-bromo-2-iodothiazole (244-2)

5-Bromothiazol-2-amine (244-1, 1.0 g, 3.8 mmol) was dissolved in THF (10 mL), followed by the addition of diiodomethane (1.2 g, 4.6 mmol) and 3-methylbutyl nitrite (0.675 g, 5.76 mmol) successively, and the resulting mixture was stirred at room temperature for 18 hours. The reaction solution was concentrated under reduced pressure. The residues were purified by silica gel column chromatography (petroleum ether/ethyl acetate = 99/1) to obtain Compound 244-2 (700 mg, yield 63%, a yellow oil).

LC-MS (ESI+): 289.8 m/z [M+H]⁺.

### Step 2: Preparation of 5-bromo-2-(trifluoromethyl)thiazole (244-3)

Compound 244-2 (0.2 g, 0.69 mmol) was dissolved in DMF (2 mL), followed by the addition of cuprous iodide (0.2 g, 1.03 mmol) and methyl 2,2-difluoro-2-(fluorosulfonyl)acetate (0.2 g, 1.03 mmol) under nitrogen atmosphere, and the resulting mixture was stirred at 70°C for 12 hours. Water (20 mL) was added to the reaction solution, and the reaction solution was extracted with EtOAc (20 mL × 3). The organic phases were combined, washed with saturated brine, dried over anhydrous sodium sulfate and filtered. The filtrate was concentrated under reduced pressure to obtain Compound 244-3 (100 mg, yield 62%, a yellow oil).

2-Amino-N',3-dimethyl-N'-(pyrimidin-2-yl)-N-((5-(2-(trifluoromethyl)thiazol-5-yl )pyridin-2-yl)methyl)quinoline-6-carbohydrazide was obtained according to the method in Step 3 of Example 226, except that Compound 244-3 was used instead of Compound 226-2.

LC-MS (ESI+): 551.3 m/z [M+H]⁺.

### Example 245: Preparation of N-((5-(2H-tetrazol-5-yl)pyridin-2-yl)methyl)-2-amino-N',3-dimethyl-N'-(pyrimidin-2-y 1)quinoline-6-carbohydrazide (245)

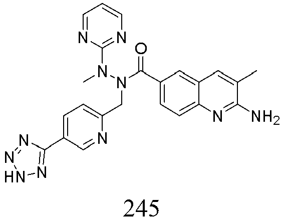

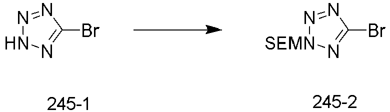

### Step 1: Preparation of 5-bromo-2-((2-(trimethylsilyl)ethoxy)methyl)-2H-tetrazole (245-2)

5-Bromo-2H-tetrazole (245-1, 0.2 g, 1.34 mmol) was dissolved in DMF (5 mL), followed by the addition of sodium hydride (60%, 80 mg, 2.01 mmol) at 0°C. The mixture was stirred for 0.5 hour, followed by the addition of 2-(trimethylsilyl)ethoxymethyl chloride (0.22 g, 1.34 mmol), and the resulting mixture was stirred at room temperature for 12 hours. Water (100 mL) was added to the reaction solution, and the reaction solution was extracted with DCM (100 mL × 3). The organic phases were combined, washed with saturated brine, dried over anhydrous sodium sulfate and filtered. The filtrate was concentrated under reduced pressure to obtain Compound 245-2 (a mixture of isomers, 200 mg, yield 53%, a yellow oil).

LC-MS (ESI+): 279.1 m/z [M+H]⁺.

Remaining steps were according to the synthetic methods in Step 3 of Example 226 and Step 5 of Example 119 to give N-((5-(2H-tetrazol-5-yl)pyridin-2-yl)methyl)-2-amino-N',3-dimethyl-N'-(pyrimidin-2-y 1)quinoline-6-carbohydrazide.

LC-MS (ESI+): 468.3 m/z [M+H]⁺.

¹H NMR (400 MHz, DMSO-*d₆*) δ 9.11 (s, 1H), 8.41 (d, *J* = 4.8 Hz, 2H), 8.36 (dd, *J* = 8.0, 2.4 Hz, 1H), 7.82 (d, *J* = 10.8 Hz, 2H), 7.76 (d, *J* = 8.0 Hz, 1H), 7.55 (d, *J* = 8.8 Hz, 1H), 7.38 (d, *J* = 8.8 Hz, 1H), 6.95 (s, 2H), 6.79 (t, *J* = 4.8 Hz, 1H), 5.45 (d, *J* = 15.2 Hz, 1H), 4.52 (d, *J=* 15.2 Hz, 1H), 3.22 (s, 3H), 2.20 (s, 3H).

### Example 246: Preparation of 2-amino-N-((6'-((2-hydroxyethyl) (methyl)amino)-[3,3'-bipyridine]-6-yl)methyl)-N',3-dimethyl-N'-(pyrimidin-2-yl)quinoli ne-6-carbohydrazide (246)

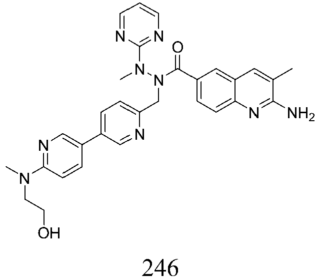

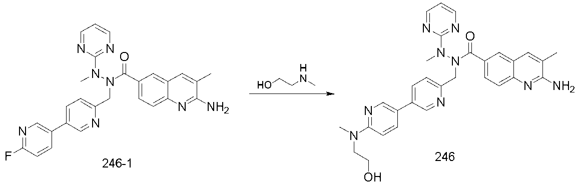

### Step 1: Preparation of 2-amino-N-((6'-((2-hydroxyethyl) (methyl)amino)-[3,3'-bipyridine]-6-yl)methyl)-N',3-dimethyl-N'-(pyrimidin-2-yl)quinoli ne-6-carbohydrazide (246)

2-Amino-N-((6'-fluoro-[3,3'-bipyridine]-6-yl)methyl)-N',3-dimethyl-N'-(pyrimidin -2-yl)quinoline-6-carbohydrazide (246-1, synthesized according to the method in Example 137) (80 mg, 0.16 mmol) and 2-(methylamino)ethanol (0.05 mL) were dissolved in N,N-dimethylformamide (3 mL) at room temperature, followed by the addition of potassium carbonate (45 mg, 0.32 mmol). The reaction solution was heated to 80°C and stirred for 22 hours. The reaction solution was quenched with water (20 mL), and then extracted with dichloromethane (20 mL x3). The organic phases were combined, dried over anhydrous sodium sulfate and filtered. The filtrate was concentrated to dryness under reduced pressure. The residues were purified by preparative high performance liquid chromatography (water/acetonitrile=95%/5% - 10%/90%) to obtain Compound 246 (24 mg, yield 26%). LC-MS (ESI+): 550.3 m/z [M+H]⁺.

The following compound was obtained according to the synthetic method in Example 246 using corresponding raw materials.

| Example | Structure | Mass spectrometry data (ESI+): m/z | Nuclear magnetic data |
|---|---|---|---|
| 247 | 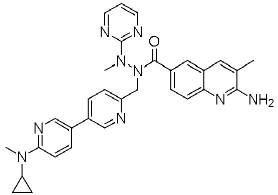 | 546.2 | |

### Example 248: Preparation of 4-amino-N',1-dimethyl-N'-(pyrimidin-2-yl)-N-((5-(1-(trifluoromethyl)-1H-pyrazol-4-yl )pyridin-2-yl)methyl)-1H-pyrazolo[4,3-c]quinoline-8-carbohydrazide (248)

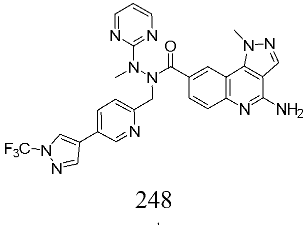

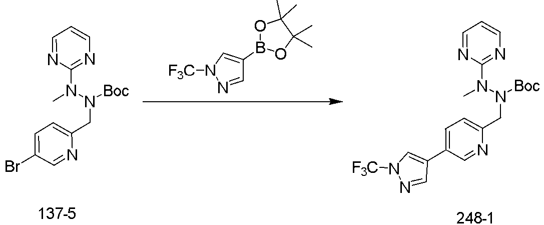

### Step 1: Preparation of tert-butyl 2-methyl-2-(pyrimidin-2-yl)-1-((5-(1-(trifluoromethyl)-1H-pyrazol-4-yl)pyridin-2-yl)m ethyl)hydrazine-1-carboxylate (248-1)

Compound 137-5 (700 mg, 1.78 mmol), 4-(4,4,5,5-tetramethyl-1,3,2-dioxaborolan-2-yl)-1-(trifluoromethyl)-1H-pyrazole (930 mg, 3.55 mmol), [1,1'-bis(diphenylphosphino)ferrocene]palladium dichloride (114 mg, 0.18 mmol) and potassium carbonate (490 mg, 3.55 mmol) were dissolved in dioxane (10 mL) and water (2 mL) at room temperature, and reacted at 100°C for 12 hours . The resulting reaction mixture was diluted with water (100 mL). The resulting mixture was extracted with ethyl acetate (100 mL x 3). The organic phases were combined, washed with saturated brine (100 mL), dried over anhydrous sodium sulfate, and filtered. The filtrate was concentrated under reduced pressure. The resulting residues were purified by silica gel column chromatography (eluent: petroleum ether/ethyl acetate=1/1) to obtain Compound 248-1 (670 mg, yield 84%).

LC-MS (ESI+): 450.2 m/z [M+H]⁺.

4-Amino-N',1-dimethyl-N'-(pyrimidin-2-yl)-N-((5-(1-(trifluoromethyl)-1H-pyrazo 1-4-yl)pyridin-2-yl)methyl)-1Hpyrazolo[4,3-c]quinoline-8-carbohydrazide was synthesized according to Steps 4-5 of Example 137, except that Compound 248-1 was used instead of Compound 137-4.

LC-MS (ESI+): 574.2 m/z [M+H]⁺.

¹H NMR (400 MHz, DMSO-*d₆*) δ 9.12 (s, 1H), 8.93 (s, 1H), 8.57 (s, 1H), 8.48 (s, 2H), 8.22 (m, 1H), 8.16-8.14 (m, 2H), 7.65 (s, 2H), 7.50 (s, 1H), 7.18 (s, 2H), 6.85 (s, 1H), 5.44 (d, *J* = 16.0 Hz, 1H), 4.53 (d, *J* = 16.0 Hz, 1H), 4.12 (s, 3H), 3.17 (s, 3H).

The following compounds were obtained according to the synthetic method in Example 248 using corresponding raw materials.

| Example | Structure | Mass spectrometry data (ESI+): m/z | Nuclear magnetic data |
|---|---|---|---|
| 249 | 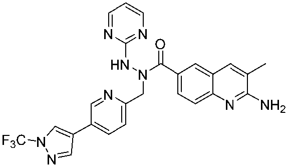 | 520.2 | ¹H NMR (400 MHz, DMSO-*d*₆) δ 10.81 (s, 1H), 9.09 (s, 1H), 8.93 (d, *J* = 1.6 Hz, 1H), 8.55 (s, 1H), 8.47 (d, *J* = 4.8 Hz, 2H), 8.18 (d, *J* = 2.0 Hz, 1H), 8.13 (dd, *J =* 8.0, 2.4 Hz, 1H), 7.90 (dd, *J =* 8.8, 2.0 Hz, 1H), 7.79 (s, 1H), 7.65 (d, *J* = 8.0 Hz, 1H), 7.48 (d, *J* = 8.8 Hz, 1H), 6.85 (t, *J* = 4.8 Hz, 1H), 6.61 (s, 2H), 5.19 (s, 2H), 2.23 (s, 3H). |
| 250 | 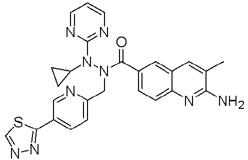 | 510.5 | ¹H NMR (400 MHz, DMSO-*d*₆) δ 9.73 (s, 1H), 9.07 (d, *J* = 2.3 Hz, 1H), 8.62 (d, *J* = 4.8 Hz, 2H), 8.43-8.45 (m, 1H), 7.88 (d, *J* = 8.1 Hz, 1H), 7.72 (d, *J* = 2.0 Hz, 1H), 7.61 (s, 1H), 7.42-7.45 (dd, 1H), 7.28 (d, *J* = 8.7 Hz, 1H), 7.30-7.05 (m, 1H), 6.51 (s, 2H), 5.43 (d, *J =* 15.5 Hz, 1H), 4.62 (d, *J* = 15.5 Hz, 1H), 2.80 - 2.70 (m, 1H), 2.18 (s, 3H), 0.11-0.74 (m, 4H). |
| 251 | 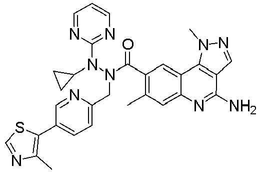 | 577.5 | |
| 252 | 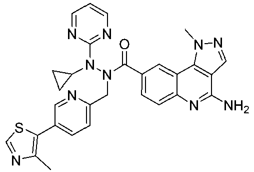 | 563.0 | |
| 253 | 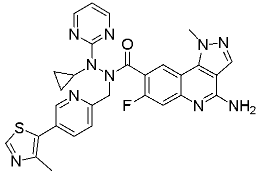 | 581.0 | |

### Example 254: Preparation of 2-amino-N-((6'-((2-methoxyethyl)(methyl)amino)-[3,3'-bipyridine]-6-yl)methyl)-N',3-d imethyl-N'-(pyrimidin-2-yl)quinoline-6-carbohydrazide (254)

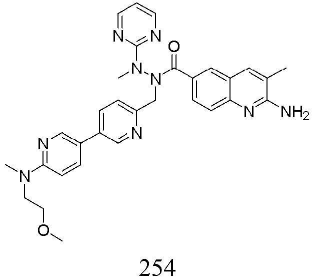

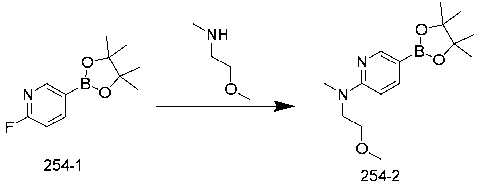

### Step 1: Preparation of N-(2-methoxyethyl)-N-methyl-5-(4,4,5,5-tetramethyl-1,3,2-dioxaborolan-2-yl)pyridin-2 -amine (254-2)

2-Fluoro-5-(4,4,5,5-tetramethyl-1,3,2-dioxaborolan-2-yl)pyridine (254-1, 500 mg, 2.25 mmol), 2-methoxy-N-methylethan-1-amine (401 mg, 4.50 mmol) and potassium carbonate (939 mg, 6.76 mmol) were dissolved in N,N-dimethylformamide (8 mL) solution, and the reaction solution was stirred at 100°C for 10 hours. After the reaction was completed, the reaction solution was quenched with water (20 mL), and then extracted with ethyl acetate (20 mL x3). The organic phases were combined, dried over anhydrous sodium sulfate and filtered. The filtrate was concentrated to dryness under reduced pressure. The resulting crude product was purified by silica gel column chromatography (eluent: ethyl acetate/petroleum ether=1/2) to obtain Compound 254-2 (300 mg, yield 45%).

LC-MS (ESI+): 293.2 m/z [M+H]⁺.

2-Amino-N-((6'-((2-methoxyethyl) (methyl)amino)-[3,3'-bipyridine]-6-yl)methyl)-N',3-dimethyl-N'-(pyrimidin-2-yl)quinol ine-6-carbohydrazide was synthesized according to Step 7 of Example 137, except that Compound 254-2 was used instead of 2-methoxy-6-methylpyridin-3-yl)boronic acid.

LC-MS (ESI+): 564.3 m/z [M+H]⁺.

¹H NMR (400 MHz, DMSO-*d*₆) δ 8.82 - 8.70 (m, 1H), 8.47 (s, 1H), 8.45 - 8.37 (m, 2H), 8.27 (s, 1H), 8.04 - 7.97 (m, 1H), 7.92 - 7.84 (m, 1H), 7.73 (s, 1H), 7.69 (s, 1H), 7.62 - 7.54 (m, 1H), 7.50 - 7.41 (m, 1H), 7.30 (d, *J* = 8.6 Hz, 1H), 6.80 - 6.76 (m, 1H), 6.76 - 6.71 (m, 1H), 6.45 (s, 2H), 5.43 (d, *J* = 14.8 Hz, 1H), 4.43 (d, *J* = 14.8 Hz, 1H), 3.74 (t, *J* = 5.6 Hz, 2H), 3.52 (t, *J* = 5.8 Hz, 2H), 3.26 (s, 3H), 3.20 (s, 3H), 3.07 (s, 3H), 2.17 (s, 3H).

The following compounds were obtained according to the synthetic method in Example 254 using corresponding raw materials.

| Example | Structure | Mass spectrometry data (ESI+): m/z | Nuclear magnetic data |
|---|---|---|---|
| 255 | 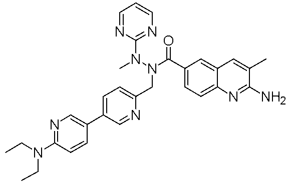 | 548.2 | ¹H NMR (400 MHz, DMSO-*d₆*) δ 8.77 (s, 1H), 8.48 (s, 1H), 8.45 - 8.38 (m, 2H), 8.06 - 7.99 (m, 1H), 7.90 - 7.83 (m, 1H), 7.75 (s, 1H), 7.71 (s, 1H), 7.59 (d, *J* = 8.0 Hz, 1H), 7.48 (d, *J* = 8.4 Hz, 1H), 7.31 (d, *J* = 8.4 Hz, 1H), 6.86 - 6.75 (m, 1H), 6.71 (d, *J* = 8.8 Hz, 1H), 6.49 (s, 2H), 5.44 (d, *J* = 15.2 Hz, 1H), 4.44 (d, *J=* 15.2 Hz, 1H), 3.55 (q, *J* = 7.6 Hz, 4H), 3.22 (s, 3H), 2.19 (s, 3H), 1.15 (t, *J* = 7.2 Hz, 6H). |
| 256 | 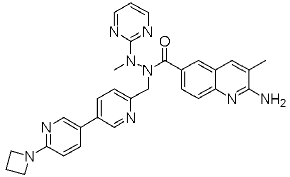 | 532.3 | ¹H NMR (400 MHz, DMSO-*d₆*) δ 8.75 (s, 1H), 8.47 - 8.43 (m, 1H), 8.42 - 8.37 (m, 2H), 8.03 - 7.97 (m, 1H), 7.93 - 7.84 (m, 1H), 7.75 - 7.66 (m, 2H), 7.62 - 7.56 (m, 1H), 7.49 - 7.40 (m, 1H), 7.34 - 7.26 (m, 1H), 6.82 - 6.75 (m, 1H), 6.50 - 6.40 (m, 3H), 5.43 (d, *J* = 15.2 Hz, 1H), 4.43 (d, *J* = 15.2 Hz, 1H), 3.99 (t, *J* = 7.2 Hz, 4H), 3.20 (s, 3H), 2.39 - 2.29 (m, 2H), 2.17 (s, 3H). |
| 257 | 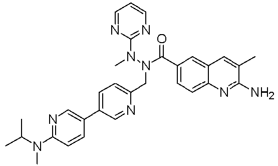 | 548.4 | |
| 258 | 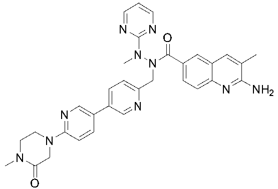 | 589.0 | |
| 259 | 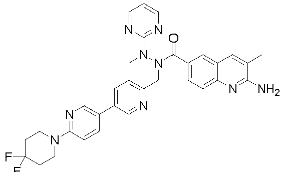 | 596.0 | |

### Example 260: Preparation of 2-amino-N-((6'-((2-(dimethylamino)ethyl)(methyl)amino)-[3,3'-bipyridine]-6-yl)methyl) -N', 3-dimethyl-N'-(pyrimidin-2-yl)quinoline-6-carbohydrazide (260)

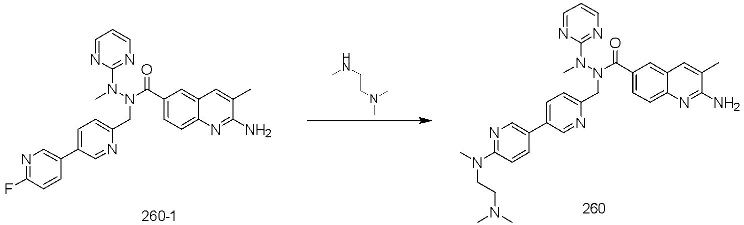

### Step 1: Preparation of 2-amino-N-((6'-((2-(dimethylamino)ethyl) (methyl)amino)-[3,3'-bipyridine]-6-yl)methyl)-N',3-dimethyl-N'-(pyrimidin-2-yl)quinoli ne-6-carbohydrazide (260)

2-Amino-N-((6'-fluoro-[3,3'-bipyridine]-6-yl)methyl)-N',3-dimethyl-N'-(pyrimidin -2-yl)quinoline-6-carbohydrazide (260-1, synthesized according to the method in Example 137) (100 mg, 0.20 mmol), N¹,N¹,N²-trimethylethane-1,2-diamine (55 mg, 0.61 mmol) and potassium carbonate (75 mg, 0.61 mmol) were dissolved in dry dimethylformamide (2 mL) at room temperature. The reaction solution was stirred at 80°C for 12 hour. The reaction solution was quenched with water (20 mL), and then extracted with ethyl acetate (20 mL x2). The organic phases were combined, dried over anhydrous sodium sulfate and filtered. The filtrate was concentrated to dryness under reduced pressure. The resulting crude product was purified by preparative high performance liquid chromatography (water/acetonitrile=95%/5% - 10%/90%) to obtain Compound 260 (27.5 mg, yield 26%).

LC-MS (ESI+): 577.2 m/z [M+H]⁺.

¹H NMR (400 MHz, DMSO-*d*₆) δ 8.77 (s, 1H), 8.50 - 8.38 (m, 3H), 8.01 (d, *J* = 8.4 Hz, 1H), 7.92 - 7.85 (m, 1H), 7.71 (d, *J* = 14.8 Hz, 2H), 7.58 (d, *J* = 8.0 Hz, 1H), 7.46 (d, *J* = 8.8 Hz, 1H), 7.30 (d, *J* = 8.8 Hz, 1H), 6.80 - 6.70 (m, 2H), 6.46 (s, 2H), 5.43 (d, *J* = 15.2 Hz, 1H), 4.43 (d, *J* = 15.2 Hz, 1H), 3.70 (t, *J* = 6.8 Hz, 2H), 3.20 (s, 3H), 3.05 (s, 3H), 2.53 (d, *J* = 7.2 Hz, 2H), 2.26 (s, 6H), 2.17 (s, 3H).

The following compound was obtained according to the synthetic method in Example 260 using corresponding raw materials.

| Example | Structure | Mass spectrometry data (ESI+): m/z | Nuclear magnetic data |
|---|---|---|---|
| 261 | 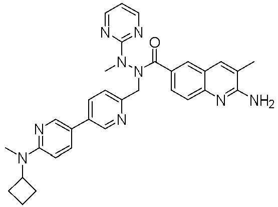 | 560.2 | ¹H NMR (400 MHz, DMSO-*d*₆) δ 8.76 (d, *J* = 2.4 Hz, 1H), 8.48 (d, *J* = 2.4 Hz, 1H), 8.40 (d, *J* = 4.8 Hz, 2H), 8.01 (d, *J* = 8.0 Hz, 1H), 7.88 (d, *J* = 9.2 Hz, 1H), 7.75 (s, 2H), 7.58 (d, *J* = 8.0 Hz, 1H), 7.49 (d, *J* = 8.8 Hz, 1H), 7.33 (d, *J* = 8.8 Hz, 1H), 6.78 (t, *J* = 4.8 Hz, 1H), 6.73 (d, *J* = 8.8 Hz, 1H), 6.66 (s, 2H), 5.42 (d, *J* = 15.2 Hz, 1H), 4.90-4.82 (m, 1H), 4.43 (d, *J* = 15.2 Hz, 1H), 3.21 (s, 3H), 3.00 (s, 3H), 2.23 - 2.15 (m, 7H), 1.70 - 1.63 (m, 2H). |

### Example 262: Preparation of 2-amino-N',3-dimethyl-N'-(pyrimidin-2-yl)-N-((1',2',3',6'-tetrahydro-[3,4'-bipyridine]-6 -yl)methyl)quinoline-6-carbohydrazide (262)

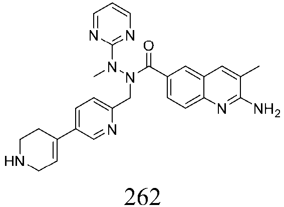

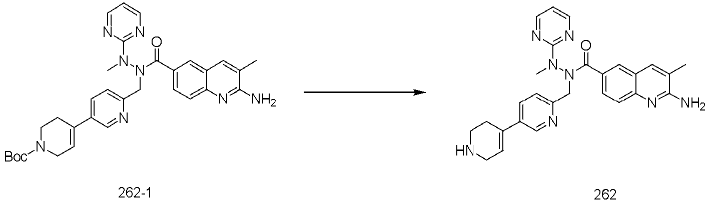

### Step 1: Preparation of 2-amino-N',3-dimethyl-N'-(pyrimidin-2-yl)-N-((1',2',3',6'-tetrahydro-[3,4'-bipyridine]-6 -yl)methyl)quinoline-6-carbohydrazide (262)

Tert-butyl 6-((1-(2-amino-3-methylquinoline-6-carbonyl)-2-methyl-2-(pyrimidin-2-yl)hydrazino) methyl)-3',6'-dihydro-[3,4'-bipyridine]-1'(2'H)-carboxylate (262-1, synthesized according to the route in Example 137) (50 mg, 0.09 mmol) was dissolved in 3 mL of dichloromethane at room temperature, followed by the addition of 4M hydrochloric acid-dioxane solution (1 mL), and reacted at room temperature for 1 hour under nitrogen atmosphere. The reaction mixture was concentrated under reduced pressure, and the residues were purified by preparative high performance liquid chromatography (water/acetonitrile=95%/5% - 10%/90%) to obtain Compound 262 (10 mg, yield 24%).

LC-MS (ESI+): 481.2 m/z [M+H]⁺.

### Example 263: Preparation of 2-amino-N-((5-iodopyridin-2-yl)methyl)-N',3-dimethyl-N'-(pyrimidin-2-yl)quinoline-6-carbohydrazide (263)

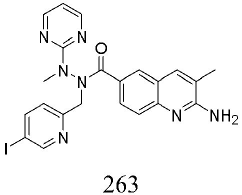

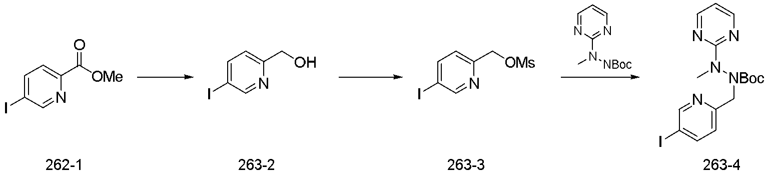

### Step 1: Preparation of (5-iodopyridin-2-yl)methanol (263-2)

Methyl 5-iodopicolinate (262-1, 900 mg, 3.42 mmol) was dissolved in methanol (10 mL) at room temperature, followed by the addition of sodium borohydride (195 mg, 5.1 mmol). The reaction solution was stirred at 25°C for 12 hour. The reaction solution was quenched with water (10 mL), and then extracted with ethyl acetate (10 mL x3). The organic phases were combined, dried over anhydrous sodium sulfate and filtered. The organic phase was concentrated to dryness under reduced pressure to obtain Compound 263-2 (800 mg, yield 99%).

LC-MS (ESI+): 236.0 m/z [M+H]⁺.

### Step 2: Preparation of (5-iodopyridin-2-yl)methylmethanesulfonate (263-3)

Compound 263-2 (800 mg, 3.4 mmol) and diisopropylethylamine (0.8 mL) were dissolved in dichloromethane (10 mL) at room temperature, followed by the addition of methylsulfonyl chloride (0.4 mL). The reaction solution was stirred at 25°C for 1 hour. The reaction solution was quenched with water (20 mL), and then extracted with dichloromethane (20 mL x3). The organic phases were combined, dried over anhydrous sodium sulfate and filtered. The filtrate was concentrated to dryness under reduced pressure. The resulting crude product was purified by silica gel column chromatography (eluent: ethyl acetate/petroleum ether=1/5) to obtain Compound 263-3 (800 mg, yield 75%).

LC-MS (ESI+): 314.1 m/z [M+H]⁺.

### Step 3: Preparation of tert-butyl 1-((5-iodopyridin-2-yl)methyl)-2-methyl-2-(pyrimidin-2-yl)hydrazine-carboxylate (263-4)

Compound 137-3 (573 mg, 2.5 mmol) was dissolved in N,N-dimethylformamide (10 mL), followed by the addition of sodium hydride (122 mg, 5.1 mmol) at 0°C. The mixture was stirred for 0.5 hour. Compound 263-3 (800 mg, 2.5 mmol) was added to the reaction solution. The reaction solution was stirred at 25°C for 1 hour. The reaction solution was quenched with water (20 mL), and then extracted with ethyl acetate (20 mL x3). The organic phases were combined, dried over anhydrous sodium sulfate and filtered. The filtrate was concentrated to dryness under reduced pressure. The resulting crude product was purified by silica gel column chromatography (eluent: ethyl acetate/petroleum ether=1/1) to obtain Compound 263-4 (1100 mg, yield 97%).

LC-MS (ESI+): 442.2 m/z [M+H]⁺.

2-Amino-N-((5-iodopyridin-2-yl)methyl)-N',3-dimethyl-N'-(pyrimidin-2-yl)quinol ine-6-carbohydrazide was obtained according to Steps 4 and 5 of Example 137, except that Compound 263-4 was used instead of 137-4.

LC-MS (ESI+): 526.2 m/z [M+H]⁺.

¹H NMR (400 MHz, DMSO-*d₆*) δ 8.73 (s, 1H), 8.39 (d, *J=* 3.6 Hz, 2H), 8.18-8.15 (m, 1H), 7.69 (d, *J* = 12.0 Hz, 2H), 7.44 (d, *J* = 7.2 Hz, 2H), 7.29 (d, *J* = 8.4 Hz, 1H), 6.82-6.75 (m, 1H), 6.45 (s, 2H), 5.31 (d, *J* = 15.2 Hz, 1H), 4.39 (d, *J* = 15.2 Hz, 1H), 3.19 (s, 3H), 2.17 (s, 3H).

The following compounds were obtained according to the synthetic method in Example 263 using corresponding raw materials.

| Example | Structure | Mass spectrometry data (ESI+): m/z | Nuclear magnetic data |
|---|---|---|---|
| 264 | 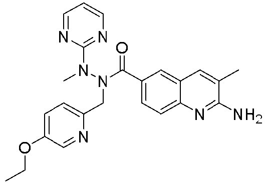 | 444.2 | ¹H NMR (400 MHz, DMSO-*d₆*) δ 8.39 (s, 2H), 8.18 (s, 1H), 7.69 (s, 2H), 7.44 (t, *J* = 8.0 Hz, 2H), 7.36 (dd, *J* = 4.0, 2.4 Hz, 1H), 7.29 (d, *J* = 8.0 Hz, 1H), 6.77 (t, *J* = 4.0 Hz, 1H), 6.56 (s, 2H), 5.37 (d, *J* = 12.0 Hz, 1H), 4.35 (d, *J* = 12.0 Hz, 1H), 4.11-4.05 (m, 2H), 3.06 (s, 3H), 2.17 (s, 3H), 1.33 (t, *J* = 8.0 Hz, 3H). |
| 265 | 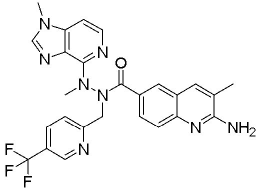 | 521.2 | ¹H NMR (400 MHz, DMSO-*d₆*) δ 8.90 (s, 1H), 8.20 (d, *J* = 8.0 Hz, 1H), 8.16 (s, 1H), 7.89 (d, *J* = 4.0 Hz, 1H), 7.82 (d, *J* = 8.0 Hz, 1H), 7.79 (s, 1H), 7.65 (s, 1H), 7.54-7.51 (m, 1H), 7.24 (d, *J =* 8.0 Hz, 1H), 7.07 (d, *J* = 8.0 Hz, 1H), 6.47 (s, 2H), 5.52 (d, *J* = 16.0 Hz, 1H), 4.40 (d, *J* = 16.0 Hz, 1H), 3.77 (s, 3H), 3.50 (s, 3H), 2.15 (s, 3H). |
| 266 | 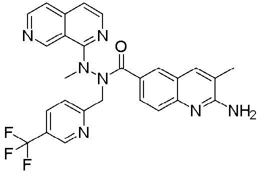 | 518.2 | |
| 267 | 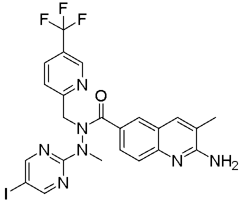 | 594.2 | |
| 268 | 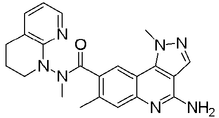 | 402.2 | |
| 269 | 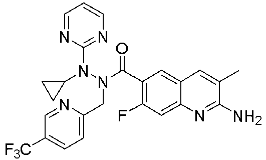 | 512.2 | ¹H NMR (400 MHz, DMSO-*d6*)δ 8.89 (d, *J* = 2.4 Hz, 1H), 8.58 (d, *J* = 4.8 Hz, 2H), 8.30 (dd, *J* = 8.4, 2.4 Hz, 1H), 7.90 (d, *J* = 8.4 Hz, 1H), 7.54 - 7.39 (m, 2H), 7.13 (d, *J =* 12.0 Hz, 1H), 7.04 (t, *J* = 4.8 Hz, 1H), 6.62 (s, 2H), 5.44 (d, *J* = 15.6 Hz, 1H), 4.72 (d, *J* = 15.6 Hz, 1H), 2.76 - 2.67 (m, 1H), 2.13 (s, 3H), 0.82 - 0.63 (m, 2H), 0.60 - 0.40 (m, 1H), 0.17 - 0.08 (m, 1H). |
| 270 | 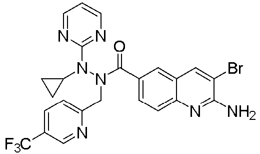 | 558.1 | |
| 271 | 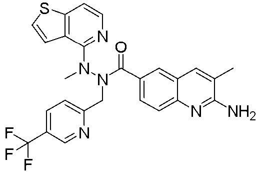 | 523.6 | ¹H NMR (400 MHz, DMSO-*d*₆) δ 8.88 (s, 1H), 8.46 (s, 1H), 8.18 (d, *J* = 8.3 Hz, 1H), 8.03 (d, *J* = 5.5 Hz, 1H), 7.92 (s, 1H), 7.80 (d, *J* = 5.7 Hz, 1H), 7.70 (s, 1H), 7.61 (d, *J* = 8.6 Hz, 1H), 7.56 (d, *J* = 5.5 Hz, 1H), 7.47 (d, *J* = 5.8 Hz, 1H), 7.33 (d, *J* = 8.9 Hz, 1H), 6.54 (s, 2H), 5.46 (d, *J* = 15.8 Hz, 1H), 4.62 (d, *J* = 15.9 Hz, 1H), 3.35 (s, 3H), 2.18 (s, 3H). |
| 272 | 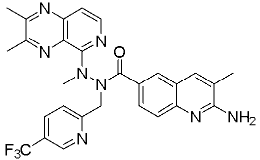 | 547.6 | ¹H NMR (400 MHz, DMSO-*d*₆) δ 8.74 (s, 1H), 8.17 (d, *J* = 5.6 Hz, 2H), 7.88 (d, *J* = 1.9 Hz, 1H), 7.78 (d, *J* = 8.5 Hz, 1H), 7.70 (s, 1H), 7.63 - 7.60 (m, 1H), 7.29 (d, *J =* 8.6 Hz, 1H), 7.21 (d, *J* = 5.7 Hz, 1H), 6.46 (s, 2H), 5.24 (d, *J* = 15.7 Hz, 1H), 4.68 (d, *J* = 15.5 Hz, 1H), 3.58 (s, 3H), 2.65 (d, *J* = 9.6 Hz, 6H), 2.17 (s, 3H). |
| 273 | 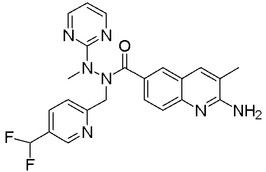 | 450.2 | |
| 274 | 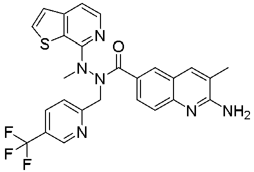 | 523.2 | |
| 275 | 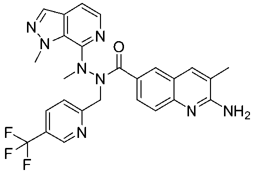 | 521.2 | |
| 276 | 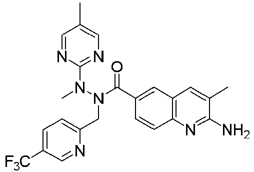 | 482.0 | ¹H NMR (400 MHz, DMSO-*d₆*) δ 8.90 (s, 1H), 8.27 (s, 2H), 8.22 (d, *J* = 10.0 Hz, 1H), 7.86 (d, *J* = 7.8 Hz, 1H), 7.78 (s, 1H), 7.72 (s, 1H), 7.51 (d, *J* = 8.2 Hz, 1H), 7.32 (d, *J* = 8.2 Hz, 1H), 6.50 (s, 2H), 5.40 (d, *J* = 15.3 Hz, 1H), 4.57 (d, *J* = 15.3 Hz, 1H), 3.28 (s, 3H), 2.20 (s, 3H), 2.08 (s, 3H). |
| 277 | 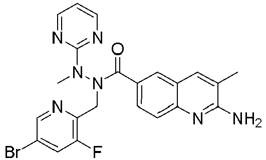 | 496.0 | ¹H NMR (400 MHz, DMSO-*d₆*) δ 8.55 (s, 1H), 8.48 - 8.36 (m, 2H), 8.25 - 8.12 (m, 1H), 7.69 (d, *J =* 6.9 Hz, 2H), 7.41 (d, *J* = 8.1 Hz, 1H), 7.30 (d, *J* = 8.7 Hz, 1H), 6.82 - 6.75 (m, 1H),, 6.50 (s, 2H), 5.42 (d, *J* = 14.5 Hz, 1H), 4.60 (d, *J =* 14.4 Hz, 1H), 3.19 (s, 3H), 2.18 (s, 3H). |
| 278 | 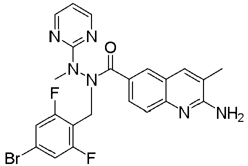 | 513.0 | |
| 279 | 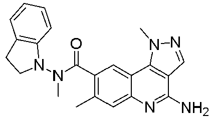 | 387.2 | ¹H NMR (400 MHz, DMSO-*d*₆) δ 8.28 (s, 1H), 8.07 (s, 1H), 7.37 (s, 1H), 7.13 (t, *J* = 7.6 Hz, 1H), 7.05 - 6.96 (m, 3H), 6.88 (d, *J* = 7.6 Hz, 1H), 6.78 (t, *J* = 7.2 Hz, 1H), 4.16 (s, 3H), 3.47 - 3.44 (m, 1H), 3.32 (t, *J* = 9.2 Hz, 1H), 3.02 (s, 3H), 2.93-2.86 (m, 1H), 2.60-2.54 (m, 1H), 2.40 (s, 3H). |
| 280 | 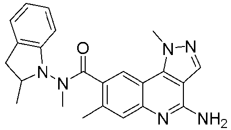 | 401.2 | |
| 281 | 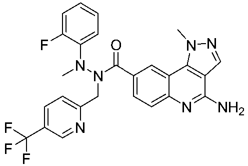 | 524.2 | ¹H NMR (400 MHz, DMSO-*d*₆) δ 8.90 (s, 1H), 8.61 (s, 1H), 8.23 (s, 1H), 8.18 - 8.15 (m, 1H), 7.83 (d, *J* = 8.8 Hz, 1H), 7.73 (d, *J* = 8.0 Hz, 1H), 7.56 (d, *J=* 8.8 Hz, 1H), 7.18 (s, 2H), 7.14 -7.07 (m, 3H), 6.99 - 6.94 (m, 1H), 5.17 (d, *J* = 15.6 Hz, 1H), 4.70 (d, *J* = 16.8 Hz, 1H), 4.18 (s, 3H), 3.23 (s, 3H). |

### Example 282: 2-amino-N-((5-bromopyrazin-2-yl)methyl)-N',3-dimethyl-N'-(pyrimidin-2-yl)quinoline -6-carbohydrazide (282)

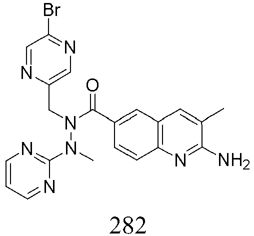

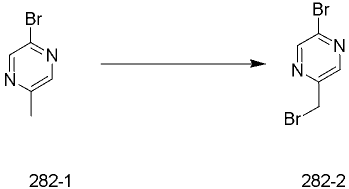

### Step 1: Preparation of 2-bromo-5-(bromomethyl)pyrazine (282-2)

2-Bromo-5-methylpyrazine (282-1, 500 mg, 2.89 mmol) was dissolved in carbon tetrachloride (10 mL), followed by the addition of NBS (566 mg, 3.18 mmol) and AIBN (24 mg, 0.14 mmol) successively, and after purging with N₂, the resulting mixed solution was stirred at 75°C for 16 hours. Ethyl acetate (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by silica gel column chromatography (petroleum ether/ethyl acetate = 10/1) to obtain Compound 282-2 (100 mg, yield 14%, a yellow oil).

LC-MS (ESI+): 250.9, 252.9 m/z [M+H]⁺.

2-Amino-N-((5-bromopyrazin-2-yl)methyl)-N',3-dimethyl-N'-(pyrimidin-2-yl)qui noline-6-carbohydrazide was synthesized according to the synthetic method of Example 263, except that Compound 282-2 was used instead of 263-3.

LC-MS (ESI+): 479.1 m/z [M+H]⁺.

The following compound was obtained according to the synthetic method in Example 282 using corresponding raw materials.

| Example | Structure | Mass spectrometry data (ESI+): m/z | Nuclear magnetic data |
|---|---|---|---|
| 283 | 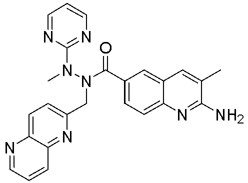 | 451.2 | ¹H NMR (400 MHz, DMSO-*d*₆) δ 8.99 (d, *J* = 4.0 Hz, 1H), 8.44 (d, *J* = 8.8 Hz, 1H), 8.42 - 4.32 (m, 3H), 8.03 (d, *J =* 8.8 Hz, 1H), 7.83 - 7.63 (m, 3H), 7.51 (d, *J* = 8.8 Hz, 1H), 7.32 (d, *J* = 8.8 Hz, 1H), 6.76 (t, *J* = 4.8 Hz, 1H), 6.48 (s, 2H), 5.58 (d, *J* = 15.2 Hz, 1H), 4.82 - 4.65 (m, 1H), 3.26 (s, 3H), 2.18 (s, 3H). |

### Example 284: Synthesis of 2-amino-N-((5-(3-methoxyoxetan-3-yl)pyridin-2-yl)methyl)-N',3-dimethyl-N'-(pyrimid in-2-yl)quinoline-6-carbohydrazide (284)

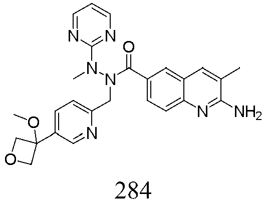

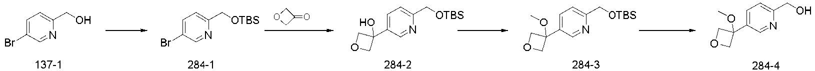

### Step 1: Preparation of 5-bromo-2-(((tert-butyldimethylsilyl)oxy)methyl)pyridine (284-1)

(5-Bromopyridin-2-yl)methanol (137-1, 5 g, 26.6 mmol) and imidazole (3.62 g, 53.2 mmol) were dissolved in dichloromethane (100 mL) at room temperature, followed by the addition of tert-butyldimethylchlorosilane (4.2 g, 27.9 mmol). The reaction solution was stirred at 25°C for 2 hour. The reaction solution was quenched with water (20 mL), and then extracted with dichloromethane (50 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/5) to obtain Compound 284-1 (8 g, yield 99%).

LC-MS (ESI+): 302.1 m/z [M+H]⁺.

### Step 2: Preparation of 3-(6-(((tert-butyldimethylsilyl)oxy)methyl)pyridin-3-yl)oxetan-3-ol (284-2)

Compound 284-1 (4 g, 13.2 mmol) was dissolved in anhydrous tetrahydrofuran (30 mL), and the reaction solution was cooled to -78 °C, followed by the dropwise addition of n-butyllithium (8 mL, 2.5 M). The reaction solution was stirred at -78°C for 1 hour. Oxetan-3-one (1.43 g, dissolved in 5 mL of tetrahydrofuran) was added to the reaction solution. The reaction solution was stirred at 25°C for 12 hour. The reaction solution was quenched with saturated aqueous ammonium chloride solution (50 mL), and then extracted with dichloromethane (50 mL x3). The organic phases were combined, dried over anhydrous sodium sulfate and filtered. The filtrate was concentrated to dryness under reduced pressure. The resulting crude product was purified by silica gel column chromatography (eluent: ethyl acetate/petroleum ether=1/5) to obtain Compound 284-2 (1.2 g, yield 30%).

LC-MS (ESI+): 296.1 m/z [M+H]⁺.

### Step 3: Preparation of 2-(((tert-butyldimethylsilyl)oxy)methyl)-5-(3-methoxyoxetan-3-yl)pyridine (284-3)

Compound 284-2 (1.2 g, 4 mmol) was dissolved in N,N-dimethylformamide (10 mL), followed by the addition of sodium hydride (60%, 195 mg, 8 mmol) at 0°C. The mixture was stirred for 0.5 hour. Iodomethane (0.5 mL) was added to the reaction solution. The reaction solution was stirred at 25°C for 1 hour. The reaction solution was quenched with water (20 mL), and then extracted with ethyl acetate (20 mL x3). The organic phases were combined, dried over anhydrous sodium sulfate and filtered. The filtrate was concentrated to dryness under reduced pressure. The resulting crude product was purified by silica gel column chromatography (eluent: ethyl acetate/petroleum ether=1/1) to obtain Compound 284-3 (900 mg, yield 71%).

LC-MS (ESI+): 310.2 m/z [M+H]⁺.

### Step 4: Preparation of (5-(3-methoxyoxetan-3-yl)pyridin-2-yl)methanol (284-4)

Compound 284-3 (900 mg, 2.9 mmol) was dissolved in dichloromethane (10 mL) at room temperature, followed by the addition of tetrabutylammonium fluoride (5 mL, 1M). The reaction solution was stirred at 25°C for 1 hour. The reaction solution was quenched with water (10 mL), and then extracted with dichloromethane (10 mL x3). The organic phases were combined, dried over anhydrous sodium sulfate and filtered, and the filtrate was concentrated to dryness under reduced pressure. The resulting crude product was purified by silica gel column chromatography (eluent: methanol/dichloromethane=1/10) to obtain Compound 284-4 (500 mg, yield 88%).

LC-MS (ESI+): 196.1 m/z [M+H]⁺.

2-Amino-N-((5-(3-methoxyoxetan-3-yl)pyridin-2-yl)methyl)-N',3-dimethyl-N'-(py rimidin-2-yl)quinoline-6-carbohydrazide was obtained according to the method of Example 263, except that Compound 284-4 was used instead of 263-2.

LC-MS (ESI+): 486.2 m/z [M+H]⁺.

### Example 285: Preparation of 2-amino-N-((5-(3-hydroxyoxetan-3-yl)pyridin-2-yl)methyl)-N',3-dimethyl-N'-(pyrimidi n-2-yl)quinoline-6-carbohydrazide (285)

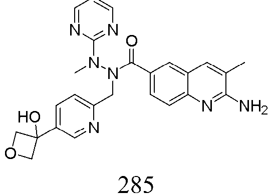

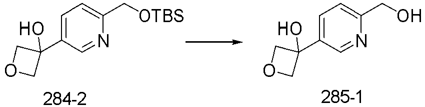

### Step 1: Preparation of 3-(6-(hydroxymethyl)pyridin-3-yl)oxetan-3-ol (285-1)

Compound 284-2 (400 mg, 1.3 mmol) was dissolved in dichloromethane (5 mL) at room temperature, followed by the addition of tetrabutylammonium fluoride (1.6 mL, 1M THF solution). The reaction solution was stirred at 25°C for 1 hour. The resulting crude product was purified by silica gel column chromatography (eluent: methanol/dichloromethane=1/10) to obtain Compound 285-1 (200 mg, yield 81%).

LC-MS (ESI+): 182.1 m/z [M+H]⁺.

2-Amino-N-((5-(3-hydroxyoxetan-3-yl)pyridin-2-yl)methyl)-N',3-dimethyl-N'-(py rimidin-2-yl)quinoline-6-carbohydrazide was obtained according to the method of Example 263, except that Compound 285-1 was used instead of 263-2.

LC-MS (ESI+): 472.2 m/z [M+H]⁺.

### Example 286: Preparation of 2-amino-N-((5-(3-fluorooxetan-3-yl)pyridin-2-yl)methyl)-N',3-dimethyl-N'-(pyrimidin-2-yl)quinoline-6-carbohydrazide (286)

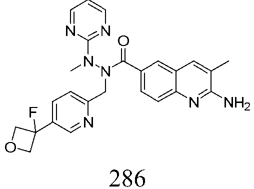

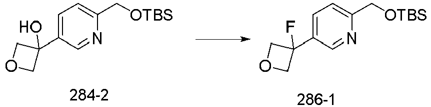

### Step 1: Preparation of 2-(((tert-butyldimethylsilyl)oxy)methyl)-5-(3-fluorooxetan-3-yl)pyridine (286-1)

Compound 284-2 (300 mg, 1 mmol) was dissolved in dichloromethane (5 mL) at room temperature, followed by the addition of diethylaminosulphur trifluoride (0.2 mL). The reaction solution was stirred at 25°C for 1 hour. The reaction solution was quenched with sodium bicarbonate saturated aqueous solution (10 mL), and then extracted with ethyl acetate (10 mL x3). The organic phases were combined, dried over anhydrous sodium sulfate and filtered. The organic phase was concentrated to dryness under reduced pressure to obtain Compound 286-1 (300 mg, yield 99%).

LC-MS (ESI+): 298.2 m/z [M+H]⁺.

2-Amino-N-((5-(3-fluorooxetan-3-yl)pyridin-2-yl)methyl)-N',3-dimethyl-N'-(pyri midin-2-yl)quinoline-6-carbohydrazide was synthesized according to the method of Example 285, except that Compound 286-1 was used instead of Compound 284-2.

LC-MS (ESI+): 474.2 m/z [M+H]⁺.

¹H NMR (400 MHz, DMSO-*d₆*) δ 8.72 (s, 1H), 8.42 (d, *J* = 4.0 Hz, 2H), 8.00 (d, *J* = 7.2 Hz, 1H), 7.75-7.71 (m, 3H), 7.48 (d, *J* = 8.4 Hz, 1H), 7.32 (d, *J* = 8.4 Hz, 1H), 6.80 (t, *J* = 4.4 Hz, 1H), 6.51 (s, 2H), 5.43 (d, *J* = 15.2 Hz, 1H), 5.03 (s, 2H), 4.98 (s, 2H), 4.50 (d, *J* = 15.2 Hz, 1H), 3.24 (s, 3H), 2.19 (s, 3H).

The following compounds were obtained according to the synthetic method in Example 286 using corresponding raw materials.

| Example | Structure | Mass spectrometry data (ESI+): m/z | Nuclear magnetic data |
|---|---|---|---|
| 287 | 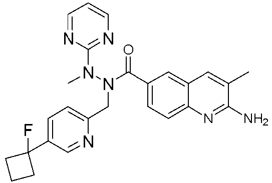 | 472.2 | |
| 288 | 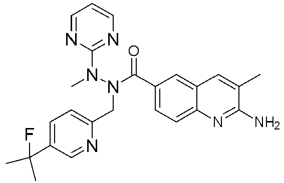 | 460.0 | ¹H NMR (400 MHz, DMSO-*d₆*) δ 8.56 (s, 1H), 8.44 - 8.34 (m, 2H), 7.84 -7.79 (m, , 1H), 7.75 - 7.65 (m, 2H), 7.62 - 7.53 (m, 1H), 7.51 - 7.41 (m, 1H), 7.33 - 7.26 (m, 1H), 6.83 - 6.71 (m, 1H), 6.46 (s, 2H), 5.37 (d, *J* = 15.2 Hz, 1H), 4.46 (d, *J* = 15.2 Hz, 1H), 3.20 (s, 3H), 2.17 (s, 3H), 1.71 (s, 3H), 1.66 (s, 3H). |

### Example 289: Preparation of 2-amino-N-((5-cyclobutylpyridin-2-yl)methyl)-N',3-dimethyl-N'-(pyrimidin-2-yl)quinol ine-6-carbohydrazide (289)

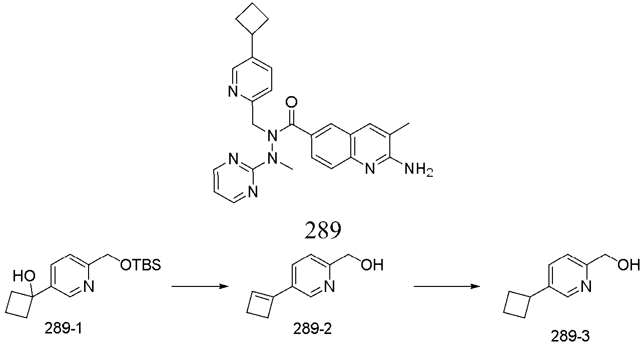

### Step 1: Preparation of (5-(cyclobut-1-en-1-yl)pyridin-2-yl)methanol (289-2)

Compound 289-1 (synthesized according to the method in Example 284) (300 mg, 1 mmol) was dissolved in toluene (5 mL) at room temperature,, followed by the addition of p-toluenesulfonic acid (352 mg, 2 mmol). The reaction solution was heated to 100°C and stirred for 2 hours. The reaction solution was quenched with water (20 mL), and then extracted with dichloromethane (50 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/1) to obtain Compound 289-2 (60 mg, yield 36%).

LC-MS (ESI+): 162.1 m/z [M+H]⁺.

### Step 2: Preparation of (5-cyclobutylpyridin-2-yl)methanol (289-3)

Palladium on carbon (20 mg, 5%) was added to 289-2 (5 mL) at room temperature, and the reaction solution was purged with hydrogen three times. The reaction solution was stirred at 25°C for 2 hours under hydrogen atmosphere. After the reaction was completed, palladium on carbon was filtered, and the organic phase was concentrated to dryness under reduced pressure to obtain Compound 289-3 (240 mg, yield 99%).

LC-MS (ESI+): 164.2 m/z [M+H]⁺.

2-Amino-N-((5-cyclobutylpyridin-2-yl)methyl)-N',3-dimethyl-N'-(pyrimidin-2-yl) quinoline-6-carbohydrazide was synthesized according to the method of Example 263, except that Compound 289-3 was used instead of Compound 263-2.

LC-MS (ESI+): 454.2 m/z [M+H]⁺.

¹H NMR (400 MHz, DMSO-*d₆*) δ 8.49 - 8.36 (m, 3H), 7.71 (m, 3H), 7.57 - 7.24 (m, 3H), 6.79 (t, *J =* 4.4 Hz, 1H), 6.50 (s, 2H), 5.41 (d, *J =* 14.8 Hz, 1H), 4.38 (d, *J =* 14.8 Hz, 1H), 3.15 (s, 3H), 2.35-2.29 (m, 2H), 2.23 - 1.95 (m, 7H), 1.89 - 1.79 (m, 1H).

### Example 290: Preparation of 2-amino-N-((5-(2-hydroxyprop-2-yl)pyridin-2-yl)methyl)-N',3-dimethyl-N'-(pyrimidin-2-yl)quinoline-6-carbohydrazide (290)

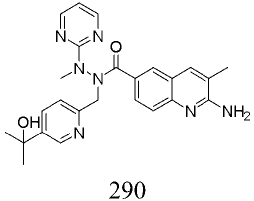

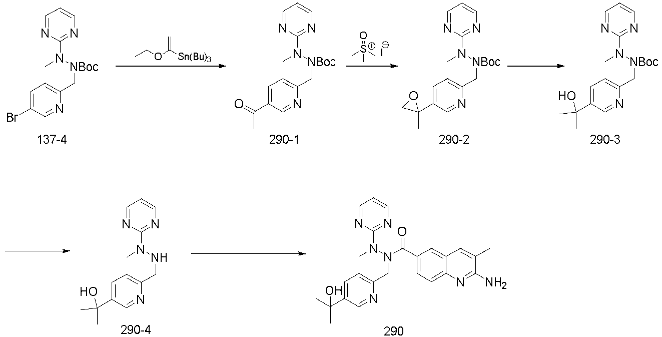

### Step 1: Preparation of tert-butyl 1-((5-acetylpyridin-2-yl)methyl)-2-methyl-2-(pyrimidin-2-yl)hydrazine-carboxylate (290-1)

Compound 137-4 (500 mg, 1.2 mmol) and tributyl(1-ethoxyvinyl)stannane (0.8 mL) were dissolved in dry 1,4-dioxane (10 mL) At room temperature, followed by the addition of tetrakis(triphenylphosphine)palladium (146 mg, 0.12 mmol). The reaction solution was purged with nitrogen three times, then heated to 100°C under nitrogen atmosphere and stirred for 12 hours. After the reaction was completed, the reaction solution was quenched with 1N hydrochloric acid (5 mL), and then extracted with ethyl acetate (30 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/1) to obtain Compound 290-1 (400 mg, yield 88%).

LC-MS (ESI+): 358.2 m/z [M+H]⁺.

### Step 2: Preparation of tert-butyl 2-methyl-1-((5-(2-methoxy-2-yl)pyridin-2-yl)methyl)-2-(pyrimidin-2-yl)hydrazine-carb oxylate (290-2)

Trimethylsulfoxonium iodide (492 mg, 2.2 mmol) and potassium tert-butoxide (251 mg, 2.2 mmol) were dissolved in dry tetrahydrofuran (10 mL) at room temperature. The mixture was heated to 50 °C and stirred for 0.5 hour. Compound 290-1 (200 mg, 0.56 mmol) was dissolved in tetrahydrofuran (2 mL), and added to the reaction solution. The reaction solution was kept at 50°C and stirred for 12 hours. After the reaction was completed, the reaction solution was quenched with water (10 mL), and then extracted with ethyl acetate (10 mL x3). The organic phases were combined, dried over anhydrous sodium sulfate and filtered, and the filtrate was concentrated to dryness under reduced pressure. The resulting crude product was purified by silica gel column chromatography (eluent: methanol/dichloromethane=1/10) to obtain Compound 290-2 (100 mg, yield 48%).

LC-MS (ESI+): 372.2 m/z [M+H]⁺.

### Step 3: Preparation of tert-butyl 1-((5-(2-hydroxyprop-2-yl)pyridin-2-yl)methyl)-2-methyl-2-(pyrimidin-2-yl)hydrazine-carboxylate (290-3)

Compound 290-2 (90 mg, 0.24 mmol) was dissolved in ethanol (2 mL) at room temperature, followed by the addition of sodium borohydride (18 mg, 0.48 mmol). The reaction solution was stirred at 25°C for 12 hour. The reaction solution was quenched with water (10 mL), and then extracted with ethyl acetate (10 mL x3). The organic phases were combined, dried over anhydrous sodium sulfate and filtered, and the filtrate was concentrated to dryness under reduced pressure. The resulting crude product was purified by silica gel column chromatography (eluent: methanol/dichloromethane=1/10) to obtain Compound 290-3 (70 mg, yield 77%).

LC-MS (ESI+): 374.4 m/z [M+H]⁺.

### Step 4: Preparation of 2-(6-((2-methyl-2-(pyrimidin-2-yl)hydrazino)methyl)pyridin-3-yl)propan-2-ol (290-4)

Compound 290-3 (70 mg, 0.18 mmol) was dissolved in dry dichloromethane (1 mL) at room temperature, followed by the addition of trifluoroacetic acid (0.2 mL). The reaction solution was stirred at 25°C for 1 hour until raw materials disappeared. The reaction solution was concentrated to dryness under reduced pressure to obtain Compound 290-4 (50 mg, yield 97%).

LC-MS (ESI+): 274.2 m/z [M+H]⁺.

### Step 5: Preparation of 6-bromo-3-trifluoromethylquinoline (290-5)

Compound 290-4 (20 mg, 0.07 mmol) and 2-amino-3-methylquinoline-6-carbonyl chloride (16 mg, 0.07 mmol) were dissolved in dry N,N-dimethylacetamide (1 mL) at room temperature, followed by the addition of diisopropylethylamine (0.1 mL). The reaction solution was stirred at 25°C for 2 hour. The resulting crude product was separated by high performance liquid chromatography (water/acetonitrile=95%/5% - 10%/90%) to obtain Compound 290 (2.9 mg, yield 8%).

LC-MS (ESI+): 458.3 m/z [M+H]⁺.

### Example 291: Preparation of 2-amino-N-((5-(1,1-difluoroethyl)pyridin-2-yl)methyl)-N',3-dimethyl-N'-(pyrimidin-2-yl)quinoline-6-carbohydrazide (291)

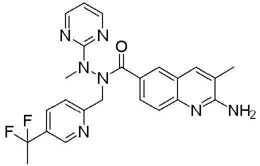

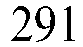

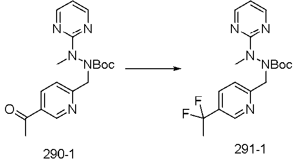

### Step 1: Preparation of tert-butyl 1-((5-(1,1-difluoroethyl)pyridin-2-yl)methyl)-2-methyl-2-(pyrimidin-2-yl)hydrazine-car boxylate (291-1)

Compound 290-1 (300 mg, 0.84 mmol) was dissolved in dichloromethane (5 mL) at room temperature, followed by the addition of diethylaminosulphur trifluoride (0.4 mL). The reaction solution was stirred at 25 °C for 36 hour. The reaction solution was quenched with saturated aqueous sodium bicarbonate solution (10 mL), and then extracted with ethyl acetate (10 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/5) to obtain Compound 291-1 (100 mg, yield 31%).

LC-MS (ESI+): 380.4 m/z [M+H]⁺.

2-Amino-N-((5-(1,1-difluoroethyl)pyridin-2-yl)methyl)-N',3-dimethyl-N'-(pyrimid in-2-yl)quinoline-6-carbohydrazide was synthesized according to Steps 4 and 5 of Example 290, except that Compound 291-1 was used instead of Compound 290-3.

LC-MS (ESI+): 464.2 m/z [M+H]⁺.

¹H NMR (400 MHz, DMSO-*d₆*) δ 8.73 (s, 1H), 8.41 (d, *J =* 4.4 Hz, 2H), 8.01 (d, *J* = 6.4 Hz, 1H), 7.81 - 7.67 (m, 3H), 7.48 (d, *J =* 8.0 Hz, 1H), 7.32 (d, *J =* 8.8 Hz, 1H), 6.81 (t, *J =* 4.4 Hz, 1H), 6.51 (s, 2H), 5.42 (d, *J =* 15.2 Hz, 1H), 4.53 (d, *J =* 15.2 Hz, 1H), 3.27 (s, 3H), 2.19 (s, 3H), 2.04 (t, *J =* 19.2 Hz, 3H).

### Preparation of Example 292: 2-amino-N-((5-(1-fluoroethyl)pyridin-2-yl)methyl)-N',3-dimethyl-N'-(pyrimidin-2-yl)q uinoline-6-carbohydrazide (292)

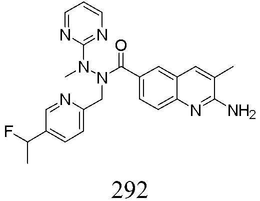

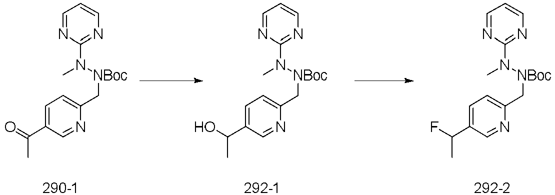

### Step 1: Preparation of tert-butyl 1-((5-(1-hydroxyethyl)pyridin-2-yl)methyl)-2-methyl-2-(pyrimidin-2-yl)hydrazine-carb oxylate (292-1)

Compound 290-1 (200 mg, 0.56 mmol) was dissolved in methanol (2 mL) at room temperature, followed by the addition of sodium borohydride (42 mg, 1.1 mmol). The reaction solution was stirred at 25°C for 1 hour. The reaction solution was quenched with water (10 mL), and then extracted with ethyl acetate (10 mL x3). The organic phases were combined, dried over anhydrous sodium sulfate and filtered. The organic phase was concentrated to dryness under reduced pressure to obtain Compound 292-1 (200 mg, yield 99%).

LC-MS (ESI+): 360.2 m/z [M+H]⁺.

### Step 2: Preparation of tert-butyl 1-((5-(1-fluoroethyl)pyridin-2-yl)methyl)-2-methyl-2-(pyrimidin-2-yl)hydrazine-carbox ylate (292-2)

Compound 292-1 (200 mg, 0.55 mmol) was dissolved in dichloromethane (5 mL) at room temperature, followed by the addition of diethylaminosulphur trifluoride (0.2 mL). The reaction solution was stirred at 25°C for 1 hour. The reaction solution was quenched with saturated aqueous sodium bicarbonate solution (10 mL), and then extracted with ethyl acetate (10 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/5) to obtain Compound 292-2 (100 mg, yield 49%).

LC-MS (ESI+): 362.4 m/z [M+H]⁺.

2-Amino-N-((5-(1-fluoroethyl)pyridin-2-yl)methyl)-N',3-dimethyl-N'-(pyrimidin-2 -yl)quinoline-6-carbohydrazide was synthesized according to Steps 4 and 5 of Example 290, except that Compound 292-2 was used instead of Compound 290-3.

LC-MS (ESI+): 446.2 m/z [M+H]⁺.

¹H NMR (400 MHz, DMSO-*d₆*) δ 8.54 (s, 1H), 8.39 (d, *J =* 4.0 Hz, 2H), 7.84 (d, *J* = 8.0 Hz, 1H), 7.70 (d, *J =* 13.6 Hz, 2H), 7.61 (d, *J =* 8.0 Hz, 1H), 7.45 (d, *J =* 8.4 Hz, 1H), 7.29 (d, *J =* 8.4 Hz, 1H), 6.78 (t, *J =* 4.0 Hz 1H), 6.47 (s, 2H), 5.88-5.72 (m, 1H), 5.40 (d, *J =* 15.2 Hz, 1H), 4.44 (d, *J =* 15.2 Hz, 1H), 3.19 (s, 3H), 2.17 (s, 3H), 1.62 (dd, *J =* 24.0, 6.4 Hz, 3H).

### Example 293: Preparation of 2-amino-N,3-dimethyl-N-((5-(perfluoroethyl)pyridin-2-yl)methyl)-N-(pyrimidin-2-yl)q uinoline-6-carbohydrazide (293)

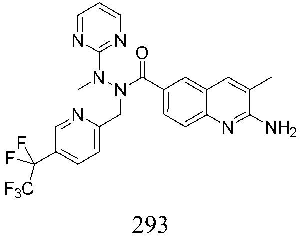

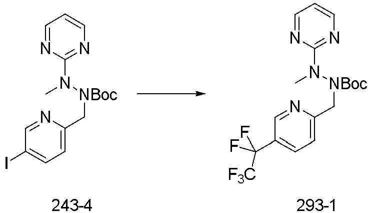

### Step 1: Preparation of tert-butyl 2-methyl-1-((5-(perfluoroethyl)pyridin-2-yl)methyl)-2-(pyrimidin-2-yl)hydrazine-carbo xylate (293-1)

Compound 263-4 (100 mg, 0.22 mmol), cuprous iodide (86 mg, 0.45 mmol) and (pentafluoroethyl)trimethylsilane (0.1 mL) were dissolved in dry N,N-dimethylformamide (1 mL) at room temperature, followed by the addition of potassium fluoride (39.4 mg, 0.67 mmol). The reaction solution was purged with nitrogen three times, then heated to 80°C under nitrogen atmosphere and stirred for 12 hours. After the reaction was completed, the reaction solution was quenched with water (10 mL), and extracted with ethyl acetate (10 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/1) to obtain Compound 293-1 (60 mg, yield 61%).

LC-MS (ESI+): 434.2 m/z [M+H]⁺.

2-Amino-*N*',3-dimethyl-*N*-((5-(perfluoroethyl)pyridin-2-yl)methyl)-N-(pyrimidin-2-yl)quinoline-6-carbohydrazide was obtained according to Steps 4 and 5 of Example 290, except that Compound 293-1 was used instead of Compound 290-3.

LC-MS (ESI+): 518.2 m/z [M+H]⁺.

¹H NMR (400 MHz, DMSO-*d₆*) δ 8.83 (s, 1H), 8.37 (d, *J =* 4.0 Hz, 2H), 8.17 (d, *J* = 6.8 Hz, 1H), 7.89 (d, *J =* 8.0 Hz, 1H), 7.75 (s, 1H), 7.69 (s, 1H), 7.48 (d, *J =* 8.4 Hz, 1H), 7.30 (d, *J =* 8.4 Hz, 1H), 6.76 (t, *J =* 8.4 Hz, 1H), 6.47 (s, 2H), 5.36 (d, *J =* 15.6 Hz, 1H), 4.67 (d*, J =* 15.6 Hz, 1H), 3.31 (s, 3H), 2.18 (s, 3H).

### Example 294: Preparation of N-((5-acetylpyridin-2-yl)methyl)-2-amino-N',3-dimethyl-N'-(pyrimidin-2-yl)quinoline-6-carbohydrazide (294)

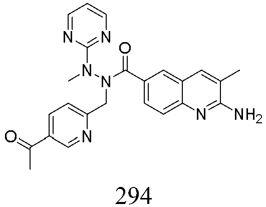

*N*-((5-acetylpyridin-2-yl)methyl)-2-amino-*N*',3-dimethyl-*N*-(pyrimidin-2-yl)quinol ine-6-carbohydrazide was synthesized according to Steps 4 and 5 of Example 137, except that Compound 290-1 was used instead of 137-4.

LC-MS (ESI+): 442.2 m/z [M+H]⁺.

### Example 295: Preparation of 2-amino-N-((5-(1-hydroxyethyl)pyridin-2-yl)methyl)-N',3-dimethyl-N-(pyrimidin-2-yl) quinoline-6-carbohydrazide (295)

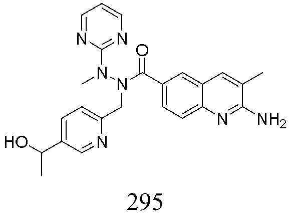

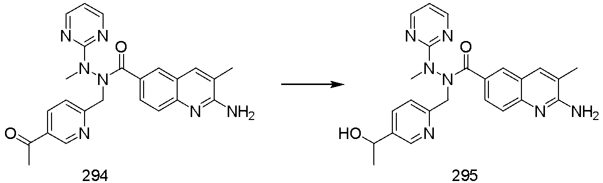

### Step 1: Preparation of 2-amino-N-((5-(1-hydroxyethyl)pyridin-2-yl)methyl)-N',3-dimethyl-N-(pyrimidin-2-yl) quinoline-6-carbohydrazide (295)

Compound 294 (50 mg, 0.11 mmol) was dissolved in methanol (1 mL), followed by the addition of sodium borohydride (4 mg, 0.11 mmol). The reaction solution was stirred at 0°C for 1 hour. The reaction solution was quenched with water (10 mL), and then extracted with ethyl acetate (10 mL x3). The organic phases were combined, dried over anhydrous sodium sulfate and filtered. The resulting crude product was separated by reversed-phase high performance liquid chromatography (water/acetonitrile=95%/5% - 10%/90%, as the mobile phase) to obtain Compound 295 (2.2 mg, yield 4%).

LC-MS (ESI+): 444.3 m/z [M+H]⁺.

### Example 296: Preparation of 2-amino-N'-(5-(dimethylphosphoryl)pyrimidin-2-yl)-N',3-dimethyl-N-((5-(trifluoromet hyl)pyridin-2-yl)methyl)quinoline-6-carbohydrazide (296)

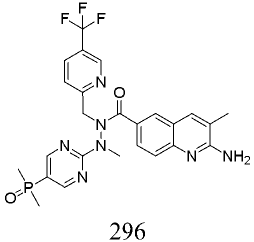

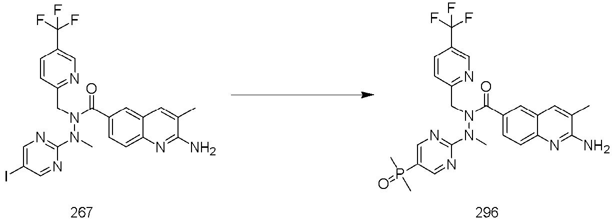

### Step 1: Preparation of 2-amino-N'-(5-(dimethylphosphoryl)pyrimidin-2-yl)-N',3-dimethyl-N-((5-(trifluoromet hyl)pyridin-2-yl)methyl)quinoline-6-carbohydrazide (296)

Compound 267 (50.0 mg, 0.08 mmol) was dissolved in 1,4-dioxane (2 mL) in a sealing tube, followed by the addition of dimethylphosphine oxide (7.00 mg, 0.08 mmol), Pd₂(dba)₃ (8mg, 0.008 mmol), Xantphos (10 mg, 0.016 mmol) and potassium phosphate (36 mg, 0.17 mmol) successively, and after purging with N₂, the resulting mixed solution was stirred at 100°C for 2 hours. Ethyl acetate (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 296 (7.8 mg, yield 17%, a white solid).

LC-MS (ESI+): 544.3 m/z [M+H]⁺.

### Example 297: Preparation of 2-amino-N-((5-(dimethylphosphoryl)pyridin-2-yl)methyl)-N',3-dimethyl-N'-(pyrimidin -2-yl)quinoline-6-carbohydrazide (297)

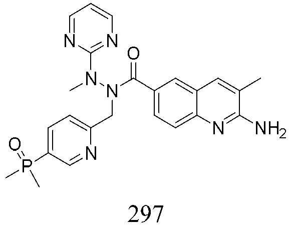

2-Amino-N-((5-(dimethylphosphoryl)pyridin-2-yl)methyl)-N',3-dimethyl-N'-(pyri midin-2-yl)quinoline-6-carbohydrazide was synthesized according to the method in Step 1 of Example 296, except that 137-6 was used instead of 267.

LC-MS (ESI+): 476.6 m/z [M+H]⁺.

### Example 298: Preparation of 2-amino-N'-(3-cyanopyridin-2-yl)-N',3-dimethyl-N-((5-(trifluoromethyl)pyridin-2-yl)m ethyl)quinoline-6-carbohydrazide (298)

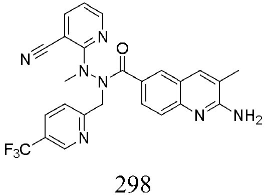

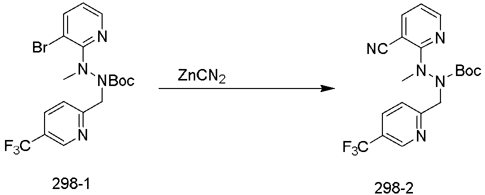

### Step 1: Preparation of tert-butyl 2-(3-cyanopyridin-2-yl)-2-methyl-1-((5-(trifluoromethyl)pyridin-2-yl)methyl)hydrazine -1-carboxylate (298-2)

Compound 298-1 (synthesized according to the method in Example 263) (90 mg, 0.19 mmol), zinc hydride (46 mg, 0.39 mmol) and bis(tri-tert-butylphosphine)palladium (10 mg, 0.02 mmol) were dissolved in N-methylpyrrolidone (5 mL) solution at room temperature, and reacted at 130°C for 12 hours, and the resulting reaction mixture was diluted with water (50 mL). The resulting mixture was extracted with ethyl acetate (50 mL x 3). The organic phases were combined, washed with saturated brine (100 mL), dried over anhydrous sodium sulfate, and filtered. The filtrate was concentrated under reduced pressure. The resulting residues were purified by silica gel column chromatography (eluent: petroleum ether/ethyl acetate=1/1) to obtain Compound 298-2 (80 mg, yield 100%).

LC-MS (ESI+): 408.2 m/z [M+H]⁺.

2-Amino-N'-(3-cyanopyridin-2-yl)-N',3-dimethyl-N-((5-(trifluoromethyl)pyridin-2 -yl)methyl)quinoline-6-carbohydrazide was synthesized according to Steps 4 and 5 of Example 290, except that Compound 298-2 was used instead of Compound 290-3.

LC-MS (ESI+): 492.2 m/z [M+H]⁺.

### Example 299: Preparation of (2-(2-(-2-amino-3-methylquinoline-6-carbonyl)-1-methyl-2-((5-(trifluoromethyl)pyridin -2-yl)methyl)hydrazino)pyrimidin-5-yl)boronic acid (299)

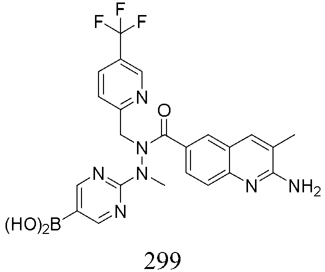

2-(2-(-2-Amino-3-methylquinoline-6-carbonyl)-1-methyl-2-((5-(trifluoromethyl)py ridin-2-yl)methyl)hydrazino)pyrimidin-5-yl)boronic acid was synthesized according to the method in Step 2 of Example 226, except that Compound 267 was used instead of Compound 137-6.

LC-MS (ESI+): 512.2 m/z [M+H]⁺

### Example 300: Preparation of 4-amino-N',1-dimethyl-N'-(pyrimidin-2-yl)-N-(4-(trifluoromethyl)benzyl)-1H-pyrazolo[ 4,3-c]quinoline-8-carbohydrazide (300)

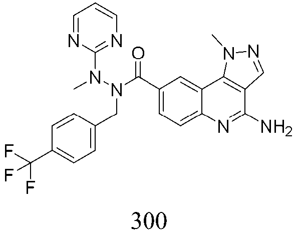

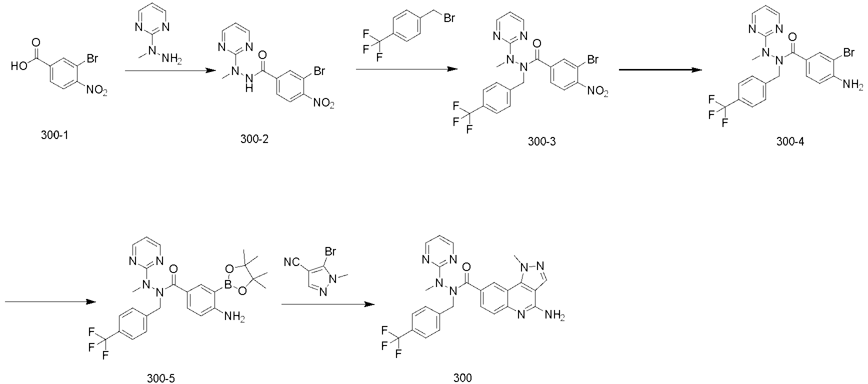

### Step 1: Preparation of 3-bromo-N'-methyl-4-nitro-N'-(pyrimidin-2-yl)benzohydrazide (300-2)

3-Bromo-4-nitrobenzoic acid (300-1) (400 mg, 1.63 mmol) was dissolved in 6 mL of dimethylacetamide at room temperature, followed by the addition of 1-4 (202 mg, 1.63 mmol), N,N-diisopropylethylamine (494 mg, 4.89 mmol) and bromo-tris-pyrrolidino-phosphonium hexafluorophosphate (1.14 g, 2.45 mmol). The reaction was carried out at room temperature overnight under nitrogen atmosphere. The reaction was quenched by addition of the reaction mixture to water, and then extracted with ethyl acetate (30 mLx 2). The organic phases were combined, dried over anhydrous sodium sulfate and filtered. The filtrate was concentrated to dryness under reduced pressure, and the resulting residues were purified by silica gel column chromatography (eluent: dichloromethane /methanol =0-5%), and concentrated under reduced pressure to obtain Compound 300-2 (300 mg, yield 58.4%).

LC-MS (ESI+): 352.0 m/z [M+H]⁺.

### Step 2: Preparation of 3-bromo-N'-methyl-4-nitro-N'-(pyrimidin-2-yl)-N-(4-(trifluoromethyl)benzyl)benzohyd razide (300-3)

Compound 300-2 (270 mg, 0.77 mmol) was dissolved in 6 mL of DMF at room temperature, followed by the addition of cesium carbonate (747mg, 2.30 mmol) and 1-bromomethyl-4-trifluoromethylbenzene (366 mg, 1.53 mmol).The reaction was carried out at 50°C under nitrogen atmosphere overnight. The reaction was quenched by addition of the reaction mixture to water, and then extracted with ethyl acetate (30 mL x 2). The organic phases were combined, dried over anhydrous sodium sulfate and filtered. The filtrate was concentrated to dryness under reduced pressure, and the resulting residues were purified by silica gel column chromatography (eluent: dichloromethane /methanol =0-5%), and concentrated under reduced pressure to obtain Compound 300-3 (240 mg, yield 44 %).

LC-MS (ESI+): 510.0 m/z [M+H]⁺.

### Step 3: Preparation of 4-amino-3-bromo-N'-methyl-N'-(pyrimidin-2-yl)-N-(4-(trifluoromethyl)benzyl)benzohy drazide (300-4)

Compound 300-3 (240 mg, 0.47 mmol) was dissolved in 3 mL of ethanol and 3 mL of ammonium chloride saturated aqueous solution at room temperature, followed by the addition of iron powder (132 mg, 2.35 mmol). The reaction was carried out at 80°C for 2 hours. The reaction mixture was filtered through Celite, washed with ethanol, and then extracted with ethyl acetate (30 mL x 2). The organic phases were combined, dried over anhydrous sodium sulfate and filtered. The filtrate was concentrated to dryness under reduced pressure, and the resulting residues were purified by silica gel column chromatography (eluent: dichloromethane /methanol =0-5%), and concentrated under reduced pressure to obtain Compound 300-4 (220 mg, yield 97%).

LC-MS (ESI+): 480.0 m/z [M+H]⁺.

### Step 4: Preparation of 4-amino-N'-methyl-N'-(pyrimidin-2-yl)-3-(4,4,5,5-tetramethyl-1,3,2-dioxaborocyclopen tan-2-yl)-N-(4-(trifluoromethyl)benzyl)benzohydrazide (300-5)

Compound 300-4 (140 mg, 0.47 mmol) was dissolved in 3 mL of dioxane at room temperature, followed by the addition of bis(pinacolato)diboron (370 mg, 1.46 mmol), potassium acetate (85.70 mg, 0.87 mmol) and 1,1'-bis(diphenylphosphino)ferrocene]palladium dichloridedichloromethane complex (21 mg, 0.03 mmol). The reaction was carried out at 90°C under nitrogen atmosphere overnight. The reaction was cooled to room temperature, and the resulting reaction mixture was directly used for the next step without treatment. LC-MS (ESI+): 528.2 m/z [M+H]⁺.

### Step 5: Preparation of 4-amino-N',1-dimethyl-N'-(pyrimidin-2-yl)-N-(4-(trifluoromethyl)benzyl)-1H-pyrazolo[ 4,3-c]quinoline-8-carbohydrazide (300)

5-Bromo-1-methyl-1H-pyrazole-4-carbonitrile (59 mg, 0.32 mmol), 1,1'-bis(diphenylphosphino)ferrocene]palladium dichloridedichloromethane complex (21 mg, 0.03 mmol), 0.5 mL of water, and potassium carbonate (108 mg, 0.78 mmol) were added to the reaction mixture from the previous step at room temperature. The reaction was carried out at 90°C under nitrogen atmosphere overnight. The reaction solution was filtered through Celite, and extracted with ethyl acetate (30 mLx2). The organic phases were combined, and concentrated under reduced pressure, and the residues were purified by preparative high performance liquid chromatography (water/acetonitrile=95%/5% - 10%/90%) to obtain Compound 300 (1.2 mg, yield 2.1%).

LC-MS (ESI+): 507.2 m/z [M+H]⁺.

### Example 301: Preparation of 2-amino-3-methyl-6-(2-methyl-2-(pyrimidin-2-yl)-1-((5-(trifluoromethyl)pyridin-2-yl) methyl)hydrazine-1-carbonyl)quinoline 1-oxide (301)

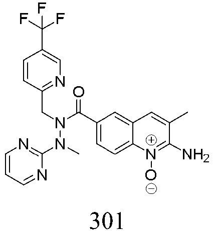

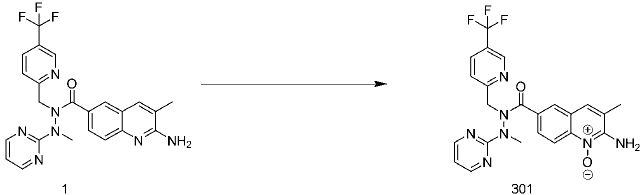

### Step 1: Preparation of 2-amino-3 -methyl-6-(2-methyl-2-(pyrimidin-2-yl)-1-((5-(trifluoromethyl)pyridin-2-yl) methyl)hydrazine-1-carbonyl)quinoline 1-oxide (301)

Compound 1 (100 mg, 0.21 mmol) was dissolved in DCM (10 mL), followed by the addition of 3-chloroperbenzoic acid (51 mg, 0.11 mmol), and then the resulting mixed solution was stirred at 20°C for 2 hours. DCM (20 mL) was added to the reaction solution, and the reaction solution was washed with aqueous sodium bicarbonate solution (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 301 (38 mg, yield 37%, a white solid).

LC-MS (ESI+): 484.2 m/z [M+H]⁺.

The following compound was obtained according to the synthetic method in Example 301, using corresponding raw materials.

| Example | Structure | Mass spectrometry data (ESI+): m/z | Nuclear magnetic data |
|---|---|---|---|
| 302 | 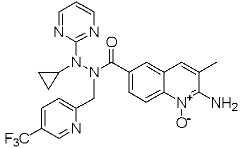 | 509.2 | ¹H NMR (400 MHz, DMSO-*d₆*) δ 8.86 (d, *J* = 2.4 Hz, 1H), 8.59 (d, *J =* 4.8 Hz, 2H), 8.24 (dd, *J* = 8.4, 2.4 Hz, 1H), 8.11 (d, *J* = 8.8 Hz, 1H), 7.94 (d, *J =* 8.4 Hz, 1H), 7.86 (d, *J =* 1.6 Hz, 1H), 7.61 (dd, *J* = 8.8, 1.6 Hz, 1H), 7.52 (s, 1H), 7.48 (s, 2H), 7.04 (t, *J =* 4.8 Hz, 1H), 5.38 (d, *J =* 15.6 Hz, 1H), 4.72 (d, *J* = 15.6 Hz, 1H), 2.85 - 2.72 (m, 1H), 2.31 (s, 3H), 0.83 - 0.71 (m, 1H), 0.60 - 0.42 (m, 2H), 0.20 - 0.10 (m, 1H). |

### Example 303: Preparation of 2-amino-3-bromo-N'-methyl-N'-(pyrimidin-2-yl)-N-((5-(trifluoromethyl)pyridin-2-yl)m ethyl)quinoline-6-carbohydrazide (303)

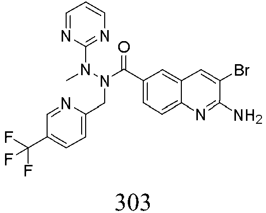

2-Amino-3-bromo-N'-methyl-N'-(pyrimidin-2-yl)-N-((5-(trifluoromethyl)pyridin-2 -yl)methyl)quinoline-6-carbohydrazide was synthesized according to the method in Steps 3-5 of Example 26, except that Compound 25-3 was used instead of Compound 26-2.

LC-MS (ESI+): 532.0 m/z [M+H]⁺.

¹H NMR (400 MHz, DMSO-*d₆*) δ 8.90 (s, 1H), 8.41 - 8.38 (m, 3H), 8.21 (d, *J =* 8.0 Hz, 1H), 7.87 - 7.80 (m, 2H), 7.57 (d, *J =* 8.0 Hz, 1H), 7.35 (d, *J =* 12.0 Hz, 1H), 6.86 (s, 2H), 6.82 - 6.75 (m, 1H), 5.39 (d, *J =* 16.0 Hz, 1H), 4.60 (d, *J =* 16.0 Hz, 1H), 3.30 (s, 3H).

### Example 304: Preparation of 2-amino-N'-methyl-N'-(pyrimidin-2-yl)-N-((5-(trifluoromethyl)pyridin-2-yl)methyl)-3-v inylquinoline-6-carbohydrazide (304)

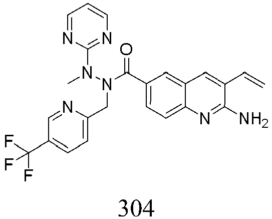

Compound 304 was synthesized according to the method in Step 1 of Example 26, except that vinylborate was used instead of isopropenylborate, and Compound 303 was used instead of Compound 25-3.

LC-MS (ESI+): 480.2 m/z [M+H]⁺.

### Example 305: Preparation of 2-amino-3-ethyl-N'-methyl-N'-(pyrimidin-2-yl)-iN-((5-(trifluoromethyl)pyridin-2-yl)met hyl)quinoline-6-carbohydrazide (305)

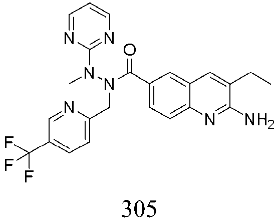

2-Amino-3-ethyl-N'-methyl-N'-(pyrimidin-2-yl)-N-((5-(trifluoromethyl)pyridin-2-yl)methyl)quinoline-6-carbohydrazide was synthesized according to the method in Step 2 of Example 26, except that Compound 304 was used instead of Compound 26-1.

LC-MS (ESI+): 482.2 m/z [M+H]⁺.

¹H NMR (400 MHz, DMSO-*d₆*) δ 8.89 (s, 1H), 8.39 (d, *J =* 4.0 Hz, 2H), 8.20 (d, *J* = 4.0 Hz, 1H), 7.85 (d, *J =* 8.0 Hz, 1H), 7.79 (s, 1H), 7.68 (s, 1H), 7.46 (d, *J =* 8.0 Hz, 1H), 7.29 (d, *J =* 8.0 Hz, 1H), 6.78 (s, 1H), 6.44 (s, 2H), 5.40 (d, *J =* 16.0 Hz, 1H), 4.58 (d, *J* = 16.0 Hz, 1H), , 3.29 (s, 3H), 2.58 - 2.52 (m, 2H), 1.23-1.18 (m, 3H).

### Example 306: Preparation of 2-amino-N',3-dimethyl-N'-(pyrimidin-2-yl)-N-((5-(trifluoromethyl)pyridin-2-yl)methyl) -1,7-naphthyridine-6-carbohydrazide (306)

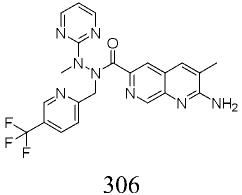

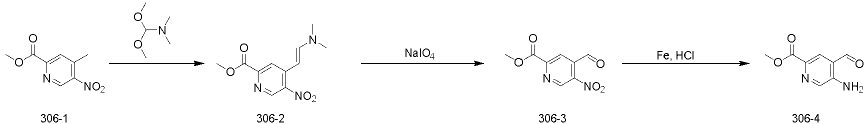

### Step 1: Preparation of methyl (E)-4-(2-(dimethylamino)vinyl)-5-nitropyridine-carboxylate (306-2)

Methyl 4-methyl-5-nitropyridine-carboxylate (306-1, 2.50 g, 12.74 mmol) and 1,1-dimethoxy-N,N-dimethylamine (3.04 g, 25.49 mmol) were dissolved in N-methylformamide (30 mL) solution at room temperature, and reacted at 90°C for 6 hours, and the resulting reaction mixture was diluted with water (300 mL). The resulting mixture was extracted with ethyl acetate (200 mL x 3). The organic phases were combined, washed with saturated brine (300 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 306-2 (2.50 g, yield 78%).

LC-MS (ESI+): 252.0 m/z [M+H]⁺.

### Step 2: Preparation of methyl 4-formyl-5-nitropyridine-carboxylate (306-3)

Compound 306-2 (3.50 g, 13.9 mmol) and sodium periodate (6.02 g, 27.9 mmol) were dissolved in tetrahydrofuran (30 mL) and water (10 mL) at room temperature, and reacted at room temperature for 12 hours, and the resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=10/1) to obtain Compound 306-3 (2.60 g, yield 88%).

LC-MS (ESI+): 211.0 m/z [M+H]⁺.

### Step 3: Preparation of methyl 5-amino-4-formyl pyridine-carboxylate (306-4)

Compound 306-3 (2.00 g, 9.52 mmol), iron powder (1.06 g, 19.0 mmol) and hydrochloric acid (19.0 mmol) were dissolved in a solution of ethanol (20 mL) and water (10 mL) at room temperature, and reacted at 80°C for 12 hours. The resulting reaction mixture was diluted with water (100 mL). The resulting mixture was extracted with ethyl acetate (100 mL x 3). The organic phases were combined, washed with saturated brine (100 mL), dried over anhydrous sodium sulfate, and filtered. The filtrate was concentrated under reduced pressure. The resulting residues were purified by silica gel column chromatography (eluent: dichloromethane/methanol=10/1) to obtain Compound 306-4 (750 mg, yield 51%).

LC-MS (ESI+): 181.0 m/z [M+H]⁺.

Remaining steps were according to the synthetic method of Example 25 to give 2-amino-N',3-dimethyl-N'-(pyrimidin-2-yl)-N-((5-(trifluoromethyl)pyridin-2-yl)methyl) -1,7-naphthyridine-6-carbohydrazide, except that Compound 306-4 was used instead of methyl 4-amino-3-formylbenzoate.

LC-MS (ESI+): 469.2 m/z [M+H]⁺.

### Example 307: Preparation of 2-amino-3-cyano-N'-methyl-N'-(pyrimidin-2-yl)-N-((5-(trifluoromethyl)pyridin-2-yl)me thyl)quinoline-6-carbohydrazide (307)

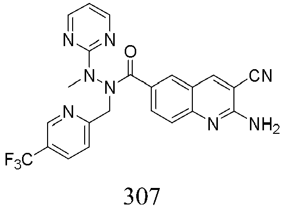

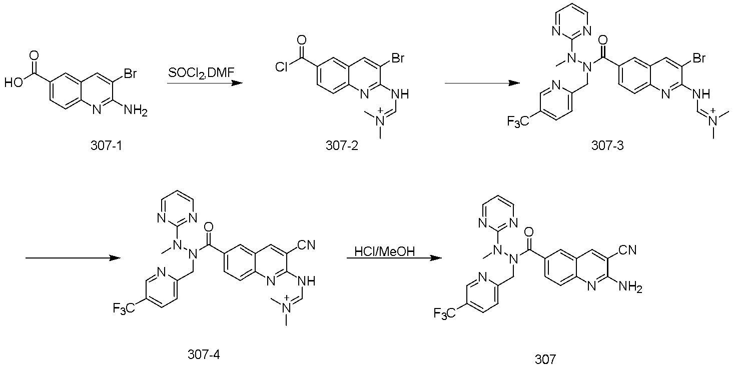

### Step 1: Preparation of N-(((3-bromo-6-(chlorocarbonyl)quinolin-2-yl)amino)methylene)-N-dimethylammoniu m (307-2)

2-Amino-3-bromoquinoline-6-carboxylic acid (307-1, obtained by hydrolysis of 25-3) (600 mg, 2.24 mmol) was dissolved in SOCl₂ (15 mL), followed by the addition of DMF (0.5 mL), and the resulting mixture was stirred at 60°C for 1 hour. The reaction solution was concentrated under reduced pressure to obtain Compound 307-2 (600 mg, yield 93%, a white solid).

LC-MS (ESI+): 341.1 m/z [M+H]⁺.

### Step 2: Preparation of N-(((3-bromo-6-(2-methyl-2-(pyrimidin-2-yl)-1-((5-(trifluoromethyl)pyridin-2-yl)meth yl)hydrazine-1-carbonyl)quinolin-2-yl)amino)methylene)-N-dimethylammonium (307-3)

Compound 307-2 (0.4 g, 1.41 mmol) was dissolved in DCM (5 mL), followed by the addition of DIEA (0.55 g, 4.23 mmol) and 2-(1-methyl-2-((5-(trifluoromethyl)pyridin-2-yl)methyl)hydrazino)pyrimidine (synthesized according to the method for intermediate 137-5 in Example 137) (0.4 g, 1.41 mmol), and the resulting mixture was stirred at room temperature for 1 hour. Water (20 mL) was added to the reaction solution, and the reaction solution was extracted with DCM (20 mL × 3). The organic phases were combined, washed with saturated brine, dried over anhydrous sodium sulfate and filtered. The filtrate was concentrated under reduced pressure, and the residues were purified by silica gel column chromatography (DCM/MeOH = 92/8) to obtain Compound 307-3 (400 mg, yield 53%, a yellow solid).

LC-MS (ESI+): 588.1 m/z [M+H]⁺.

### Step 3: Preparation of N-(((3-cyano-6-(2-methyl-2-(pyrimidin-2-yl)-1-((5-(trifluoromethyl)pyridin-2-yl)methy 1)hydrazine-1-carbonyl)quinolin-2-yl)amino)methylene)-N-dimethylammonium (307-4)

Compound 307-3 (0.1g, 0.188 mmol) was dissolved in 1,4-dioxane (3 mL) in a sealing tube, followed by the addition of CuCN (50 mg, 0.56 mmol), Pd₂ (dba)₃ (17 mg, 0.018 mmol) and DPPF (31 mg, 0.056 mmol) successively, and after purging with N₂, the resulting mixed solution was stirred at 100°C for 12 hours. Ethyl acetate (20 mL) was added to the reaction solution, and the reaction solution was washed with saturated brine (5 mL × 3). The organic phase was dried over anhydrous sodium sulfate and filtered, and the filtrate was concentrated under reduced pressure to obtain Compound 307-4 (60 mg, yield 67%, a yellow solid).

LC-MS (ESI+): 535.2 m/z [M+H]⁺.

### Step 4: Preparation of 2-amino-3-cyano-N'-methyl-N'-(pyrimidin-2-yl)-N-((5-(trifluoromethyl)pyridin-2-yl)me thyl)quinoline-6-carbohydrazide (307)

Compound 307-4 (0.1 g, 0.18 mmol) was dissolved in methanol (1 mL), followed by the addition of 1 N HCl (1mL). The resulting mixture was stirred at 60°C for 1 hour. The reaction solution was concentrated under reduced pressure, and the residues were purified by preparative high performance liquid chromatography (water/acetonitrile=95%/5% - 10%/90%), and subjected to lyophilization to obtain Compound 307 (7.9 mg, yield 9%, a white solid).

LC-MS (ESI+): 479.2 m/z [M+H]⁺.

¹H NMR (400 MHz, DMSO-*d₆*) δ 8.90 (s, 1H), 8.70 (s, 1H), 8.40 (d, *J =* 4.8 Hz, 2H), 8.21 (d, *J =* 8.4 Hz, 1H), 7.93 (s, 1H), 7.85 (d, *J =* 8.4 Hz, 1H), 7.68 (d, *J =* 8.8 Hz, 1H), 7.39 (s, 1H), 7.15 (s, 2H), 6.80 (s, 1H), 5.39 (d, *J=* 15.6 Hz, 1H), 4.62 (d, *J =* 15.6 Hz, 1H), 3.32 (s, 3H).

### Example 308: Preparation of 5-amino-N'-methyl-N'-(pyrimidin-2-yl)-N-((5-(trifluoromethyl)pyridin-2-yl)methyl)ben zo[c][2,7]naphthyridine-9-carbohydrazide (308)

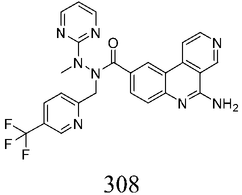

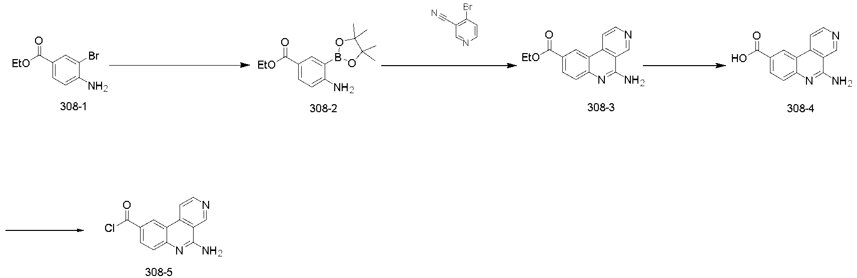

### Step 1: Preparation of ethyl 4-amino-3-(4,4,5,5-tetramethyl-1,3,2-dioxaborolan-2-yl)benzoate (308-2)

Ethyl 4-amino-3-bromobenzoate (308-1, 1.0 g, 4.1 mmol), (BPin)₂ (1.56 mg, 6.2 mmol), [1,1'-bis(diphenylphosphino)ferrocene]palladium dichloride (150 mg, 0.2 mmol) and potassium acetate (1204 mg, 12.3 mmol) were dissolved in 1,4-dioxane (30 mL) at room temperature. The reaction solution was stirred at 90°C for 12 hours under nitrogen atmosphere. After the reaction was completed, water (50 mL) was added to the reaction solution, and then the reaction solution was extracted with ethyl acetate (80 mL x2). The organic phases were combined, washed with saturated brine (60 mL), dried over anhydrous sodium sulfate and filtered, and the filtrate was concentrated to dryness under reduced pressure. The crude product was purified by silica gel column chromatography (eluent: ethyl acetate/petroleum ether=1/2) to obtain Compound 308-2 (900 mg, yield 75%).

LC-MS (ESI+): m/z 292.1 [M+1]⁺.

### Step 2: Preparation of ethyl 5-aminobenzo[c][2,7]naphthyridine-9-carboxylate (308-3)

Compound 308-2 (800 mg, 2.7 mmol), 4-bromonicotinonitrile (553 mg, 3.0 mmol), tetrakis(triphenylphosphine)palladium (158 mg, 0.14 mmol) and potassium carbonate (1138 mg, 8.24 mmol) were dissolved in 1,4-dioxane (20 mL) and water (5 mL) at room temperature. The reaction solution was stirred at 90°C for 12 hours under nitrogen atmosphere. After the reaction was completed, water (40 mL) was added to the reaction solution, and then the reaction solution was extracted with ethyl acetate (50 mL x2). The organic phases were combined, washed with saturated brine (40 mL), dried over anhydrous sodium sulfate and filtered, and the filtrate was concentrated to dryness under reduced pressure. The crude product was purified by silica gel column chromatography (eluent: ethyl acetate) to obtain Compound 308-3 (250 mg, yield 34%).

LC-MS (ESI+): m/z 268.1 [M+1]⁺.

### Step 3: Preparation of 5-aminobenzo[c][2,7]naphthyridine-9-carboxylic acid (308-4)

Compound 308-3 (250 mg, 0.94 mmol) and lithium hydroxide (112 mg, 4.68 mmol) were dissolved in methanol (5 mL) and water (2 mL) at room temperature. The reaction solution was stirred at 50°C for 12 hour. After the reaction was completed, the reaction solution was adjusted to pH 5-6 with 2 N hydrochloric acid, and filtered to obtain Compound 308-4 (200 mg, yield 89%).

LC-MS (ESI+): m/z 240.2 [M+1]⁺.

### Step 4: Preparation of 5-aminobenzo[c][2,7]naphthyridine-9-carbonyl chloride (308-5)

Compound 308-4 (200 mg, 2.24 mmol) was dissolved in SOCl₂ (15 mL), followed by the addition of DMF (1 drop), and the resulting mixture was stirred at 60°C for 1 hour. The reaction solution was concentrated under reduced pressure to obtain Compound 308-5 (a crude product, 200 mg, a white solid), which was directly used for the next step.

5-Amino-*N'*-methyl-*N'*-(pyrimidin-2-yl)-*N*-((5-(trifluoromethyl)pyridin-2-yl)methy 1)benzo[c][2,7]naphthyridine-9-carbohydrazide was synthesized according to the method of Example 137, except that Compound 308-5 was used instead of compound 2-amino-3-methylquinoline-6-carbonyl chloride, and (5-(trifluoromethyl)pyridin-2-yl)methanol was used instead of Compound 137-1.

LC-MS (ESI+): m/z 505.2 [M+1]⁺.

¹H NMR (400 MHz, DMSO-*d₆*) δ 9.64 (s, 1H), 8.93 (s, 1H), 8.86 (d, *J =* 5.6 Hz, 1H), 8.58 (s, 1H), 8.48 - 8.41 (m, 2H), 8.37 (d, *J =* 5.6 Hz, 1H), 8.28 - 8.22 (m, 1H), 7.96 (d, *J =* 8.4 Hz, 1H), 7.68 (d, *J =* 8.4 Hz, 1H), 7.58 (s, 2H), 7.43 (d, *J =* 8.8 Hz, 1H), 6.82 (t, *J =* 4.8 Hz, 1H), 5.45 (d, *J =* 15.6 Hz, 1H), 4.68 (d, *J =* 15.6 Hz, 1H), 3.33 (s, 3H).

### Example 309: Preparation of 2-amino-N'-(3-fluoropyridin-2-yl)-N'-methyl-3-(methyl-d3)-N-((5-(trifluoromethyl)pyri din-2-yl)methyl)quinoline-6-carbohydrazide (309)

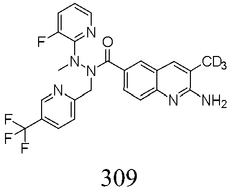

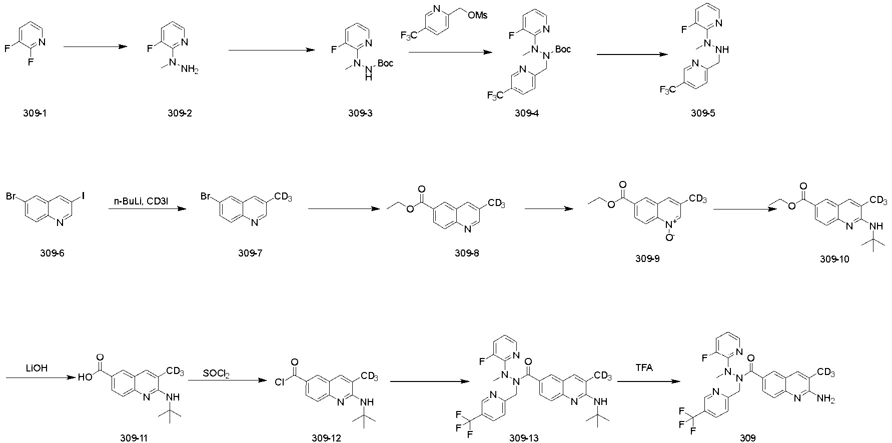

### Step 1: Preparation of 3-fluoro-2-(1-methylhydrazino)pyridine (309-2)

2,3-Difluoropyridine (309-1) (10 g, 87 mmol) was dissolved in 100 mL of ethanol at room temperature,=, followed by the addition of methylhydrazine sulfate (12 g, 87 mmol) and sodium carbonate (28 g, 261 mmol). The reaction was carried out at 85°C under nitrogen atmosphere overnight. The reaction mixture was filtered, and concentrated under reduced pressure to obtain Compound 309-2 (7 g, yield 57%).

LC-MS (ESI+): 142.2 m/z [M+H]⁺.

### Step 2: Preparation of tert-butyl 2-methyl-2-(pyrimidin-2-yl)hydrazine-1-carboxylate (309-3)

Compound 309-2 (13 g, 92.10 mmol) was dissolved in 150 mL of tetrahydrofuran and 150 mL of water at room temperature, followed by the addition of di-tert-butyl dicarbonate (30 g, 138 mmol) and sodium carbonate (29 g, 276 mmol). The reaction was carried out at room temperature under nitrogen atmosphere overnight. The reaction mixture was filtered, and concentrated under reduced pressure, and the resulting mixture was extracted with ethyl acetate (200 mL x2). The organic phases were combined, dried over sodium sulfate, filtered and concentrated, and the resulting residues were purified by silica gel column chromatography (eluent: petroleum ether/ethyl acetate=100%-50%) to obtain Compound 309-3 (19 g, yield 85%).

LC-MS (ESI+): 242.2 m/z [M+H]⁺.

### Step 3: Preparation of tert-butyl 2-(3-fluoropyridin-2-yl)-2-methyl-1-((5-(trifluoromethyl)pyridin-2-yl)methyl)hydrazine -1-carboxylate (309-4)

Compound 309-3 (14 g, 58 mmol) was dissolved in 150 mL of DMF at room temperature, followed by the addition of potassium carbonate (24 g, 174 mmol) and methyl (5-(trifluoromethyl)pyridin-2-yl)methanesulfonate (22 g, 87 mmol). The reaction was carried out at 65°C under nitrogen atmosphere overnight. 200 ML of water was added to the reaction mixture, and the resulting mixture was extracted with ethyl acetate (200 ml x2). The organic phases were combined, dried over sodium sulfate, filtered and concentrated, and the resulting residues were purified by silica gel column chromatography (eluent: petroleum ether/ethyl acetate=100%-50%) to obtain Compound 309-4 (14 g, yield 61 %).

LC-MS (ESI+): 401.2 m/z [M+H]⁺.

### Step 4: Preparation of 3-fluoro-2-(1-methyl-2-((5-(trifluoromethyl)pyridin-2-yl)methyl)hydrazino)pyridine (309-5)

Compound 309-4 (14 g, 34 mmol) was dissolved in 150 mL of dichloromethane at room temperature, followed by the addition of 4M hydrochloric acid-dioxane solution (20 mL). The reaction was carried out at room temperature under nitrogen atmosphere for 1 hour. The reaction mixture was concentrated under reduced pressure to obtain Compound 309-5 (hydrochloride, 13 g, yield 87%).

LC-MS (ESI+): 301.2 m/z [M+H]⁺.

### Step 5: Preparation of 6-bromo-3-(methyl-d3)quinoline (309-7)

6-Bromo-3-iodoquinoline (309-6, 10 g, 29.9 mmol) was dissolved in THF (200 mL), followed by the addition of n-butyllithium (13.2 mL, 33 mmol) at -78°C. The mixture was reacted at low temperature for 0.5 hour, followed by the dropwise addition of ICD3 (4.3 g, 29.9 mmol) dissolved in THF (20 mL) to the reaction solution. The mixture was further reacted at low temperature for 0.5 hour, then returned to room temperature and reacted for 2 hours. Aqueous ammonium chloride solution (100 mL) was added to the reaction solution to quench the reaction, and the reaction solution was extracted with ethyl acetate (300 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 5/1) to obtain Compound 309-7 (8 g, yield 80%, a colourless oil).

LC-MS (ESI+): 225.1 m/z [M+H]⁺.

### Step 6: Preparation of ethyl 3-(methyl-d3)quinoline-6-carboxylate (309-8)

Compound 309-7 (25 g, 111.0 mmol) was dissolved in EtOH (250 mL), followed by the addition of molybdenum hexacarbonyl (14.6 g, 55.5 mmol), TEA (33.6 g, 333.2 mmol), Pd (dppf)Cl₂ (9.0 g, 11.1 mmol) and Xantphos (12.8 g, 22.2 mmol). The mixture was purged with N₂, and reacted at 85°C for 6 hours. MeOH and DCM were added to the reaction solution, and the reaction solution was filtered and concentrated under reduced pressure. The resulting residues were purified by silica gel column chromatography (petroleum ether/ethyl acetate = 5/1) to obtain Compound 309-8 (20 g, yield 88%, a yellow oil).

LC-MS (ESI+): 219.1 m/z [M+H]⁺.

### Step 7: Preparation of 6-(ethoxycarbonyl)-3-(methyl-d3)quinoline 1-oxide (309-9)

Compound 309-8 (22.0 g, 101 mmol) was dissolved in DCM (250 mL), followed by the addition of 3-chloroperbenzoic acid (26.0 g, 151 mmol) at 0°C, and the resulting mixture was stirred at room temperature for 2 hours. The reaction solution was extracted with DCM (100 mL) and sodium bicarbonate aqueous solution (100 mL × 3). The organic phase was concentrated under reduced pressure to obtain Compound 309-9 (23 g, yield 97%, a yellow solid).

LC-MS (ESI+): 234.2 m/z [M+H]⁺.

### Step 8: Preparation of ethyl 2-(tert-butylamino)-3-(methyl-d3)quinoline-6-carboxylate (309-10)

Compound 309-9 (23 g, 104.4 mmol) was dissolved in DCM (250 mL), followed by the addition of tert-butylamine (34.3 g, 470 mmol) and 4-toluenesulfonic anhydride (59.5 g, 183 mmol) at 0°C, and the reaction solution was stirred at room temperature for 12 hours. DCM (100 mL) was added to the reaction solution, and the reaction solution was extracted with sodium bicarbonate aqueous solution (100 mL × 3). The organic phase was washed with saturated brine, dried over anhydrous sodium sulfate and filtered. The filtrate was concentrated under reduced pressure, and the residues were purified by silica gel column chromatography (petroleum ether/ethyl acetate = 20/1) to obtain Compound 309-10 (8.2g, yield 30%, a yellow solid).

LC-MS (ESI+): 290.4 m/z [M+H]⁺.

### Step 9: Preparation of 2-(tert-butylamino)-3-(methyl-d3)quinoline-6-carboxylic acid (309-11)

Compound 309-10 (8.0 g, 34.3 mmol) was dissolved in THF/H₂O/MeOH (50/50/50 mL), followed by the addition of lithium hydroxide monohydrate (4.3 g, 102.9 mmol), and the resulting mixture was stirred at 50°C for 2 hours. The reaction solution was concentrated under reduced pressure to remove some solvent, and adjusted to pH~3 with 1N HCl. A solid was precipitated and filtered to obtain a product, which was subjected to lyophilization to obtain Compound 309-11 (6 g, yield 85%, a white solid).

LC-MS (ESI-): 262.3 m/z [M-H]⁻.

### Step 10: Preparation of 2-(tert-butylamino)-3-(methyl-D3)quinoline-6-carbonyl chloride (309-12)

Compound 309-11 (300 mg, a crude product) was dissolved in 5 mL of dichloromethane at room temperature, followed by the addition of 1 mL of thionyl chloride, and reacted at room temperature for 1 hour under nitrogen atmosphere. The reaction mixture was concentrated under reduced pressure to obtain Compound 309-12 (300 mg, a crude product).

### Step 11: Preparation of 2-(tert-butylamino)-N'-(3-fluoropyridin-2-yl)-N'-methyl-3-(methyl-d3)-N-((5-(trifluoro methyl)pyridin-2-yl)methyl)quinoline-6-carbohydrazide (309-13)

Compound 309-5 (100 mg, 0.33 mmol) was dissolved in 5 mL of dichloromethane at room temperature, followed by the addition of N,N-diisopropylethylamine (129 mg, 1.0 mmol), and then Compound 309-12 (a crude product, 100 mg) was added in an ice bath. The reaction was carried out at room temperature for 1 hour under nitrogen atmosphere. The reaction mixture was concentrated under reduced pressure, and the resulting residues were purified by silica gel column chromatography (eluent: dichloromethane/methanol=0-10%) to obtain Compound 309-13 (150 mg, yield 65%).

LC-MS (ESI+): 544.2 m/z [M+H]⁺.

### Step 12: Preparation of 2-amino-N'-(3-fluoropyridin-2-yl)-N'-methyl-3-(methyl-d3)-N-((5-(trifluoromethyl)pyri din-2-yl)methyl)quinoline-6-carbohydrazide (309)

Compound 309-13 (150 mg, 0.28 mmol) was dissolved in 5 mL of dichloromethane at room temperature, followed by the addition of 2 mL of trifluoroacetic acid, and reacted at room temperature for 1 hour under nitrogen atmosphere. The reaction mixture was concentrated under reduced pressure, and the resulting residues were purified by high performance liquid chromatography (water/acetonitrile=95%/5% - 10%/90%) to obtain Compound 309 (12 mg, yield 9%).

LC-MS (ESI+): 488.2 m/z [M+H]⁺.

¹H NMR (400 MHz, DMSO-*d₆*) δ 8.21 (d, *J =* 6.1 Hz, 2H), 8.01 (d, *J =* 4.7 Hz, 1H), 7.89 - 7.74 (m, 3H), 7.61 (d, *J =* 8.7 Hz, 1H), 7.49 (dd, *J =* 13.9, 8.1 Hz, 1H), 7.38 (d, *J =* 8.8 Hz, 1H), 6.91 (ddd, *J =* 7.8, 4.7, 2.8 Hz, 1H), 6.53 (s, 2H), 5.32 (d, *J =* 15.9 Hz, 1H), 4.69 (d, *J =* 15.8 Hz, 1H).

The following compounds were obtained according to the synthetic method in Example 309 using corresponding raw materials.

| Example | Structure | Mass spectrometry data (ESI+): m/z | Nuclear magnetic data |
|---|---|---|---|
| 310 | 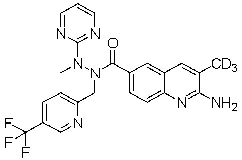 | 471.2 | |
| 311 | 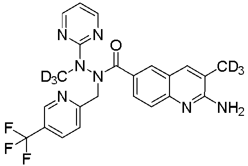 | 474.2 | |
| 312 | 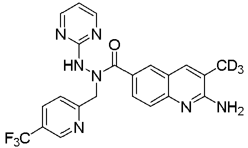 | 457.2 | ¹HNMR (400 MHz, DMSO-*d₆*) δ 10.90 (s, 1H), 8.47 (d, *J =* 4.8 Hz, 2H), 8.27 - 8.10 (m, 3H), 7.94 (dd, *J* = 8.7, 2.1 Hz, 1H), 7.91 - 7.81 (m, 2H), 7.52 (d, *J =* 8.7 Hz, 1H), 6.88 (d, *J =* 4.8 Hz, 1H), 5.25 (s, 2H). |
| 313 | 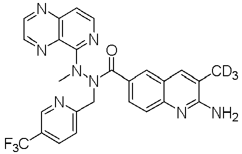 | 522.2 | ¹HNMR (400 MHz, DMSO-*d₆*) δ 8.98 (s, 1H), 8.85 (s, 1H), 8.81 (s, 1H), 8.27 (d, *J =* 4.0 Hz, 1H), 8.15 (d, *J =* 8.0 Hz, 1H), 7.82 (s, 1H), 7.78 (d, *J* = 8.0 Hz, 1H), 7.66 (s, 1H), 7.56 (d, *J =* 8.0 Hz, 1H), 7.30 (d, *J* = 4.0 Hz, 1H), 7.26 (d, *J =* 8.0 Hz, 1H), 6.45 (s, 2H), 5.38 (d, *J =* 16.0 Hz, 1H), 4.69 (d, *J =* 16.0 Hz, 1H), 3.66 (s, 3H). |
| 314 | 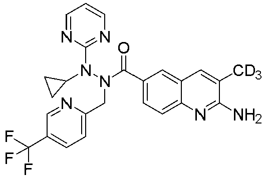 | 497.2 | ¹HNMR (400 MHz, DMSO-*d₆*) δ 8.84 (s, 1H), 8.57 (d, *J* = 4.4 Hz, 2H), 8.23-8.20 (m, 1H), 7.92 (d, *J* = 8.4 Hz, 1H), 7.69 (d, *J* = 1.6 Hz, 1H), 7.60 (s, 1H), 7.43-7.40 (m, 1H), 7.27 (d, *J =* 8.8 Hz, 1H), 7.01 (t, *J =* 4.8 Hz, 1H), 6.51 (s, 2H), 5.34 (d, *J =* 15.6 Hz, 1H), 4.67 (d, *J =* 15.6 Hz, 1H), 2.79-2.74 (m, 1H), 0.77-0.67 (m, 1H), 0.45-0.39 (m, 2H), 0.18-0.12 (m, 1H). |

### Example 315: Preparation of N-((5-(1,3,4-thiadiazol-2-yl)pyridin-2-yl)methyl)-2-amino-N'-cyclopropyl-3-(methyl-d3 )-N'-(pyrimidin-2-yl)quinoline-6-carbohydrazide (315)

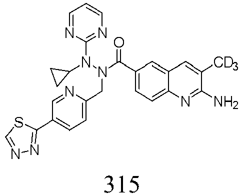

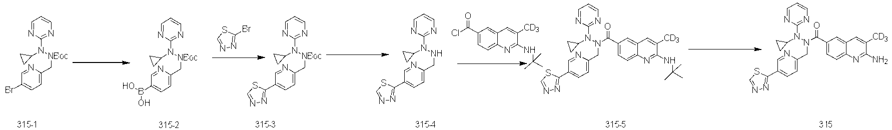

### Step 1: Preparation of (6-((1-(tert-butoxycarbonyl)-2-cyclopropyl-2-(pyrimidin-2-yl)hydrazino)methyl)pyridin -3-yl)boronic acid (315-2)

Compound 315-1 (synthesized according to the method in Example 137) (300 mg, 0.714 mmol) was dissolved in dioxane (5 mL), followed by the addition of B₂Pin₂ (272 mg, 1.07 mmol), KOAc (140 mg, 1.43 mmol) and Pd (dppf)Cl₂ (52 mg, 0.071 mmol) successively at room temperature. The mixture was reacted under stirring at 90°C overnight under N₂ atmosphere. After the reaction was completed, the reaction mixture (315-2) as a crude product was directly used for the next step.

LC-MS (ESI+): 386.2 m/z [M+H]⁺.

### Step 2: Preparation of tert-butyl 1-((5-(1,3,4-thiadiazol-2-yl)pyridin-2-yl)methyl)-2-cyclopropyl-2-(pyrimidin-2-yl)hydra zine-1-carboxylate (315-3)

Compound 315-2 (275 mg, 0.714 mmol) was dissolved in dioxane (5 mL) and H₂O (1 mL), followed by the addition of 2-bromo-1,3,4-thiadiazole (118 mg, 0,714 mmol), Pd (dppf)Cl₂ (78 mg, 0.107 mmol) and K₂CO₃ (296 mg, 2.14 mmol) successively at room temperature. The mixture was reacted under stirring at 100°C overnight under N₂ atmosphere. After the reaction was completed, the reaction mixture was diluted with an appropriate amount of aqueous solution, and extracted with ethyl acetate (20 mL x 3). The organic phases were collected, washed with saturated brine (20 mL), dried over anhydrous sodium sulfate and filtered. The filtrate was concentrated under reduced pressure, and the residues were purified by column chromatography to obtain Compound 315-3.

LC-MS (ESI+): 426.5 m/z [M+H]⁺.

### Step 3: Preparation of 2-(6-((2-cyclopropyl-2-(pyrimidin-2-yl)hydrazino)methyl)pyridin-3-yl)-1,3,4-thiadiazol e (315-4)

Compound 315-3 (100 mg, 0.515 mmol) was dissolved in HCl-2.5 M ethyl acetate solution (5 mL), and reacted under stirring at 40°C for 1h. After the reaction was completed, the reaction mixture was concentrated under reduced pressure to obtain a crude product 315-4 (hydrochloride), which was directly used for the next step.

LC-MS (ESI+): 326.5 m/z [M+H]⁺

### Step 4: Preparation of N-((5-(1,3,4-thiadiazol-2-yl)pyridin-2-yl)methyl)-2-(tert-butylamino)-N'-cyclopropyl-3-(methyl-d3)-N'-(pyrimidin-2-yl)quinoline-6-carbohydrazide (315-5)

Compound 315-4 (60 mg, 0.184 mmol) was dissolved in DCM (5 mL), followed by the addition of DIPEA (119 mg, 0.922 mmol) at room temperature. The mixture was reacted under stirring at room temperature for 5 minutes, followed by the addition of 309-12 (41 mg, 0.148 mmol). The mixture was reacted under stirring at 40°C for 1h. After the reaction was completed, the reaction mixture was concentrated under reduced pressure to obtain a crude product (315-5) which was directly used for the next step.

LC-MS (ESI+): 596.5 m/z [M+H]⁺.

### Step 5: Preparation of N-((5-(1,3,4-thiadiazol-2-yl)pyridin-2-yl)methyl)-2-amino-N'-cyclopropyl-3-(methyl-d3 )-N'-(pyrimidin-2-yl)quinoline-6-carbohydrazide (315)

Compound 315-5 (80 mg, 0.141 mmol) was dissolved in TFA (3 mL), and reacted under stirring at 70°C for 3h. After the reaction was completed, the reaction mixture was concentrated under reduced pressure, and the crude product was purified by preparative high performance liquid chromatography (water/acetonitrile=95%/5% - 10%/90%) to obtain Compound 315.

LC-MS (ESI+): 540.6 m/z [M+H]⁺.

¹H NMR (400 MHz, DMSO-*d₆*) δ 8.58 (d, *J =* 4.8 Hz, 2H), 8.51 (d, *J* = 2.4 Hz, 1H), 7.90-7.86 (m, , 1H), 7.73 (d, *J =* 8.1 Hz, 1H), 7.70 (d, *J =* 2.0 Hz, 1H), 7.60 (s, 1H), 7.43-7.40 (m, 1H), 7.27 (d, *J =* 8.7 Hz, 1H), 7.00 (t, *J =* 4.8 Hz, 1H), 6.49 (s, 2H), 5.33 (d, *J* = 15.1 Hz, 1H), 4.63 (d, *J =* 15.1 Hz, 1H), 2.65 (s, 3H), 2.80 - 2.73 (m, 1H), 2.38 (s, 3H), 0.77 - 0.67 (m, 1H), 0.55 - 0.39 (m, 2H), 0.14 - 0.07 (m, 1H).

The following compounds were obtained according to the synthetic method in Example 315 using corresponding raw materials.

| Example | Structure | Mass spectrometry data (ESI+): m/z | Nuclear magnetic data |
|---|---|---|---|
| 316 | 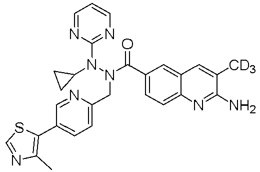 | 526.6 | ¹HNMR (400 MHz, DMSO-*d₆*) δ 9.08 (s, 1H), 8.58 - 8.53 (m, 3H), 8.15 (s, 1H), 7.96 - 7.87 (m, 2H), 7.76 - 7.67 (m, 2H), 7.53 (d, *J =* 8.6 Hz, 1H), 7.00 (t, *J* = 4.8 Hz, 1H), 5.31 (d, *J* = 15.0 Hz, 1H), 4.68 (d, *J* = 15.0 Hz, 1H), 2.81-2.78 (m, 1H), 2.46 (s, 3H), 0.79-0.75 (m, 1H), 0.58 - 0.45 (m, 2H), 0.16-0.13 (m, 1H). |
| 317 | 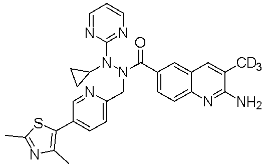 | 540.6 | ¹H NMR (400 MHz, DMSO-*d₆*) δ 8.58 (d, *J* = 4.8 Hz, 2H), 8.51 (d, *J =* 2.4 Hz, 1H), 7.90-7.86 (m, , 1H), 7.73 (d, *J =* 8.1 Hz, 1H), 7.70 (d, *J* = 2.0 Hz, 1H), 7.60 (s, 1H), 7.43-7.40 (m, 1H), 7.27 (d, *J* = 8.7 Hz, 1H), 7.00 (t, *J =* 4.8 Hz, 1H), 6.49 (s, 2H), 5.33 (d, *J =* 15.1 Hz, 1H), 4.63 (d, *J =* 15.1 Hz, 1H), 2.65 (s, 3H), 2.80 - 2.73 (m, 1H), 2.38 (s, 3H), 0.77 - 0.67 (m, 1H), 0.55 - 0.39 (m, 2H), 0.14 - 0.07 (m, 1H). |
| 318 | 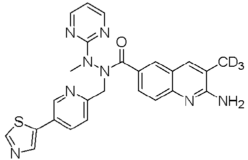 | 486.2 | |

### Example 319: Preparation of 2-amino-3-methoxy-N'-methyl-N'-(pyrimidin-2-yl)-N-((5-(trifluoromethyl)pyridin-2-yl) methyl)quinoline-6-carbohydrazide (319)

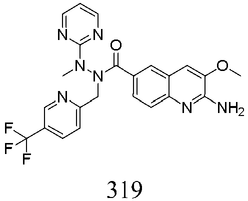

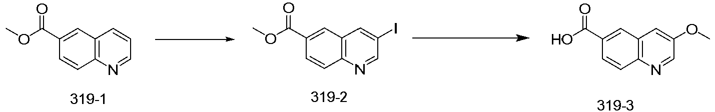

### Step 1: Preparation of methyl 3-iodoquinoline-6-carboxylate (319-2)

Methyl quinoline-6-carboxylate (319-1, 1.0 g, 5.35 mmol) was added to AcOH (9.0 mL)/NIS (1.68 g, 7.49 mmol) solution under nitrogen atmosphere. The resulting mixture was stirred at 100°C for 16 hours. The reaction mixture was concentrated under reduced pressure. The crude product was purified by silica gel column chromatography (eluent: petroleum ether/ethyl acetate=5/1) to obtain methyl 3-iodoquinoline-6-carboxylate (800 mg, yield 48%).

LC-MS (ESI+): 204.0 m/z [M+H]⁺.

### Step 2: Preparation of 3-methoxyquinoline-6-carboxylic acid (319-3)

Compound 319-2 (500 mg, 1.59 mmol), cuprous iodide (30 mg, 0.16 mmol), and a solution of sodium methoxide in methanol (431 mg, 7.98 mmol) were dissolved in N-methylformamide (10 mL) solution at room temperature, and reacted at 90°C for 12 hours, and the resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure, and the resulting residues were purified by silica gel column chromatography (eluent: petroleum ether/ethyl acetate=1/1) to obtain Compound 319-3 (200 mg, yield 61%).

LC-MS (ESI+): 204.0 m/z [M+H]⁺.

2-Amino-3-methoxy-N'-methyl-N'-(pyrimidin-2-yl)-N-((5-(trifluoromethyl)pyridin -2-yl)methyl)quinoline-6-carbohydrazide was synthesized according to the route of Example 309, except that Compound 319-3 was used instead of Compound 309-8.

LC-MS (ESI+): 484.1 m/z [M+H]⁺.

¹H NMR (400 MHz, DMSO-*d₆*) δ 8.89 (s, 1H), 8.39 (d, *J =* 8.0 Hz, 2H), 8.21 (d, *J* = 8.0 Hz, 1H), 7.93 (s, 1H), 7.83 (d, *J =* 12.0 Hz, 1H), 7.73 (s, 1H), 7.58 (d, *J =* 8.0 Hz, 1H), 7.48 (d, *J =* 8.0 Hz, 1H), 6.80 (t, *J =* 4.0 Hz, 1H), 5.39 (d, *J =* 16.0 Hz, 1H), 4.60 (d, *J* = 16.0 Hz, 1H), 3.97 (s, 3H), 3.32 (s, 3H).

### Example 320: Preparation of 2-amino-N'-cyclopropyl-3-(hydroxymethyl)-N'-(pyrimidin-2-yl)-N-((5-(trifluoromethyl) pyridin-2-yl)methyl)quinoline-6-carbohydrazide (320)

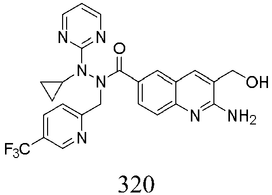

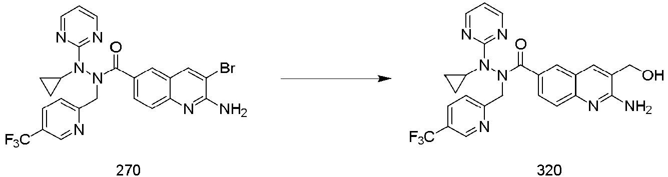

### Step 1: Preparation of 2-amino-N'-cyclopropyl-3-(hydroxymethyl)-N'-(pyrimidin-2-yl)-N-((5-(trifluoromethyl) pyridin-2-yl)methyl)quinoline-6-carbohydrazide (320)

Compound 270 (200 mg, 0.358 mmol) was dissolved in 1,4-dioxane (2 mL) in a sealing tube, followed by the addition of (tributylstannyl)methanol (115 mg, 0.358 mmol) and Pd (PPh₃)₄ (41 mg, 0.035 mmol) successively, and after purging with N₂, the resulting mixed solution was stirred at 100°C for 2 hours. Ethyl acetate (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by silica gel column chromatography (DCM/MeOH = 10/1) to obtain a crude product, which was purified by preparative high performance liquid chromatography (water/acetonitrile=95%/5% - 10%/90%) and subjected to lyophilization to obtain Compound 320 (8.0 mg, yield 5%, a white solid).

LC-MS (ESI+): 510.2 m/z [M+H]⁺.

The following compounds were obtained according to the synthetic method in Example 320, using corresponding raw materials.

| Example | Structure | Mass spectrometry data (ESI+): m/z | Nuclear magnetic data |
|---|---|---|---|
| 321 | 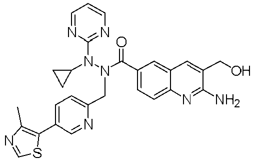 | 539.2 | |
| 322 | 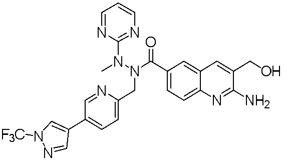 | 550.2 | ¹H NMR (400 MHz, DMSO-*d₆*) δ 9.11 (s, 1H), 8.93 (s, 1H), 8.57 (s, 1H), 8.41 (d, J = 4.8 Hz, 2H), 8.15 (dd, J = 8.0, 2.3 Hz, 1H), 7.81 (d, J = 17.6 Hz, 2H), 7.63 (d, J = 8.0 Hz, 1H), 7.49 (d, J = 8.8 Hz, 1H), 7.33 (d, J = 8.8 Hz, 1H), 6.79 (d, J = 5.2 Hz, 1H), 6.38 (s, 2H), 5.44 (d, J = 15.2 Hz, 2H), 4.46 (s, 1H), 4.44 (d, J = 6.4 Hz, 2H), 3.19 (s, 3H). |

### Example 323: Preparation of 2-amino-N'-cyclopropyl-3-(fluoromethyl)-N'-(pyrimidin-2-yl)-N-((5-(trifluoromethyl)p yridin-2-yl)methyl)quinoline-6-carbohydrazide (323)

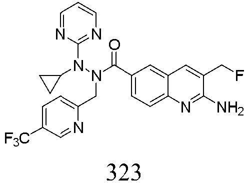

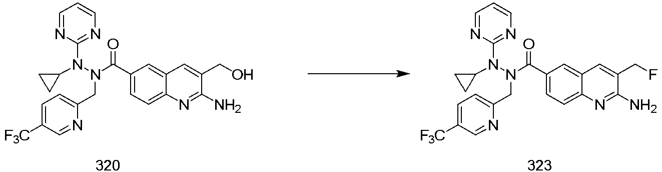

### Step 1: Preparation of 2-amino-N'-cyclopropyl-3-(fluoromethyl)-N'-(pyrimidin-2-yl)-N-((5-(trifluoromethyl)p yridin-2-yl)methyl)quinoline-6-carbohydrazide (323)

Compound 320 (50 mg, 0.098 mmol) was dissolved in DCM (2 mL), followed by the addition of diethylaminosulphur trifluoride (0.2 mL), and the resulting mixed solution was stirred at room temperature for 2 hours. DCM (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 323 (6.1 mg, yield 12%, a white solid).

LC-MS (ESI+): 512.2 m/z [M+H]⁺.

¹H NMR (400 MHz, DMSO-*d₆*) δ 8.67 (s, 1H), 8.41 (d, J = 4.8 Hz, 2H), 8.05 (dd, J = 8.4, 2.4 Hz, 1H), 7.75 (d, J = 8.0 Hz, 2H), 7.64 (s, 1H), 7.33 (s, 1H), 7.15 (d, J = 8.8 Hz, 1H), 6.85 (t, J = 4.8 Hz, 1H), 6.55 (s, 2H), 5.28 (d, J = 48 Hz, 1H), 5.18 (d, J = 15.6 Hz, 1H),4.52 (d, J = 15.6 Hz, 1H), 2.61 (s, 1H), 0.59 (s, 1H), 0.41 - 0.22 (m, 2H), 0.02-0.00 (m, 1H).

The following compound was obtained according to the synthetic method in Example 323, using corresponding raw materials.

| Example | Structure | Mass spectrometry data (ESI+): m/z | Nuclear magnetic data |
|---|---|---|---|
| 324 | 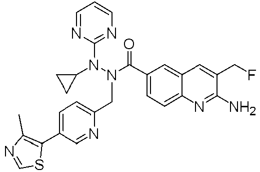 | 541.2 | |

### Example 325: Preparation of 2-amino-3-hydroxymethyl-N'-methyl-N'-(pyrimidin-2-yl)-N-((5-(trifluoromethyl)pyridi n-2-yl)methyl)quinoline-6-carbohydrazide (325)

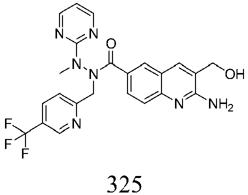

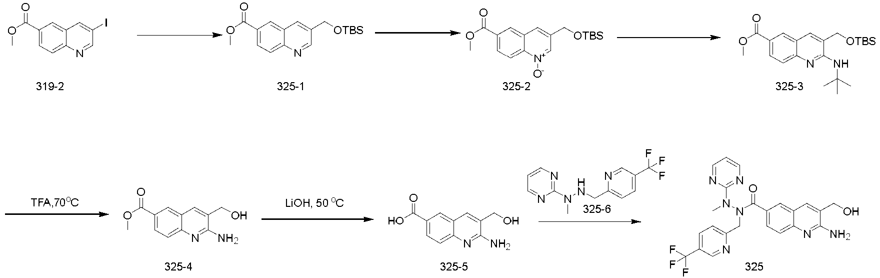

### Step 1: Preparation of methyl 3-(( (tert-butyldimethylsilyl)oxy)methyl)quinoline-6-carboxylate (325-1)

Methyl 3-iodoquinoline-6-carboxylate (319-2) (500 mg, 1.60 mmol) was dissolved in 5 mL of toluene at room temperature, followed by the addition of tert-butyldimethyl-(tributylstannylmethoxy)silane (1.39 g, 3.20 mmol), lithium chloride (206 mg, 4.80 mmol) and bis(triphenylphosphine)palladium dichloride (103 mg, 0.16 mmol). The reaction was carried out at 100°C overnight under nitrogen atmosphere. Water was added to the reaction mixture, and the reaction mixture was filtered through Celite, and then the filtrate was extracted with ethyl acetate (50 mL x 2). The organic phases were combined, dried over anhydrous sodium sulfate and filtered. The filtrate was concentrated to dryness under reduced pressure, and the resulting residues were purified by silica gel column chromatography (eluent: petroleum ether/ethyl acetate=100%-50%) to obtain 325-1 (450 mg, yield 85%).

LC-MS (ESI+): 332.2 m/z [M+H]⁺.

### Step 2: Preparation of 3-(( (tert-butyldimethylsilyl)oxy)methyl)-6-(methoxycarbonyl)quinoline-1-oxide (325-2)

Compound 325-1 (450 mg, 1.36 mmol) was dissolved in 5 mL of dichloromethane at room temperature, followed by the addition of 3-chloroperbenzoic acid (700 mg, 4.07 mmol), and reacted in an ice bath overnight under nitrogen atmosphere. The reaction mixture was quenched with aqueous sodium sulfite solution, and extracted with ethyl acetate (50 mL x2). The organic phases were combined, then washed with saturated sodium bicarbonate solution and saturated brine, dried over anhydrous sodium sulfate and filtered. The filtrate was concentrated to dryness under reduced pressure, and the resulting residues were purified by silica gel column chromatography (eluent: petroleum ether/ethyl acetate=100%-50%) to obtain Compound 325-2 (300 mg, yield 64%).

LC-MS (ESI+): 348.2 m/z [M+H]⁺.

### Step 3: Preparation of methyl 2-(tert-butylamino)-3-(( (tert-butyldimethylsilyl)oxy)methyl)quinoline-6-carboxylate (325-3)

Compound 325-2 (450 mg, 1.36 mmol) was dissolved in 5 mL of dichloromethane at room temperature, followed by the addition of tert-butylamine (315 mg, 4.32 mmol) in an ice bath. The mixture was stirred for 10 minutes, followed by the addition of methanesulfonic anhydride (563 mg, 1.73 mmol). The reaction was carried out in an ice bath overnight under nitrogen atmosphere. The reaction mixture was concentrated to dryness under reduced pressure, and the resulting residues were purified by silica gel column chromatography (eluent: petroleum ether/ethyl acetate=100%-50%) to obtain Compound 325-3 (280 mg, yield 85%).

LC-MS (ESI+): 403.2 m/z [M+H]⁺.

### Step 4: Preparation of methyl 2-amino-3-hydroxymethylquinoline-6-carboxylate (325-4)

Compound 325-3 (280 mg, 0.70 mmol) was dissolved in 5 mL of dichloromethane at room temperature, followed by the addition of 2 mL of trifluoroacetic acid in an ice bath. The mixture was reacted at 70°C overnight under nitrogen atmosphere. The reaction mixture was concentrated to dryness under reduced pressure, and the resulting residues were purified by silica gel column chromatography (eluent: dichloromethane/methanol=0-10%) to obtain Compound 325-4 (180 mg, yield 98%).

LC-MS (ESI+): 233.2 m/z [M+H]⁺.

### Step 5: Preparation of 2-amino-3-hydroxymethylquinoline-6-carboxylic acid (325-5)

Compound 325-4 (180 mg, 0.78 mmol) was dissolved in 5 mL of methanol at room temperature, followed by the addition of 3 ml of 5% lithium hydroxide solution. The mixture was reacted for 3 hours under nitrogen atmosphere. The reaction mixture was adjusted to neutral with 1N hydrochloric acid, and concentrated to dryness under reduced pressure to obtain Compound 325-5 (100 mg, a crude product).

LC-MS (ESI+): 219.2 m/z [M+H]⁺.

### Step 6: Preparation of 2-amino-3-hydroxymethyl-N'-methyl-N'-(pyrimidin-2-yl)-N-((5-(trifluoromethyl)pyridi n-2-yl)methyl)quinoline-6-carbohydrazide (325)

Compound 325-5 (50 mg, 0.23 mmol) was dissolved in 5 mL DMA at room temperature, followed by the addition of 2-(1-methyl-2-((5-(trifluoromethyl)pyridin-2-yl)methyl)hydrazino)pyrimidine (325-6, synthesized according to the method in Example 137) (65 mg, 0.23 mmol), bromo-tris-pyrrolidino-phosphonium hexafluorophosphate (160 mg, 0.34 mmol) and N,N-diisopropylethylamine (89 mg, 0.69 mmol). The reaction was carried out overnight under nitrogen atmosphere. The reaction solution was filtered and concentrated. The residues were purified by high performance liquid chromatography (water/acetonitrile=95%/5% - 10%/90%) to obtain Compound 325 (7.2 mg, yield 6.4%).

LC-MS (ESI+): 484.2 m/z [M+H]⁺.

¹H NMR (400 MHz, DMSO-*d₆*) δ 8.41 (d, *J =* 4.8 Hz, 2H), 8.34 (s, 1H), 8.23 (dd, *J =* 8.5, 2.4 Hz, 1H), 7.87 (dd, *J =* 16.6, 10.0 Hz, 3H), 7.52 (d, *J =* 8.8 Hz, 1H), 7.35 (d, *J =* 8.7 Hz, 1H), 6.81 (t, *J* = 4.7 Hz, 1H), 6.42 (s, 2H), 5.42 (d, *J =* 15.6 Hz, 1H), 4.62 (d, *J =* 15.6 Hz, 1H), 4.46 (s, 2H).

### Example 326: Preparation of 2-amino-3-(methoxymethyl)-N'-methyl-N'-(pyrimidin-2-yl)-N-((5-(trifluoromethyl)pyri din-2-yl)methyl)quinoline-6-carbohydrazide (326)

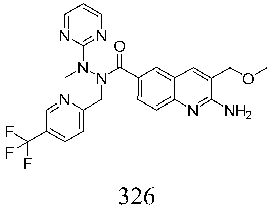

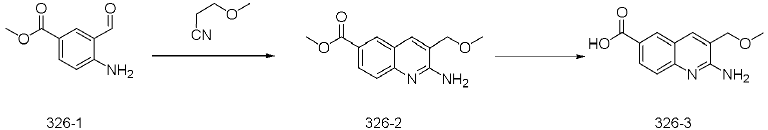

### Step 1: Preparation of methyl 2-amino-3-methoxymethylquinoline-6-carboxylate (326-2)

3-Methoxypropionitrile (190 mg, 2.23 mmol) was dissolved in THF (2 mL), followed by the addition of NaH (60%, 267 mg, 6.69 mmol) at 0°C. The mixture was further stirred at 0°C for 0.5 hour, and then methyl 4-amino-3-formylbenzoate (326-1, 200 mg, 1.12 mmol) was added in one portion. The mixture was reacted at 0°C to room temperature for 1h. After the reaction was completed, the reaction mixture was poured into ammonium chloride saturated solution, and extracted with ethyl acetate (20 mL x 3). The product was present in the aqueous phase. The aqueous phase was directly concentrated under reduced pressure to obtain a crude product 326-2, which was directly used for the next step.

LC-MS (ESI+): 247.2 m/z [M+H]⁺.

### Step 2: Preparation of 2-amino-3-methoxymethylquinoline-6-carboxylic acid (326-3)

The crude product 326-2 (200 mg, 3.25 mmol) was dissolved in water (1 mL) and methanol (1 mL), followed by the addition of lithium hydroxide solid (136 mg, 3.25 mmol) at normal temperature, and reacted at normal temperature for 1 h. After the reaction was completed, the reaction mixture was adjusted to acid pH with formic acid, and directly concentrated under reduced pressure to obtain a crude product 326-3, which was directly used for the next step.

LC-MS (ESI+): 233.2 m/z [M+H]⁺.

2-Amino-3-(methoxymethyl)-N'-methyl-N'-(pyrimidin-2-yl)-N-((5-(trifluoromethy 1)pyridin-2-yl)methyl)quinoline-6-carbohydrazide was obtained through remaining steps according to the method of Example 309, except that Compound 326-3 was used instead of Compound 309-11.

LC-MS (ESI+): 498.2 m/z [M+H]⁺.

### Example 327: Preparation of 2-amino-N'-methyl-N'-(pyrimidin-2-yl)-3-(trifluoromethyl)-N-((5-(trifluoromethyl)pyri din-2-yl)methyl)quinoline-6-carbohydrazide (327)

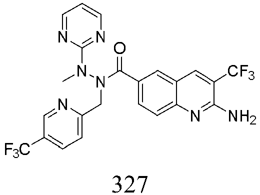

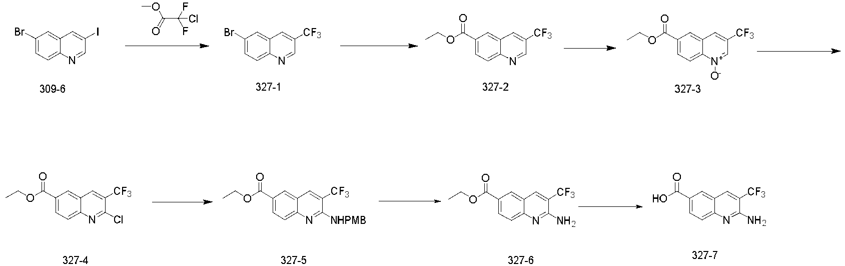

### Step 1: Preparation of 6-bromo-3-trifluoromethylquinoline (327-1)

6-Bromo-3-iodoquinoline (309-6, 900 mg, 2.70 mmol), methyl 2-chloro-2,2-difluoroacetate (1.56 g, 10.8 mmol), CuI (1.03 g, 5.39 mmol) and potassium fluoride (360 mg, 6.20 mmol) were dissolved in DMF (20 mL), and reacted at 120°C for 8h. After the reaction was completed, the reaction solution was quenched with water (20 mL), and then extracted with ethyl acetate (50 mL x 3). The organic phases were combined, dried over anhydrous sodium sulfate and filtered, and the filtrate was concentrated to dryness under reduced pressure. The crude product was purified by silica gel column chromatography (eluent: petroleum ether/ethyl acetate=5/1) to obtain Compound 327-1 (740 mg, yield 99%).

LC-MS (ESI+): 276.2 m/z [M+H]⁺.

### Step 2: Preparation of ethyl 3-(trifluoromethyl)quinoline-6-carboxylate (327-2)

Compound 327-1 (900 mg, 3.26 mmol), Mo(CO)₆ (2.15 g, 8.15 mmol), Pd(dppf)Cl₂ (665 mg, 0.82 mmol), Xantphos (942 mg, 1.64 mmol) and TEA (1.98 g, 19.6 mmol) were dissolved in ethanol (10 mL) and purged with nitrogen three times. The mixture was reacted at 95°C for 6 h. After completion of the reaction, the reaction mixture was filtered. The filtrate was concentrated under reduced pressure, and the residues were purified by silica gel column chromatography (petroleum ether/ethyl acetate=10/1) to obtain Compound 327-2 (870 mg, yield 99%).

LC-MS (ESI+): 270.2 m/z [M+H]⁺.

### Step 3: Preparation of 6-(ethoxycarbonyl)-3-(trifluoromethyl)quinoline-1-oxide (327-3)

Compound 327-2 (350 mg, 1.30 mmol) was dissolved in DCM (3 mL), followed by the addition of m-CPBA (335 mg, 1.95 mmol). The mixture was reacted at normal temperature for 2 h. After completion of the reaction, the reaction mixture was poured into water (20 mL), and extracted with DCM (20 mL x 3). The organic phases were combined, washed with saturated aqueous sodium bicarbonate solution (20 mL) three times, dried over anhydrous sodium sulfate and filtered, and the filtrate was concentrated under reduced pressure to obtain a crude product 327-3, which was directly used for the next step.

LC-MS (ESI+): 286.2 m/z [M+H]⁺.

### Step 4: Preparation of ethyl 2-chloro-3-(trifluoromethyl)quinoline-6-carboxylate (327-4)

The crude product 327-3 (100 mg, 0.35 mmol) was dissolved in toluene (2 mL), followed by the addition of POCl₃ (268 mg, 1.75 mmol) at normal temperature. The mixture was heated to 80°C and reacted for 10 h. After completion of the reaction, the reaction mixture was poured into ice water (10 mL), and extracted with ethyl acetate (3x10 mL). The organic phases were combined, washed with saturated aqueous sodium bicarbonate solution (20 mL) three times, dried over anhydrous sodium sulfate and filtered, and the filtrate was concentrated under reduced pressure to obtain a crude product 327-4, which was directly used for the next step.

LC-MS (ESI+): 304.2 m/z [M+H]⁺.

### Step 5: Preparation of ethyl 2-((4-methoxybenzyl)amino)-3-(trifluoromethyl)quinoline-6-carboxylate (327-5)

Compound 327-4 (87 mg, 0.29 mmol), PMBNH₂ (34 mg, 0.34 mmol) and DIEA (110 mg, 0.86 mmol) were dissolved in DMF (2 mL), and reacted at 60°C for 1 h. After the reaction was completed, water (5 mL) was added to the reaction solution, and then the reaction solution was extracted with ethyl acetate (3x10 mL). The organic phases were combined, dried over anhydrous sodium sulfate and filtered, and the filtrate was concentrated under reduced pressure. The crude product was purified by silica gel column chromatography (eluent: petroleum ether/ethyl acetate=2/1) to obtain Compound 327-5 (85 mg, yield 73%).

LC-MS (ESI+): 405.2 m/z [M+H]⁺.

### Step 6: Preparation of ethyl 2-amino-3-(trifluoromethyl)quinoline-6-carboxylate (327-6)

Compound 327-5 (85 mg, 0.21 mmol) was dissolved in TFA (2 mL), and reacted at 80°C for 16h. After the reaction was completed, the reaction mixture was concentrated under reduced pressure, and extracted with ethyl acetate (10 mLx3). The organic phases were combined, washed with saturated aqueous sodium bicarbonate solution (10 mL) three times, dried over anhydrous sodium sulfate and filtered, and the filtrate was concentrated under reduced pressure. The crude product was purified by silica gel column chromatography (eluent: petroleum ether/ethyl acetate=2/1) to obtain Compound 327-6 (59 mg, yield 99%).

LC-MS (ESI+): 285.2 m/z [M+H]⁺.

### Step 7: Preparation of 2-amino-3-(trifluoromethyl)quinoline-6-carboxylic acid (327-7)

Compound 327-6 (79 mg, 0.28 mmol) was dissolved in THF (1 mL), methanol (1 mL) and water (1 mL), followed by the addition of lithium hydroxide (47 mg, 1.11 mmol). The mixture was reacted at 50°C for 2 h. After the reaction was completed, the reaction mixture was adjusted to acid pH with formic acid, and a solid was precipitated, filtered and dried to obtain solid 327-7 (50 mg, yield 70%).

LC-MS (ESI+): 257.2 m/z [M+H]⁺.

Remaining steps were according to the synthetic method of Example 309 to give 2-amino-N'-methyl-N'-(pyrimidin-2-yl)-3-(trifluoromethyl)-N-((5-(trifluoromethyl)pyri din-2-yl)methyl)quinoline-6-carbohydrazide, except that Compound 327-7 was used instead of Compound 309-11.

LC-MS (ESI+): 522.2 m/z [M+H]⁺.

### Example 328: Preparation of (4-amino-1,7-dimethyl-1H-pyrazolo[4,3-c]quinolin-8-yl)(2-(3-fluoropyridin-2-yl)-4-me thylpyrazolidin-1-yl)methanone (328)

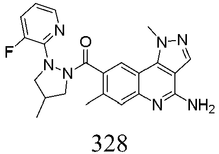

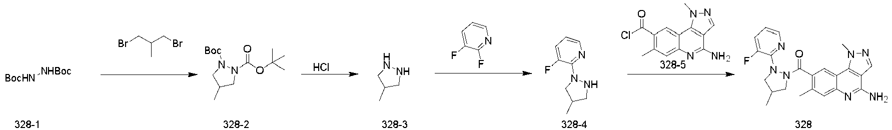

### Step 1: Preparation of di-tert-butyl 4-methylpyrazolidine-1,2-dicarboxylate (328-2)

Di-tert-butylhydrazine-1,2-dicarboxylic acid (328-1, 2.00 g, 8.6 mmol), 1,3-dibromo-2-methylpropane (2.05 g, 9.47 mmol) and cesium carbonate (8.40 g, 25.8 mmol) were dissolved in N,N-dimethylformamide (30 mL) solution at room temperature, and reacted at room temperature for 12 hours. The resulting reaction mixture was diluted with water (100.0 mL), and the resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=4/1) to obtain Compound 328-2 (2.4 g, yield 97%).

### Step 2: Preparation of 4-methylpyrazolidine (328-3)

Compound 328-2 (2.10 g, 7.33 mmol) was dissolved in hydrochloric acid-ethyl acetate (2 mL) solution at room temperature, and reacted at room temperature for 12 hours. The reaction solution was concentrated under reduced pressure to obtain Compound 328-3 (hydrochloride, 600 mg, yield 95%).

### Step 3: Preparation of 3-fluoro-2-(4-methylpyrazolidin-1-yl)pyridine (328-4)

Compound 328-3 (hydrochloride, 200 mg, 2.32 mmol), 2,3-difluoropyridine (267 mg, 2.32 mmol) and cesium carbonate (2.26 g, 6.96 mmol) were dissolved in dimethyl sulfoxide (5 mL) at room temperature, and reacted at 80°C for 12 hours. The resulting reaction mixture was diluted with water (100 mL), and the resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 328-4 (100 mg, yield 23%).

### Step 4: Preparation of (4-amino-1,7-dimethyl-1H-pyrazolo[4,3-c]quinolin-8-yl)(2-(3-fluoropyridin-2-yl)-4-me thylpyrazolidin-1-yl)methanone (328)

Compound 328-4 (50 mg, 0.28 mmol), 4-amino-1,7-dimethyl-1H-pyrazolo[4,3-c]quinoline-8-carbonyl chloride (328-5, 76 mg, 0.28 mmol) and N,N-diisopropylethylamine (107 mg, 0.83 mmol) were dissolved in N,N-dimethylacetamide (4 mL) solution at room temperature, and reacted at room temperature for 12 hours. The resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: dichloromethane/methanol=10/1) to obtain Compound 328 (10 mg, yield 18%).

LC-MS (ESI+): 420.2 m/z [M+H]⁺.

¹H NMR (400 MHz, DMSO-*d₆*) δ 8.16 - 8.11 (m, 2H), 7.82 (s, 1H), 7.47 (s, 1H), 7.30 (s, 1H), 7.07 - 6.99 (m, 3H), 4.01 (s, 3H), 3.79 (s, 1H), 3.46 (s, 1H), 3.32 - 3.25 (m, 2H), 3.20 (s, 1H), 2.38 (s, 3H), 1.04 (s, 3H).

The following compounds were obtained according to the synthetic method in Example 328 using corresponding raw materials.

| Example | Structure | Mass spectrometry data (ESI+): m/z | Nuclear magnetic data |
|---|---|---|---|
| 329 | 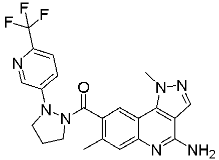 | 456.1 | ¹H NMR (400 MHz, DMSO-*d₆*) δ 8.50 (s, 1H), 8.19 (s, 1H), 8.07 (s, 1H), 7.72 (d, *J =* 8.0 Hz, 1H), 7.61 (s, 1H), 7.40 (s, 1H), 7.22 (s, 2H), 4.40 - 4.00 (m, 7H), 2.42 (s, 3H), 2.11 (s, 2H). |
| 330 | 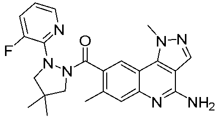 | 434.2 | |
| 331 | 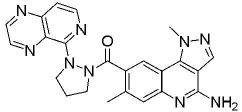 | 440.2 | |
| 332 | 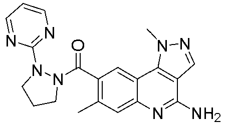 | 389.2 | |
| 333 | 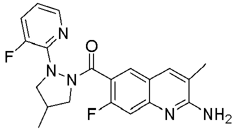 | 384.2 | ¹H NMR (400 MHz, DMSO-*d₆*) δ 8.05 (d, *J =* 4.8 Hz, 1H), 7.60 (s, 1H), 7.46 (s, 2H), 7.09 - 6.94 (m, 2H), 6.57 (s, 2H), 4.11 - 3.70 (m, 3H), 3.19 (s, 2H), 2.14 (s, 3H), 1.17 - 0.90 (m, 3H). |
| 334 | 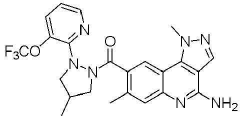 | 486.2 | |
| 335 | 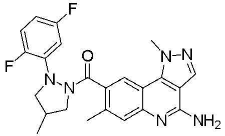 | 437.2 | |
| 336 | 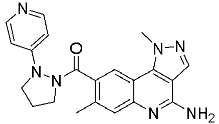 | 388.6 | |
| 337 | 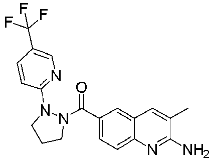 | 402.2 | ¹H NMR (400 MHz, DMSO-*d₆*) δ 8.57 (d, J = 2.4 Hz, 1H), 8.38 - 8.09 (m, 3H), 8.01 - 7.89 (m, 2H), 7.67 (d, J = 8.8 Hz, 1H), 7.05 (s, 1H), 4.39 - 4.05 (m, 2H), 3.14 - 2.86 (m, 2H), 2.31 (s, 3H), 2.22 - 1.92 (m, 2H). |
| 338 | 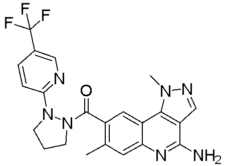 | 456.2 | |
| 339 | 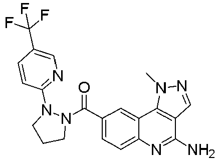 | 442.2 | ¹H NMR (400 MHz, DMSO-*d₆*) δ 8.63 - 8.56 (m, 2H), 8.44 (s, 1H), 8.23 - 8.18 (m, 1H), 8.02 - 7.90 (m, 2H), 7.69 (d, J = 8.4 Hz, 1H), 7.20 (s, 1H), 4.30 - 4.26 (m, 5H), 3.60 - 3.40 (m, 2H), 2.40 - 2.20 (m, 2H). |
| 340 | 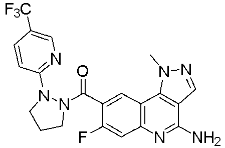 | 460.2 | |
| 341 | 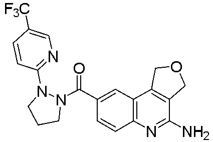 | 430.2 | |
| 342 | 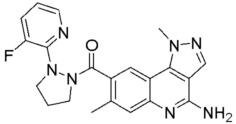 | 406.2 | |
| 343 | 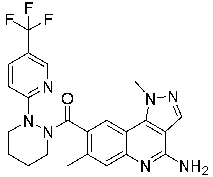 | 470.0 | ¹H NMR (400 MHz, DMSO-*d₆*) δ 8.55 (s, 1H), 8.46 (s, 1H), 8.09 - 8.03 (m, 1H), 7.97 (s, 1H), 7.41 (s, 1H), 7.35 (d, *J* = 8.8 Hz, 1H), 7.02 (s, 2H), 4.59 (d, J = 12.9 Hz, 2H), 4.00 (s, 3H), 3.20 - 3.11 (m, 1H), 3.06 - 2.93 (m, 1H), 2.50 (s, 3H), 2.03 - 1.52 (m, 4H). |
| 344 | 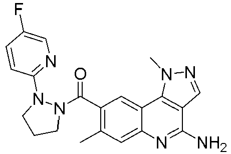 | 406.0 | ¹H NMR (400 MHz, DMSO-*d₆*) δ 8.32 (s, 2H), 8.20 (s, 1H), 8.11 (s, 1H), 7.70 - 7.60 (m, 1H), 7.45 - 7.35 (m, 1H), 7.05 (s, 2H), 4.30 - 4.10 (m, 5H), 3.50 - 3.40 (m, 2H), 2.43 (s, 3H), 2.20 - 2.00 (m, 2H). |
| 345 | 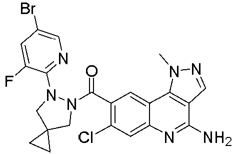 | 530.2 | ¹H NMR (400 MHz, DMSO-*d₆*) δ 8.38 (s, 1H), 8.24 (s, 1H), 7.93 (d, *J =* 10.8 Hz, 1H), 7.85 (s, 1H), 7.53 (s, 1H), 7.28 (s, 2H), 4.12 (s, 3H), 3.95 - 3.75 (m, 2H), 3.59 (s, 2H), 0.72-0.64 (m, 4H). |
| 346 | 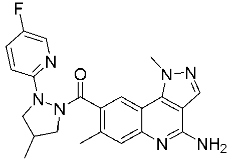 | 420.1 | ¹H NMR (400 MHz, DMSO-*d₆*) δ 8.22 - 8.18 (m, 2H), 8.12 (s, 1H), 7.66-7.62 (m, 1H), 7.38 (s, 1H), 7.26 (s, 1H), 7.11 (s, 2H), 4.20 (s, 3H), 4.00 - 3.80 (m, 4H), 2.55 (s, 1H), 2.45 (s, 3H), 1.05 (s, 3H). |
| 347 | 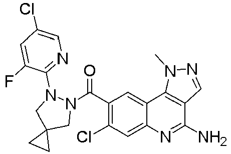 | 486.2 | ¹H NMR (400 MHz, DMSO-*d₆*) δ 8.29 (s, 2H), 7.92 - 7.78 (m, 3H), 7.75 - 7.47 (m, 3H), 4.13 (s, 3H), 3.83 - 3.58 (m, 4H), 0.70 - 0.61 (m, 4H). |

### Example 348:Preparation of (4-amino-1,7-dimethyl-1H-pyrazolo[4,3-c]quinolin-8-yl)(7-(3-fluoropyridin-2-yl)-6,7-d iazaspiro[3.4]octan-6-yl)methanone (348)

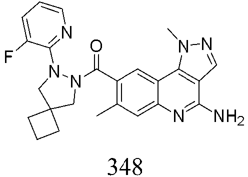

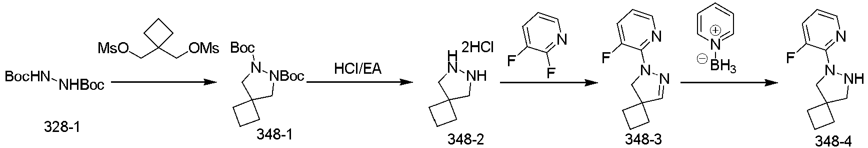

### Step 1: Preparation of di-tert-butyl 6,7-diazaspiro[3.4]octane-6,7-dicarboxylate (348-1)

Compound 328-1 (1000 mg, 4.31 mmol) was dissolved in DMF (20 mL) at room temperature, followed by the addition of cyclobutane-1,1-diylbis(methylene)dimethanesulfonate (1407 mg, 5.17 mmol) and cesium carbonate (4210 mg, 12.9 mmol). The mixture was further stirred at 50°C for 12 hours. After the reaction was completed, water (60 mL) was added to the reaction mixture, and the reaction mixture was extracted with ethyl acetate (80 mL x2). The organic phases were combined, washed with saturated brine (60 mL), dried over anhydrous sodium sulfate and filtered, and the filtrate was concentrated under reduced pressure. The crude product was purified by silica gel column chromatography (eluent: ethyl acetate/petroleum ether=3/20) to obtain 348-1 (800 mg, yield 59.5%) as a colourless oil liquid.

LCMS(ESI+): 213.2 m/z [M+H-100]⁺.

### Step 2: Preparation of 6,7-diazaspiro[3.4]octane dihydrochloride (348-2)

Compound 348-1 (800 mg, 2.56 mmol) was dissolved in HCl/EA (12 mL), and reacted at room temperature for 2 hours. After the reaction was completed, the reaction solution was directly concentrated under reduced pressure to obtain a crude product 348-2 (280 mg, yield 98%) as a white solid, which was directly used for the next step.

LC-MS (ESI+): 113.1 m/z [M+H]⁺.

### Step 3: Preparation of 7-(3-fluoropyridin-2-yl)-6,7-diazaspiro[3.4]octan-5-ene (348-3)

The crude product 348-2 (280 mg, 2.5 mmol) was dissolved in DMSO (10 mL), followed by the addition of 2,3-difluoropyridine (287 mg, 2.5 mmol) and cesium carbonate (3255 mg, 9.98 mmol) at room temperature, and stirred at 80°C for 12 hours under nitrogen atmosphere. After the reaction was completed, water (25 mL) was added to the reaction mixture, and the reaction mixture was extracted with ethyl acetate (30 mLx2). The organic phases were combined, washed with saturated brine (20 mL), dried over anhydrous sodium sulfate and filtered, and the filtrate was concentrated under reduced pressure. The crude product was purified by silica gel column chromatography (eluent: ethyl acetate/petroleum ether=1/5) to obtain 348-3 (100 mg, 32%) as a yellow liquid.

LC-MS (ESI+): 206.1 m/z [M+H]⁺.

### Step 4: Preparation of 6-(3-fluoropyridin-2-yl)-6,7-diazaspiro[3.4]octane (348-4)

Compound 348-3 (30 mg, 0.15 mmol) was dissolved in EtOH (2 mL), followed by the addition of borane-pyridine (162 mg, 1.74 mmol) and 2N HCl (2 mL) at room temperature. The mixture was stirred at room temperature for 12 hours. After the reaction was completed, the reaction solution was concentrated to obtain a crude product 348-4 (30 mg, yield 99%) as a yellow liquid.

LC-MS (ESI+): 208.1 m/z [M+H]⁺.

(4-Amino-1,7-dimethyl-1H-pyrazolo[4,3-c]**quinolin**-8-yl) (7-(3-fluoropyridin-2-yl)-6,7-diazaspiro[3.4]octan-6-yl)methanone was synthesized according to Step 4 of Example 328, except that Compound 348-4 was used instead of Compound 328-4.

LC-MS (ESI+): 446.2 m/z [M+H]⁺.

¹H NMR (400 MHz, DMSO-*d₆*) δ 8.17 (s, 1H), 8.12 (d, *J* = 4.8 Hz, 1H), 7.83 (s, 1H), 7.48 (s, 1H), 7.30 (s, 1H), 7.07 - 7.04 (m, 1H), 7.00 (s, 2H), 4.03 (s, 3H), 3.97 (s, 2H), 3.70 (s, 2H), 2.38 (s, 3H), 2.06 (s, 2H), 1.82 (s, 4H).

### Example 349: Preparation of (4-amino-1,7-dimethyl-1H-pyrazolo[4,3-c]quinolin-8-yl)(2-(pyridin-2-yl)pyrazolidin-1-yl)methanone (349)

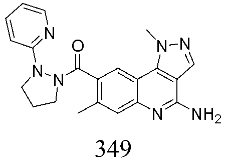

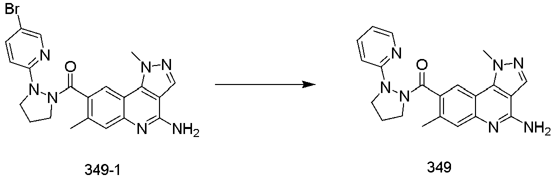

### Step 1: Preparation of (4-amino-1,7-dimethyl-1H-pyrazolo[4,3-c]quinolin-8-yl)(2-(pyridin-2-yl)pyrazolidin-1 -yl)methanone (349)

Compound 349-1 (synthesized according to the method in Example 328) (2 mg, 0.004 mmol) was dissolved in methanol (3 mL) at room temperature, followed by the addition of palladium on carbon (10% w/w, 50 mg). The mixture was purged with hydrogen three times. The reaction was further carried out at room temperature for 4 hours under hydrogen atmosphere. The reaction solution was filtered, and the filtrate was collected. The filtrate was concentrated under reduced pressure, and purified by preparative high performance liquid chromatography (water/acetonitrile=95%/5% - 10%/90%) to obtain Compound 349 (1 mg, yield 62%).

LC-MS (ESI+): 388.0 m/z [M+H]⁺.

### Example 350: Preparation of (4-amino-1,7-dimethyl-1H-pyrazolo[4,3-c]quinolin-8-yl)(2-(3-fluoropyridin-2-yl)-4-(tri fluoromethyl)pyrazolidin-1-yl)methanone (350)

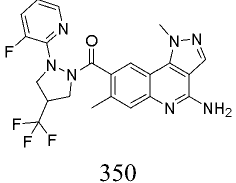

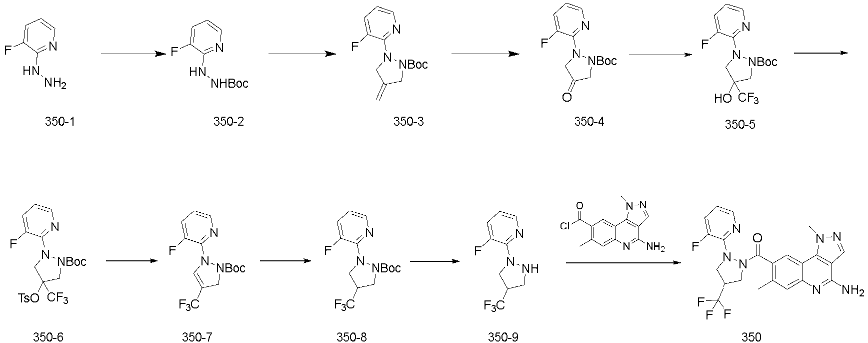

### Step 1: Preparation of tert-butyl 2-(3-fluoropyridin-2-yl)hydrazine-1-carboxylate (350-2)

3-Fluoro-2-hydrazinopyridine (350-1, 1000 mg, 7.87 mmol) was dissolved in DCM (10 mL), followed by the addition of di-tert-butyl dicarbonate (2.5 g, 11.8 mmol) at room temperature. The mixture was reacted for 12h. After the reaction was completed, the reaction mixture was diluted with DCM (50 mL), and then extracted with water (3x50 mL). The organic phase was separated, and dried over anhydrous sodium sulfate. The resulting mixture was directly concentrated under reduced pressure to obtain a crude product 350-2, which was directly used for the next step.

LC-MS (ESI+): 228.2 m/z [M+H]⁺.

### Step 2: Preparation of tert-butyl 2-(3-fluoropyridin-2-yl)-4-methylenepyrazoline-1-carboxylate (350-3)

Compound 350-2 (2500 mg, 11.0 mmol) was dissolved in DMF (1 mL), followed by the addition of Cs₂CO₃ (10.7 g, 33.0 mmol) and 3-chloro-2-(chloromethyl)prop-1-ene (1.37 g, 11.0 mmol) at normal temperature. The mixture was reacted at normal temperature for 16h. After the reaction was completed, the reaction mixture was diluted with ethyl acetate (50 mL), and then extracted with brine (3x50 mL). The organic phase was dried over anhydrous sodium sulfate and filtered, and the filtrate was concentrated under reduced pressure. The residues were purified by silica gel column chromatography (petroleum ether/ethyl acetate = 3/1) to obtain Compound 350-3 (2200 mg, yield 71%, a yellow oil).

LC-MS (ESI+): 280.2 m/z [M+H]⁺.

### Step 3: Preparation of tert-butyl 2-(3-fluoropyridin-2-yl)-4-oxopyrazoline-1-carboxylate (350-4)

Compound 350-3 (1 g, 3.58 mmol) was dissolved in THF (9 mL)/H₂O (3 mL), followed by the addition of potassium osmate (132 mg, 0.358 mmol), reacted for 0.5 hour, followed by the addition of sodium periodate (3.83 g, 17.9 mmol). The resulting mixture was stirred at room temperature for 18 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 3/1) to obtain Compound 350-4 (800 mg, yield 89%, a yellow oil).

LC-MS (ESI+): 282.2 m/z [M+H]⁺.

### Step 4: Preparation of tert-butyl 2-(3-fluoropyridin-2-yl)-4-hydroxy-4-(trifluoromethyl)pyrazoline-1-carboxylate (350-5)

Compound 350-4 (500 mg, 1.7 mmol) and (trifluoromethyl)trimethylsilane (0.8 mL) were dissolved in tetrahydrofuran (10 mL) at room temperature, followed by the addition of tetrabutylammonium fluoride (5.3 mL, 1M). The reaction solution was stirred at 0°C for 1 hour. The reaction solution was quenched with water (20 mL), and then extracted with dichloromethane (20 mL x3). The organic phases were combined, dried over anhydrous sodium sulfate and filtered, and the filtrate was concentrated under reduced pressure. The resulting crude product was purified by silica gel column chromatography (eluent: ethyl acetate/petroleum ether=1/1) to obtain Compound 350-5 (450 mg, yield 72%).

LC-MS (ESI+): 352.1 m/z [M+H]⁺.

### Step 5: Preparation of tert-butyl 2-(3-fluoropyridin-2-yl)-4-(tolyloxy)-4-trifluoromethylpyrazoline-1-carboxylate (350-6)

Compound 350-5 (400 mg, 1.1 mmol) was dissolved in N,N-dimethylformamide (5 mL), followed by the addition of sodium hydride (54 mg, 2.2 mmol) at 0°C. The mixture was stirred for 0.5 hour. p-Toluenesulfonyl chloride (434 mg, 2.2 mmol) was added to the reaction solution. The reaction solution was stirred at 25°C for 12 hour. The reaction solution was quenched with water (20 mL), and extracted with ethyl acetate (20 mL x3). The organic phases were combined, dried over anhydrous sodium sulfate and filtered, and the filtrate was concentrated to dryness under reduced pressure. The resulting crude product was purified by silica gel column chromatography (eluent: ethyl acetate/petroleum ether=1/1) to obtain Compound 350-6 (300 mg, yield 52%).

LC-MS (ESI+): 506.2 m/z [M+H]⁺.

### Step 6: Preparation of tert-butyl 2-(3-fluoropyridin-2-yl)-4-(trifluoromethyl)-2,5-dihydro-1H-pyrazole-1-carboxylate (350-7)

Compound 350-6 (300 mg, 0.59 mmol) was dissolved in tetrahydrofuran (5 mL) at room temperature, followed by the addition of potassium tert-butoxide (133 mg, 1.2 mmo). The reaction solution was stirred at 25°C for 1 hour. The reaction solution was quenched with water (20 mL), and then extracted with dichloromethane (20 mL x3). The organic phases were combined, dried over anhydrous sodium sulfate and filtered, and the filtrate was concentrated under reduced pressure. The resulting crude product was purified by silica gel column chromatography (eluent: ethyl acetate/petroleum ether=1/1) to obtain Compound 350-7 (100 mg, yield 50%).

LC-MS (ESI+): 334.1 m/z [M+H]⁺.

### Step 7: Preparation of tert-butyl 2-(3-fluoropyridin-2-yl)-4-(trifluoromethyl)pyrazoline-1-carboxylate (350-8)

Compound 350-7 (100 mg, 0.3 mmol) was dissolved in ethanol (5 mL) at room temperature, followed by the addition of palladium on carbon (10 mg), and the reaction solution was stirred at 30°C for 12 hours under hydrogen atmosphere. After the reaction was completed, palladium on carbon was filtered, and the organic phase was concentrated under reduced pressure to obtain Compound 350-8 (70 mg, yield 69%).

LC-MS (ESI+): 336.2 m/z [M+H]⁺.

### Step 8: Preparation of 3-fluoro-2-(4-(trifluoromethyl)pyrazolin-1-yl)pyridine (350-9)

Compound 350-4 (70 mg, 0.2 mmol) was dissolved in dry dioxane (1 mL) at room temperature, followed by the addition of hydrochloric acid-dioxane solution (0.2 mL). The reaction solution was stirred at 25°C for 12 hour until raw materials disappeared. The reaction solution was concentrated under reduced pressure to obtain Compound 350-9 (45 mg, yield 91%).

LC-MS (ESI+): 236.1 m/z [M+H]⁺.

### Step 9: Preparation of (4-amino-1,7-dimethyl-1H-pyrazolo[4,3-c]quinolin-8-yl)(2-(3-fluoropyridin-2-yl)-4-(tri fluoromethyl)pyrazolidin-1-yl)methanone (350)

Compound 350-5 (45 mg, 0.19 mmol) and 4-amino-1,7-dimethyl-1H-pyrazolo[4,3-c]quinoline-8-carbonyl chloride (328-5, 52 mg, 0.19 mmol) were dissolved in dry N,N-dimethylacetamide (1 mL) at room temperature, followed by the addition of diisopropylethylamine (0.1 mL). The reaction solution was stirred at 25°C for 2 hour. The resulting crude product was purified by preparative high performance liquid chromatography (water/acetonitrile=95%/5% - 10%/90%) to obtain Compound 350 (6.8 mg, yield 7%).

LC-MS (ESI+): 474.2 m/z [M+H]⁺.

The following compounds were obtained according to the synthetic method in Example 350 using corresponding raw materials.

| Example | Structure | Mass spectrometry data (ESI+): m/z | Nuclear magnetic data |
|---|---|---|---|
| 351 | 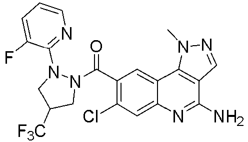 | 494.2 | ¹H NMR (400 MHz, DMSO-*d₆*) δ 8.25 - 8.16 (m, 2H), 7.85 (s, 1H), 7.53 (s, 2H), 7.30 (s, 2H), 7.14 (dt, *J =* 8.0, 4.0 Hz, 1H), 4.20-3.65 (m, 8H). |
| 352 | 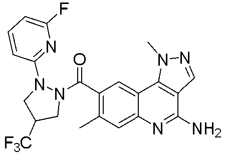 | 474.0 | |
| 353 | 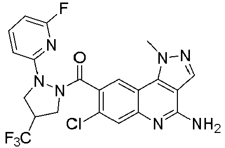 | 494.2 | |

### Example 354: Preparation of 1-(4-amino-1,7-dimethyl-1H-pyrazolo[4,3-c]quinoline-8-carbonyl)-2-(3-fluoropyridin-2 -yl)pyrazolin-4-one (354)

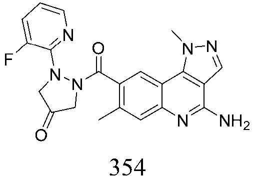

1-(4-Amino-1,7-dimethyl-1H-pyrazolo[4,3-c]quinoline-8-carbonyl)-2-(3-fluoropyr idin-2-yl)pyrazolin-4-one was synthesized according to Steps 8 and 9 of Example 350, except that Compound 350-4 was used instead of Compound 350-8.

LC-MS (ESI+): 420.2 m/z [M+H]⁺.

¹ H NMR (400 MHz, DMSO-*d₆*) δ 8.20 (s, 1H), 8.17 - 8.10 (m, 2H), 7.57 (dd, *J =* 11.6, 8.0 Hz, 1H), 7.35 (s, 1H), 7.18 -7.10 (m , 1H), 7.06 (s, 2H), 4.22 (s, 4H), 4.16 (s, 3H), 2.43 (s, 3H).

### Example 355: Preparation of (4-amino-1,7-dimethyl-1H-pyrazolo[4,3-c]quinolin-8-yl)(2-(3-fluoropyridin-2-yl)-4-hy droxy-4-(trifluoromethyl)pyrazolidin-1-yl)methanone (355)

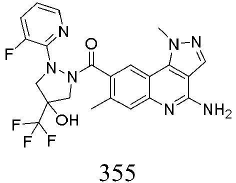

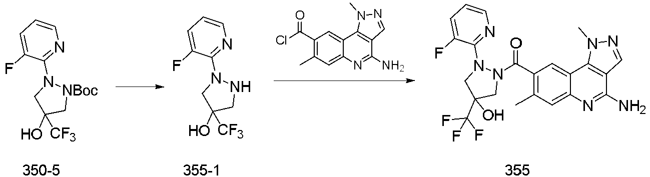

### Step 1: Preparation of 1-(3-fluoropyridin-2-yl)-4-(trifluoromethyl)pyrazolin-4-ol (355-1)

Compound 350-5 (90 mg, 0.25 mmol) was dissolved in dry dioxane (1 mL) at room temperature, followed by the addition of hydrochloric acid-dioxane solution (0.2 mL). The reaction solution was stirred at 25°C for 12 hour until raw materials disappeared. The reaction solution was concentrated under reduced pressure to obtain Compound 355-1 (hydrochloride, 60 mg, yield 93%).

LC-MS (ESI+): 252.1 m/z [M+H]⁺.

### Step 2: Preparation of (4-amino-1,7-dimethyl-1H-pyrazolo[4,3-c]quinolin-8-yl)(2-(3-fluoropyridin-2-yl)-4-hy droxy-4-(trifluoromethyl)pyrazolidin-1-yl)methanone (355)

Compounds 355-1 (60 mg, 0.24 mmol) and 328-5 (65 mg, 0.24 mmol) were dissolved in dry N,N-dimethylacetamide (1 mL) at room temperature, followed by the addition of diisopropylethylamine (0.1 mL). The reaction solution was stirred at 25°C for 2 hour. The resulting crude product was purified by reversed-phase high performance liquid chromatography (water/acetonitrile=95%/5% - 10%/90%, as the mobile phase) to obtain Compound 355 (52 mg, yield 44%).

LC-MS (ESI+): 490.2 m/z [M+H]⁺.

### Example 356: Preparation of (4-amino-1,7-dimethyl-1H-pyrazolo[4,3-c]quinolin-8-yl)(2-(3-fluoropyridin-2-yl)-4-hy droxypyrazolidin-1-yl)methanone (356)

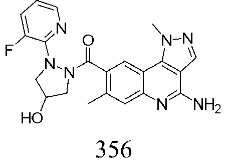

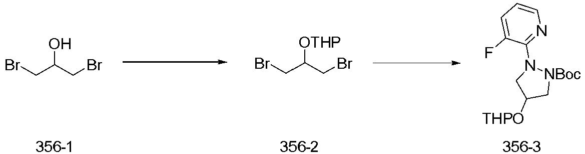

### Step 1: 2-Preparation of ((1,3-dibromo-2-propyl)oxy)tetrahydro-2H-pyrane (356-2)

1,3-Dibromo-2-propanol (356-1, 800 mg, 3.45 mmol) and DHP (580 mg, 6.90 mmol) were dissolved in THF (8 mL), followed by the addition of PPTS (86 mg, 0.35 mmol). The mixture was reacted at room temperature for 12 h. After the reaction was completed, the reaction solution was quenched with water (30 mL), and then extracted with ethyl acetate (30 mL x3). The organic phases were combined, dried over anhydrous sodium sulfate and filtered, and the filtrate was concentrated under reduced pressure. The crude product was purified by silica gel column chromatography (eluent: petroleum ether/ethyl acetate=5/1) to obtain Compound 356-2 (1090 mg, yield 100%).

### Step 2: Preparation of tert-butyl 2-(3-fluoropyridin-2-yl)-4-((tetrahydro-2H-pyran-2-yl)oxy)pyrazolidine-1-carboxylate (356-3)

Compounds 350-2 (795 mg, 3.50 mmol) and 356-2 (1.10 g, 3.50 mmol) were dissolved in DMF (10 mL), followed by the addition of cesium carbonate (3.42 g, 10.5 mmol). The mixture was reacted at normal temperature for 16 h. After the reaction was completed, the reaction solution was quenched with water (30 mL), and then extracted with ethyl acetate (30 mL x3). The organic phases were combined, dried over anhydrous sodium sulfate and filtered, and the filtrate was concentrated under reduced pressure. The crude product was purified by silica gel column chromatography (eluent: PE/EA=2/1) to obtain Compound 356-3 (400 mg, yield 30%).

LC-MS (ESI+): 368.2 m/z [M+H]⁺.

Remaining steps were according to the synthetic method of Example 350 to give (4-amino-1,7-dimethyl-1H-pyrazolo[4,3-c]quinolin-8-yl)(2-(3-fluoropyridin-2-yl)-4-hy droxypyrazolidin-1-yl)methanone, except that Compound 356-3 was used instead of 350-8.

LC-MS (ESI+): 422.2 m/z [M+H]⁺.

¹H NMR (400 MHz, DMSO-*d₆*) δ 8.18 (s, 1H), 8.09 (d, J = 4.8 Hz, 1H), 7.89 (s, 1H), 7.47 - 7.42 (m, 1H), 7.33 - 7.28 (m, 1H), 7.07 (s, 2H), 7.03 - 6.95 (m, 1H), 5.24 (s, 1H), 4.64 (s, 1H), 4.03 (s, 3H), 3.71 - 3.59 (m, 4H), 2.39 (s, 3H).

### Example 357: Preparation of (4-amino-1,7-dimethyl-1H-pyrazolo[4,3-c]quinolin-8-yl)(2-(3-fluoropyridin-2-yl)-4-(m ethoxymethyl)pyrazolin-1-yl)methanone (357)

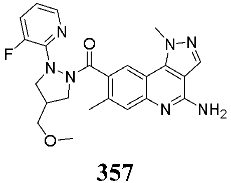

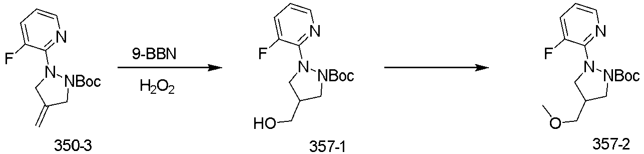

### Step 1: Preparation of tert-butyl 2-(3-fluoropyridin-2-yl)-4-(hydroxymethyl)pyrazoline-1-carboxylate (357-1)

Compound 350-3 (0.50 g, 1.79 mmol) was dissolved in THF (10 mL), followed by the addition of 9-BBN (3.58 mL, 3.58 mmol) at 0°C, and reacted at room temperature overnight. The resulting reaction mixture was cooled to 0°C, followed by the addition of water (0.5 mL), 3 M NaOH .aq (1.5 mL) and 1.5 mL H₂O₂ (1 mL). The mixture was stirred at room temperature for 2 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 3/1) to obtain Compound 357-1 (0.3 g, yield 56%, a yellow oil).

LC-MS (ESI+): 298.1 m/z [M+H]⁺.

### Step 2: Preparation of tert-butyl 2-(3-fluoropyridin-2-yl)-4-(methoxymethyl)pyrazoline-1-carboxylate (357-2)

Compound 357-1 (0.1 g, 0.33 mmol) was dissolved in THF (2 mL), followed by the addition of NaH (60%, 16 mg, 0.67 mmol) at 0°C. The mixture was reacted for 0.5 hour, followed by the addition of iodomethane (71.6 mg, 0.50 mmol), and the resulting mixture was stirred at room temperature for 18 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 3/1) to obtain Compound 357-2 (0.1 g, yield 95%, a yellow oil).

LC-MS (ESI+): 312.1 m/z [M+H]⁺.

Remaining steps were according to the synthetic method of Example 350 to give (4-amino-1,7-dimethyl-1H-pyrazolo[4,3-c]quinolin-8-yl)(2-(3-fluoropyridin-2-yl)-4-(m ethoxymethyl)pyrazolin-1-yl)methanone, except that Compound 357-2 was used instead of Compound 350-8.

LC-MS (ESI+): 450.2 m/z [M+H]⁺.

¹H NMR (400 MHz, DMSO-*d₆*) δ 8.20 - 8.10 (m, 2H), 7.85 (s, 1H), 7.51 (s, 1H), 7.32 (s, 1H), 7.10 - 7.07 (m, 1H), 7.01 (s, 2H), 4.03 (s, 3H), 3.77 (s, 2H), 3.50 - 3.40 (m, 2H), 3.30 (s, 2H), 3.21 (s, 3H), 2.78 (s, 1H), 2.40 (s, 3H).

### Example 358: Preparation of (4-amino-1,7-dimethyl-1H-pyrazolo[4,3-c]quinolin-8-yl)(2-(3-fluoropyridin-2-yl)-4-(h ydroxymethyl)pyrazolidin-1-yl)methanone (358)

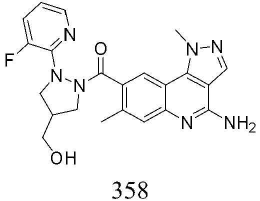

(4-Amino-1,7-dimethyl-1H-pyrazolo[4,3-c]quinolin-8-yl)(2-(3-fluoropyridin-2-yl)-4-(hydroxymethyl)pyrazolidin-1-yl)methanone was obtained according to the synthetic method of Example 350, except that Compound 357-1 was used instead of 350-8.

LC-MS (ESI+): 436.2 m/z [M+H]⁺.

### Example 359: Preparation of (4-amino-7-chloro-1-methyl-1H-pyrazolo[4,3-c]quinolin-8-yl)(4-(fluoromethyl)-2-(3-fl uoropyridin-2-yl)pyrazolin-1-yl)methanone (359)

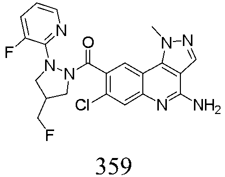

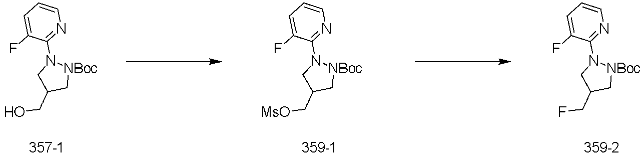

### Step 1: Preparation of tert-butyl 2-(3-fluoropyridin-2-yl)-4-(((methylsulfonyl)oxy)methyl)pyrazoline-1-carboxylate (359-1)

Compound 357-1 (0.1 g, 0.33 mmol) was dissolved in DCM (1 mL), followed by the addition of DIEA (0.086 g, 0.67 mmol) and methanesulfonic anhydride (0.064 g, 0.369 mmol) successively, and the resulting mixture was stirred at room temperature for 2 hours. Water (100 mL) was added to the reaction solution, and the reaction solution was extracted with DCM (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 3/1) to obtain Compound 359-1 (0.1 g, yield 79%, a yellow oil).

LC-MS (ESI+): 376.2 m/z [M+H]⁺.

### Step 2: Preparation of tert-butyl 4-(fluoromethyl)-2-(3-fluoropyridin-2-yl)pyrazoline-1-carboxylate (359-2)

Compound 359-1 (0.1 g, 0.26 mmol) was dissolved in THF (2 mL), followed by the addition of TBAF (1 mL, 1.06 mmol), and the resulting mixture was stirred at 60°C for 18 hours. Water (100 mL) was added to the reaction solution, and the reaction solution was extracted with ethyl acetate (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 1/1) to obtain Compound 359-2 (60 mg, yield 75%, a yellow oil).

LC-MS (ESI+): 300.1 m/z [M+H]⁺.

Remaining steps were according to the synthetic method of Example 350 to give (4-amino-7-chloro-1-methyl-1H-pyrazolo[4,3-c]quinolin-8-yl)(4-(fluoromethyl)-2-(3-fl uoropyridin-2-yl)pyrazolin-1-yl)methanone, except that Compound 359-2 was used instead of Compound 350-8.

LC-MS (ESI+): 458.1 m/z [M+H]⁺.

¹H NMR (400 MHz, DMSO-*d₆*) δ 8.21 (s, 1H), 8.18 (d, *J* = 4.8 Hz, 1H), 7.83 (s, 1H), 7.51 (s, 2H), 7.25 (s, 2H), 7.08 (s, 1H), 4.48 (d, *J =* 45.8 Hz, 2H), 4.07 (s, 3H), 3.90 - 3.80 (m, 2H), 3.55 - 3.45 (m, 2H), 2.98 (s, 1H).

### Example 360: Preparation of (4-amino-1,7-dimethyl-1H-pyrazolo[4,3-c]quinolin-8-yl)(2-(6-fluoropyridin-2-yl)-4-me thylpyrazolidin-1-yl)methanone (360)

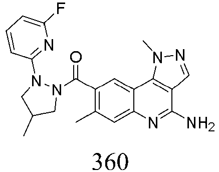

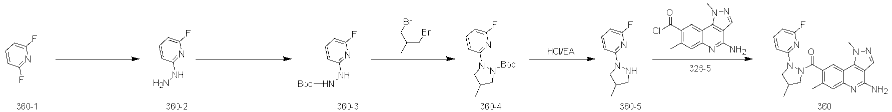

### Step 1: Preparation of 2-fluoro-6-hydrazinopyridine (360-2)

2,6-Difluoropyridine (360-1, 1.00 g, 8.69 mmol) and hydrazine hydrate (543 mg, 8.69 mmol) were dissolved in ethanol (20 mL) solution at room temperature, and reacted at 60°C for 12 hours. The resulting reaction mixture was diluted with water (100.0 mL). The resulting mixture was extracted with ethyl acetate (100.0 mL x 3). The organic phases were combined, washed with saturated brine (200 mL), dried over anhydrous sodium sulfate and filtered. The filtrate was concentrated under reduced pressure. The resulting residues were purified by silica gel column chromatography (eluent: petroleum ether/ethyl acetate=0/1) to obtain 360-2 (1.00 g, yield 90%).

LC-MS (ESI+): 128.0 m/z [M+H]⁺.

### Step 2: Preparation of tert-butyl 2-(6-fluoropyridin-2-yl)hydrazine-1-carboxylate (360-3)

Compound 360-2 (500 mg, 3.93 mmol), di-tert-butyl dicarbonate (1.11 g, 5.11 mmol) and sodium carbonate (1.23 g, 11.8 mmol) were dissolved in a solution of tetrahydrofuran (10 mL) and water (5 mL) at room temperature, and reacted at room temperature for 12 hours. The resulting reaction mixture was diluted with water (100.0 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=4/1) to obtain 360-3 (900 mg, yield 100%).

LC-MS (ESI+): 228.1 m/z [M+H]⁺.

### Step 3: Preparation of tert-butyl 2-(6-fluoropyridin-2-yl)-4-methylpyrazolidinyl-1-carboxylate (360-4)

Compound 360-3 (900 mg, 3.96 mmol), 1,3-dibromo-2-methylpropane (1.03 g, 4.75 mmol) and cesium carbonate (3.86 g, 11.9 mmol) were dissolved in N,N-dimethylformamide (20 mL) at room temperature, and reacted at room temperature for 12 hours, and the resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=4/1) to obtain 360-4 (380 mg, yield 34%).

LC-MS (ESI+): 282.2 m/z [M+H]⁺.

### Step 4: Preparation of 2-fluoro-6-(4-methylpyrazolidin-1-yl)pyridine (360-5)

Compound 360-4 (380 mg, 1.35 mmol) was dissolved in a solution of hydrochloric acid-ethyl acetate (4 mL) at room temperature, and reacted at room temperature for 12 hours. The reaction solution was concentrated under reduced pressure to obtain 360-5 (244 mg, yield 99%).

LC-MS (ESI+): 182.1 m/z [M+H]⁺.

### Step 5: Preparation of (4-amino-1,7-dimethyl-1H-pyrazolo[4,3-c]quinolin-8-yl)(2-(6-fluoropyridin-2-yl)-4-me thylpyrazolidin-1-yl)methanone (360)

Compounds 360-5 (100 mg, 0.55 mmol), 328-5 (166 mg, 0.61 mmol) and N,N-diisopropylethylamine (213 mg, 1.66 mmol) were dissolved in a solution of N,N-dimethylacetamide (5 mL) at room temperature, and reacted at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: dichloromethane/methanol=10/1) to obtain 360 (10 mg, yield 5%).

LC-MS (ESI+): 420.2 m/z [M+H]⁺.

¹H NMR (400 MHz, DMSO-*d₆*) δ 8.19 (s, 1H), 8.16 (s, 1H), 7.77- 7.83 (m, 1H), 7.37 (s, 1H), 7.13 (s, 3H), 6.55 (d, *J =* 4.0 Hz, 1H), 4.50 - 4.10 (m, 5H), 3.74 (s, 2H), 2.46 - 2.36 (m, 4H), 0.94-1.14 (m, 3H).

The following compounds were obtained according to the synthetic method in Example 360 using corresponding raw materials.

| Example | Structure | Mass spectrometry data (ESI+): m/z | Nuclear magnetic data |
|---|---|---|---|
| 361 | 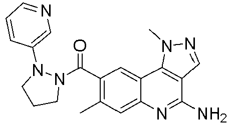 | 388.2 | |
| 362 | 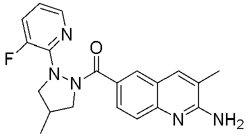 | 366.2 | ¹H NMR (400 MHz, DMSO-*d₆*) δ8.03 (d, *J* = 4.0 Hz, 1H), 7.84 (s, 1H), 7.67 (s, 1H), 7.61 - 7.55 (m, 2H), 7.31 (s, 1H), 7.02 (d, *J* = 4.0 Hz, 1H), 6.50 (s, 2H), 3.89 (s, 2H), 3.24 (s, 3H), 2.18 (s, 3H), 1.02 (s, 3H). |
| 363 | 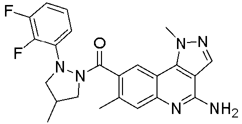 | 437.2 | ¹H NMR (400 MHz, DMSO-*d₆*) δ 8.16 (s, 1H), 7.88 (s, 1H), 7.35 (s, 1H), 7.15 - 7.10 (m, 2H), 7.05 (s, 2H), 7.02 - 6.97 (m, 1H), 3.97 (s, 4H), 3.65 (s, 3H), 3.09 (s, 1H), 2.36 (s, 3H), 1.07 (d, *J =* 4.0 Hz, 3H). |
| 364 | 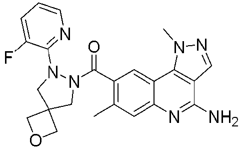 | 488.2 | ¹H NMR (400 MHz, DMSO-*d₆*) δ 8.19 (s, 1H), 8.11 (d, *J =* 4.0 Hz, 1H), 7.82 (s, 1H), 7.52 (s, 1H), 7.32 (s, 1H), 7.12 - 7.08 (m, 1H), 7.03 (s, 2H), 4.55 (s, 2H), 4.35 (s, 2H), 4.17 (s, 2H), 4.02 (s, 2H), 3.93 (s, 3H), 2.36 (s, 3H). |
| 365 | 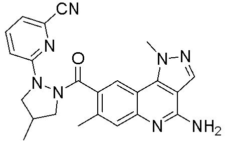 | 427.2 | ¹H NMR (400 MHz, DMSO-*d₆*) δ 8.15-8.18 (m, 3H), 7.84 (t, *J* = 8.0 Hz, 1H), 7.44 (d, *J* = 8.0 Hz, 2H), 7.16 (s, 2H), 4.29-4.37 (m, 4H), 3.33 (s, 3H), 3.21 - 3.02 (m, 1H), 2.46 (s, 3H), 0.98 (s, 3H). |
| 366 | 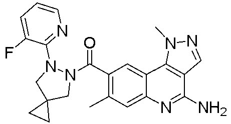 | 432.2 | ¹H NMR (400 MHz, DMSO-*d₆*) δ 8.19 (s, 2H), 7.84 (s, 1H), 7.49 (s, 1H), 7.32 (s, 1H), 7.13 - 7.06 (m, 1H), 7.00 (s, 2H), 4.53 - 3.72 (m, 5H), 3.56 (s, 2H), 2.41 (s, 3H), 0.66 - 0.57 (m, 4H). |
| 367 | 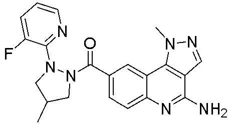 | 406.2 | 1H NMR (400 MHz, DMSO-d6) δ 8.51 (d, J = 1.6 Hz, 1H), 8.24 (s, 1H), 8.08 (d, J = 4.8 Hz, 1H), 7.72 (dd, J = 8.8, 1.6 Hz, 1H), 7.66 - 7.57 (m, 1H), 7.48 (d, J = 8.4 Hz, 1H), 7.18 (s, 2H), 7.12 - 7.04 (m, 1H), 4.17 (s, 3H), 3.93 - 3.86 (m, 1H), 3.59 - 3.38 (m, 3H), 3.34 - 3.23 (m, 1H), 1.06 (s, 3H). |
| 368 | 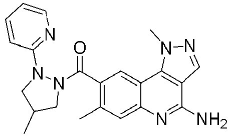 | 402.2 | ¹H NMR (400 MHz, DMSO-*d₆*) δ 8.21 - 8.16 (m, 2H), 7.73 - 7.64 (m, 1H), 7.38 (s, 1H), 7.23 (s, 1H), 7.07 - 7.01 (m, 2H), 6.93 - 6.86 (m, 1H), 4.50 - 4.35 (m, 1H), 4.14 (s, 3H), 3.79 - 3.75 (m, 1H), 3.65 - 3.61 (m, 1H), 2.93 - 2.89 (m, 1H), 2.48 - 2.43 (m, 4H), 1.17 - 0.93 (m, 3H). |
| 369 | 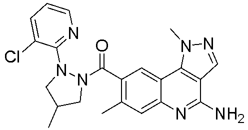 | 436.2 | 1H NMR (400 MHz, DMSO-d6) δ 8.26 (dd, J = 4.8, 2.0 Hz, 1H), 8.21 - 8.13 (m, 1H), 7.78 (s, 1H), 7.68 - 7.55 (m, 1H), 7.25 (s, 1H), 7.03 (s, 1H), 6.95 (s, 2H), 4.56 - 4.18 (m, 1H), 4.06 (s, 3H), 3.82 - 3.71 (m, 1H), 3.60 - 3.44 (m, 1H), 3.42 - 3.36 (m, 1H), 3.27 - 3.19 (m, 1H), 2.34 (s, 3H), 1.07 (s, 3H). |
| 370 | 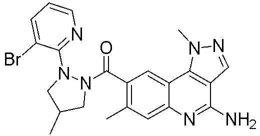 | 480.2 | |
| 371 | 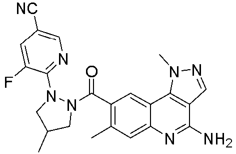 | 445.2 | |
| 372 | 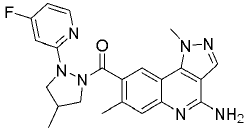 | 420.2 | |
| 373 | 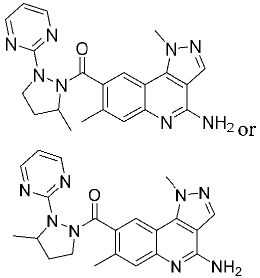 Compound with short retention time in HPLC | 403.2 | |
| 374 | 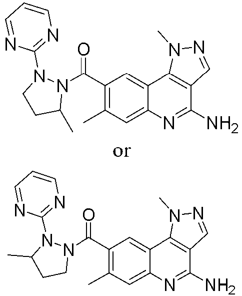 Compound with long retention time in HPLC | 403.2 | |
| 375 | 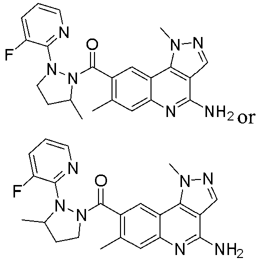 Compound with short retention time in HPLC | 420.2 | |
| 376 | 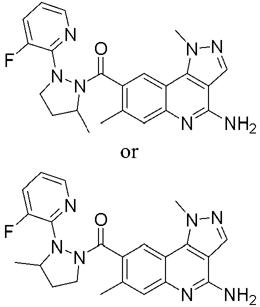 Compound with long retention time in HPLC | 420.2 | |
| 377 | 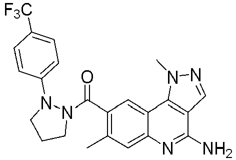 | 455.6 | |
| 378 | 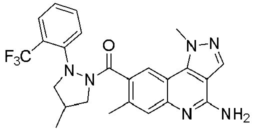 | 469.5 | |
| 379 | 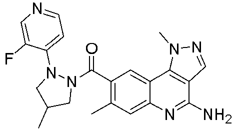 | 420.5 | |
| 380 | 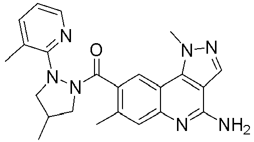 | 416.0 | ¹H NMR (400 MHz, DMSO-*d₆*) δ 8.23 - 8.18 (m, 1H), 8.15 (s, 1H), 7.53 (s, 1H), 7.32 - 7.26 (m, 2H), 7.01 - 6.92 (m, 3H), 4.22 - 4.06 (m, 1H), 3.92 (s, 3H), 3.55 - 3.50 (m, 2H), 3.12 - 2.97 (m, 2H), 2.33 (s, 3H), 1.67 (s, 3H), 1.15 - 1.03 (m, 3H). |
| 381 | 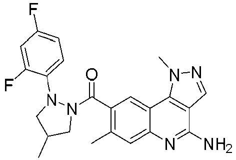 | 437.0 | |
| 382 | 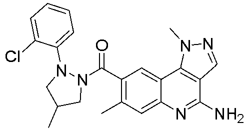 | 435.0 | ¹H NMR (400 MHz, DMSO-*d₆*) δ 8.18 - 8.14 (m, 1H), 7.82 (s, 1H), 7.40 - 7.30 (m, 3H), 7.18 (s, 1H), 7.06 - 6.96 (m, 3H), 4.23 - 4.11 (m, 1H), 3.96 (s, 3H), 3.60 - 3.54 (m, 2H), 3.18 - 3.03 (m, 2H), 2.36 (s, 3H), 1.16 - 1.06 (m, 3H). |
| 383 | 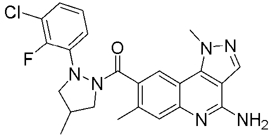 | 453.2 | |
| 384 | 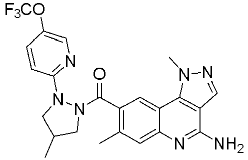 | 486.0 | |
| 385 | 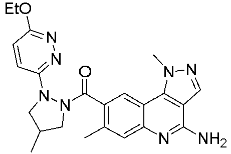 | 447 | |
| 386 | 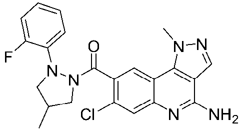 | 439.2 | |
| 387 | 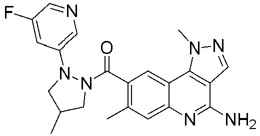 | 420.0 | |
| 388 | 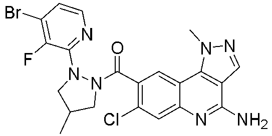 | 518.2 | |
| 389 | 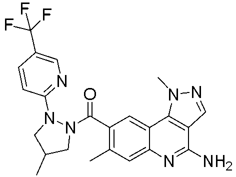 | 470.2 | |
| 390 | 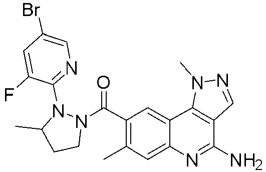 or 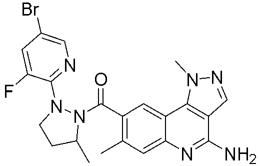 | 498.2 | |
| 391 | 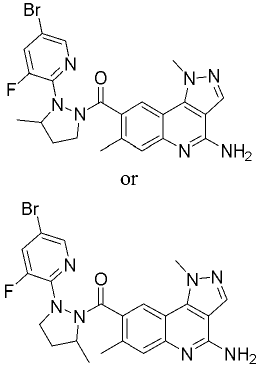 | 498.2 | |
| 392 | 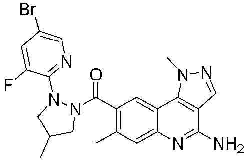 | 498.2 | ¹H NMR (400 MHz, DMSO-*d₆*) δ 8.36 - 8.20 (m, 3H), 8.00 - 7.80 (m, 1H), 7.48 - 7.26 (m, 3H), 4.50 - 4.30 (m, 1H), 4.12 (s, 3H), 3.90 - 3.77 (m, 1H), 3.56 - 3.35 (m, 1H), 3.31 - 3.19 (m, 1H), 2.60 - 2.55 (m, 1H), 2.41 (s, 3H), 1.20 - 1.00 (m, 3H). |
| 393 | 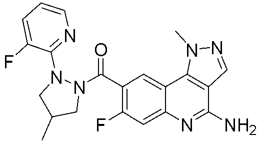 | 424.0 | |
| 394 | 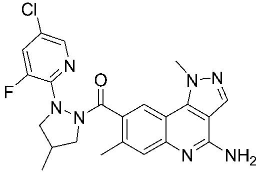 | 454.0 | ¹H NMR (400 MHz, DMSO-*d₆*) δ 8.29 - 8.20 (m, 2H), 7.87 (s, 2H), 7.34 (s, 1H), 7.14 (s, 2H), 4.11 (s, 3H), 3.85 (s, 2H), 3.25 (s, 2H), 2.70 - 2.60 (m, 1H), 2.40 (s, 3H), 1.07 (s, 3H). |
| 395 | 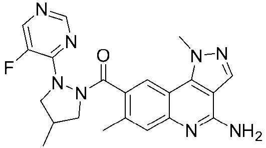 | 421.0 | |
| 396 | 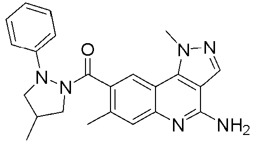 | 401.0 | |
| 397 | 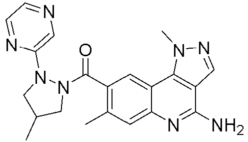 | 403.0 | |
| 398 | 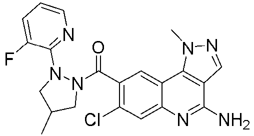 | 440.0 | ¹H NMR (400 MHz, CDCl₃) δ 8.07 (d, *J* = 4.7 Hz, 1H), 7.90 (d, *J =* 4.7 Hz, 2H), 7.67 (s, 1H), 7.21 - 7.02 (m, 1H), 6.93 - 6.80 (m, 1H), 5.56 (s, 2H), 4.27 - 4.13 (m, 4H), 3.98 - 3.84 (m, 1H), 3.65 (s, 1H), 3.39 - 3.21 (m, 1H), 2.64 (s, 1H), 1.20 (d, *J =* 6.5 Hz, 3H). |
| 399 | 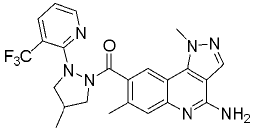 | 470.1 | ¹H NMR (400 MHz, DMSO-*d₆*) δ 8.63 (s, 1H), 8.25 - 8.15 (m, 2H), 7.87 (s, 1H), 7.35 - 7.20 (m, 2H), 7.10 - 6.99 (m, 2H), 4.03 (s, 3H), 3.64 (s, 2H), 3.52 (s, 1H), 3.21 (s, 1H), 2.65 - 2.55 (m, 1H), 2.38 - 2.21 (m, 3H), 1.10 (s, 3H). |
| 400 | 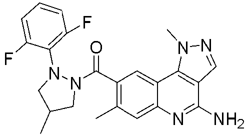 | 437.1 | ¹H NMR (400 MHz, DMSO-*d*₆) δ 8.21 (s, 1H), 8.18 (s, 1H), 7.85 (s, 1H), 7.33 (s, 1H), 7.15 (s, 2H), 7.00 - 6.80 (m, 2H), 4.38 (s, 1H), 4.10 (s, 3H), 4.00 - 3.80 (m, 2H), 3.16-3.12 (m, 1H), 2.63 (d, *J* = 8.8 Hz, 1H), 2.32 (s, 3H), 1.14 (s, 3H). |
| 401 | 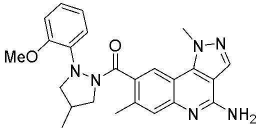 | 431.1 | ¹H NMR (400 MHz, DMSO-*d*₆) δ 8.15 (s, 1H), 7.86 (s, 1H), 7.34 (s, 1H), 7.20 (d, *J* = 7.2 Hz, 1H), 7.02 - 6.94 (m, 4H), 6.85 (d, *J* = 6.8 Hz, 1H), 4.07 (s, 1H), 3.82 (s, 3H), 3.66-3.59 (m, 2H), 3.45 (s, 3H), 2.99-2.96 (m, 1H), 2.41 (s, 1H), 2.39 (s, 3H), 1.08 (d, *J =* 6.8 Hz, 3H). |
| 402 | 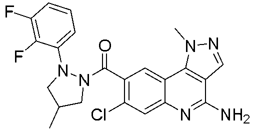 | 457.1 | ¹H NMR (400 MHz, DMSO-*d*₆) δ 8.22 (s, 1H), 7.96 (s, 1H), 7.52 (s, 1H), 7.27 (s, 2H), 7.23 - 7.10 (m, 2H), 6.96 (d, *J =* 8.8 Hz, 1H), 4.50 - 4.40 (m, 1H), 4.07 (s, 3H), 3.70 (s, 1H), 3.20 - 3.10 (m, 2H), 2.56 (s, 1H), 1.11 (d, *J =* 6.8 Hz, 3H). |
| 403 | 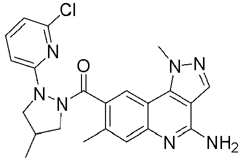 | 436.2 | |
| 404 | 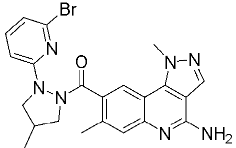 | 480.2 | |

### Example 405: Preparation of (4-amino-1,7-dimethyl-1H-pyrazolo[4,3-c]quinolin-8-yl)(2-(6-cyclopropylpyridin-2-yl) -4-methylpyrazolidin-1-yl)methanone (405)

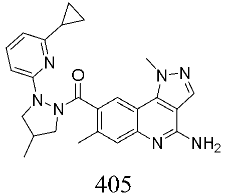

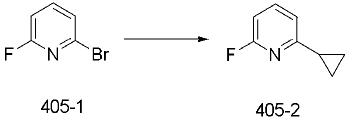

### Step 1: Preparation of 2-cyclopropyl-6-fluoropyridine (405-2)

2-Bromo-6-fluoro-pyridine (3.5 g, 20 mmol) was added into a 250 mL-flask, followed by the addition of toluene (53 mL), cyclopropylboronic acid (3.44 g, 40 mmol), K₃PO₄ (8.48 g, 40 mmol), Pd (OAc)₂ (224 mg, 1 mmol), PCy₃ (560 mg, 2mmol) and water (14 mL).The resulting mixture was heated to 90°C under nitrogen atmosphere and reacted under stirring for 4-6 h. The resulting reaction mixture was cooled to room temperature, followed by the addition of 50 mL of water, and extracted with ethyl acetate (50 mL). The organic phase was washed with water, dried, concentrated, and purified by silica gel column chromatography to obtain a product 405-2 (2.4 g, yield 87%).

Remaining steps were according to the synthetic method of Example 360 to give (4-amino-1,7-dimethyl-1H-pyrazolo[4,3-c]quinolin-8-yl)(2-(6-cyclopropylpyridin-2-yl) -4-methylpyrazolidin-1-yl)methanone, except that Compound 405-2 was used instead of Compound 360-1.

LC-MS (ESI+): 442.2 m/z [M+H]⁺.

### Example 406: Preparation of (4-amino-1,7-dimethyl-1H-pyrazolo[4,3-c]quinolin-8-yl)(2-(6-methoxypyridin-2-yl)-4-methylpyrazolin-1-yl)methanone (406)

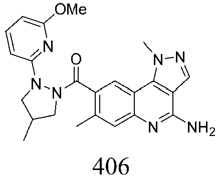

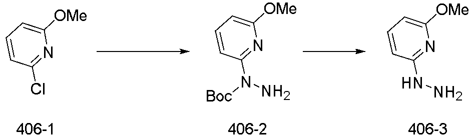

### Step : Preparation of tert-butyl 1-(6-methoxypyridin-2-yl)hydrazine-carboxylate (406-2)

2-Chloro-6-methoxypyridine (406-1, 1.0 g, 6.9 mmol) and tert-butyl carbazate (920 mg, 6.9 mmol) were dissolved in toluene (15 mL) at room temperature, followed by the addition of tris(dibenzylideneacetone)dipalladium (637 mg, 0.69 mmol), 1,1'-bis(diphenylphosphino)ferrocene (772 mg, 1.4 mmol) and cesium carbonate (3.4 g, 10.4 mmol) successively. The reaction solution was heated to 100°C under nitrogen atmosphere and stirred for 12 hours. After the reaction was completed, the reaction solution was quenched with water (20 mL), and extracted with ethyl acetate (30 mL x3). The organic phases were combined, dried over anhydrous sodium sulfate and filtered, and the filtrate was concentrated under reduced pressure. The resulting crude product was purified by silica gel column chromatography (eluent: ethyl acetate/petroleum ether=1/1) to obtain Compound 406-2 (700 mg, yield 42%).

LC-MS (ESI+): 240.2 m/z [M+H]⁺.

### Step 2: Preparation of 2-hydrazino-6-methoxypyridine (406-3)

Tert-butyl 1-(6-methoxypyridin-2-yl)hydrazine-carboxylate (600 mg, 2.5 mmol) was dissolved in methanol (5 mL) at room temperature, followed by the addition of a solution of hydrochloric acid in methanol (1 mL). The reaction solution was stirred at 25°C for 12 hour until raw materials disappeared. The reaction solution was concentrated under reduced pressure to obtain Compound 406-3 (340 mg, yield 97%).

LC-MS (ESI+): 140.2 m/z [M+H]⁺.

Remaining steps were according to the synthetic method of Example 360 to give (4-amino-1,7-dimethyl-1H-pyrazolo[4,3-c]quinolin-8-yl)(2-(6-methoxypyridin-2-yl)-4-methylpyrazolin-1-yl)methanone, except that Compound 406-3 was used instead of Compound 360-2.

LC-MS (ESI+): 432.5 m/z [M+H]⁺.

The following compound was obtained according to the synthetic method in Example 406 using corresponding raw materials.

| Example | Structure | Mass spectrometry data (ESI+): m/z | Nuclear magnetic data |
|---|---|---|---|
| 407 | 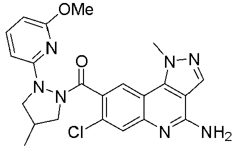 | 452.1 | ¹H NMR (400 MHz, DMSO-*d₆*) δ 8.22 (d, *J* = 6.0 Hz, 2H), 7.60 - 7.49 (m, 2H), 7.23 (s, 2H), 6.89-6.86 (m, 1H), 6.32 - 6.20 (m, 1H), 4.51-4.39 (m, 2H), 4.22 (s, 3H), 3.71 (s, 3H), 3.69 - 3.59 (m, 2H), 2.93 (s, 1H), 1.23-0.9 (m, 3H). |

### Example 408: Preparation of (4-amino-1,7-dimethyl-1H-pyrazolo[4,3-c]quinolin-8-yl)(2-(3-fluoropyridin-2-yl)-4-(2, 2,2-trifluoroethyl)pyrazolin-1-yl)methanone (408)

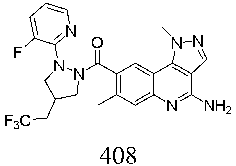

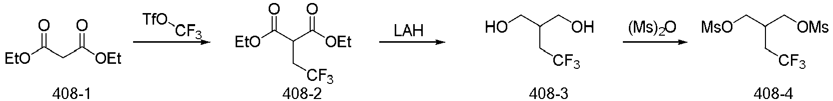

### Step 1: Preparation of diethyl 2-(2,2,2-trifluoroethyl)malonate (408-2)

Diethyl malonate (408-1, 1000 mg, 6.24 mmol) was dissolved in MeCN (15 mL), followed by the addition of trifluoromethyl trifluoromethanesulfonate (1739 mg, 7.49 mmol) and potassium carbonate (1723 mg, 12.49 mmol) at room temperature. The mixture was further stirred at 80°C for 12 hours. After the reaction was completed, water (50 mL) was added to the reaction mixture, and the reaction mixture was extracted with ethyl acetate (60 mLx2). The organic phases were combined, washed with saturated brine (50 mL), dried over anhydrous sodium sulfate and filtered, and the filtrate was concentrated under reduced pressure. The crude product was purified by silica gel column chromatography (eluent: ethyl acetate/petroleum ether=1/20) to obtain 408-2 (500 mg, yield 33%, a yellow solid).

¹H NMR (400 MHz, CHCl₃-*d*) δ 4.28-4.21 (m, 4H), 3.65-3.62 (m, 1H), 2.86-2.77 (m, 2H), 1.30-1.27 (m, 6H).

¹⁹F NMR (376 MHz, CHCl₃-*d*) δ -65.87.

### Step 2: Preparation of 2-(2,2,2-trifluoroethyl)propane-1,3-diol (408-3)

Compound 408-2 (250 mg, 1.03 mmol) was dissolved in tetrahydrofuran (6 mL), followed by the addition of lithium aluminum tetrahydride (118 mg, 3.10 mmol) at 0°C. The mixture was reacted at 60°C for 12 hours. After the reaction was completed, the reaction mixture was quenched with sodium sulfate decahydrate, and filtered, and the filtrate was concentrated under reduced pressure to obtain a crude product 408-3 as a colourless oil liquid, which was directly used for the next step.

LC-MS (ESI+): 159.1 m/z [M+H]⁺.

### Step 3: Preparation of 2-(2,2,2-trifluoroethyl)propane-1,3-diyl dimethanesulfonate (408-4)

The crude product 408-3 was dissolved in DCM (5 mL), followed by the addition of DIEA (403 mg, 3.1 mmol) and methanesulfonic anhydride (363 mg, 2.08 mmol) at room temperature, and further stirred at room temperature for 12 hours. After the reaction was completed, water (25 mL) was added to the reaction mixture, and the reaction mixture was extracted with ethyl acetate (30 mLx2). The organic phases were combined, washed with saturated brine (20 mL), dried over anhydrous sodium sulfate and filtered, and the filtrate was concentrated under reduced pressure. The crude product was purified by silica gel column chromatography (eluent: ethyl acetate/petroleum ether=3/20) to obtain 408-4 (200 mg, yield 64%, a yellow solid).

LC-MS (ESI+): 315.2 m/z [M+H]⁺.

Remaining steps were according to the synthetic method of Example 360 to give (4-amino-1,7-dimethyl-1 H-pyrazolo[4,3-c]quinolin-8-yl)(2-(3-fluoropyridin-2-yl)-4-(2, 2,2-trifluoroethyl)pyrazolin-1-yl)methanone, except that Compound 408-4 was used instead of 1,3-dibromo-2-methylpropane.

LC-MS (ESI+): 488.2 m/z [M+H]⁺.

¹H NMR (400 MHz, DMSO-*d*₆) δ 8.22 - 8.15 (m, 3H), 7.53 (s, 1H), 7.32 (s, 1H), 7.15-7.09 (m, 1H), 7.04 (s, 2H), 4.05 (s, 3H), 3.90 (s, 1H), 3.71 (s, 1H), 3.60 - 3.40 (m, 2H), 2.63 (s, 3H), 2.41 (s, 3H).

The following compounds were obtained according to the synthetic method in Example 408 using corresponding raw materials.

| Example | Structure | Mass spectrometry data (ESI+): m/z | Nuclear magnetic data |
|---|---|---|---|
| 409 | 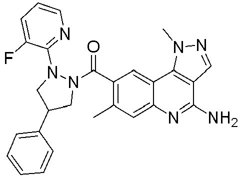 | 482.2 | |
| 410 | 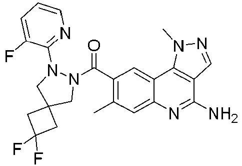 | 482.1 | ¹H NMR (400 MHz, DMSO-*d₆*) δ 8.10 (s, 1H), 8.07 - 8.04 (m, 1H), 7.77 (s, 1H), 7.44 (s, 1H), 7.24 (s, 1H), 7.05 - 6.88 (m, 3H), 3.96 (s, 4H), 3.73 (s, 3H), 2.70 - 2.58 (m, 2H), 2.47 (s, 2H), 2.32 (s, 3H). |
| 411 | 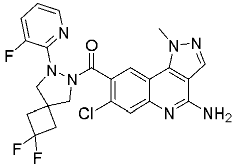 | 502.2 | |
| 412 | 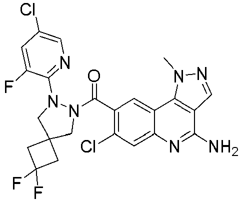 | 536.1 | ¹H NMR (400 MHz, DMSO-*d₆*) δ 8.26 (s, 1H), 8.23 (s, 1H),7.85 (s, 2H), 7.51 (s, 1H), 7.29 (s, 2H), 4.13 (s, 3H), 4.10-4.02 (m, 2H), 3.83 (s, 2H), 2.68 (s, 4H). |
| 413 | 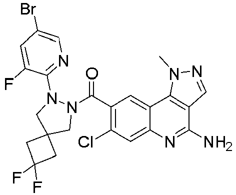 | 580.2 | ¹H NMR (400 MHz, DMSO-*d₆*) δ 8.32 - 8.29 (m, 1H), 8.21 (s, 1H), 7.89 (d, *J* = 10.8 Hz, 1H), 7.82 (s, 1H), 7.49 (s, 1H), 7.24 (s, 2H), 4.11 (s, 3H), 4.05 (s, 2H), 3.81 (s, 2H), 2.74 - 2.61 (m, 4H). |
| 414 | 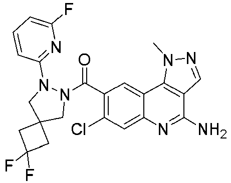 | 502.1 | ¹H NMR (400 MHz, DMSO-*d₆*) δ 8.40 - 8.11 (m, 3H), 7.81 (d, *J* = 8.4 Hz, 1H), 7.50 (s, 1H), 7.25 (s, 2H), 6.57 (d, *J* = 7.9 Hz, 1H), 4.46-4.36 (m, 2H), 4.27 (s, 3H), 3.66 (s, 2H), 3.0-2.84 (m, 2H), 2.72 - 2.61 (m, 1H), 2.42 - 2.29 (m, 1H). |
| 415 | 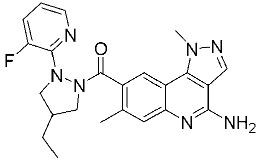 | 434.1 | ¹H NMR (400 MHz, DMSO-*d₆*) δ 8.19 (s, 1H), 8.14 (d, *J =* 4.8 Hz, 1H), 7.84 (s, 1H), 7.49 (s, 1H), 7.32 (s, 1H), 7.14 - 7.05 (m, 3H), 4.03 (s, 3H), 3.81 (s, 2H), 3.57 (s, 2H), 3.27 (s, 1H), 2.40 (s, 3H), 1.44 (s, 2H), 0.90 (s, 3H). |

### Example 416: (4-amino-1,7-dimethyl-1H-pyrazolo[4,3-c]quinolin-8-yl)(2-(3-fluoropyridin-2-yl)-4-me thyltetrahydropyridazin-1(2H)-yl)methanone and (4-amino-1,7-dimethyl-1H-pyrazolo[4,3-c]quinolin-8-yl)(2-(3-fluoropyridin-2-yl)-5-me thyltetrahydropyridazin-1(2H)-yl)methanone (416)

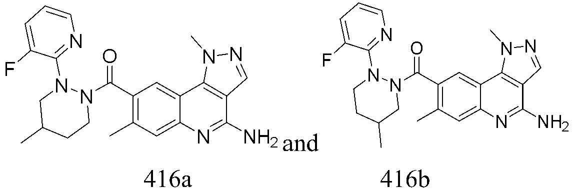

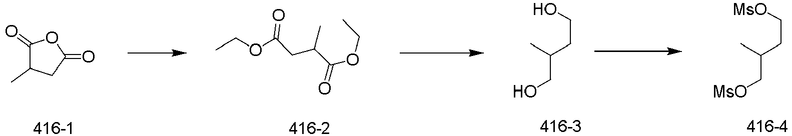

### Step 1: Preparation of diethyl 2-methylsuccinate (416-2)

3-Methyldihydrofuran-2,5-dione (416-1, 2.50 g, 21.9 mmol) was dissolved in ethanol (20 mL), followed by the dropwise addition of concentrated sulfuric acid (1 mL), and the resulting mixture was stirred at 70°C for 18 hours. The reaction solution was directly concentrated under reduced pressure to remove ethanol. The residues were diluted with ethyl acetate (100 mL), and then extracted with water (100 mL). The organic phase was washed with saturated brine, dried over anhydrous sodium sulfate and filtered, and the filtrate was concentrated under reduced pressure to obtain Compound 416-2 (3.0 g, yield 73%, a colourless oil).

### Step 2: Preparation of 2-methylbutane-1,4-diol (416-3)

Compound 416-2 (2.0 g, 10.6 mmol) was dissolved in THF (20 mL), followed by the addition of LiAlH₄ (6.36 mL, 15.9 mmol) at 0°C, and the resulting mixture was stirred at 60°C for 2 hours. The reaction mixture was returned to room temperature and stirred for 16 hours. Water (4 mL) and 15% aqueous sodium hydroxide solution (4 mL) were added to the reaction solution at 0°C, and after stirring for 0.5 hour, water (12 mL) was added and further stirred for 0.5 hour. The mixed solution was diluted with ethyl acetate and then filtered, and the filtrate was concentrated under reduced pressure to obtain Compound 416-3 (800 mg, yield 72%, a colourless oil).

### Step 3: Preparation of 2-methylbutane-1,4-diyl dimethanesulfonate (416-4)

Compound 416-3 (0.5 g, 4.8 mmol) was dissolved in DCM (10 mL), followed by the slow addition of DIEA (3.71 g, 28.8 mmol) and methanesulfonic anhydride (2.5 g, 14.4 mmol) at 0°C, and the resulting mixture was stirred at room temperature for 2 hours. Water (20 mL) was added to the reaction solution, and the reaction solution was extracted with DCM (20 mL × 3). The organic phases were combined, washed with saturated brine, dried over anhydrous sodium sulfate and filtered, and the filtrate was concentrated under reduced pressure to obtain Compound 416-4 (0.5 g, yield 40%, a yellow oil).

Remaining steps were according to the synthetic method of Example 360 to give (4-amino-1,7-dimethyl-1H-pyrazolo[4,3-c]**quinolin**-8-yl)(2-(3-fluoropyridin-2-yl)-4-me thyltetrahydropyridazin-1(2H)-yl)methanone, except that Compound 416-4 was used instead of 1,3-dibromo-2-methylpropane.

LC-MS (ESI+): 434.2 m/z [M+H]⁺.

### Example 417: Preparation of (4-amino-1,7-dimethyl-1H-pyrazolo[4,3-c]quinolin-8-yl)(2-(3-fluoropyridin-2-yl)-4-me thoxypyrazolidin-1-yl)methanone (417)

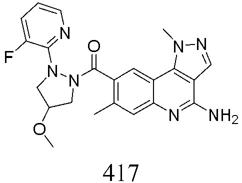

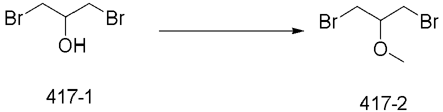

### Step 1: Preparation of 1,3-dibromo-2-methoxypropane (417-2)

1,3-Dibromo-2-propanol (417-1, 1.00 g, 4.63 mmol) and 1,8-bis(dimethylamino)naphtalene (1.98 g, 9.26 mmol) were dissolved in DCM (5 mL), followed by the addition of trimethyloxonium tetrafluoroborate (1.16 g, 7.87 mmol) at 0°C in one portion. The mixture was heated to room temperature and reacted for 12 h. After the reaction was completed, the reaction solution was filtered, and the filtrate was concentrated under reduced pressure. The crude product was purified by silica gel column chromatography (eluent: petroleum ether/dichloromethane=5/1) to obtain Compound 417-2 (1.06 g, yield 66%).

Remaining steps were according to the synthetic method of Example 360 to give (4-Amino-1,7-dimethyl-1H-pyrazolo[4,3-c]quinolin-8-yl)(2-(3-fluoropyridin-2-yl)-4-m ethoxypyrazolidin-1-yl)methanone, except that Compound 417-2 was used instead of 1,3-dibromo-2-methylpropane.

LC-MS (ESI+): 436.2 m/z [M+H]⁺.

### Example 418: Preparation of 2-(2-(4-amino-1,7-dimethyl-1H-pyrazolo[4,3-c]quinoline-8-carbonyl)-4-methylpyrazoli din-1-yl)nicotinonitrile (418)

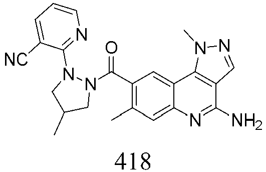

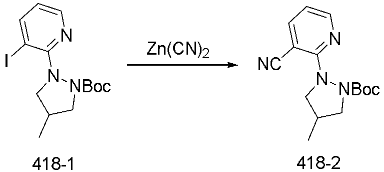

### Step 1: Preparation of tert-butyl 2-(3-cyanopyridin-2-yl)-4-methylpyrazolidine-1-carboxylate (418-2)

Tert-butyl 2-(3-iodopyridin-2-yl)-4-methylpyrazolidine-1-carboxylate (418-1, synthesized according to the method in Example 360) (1.25 g, 3.21 mmol) was dissolved in NMP (13 mL), and then zinc cyanide (1.88 g, 16.1 mmol), tetrakis(triphenylphosphine)palladium (370 mg, 0.32 mmol) and cuprous iodide (610 mg, 3.21 mmol) were added successively. The resulting mixture was purged with nitrogen and then stirred at 125°C for 30 minutes. After the reaction was completed, the reaction solution was filtered, and the filter cake was washed with dichloromethane, and the filtrate was concentrated under reduced pressure. The residues were purified by silica gel column chromatography (petroleum ether/ethyl acetate = 10/1) to obtain Compound 418-2 (630 mg, yield 68%, a light yellow oil).

LC-MS (ESI+): 289.2 m/z [M+H]⁺.

Remaining steps were according to the synthetic method of Example 360 to give 2-(2-(4-amino-1,7-dimethyl-1H-pyrazolo[4,3-c]quinoline-8-carbonyl)-4-methylpyrazoli din-1-yl)nicotinonitrile, except that Compound 418-2 was used instead of Compound 360-4.

LC-MS (ESI+): 427.2 m/z [M+H]⁺.

### Example 419: Preparation of (4-amino-7-chloro-1-methyl-1H-pyrazolo[4,3-c]quinolin-8-yl)(4-fluoro-2-(2-fluorophen yl)-4-methylpyrrolidin-1-yl)methanone (419)

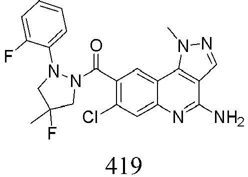

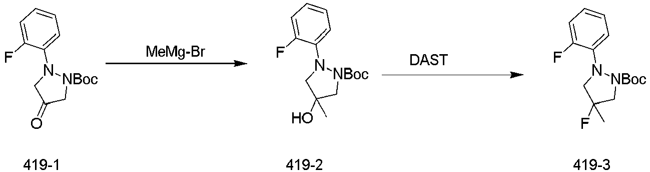

### Step 1: Preparation of tert-butyl 2-(2-fluorophenyl)-4-hydroxy-4-methylpyrazolidine-1-carboxylate (419-2)

Compound 419-1 (synthesized according to Example 350) (400 mg, 1.43 mmol) was dissolved in tetrahydrofuran (10 mL) at room temperature, followed by the dropwise addition of methyl magnesium bromide (340 mg, 2.85 mmol) at -78°C. The reaction was carried out at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 419-2 (320 mg, yield 75%).

### Step 2: Preparation of tert-butyl 4-fluoro-2-(2-fluorophenyl)-4-methylpyrazolidine-1-carboxylate (419-3)

Compound 419-2 (350.00 mg, 1.18 mmol) was dissolved in dichloromethane (10mL) at room temperature, followed by the dropwise addition of diethylaminosulphur trifluoride (571 mg, 3.54 mmol) at 0°C. The reaction was carried out at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 419-3 (180 mg, yield 51%).

LC-MS (ESI+): 299.2 m/z [M+H]⁺.

Remaining steps were according to the synthetic method of Example 360 to give (4-amino-7-chloro-1-methyl-1H-pyrazolo[4,3-c]quinolin-8-yl)(4-fluoro-2-(2-fluorophen yl)-4-methylpyrrolidin-1-yl)methanone, except that Compound 419-3 was used instead of Compound 360-4.

LC-MS (ESI+): 457.1 m/z [M+H]^{+,}.

¹H NMR (400 MHz, DMSO-*d₆*) δ 8.20 (s, 1H), 8.02 (s, 1H), 7.48 (s, 1H), 7.40 (t, *J* = 8.0 Hz, 1H), 7.24 (s, 2H), 7.10 (t, *J =* 6.8 Hz, 1H), 6.90 (s, 2H), 4.05 (s, 3H), 3.86-3.75 (m, 2H), 3.64-3.52 (m, 2H), 1.62 (d, *J =* 20.0 Hz, 3H).

The following compounds were obtained according to the synthetic method in Example 419 using corresponding raw materials.

| Example | Structure | Mass spectrometry data (ESI+): m/z | Nuclear magnetic data |
|---|---|---|---|
| 420 | 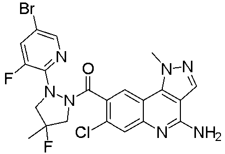 | 536.2 | |
| 421 | 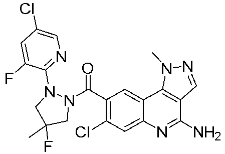 | 492.2 | ¹H NMR (400 MHz, DMSO-*d₆*) δ 8.24 (d, *J =* 6.0 Hz, 2H), 7.97 (s, 1H), 7.84 (d, *J* = 10.8 Hz, 1H), 7.50 (s, 1H), 7.29 (s, 2H), 4.19 (s, 3H), 4.14 - 3.95 (m, 3H), 3.75 (dd, *J =* 34.4, 13.2 Hz, 1H), 1.66 (d, *J =* 21.0 Hz, 3H). |
| 422 | 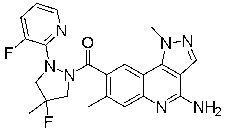 | 438.2 | |
| 423 | 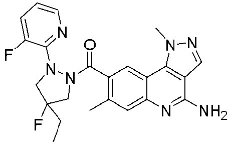 | 452.1 | |
| 424 | 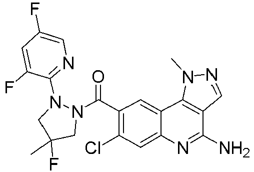 | 476.2 | ¹H NMR (400 MHz, DMSO-*d₆*) δ 8.26 (d, *J =* 2.4 Hz, 2H), 7.96 (s, 1H), 7.77 (t, *J =* 9.6 Hz, 1H), 7.51 (s, 1H), 7.29 (s, 2H), 4.20 (s, 3H), 4.02 (d, *J =* 15.2 Hz, 2H), 3.73 (dd, *J =* 33.6, 13.2 Hz, 2H), 1.67 (d, *J =* 21.2 Hz, 3H). |
| 425 | 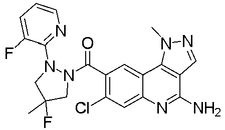 | 458.2 | |
| 426 | 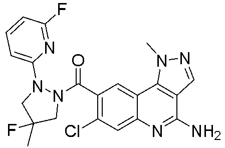 | 458.2 | |

### Example 427: (4-amino-1,7-dimethyl-1H-pyrazolo[4,3-c]quinolin-8-yl)(2-(3-fluoropyridin-2-yl)-4-hy droxy-4-methylpyrazolin-1-yl)methanone (427)

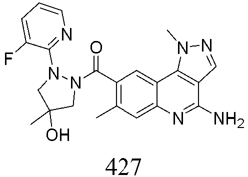

(4-Amino-1,7-dimethyl-1H-pyrazolo[4,3-c]quinolin-8-yl)(2-(3-fluoropyridin-2-yl)-4-hydroxy-4-methylpyrazolin-1-yl)methanone was obtained according to the method of Example 360, except that Compound 419-2 was used instead of 360-4. LC-MS (ESI+): 436.2 m/z [M+H]⁺.

### Example 428: (4-amino-1,7-dimethyl-1H-pyrazolo[4,3-c]quinolin-8-yl)(2-(3-fluoropyridin-2-yl)-4-me thoxy-4-methylpyrazolin-1-yl)methanone (428)

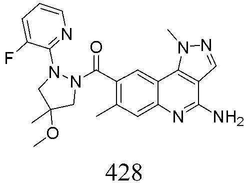

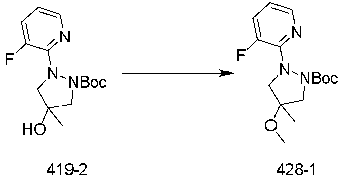

### Step 1: Preparation of tert-butyl 2-(3-fluoropyridin-2-yl)-4-methoxy-4-methylpyrazoline-1-carboxylate (428-1)

Compound 419-2 (0.1 g, 0.34 mmol) was dissolved in THF (5 mL), followed by the addition of NaH (60%, 20 mg, 0.50 mmol) at 0°C. The mixture was stirred for 0.5 hour, followed by the addition of iodomethane (71.6 mg, 0.50 mL), and the resulting mixture was further stirred at room temperature for 2 hours. Water (10 mL) was added to the reaction solution, and the reaction solution was extracted with ethyl acetate (10 mL × 3). The organic phases were combined, washed with saturated brine, dried over anhydrous sodium sulfate and filtered. The filtrate was concentrated under reduced pressure, and the residues were purified by Prep-TLC (petroleum ether/ethyl acetate = 3/1) to obtain Compound 428-1 (50 mg, yield 48%, a yellow oil).

LC-MS (ESI+): 312.2 m/z [M+H]⁺.

Remaining steps were according to the synthetic method of Example 360 to give (4-amino-1,7-dimethyl-1H-pyrazolo[4,3-c]quinolin-8-yl)(2-(3-fluoropyridin-2-yl)-4-me thoxy-4-methylpyrazolin-1-yl)methanone, except that Compound 428-1 was used instead of Compound 360-4.

LC-MS (ESI+): 450.2 m/z [M+H]⁺.

### Example 429: Preparation of (4-amino-1,7-dimethyl-1H-pyrazolo[4,3-c]quinolin-8-yl)(2-(3-fluoropyridin-2-yl)-4-me thylpyrazolin-1-yl)methanone (429)

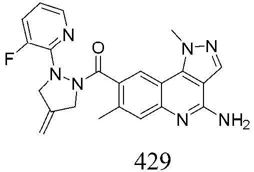

((4-Amino-1,7-dimethyl-1H-pyrazolo[4,3-c]quinolin-8-yl)(2-(3-fluoropyridin-2-yl) -4-methylpyrazolin-1-yl)methanone was synthesized according to Steps 4 and 5 of Example 360, using Compound 350-3.

LC-MS (ESI+): 437.1 m/z [M+H]⁺.

The following compounds were obtained according to the synthetic method in Example 429 using corresponding raw materials.

| Example | Structure | Mass spectrometry data (ESI+): m/z | Nuclear magnetic data |
|---|---|---|---|
| 430 | 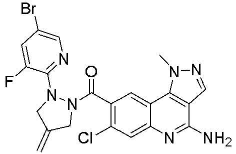 | 516.2 | |
| 431 | 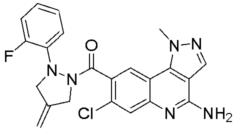 | 437.1 | ¹H NMR (400 MHz, DMSO-*d₆*) δ 8.20 (s, 1H), 7.95 (s, 1H), 7.51 (s, 1H), 7.37 (t, *J =* 8.0 Hz, 1H), 7.25 (s, 2H), 7.16-7.12 (m, 1H), 7.00 (d, *J =* 8.0 Hz, 2H), 5.24 (s, 1H), 5.16 (s, 1H), 4.55 (s, 2H), 4.04 (s, 2H), 3.95 (s, 3H). |
| 432 | 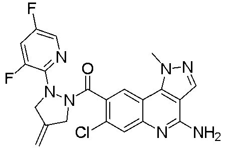 | 456.2 | |
| 433 | 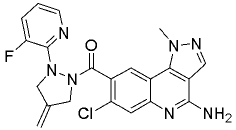 | 438.0 | ¹H NMR (400 MHz, DMSO-*d₆*) δ 8.22 (s, 1H), 8.21 - 8.16 (m, 1H), 7.83 (s, 1H), 7.61 - 7.46 (m, 2H), 7.27 (s, 2H), 7.19 - 7.06 (m, 1H), 5.25 (s, 1H), 5.19 (s, 1H), 4.65 - 4.32 (m, 2H), 4.32 - 4.18 (m, 2H), 4.06 (s, 3H). |
| 434 | 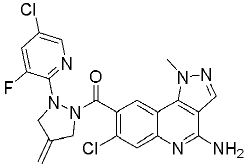 | 472.0 | |
| 435 | 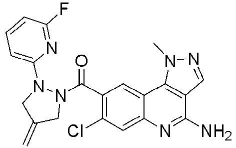 | 438.2 | ¹H NMR (400 MHz, DMSO-*d₆*) δ 8.35 (s, 1H), 8.27 (s, 1H), 7.92-7.86 (m, 1H), 7.58 (s, 1H), 7.30 (m, 3H), 6.65 (dd, *J =* 8.0 , 2.4 Hz, 1H), 5.24 (s, 2 H), 4.93-4.76 (m, 2H), 4.31 (s, 2H), 4.25 - 3.90 (m, 2H). |

### Example 436: Preparation of (4-amino-1,7-dimethyl-1H-pyrazolo[4,3-c]quinolin-8-yl)(2-(2-fluorophenyl)-4-methoxy pyrrolidin-1-yl)methanone (436)

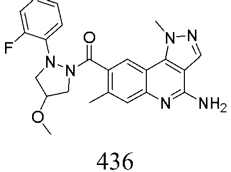

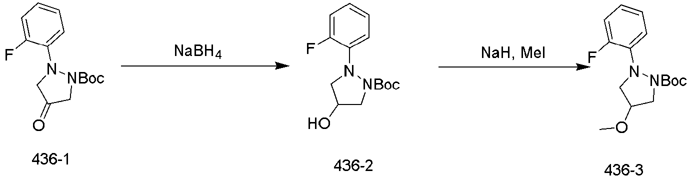

### Step 1: Preparation of tert-butyl 2-(2-fluorophenyl)-4-hydroxypyrrolidine-1-carboxylate (436-2)

Compound 436-1 (synthesized according to the method in Example 350) (200 mg, 0.71 mmol) was dissolved in methanol (5mL) solution at room temperature, followed by the addition of sodium borohydride (80.91 mg, 2.14 mol) at 0°C. The reaction was carried out at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 436-2 (200 mg, yield 99%).

### Step 2: Preparation of tert-butyl 2-(2-fluorophenyl)-4-methoxypyrazolidine-1-carboxylate (436-3)

Compound 436-2 (200 mg, 0.71 mmol) was dissolved in N,N-dimethylformamide (4 mL) solution at room temperature, followed by the addition of sodium hydride (60%, 57 mg, 1.42 mmol) at 0°C. The mixture was stirred for 1 hour, followed by the addition of iodomethane (151 mg, 1.06 mmol), and reacted at room temperature for 12 hours. The resulting reaction mixture was diluted with water (50.0 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 436-3 (200 mg, yield 100%).

LC-MS (ESI+): 297.2 m/z [M+H]⁺.

Remaining steps were according to the synthetic method of Example 360 to give (4-amino-1,7-dimethyl-1H-pyrazolo[4,3-c]quinolin-8-yl)(2-(2-fluorophenyl)-4-methoxy pyrrolidin-1-yl)methanone, except that Compound 436-3 was used instead of Compound 360-4.

LC-MS (ESI+): 435.2 m/z [M+H]⁺.

¹H NMR(400 MHz, DMSO-*d₆*) δ 8.19 (s, 1H), 8.15 (s, 1H), 7.85 (s, 1H), 7.35 (s, 2H), 7.14 (t, *J =* 7.9 Hz, 1H), 7.03 (s, 2H), 6.97 - 6.95 (m, 1H), 4.33 (s, 1H), 3.83 (s, 2H), 3.58 (s, 2H), 3.47 (s, 3H), 3.17 (s, 3H), 2.36 (s, 3H).

The following compounds were obtained according to the synthetic method in Example 436 using corresponding raw materials.

| Example | Structure | Mass spectrometry data (ESI+): m/z | Nuclear magnetic data |
|---|---|---|---|
| 437 | 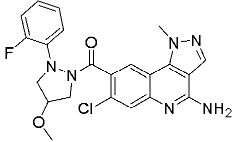 | 455.1 | ¹H NMR (400 MHz, DMSO-*d₆*) δ 8.18 (s, 1H), 7.94 (s, 1H), 7.48 (s, 1H), 7.38 - 7.31 (m, 1H), 7.23 (s, 2H), 7.09 (t, *J =* 6.9 Hz, 1H), 6.93-6.90 (m, 2H), 4.37 (s, 1H), 3.99 (s, 3H), 3.63-3.62 (m, 2H), 3.52 (d, *J =* 8.0 Hz, 2H), 3.13 (s, 3H). |
| 438 | 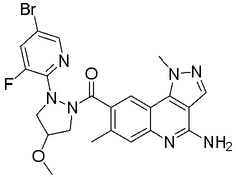 | 514.2 | |
| 439 | 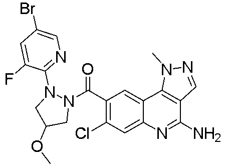 | 534.2 | |
| 440 | 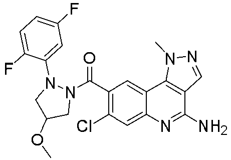 | 473.2 | |
| 441 | 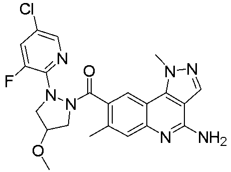 | 470.2 | |
| 442 | 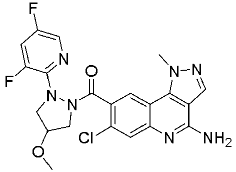 | 474.1 | ¹H NMR (400 MHz, DMSO-*d₆*) δ 8.27 - 8.18 (m, 2H), 7.89 (s, 1H), 7.75 (t, *J =* 9.6 Hz, 1H), 7.50 (s, 1H), 7.27 (s, 2H), 4.40 (s, 1H), 4.15 (s, 3H), 3.90 - 3.60 (m, 4H), 3.15 (s, 3H). |
| 443 | 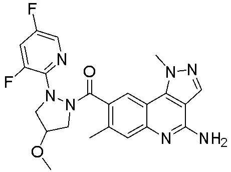 | 454.1 | ¹H NMR (400 MHz, DMSO-*d₆*) δ 8.20 - 8.17(m, 2H), 8.00 - 7.80 (m, 1H), 7.80 - 7.65 (m, 1H), 7.32 (s, 1H), 7.02 (s, 2H), 4.33 (s, 1H), 4.09 (s, 3H), 3.74 (s, 4H), 3.15 (s, 3H), 2.38 (s, 3H). |
| 444 | 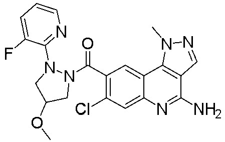 | 456.2 | |
| 445 | 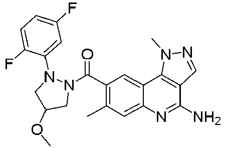 | 453.2 | |
| 446 | 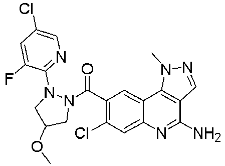 | 490.0 | |
| 447 | 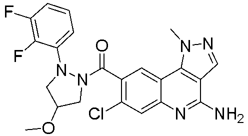 | 473.1 | ¹H NMR (400 MHz, DMSO-*d₆*) δ 8.22 (s, 1H), 8.01 (s, 1H), 7.51 (s, 1H), 7.27 (s, 2H), 7.20 (d, *J =* 7.6 Hz, 1H), 7.10 (s, 1H), 6.92 (d, *J* = 8.4 Hz, 1H), 4.42 (s, 1H), 4.09 (s, 3H), 3.69 (d, *J =* 12.0 Hz, 1H), 3.59 (d, *J =* 12.4 Hz, 2H), 3.17 (s, 3H), 2.54 (s, 1H). |
| 448 | 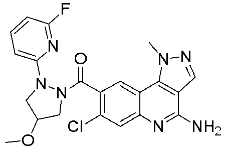 | 456.2 | |
| 449 | 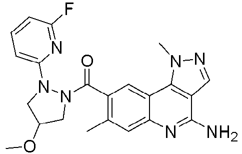 | 436.2 | |

### Example 450: Preparation of (4-amino-1,7-dimethyl-1H-pyrazolo[4,3-c]quinolin-8-yl)(4-fluoro-2-(3-fluoropyridin-2-yl)pyrazolidin-1-yl)methanone (450)

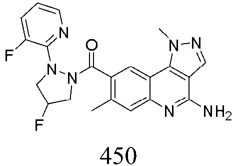

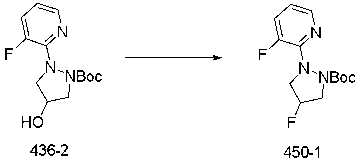

### Step 1: Preparation of tert-butyl 4-fluoro-2-(3-fluoropyridin-2-yl)pyrazoline-1-carboxylate (450-1)

Compound 436-2 (60 mg, 0.21 mmol) was dissolved in DCM (2 mL), followed by the addition of DAST (68 mg, 0.42 mmol) at normal temperature, and reacted at room temperature for 3 h. After completion of the reaction, the reaction solution was quenched with sodium bicarbonate saturated solution (20 mL), and then extracted with DCM (20 mLx3). The organic phases were combined, dried over anhydrous sodium sulfate and filtered, and the filtrate was concentrated under reduced pressure. The crude product was purified by silica gel column chromatography (eluent: petroleum ether/ethyl acetate=2/1) to obtain 450-1 (20 mg, yield 33%).

LC-MS (ESI+): 286.2 m/z [M+H]⁺.

Remaining steps were according to the synthetic method of Example 360 to give (4-amino-1,7-dimethyl-1H-pyrazolo[4,3-c]quinolin-8-yl)(4-fluoro-2-(3-fluoropyridin-2-yl)pyrazolidin-1-yl)methanone, except that Compound 450-1 was used instead of Compound 360-4.

LC-MS (ESI+): 424.2 m/z [M+H]⁺.

¹H NMR (400 MHz, DMSO-*d₆*) δ 8.19 (s, 1H), 8.17 - 8.13 (m, 1H), 7.92 (s, 1H), 7.54 (s, 1H), 7.35 (s, 1H), 7.11 - 7.03 (m, 3H), 5.81 - 5.60 (m, 1H), 4.40 - 3.73 (m, 7H), 2.41 (s, 3H).

### Example 451: (4-amino-7-chloro-1-methyl-1H-pyrazolo[4,3-c]quinolin-8-yl)(2-(3-fluoropyridin-2-yl)-4-(2,2,2 -trifluoroethoxy)pyrazolidin-1-yl)methanone (451)

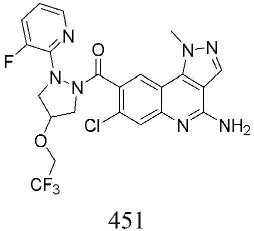

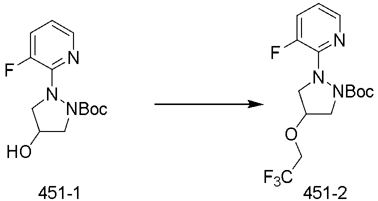

### Step 1: Preparation of tert-butyl 2-(3-fluoropyridin-2-yl)-4-(2,2,2-trifluoroethoxy)pyrazolidine-1-carboxylate (451-2)

Tert-butyl 2-(3-fluoropyridin-2-yl)-4-hydroxypyrazolidine-1-carboxylate (451-1, synthesized according to the method in Example 436) (200 mg, 0.70 mmol) was dissolved in THF (5 mL), and NaH (60%, 84 mg, 2.80 mmol) was added at 0°C. After 10 minutes, 2,2,2-trifluoroethyl trifluoromethanesulfonate (649 mg, 2.80 mmol) was added dropwise slowly. The resulting mixture was stirred at room temperature for 16 hours. After the reaction was completed, the reaction solution was poured into ice water (15 mL), and extracted with ethyl acetate (15 mL x3). The organic phases were combined, washed with saturated brine, dried over anhydrous sodium sulfate and filtered. The filtrate was concentrated under reduced pressure, and the residues were purified by silica gel column chromatography (petroleum ether/ethyl acetate = 3/1) to obtain Compound 451-2 (100 mg, yield 38%, a yellow oil).

LC-MS (ESI+): 366.2 m/z [M+H]⁺.

Remaining steps were according to the synthetic method of Example 360 to give (4-amino-7-chloro-1-methyl-1H-pyrazolo[4,3-c]quinolin-8-yl)(2-(3-fluoropyridin-2-yl)-4-(2,2,2-trifluoroethoxy)pyrazolidin-1-yl)methanone, except that Compound 451-2 was used instead of Compound 360-4.

LC-MS (ESI+): 524.2 m/z [M+H]⁺.

The following compounds were obtained according to the synthetic method in Example 451 using corresponding raw materials.

| Example | Structure | Mass spectrometry data (ESI+): m/z | Nuclear magnetic data |
|---|---|---|---|
| 452 | 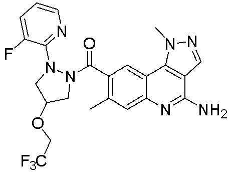 | 504.2 | |
| 453 | 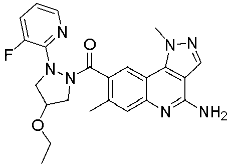 | 450.0 | ¹H NMR (400 MHz, DMSO-*d₆*) δ 8.17 - 8.14 (m, 1H), 8.11 - 8.07 (m, 1H), 7.88 (s, 1H), 7.48 (s, 1H), 7.31 (s, 1H), 7.15 - 6.90 (m, 3H), 4.52 - 4.34 (m, 1H), 4.01 (s, 3H), 3.80 - 3.64 (m, 2H), 3.46 - 3.25 (m, 4H), 2.39 (s, 3H), 1.06 - 0.76 (m, 3H). |
| 454 | 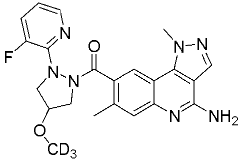 | 439.0 | |

### Example 455: Preparation of (4-amino-1,7-dimethyl-1H-pyrazolo[4,3-c]quinolin-8-yl)(4,4-difluoro-2-(3-fluoropyridi n-2-yl)pyrazolin-1-yl)methanone (455)

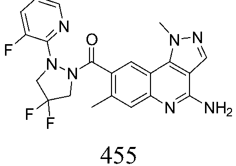

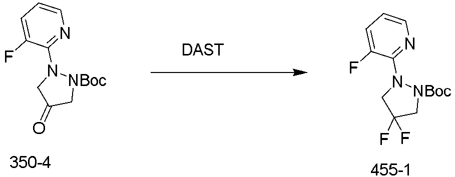

### Step 1: Preparation of tert-butyl 4,4-difluoro-2-(3-fluoropyridin-2-yl)pyrazoline-1-carboxylate (455-1)

Compound 350-4 (200 mg, 0.71 mmol) was dissolved in dichloromethane (10 mL) at room temperature, followed by the dropwise addition of diethylaminosulphur trifluoride (1.15 g, 7.13 mmol) at 0°C. The reaction was carried out at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 455-1 (100 mg, yield 46%).

LC-MS (ESI+): 304.2 m/z [M+H]⁺.

Remaining steps were according to the synthetic method of Example 360 to give (4-amino-1,7-dimethyl-1H-pyrazolo[4,3-c]quinolin-8-yl)(4,4-difluoro-2-(3-fluoropyridi n-2-yl)pyrazolin-1-yl)methanone, except that Compound 455-1 was used instead of Compound 360-4.

LC-MS (ESI+): 442.2 m/z [M+H]⁺.

¹H NMR (400 MHz, DMSO-*d₆*) δ 8.20 (d, *J =* 4.4 Hz, 2H), 8.03 (s, 1H), 7.63 (t, *J* = 10.0 Hz, 1H), 7.36 (s, 1H), 7.18 (td, *J =* 4.8, 2.4 Hz, 1H), 7.14 (s, 1H), 4.33 (s, 2H), 4.20 (t, *J =* 12.4 Hz, 2H), 4.09 (s, 3H), 2.43 (s, 3H).

The following compounds were obtained according to the synthetic method in Example 455 using corresponding raw materials.

| Example | Structure | Mass spectrometry data (ESI+): m/z | Nuclear magnetic data |
|---|---|---|---|
| 456 | 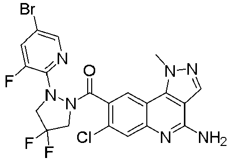 | 540.2 | |
| 457 | 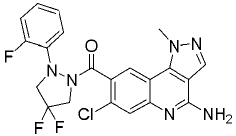 | 461.1 | ¹H NMR (400 MHz, DMSO-*d₆*) δ 8.21 (s, 1H), 8.08 (s, 1H), 7.51 - 7.47 (m, 2H), 7.29 (s, 2H), 7.19-7.15 (m, 1H), 7.03 (d, *J =* 8.0 Hz, 2H), 4.46 (s, 2H), 4.03 (s, 3H), 4.03-3.95 (m, 2H). |
| 458 | 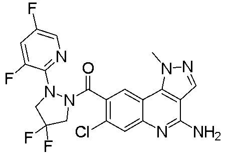 | 480.2 | |
| 459 | 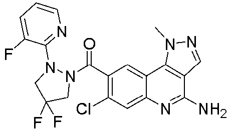 | 462.0 | ¹H NMR (400 MHz, DMSO-*d₆*) δ 8.24 - 8.22 (m, 2H), 7.97 (s, 1H), 7.64 - 7.51 (m, 2H), 7.32 (s, 2H), 7.22 - 7.12 (m, 1H), 4.36 (s, 3H), 4.18 - 4.10 (m, 4H). |
| 460 | 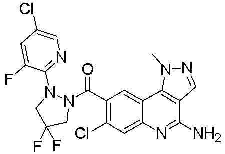 | 496.0 | |
| 461 | 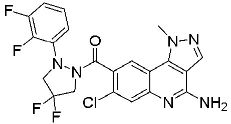 | 479.1 | ¹H NMR (400 MHz, DMSO-*d₆*) δ 8.24 (s, 1H), 8.16 (d, *J =* 8.0 Hz, 1H), 7.54 (s, 1H), 7.34 (d, *J =* 7.2 Hz, 3H), 7.21-7.15 (m, 1H), 7.09-7.03 (m, 1H), 4.47-4.41 (m, 2H), 4.13-4.05 (m, 5H). |
| 462 | 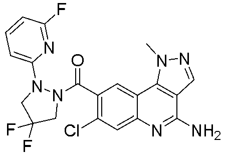 | 462.2 | ¹H NMR (400 MHz, DMSO-*d₆*) δ 8.36 (s, 1H), 8.24 (s, 1H), 7.91 (m, 1H), 7.55 (s, 1H), 7.40 -7.15 (m, 3H), 6.69 (dd, *J =* 8.0 , 2.0 Hz, 1H), 4.29 (s, 3H), 4.80-4.50 (m, 2H), 4.15-3.80 (m, 2H). |

### Example 463: Preparation of 1-(4-amino-1,7-dimethyl-1H-pyrazolo[4,3-c]quinoline-8-carbonyl)-2-(5-(trifluoromethy l)pyridin-2-yl)pyrazolidin-3-one (463)

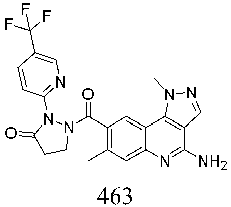

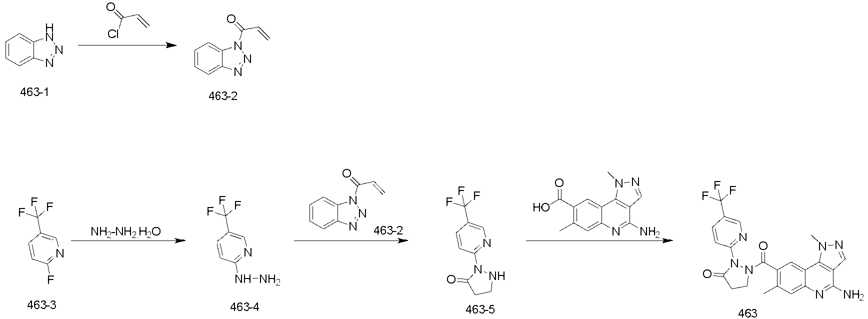

### Step 1: Preparation of 1-(benzotriazol-1-yl)acryloyl (463-2)

Benzotriazole (463-1, 1.00 g, 8.39 mmol) and acryloyl chloride (1.52 g, 16.8 mmol) were dissolved in dichloromethane (10 mL) at room temperature. The reaction was carried out at room temperature for 12 hours, and the resulting reaction mixture was diluted with water (100 mL). The resulting mixture was extracted with dichloromethane (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=4/1) to obtain Compound 463-2 (1.00 g, yield 68%).

### Step 2: Preparation of 2-hydrazino-5-(trifluoromethyl)pyridine (463-4)

2-Fluoro-5-(trifluoromethyl)pyridine (463-3, 1.00 g, 6.06 mmol) and hydrazine hydrate (775 mg, 24 mmol) were dissolved in ethanol (20 mL) at room temperature. The reaction was carried out at 80°C for 12 hours, and the resulting reaction mixture was diluted with water (200 mL). The resulting mixture was extracted with ethyl acetate (200 mL x 3). The organic phases were combined, washed with saturated brine (200 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 463-4 (1.00 g, yield 93%).

### Step 3: Preparation of 2-(5-(trifluoromethyl)pyridin-2-yl)pyrazolidin-3-one (463-5)

Compound 463-4 (500 mg, 2.82 mmol), 1-(benzotriazol-1-yl)acryloyl (978 mg, 5.65 mmol) and triethylamine (857 mg, 8.47 mmol) were dissolved in tetrahydrofuran (10 mL) at room temperature. The reaction was carried out at 50°C for 12 hours, and the resulting reaction mixture was diluted with water (100 mL). The resulting mixture was extracted with ethyl acetate (100 mL x 3). The organic phases were combined, washed with saturated brine (100 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 463-5 (50 mg, yield 8%).

### Step 4: Preparation of 1-(4-amino-1,7-dimethyl-1H-pyrazolo[4,3-c]quinoline-8-carbonyl)-2-(5-(trifluoromethy l)pyridin-2-yl)pyrazolidin-3-one (463)

Compound 463-5 (20 mg, 0.08 mmol), 4-amino-1,7-dimethyl-1H-pyrazolo[4,3-c]quinoline-8-carboxylic acid (18 mg, 0.08 mmol), N,N-diisopropylethylamine (30 mg, 0.23 mmol) and bromo-tris-pyrrolidino-phosphonium hexafluorophosphate (54 mg, 0.12 mmol) were dissolved in N.N-dimethylacetamide (10 mL) at room temperature. The reaction was carried out at room temperature for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=0/1) to obtain Compound 463 (2.00 mg, yield 5%).

LC-MS (ESI+): 470.1 m/z [M+H]⁺.

### Examples 464 and 465: Preparation of rel-(R)-(4-amino-1,7-dimethyl-1H-pyrazolo[4,3-c]quinolin-8-yl)(2-(3-fluoropyridin-2-y l)-4-methylpyrazolin-1-yl)methanone (464) and rel-(S)-(4-amino-1,7-dimethyl-1H-pyrazolo[4,3-c]quinolin-8-yl)(2-(3-fluoropyridin-2-yl )-4-methylpyrazolin-1-yl)methanone (465)

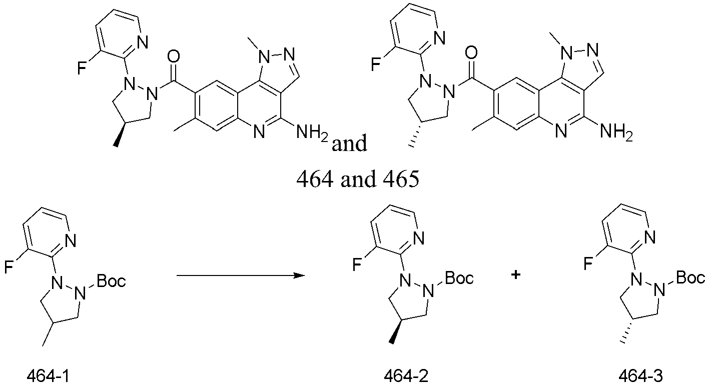

Compound 464-1 (synthesized according to the method in Example 360) was subjected to chiral resolution (chiral column: Whelk-O1(S,S), 0.46cm × 5cm; mobile phase: CO2: EtOH=95:5; flow rate: 2.5 mL/minute; column temperature: 25°C) to obtain Compound 464-2 (retention time: 1.18 minutes) which was randomly defined as tert-butyl rel-(R)-2-(3-fluoropyridin-2-yl)-4-methylpyrazoline-1-carboxylate, and Compound 464-3 (retention time: 1.42 minutes) which was randomly defined as tert-butyl rel-(S)-2-(3-fluoropyridin-2-yl)-4-methylpyrazoline-1-carboxylate.

Remaining steps were according to the synthetic method of Example 360 to give rel-(R)-(4-amino-1,7-dimethyl-1H-pyrazolo[4,3-c]quinolin-8-yl)(2-(3-fluoropyridin-2-y l)-4-methylpyrazolin-1-yl)methanone (464), except that Compound 464-2 was used instead of Compound 360-4.

LC-MS (ESI+): 420.2 m/z [M+H]⁺.

¹H NMR (400 MHz, DMSO-*d₆*) δ 8.16 (s, 1H), 8.12 (d, *J =* 4.0 Hz, 1H), 7.82 (s, 1H), 7.47 (s, 1H), 7.30 (s, 1H), 7.07 - 7.03 (m, 1H), 6.98 (s, 2H), 4.01 (s, 4H), 3.79 (s, 1H), 3.47 (s, 1H), 3.20 (s, 1H), 2.38 (s, 4H), 1.05 (s, 3H).

Remaining steps were according to the synthetic method of Example 360 to give rel-(S)-(4-amino-1,7-dimethyl-1H-pyrazolo[4,3-c]quinolin-8-yl)(2-(3-fluoropyridin-2-yl )-4-methylpyrazolin-1-yl)methanone (465), except that Compound 464-3 was used instead of Compound 360-4.

LC-MS (ESI+): 420.2 m/z [M+H]⁺.

¹H NMR (400 MHz, DMSO-*d₆*) δ 8.15 (s, 1H), 8.12 (d, *J =* 4.0 Hz, 1H), 7.82 (s, 1H), 7.46 (s, 1H), 7.29 (s, 1H), 7.07-7.03 (m, 1H), 6.99 (s, 2H), 4.01 (s, 4H), 3.79 (s, 1H), 3.47 (s, 1H), 3.19 (s, 1H), 2.38 (s, 4H), 1.04 (s, 3H).

The following compounds were obtained according to the synthetic method in Example 464 using corresponding raw materials.

| Example | Structure | Mass spectrometry data (ESI+): m/z | Nuclear magnetic data |
|---|---|---|---|
| 466 | 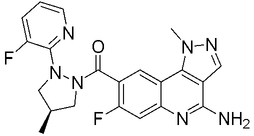 | 424.0 | ¹H NMR (400 MHz, DMSO-*d₆*) δ 8.20 (s, 1H), 8.10 (d, *J =* 4.7 Hz, 1H), 7.97 (s, 1H), 7.57 - 7.42 (m, 1H), 7.24 (s, 2H), 7.21 - 7.13 (m, 1H), 7.08 - 6.98 (m, 1H), 4.11 (s, 3H), 3.97 (s, 1H), 3.90 - 3.77 (m, 1H), 3.29 - 3.14 (m, 2H), 2.53 (d, *J =* 6.7 Hz, 1H), 1.05 (d, *J =* 5.1 Hz, 3H). |
| 467 | 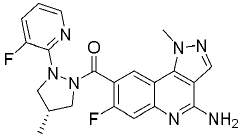 | 424.0 | ¹H NMR (400 MHz, DMSO-*d₆*) δ 8.20 (s, 1H), 8.09 (J, *J =* 4.7 Hz, 1H), 8.02 - 7.91 (m, 1H), 7.57 - 7.42 (m, 1H), 7.26 (s, 2H), 7.21 - 7.12 (m, 1H), 7.06 - 6.99 (m, 1H), 4.10 (s, 3H), 4.00 - 3.30 (m, 4H),2.69 - 2.51 (m, 1H), 1.05 (s, 3H). |

### Example 468: Preparation of (4-amino-1,7-dimethyl-1H-pyrazolo[4,3-c]quinolin-8-yl)(2-(5-cyclopropylpyridin-2-yl) pyrazolidin-1-yl)methanone (468)

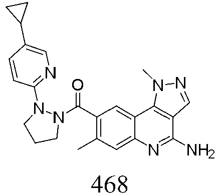

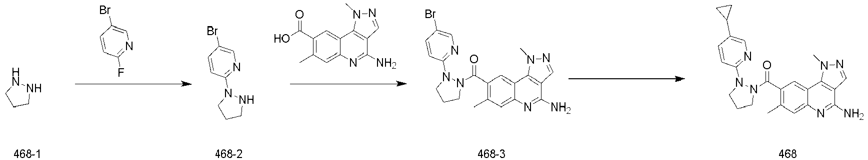

### Step 1: Preparation of 5-bromo-2-(pyrazolidin-1-yl)pyridine (468-2)

Pyrazolidine 468-1 (synthesized according to the method in Example 328) (100 mg, 141 mmol) was dissolved in 3 mL of water at room temperature, followed by the addition of 5-bromo-2-fluoropyridine (247 g, 1.41 mmol) and cesium carbonate (457 mg, 1.41 mmol). The reaction was carried out under microwave at 150°C for 1 hour. The reaction solution was directly concentrated to dryness, and the resulting residues were purified by silica gel column chromatography (eluent: petroleum ether/ethyl acetate=100%-50%). 3 ML of hydrochloric acid in methanol was added to the eluate, and then the eluate was concentrated under reduced pressure to obtain Compound 468-2 (hydrochloride, 120 mg, yield 37%).

LC-MS (ESI+): 228.0 m/z [M+H]⁺.

### Step 2: Preparation of (4-amino-1,7-dimethyl-1H-pyrazolo[4,3-c]quinolin-8-yl)(2-(5-bromopyridin-2-yl)pyraz olidin-1-yl)methanone (468-3)

Compound 468-2 (hydrochloride, 100 mg, 0.44 mmol) was dissolved in 3 mL of DMF at room temperature, followed by the addition of 4-amino-1,7-dimethyl-1H-pyrazolo[4,3-c]quinoline-8-carboxylic acid (112 mg, 0.44 mmol), bromo-tris-pyrrolidino-phosphonium hexafluorophosphate (306 mg, 0.66 mmol) and N,N-diisopropylethylamine (170 mg, 1.32 mmol). The reaction was carried out at room temperature overnight under nitrogen atmosphere. The reaction solution was quenched with water, and extracted with ethyl acetate (20 mLx2). The organic phases were combined, dried over anhydrous sodium sulfate and filtered. The filtrate was concentrated under reduced pressure, and the resulting residues were purified by silica gel column chromatography (eluent: dichloromethane/methanol=0-10%) to obtain Compound 468-3 (70 mg, yield 34%).

LC-MS (ESI+): 466.1 m/z [M+H]⁺.

### Step 3: Preparation of (4-amino-1,7-dimethyl-1H-pyrazolo[4,3-c]quinolin-8-yl)(2-(5-cyclopropylpyridin-2-yl) pyrazolidin-1-yl)methanone (468)

Compound 468-3 (50 mg, 0.11 mmol) was dissolved in 3 mL of dioxane at room temperature, followed by the addition of 2-cyclopropyl-4,4,5,5-tetramethyl-1,3,2-dioxaborolane (180 mg, 1.11 mmol), potassium carbonate (44 mg, 0.32 mmol), 1,1'-bis(diphenylphosphino)ferrocene]palladium dichloridedichloromethane complex (7.82 mg, 0.01 mmol) and 0.5 mL of water. The reaction was carried out at 90°C overnight under nitrogen atmosphere. The reaction solution was filtered through Celite, and concentrated under reduced pressure, and the resulting residues were separated by preparative liquid phase with methanol solvent to obtain Compound 468 (11 mg, yield 24%).

LC-MS (ESI+): 428.2 m/z [M+H]⁺.

The following compounds were obtained according to the synthetic method in Example 468 using corresponding raw materials.

| Example | Structure | Mass spectrometry data (ESI+): m/z | Nuclear magnetic data |
|---|---|---|---|
| 469 | 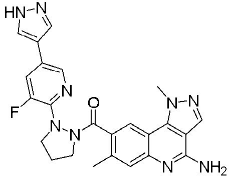 | 472.2 | |
| 470 | 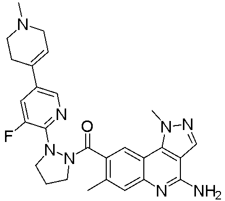 | 501.2 | |
| 471 | 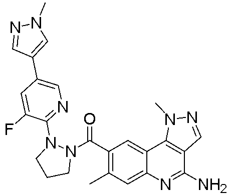 | 486.2 | ¹H NMR (400 MHz, DMSO-*d₆*) δ 8.40 (s, 1H), 8.20 - 8.14 (m, 2H), 7.89 (s, 1H), 7.86 - 7.73 (m, 2H), 7.33 (s, 1H), 6.98 (s, 2H), 3.97 (s, 3H), 3.88 - 3.76 (m, 5H), 3.59 (s, 2H), 2.38 (s, 3H), 2.08 (s, 2H). |
| 472 | 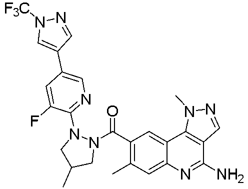 | 554.2 | ¹H NMR (400 MHz, DMSO-*d₆*) δ 9.00 (s, 1H), 8.54 (s, 1H), 8.48 (s, 1H), 8.14 (s, 1H), 7.89 (s, 2H), 7.29 (s, 1H), 6.99 (s, 2H), 4.05 (s, 3H), 3.84 (s, 2H), 3.42 (s, 2H), 2.54 (s, 1H), 2.39 (s, 3H), 1.06 (s, 3H). |
| 473 | 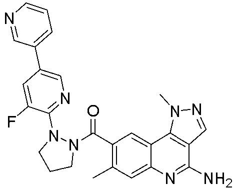 | 483.2 | |
| 474 | 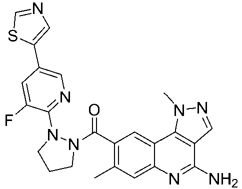 | 489.2 | ¹H NMR (400 MHz, DMSO-d6) δ 9.14 (s, 1H), 8.49 (s, 1H), 8.35 (s, 1H), 8.25 (s, 1H), 8.17 (s, 1H), 7.98 - 7.89 (m, 1H), 7.35 (s, 1H), 7.06 (s, 2H), 4.10 - 4.03 (m, 2H), 3.91 - 3.81 (m, 2H), 3.69 (s, 3H), 2.42 (s, 3H), 2.22 - 2.08 (m, 2H). |
| 475 | 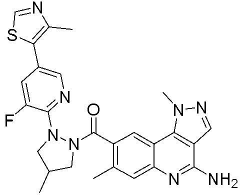 | 517.2 | |
| 476 | 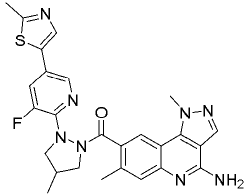 | 517.2 | |
| 477 | 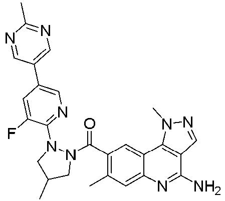 | 512.2 | |
| 478 | 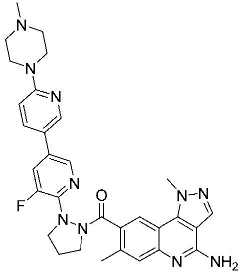 | 581.3 | |
| 479 | 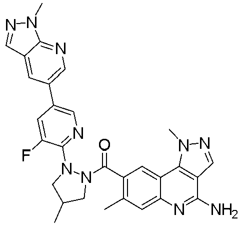 | 551.6 | |
| 480 | 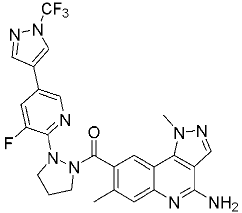 | 540.2 | ¹H NMR (400 MHz, DMSO-*d₆*) δ 9.03 (s, 1H), 8.57 (d, J = 1.6 Hz, 1H), 8.51 (s, 1H), 8.18 (s, 1H), 7.99 - 7.95 (m, 2H), 7.35 (s, 1H), 7.08 (s, 2H), 4.07 (s, 3H), 3.87 - 3.83 (m, 2H), 3.70 - 3.65 (m, 2H), 2.42 (s, 3H), 2.13 - 2.10 (m, 2H). |
| 481 | 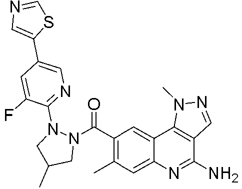 | 503.2 | |
| 482 | 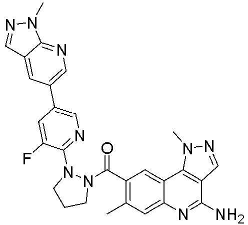 | 537.0 | |
| 483 | 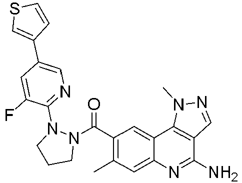 | 488.0 | |
| 484 | 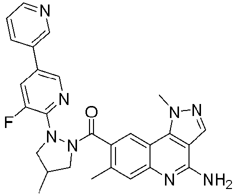 | 497.0 | |

### Example 485: Preparation of (4-amino-1,7-dimethyl-1H-pyrazolo[4,3-c]quinolin-8-yl)(2-(3-fluoro-5-(1-methylpiperi d-4-yl)pyridin-2-yl)pyrazolidin-1-yl)methanone (485)

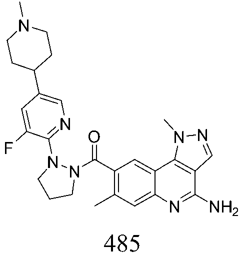

(4-Amino-1,7-dimethyl-1H-pyrazolo[4,3-c]quinolin-8-yl)(2-(3-fluoro-5-(1-methyl piperid-4-yl)pyridin-2-yl)pyrazolidin-1-yl)methanone was synthesized according to the method of Example 217 using Compound 485.

LC-MS (ESI+): 503.2 m/z [M+H]⁺.

### Example 486: Preparation of (4-amino-1,7-dimethyl-1H-pyrazolo[4,3-c]quinolin-8-yl)(2-(3-fluoro-5-(thiazol-4-yl)pyr idin-2-yl)pyrazolidin-1-yl)methanone (486)

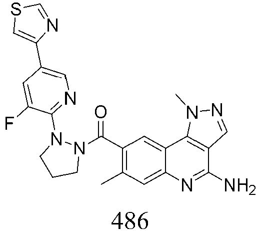

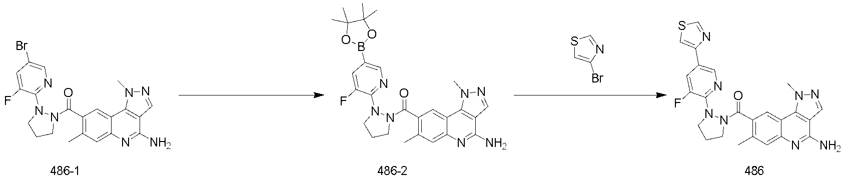

### Step 1: Preparation of (4-amino-1,7-dimethyl-1H-pyrazolo[4,3-c]quinolin-8-yl)(2-(3-fluoro-5-(4,4,5,5-tetrame thyl-1,3,2-dioxaborolan-2-yl)pyridin-2-yl)pyrazolidin-1-yl)methanone (486-2)

Compound 486-1 (synthesized according to the method in Example 360) (50 mg, 0.10 mmol), bis(pinacolato)diboron (52 mg, 0.21 mmol), potassium acetate (24 mg, 0.31 mmol) and [1,1'-bis(diphenylphosphino)ferrocene]palladium dichloride (7.5 mg, 0.01 mmol) were dissolved in dioxane (4 mL) at room temperature. The reaction was carried out at 100°C for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate, and filtered. The filtrate was concentrated under reduced pressure. The resulting residues were purified by silica gel column chromatography (eluent: dichloromethane/methanol=10/1) to obtain Compound 486-2 (50 mg, yield 91%).

### Step 2: Preparation of (4-amino-1,7-dimethyl-1H-pyrazolo[4,3-c]quinolin-8-yl)(2-(3-fluoro-5-(thiazol-4-yl)pyr idin-2-yl)pyrazolidin-1-yl)methanone (486)

Compound 486-2 (50 mg, 0.09 mmol), 4-bromothiazole (15 mg, 0.09 mmol), cesium carbonate (92 mg, 0.28 mmol) and [1,1'-bis(diphenylphosphino)ferrocene]palladium dichloride (6.0 mg, 0.01 mmol) were dissolved in dioxane (4 mL) and water (1 mL) at room temperature. The mixture was reacted at 100°C for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: dichloromethane/methanol=10/1) to obtain Compound 486 (5.00 mg, yield 10%).

LC-MS (ESI+): 489.1 m/z [M+H]⁺.

The following compounds were obtained according to the synthetic method in Example 486 using corresponding raw materials.

| Example | Structure | Mass spectrometry data (ESI+): m/z | Nuclear magnetic data |
|---|---|---|---|
| 487 | 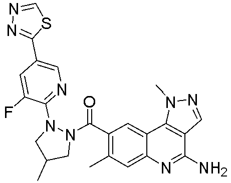 | 504.2 | ¹H NMR (400 MHz, DMSO-*d₆*) δ 9.63 (s, 1H), 8.73 (s, 1H), 8.18 (s, 1H), 7.96 (s, 2H), 7.32 (s, 1H), 7.04 (s, 2H), 4.13 (s, 2H), 4.00 (s, 2H), 3.50- 3.40 (m, 3H) 2.58 (d, *J* = 12.8 Hz, 1H), 2.44 (s, 3H), 1.07 (s, 3H). |
| 488 | 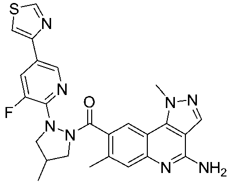 | 503.2 | ¹H NMR (400 MHz, DMSO-*d₆*) δ 9.22 (d, J = 1.6 Hz, 1H), 8.74 (s, 1H), 8.22 (s, 1H), 8.15 (s, 1H), 7.96 (d, J *=* 45.2 Hz, 2H), 7.32 (s, 1H), 7.01 (s, 2H), 4.12 - 3.96 (m, 3H), 3.88 (s, 2H), 3.28 (d, J = 10.0 Hz, 2H), 2.57 (d, J *=* 8.8 Hz, 1H), 2.42 (s, 3H), 1.07 (s, 3H). |

### Example 489: Preparation of (4-amino-1,7-dimethyl-1H-pyrazolo[4,3-c]quinolin-8-yl)(2-(5-cyclopropyl-3-fluoropyri din-2-yl)-4-methylpyrrolidin-1-yl)methanone (489)

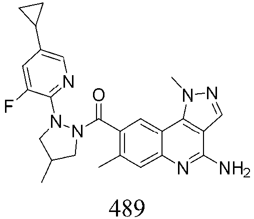

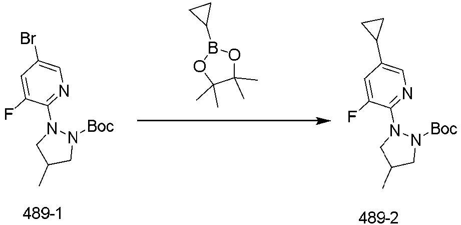

### Step 1: Preparation of tert-butyl 2-(5-cyclopropyl-3-fluoropyridin-2-yl)-4-methylpyrazolidine-1-carboxylate (489-2)

Compound 489-1 (synthesized according to the method in Example 360) (140 mg, 0.39 mmol), 2-cyclopropyl-4,4,5,5-tetramethyl-1,3,2-dioxaborolane (261 mg, 1.55 mmol), potassium carbonate (161 mg, 1.16 mmol) and [1,1'-bis(di-tert-butylphosphino)ferrocene]palladium dichloride (25 mg, 0.04 mmol) were added to dioxane (4 mL) and water (1 mL) at room temperature. The reaction was carried out at 100°C for 12 hours, and the resulting reaction mixture was diluted with water (50 mL). The resulting mixture was extracted with ethyl acetate (50 mL x 3). The organic phases were combined, washed with saturated brine (50 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 489-2 (100 mg, yield 80%).

LC-MS (ESI+): 322.2 m/z [M+H]⁺.

Remaining steps were according to the synthetic method of Example 360 to give (4-amino-1,7-dimethyl-1H-pyrazolo[4,3-c]quinolin-8-yl)(2-(5-cyclopropyl-3-fluoropyri din-2-yl)-4-methylpyrrolidin-1-yl)methanone, except that Compound 489-2 was used instead of Compound 360-4.

LC-MS (ESI+): 460.2 m/z [M+H]⁺.

¹H NMR (400 MHz, DMSO-*d₆*) δ 8.15 (s, 1H), 7.99 (s, 1H), 7.74 (s, 1H), 7.30 (s, 1H), 7.15 (s, 1H), 6.97 (s, 2H), 3.96 (s, 3H), 3.70 (s, 1H), 3.45 (s, 1H), 3.12 (s, 1H), 2.46 (s, 1H), 2.36 (s, 3H), 1.89 (s, 1H), 1.23 (s, 1H), 1.03 (s, 3H), 0.92 (d, *J =* 8.0 Hz, 2H), 0.61 (s, 2H).

The following compound was obtained according to the synthetic method in Example 489 using corresponding raw materials.

| Example | Structure | Mass spectrometry data (ESI+): m/z | Nuclear magnetic data |
|---|---|---|---|
| 490 | 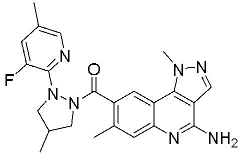 | 434.5 | ¹H NMR (400 MHz, DMSO-*d₆*) δ 8.25 - 8.13 (m, 1H), 7.99 (s, 1H), 7.82 (s, 1H), 7.32-7.38 (m, 2H), 7.00 (s, 2H), 4.00 (s, 3H), 3.47-3.75 (m,2H), 3.25-3.05 (m,2H), 2.50-2.30 (m,4H), 2.21 (s, 3H), 1.06 (s, 3H). |

### Example 491: Preparation of (4-amino-1,7-dimethyl-1H-pyrazolo[4,3-c]quinolin-8-yl)(2-(3-fluoro-5-(3-methoxy-3-m ethylbut-1-yn-1-yl)pyridin-2-yl)-4-methylpyrazolidin-1-yl)methanone (491)

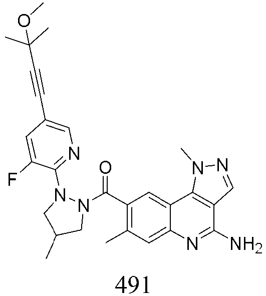

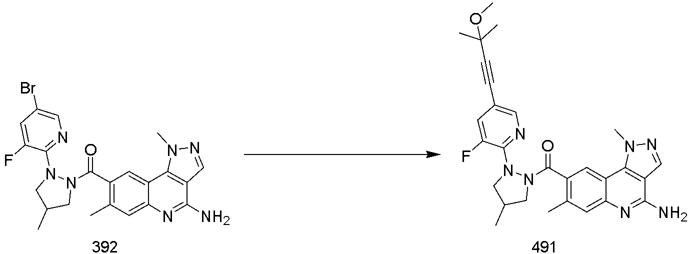

### Step 1 : Preparation of (4-amino-1,7-dimethyl-1H-pyrazolo[4,3-c]quinolin-8-yl)(2-(3-fluoro-5-(3-methoxy-3-m ethylbut-1-yn-1-yl)pyridin-2-yl)-4-methylpyrazolidin-1-yl)methanone (491)

Compound 392 (30.0 mg, 0.06 mmol) was dissolved in 1,4-dioxane (1 mL) in a sealing tube, followed by the addition of 3-methoxy-3-methylbut-1-yne (5.8 mg, 0.06 mmol), CuI (1.1 mg, 0.006 mmol), Pd(Phh₃)₂Cl₂ (4.20 mg, 0.006 mmol) and TEA (18.2 mg, 0.18 mmol) successively. The resulting mixed solution was stirred at 90°C for 3 hours. Ethyl acetate (20 mL) was added to the reaction solution, and the reaction solution was washed with saturated brine (5 mL × 3). The organic phase was dried over anhydrous sodium sulfate and filtered. The filtrate was concentrated under reduced pressure. The residues were purified by preparative high performance liquid chromatography (water/acetonitrile=95%/5% - 10%/90%), and subjected to lyophilization to obtain Compound 491 (5.4 mg, yield 17%, a white solid).

LC-MS (ESI+): 516.2 m/z [M+H]⁺.

The following compounds were obtained according to the synthetic method in Example 491 using corresponding raw materials.

| Example | Structure | Mass spectrometry data (ESI+): m/z | Nuclear magnetic data |
|---|---|---|---|
| 492 | 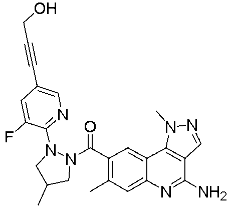 | 474.5 | |
| 493 | 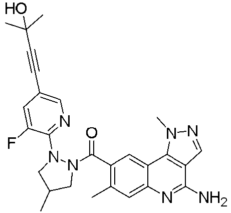 | 502.2 | |

### Example 494: Preparation of (4-amino-1,7-dimethyl-1H-pyrazolo[4,3-c]quinolin-8-yl)(2-(5-ethynyl-3-fluoropyridin-2-yl)-4-methylpyrazolidin-1-yl)methanone (494)

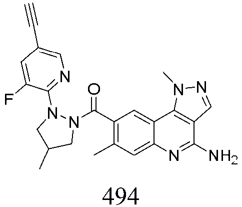

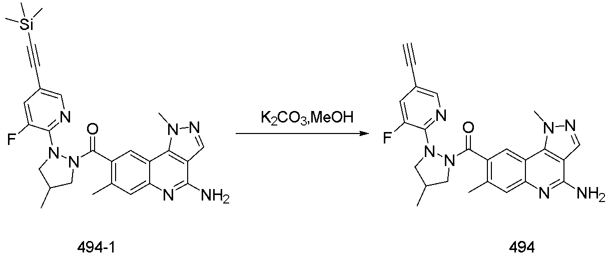

### Step 1: Preparation of (4-amino-1,7-dimethyl-1H-pyrazolo[4,3-c]quinolin-8-yl)(2-(5-ethynyl-3-fluoropyridin-2-yl)-4-methylpyrazolidin-1-yl)methanone (494)

(4-Amino-1,7-dimethyl-1H-pyrazolo[4,3-c]quinolin-8-yl)(2-(3-fluoro-5-((trimethyl silyl)ethynyl)pyridin-2-yl)-4-methylpyrazolidin-1-yl)methanone (494-1, synthesized according to the method in Example 491) (60 mg, 0.12 mmol) was dissolved in methanol (2 mL), and potassium carbonate (17 mg, 0.12 mmol) was added thereto. The resulting mixture was stirred at room temperature for 1.5 hours. After the reaction was completed, EtOAc (20 mL) was added to the reaction solution, and the reaction solution was washed with saturated brine (5 mL × 3). The organic phase was dried over anhydrous sodium sulfate and filtered, and the filtrate was concentrated under reduced pressure. The residues were purified by preparative high performance liquid chromatography (water/acetonitrile=95%/5% - 10%/90%), and subjected to lyophilization to obtain Compound 494 (2.2 mg, yield 4%, a white solid).

LC-MS (ESI+): 444.2 m/z [M+H]⁺.

### Example 495: Preparation of (4-amino-1,7-dimethyl-1H-pyrazolo[4,3-c]quinolin-8-yl)(2-(3-fluoro-5-(3-fluoroprop-1-yn-1-yl)pyridin-2-yl)-4-methylpyrazolidin-1-yl)methanone (495)

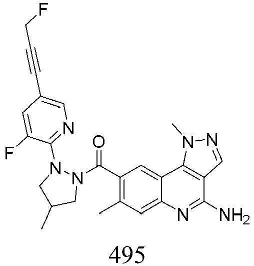

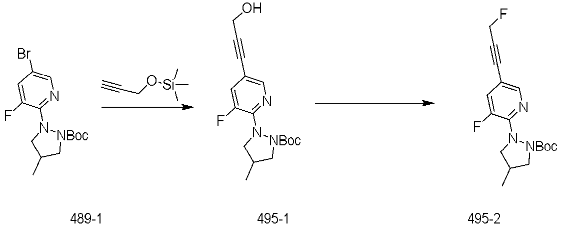

### Step 1: Preparation of tert-butyl 2-(3-fluoro-5-(3-hydroxypropyl-1-yl)pyridin-2-yl)-4-methylpyrazoline-1-carboxylate (495-1)

Compound 389-1 (100 mg, 0.555 mmol) was dissolved in DMF (5 mL), followed by the addition of trimethyl(prop-2-yn-1-yloxy)silane (107 mg, 0.83 mmol), Pd (PPh₃)₂Cl₂ (39 mg, 0.055 mmol), CuI (10 mg, 0.055 mmol) and TEA (168 mg, 1.66 mmol) successively at room temperature. The mixture was reacted under stirring at 100°C overnight. After the reaction was completed, the reaction mixture was concentrated under reduced pressure, and the residues were purified by column chromatography (DCM:MeOH=10:1) to obtain Compound 495-1.

LC-MS (ESI+): 336.4 m/z [M+H]⁺.

### Step 2: Preparation of tert-butyl 2-(3-fluoro-5-(3-fluoroprop-1-yn-1-yl)pyridin-2-yl)-4-methylpyrazoline-1-carboxylate (495-2)

Compound 495-1 (75 mg, 0.224 mmol) was dissolved in DCM (5 mL), followed by the dropwise addition of a solution of DAST (90 mg, 0.559 mmol) in DCM (2 mL) slowly at 0°C. The mixture was reacted under stirring at 0°C for 15 minutes. After the reaction was completed, the reaction mixture was quenched with an appropriate amount of sodium bicarbonate saturated aqueous solution, and extracted with DCM (3x10 mL). The organic phases were collected, washed with saturated brine (20 mL), dried over anhydrous sodium sulfate and filtered. The filtrate was concentrated under reduced pressure to obtain a crude product (495-2), which was directly used for the next step.

LC-MS (ESI+): 338.3 m/z [M+H]⁺.

Remaining steps were according to the synthetic method of Example 360 to give (4-amino-1,7-dimethyl-1H-pyrazolo[4,3-c]quinolin-8-yl)(2-(3-fluoro-5-(3-fluoroprop-1 -yn-1-yl)pyridin-2-yl)-4-methylpyrazolidin-1-yl)methanone, except that Compound 495-2 was used instead of Compound 360-4.

LC-MS (ESI+): 476.6 m/z [M+H]⁺.

### Example 496: Preparation of (4-amino-7-ethyl-1-methyl-1H-pyrazolo[4,3-c]quinolin-8-yl)(2-(5-(trifluoromethyl)pyri din-2-yl)pyrazolidin-1-yl)methanone (496)

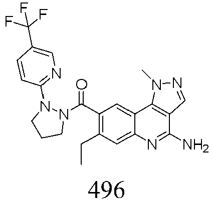

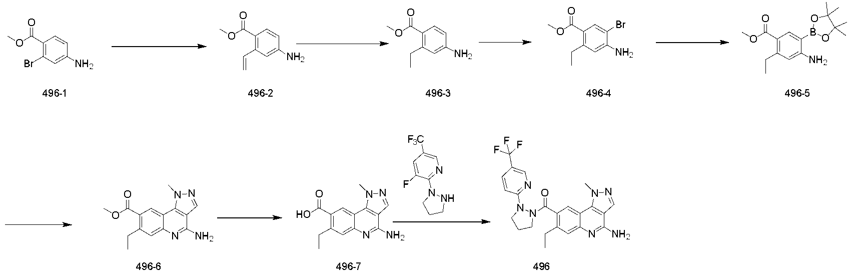

### Step 1: Preparation of methyl 4-amino-2-vinylbenzoate (496-2)

Methyl 4-amino-2-bromobenzoate (496-1, 5 g, 21.7 mmol) was dissolved in 50 mL of dioxane at room temperature, followed by the addition of potassium vinyltrifluoroborate (3.17 g, 43.5 mmol), potassium carbonate (9.0 g, 65.2 mmol), 1,1'-bis(diphenylphosphino)ferrocene]palladium dichloridedichloromethane complex (1.59 g, 2.17 mmol) and 10mL of water. The reaction was carried out at 100°C overnight under nitrogen atmosphere. The reaction mixture was filtered through Celite, and then extracted with ethyl acetate (100 mLx 2). The organic phases were combined, dried over anhydrous sodium sulfate and filtered. The filtrate was concentrated to dryness under reduced pressure, and the resulting residues were purified by silica gel column chromatography (petroleum ether/ethyl acetate=100%-50%) to obtain Compound 496-2 (4.5 g, yield 96%).

LC-MS (ESI+): 178.2 m/z [M+H]⁺.

### Step 2: Preparation of methyl 4-amino-2-ethylbenzoate (496-3)

Compound 496-2 (2 g, 11.3 mmol) was dissolved in 30 mL of methanol at room temperature, followed by the addition of 10% palladium on carbon (200 mg), and reacted at room temperature overnight under hydrogen atmosphere. The reaction mixture was filtered through Celite, and concentrated under reduced pressure to obtain Compound 496-3 (1.9 g, yield 96%).

LC-MS (ESI+): 180.2 m/z [M+H]⁺.

### Step 3: Preparation of methyl 4-amino-5-bromo-2-ethylbenzoate (496-4)

Compound 496-3 (500 mg, 2.79 mmol) was dissolved in 10 mL of dichloromethane at room temperature, followed by the addition of N-bromosuccinimide (779 mg, 3.07 mmol), and reacted for 1 hour in an ice bath under nitrogen atmosphere. The reaction mixture was directly concentrated, and the resulting residues were purified by silica gel column chromatography (eluent: petroleum ether/ethyl acetate=100%-50%) to obtain Compound 496-4 (350 mg, yield 49%).

LC-MS (ESI+): 258.2 m/z [M+H]⁺.

### Step 4: Preparation of methyl 4-amino-2-ethyl-5-(4,4,5,5-tetramethyl-1,3,2-dioxaborocyclopentan-2-yl)benzoate (496-5)

Compound 496-4 (2 g, 7.43 mmol) was dissolved in 20 mL of dioxane at room temperature, followed by the addition of bis(pinacolato)diboron (2.95 g, 11.6 mmol), potassium acetate (2.28 g, 23.2 mmol) and 1,1'-bis(diphenylphosphino)ferrocene]palladium dichloridedichloromethane complex (566 mg, 0.77 mmol). The reaction was carried out at 90°C overnight under nitrogen atmosphere. The reaction mixture was filtered through Celite and concentrated, and the resulting residues were purified by silica gel column chromatography (eluent: petroleum ether/ethyl acetate=100%-50%) to obtain Compound 496-5 (1.9 g, yield 83%).

LC-MS (ESI+): 306.2 m/z [M+H]⁺.

### Step 5: Preparation of methyl 4-amino-7-ethyl-1-methyl-1H-pyrazolo[4,3-c]quinoline-8-carboxylate (496-6)

Compound 496-5 (1 g, 3.28 mmol) was dissolved in 10 mL of dioxane at room temperature, followed by the addition of 5-bromo-1-methyl-1H-pyrazole-4-carbonitrile (830 mg, 4.92 mmol), potassium carbonate (1.36 g, 9.83 mmol), 1,1'-bis(diphenylphosphino)ferrocene]palladium dichloridedichloromethane complex (239 mg, 0.33 mmol) and water (1mL). The reaction was carried out at 100°C overnight under nitrogen atmosphere. The reaction mixture was filtered through Celite, and concentrated, and the resulting residues were purified by silica gel column chromatography (eluent: dichloromethane/methanol =0-10%) to obtain Compound 496-6 (300 mg, yield 32%).

LC-MS (ESI+): 285.2 m/z [M+H]⁺.

### Step 6: Preparation of 4-amino-7-ethyl-1-methyl-1H-pyrazolo[4,3-c]quinoline-8-carboxylic acid (496-7)

Compound 496-6 (200 mg, 0.70 mmol) was dissolved in 5 mL of methanol at room temperature, followed by the addition of 5% aqueous lithium hydroxide solution (2 mL). The reaction was carried out at 50°C overnight under nitrogen atmosphere. The reaction mixture was adjusted to neutral with 1N hydrochloric acid, and directly concentrated to obtain Compound 496-7 (200 mg) as a crude product.

LC-MS (ESI+): 271.2 m/z [M+H]⁺.

### Step 7: Preparation of (4-amino-7-ethyl-1-methyl-1H-pyrazolo[4,3-c]quinolin-8-yl)(2-(5-(trifluoromethyl)pyri din-2-yl)pyrazolidin-1-yl)methanone (496)

Compound 496-7 (100 mg, 0.37 mmol) was dissolved in 3 mL of DMA at room temperature, followed by the addition of 3-trifluoromethyl-2-(pyrazolidin-1-yl)pyridine (synthesized according to the method in Example 328) (62 mg, 0.37 mmol), N,N-diisopropylethylamine (143 mg, 1.11 mmol) and bromo-tris-pyrrolidino-phosphonium hexafluorophosphate (259 mg, 0.56 mmol). The reaction was carried out at room temperature overnight under nitrogen atmosphere. The reaction solution was filtered, concentrated, and purified by preparative high performance liquid chromatography (water/acetonitrile=95%/5% - 10%/90%) to obtain Compound 496 (12 mg, yield 6.9%).

LC-MS (ESI+): 470.2 m/z [M+H]⁺.

The following compounds were obtained according to the synthetic method in Example 496 using corresponding raw materials.

| Example | Structure | Mass spectrometry data (ESI+): m/z | Nuclear magnetic data |
|---|---|---|---|
| 497 | 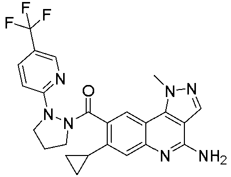 | 482.2 | |
| 498 | 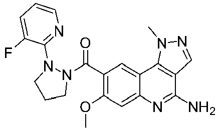 | 422.2 | |
| 499 | 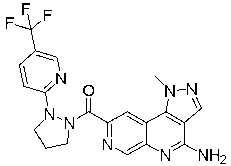 | 443.2 | |
| 500 | 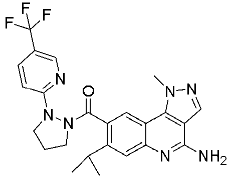 | 484.6 | |
| 501 | 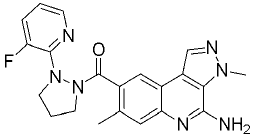 | 406.6 | ¹H NMR (400 MHz, DMSO-*d₆*) δ 8.25 (s, 1H), 8.05 (s, 1H), 7.64 (s, 1H), 7.46 (s, 1H), 7.28 (s, 1H), 7.08 (s, 1H), 6.73 (s, 2H), 4.31 (s, 3H), 3.85 (s, 4H), 2.38 - 2.32 (m, 3H), 2.09 (s, 2H). |
| 502 | 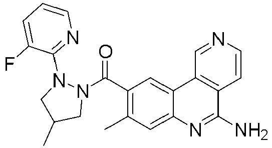 | 417.5 | |
| 503 | 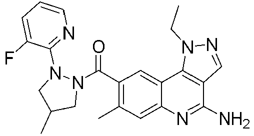 | 434.5 | |
| 504 | 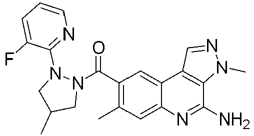 | 420.5 | |
| 505 | 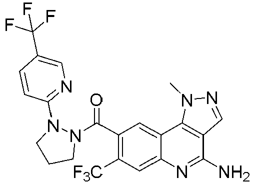 | 510.2 | |
| 506 | 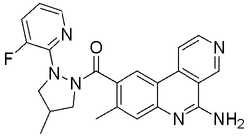 | 417.1 | ¹H NMR (400 MHz, DMSO-*d₆*) δ 9.57 (s, 1H), 8.77 (s, 1H), 8.21 (s, 2H), 8.06 (d, *J* = 22.8 Hz, 1H), 7.39 (s, 3H), 7.27-7.23 (m, 1H), 7.04 (s, 1H), 3.82 (s, 2H), 3.30 - 3.26 (m, 2H), 3.08 (s, 1H), 2.41 (s, 3H), 1.09 (d, *J =* 6.8 Hz, 3H). |

### Example 507: Preparation of (4-amino-7-methyl-1-(methyl-D3)-1H-pyrazolo[4,3-c]quinolin-8-yl)(2-(3-fluoropyridin -2-yl)-4-methylpyrazolidin-1-yl)methanone (507)

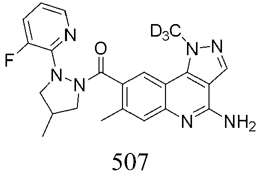

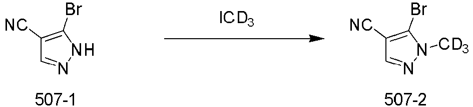

### Step 1: Preparation of 5-bromo-1-(deuterated methyl)-1H-pyrazole-4-carbonitrile (507-2)

5-Bromo-1H-pyrazole-4-carbonitrile (507-1, 500 mg, 2.91 mmol), deuterated iodomethane (632 mg, 4.36 mmol) and cesium carbonate (2.83 g, 8.72 mmol) were dissolved in N,N-dimethyldioxane (10 mL) at room temperature. The reaction was carried out at room temperature for 12 hours, and the resulting reaction mixture was diluted with water (100 mL). The resulting mixture was extracted with ethyl acetate (100 mL x 3). The organic phases were combined, washed with saturated brine (150 mL), dried over anhydrous sodium sulfate and filtered. The filtrate was concentrated under reduced pressure. The resulting residues were purified by silica gel column chromatography (eluent: petroleum ether/ethyl acetate=1/1) to obtain Compound 507-2 (500 mg, a mixture of isomers, yield 90%).

LC-MS (ESI+): 189.0 m/z [M+H]⁺.

Remaining steps were according to the synthetic method of Example 496 to give 4-amino-7-methyl-1-(methyl-d3)-1H-pyrazolo[4,3-c]quinolin-8-yl)(2-(3-fluoropyridin-2 -yl)-4-methylpyrazolidin-1-yl)methanone, except that Compound 507-2 was used instead of compound 5-bromo-1-methyl-1H-pyrazole-4-carbonitrile, and methyl 4-amino-2-methylbenzoate was used instead of compound 496-3.

LC-MS (ESI+): 423.2 m/z [M+H]⁺.

¹H NMR (400 MHz, DMSO-*d₆*) δ 8.34 (s, 1H), 8.07 (s, 1H), 7.65-7.78 (m, 1H), 7.32-7.43 (m, 1H), 6.88-7.10 (m, 4H), 3.82 (s, 2H), 3.08 (s, 2H), 2.47 (s, 3H), 2.32 (s, 1H), 1.04 (s, 3H).

The following compound was obtained according to the synthetic method in Example 507 using corresponding raw materials.

| Example | Structure | Mass spectrometry data (ESI+): m/z | Nuclear magnetic data |
|---|---|---|---|
| 508 | 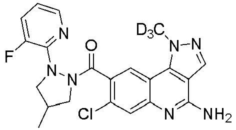 | 443.0 | |

### Example 509: Preparation of (4-amino-1,3,7-trimethyl-1H-pyrazolo[4,3-c]quinolin-8-yl)(2-(3-fluoropyridin-2-yl)-4-methylpyrazolidin-1-yl) methanone (509)

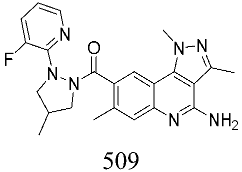

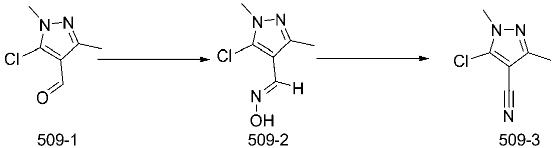

### Step 1: Preparation of (E)-5-chloro-1,3-dimethyl-1H-pyrazole-4-formaldoxime (509-2)

5-Chloro-1,3-dimethyl-1H-pyrazole-4-carbaldehyde (509-1) (2.0 g, 13 mmol) was dissolved in methanol (20 mL), and hydroxylamine hydrochloride (1.7 g, 25 mmol) was added thereto. The resulting mixture was stirred at 70°C for 3 hours. After the reaction was completed, the reaction solution was cooled, and filtered to obtain Compound 509-2 (2 g, yield 91%, a white solid).

LC-MS (ESI+): 174.2 m/z [M+H]⁺.

### Step 2: Preparation of 5-chloro-1,3-dimethyl-1H-pyrazole-4-carbonitrile (509-3)

Compound 509-2 (300 mg, 1.73 mmol) was dissolved in DCM (4 mL), and then thionyl chloride (1 mL) was added dropwise thereto at 0°C. The resulting mixture was stirred at 40°C for 2 hours. After the reaction was completed, the reaction solution was concentrated under reduced pressure. The residues were purified by silica gel column chromatography (petroleum ether/ethyl acetate = 4/1) to obtain Compound 509-3 (230 mg, yield 85%, a white solid).

LC-MS (ESI+): 156.2 m/z [M+H]⁺.

Remaining steps were according to the synthetic method of Example 496 to give (4-amino-1,3,7-trimethyl-1H-pyrazolo[4,3-c]quinolin-8-yl)(2-(3-fluoropyridin-2-yl)-4-methylpyrazolidin-1-yl)methanone, except that Compound 509-3 was used instead of compound 5-bromo-1-methyl-1H-pyrazole-4-carbonitrile, and methyl 4-amino-2-methylbenzoate was used instead of 496-3.

LC-MS (ESI+): 434.2 m/z [M+H]⁺.

¹H NMR (400 MHz, DMSO-*d₆*) δ 8.14 (d, *J =* 4.4 Hz, 1H), 7.80 (s, 1H), 7.49 (s, 1H), 7.30 (s, 1H), 7.14 - 6.92 (m, 1H), 6.47 (s, 2H), 4.39 - 3.88 (m, 4H), 3.87 - 3.67 (m, 1H), 3.56 - 3.40 (m, 1H), 3.30 - 2.96 (m, 2H), 2.56 (s, 3H), 2.39 (s, 3H), 1.06 (s, 3H).

### Example 510: (4-Amino-3,7-dimethyl-1,3-dihydrofuro[3,4-c]quinolin-8-yl)(2-(3-fluoropyridin-2-yl)-4 -methylpyrazolin-1-yl)methanone (510)

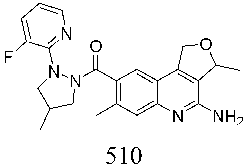

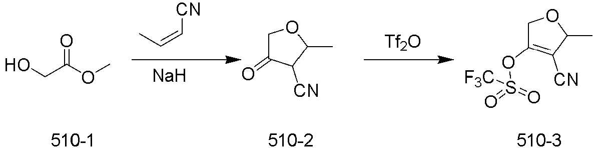

### Step 1: Preparation of 2-methyl-4-oxotetrahydrofuran-3-carbonitrile (510-2)

Methyl 2-hydroxyacetate (510-1, 4.0 g, 59.6 mmol) was dissolved in THF (20 mL), followed by the addition of sodium hydride (0.715 g, 29.8 mmol) at 0°C. The mixture was stirred for 0.5 hour, and then heated to 65°C, followed by the addition of (Z)-but-2-enenitrile (5.37 g, 59.6 mmol). The resulting mixture was reacted at 65°C for 2 hours. Water (100 mL) was added to the reaction solution, and the reaction solution was extracted with DCM (100 mL × 3). The organic phases were combined, washed with saturated brine, dried over anhydrous sodium sulfate and filtered, and the filtrate was concentrated under reduced pressure. The residues were purified by silica gel column chromatography (petroleum ether/ethyl acetate = 10/1) to obtain Compound 510-2 (0.9 g, yield 12%, a brown solid).

### Step 2: Preparation of 4-cyano-5-methyl-2,5-dihydrofuran-3-yltrifluoromethanesulfonate (510-3)

Compound 510-2 (0.9 g, 7.2 mmol) was dissolved in DCM (10 mL), followed by the addition of DIEA (1.85 g, 14.4 mmol) and trifluoroformic anhydride (2.84 g, 10.1 mmol) at -78°C, and the resulting mixture was stirred at -78°C for 15 minutes. Water (20 mL) was added to the reaction solution, and the reaction solution was extracted with DCM (20 mL × 3). The organic phases were combined, washed with saturated brine, dried over anhydrous sodium sulfate and filtered, and the filtrate was concentrated under reduced pressure to obtain Compound 510-3 (2000 mg, yield 99%, a brown solid). The compound was directly used for the next step without purification.

Remaining steps were according to the synthetic method of Example 496 to give (4-amino-3,7-dimethyl-1,3-dihydrofuro[3,4-c]quinolin-8-yl)(2-(3-fluoropyridin-2-yl)-4-methylpyrazolin-1-yl)methanone, except that Compound 510-3 was used instead of 5-bromo-1-methyl-1H-pyrazole-4-carbonitrile, and methyl 4-amino-2-methylbenzoate was used instead of compound 496-3.

LC-MS (ESI+): 422.2 m/z [M+H]⁺.

The following compounds were obtained according to the synthetic method in Example 510 using corresponding raw materials.

| Example | Structure | Mass spectrometry data (ESI+): m/z | Nuclear magnetic data |
|---|---|---|---|
| 511 | 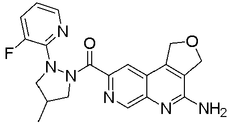 | 395.1 | |
| 512 | 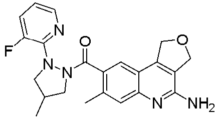 | 408.2 | |

### BIOLOGICAL TEST

### Test Example 1: Assay of enzymatic inhibitory activity of the compounds against PRMT5-MTA

The AlphaScreen method was used to detect the enzymatic inhibitory activity of the compounds against PRMT5-MTA. 1 x Methyltransferase assay buffer IV (signalchem) was prepared as a solution for diluting each component for the enzymatic reaction. PRMT5/MET50 protein of a final concentration of 1 nM, 5'-deoxy-5'-adenosine (sigma) of a final concentration of 1 uM and S-(5'-adenosine)-L-methionine chloride dihydrochloride (sigma) of a final concentration of 2.5 uM were mixed, then incubated for 30 minutes at room temperature, and transferred to an OptiPlate-384 well plate (PerkinElmer) at a volume of 6 uL per well, for which no PRMT5/MET50 protein was added to the control wells. The test compound was 3-fold serially diluted into 10 concentrations, starting from a concentration of 1000 nM, and the series of diluted compounds were added to all wells except the control wells in the well plate using Tecan D300e. The well plate was subjected to centrifugation for 1 minute at 1000 rpm in a centrifuge, and then incubated for 15 minutes at 23°C. Histone H4 substrate of a final concentration of 5 nM was added to the well plate at 4 uL per well, centrifuged for 1 minute at 1000 rpm in a centrifuge, then placed in an incubator at 23°C and reacted for 45 minutes. Anti-histone H4 (symmetrical dimethyl R3) antibody (Abcam) was incubated with 50 ug/mL of AlphaScreen Protein A Acceptor beads (PerkinElmer) for 10 minutes at room temperature beforehand, and then added to the well plate at a volume of 10 uL per well. The mixture was reacted for 1 hour at room temperature. 50 Ug/mL of AlphaScreen Streptavidin Donor beads (PerkinElmer) was added at 10 uL per well, and reacted for 1 hour at room temperature. The signal values were detected for the well plate through the Alpha method using an Envision multifunctional microplate-reader, and the IC₅₀ values for inhibition of the compounds on PRMT5/MET50 enzyme activity were calculated in Xlfit 5.0 software.

**Table 1. IC₅₀ values for inhibition of the compounds on PRMT5+MTA enzyme activity**

| Compound No. | IC₅₀ (nM) | Compound No. | IC₅₀ (nM) |
|---|---|---|---|
| 1 | 1.0 | 40 | 1.7 |
| 2 | 2.1 | 41 | 3.9 |
| 3 | 2.6 | 42 | 3.0 |
| 4 | 2.0 | 43 | 3.8 |
| 5 | 1.5 | 44 | 1.9 |
| 6 | 1.5 | 45 | 3.1 |
| | | 46 | LA |
| 9 | 3.2 | 47 | 3.2 |
| | | 48 | 3.7 |
| 11 | 1.0 | 49 | 2.2 |
| 12 | 7.8 | 50 | 2.1 |
| 13 | 4.0 | 51 | 1.7 |
| 14 | 2.0 | 52 | 3.7 |
| 15 | 3.7 | 53 | 1.5 |
| 16 | 2.7 | 54 | 15.8 |
| 17 | 7.0 | 55 | 4.6 |
| | | 56 | 3.5 |
| | | 57 | 1.9 |
| 21 | 1.9 | 58 | 4.0 |
| 22 | 4.3 | 59 | 2.0 |
| | | 60 | 2.3 |
| | | 61 | 2.4 |
| 25 | 2.0 | 62 | 5.1 |
| 26 | 2.4 | 63 | 3.2 |
| 27 | 4.0 | 64 | 1.7 |
| | | 65 | 4.0 |
| 29 | 1.6 | 66 | 3.9 |
| 30 | 1.9 | 67 | 1.5 |
| 31 | 3.5 | 69 | 2.4 |
| 32 | 2.5 | 71 | 3.3 |
| 33 | 3.3 | 72 | 2.7 |
| 34 | 1.5 | 73 | 2.5 |
| | | | |
| 37 | 3.5 | 75 | 3.1 |
| 39 | 2.7 | | |
| 76 | 1.6 | 112 | 1.9 |
| 77 | 1.1 | | |
| 78 | 2.0 | 114 | 4.0 |
| 79 | 9.7 | 115 | 2.0 |
| 80 | 1.9 | 116 | 4.7 |
| 81 | 5.8 | 117 | 1.8 |
| 82 | 2.2 | 118 | 2.3 |
| 83 | 4.5 | 119 | 2.3 |
| 84 | 3.4 | 120 | 2.5 |
| | | 121 | LA |
| | | 123 | 2.9 |
| 88 | 2.5 | 124 | 5.4 |
| 89 | 2.0 | 125 | 1.7 |
| 90 | 4.5 | 126 | 1.3 |
| 91 | 2.9 | 127 | 4.9 |
| 92 | 2.1 | | |
| 93 | 1.4 | | |
| 94 | 2.3 | | |
| 95 | 2.1 | | |
| 96 | 2.6 | 133 | 3.6 |
| 97 | 5.1 | 134 | 2.3 |
| 98 | 17.2 | 135 | 3.2 |
| 99 | 7.0 | 136 | 3.6 |
| | | 137 | 1.3 |
| | | 139 | 3.0 |
| 102 | 1.7 | 140 | 1.6 |
| 103 | 2.9 | 141 | 2.3 |
| 104 | 3.4 | 142 | 4.1 |
| 106 | 1.9 | 144 | 1.5 |
| 107 | 2.5 | 145 | 5.7 |
| 108 | 1.9 | 147 | 2.2 |
| 109 | 1.2 | 148 | 1.0 |
| 110 | 2.5 | 149 | 2.9 |
| 111 | 1.7 | 151 | 6.4 |
| 152 | 4.4 | 188 | 2.7 |
| 153 | 1.6 | 189 | 1.7 |
| 154 | 1.6 | 190 | 0.8 |
| 155 | 2.3 | 191 | 2.5 |
| 156 | 1.8 | 192 | 2.1 |
| 157 | 6.9 | 193 | 2.7 |
| 158 | 4.5 | 196 | 5.7 |
| 159 | 3.3 | 197 | 1.1 |
| 160 | 2.0 | 198 | 2.9 |
| 161 | 1.7 | 199 | 2.5 |
| 162 | 5.4 | 200 | 1.7 |
| 163 | 1.4 | 201 | 2.1 |
| 164 | 0.9 | 202 | 2.9 |
| 165 | 3.3 | 203 | 1.8 |
| 166 | 4.8 | 211 | 2.3 |
| 167 | 1.3 | 215 | 5.3 |
| 168 | 2.6 | 216 | 3.3 |
| 169 | 2.5 | 217 | 2.9 |
| 171 | 5.2 | 218 | 7.0 |
| 172 | 2.1 | 220 | 1.2 |
| 173 | 1.9 | 221 | 2.3 |
| 174 | 2.3 | 222 | 1.4 |
| 175 | 3.3 | 224 | 16 |
| 176 | 2.0 | 225 | 16 |
| 177 | 1.1 | 226 | 5.0 |
| 178 | 1.6 | 229 | 3.7 |
| 179 | 3.4 | 232 | 4.3 |
| 180 | 3.2 | 233 | 4.4 |
| 181 | 0.69 | 235 | 3.8 |
| 183 | 3.9 | 236 | 6.0 |
| 184 | 7.4 | 237 | 8.0 |
| 185 | 4.7 | 238 | 5.7 |
| 186 | 5.6 | 239 | 7.3 |
| 187 | 2.3 | 240 | 3.9 |
| 241 | 7.3 | 286 | 2.7 |
| 242 | 5.9 | 287 | 2.2 |
| 243 | 3.4 | 289 | 0.9 |
| 244 | 4.2 | 290 | 3.7 |
| 245 | 9.5 | 291 | 3.9 |
| 247 | 7.7 | 292 | 1.4 |
| 249 | 40 | 294 | 1.9 |
| 250 | 3.7 | 295 | 2.0 |
| 251 | 4.1 | 296 | 1.3 |
| 252 | 4.8 | 297 | 6.4 |
| 253 | 6.1 | | |
| 262 | 8.6 | | |
| 264 | 1.0 | | |
| 265 | 2.0 | 303 | 3.0 |
| 266 | 2.6 | 304 | 3.9 |
| 267 | 1.8 | 305 | 2.3 |
| 268 | 3.5 | 306 | 4.9 |
| 269 | 5.7 | 308 | 1.1 |
| 270 | 4.9 | 310 | 1.8 |
| 271 | 2.7 | 311 | 3.0 |
| 272 | 2.6 | 313 | 4.2 |
| 273 | 2.8 | 314 | 4.7 |
| 274 | 3.7 | 316 | 6.8 |
| 275 | 2.9 | 317 | 6.4 |
| 276 | 1.6 | 318 | 6.3 |
| 277 | 2.2 | 319 | 4.7 |
| 278 | 1.5 | 320 | 5.5 |
| 279 | 4.3 | 322 | 4.4 |
| 280 | 4.0 | 323 | 7.1 |
| 282 | 4.5 | 324 | 4.4 |
| 283 | 1.7 | 325 | 2.3 |
| 284 | 2.3 | 326 | 2.0 |
| 285 | 2.0 | 327 | 8.4 |
| 328 | 6.5 | 360 | 5.2 |
| 329 | 3.4 | | |
| 330 | 13 | 363 | 3.1 |
| 331 | 2.1 | 364 | 4.8 |
| 332 | 6.0 | 365 | 3.2 |
| 333 | 7.7 | 366 | 7.9 |
| 334 | 9.2 | 367 | 4.1 |
| 335 | 3.3 | 368 | 4.7 |
| 336 | 2.9 | 369 | 3.9 |
| 337 | 59 | 370 | 5.7 |
| 338 | 2.8 | 371 | 5.5 |
| 339 | 4.6 | 372 | 3.4 |
| 340 | 4.2 | 373 | 7.5 |
| 341 | 4.7 | 374 | 8.1 |
| 342 | 3.8 | 375 | 2.5 |
| 343 | 3.9 | 377 | 3.1 |
| 344 | 4.7 | 378 | 6.6 |
| 345 | 4.4 | 379 | 7.8 |
| 346 | 3.6 | 380 | 3.4 |
| 347 | 7.1 | 381 | 4.0 |
| 348 | 7.5 | 382 | 4.8 |
| 349 | 6.1 | 383 | 6.0 |
| 350 | 3.5 | 384 | 4.1 |
| 351 | 4.9 | 385 | 5.4 |
| 352 | 12.5 | 386 | 6.2 |
| 353 | 6.3 | 387 | 3.7 |
| 354 | 3.8 | 389 | 6.3 |
| 356 | 3.7 | 390 | 8.6 |
| 357 | 3.4 | 391 | 7.4 |
| 358 | 6.7 | 392 | 4.0 |
| 359 | 2.7 | 393 | 3.4 |
| 395 | 4.9 | 432 | 4.5 |
| 396 | 5.2 | 433 | 3.8 |
| 397 | 15 | 434 | 6.1 |
| 398 | 3.9 | 435 | 7.3 |
| 399 | 6.2 | 436 | 4.8 |
| 400 | 5.8 | 437 | 4.7 |
| 401 | 3.5 | 439 | 5.5 |
| 402 | 6.4 | 440 | 3.7 |
| 403 | 3.8 | 441 | 7.2 |
| 404 | 6.6 | 442 | 4.6 |
| 405 | 12.3 | 443 | 5.0 |
| 406 | 4.3 | 444 | 3.7 |
| 408 | 4.8 | 445 | 6.4 |
| 409 | 3.1 | 447 | 6.8 |
| 410 | 5.2 | 448 | 5.1 |
| 411 | 6.3 | 449 | 5.2 |
| 412 | 6.2 | 450 | 3.8 |
| 413 | 4.3 | 451 | 7.0 |
| 414 | 6.2 | 452 | 7.3 |
| 415 | 2.6 | 453 | 5.2 |
| 416 | 13 | 454 | 5.9 |
| 417 | 3.8 | 455 | 10.1 |
| | | 456 | 7.0 |
| 419 | 4.6 | 457 | 7.5 |
| 421 | 3.3 | 458 | 5.1 |
| 422 | 9.3 | 459 | 4.7 |
| 423 | 6.7 | 460 | 8.2 |
| 424 | 7.3 | 461 | 6.8 |
| 425 | 8.3 | 462 | 6.0 |
| 427 | 6.2 | 463 | 1.3 |
| 429 | 6.7 | 464 | 3.1 |
| 430 | 5.5 | 465 | 3.4 |
| 431 | 5.4 | 466 | 4.4 |
| 468 | 3.2 | 494 | 4.6 |
| 472 | 4.6 | 495 | 15 |
| 473 | 9.0 | 496 | 3.4 |
| 474 | 7.0 | 497 | 5.1 |
| 475 | 5.1 | 498 | 5.0 |
| 476 | 5.6 | | |
| 477 | 4.7 | 500 | 41 |
| 478 | 19 | 501 | 4.4 |
| 479 | 5.3 | 502 | 5.2 |
| 481 | 3.7 | 503 | 5.2 |
| 484 | 6.4 | 504 | 8.6 |
| 486 | 6.8 | 505 | 3.0 |
| 487 | 6.7 | 506 | 5.8 |
| 488 | 5.3 | 507 | 10.3 |
| 489 | 4.8 | 508 | 16.6 |
| 490 | 5.1 | 509 | 4.3 |
| 491 | 8.6 | 510 | 4.8 |
| 492 | 3.3 | 511 | 6.1 |
| 493 | 5.1 | 512 | 5.0 |

Conclusion: as shown in the table above, the compounds of the present invention have exhibited good inhibitory activity against PRMT5 enzyme activity. For example, the measured IC₅₀ values for enzyme inhibition are less than 10 nM.

### Test Example 2: Assay for inhibitory activity of the compounds against cell proliferation

HCT116-WT (ATCC, CCL-247) and HCT116 MTAP KO (Nanjing Cobioer, CBP75002) cells were seeded into white low-transparency sterile 96-well cell culture plates at a density of 250 cells per well respectively, and the culture medium was McCoy's 5A Medium+10% FBS. The plates were placed under the condition of 37°C and 5% CO₂ to be cultured overnight. The cells were treated with 4-fold serially diluted compounds of 8 concentration points starting at an initial concentration of 10000 nM, and the last two columns were used as control wells to which only DMSO was added. The drug-treated cells were further cultured for 6 days. On day 7, CellTiter Glo^{®} reagent was added at 50 uL per well, and after reaction under shaking for 10 minutes at room temperature, the chemiluminescence signals were detected for the well plates through the chemiluminescence detection method using a BIOTEK H1 multifunctional microplate-reader. The IC₅₀ values for inhibition of the compounds on cell viability were calculated in X1fit 5.0 software.

**Table 2. IC₅₀ values for inhibition of the compounds on HCT116 MTAP KO/WT cell growth**

| Compound No. | HCT116 MTAP KO IC₅₀ (nM) | HCT116-WT IC₅₀ (nM) | Selectivity |
|---|---|---|---|
| | | | HCT 116-WT/ HCT116 MTAP KO |
| 1 | 32 | 623 | 19.47 |
| 2 | 30 | 820 | 27.33 |
| 3 | 27 | 568 | 21.04 |
| 5 | 42 | 852 | 20.29 |
| 6 | 95 | 1382 | 14.55 |
| 15 | 26 | 475 | 18.27 |
| 16 | 5.6 | 81 | 14.46 |
| 21 | 85 | 2340 | 27.53 |
| 22 | 129 | 2294 | 17.78 |
| 25 | 110 | 2820 | 25.64 |
| 26 | 444 | 9203 | 20.73 |
| 28 | 139 | 2532 | 18.22 |
| 32 | 122 | 3642 | 29.85 |
| 33 | 125 | 2484 | 19.87 |
| 34 | 58 | 1270 | 21.90 |
| 36 | LA | NA | |
| 39 | 235 | 3748 | 15.95 |
| 40 | 83 | 2589 | 31.19 |
| 42 | 49 | 2653 | 54.14 |
| 48 | 13 | 466 | 35.85 |
| 49 | 80 | 1774 | 22.18 |
| 50 | 219 | 3795 | 17.33 |
| 51 | 116 | 2062 | 17.78 |
| 52 | 13 | 417 | 32.08 |
| 53 | 90 | 2281 | 25.34 |
| 56 | 139 | 3334 | 23.99 |
| 57 | 490 | 5640 | 11.51 |
| 58 | 210 | 3133 | 14.92 |
| 59 | 257 | 4222 | 16.43 |
| 60 | 60 | 708 | 11.80 |
| 61 | 126 | 2388 | 18.95 |
| 62 | 86 | 1498 | 17.42 |
| 63 | 53 | 1008 | 19.02 |
| 64 | 38 | 918 | 24.16 |
| 65 | 48 | 2149 | 44.77 |
| 66 | 124 | 1991 | 16.06 |
| 67 | 40 | 1603 | 40.08 |
| 69 | 216 | 3160 | 14.63 |
| 70 | 21 | 222 | 10.57 |
| 72 | 92 | 1610 | 17.50 |
| 73 | 40 | 1195 | 29.88 |
| 74 | 4.4 | 90 | 20.45 |
| 75 | 80 | 901 | 11.26 |
| 76 | 98 | 2150 | 21.94 |
| 78 | 14 | 169 | 12.07 |
| 80 | 132 | 1872 | 14.18 |
| 82 | 16 | 282 | 17.63 |
| 83 | 134 | 1928 | 14.39 |
| 84 | 58 | 1634 | 28.17 |
| 88 | 176 | 2034 | 11.56 |
| 90 | 98 | 2169 | 22.13 |
| 91 | 43 | 1695 | 39.42 |
| 92 | 19 | 569 | 29.95 |
| 93 | 20 | 612 | 30.60 |
| 94 | 130 | 3068 | 23.60 |
| 95 | 70 | 2460 | 35.14 |
| 97 | 246 | LA | |
| 102 | 348 | 3547 | 10.19 |
| 104 | 67 | 1537 | 22.94 |
| 105 | 202 | 3952 | 19.56 |
| 107 | 46 | 1617 | 35.15 |
| 108 | 141 | 3420 | 24.26 |
| 109 | 74 | 1066 | 14.41 |
| 110 | 172 | 2702 | 15.71 |
| 111 | 38 | 1044 | 27.47 |
| 112 | 360 | LA | |
| 115 | NA | NA | |
| 116 | 78 | 2952 | 37.85 |
| 117 | 89 | 1246 | 14.00 |
| 119 | 90 | 1812 | 20.13 |
| 120 | 10 | 170 | 17.00 |
| 123 | 88 | 1902 | 21.61 |
| 124 | 220 | 6453 | 29.33 |
| 125 | 84 | 2313 | 27.54 |
| 126 | 90 | 1477 | 16.41 |
| 127 | 115 | 1903 | 16.55 |
| 133 | 312 | 3398 | 10.89 |
| 135 | 163 | 2079 | 12.75 |
| 137 | 110 | 1988 | 18.07 |
| 138 | 290 | 3420 | 11.79 |
| 139 | 22 | 281 | 12.77 |
| 140 | 46 | 2055 | 44.67 |
| 141 | 76 | 2034 | 26.76 |
| 142 | 201 | 3906 | 19.43 |
| 143 | 192 | 3358 | 17.49 |
| 144 | 26 | 616 | 23.69 |
| 145 | 54 | 1912 | 35.41 |
| 146 | 75 | 1189 | 15.85 |
| 147 | 46 | 1377 | 29.93 |
| 148 | 179 | 3087 | 17.25 |
| 149 | 99 | 1586 | 16.02 |
| 150 | 29 | 708 | 24.41 |
| 151 | 19 | 601 | 31.63 |
| 152 | 35 | 998 | 28.51 |
| 153 | 20 | 1056 | 52.80 |
| 154 | 60 | 833 | 13.88 |
| 155 | 42 | 1260 | 30.00 |
| 156 | 76 | 1540 | 20.26 |
| 157 | 14 | 361 | 25.79 |
| 158 | 12 | 225 | 18.75 |
| 159 | 72 | 2071 | 28.76 |
| 160 | 27 | 580 | 21.48 |
| 161 | 87 | 1782 | 20.48 |
| 162 | 36 | 850 | 23.61 |
| 163 | 15 | 465 | 31.00 |
| 164 | 20 | 443 | 22.15 |
| 166 | 68 | 1246 | 18.32 |
| 168 | 194 | 2572 | 13.26 |
| 169 | 39 | 973 | 24.95 |
| 170 | 35 | 640 | 18.29 |
| 171 | 43 | 622 | 14.47 |
| 172 | 24 | 1754 | 73.08 |
| 173 | 21 | 633 | 30.14 |
| 174 | 41 | 1075 | 26.22 |
| 175 | 116 | 2198 | 18.95 |
| 176 | 57 | 997 | 17.49 |
| 177 | 30 | 936 | 31.20 |
| 178 | 21 | 486 | 23.14 |
| 179 | 64 | 1316 | 20.56 |
| 180 | 41 | 1875 | 45.73 |
| 181 | 36 | 865 | 24.03 |
| 182 | 131 | 3392 | 25.89 |
| 183 | 17 | 318 | 18.71 |
| 184 | 13 | 240 | 18.46 |
| 185 | 7.5 | 128 | 17.07 |
| 186 | 122 | 2830 | 23.20 |
| 187 | 33 | 788 | 23.88 |
| 188 | 126 | 2828 | 22.44 |
| 189 | 55 | 2310 | 42.00 |
| 190 | 32 | 835 | 26.09 |
| 191 | 112 | 2341 | 20.90 |
| 192 | 44 | 1217 | 27.66 |
| 193 | 94 | 2383 | 25.35 |
| 194 | 257 | 4229 | 16.46 |
| 195 | 216 | 3139 | 14.53 |
| 196 | 34 | 441 | 12.97 |
| 197 | 34 | 645 | 18.97 |
| 198 | 32 | 835 | 26.09 |
| 199 | 106 | 1964 | 18.53 |
| 200 | 36 | 1237 | 34.36 |
| 201 | 51 | 1237 | 24.25 |
| 202 | 74 | 1055 | 14.26 |
| 203 | 40 | 1026 | 25.65 |
| 204 | 14 | 396 | 28.29 |
| 205 | 58 | 1168 | 20.14 |
| 206 | 18 | 921 | 51.17 |
| 207 | 26 | 488 | 18.77 |
| 209 | 26 | 716 | 27.54 |
| 210 | 25 | 492 | 19.68 |
| 211 | 84 | 1593 | 18.96 |
| 212 | 30 | 646 | 21.53 |
| 213 | 39 | 863 | 22.13 |
| 214 | 29 | 694 | 23.93 |
| 215 | 7.1 | 178 | 25.07 |
| 216 | 23 | 627 | 27.26 |
| 217 | 70 | 2948 | 42.11 |
| 218 | 291 | 3073 | 10.56 |
| 219 | 216 | 3139 | 14.53 |
| 220 | 23 | 405 | 17.61 |
| 221 | 69 | 1874 | 27.16 |
| 222 | 20 | 328 | 16.40 |
| 224 | 470 | LA | |
| 225 | 204 | 2653 | 13.00 |
| 226 | 28 | 891 | 31.82 |
| 227 | 140 | 3421 | 24.44 |
| 228 | 51 | 1322 | 25.92 |
| 229 | 14 | 670 | 47.86 |
| 230 | 27 | 279 | 10.33 |
| 231 | 212 | 2695 | 12.71 |
| 232 | 46 | 995 | 21.63 |
| 233 | 39 | 1025 | 26.28 |
| 234 | 20 | 750 | 37.50 |
| 235 | 5.7 | 183 | 32.11 |
| 236 | 26 | 1114 | 42.85 |
| 237 | 32 | 1183 | 36.97 |
| 238 | 5.1 | 112 | 21.96 |
| 239 | 14 | 261 | 18.64 |
| 240 | 36 | 396 | 11.00 |
| 241 | 37 | 676 | 18.27 |
| 242 | 8.4 | 235 | 27.98 |
| 243 | 6.5 | 148 | 22.77 |
| 244 | 41 | 581 | 14.17 |
| 246 | 18 | 482 | 26.78 |
| 248 | 3.9 | 63 | 16.15 |
| 250 | 41 | 820 | 20.00 |
| 254 | 42 | 503 | 11.98 |
| 255 | 81 | 978 | 12.07 |
| 256 | 52 | 875 | 16.83 |
| 257 | 76 | 1020 | 13.42 |
| 258 | 31 | 716 | 23.10 |
| 260 | 21 | 465 | 22.14 |
| 261 | 40 | 684 | 17.10 |
| 263 | 17 | 404 | 23.76 |
| 264 | 138 | 2480 | 17.97 |
| 265 | 38 | 549 | 14.45 |
| 266 | 231 | 3142 | 13.60 |
| 270 | 110 | 1511 | 13.74 |
| 271 | 89 | 1194 | 13.42 |
| 272 | 242 | 3178 | 13.13 |
| 273 | 78 | 2283 | 29.27 |
| 274 | 167 | 2521 | 15.10 |
| 276 | 80 | 1976 | 24.70 |
| 277 | 79 | 1914 | 24.23 |
| 278 | 28 | 395 | 14.11 |
| 279 | 284 | LA | |
| 281 | 3.3 | 42 | 12.73 |
| 283 | 244 | 2712 | 11.11 |
| 284 | 194 | 3436 | 17.71 |
| 286 | 91 | 2421 | 26.60 |
| 287 | 113 | 2564 | 22.69 |
| 288 | 66 | 1908 | 28.91 |
| 289 | 53 | 691 | 13.04 |
| 291 | 39 | 1049 | 26.90 |
| 292 | 50 | 1515 | 30.30 |
| 293 | 25 | 666 | 26.64 |
| 294 | 113 | 2905 | 25.71 |
| 295 | 399 | 4940 | 12.38 |
| 303 | 110 | 2992 | 27.20 |
| 304 | 204 | 3633 | 17.81 |
| 305 | 85 | 1666 | 19.60 |
| 306 | 319 | 3877 | 12.15 |
| 308 | 11 | 216 | 19.64 |
| 309 | 30 | 466 | 15.53 |
| 310 | 33 | 1125 | 34.09 |
| 311 | 59 | 1757 | 29.78 |
| 312 | 94 | 3039 | 32.33 |
| 313 | 38 | 508 | 13.37 |
| 314 | 12 | 283 | 23.58 |
| 315 | 54 | 1348 | 24.96 |
| 316 | 11 | 215 | 19.55 |
| 317 | 23 | 241 | 10.48 |
| 318 | 16 | 338 | 21.13 |
| 320 | 102 | 2519 | 24.70 |
| 322 | 268 | 4370 | 16.31 |
| 325 | 82 | 3175 | 38.72 |
| 328 | 38 | 2342 | 61.63 |
| 329 | 831 | NA | |
| 330 | 24 | 603 | 25.13 |
| 332 | 405 | NA | |
| 335 | 15 | 644 | 42.93 |
| 338 | 153 | 3770 | 24.64 |
| 340 | 295 | LA | |
| 341 | LA | NA | |
| 342 | 100 | 6795 | 67.95 |
| 343 | 212 | 4855 | 22.90 |
| 344 | 458 | NA | |
| 345 | 44 | 773 | 17.57 |
| 346 | 53 | 2186 | 41.25 |
| 347 | 32 | 1276 | 39.88 |
| 348 | 6.9 | 208 | 30.14 |
| 349 | 458 | NA | |
| 350 | 29 | 1026 | 35.38 |
| 351 | 85 | 1835 | 21.59 |
| 352 | 77 | 2265 | 29.42 |
| 353 | 108 | 1815 | 16.81 |
| 354 | 62 | 2527 | 40.76 |
| 355 | 150 | 4800 | 32.00 |
| 357 | 16 | 852 | 53.25 |
| 358 | 303 | >10000 | >33.00 |
| 359 | 50 | 1175 | 23.50 |
| 360 | 23 | 1167 | 50.74 |
| 364 | 18 | 694 | 38.56 |
| 365 | 35 | 1693 | 48.37 |
| 366 | 7.7 | 319 | 41.43 |
| 367 | 106 | 5260 | 49.62 |
| 368 | 37 | 2905 | 78.51 |
| 369 | 84 | 2698 | 32.12 |
| 370 | 118 | 3471 | 29.42 |
| 372 | 83 | 2525 | 30.42 |
| 374 | 210 | LA | |
| 375 | 280 | LA | |
| 376 | 105 | 3725 | 35.48 |
| 377 | 311 | 4308 | 13.85 |
| 378 | 316 | 6759 | 21.39 |
| 379 | 40 | 1815 | 45.38 |
| 381 | 63 | 2326 | 36.92 |
| 382 | 124 | 1537 | 12.40 |
| 383 | 50 | 1767 | 35.34 |
| 384 | 238 | 3415 | 14.35 |
| 385 | 104 | 3767 | 36.22 |
| 386 | 13 | 336 | 25.85 |
| 387 | 90 | 2466 | 27.40 |
| 390 | 233 | >10000 | >42.92 |
| 391 | 84 | 2851 | 33.94 |
| 392 | 28 | 1126 | 40.21 |
| 393 | 9.8 | 502 | 51.22 |
| 394 | 16 | 717 | 44.81 |
| 395 | 66 | 2709 | 41.05 |
| 396 | 298 | 4831 | 16.21 |
| 397 | 197 | 6789 | 34.46 |
| 398 | 39 | 1618 | 41.49 |
| 400 | 33 | 1874 | 56.79 |
| 401 | 204 | > 10000 | >49.02 |
| 402 | 24 | 569 | 23.71 |
| 403 | 14 | 277 | 19.79 |
| 404 | 4.6 | 114 | 24.78 |
| 405 | 29 | 926 | 31.93 |
| 406 | 23 | 906 | 39.39 |
| 408 | 10 | 203 | 20.30 |
| 410 | 9 | 219 | 24.33 |
| 411 | 15 | 221 | 14.73 |
| 412 | 10 | 179 | 17.90 |
| 413 | 26 | 390 | 15.00 |
| 414 | 18 | 305 | 16.94 |
| 415 | 19 | 810 | 42.63 |
| 416 | 126 | 2924 | 23.21 |
| 417 | 20 | 790 | 39.50 |
| 419 | 14 | 226 | 16.14 |
| 420 | 26 | 743 | 28.58 |
| 421 | 29 | 669 | 23.07 |
| 422 | 21 | 806 | 38.38 |
| 423 | 25 | 682 | 27.28 |
| 424 | 23 | 374 | 16.26 |
| 425 | 54 | 850 | 15.74 |
| 426 | 49 | 700 | 14.29 |
| 427 | 235 | > 10000 | >42.55 |
| 428 | 12 | 321 | 26.75 |
| 429 | 14 | 876 | 62.57 |
| 430 | 22 | 585 | 26.59 |
| 431 | 10 | 248 | 24.80 |
| 432 | 16 | 657 | 41.06 |
| 433 | 33 | 1110 | 33.64 |
| 434 | 36 | 906 | 25.17 |
| 435 | 15 | 407 | 27.13 |
| 436 | 27 | 734 | 27.19 |
| 437 | 13 | 242 | 18.62 |
| 438 | 40 | 1506 | 37.65 |
| 439 | 37 | 735 | 19.86 |
| 441 | 48 | 1791 | 37.31 |
| 442 | 42 | 634 | 15.10 |
| 443 | 26 | 468 | 18.00 |
| 444 | 80 | 1234 | 15.43 |
| 445 | 22 | 548 | 24.91 |
| 447 | 24 | 315 | 13.13 |
| 448 | 27 | 586 | 21.70 |
| 449 | 63 | 1472 | 23.37 |
| 450 | 20 | 1224 | 61.20 |
| 451 | 40 | 1012 | 25.30 |
| 452 | 45 | 1425 | 31.67 |
| 453 | 23 | 523 | 22.74 |
| 454 | 36 | 752 | 20.89 |
| 455 | 9.5 | 305 | 32.11 |
| 456 | 28 | 1929 | 68.89 |
| 457 | 12 | 123 | 10.25 |
| 458 | 15 | 371 | 24.73 |
| 459 | 30 | 408 | 13.60 |
| 460 | 18 | 479 | 26.61 |
| 461 | 15 | 295 | 19.67 |
| 462 | 30 | 484 | 16.13 |
| 464 | 17 | 1089 | 64.06 |
| 465 | 23 | 1043 | 45.35 |
| 466 | 13 | 660 | 50.77 |
| 467 | 94 | 3039 | 32.33 |
| 468 | 180 | 4336 | 24.09 |
| 470 | 94 | 3039 | 32.33 |
| 471 | 97 | 5856 | 60.37 |
| 472 | 30 | 971 | 32.37 |
| 473 | 91 | 5104 | 56.09 |
| 474 | 28 | 929 | 33.18 |
| 475 | 63 | 2760 | 43.81 |
| 476 | 28 | 942 | 33.64 |
| 477 | 28 | 1456 | 52.00 |
| 478 | 166 | 4317 | 26.01 |
| 479 | 32 | 1269 | 39.66 |
| 480 | 55 | 1769 | 32.16 |
| 481 | 15 | 708 | 47.20 |
| 482 | 60 | 5368 | 89.47 |
| 483 | 100 | 4172 | 41.72 |
| 484 | 15 | 836 | 55.73 |
| 485 | 415 | 4831 | 11.64 |
| 486 | 34 | 1398 | 41.12 |
| 487 | 58 | 2350 | 40.52 |
| 488 | 25 | 531 | 21.24 |
| 489 | 54 | 1406 | 26.04 |
| 490 | 26 | 1013 | 38.96 |
| 491 | 189 | 2233 | 11.81 |
| 492 | 37 | 1942 | 52.49 |
| 493 | 68 | 1900 | 27.94 |
| 494 | 29 | 1004 | 34.62 |
| 495 | 69 | 1656 | 24.00 |
| 502 | 140 | 1511 | 10.79 |
| 507 | 38 | 1685 | 44.34 |
| 508 | 114 | 2711 | 23.78 |
| 509 | 443 | LA | |
| 510 | 222 | 7449 | 33.55 |
| 512 | 259 | 5834 | 22.53 |

Conclusion: as shown in the table above, the compounds of the present invention have exhibited good inhibitory activity against HCT116 MTAP KO cell growth, while the compounds of the present invention have exhibited good selectivity towards HCT116-WT cells. For example, the measured IC₅₀ values for cell growth inhibition are less than 10 nM and the selectivity is greater than 20 times.

### Test Example 3: Pharmacodynamic test of the compounds of the present invention in a human colon cancer xenograft model (MTAP-deficient HCT116)

The compounds (100 mpk, blank vehicle as a control) were orally administered to the HCT116 MTAP deficient mice (cell source: creative biogene-CSC-RT2701) of human colon cancer xenograft model by gavage daily during the experimental planning period. The positive compound AMG9747 was synthesized according to the method reported in the patent WO2021163344A1. The compounds were formulated as follows: weighing 100.00 mg of a test compound, adding it to 10 mL of 0.5% MC4000+0.2% Tween 80, vortexing and shaking to prepare a suspension, i.e., a dose of 100 mpk. Each group had 8 mice and was subjected to administration by gavage once a day. The tumor volume was measured once in groups before administration, and measured on days 3, 6, 10, 13, 16 and 19 respectively after administration. The positive drug (synthesized according to the patent WO2021163344A1) AMG-9747 was used as a control, and compound AMG-9747 has a structure of:

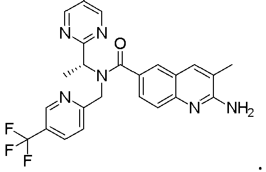

**Table 3. Percentage of inhibition of the compounds of the present invention on tumor growth in the human colon cancer xenograft (MTAP-deficient HCT116) model**

| Compound No. | AMG-9747 | 1 | 3 | 184 | 189 | 392 |
|---|---|---|---|---|---|---|
| Percentage of tumor growth inhibition | 68% | 80% | 86% | 81% | 93% | 69% |

The results showed that in the HCT116 MTAP-deficient mice tumor model, the compounds of the present invention exhibit superior inhibitory effect on tumor growth at a dose of 100 mpk than that of AMG-9747.

## Claims

1. A compound of general formula (I) or a tautomer, mesomer, racemate, enantiomer, diastereomer thereof, or a mixture thereof, or an oxynitride thereof, or a pharmaceutically acceptable salt thereof, 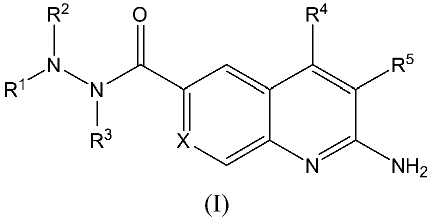 wherein:
X is CR⁶ or N;
R¹ and R² are each independently selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, -B(OH)₂, alkyl, -OR^{a}, haloalkyl, haloalkoxy, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)₂R^{b}, alkyl, -OR^{a}, haloalkyl, haloalkoxy, alkenyl, alkynyl, cycloalkyl, heterocyclyl, aryl and heteroaryl;
R³ is selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)R^{b}, alkyl, -OR^{a}, haloalkyl, haloalkoxy, alkenyl, alkynyl, cycloalkyl, heterocyclyl, aryl and heteroaryl;
or, R¹ and R², or R¹ and R³, together with the nitrogen atom to which they are attached, form a nitrogen-containing heterocyclyl or nitrogen-containing heteroaryl, and the nitrogen-containing heterocyclyl or nitrogen-containing heteroaryl is optionally further substituted by one or more groups selected from the group consisting of halogen, -NR^{a}R^{b}, nitro, cyano, hydroxy, thiol, carboxyl, oxo, methylene, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -(CH₂)_{q}-OR^{a}, alkyl, alkoxy, haloalkyl, haloalkoxy, deuterated alkyl, deuterated alkoxy, alkenyl, alkynyl, cycloalkyl, heterocyclyl, aryl and heteroaryl;
R⁴ and R⁵ are each independently selected from the group consisting of hydrogen, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -CH₂-R⁷, -NR^{a}R^{b}, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are optionally further substituted by one or more groups selected from the group consisting of deuterium, halogen, amino, -NR^{a}R^{b}, nitro, cyano, hydroxy, thiol, carboxyl, ester, amido, oxo, alkyl, alkoxy, haloalkyl, haloalkoxy, alkenyl, alkynyl, cycloalkyl, heterocyclyl, aryl and heteroaryl;
or, R⁴ and R⁵, together with the atoms to which they are attached, form a heteroaryl, heterocyclyl, aryl or cycloalkyl, and the heteroaryl, heterocyclyl, aryl or cycloalkyl is optionally further substituted by one or more groups selected from the group consisting of halogen, amino, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, alkyl, alkoxy, haloalkyl, haloalkoxy, alkenyl, alkynyl, cycloalkyl, heterocyclyl, aryl and heteroaryl;
R⁶ is selected from the group consisting of hydrogen, halogen, amino, nitro, cyano, hydroxy, thiol, carboxyl, ester, oxo, -NR^{a}R^{b}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, ester, oxo, alkyl, alkoxy, haloalkyl, haloalkoxy, alkenyl, alkynyl, cycloalkyl, heterocyclyl, aryl and heteroaryl;
R⁷ is selected from the group consisting of heteroaryl, aryl, heterocyclyl and cycloalkyl, and the heteroaryl, aryl, heterocyclyl and cycloalkyl are optionally further substituted by one or more groups selected from the group consisting of deuterium, halogen, amino, -NR^{a}R^{b}, nitro, cyano, hydroxy, thiol, carboxyl, oxo, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)R^{b}, alkyl, alkoxy, haloalkyl, haloalkoxy, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, oxo, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, alkyl, -OR^{a}, -(CH₂)_{q}-OR^{a}, haloalkyl, haloalkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxyl, -NR^{c}R^{d}, -C(=O)OR^{c}, -C(=O)R^{c}, -C(=O)NR^{c}R^{d}, -OC(=O)R^{c}, -NR^{c}C(=O)R^{d}, -S(=O)₂R^{c}, -NR^{c}S(=O)₂R^{d}, -S(=O)₂NR^{c}R^{d}, -S(=O)R^{c}, -P(=O)R^{c}R^{d}, -NR^{c}S(=O)₂R^{d}, alkyl, alkoxy, alkenyl, alkynyl, cycloalkyl, heterocyclyl, aryl and heteroaryl;
or R^{a} and R^{b}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
R^{c} and R^{d} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of halogen, amino, nitro, cyano, hydroxy, thiol, carboxyl, alkyl, alkoxy, alkenyl, alkynyl, cycloalkyl, heterocyclyl, aryl and heteroaryl;
or R^{c} and R^{d}, together with the atoms to which they are attached, form a heterocyclyl, and the heterocyclyl is optionally further substituted by one or more groups selected from the group consisting of halogen, amino, nitro, cyano, oxo, hydroxy, thiol, carboxyl, ester, alkyl, alkoxy, alkenyl, alkynyl, cycloalkyl, heterocyclyl, aryl and heteroaryl;
p is an integer from 1 to 6;
q is an integer from 1 to 6.

2. The compound of general formula (I) or a tautomer, mesomer, racemate, enantiomer, diastereomer thereof, or a mixture thereof, or an oxynitride thereof, or a pharmaceutically acceptable salt thereof according to claim 1, which is a compound of general formula (II) or a tautomer, mesomer, racemate, enantiomer, diastereomer thereof, or a mixture thereof, or an oxynitride thereof, or a pharmaceutically acceptable salt thereof, 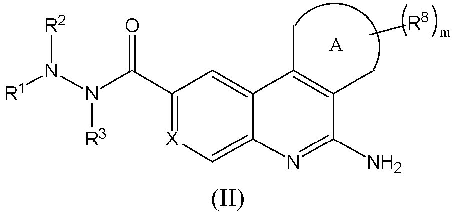
wherein, ring A is selected from the group consisting of heteroaryl, heterocyclyl, aryl and cycloalkyl;
each R⁸ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, alkyl, alkoxy, haloalkyl, haloalkoxy, alkenyl, alkynyl, cycloalkyl, heterocyclyl, aryl and heteroaryl;
m is an integer from 0 to 4;
X, R¹ to R³, R^{a} and R^{b} are as defined in claim 1.

3. The compound of general formula (I) or a tautomer, mesomer, racemate, enantiomer, diastereomer thereof, or a mixture thereof, or an oxynitride thereof, or a pharmaceutically acceptable salt thereof according to claim 1, wherein R⁴ is selected from the group consisting of hydrogen, C₁₋₆ alkyl and C₃₋₆ cycloalkyl.

4. The compound of general formula (I) or a tautomer, mesomer, racemate, enantiomer, diastereomer thereof, or a mixture thereof, or an oxynitride thereof, or a pharmaceutically acceptable salt thereof according to claim 1, wherein R⁵ is selected from the group consisting of halogen, cyano, C₁₋₆ alkyl, C₁₋₆ alkoxy, C₃₋₆ cycloalkyl and C₂₋₆ alkenyl, and the C₁₋₆ alkyl is optionally substituted by deuterium, halogen, hydroxy or C₁₋₆ alkoxy.

5. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 4, wherein R¹ is selected from the group consisting of hydrogen, C₁₋₆ alkyl, C₃₋₆ cycloalkyl and 4 to 6 membered heterocyclyl, wherein the C₁₋₆ alkyl, C₃₋₆ cycloalkyl and 4 to 6 membered heterocyclyl are optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

6. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 5, wherein R² is selected from the group consisting of C₁₋₆ alkyl, C₃₋₆ cycloalkyl, 4 to 6 membered heterocyclyl, C₆₋₁₀ aryl and 5 to 10 membered heteroaryl, wherein the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, ester, oxo, -S(=O)₂R^{a}, -S(=O)R^{a}, -C(=O)R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl and C₂₋₆ alkynyl;
R^{a} and R^{b} are each independently hydroxy or C₁₋₆ alkyl.

7. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 5, wherein R² is selected from the group consisting of C₆₋₁₀ aryl and 5 to 10 membered heteroaryl, wherein the C₆₋₁₀ aryl and 5 to 10 membered heteroaryl are optionally further substituted by one or more groups selected from the group consisting of deuterium, halogen, cyano, carboxyl, -S(=O)₂R^{a}, -S(=O)R^{a}, -C(=O)R^{a}, -P(=O)R^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl.

8. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 4, wherein, R¹ and R², together with the nitrogen atom to which they are attached, form a 5 to 10 membered nitrogen-containing heterocyclyl or 5 to 10 membered nitrogen-containing heteroaryl, and the 5 to 10 membered nitrogen-containing heterocyclyl or 5 to 10 membered nitrogen-containing heteroaryl is optionally further substituted by one or more groups selected from the group consisting of halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy.

9. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 8, wherein, R³ is selected from the group consisting of hydrogen, -(CH₂)ₚ-R⁷, -NR^{a}R^{b}, -C(=O)R^{a}, -S(=O)₂R^{a}, C₁₋₆ alkyl, C₃₋₆ cycloalkyl, 4 to 10 membered heterocyclyl, C₆₋₁₀ aryl and 5 to 10 membered heteroaryl, wherein the C₁₋₆ alkyl, C₃₋₆ cycloalkyl, 4 to 10 membered heterocyclyl, C₆₋₁₀ aryl and 5 to 10 membered heteroaryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)R^{b}, C₁₋₆ alkyl, -OR^{a}, C₁₋₆ haloalkyl and C₁₋₆ haloalkoxy;
R⁷ is selected from the group consisting of 5 to 6 membered heteroaryl and C₆₋₁₀ aryl, and the 5 to 6 membered heteroaryl and C₆₋₁₀ aryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, C₁₋₆ alkyl, C₁₋₆ alkoxy, C₁₋₆ haloalkyl and C₁₋₆ haloalkoxy;
p is an integer from 1 to 6;
R^{a} and R^{b} are as defined in claim 1.

10. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 8, wherein, R³ is -(CH₂)ₚ-R⁷; R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, oxo, -NR^{a}R^{b}, -(CH₂)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen, C₁₋₆ alkyl and hydroxy substituted C₁₋₆ alkyl;
p is 1;
q is 1 or 2.

11. The compound of general formula (I) or a tautomer, mesomer, racemate, enantiomer, diastereomer thereof, or a mixture thereof, or an oxynitride thereof, or a pharmaceutically acceptable salt thereof according to claim 1, wherein, R¹ and R³, together with the nitrogen atom to which they are attached, form a 5 to 10 membered nitrogen-containing heterocyclyl, and the 5 to 10 membered nitrogen-containing heterocyclyl is optionally substituted by one or more groups selected from the group consisting of halogen, hydroxy, thiol, oxo, methylene, -(CH₂)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, C₆₋₁₀ aryl and 5 to 10 membered heteroaryl;
R^{a} is hydrogen or C₁₋₆ alkyl;
q is 1 or 2.

12. The compound of general formula (I) or a tautomer, mesomer, racemate, enantiomer, diastereomer thereof, or a mixture thereof, or an oxynitride thereof, or a pharmaceutically acceptable salt thereof according to claim 1 or 2, wherein,
R¹ is selected from the group consisting of hydrogen, C₁₋₆ alkyl and C₃₋₆ cycloalkyl;
R² is phenyl or 5 to 6 membered heterocyclyl, preferably phenyl or 6 membered heterocyclyl, which is optionally further substituted by one or more groups selected from the group consisting of deuterium, halogen, C₁₋₆ alkyl, C₁₋₆ alkoxy, C₁₋₆ haloalkyl and C₁₋₆ haloalkoxy;
R³ is selected from the group consisting of hydrogen, C₁₋₆ alkyl, C₁₋₆ haloalkyl, C₃₋₆ cycloalkyl and 4 to 6 membered heterocyclyl.

13. The compound of general formula (I) or a tautomer, mesomer, racemate, enantiomer, diastereomer thereof, or a mixture thereof, or an oxynitride thereof, or a pharmaceutically acceptable salt thereof according to claim 1 or 2, wherein,
R¹ is selected from the group consisting of hydrogen, C₁₋₆ alkyl and C₃₋₆ cycloalkyl;
R² is selected from the group consisting of hydrogen, C₁₋₆ alkyl and C₃₋₆ cycloalkyl; and the C₁₋₆ alkyl is optionally substituted by hydroxy or C₁₋₆ alkoxy;
R³ is -(CH₂)ₚ-R⁷;
R⁷ is a 5 to 6 membered heteroaryl or phenyl, preferably 6 membered heteroaryl or phenyl, which is optionally further substituted by one or more groups selected from the group consisting of deuterium, halogen, C₁₋₆ alkyl, C₁₋₆ alkoxy, C₁₋₆ haloalkyl and C₁₋₆ haloalkoxy;
p is 1.

14. The compound of general formula (I) or a tautomer, mesomer, racemate, enantiomer, diastereomer thereof, or a mixture thereof, or an oxynitride thereof, or a pharmaceutically acceptable salt thereof according to claim 1, which is a compound of general formula (IA) or (IIA) or a tautomer, mesomer, racemate, enantiomer, diastereomer thereof, or a mixture thereof, or an oxynitride thereof, or a pharmaceutically acceptable salt thereof, 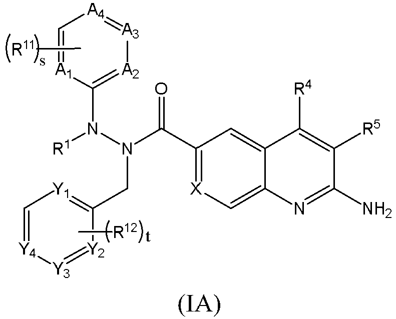 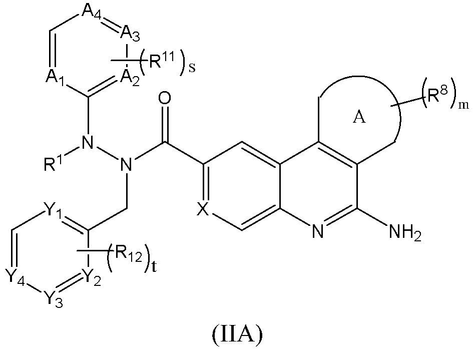 wherein,
A₁, A₂, A₃ and A₄ are each independently selected from the group consisting of CH and N, and preferably, one of them is N and the others are CH, or two of them are N and the others are CH;
Y₁, Y₂, Y₃ and Y₄ are each independently selected from the group consisting of CH and N, and preferably, one of them is N and the others are CH, or two of them are N and the others are CH;
ring A is selected from the group consisting of 5 to 6 membered heteroaryl, 5 to 6 membered heterocyclyl, phenyl and 5 to 6 membered cycloalkyl;
R¹ is selected from the group consisting of hydrogen, C₁₋₆ alkyl, C₃₋₆ cycloalkyl and 4 to 6 membered heterocyclyl, wherein the C₁₋₆ alkyl, C₃₋₆ cycloalkyl or 4 to 6 membered heterocyclyl is optionally further substituted by one or more groups selected from the group consisting of deuterium, halogen, amino, nitro, cyano, hydroxy, thiol, carboxyl, ester, oxo, C₁₋₆ alkyl, C₁₋₆ alkoxy, C₁₋₆ haloalkyl and C₁₋₆ haloalkoxy;
R⁴ is selected from the group consisting of hydrogen, C₁₋₆ alkyl and C₃₋₆ cycloalkyl;
R⁵ is selected from the group consisting of halogen, cyano, C₁₋₆ alkyl, C₁₋₆ alkoxy, C₃₋₆ cycloalkyl and C₂₋₆ alkenyl, and the C₁₋₆ alkyl is optionally substituted by deuterium, halogen, hydroxy or C₁₋₆ alkoxy;
each R¹¹ is independently selected from the group consisting of hydrogen, deuterium, halogen, cyano, C₁₋₆ alkyl, C₁₋₆ alkoxy, C₁₋₆ haloalkyl, C₁₋₆ haloalkoxy, C₂₋₆ alkenyl, C₂₋₆ alkynyl, -S(=O)₂R^{a}, -S(=O)R^{a}, -C(=O)R^{a}, -P(=O)R^{a}R^{b}, -B(OH)₂, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy; or,
any two adjacent R¹¹, together with the atoms to which they are attached, form a phenyl, 5 to 6 membered heteroaryl, 5 to 6 membered heterocyclyl or 4 to 6 membered cycloalkyl, and the phenyl, 5 to 6 membered heteroaryl, 5 to 6 membered heterocyclyl or 4 to 6 membered cycloalkyl is optionally substituted by a group(s) selected from the group consisting of halogen and C₁₋₆ alkyl;
each R¹² is independently selected from the group consisting of hydrogen, deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, cyano, hydroxy, amino, oxo, -NR^{a}R^{b}, -(CH₂)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl; or
any two adjacent R¹², together with the atoms to which they are attached, form a phenyl, 5 to 6 membered heteroaryl, 5 to 6 membered heterocyclyl or 4 to 6 membered cycloalkyl, and the phenyl, 5 to 6 membered heteroaryl, 5 to 6 membered heterocyclyl or 4 to 6 membered cycloalkyl is optionally substituted by a group(s) selected from the group consisting of halogen, C₁₋₆ alkyl and 4 to 6 membered heterocyclyl, and the 4 to 6 membered heterocyclyl is optionally substituted by C₁₋₆ alkyl;
each R⁸ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by one or more groups selected from the group consisting of halogen, oxo and C₁₋₆ alkyl;
R^{c} and R^{d} are each independently selected from the group consisting of hydrogen, C₁₋₆ alkyl and hydroxy substituted C₁₋₆ alkyl;
m is an integer from 0 to 4;
q is 1 or 2;
s is an integer from 1 to 4; and preferably 1 or 2;
t is an integer from 1 to 4, and preferably 1, 2 or 3;
X is as defined in claim 1.

15. The compound of general formula (I) or a tautomer, mesomer, racemate, enantiomer, diastereomer thereof, or a mixture thereof, or an oxynitride thereof, or a pharmaceutically acceptable salt thereof according to claim 14, wherein, 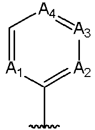 is selected from the group consisting of pyrimidinyl, pyridyl, phenyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl, preferably, is selected from the group consisting of 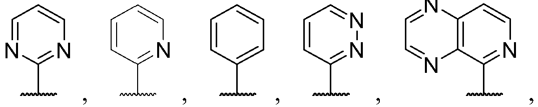 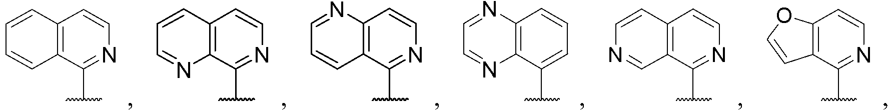 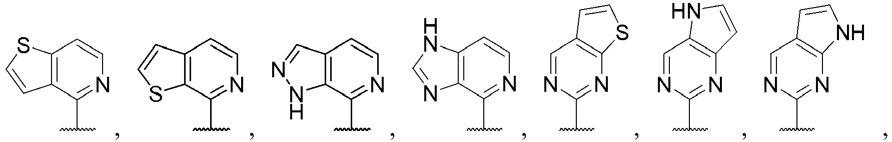 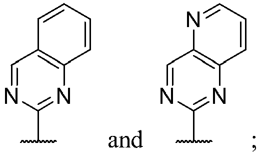 and more preferably, is

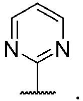

16. The compound of general formula (I) or a tautomer, mesomer, racemate, enantiomer, diastereomer thereof, or a mixture thereof, or an oxynitride thereof, or a pharmaceutically acceptable salt thereof according to claim 14 or 15, wherein 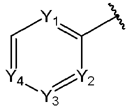 is selected from the group consisting of pyridyl, phenyl, pyrimidinyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl, preferably, is selected from the group consisting of 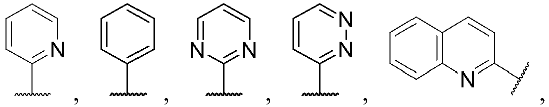 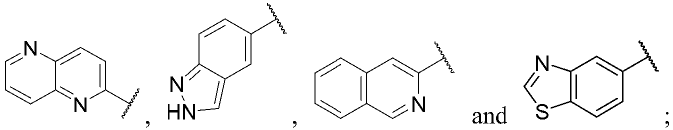 and more preferably, is selected from the group consisting of

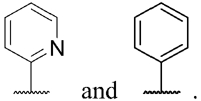

17. The compound of general formula (I) or a tautomer, mesomer, racemate, enantiomer, diastereomer thereof, or a mixture thereof, or an oxynitride thereof, or a pharmaceutically acceptable salt thereof according to any one of claims 1 to 10 and 14 to 16, wherein, R¹ is selected from the group consisting of hydrogen, C₁₋₆ alkyl and C₃₋₆ cycloalkyl, wherein the C₁₋₆ alkyl is optionally substituted by one or more groups selected from the group consisting of deuterium, halogen and C₁₋₆ alkoxy;
preferably, R¹ is selected from the group consisting of C₁₋₆ alkyl, C₁₋₆ haloalkyl, C₁₋₆ deuterated alkyl, C₃₋₆ cycloalkyl and 4 to 6 membered heterocyclyl, wherein the C₁₋₆ alkyl is optionally substituted by C₁₋₆ alkoxy.

18. The compound of general formula (I) or a tautomer, mesomer, racemate, enantiomer, diastereomer thereof, or a mixture thereof, or an oxynitride thereof, or a pharmaceutically acceptable salt thereof according to any one of claims 14 to 17, wherein, each R¹¹ is each independently selected from the group consisting of hydrogen, deuterium, halogen, cyano, carboxyl, C₁₋₆ alkyl, C₁₋₆ alkoxy, C₁₋₆ haloalkyl, C₁₋₆ haloalkoxy, C₂₋₆ alkenyl, C₂₋₆ alkynyl, -P(=O)R^{a}R^{b} and -B(OH)₂;
R^{a} and R^{b} are each independently selected from the group consisting of hydrogen and C₁₋₆ alkyl;
s is 1 or 2.

19. The compound of general formula (I) or a tautomer, mesomer, racemate, enantiomer, diastereomer thereof, or a mixture thereof, or an oxynitride thereof, or a pharmaceutically acceptable salt thereof according to any one of claims 14 to 18, wherein, each R¹² is independently selected from the group consisting of hydrogen, deuterium, halogen, -NR^{a}R^{b}, cyano, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₁₋₆ alkyl, C₃₋₆ cycloalkyl and 4 to 6 membered heterocyclyl are optionally further substituted by one or more groups selected from the group consisting of deuterium, halogen, hydroxy and C₁₋₆ alkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by halogen;
t is 1, 2 or 3;
preferably, R¹² is C₁₋₆ haloalkyl, and t is 1.

20. The compound of general formula (I) or a tautomer, mesomer, racemate, enantiomer, diastereomer thereof, or a mixture thereof, or an oxynitride thereof, or a pharmaceutically acceptable salt thereof according to any one of claims 14 to 19, wherein 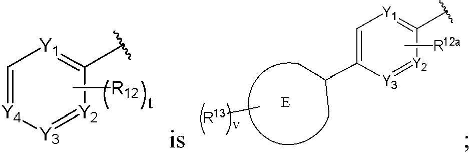 wherein,
Y₁, Y₂ and Y₃ are each independently CH or N;
ring E is selected from the group consisting of 5 to 10 membered heteroaryl and 5 to 10 membered heterocyclyl; preferably selected from the group consisting of pyridyl, dihydropyridyl, tetrahydropyridyl, phenyl, pyrazinyl, pyrimidinyl, furanyl, dihydrofuranyl, tetrahydrofuranyl, oxazolyl, isoxazolyl, pyrrolyl, pyrazolyl, imidazolyl, triazolyl, tetrazolyl, thiazolyl, thiadiazolyl, thienyl, pyranyl, dihydropyranyl, tetrahydropyranyl, piperidyl, morpholinyl, pyridopyrazolyl and quinolyl;
R^{12a} is selected from the group consisting of hydrogen and halogen, preferably hydrogen;
each R¹³ is independently selected from the group consisting of hydrogen, deuterium, halogen, cyano, hydroxy, oxo, -NR^{a}R^{b}, -(CH₂)_{q}-OR^{a}, C₁₋₆ alkyl, C₁₋₆ alkoxy, C₁₋₆ haloalkyl, C₁₋₆ haloalkoxy, C₃₋₆ cycloalkyl and 4 to 6 membered heterocyclyl;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}, or
R^{a} and R^{b}, together with the atoms to which they are attached, form a 4 to 6 membered heterocyclyl, and the 4 to 6 membered heterocyclyl is optionally substituted by halogen, oxo, C₁₋₆ alkyl;
R^{c} and R^{d} are each independently selected from the group consisting of hydrogen and C₁₋₆ alkyl;
q is 1 or 2, preferably 1;
t is 1, 2 or 3;
v is 1 or 2.

21. The compound of general formula (I) or a tautomer, mesomer, racemate, enantiomer, diastereomer thereof, or a mixture thereof, or an oxynitride thereof, or a pharmaceutically acceptable salt thereof according to claim 1 or 2, which is a compound of general formula (IIIA) or general formula (IIIB) or a tautomer, mesomer, racemate, enantiomer, diastereomer thereof, or a mixture thereof, or an oxynitride thereof, or a pharmaceutically acceptable salt thereof, 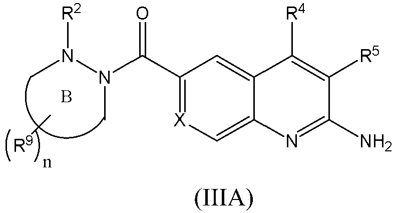 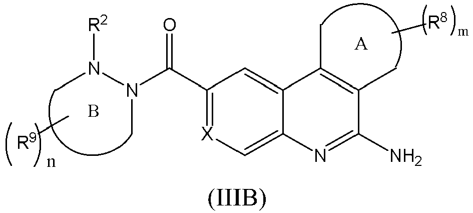 wherein,
ring B is selected from the group consisting of 5 to 10 membered nitrogen-containing heterocyclyl and 5 to 10 membered nitrogen-containing heteroaryl;
each R⁹ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, -(CH₂)_{q}-OR^{a}, oxo, methylene, alkyl, alkoxy, haloalkyl, haloalkoxy, deuterated alkyl, deuterated alkoxy, alkenyl, alkynyl, cycloalkyl, heterocyclyl, aryl and heteroaryl;
n is an integer from 0 to 4;
q is 1 or 2;
R² is selected from the group consisting of hydrogen, -(CH₂)ₚ-R⁷, -NR^{a}R^{b}, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, -B(OH)₂, alkyl, -OR^{a}, haloalkyl, haloalkoxy, alkenyl, alkynyl, cycloalkyl, heterocyclyl, aryl and heteroaryl, and the alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -NR^{a}R^{b}, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b} , -S(=O)R^{a}, -P(=O)R^{a}R^{b}, -NR^{a}S(=O)R^{b}, alkyl, -OR^{a}, haloalkyl, haloalkoxy, alkenyl, alkynyl, cycloalkyl, heterocyclyl, aryl and heteroaryl;
X, R⁴, R⁵, R⁷, R^{a}, R^{b} and p are as defined in claim 1;
ring A, R⁸ and m are as defined in claim 2;
preferably, ring B is a 5 to 10 membered nitrogen-containing heterocyclyl;
each R⁹ is independently selected from the group consisting of halogen, hydroxy, thiol, oxo, methylene, -(CH₂)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, and phenyl;
n is an integer from 0 to 4;
q is 1 or 2;
R² is selected from the group consisting of C₆₋₁₀ aryl and 5 to 10 membered heteroaryl, wherein the C₆₋₁₀ aryl and 5 to 10 membered heteroaryl are optionally further substituted by one or more groups selected from the group consisting of deuterium, halogen, amino, nitro, cyano, hydroxy, thiol, carboxyl, -NR^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, wherein the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl;
X, R⁴ and R⁵ are as defined in claim 1;
ring A, R⁸ and m are as defined in claim 2.

22. The compound of general formula (I) or a tautomer, mesomer, racemate, enantiomer, diastereomer thereof, or a mixture thereof, or an oxynitride thereof, or a pharmaceutically acceptable salt thereof according to claim 21, wherein, ring B is a 5 to 10 membered nitrogen-containing heterocyclyl, preferably 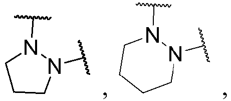 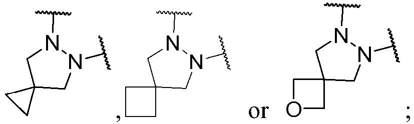
each R⁹ is independently selected from the group consisting of halogen, hydroxy, thiol, oxo, methylene, -(CH₂)_{q}-OR^{a}, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy, and phenyl;
R^{a} is hydrogen or C₁₋₆ alkyl;
q is 1 or 2; and preferably 1;
n is 1 or 2.

23. The compound of general formula (I) or a tautomer, mesomer, racemate, enantiomer, diastereomer thereof, or a mixture thereof, or an oxynitride thereof, or a pharmaceutically acceptable salt thereof according to claim 21 or 22, wherein, R² is selected from the group consisting of C₆₋₁₀ aryl and 5 to 10 membered heteroaryl, preferably, is selected from the group consisting of phenyl, pyridyl, pyrimidinyl, pyrazinyl, pyridazinyl, quinolyl, isoquinolyl, quinoxalinyl, quinazolinyl, indolyl, isoindolyl, naphthyl, naphthyridinyl, pyridofuranyl, pyridothienyl, pyridothiazolyl, pyridopyridazinyl, pyridopyrrolyl, pyridopyrrolidinyl, pyridoimidazolyl, pyridopyrazolyl, pyridopiperidyl, pyridomorpholinyl, pyridopiperazinyl, pyrimidofuranyl, pyrimidopyrrolyl, pyrimidothienyl, pyrimidoimidazolyl, pyrimidopyrazolyl, pyrimidopiperidyl, pyrimidomorpholinyl, pyrimidopiperazinyl, pyridazinopyrrolyl, pyridazinoimidazolyl, pyridazinopiperidyl, pyridazinomorpholinyl, pyridazinopiperazinyl, benzopyrrolyl, benzopyrimidinyl, benzopyridyl, benzopyridazinyl, benzofuranyl, benzothienyl and benzothiazolyl; and more preferably, is selected from the group consisting of phenyl and pyridyl;
which is optionally further substituted by one or more groups selected from the group consisting of deuterium, halogen, cyano, -NR^{a}R^{b}, -B(OH)₂, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl.

24. The compound of general formula (I) or a tautomer, mesomer, racemate, enantiomer, diastereomer thereof, or a mixture thereof, or an oxynitride thereof, or a pharmaceutically acceptable salt thereof according to any one of claims 21 to 23, which is a compound of general formula (IIIAa) or general formula (IIIBa) or a tautomer, mesomer, racemate, enantiomer, diastereomer thereof, or a mixture thereof, or an oxynitride thereof, or a pharmaceutically acceptable salt thereof, 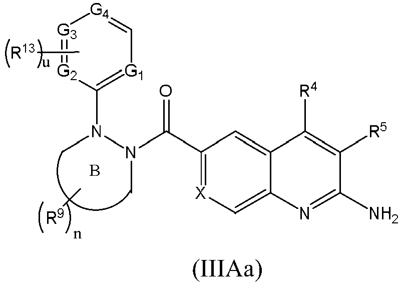 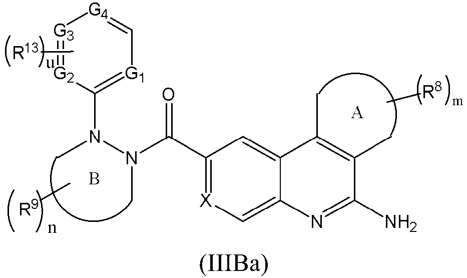 wherein,
G₁, G₂, G₃ and G₄ are each independently selected from the group consisting of CH and N; preferably, G₁, G₂, G₃ and G₄ are all CH; or one of G₁, G₂, G₃ and G₄ is N and the others are CH, or two of G₁, G₂, G₃ and G₄ are N and the others are CH;
each R¹³ is independently selected from the group consisting of hydrogen, deuterium, halogen, cyano, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl, and the C₂₋₆ alkenyl, C₂₋₆ alkynyl, C₃₋₆ cycloalkyl, 4 to 6 membered heterocyclyl, C₆₋₁₀ aryl and 5 to 10 membered heteroaryl are optionally further substituted by one or more groups selected from the group consisting of deuterium, halogen, hydroxy, -NR^{a}R^{b}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen and C₁₋₆ alkyl; or
R^{a} and R^{b}, together with the atoms to which they are attached, form a 4 to 6 membered heterocyclyl, and the 4 to 6 membered heterocyclyl is optionally substituted by C₁₋₆ alkyl;
u is 1 or 2;
ring B, ring A, X, R⁴, R⁵, R⁸, R⁹, n and m are as defined in any one of claims 21 to 23.

25. The compound of general formula (I) or a tautomer, mesomer, racemate, enantiomer, diastereomer thereof, or a mixture thereof, or an oxynitride thereof, or a pharmaceutically acceptable salt thereof according to claim 1 or 2, which is a compound of general formula (IVA) or general formula (IVB) or a tautomer, mesomer, racemate, enantiomer, diastereomer thereof, or a mixture thereof, or an oxynitride thereof, or a pharmaceutically acceptable salt thereof, 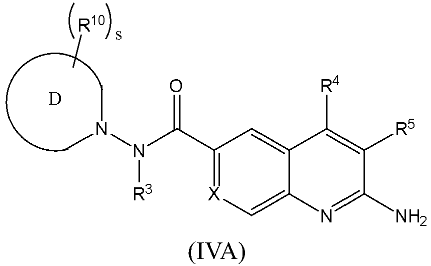 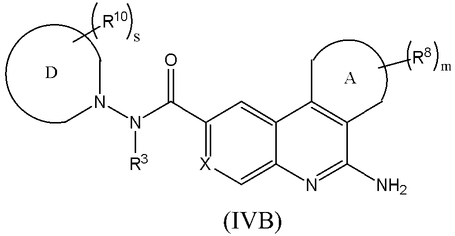 wherein,
ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -S(=O)R^{a}, -P(=O)R^{a}R^{b}, -NR^{a}S(=O)₂R^{b}, oxo, alkyl, alkoxy, haloalkyl, haloalkoxy, alkenyl, alkynyl, cycloalkyl, heterocyclyl, aryl and heteroaryl;
s is an integer from 0 to 4;
R³ is selected from the group consisting of hydrogen, -(CH₂)ₚ-R⁷, -NR^{a}R^{b}, -C(=O)R^{a}, -S(=O)₂R^{a}, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are optionally further substituted by one or more groups selected from the group consisting of deuterium, halogen, amino, nitro, cyano, hydroxy, thiol, carboxyl, -C(=O)OR^{a}, -C(=O)R^{a}, -C(=O)NR^{a}R^{b}, -OC(=O)R^{a}, -NR^{a}C(=O)R^{b}, -S(=O)₂R^{a}, -NR^{a}S(=O)₂R^{b}, -S(=O)₂NR^{a}R^{b}, -S(=O)R^{a}, -P(=O)R^{a}R^{b}, -NR^{a}S(=O)R^{b}, alkyl, -OR^{a}, haloalkyl, haloalkoxy, alkenyl, alkynyl, cycloalkyl, heterocyclyl, aryl and heteroaryl;
X, R⁴, R⁵, R⁷, R^{a}, R^{b} and p are as defined in claim 1;
ring A, R⁸ and m are as defined in claim 2;
preferably, ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, oxo, C₁₋₆ alkyl, C₁₋₆ alkoxy, C₁₋₆ haloalkyl and C₁₋₆ haloalkoxy;
s is an integer from 0 to 4;
R³ is selected from the group consisting of hydrogen, -(CH₂)ₚ-R⁷, C₁₋₆ alkyl, C₃₋₆ cycloalkyl and 4 to 10 membered heterocyclyl; R⁷ is selected from the group consisting of C₆₋₁₀ aryl, 5 to 10 membered heteroaryl, C₃₋₆ cycloalkyl and 4 to 10 membered heterocyclyl, and the C₆₋₁₀ aryl, 5 to 10 membered heteroaryl, C₃₋₆ cycloalkyl and 4 to 10 membered heterocyclyl are optionally further substituted by one or more groups selected from the group consisting of deuterium, halogen, -NR^{a}R^{b}, cyano, hydroxy, thiol, oxo, -C(=O)R^{a}, -P(=O)R^{a}R^{b}, C₁₋₆ alkyl, C₁₋₆ alkoxy, C₁₋₆ haloalkyl, C₁₋₆ haloalkoxy, C₂₋₆ alkenyl, C₂₋₆ alkynyl, and the C₁₋₆ alkyl is optionally further substituted by one or more groups selected from the group consisting of deuterium, halogen, cyano, hydroxy, -NR^{a}R^{b}, -(CH₂)_{q}-OR^{a}, C₁₋₆ alkyl, C₁₋₆ alkoxy, C₁₋₆ haloalkyl and C₁₋₆ haloalkoxy;
R^{a} and R^{b} are each independently selected from the group consisting of hydrogen, C₁₋₆ alkyl and C₃₋₆ cycloalkyl, and the C₁₋₆ alkyl is optionally substituted by halogen, hydroxy, C₁₋₆ alkoxy, -NR^{c}R^{d}; or
R^{a} and R^{b}, together with the atoms to which they are attached, form a 4 to 6 membered heterocyclyl, and the 4 to 6 membered heterocyclyl is optionally substituted by halogen, oxo , C₁₋₆ alkyl;
R^{c} and R^{d} are each independently selected from the group consisting of hydrogen, C₁₋₆ alkyl and hydroxy substituted C₁₋₆ alkyl;
p is 1;
q is 1 or 2;
X, R⁴ and R⁵ are as defined in claim 1;
ring A, R⁸ and m are as defined in claim 2;
more preferably, ring D is selected from the group consisting of 5 to 10 membered nitrogen-containing heterocyclyl and 5 to 10 membered nitrogen-containing heteroaryl;
each R¹⁰ is independently selected from the group consisting of hydrogen, halogen, -NR^{a}R^{b}, nitro, cyano, hydroxy, thiol, carboxyl, oxo, C₁₋₆ alkyl, C₁₋₆ alkoxy, C₁₋₆ haloalkyl and C₁₋₆ haloalkoxy;
s is an integer from 0 to 4;
R³ is selected from the group consisting of hydrogen, -(CH₂)ₚ-R⁷ and C₁₋₆ alkyl; R⁷ is selected from the group consisting of C₆₋₁₀ aryl and 5 to 10 membered heteroaryl, and the C₆₋₁₀ aryl or 5 to 10 membered heteroaryl is optionally further substituted by one or more groups selected from the group consisting of deuterium, halogen, cyano, hydroxy, oxo, -C₁₋₆ alkyl, C₁₋₆ alkoxy, C₁₋₆ haloalkyl, C₁₋₆ haloalkoxy, C₂₋₆ alkenyl and C₂₋₆ alkynyl;
p is 1;
X, R⁴ and R⁵ are as defined in claim 1;
ring A, R⁸ and m are as defined in claim 2.

26. The compound of general formula (I) or a tautomer, mesomer, racemate, enantiomer, diastereomer thereof, or a mixture thereof, or an oxynitride thereof, or a pharmaceutically acceptable salt thereof according to claim 25, wherein ring D is selected from the group consisting of 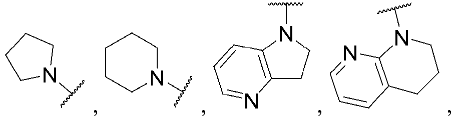 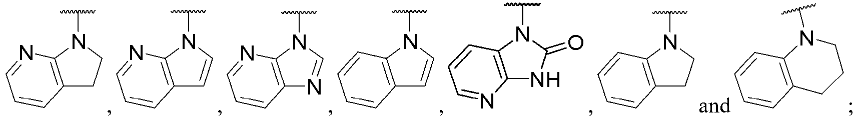
each R¹⁰ is independently selected from the group consisting of hydrogen, halogen, C₁₋₆ alkyl and C₁₋₆ haloalkyl;
s is 0 or 1.

27. The compound of general formula (I) or a tautomer, mesomer, racemate, enantiomer, diastereomer thereof, or a mixture thereof, or an oxynitride thereof, or a pharmaceutically acceptable salt thereof according to claim 25 or 26, wherein R³ is selected from the group consisting of hydrogen, -(CH₂)ₚ-R⁷ and C₁₋₆ alkyl;
R⁷ is selected from the group consisting of 5 to 6 membered heteroaryl and phenyl, preferably selected from the group consisting of 6 membered heteroaryl and phenyl, which is optionally further substituted by one or more groups selected from the group consisting of deuterium, halogen, oxo, C₁₋₆ alkyl, C₁₋₆ alkoxy, C₁₋₆ haloalkyl and C₁₋₆ haloalkoxy.

28. The compound of general formula (I) or a tautomer, mesomer, racemate, enantiomer, diastereomer thereof, or a mixture thereof, or a pharmaceutically acceptable salt thereof according to any one of claims 2 to 27, wherein, ring A is selected from the group consisting of 5 to 6 membered heteroaryl and 5 to 6 membered heterocyclyl; preferably, is pyrazolyl or tetrahydrofuranyl; and especially, is 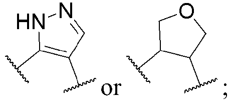
each R⁸ is each independently selected from the group consisting of hydrogen and C₁₋₆ alkyl;
m is 1 or 2, preferably 1.

29. The compound of general formula (I) or a tautomer, mesomer, racemate, enantiomer, diastereomer thereof, or a mixture thereof, or a pharmaceutically acceptable salt thereof according to any one of claims 1 to 28, wherein, X is CR⁶ or N, and R⁶ is selected from the group consisting of hydrogen, halogen, C₁₋₆ alkyl, C₁₋₆ alkoxy, C₁₋₆ haloalkyl, C₁₋₆ haloalkoxy, C₁₋₆ deuterated alkyl, C₁₋₆ deuterated alkoxy and C₃₋₆ cycloalkyl.

30. The compound of general formula (I) or a tautomer, mesomer, racemate, enantiomer, diastereomer thereof, or a mixture thereof, or a pharmaceutically acceptable salt thereof according to any one of claims 1 to 29, wherein, the compound is selected from the group consisting of:

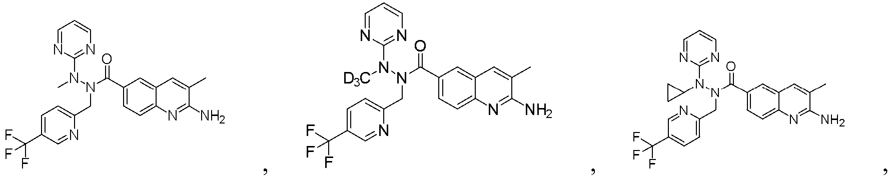

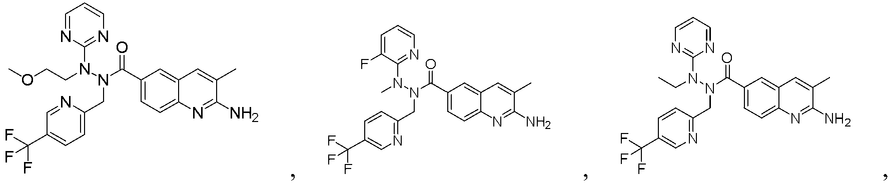

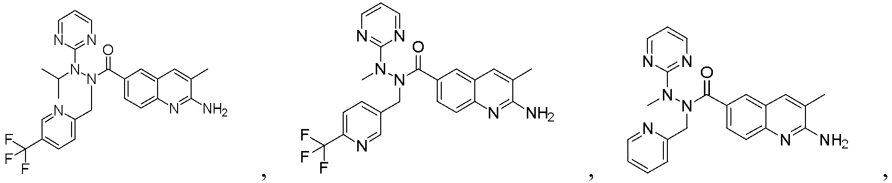

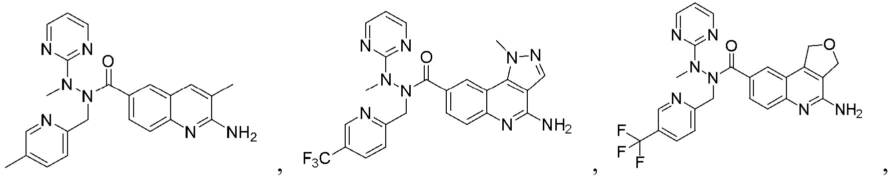

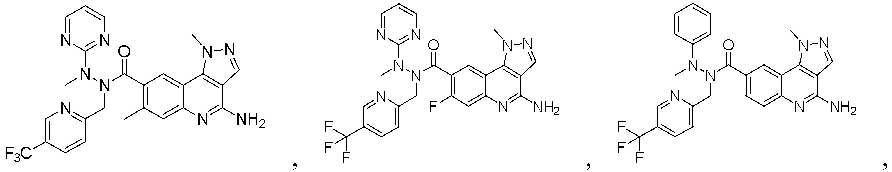

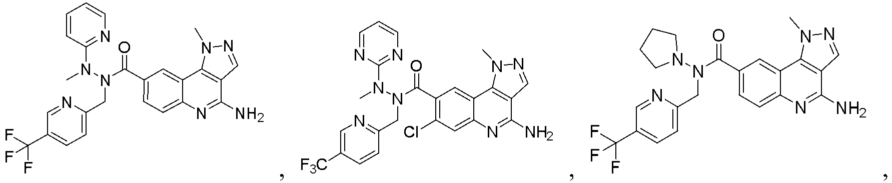

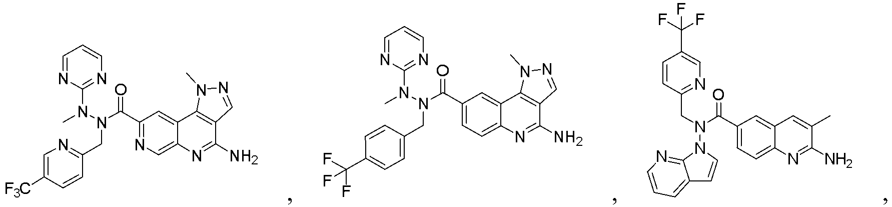

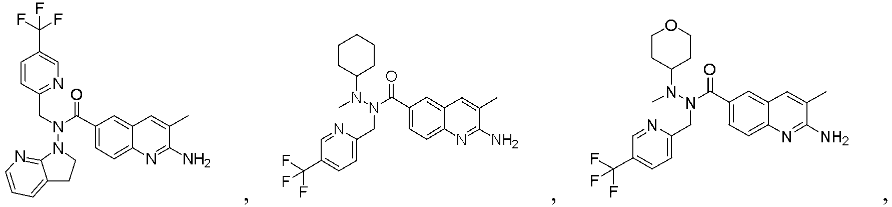

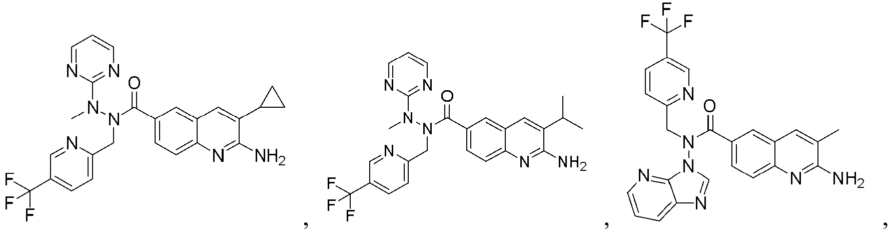

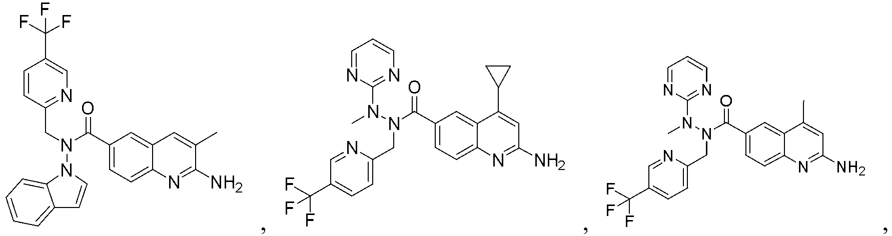

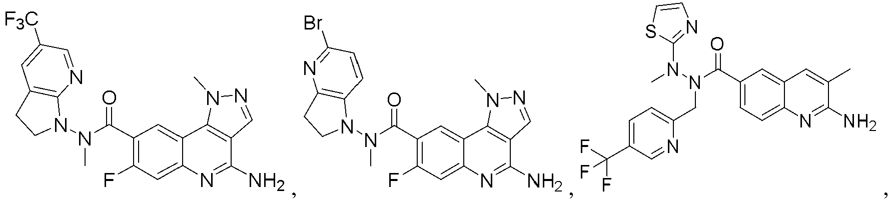

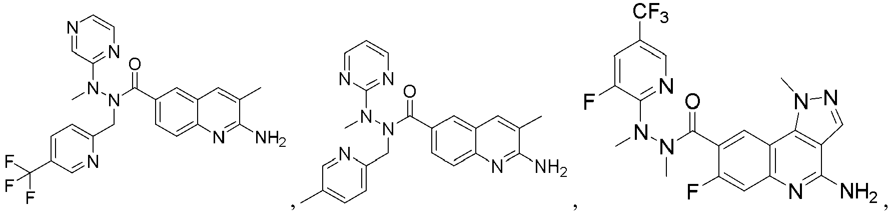

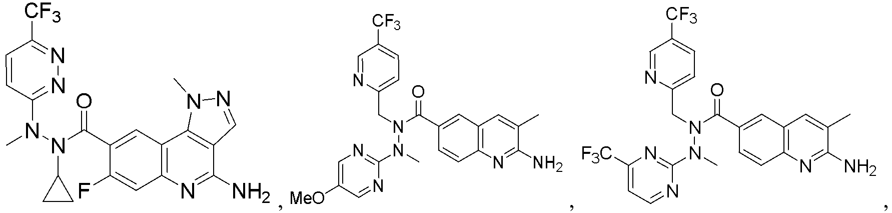

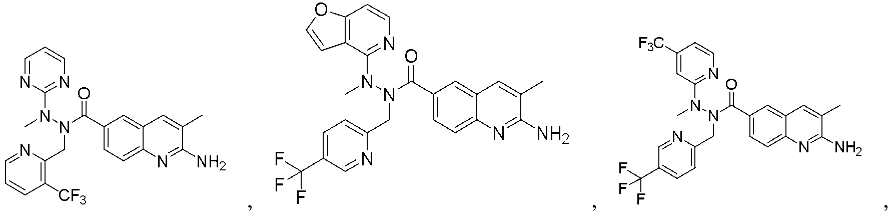

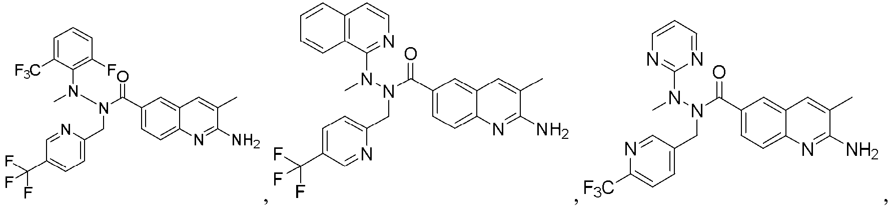

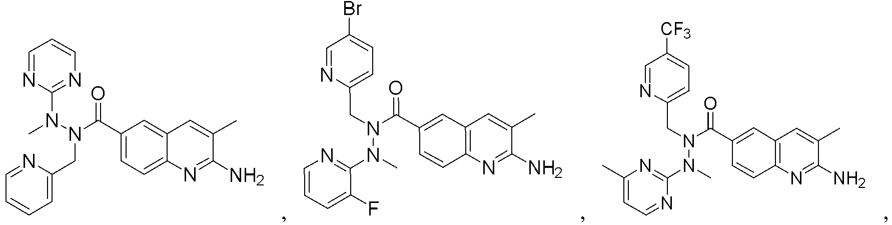

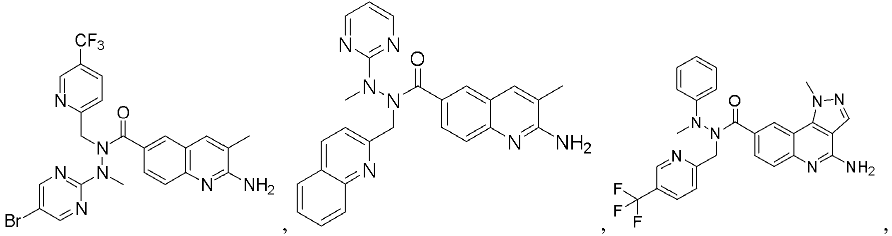

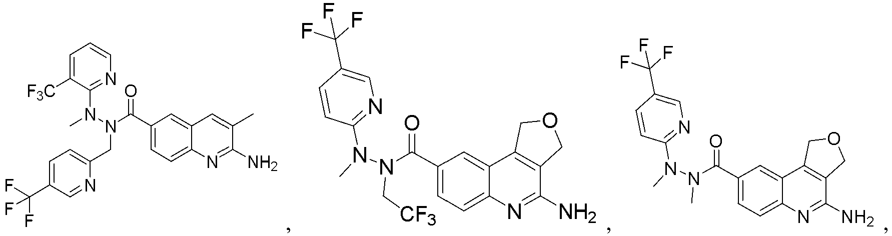

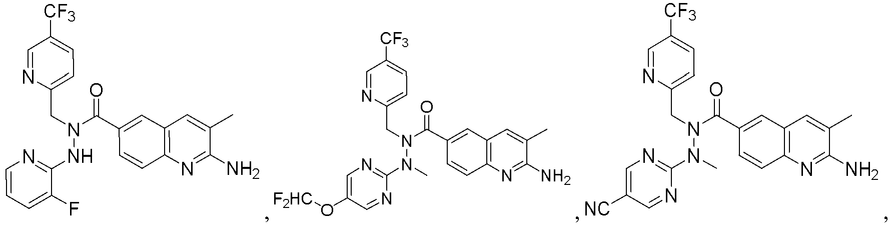

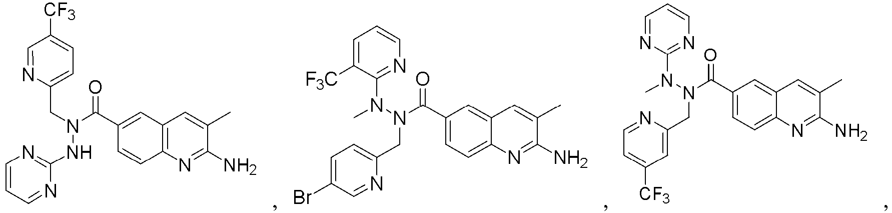

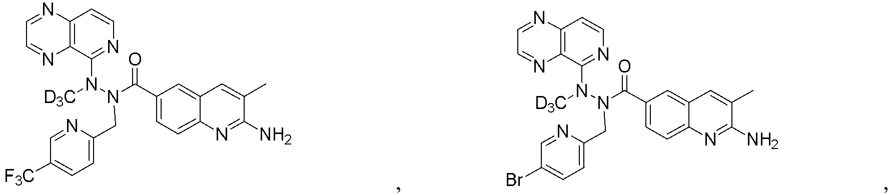

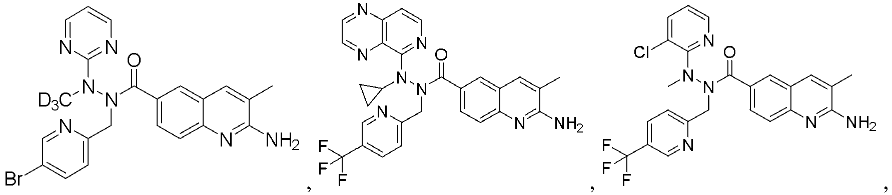

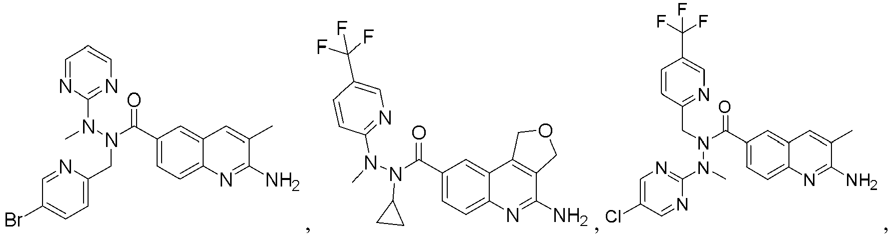

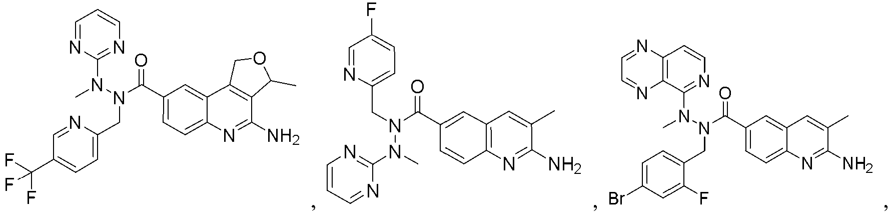

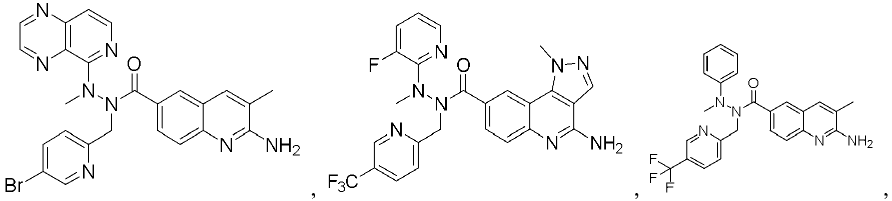

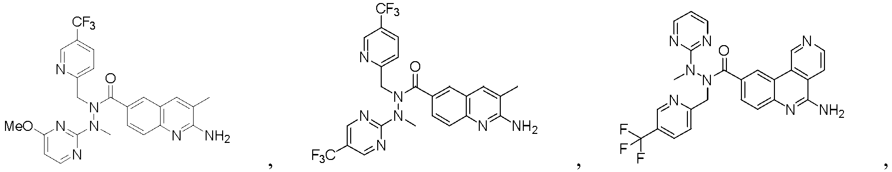

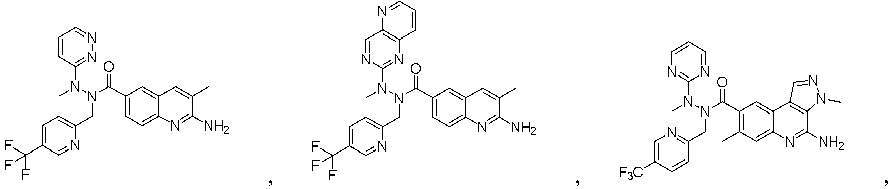

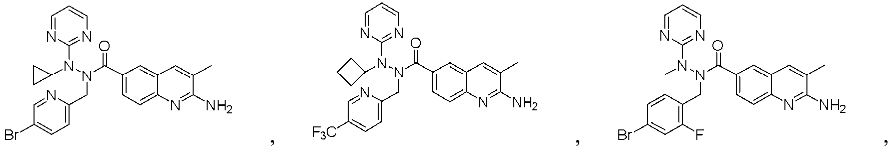

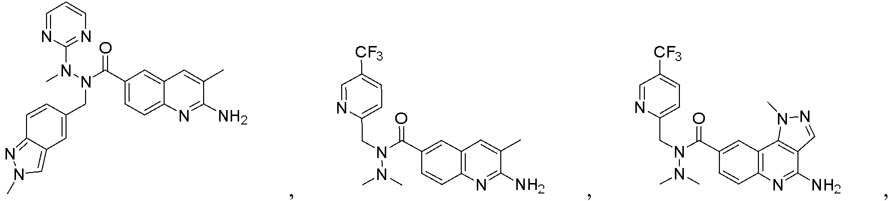

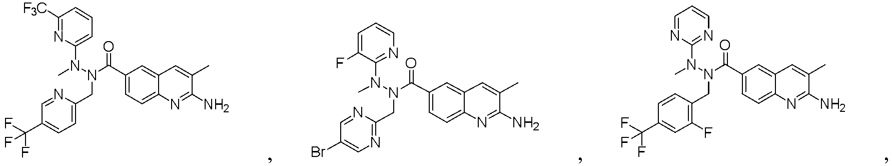

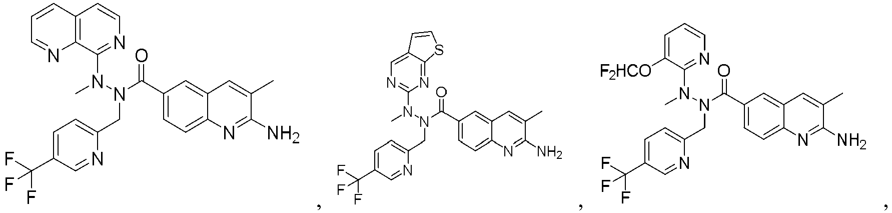

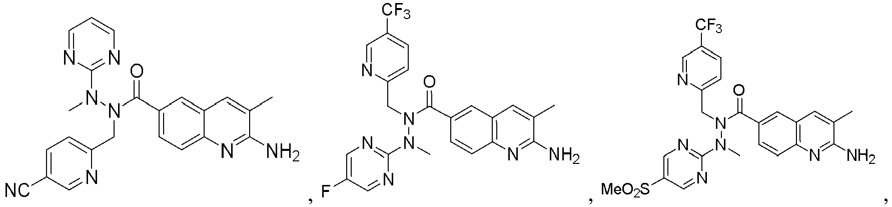

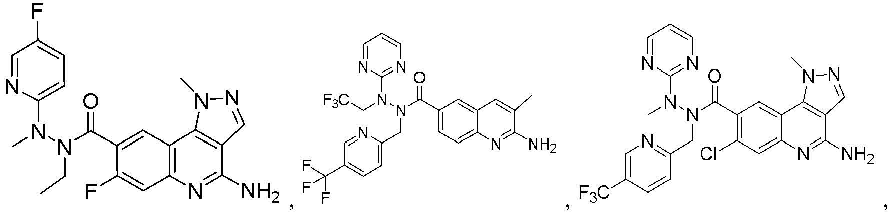

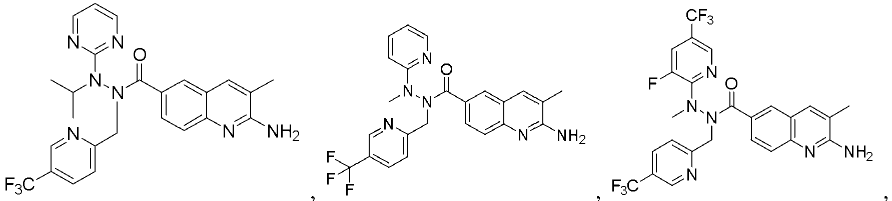

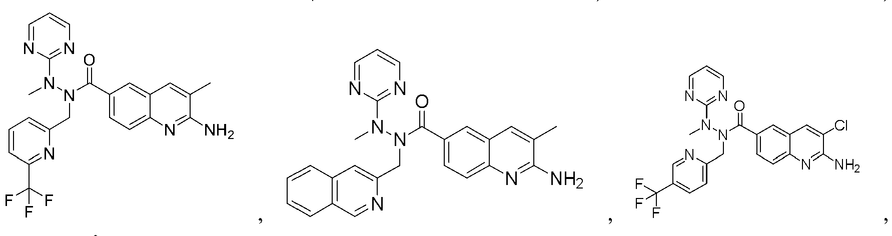

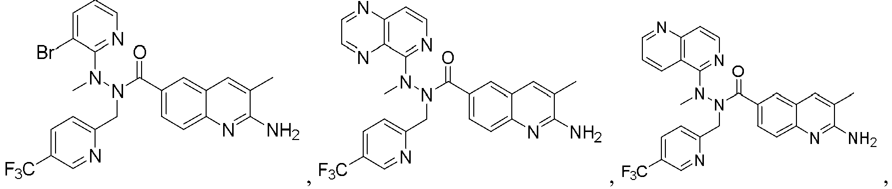

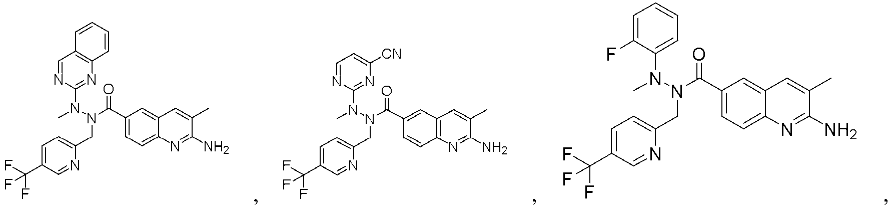

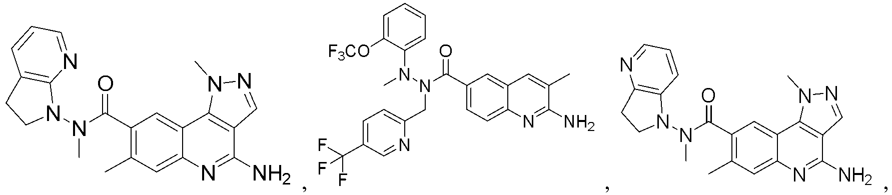

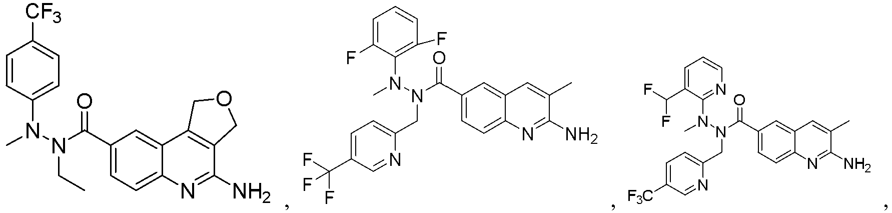

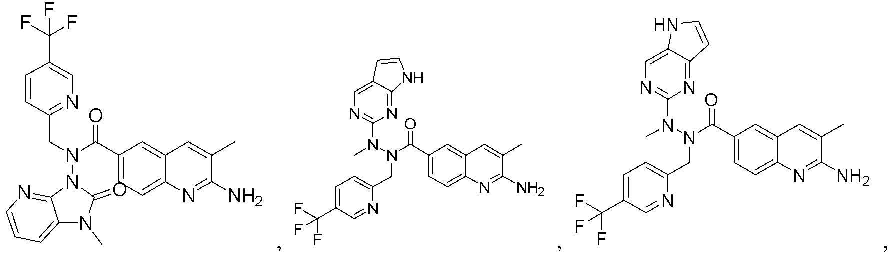

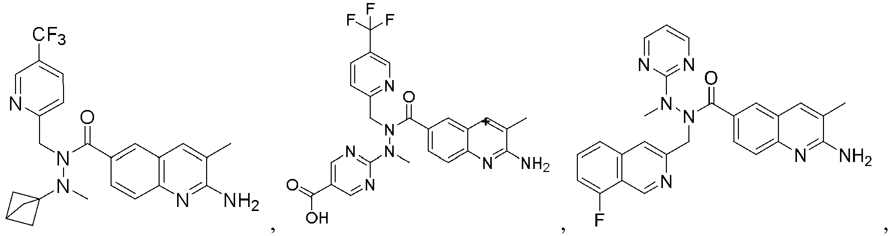

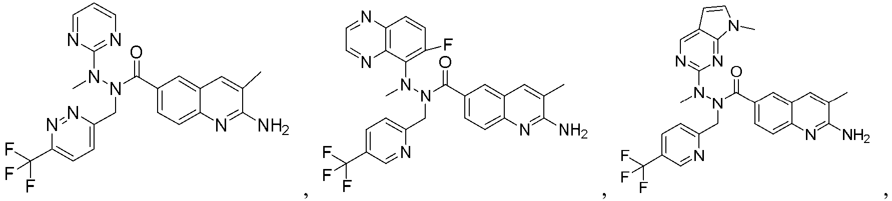

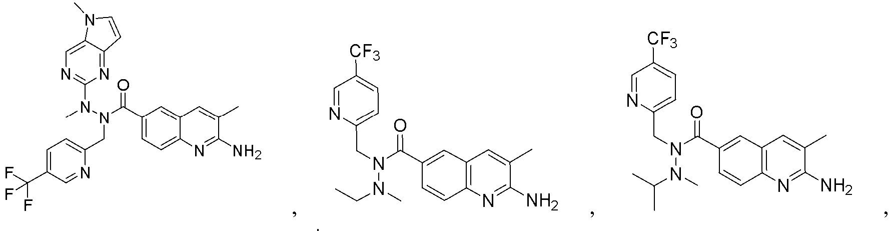

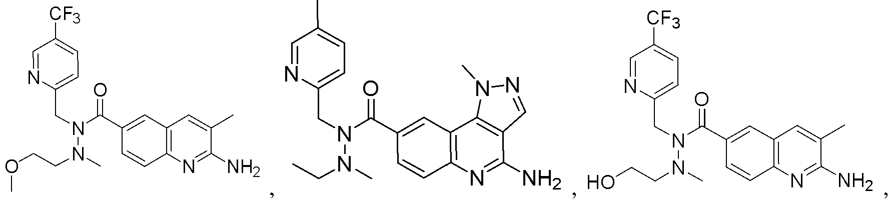

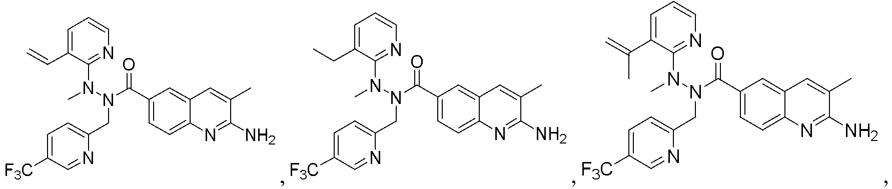

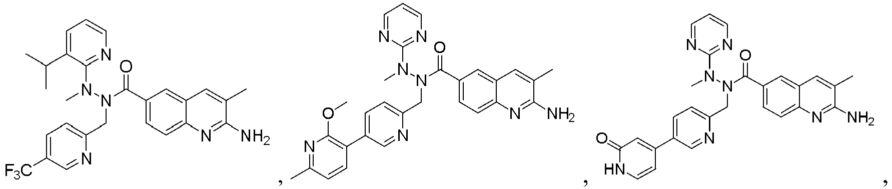

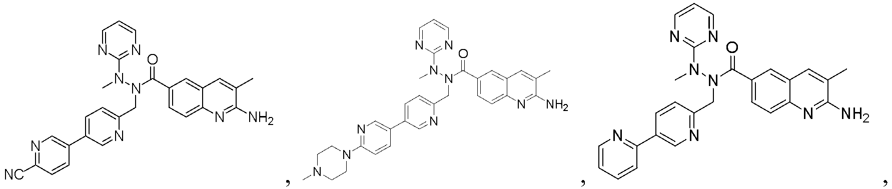

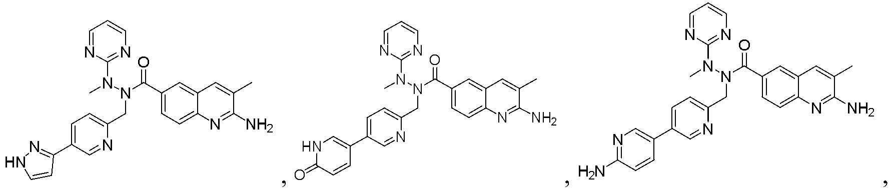

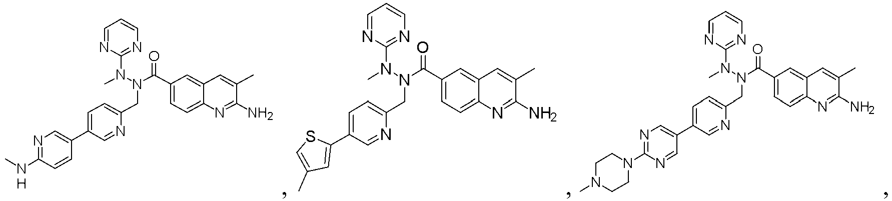

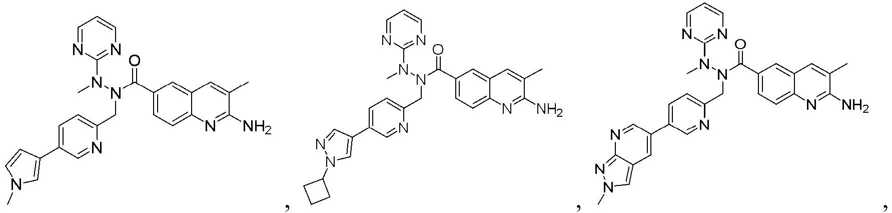

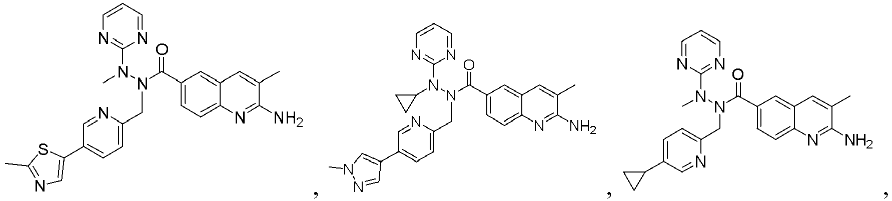

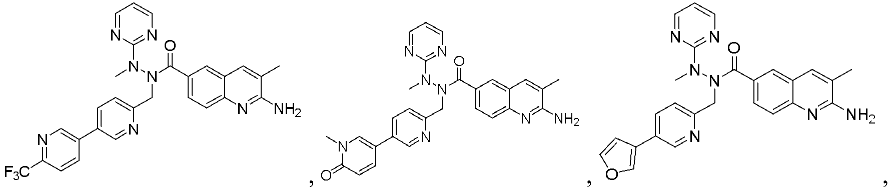

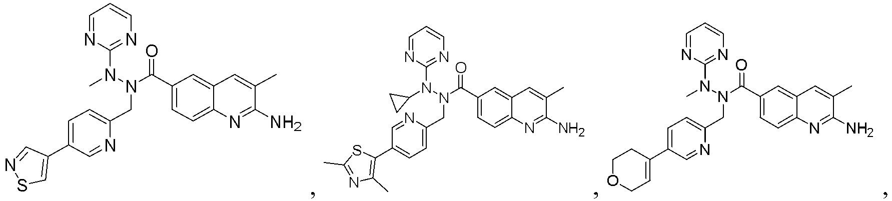

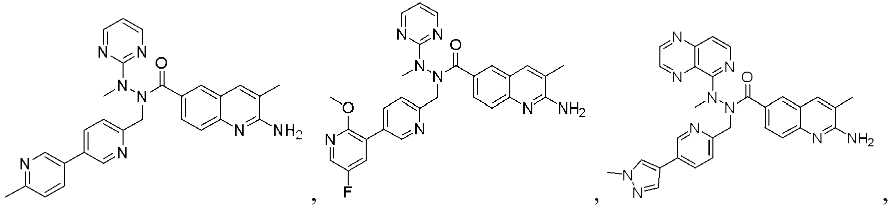

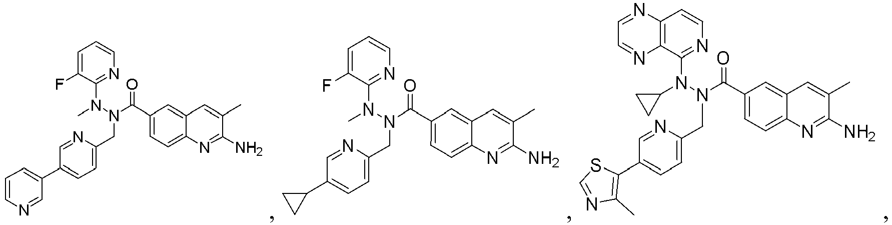

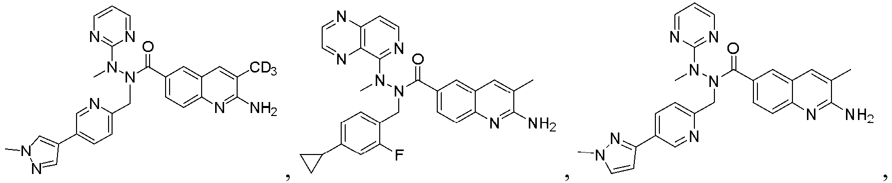

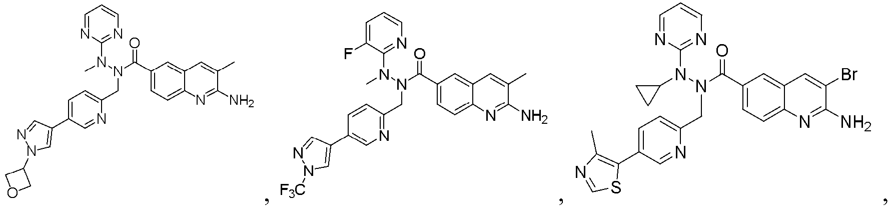

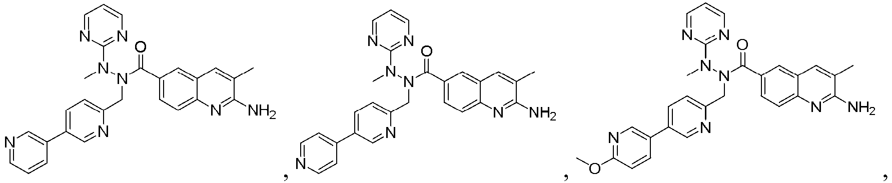

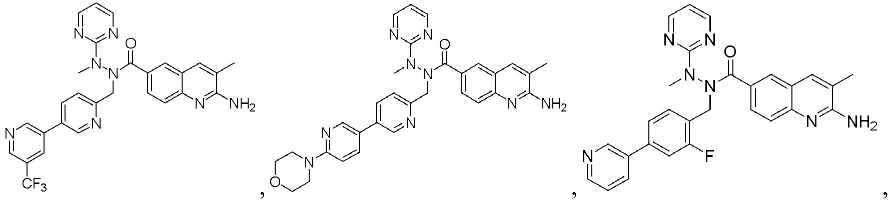

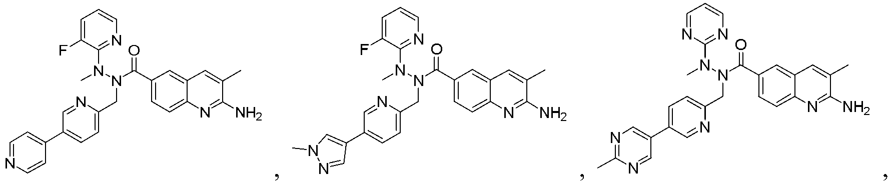

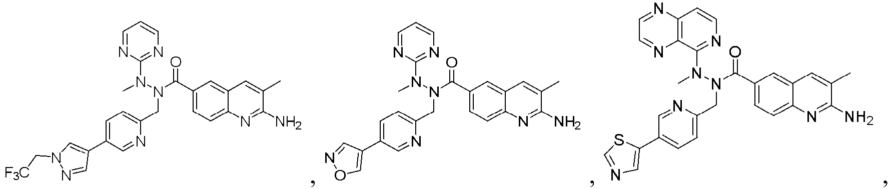

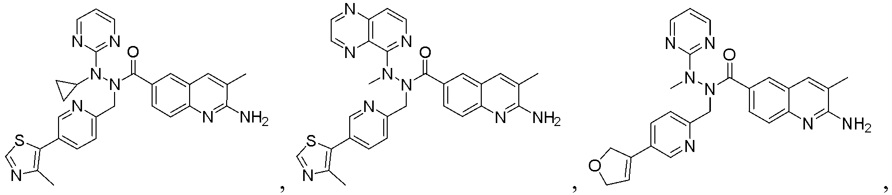

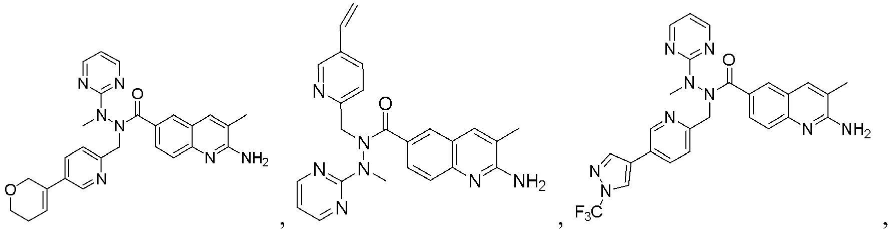

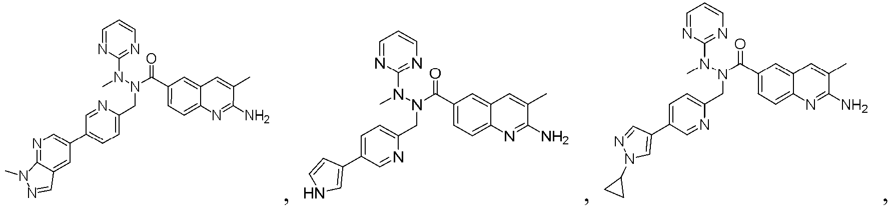

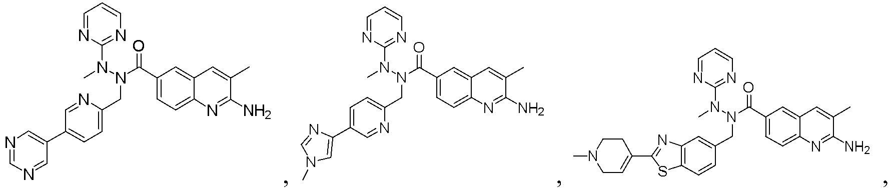

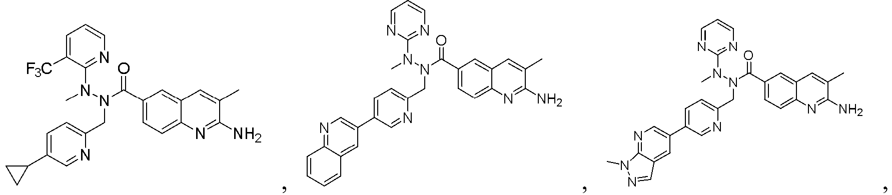

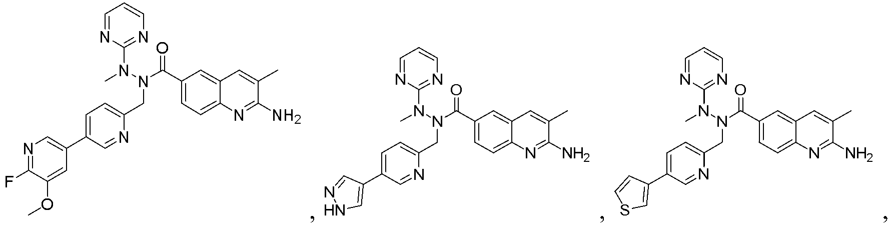

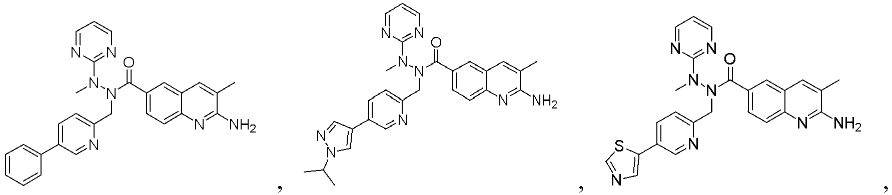

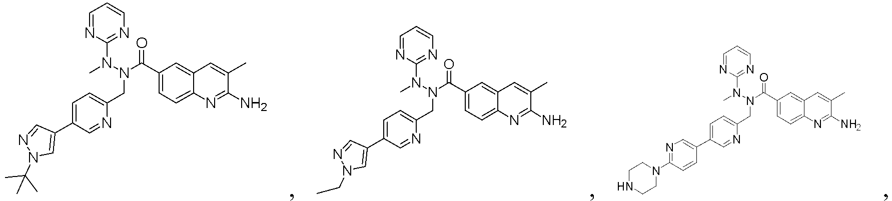

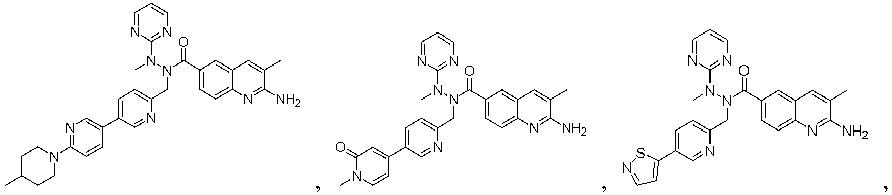

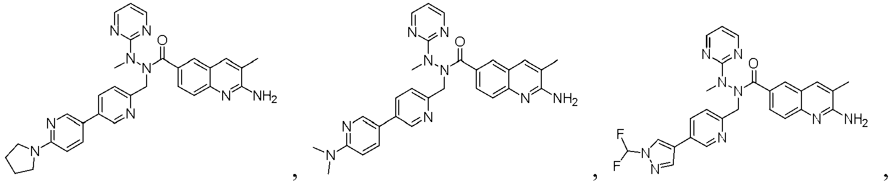

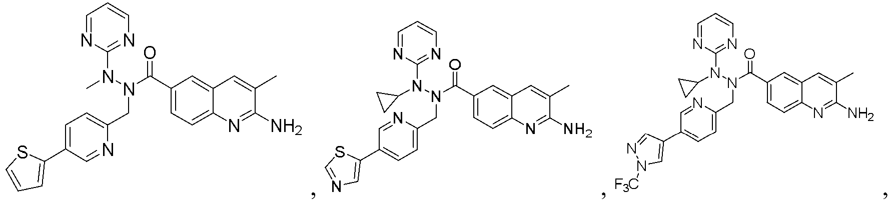

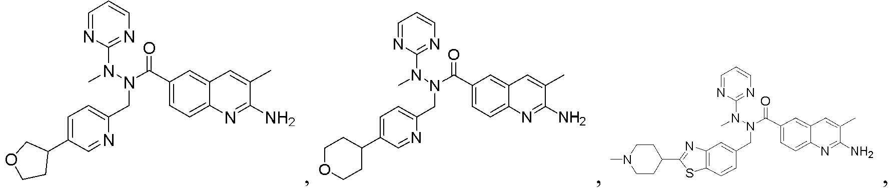

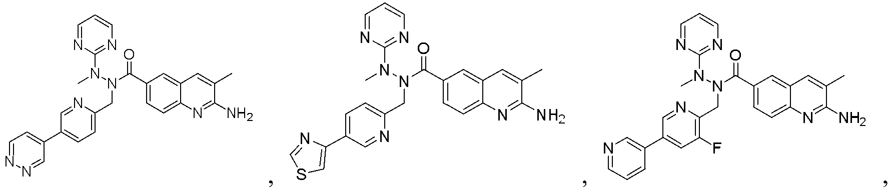

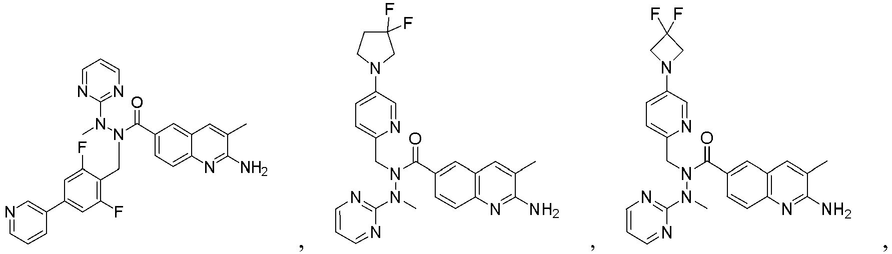

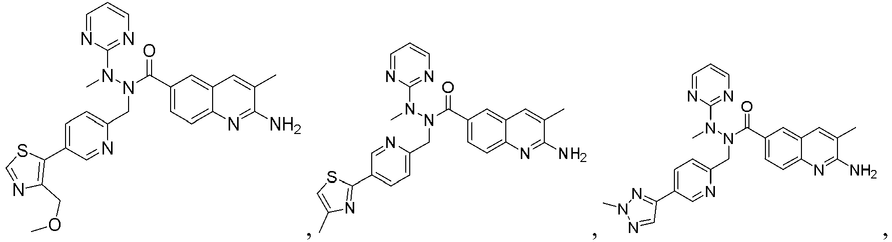

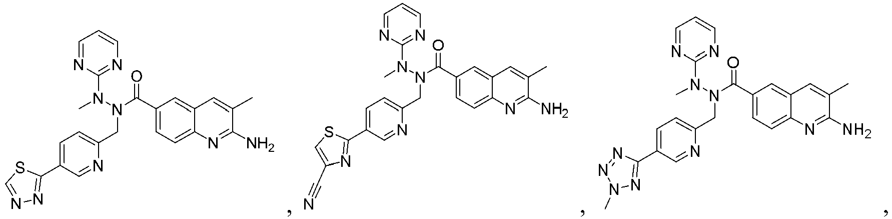

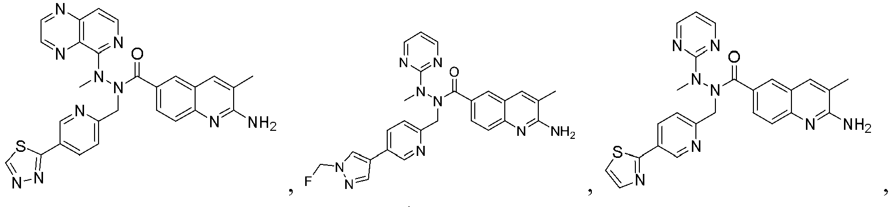

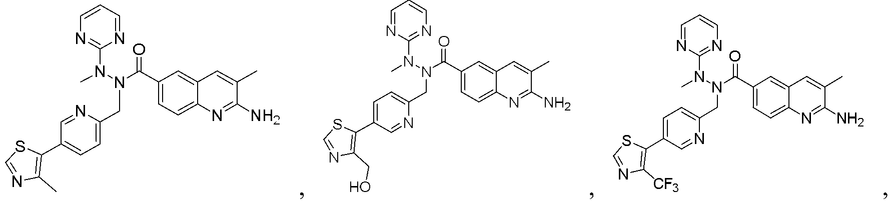

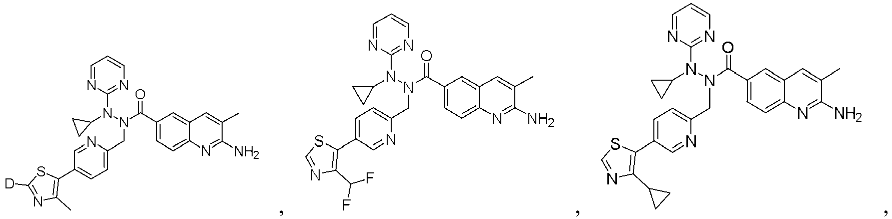

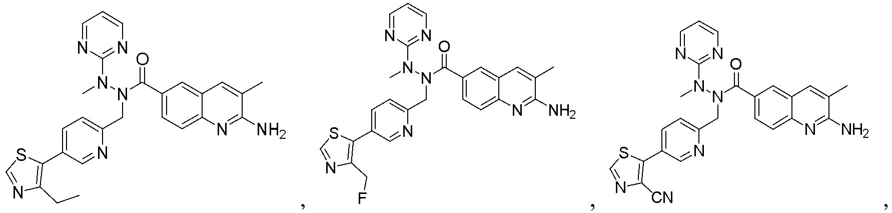

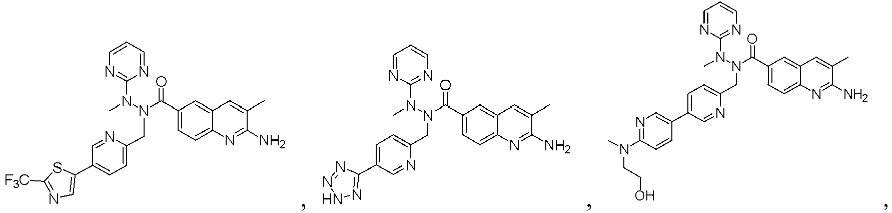

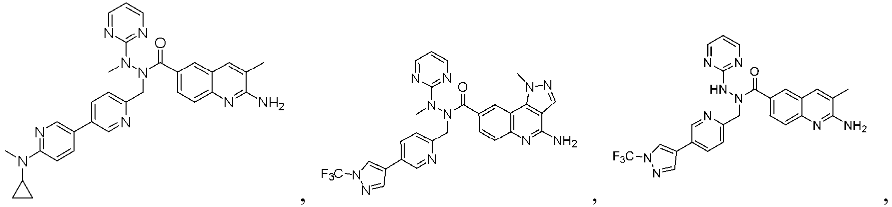

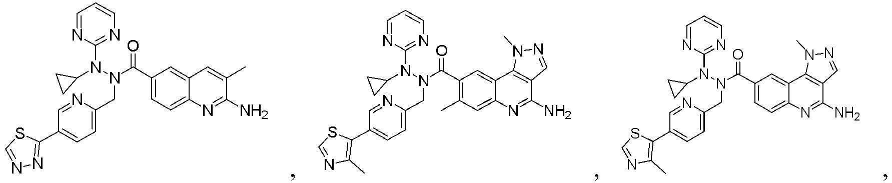

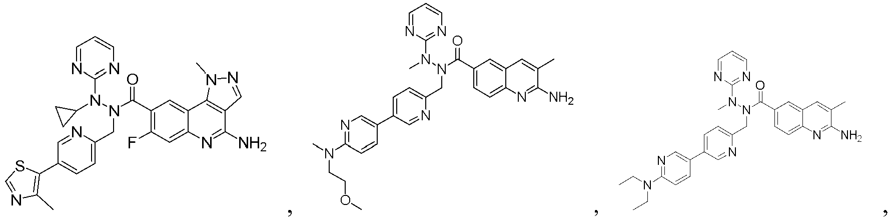

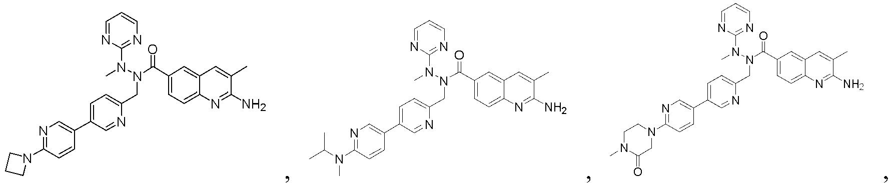

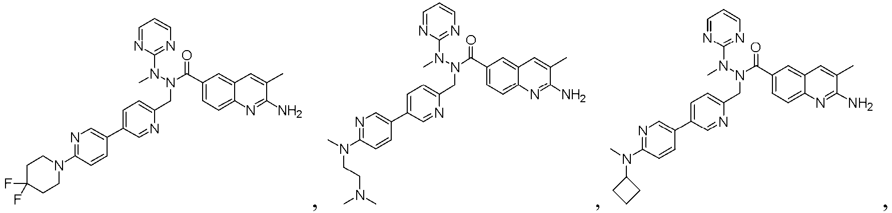

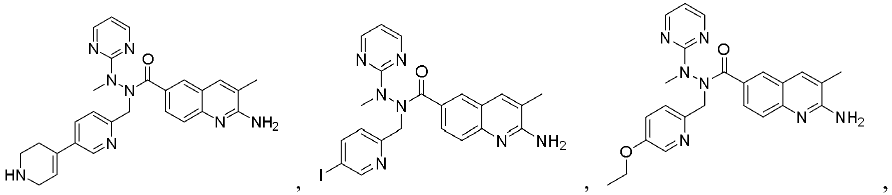

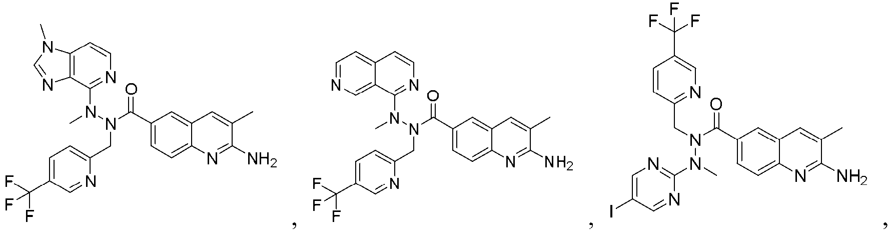

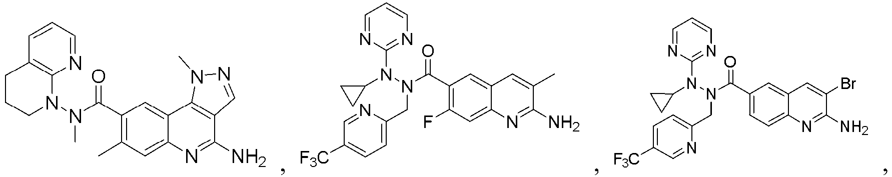

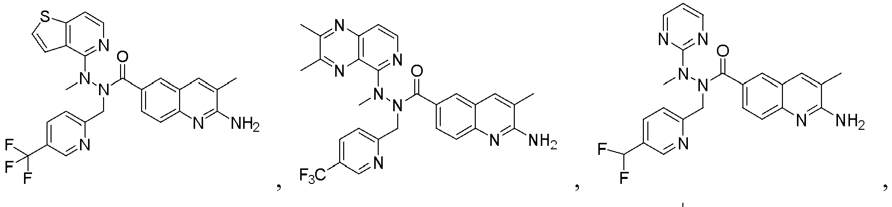

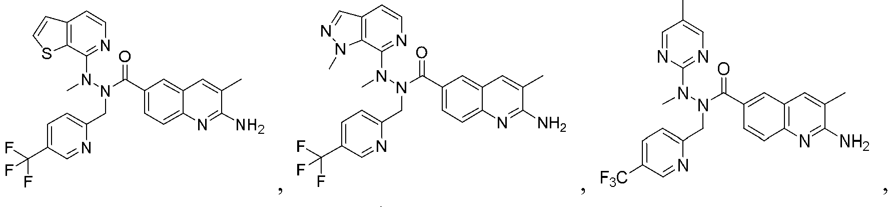

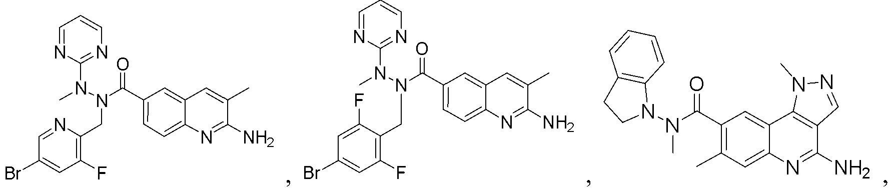

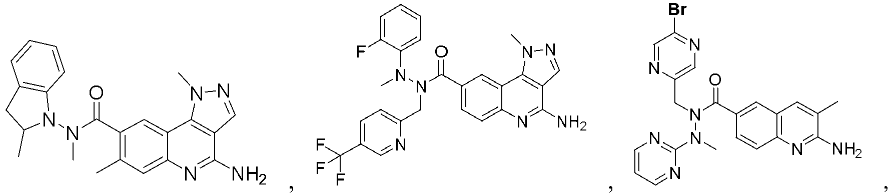

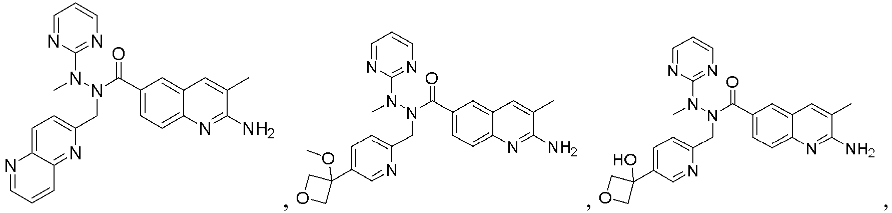

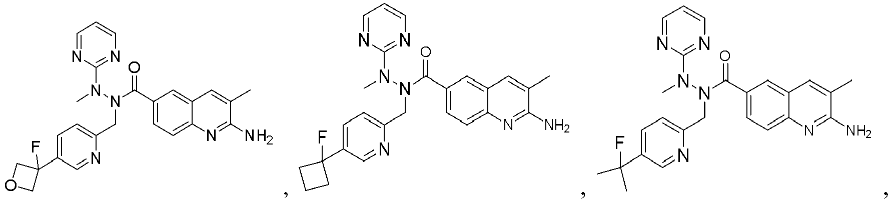

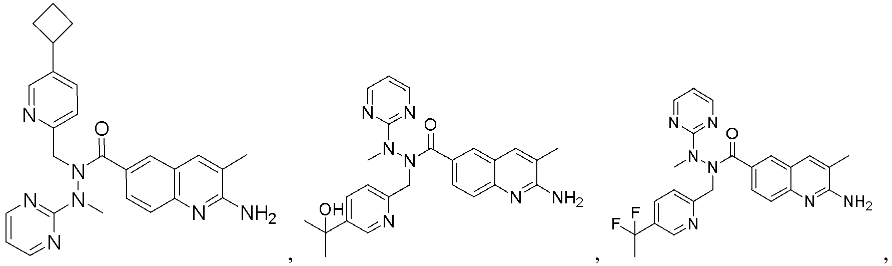

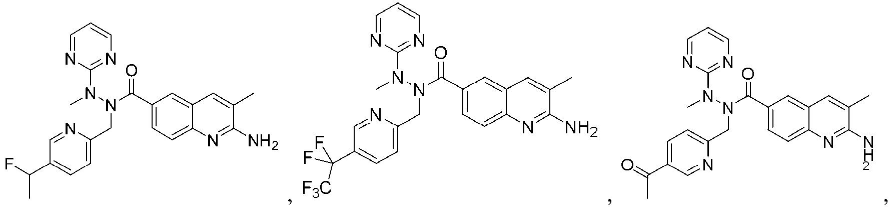

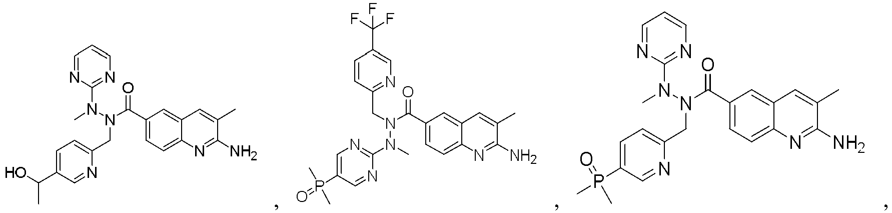

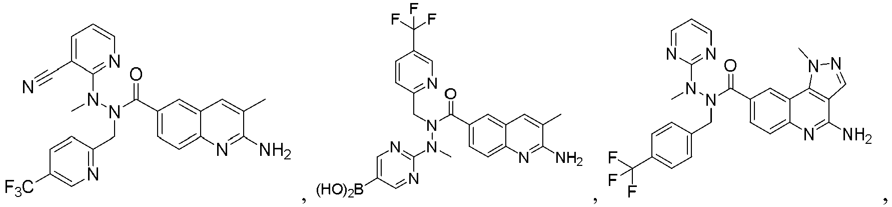

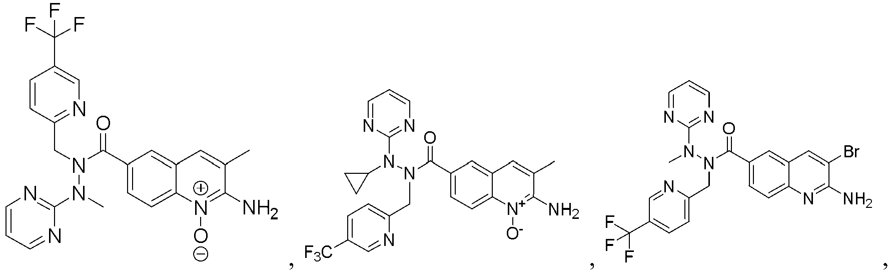

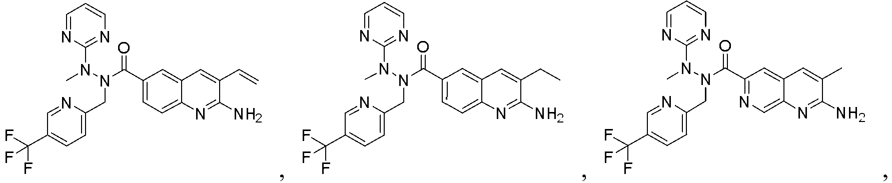

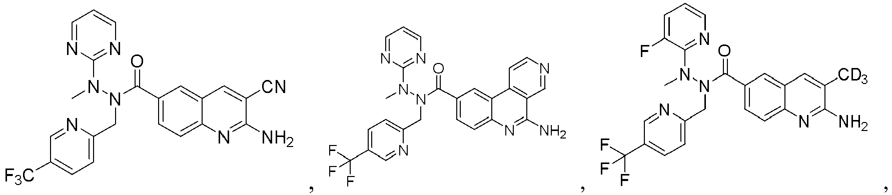

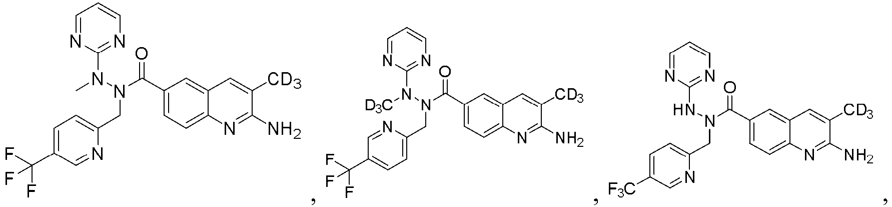

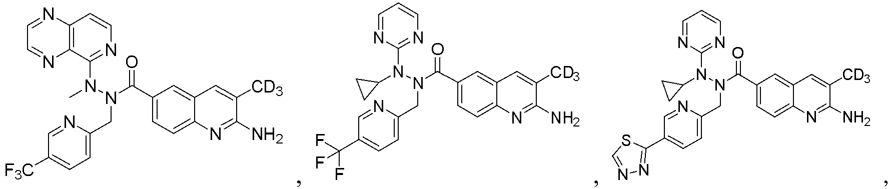

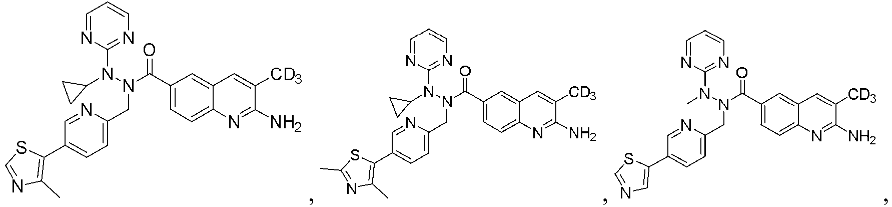

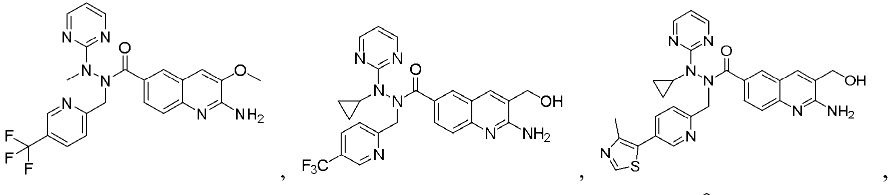

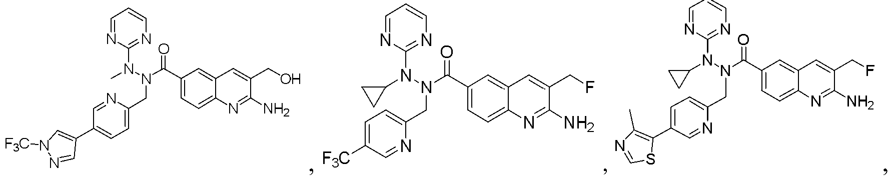

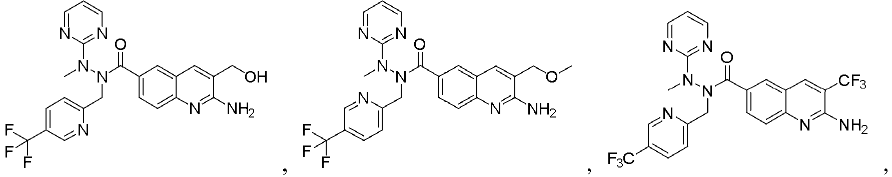

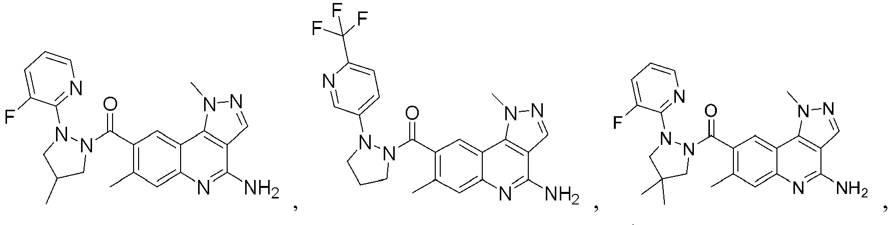

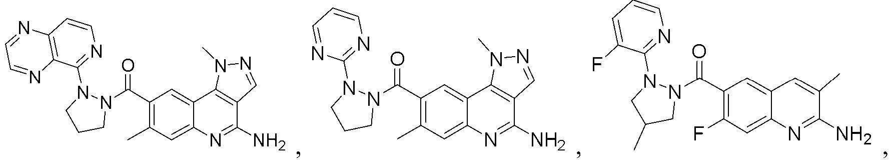

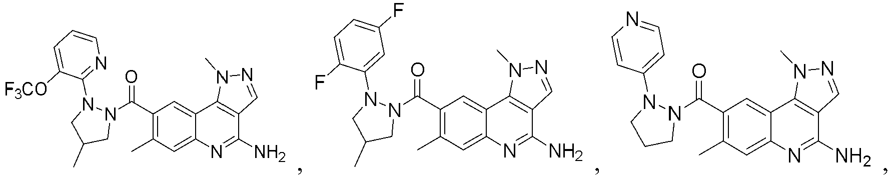

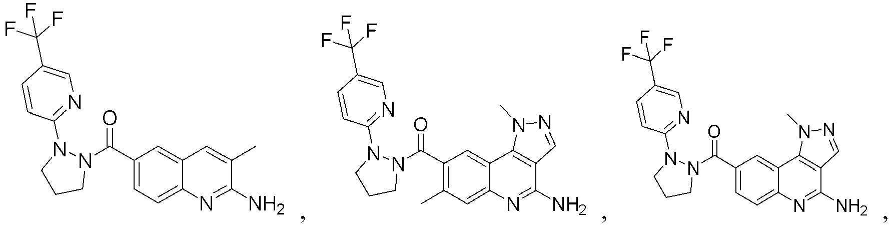

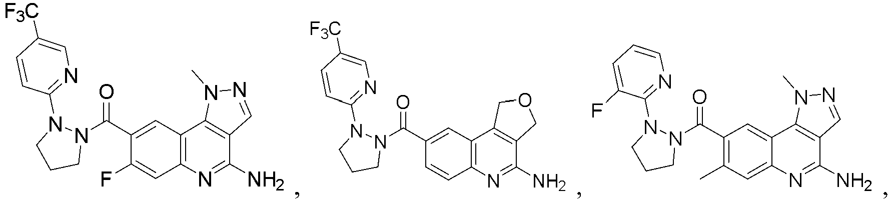

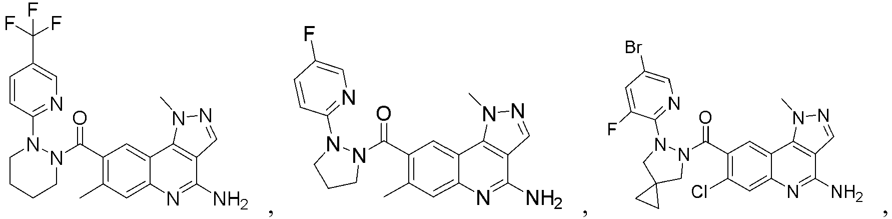

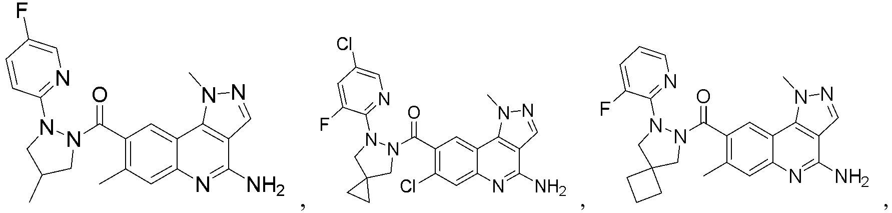

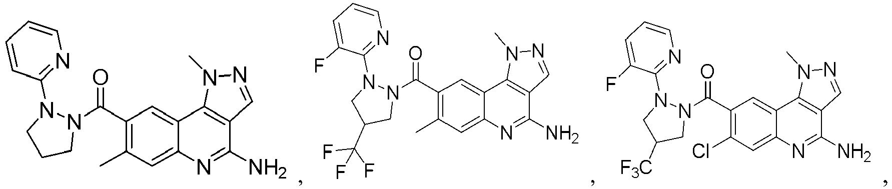

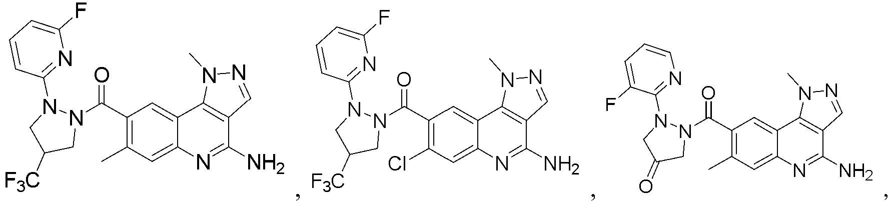

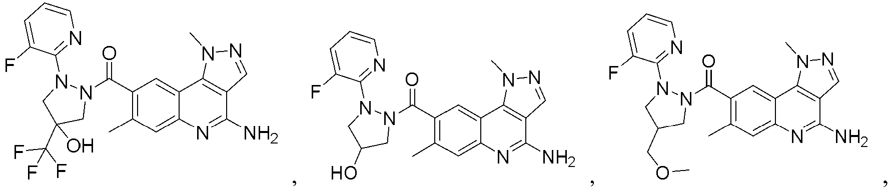

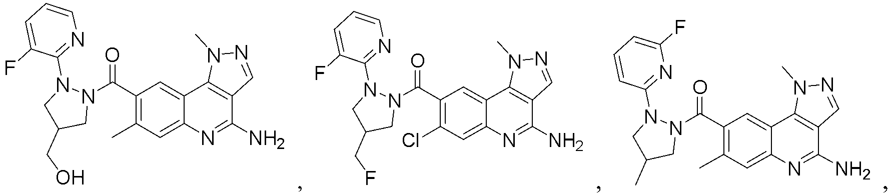

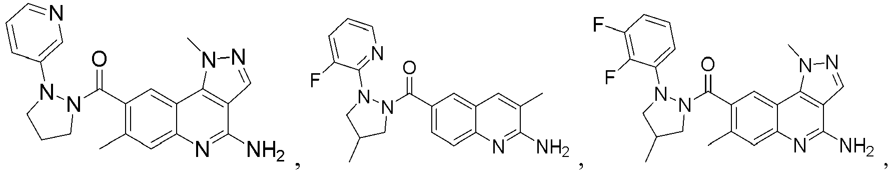

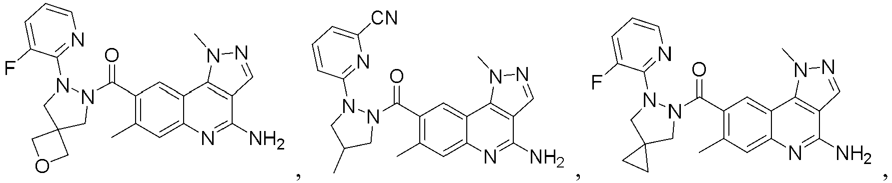

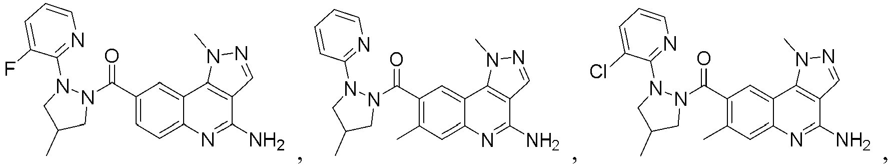

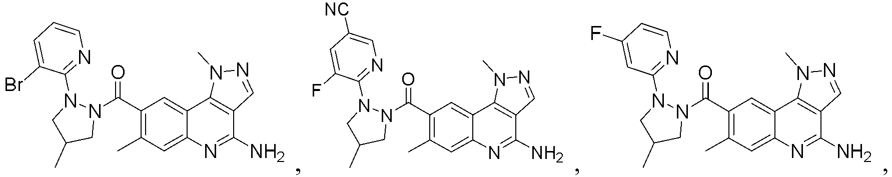

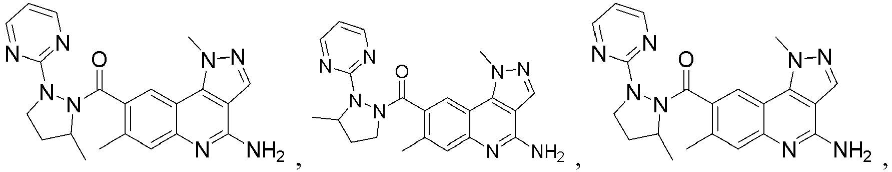

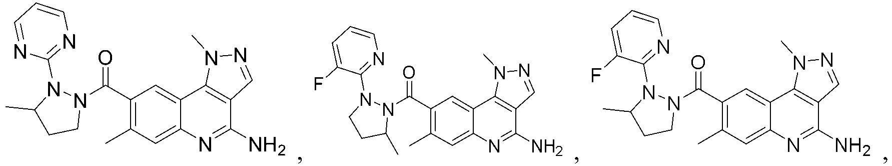

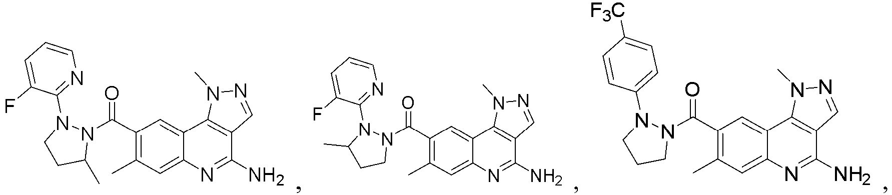

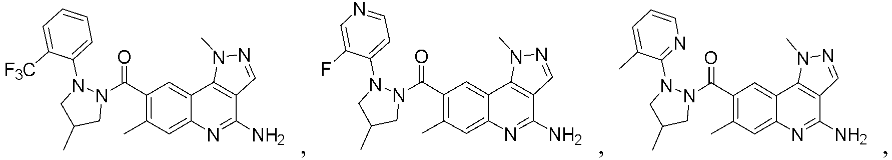

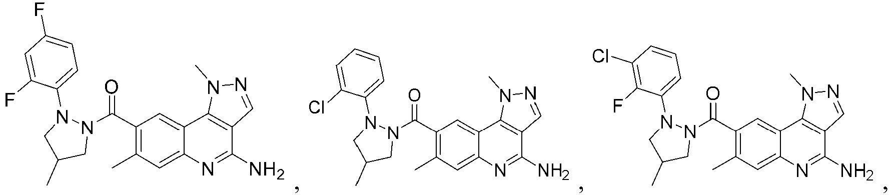

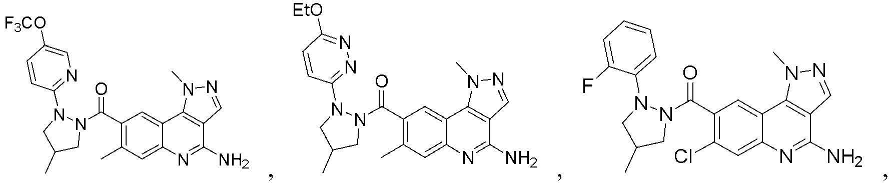

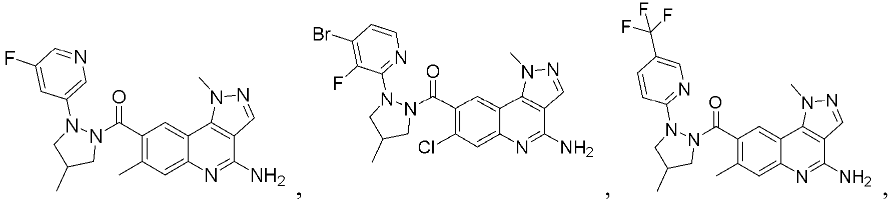

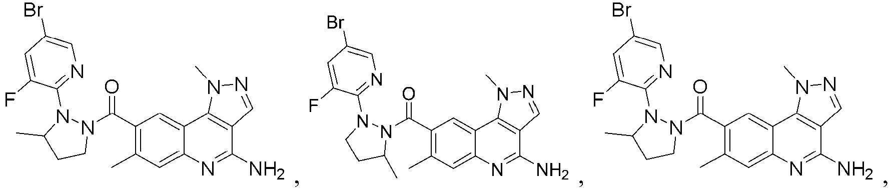

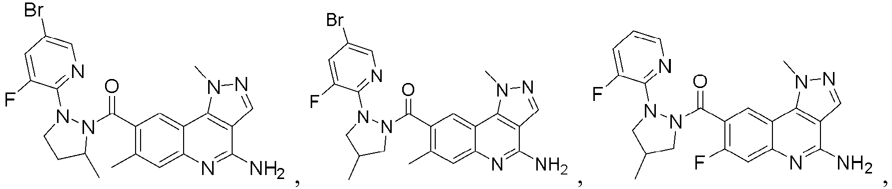

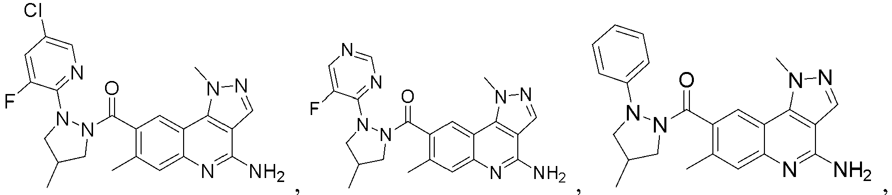

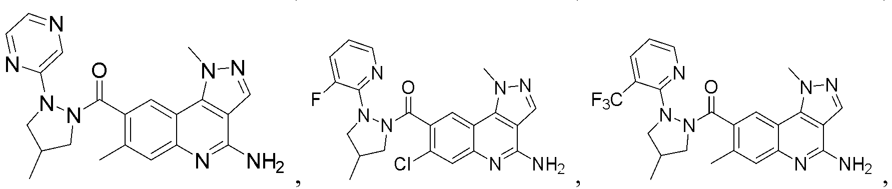

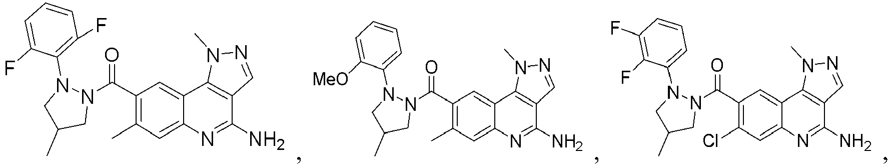

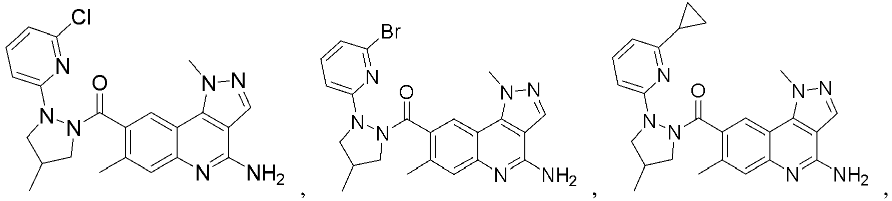

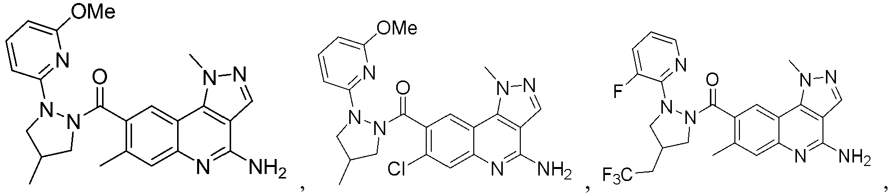

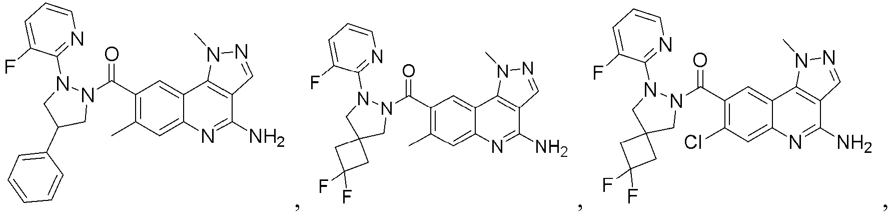

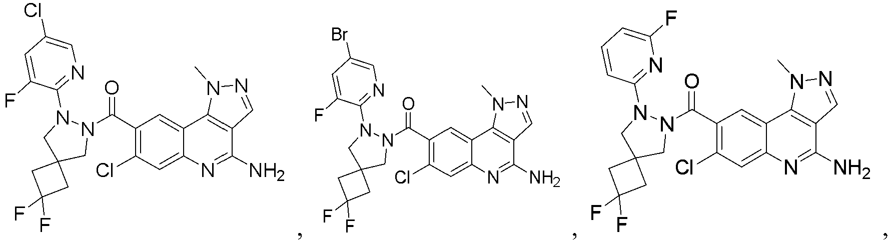

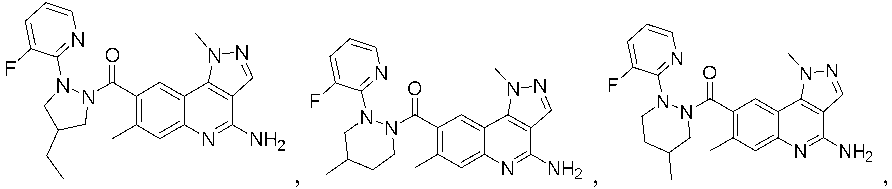

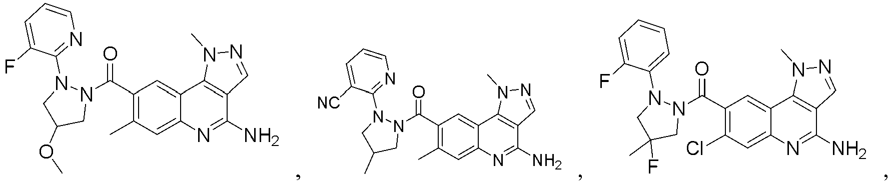

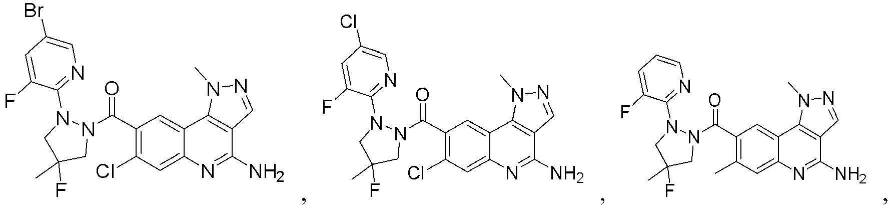

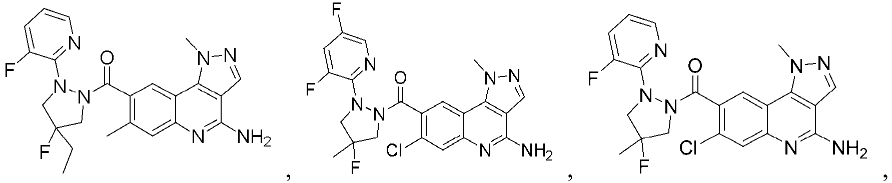

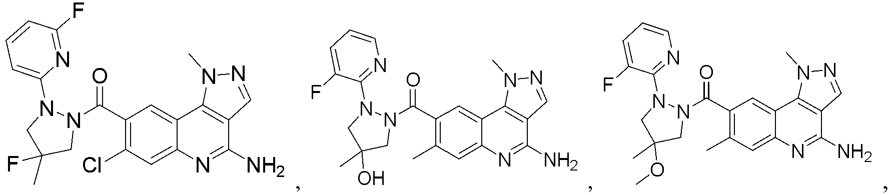

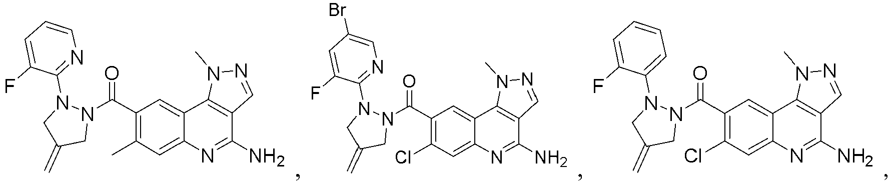

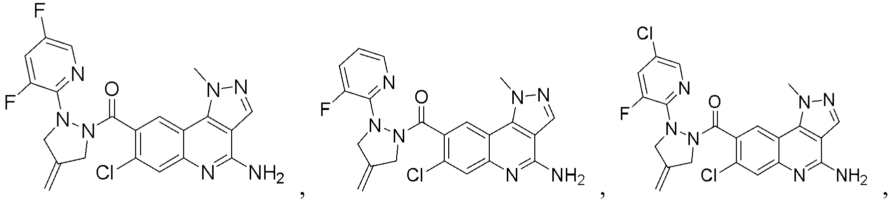

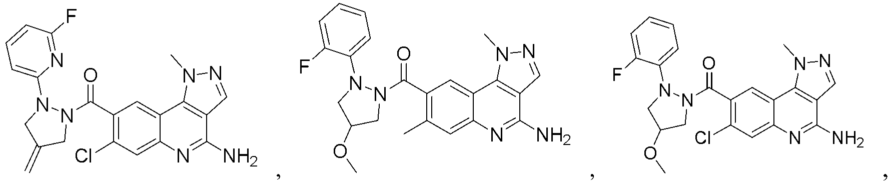

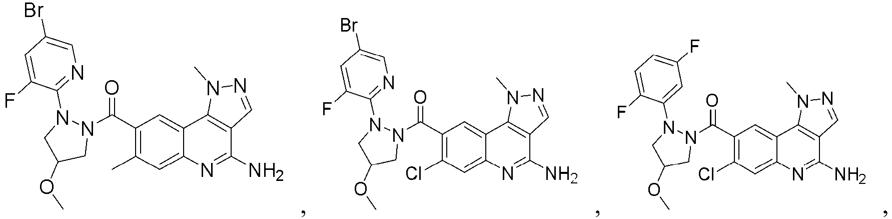

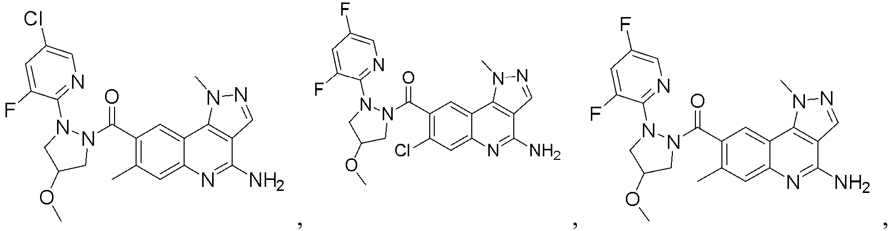

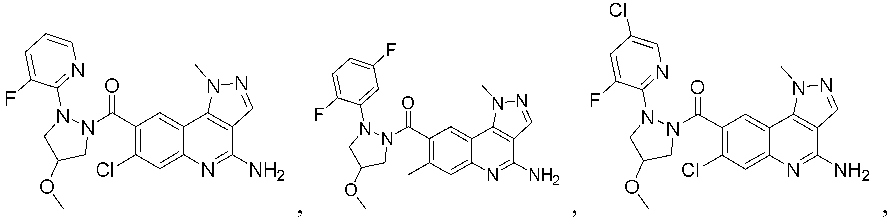

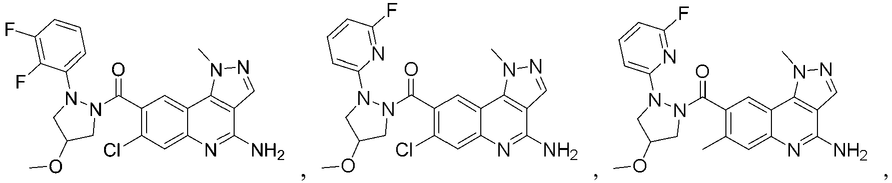

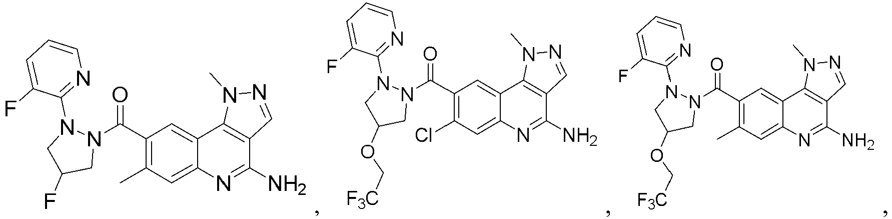

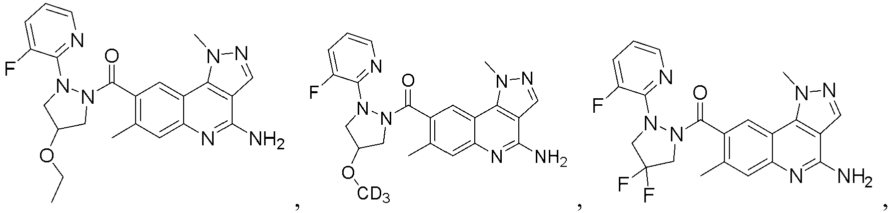

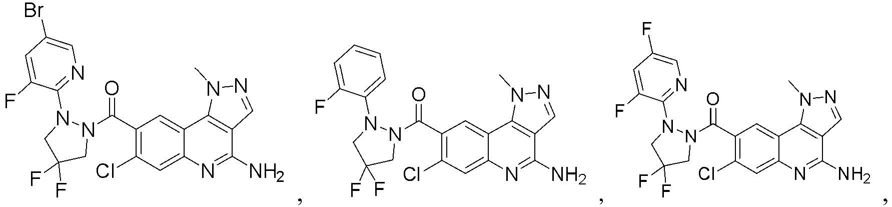

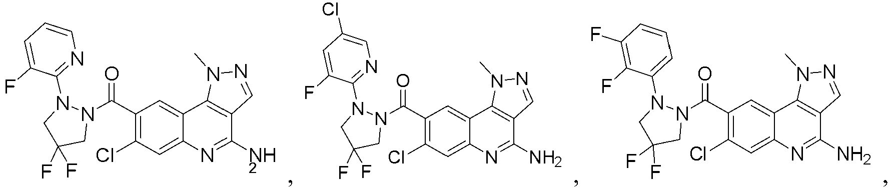

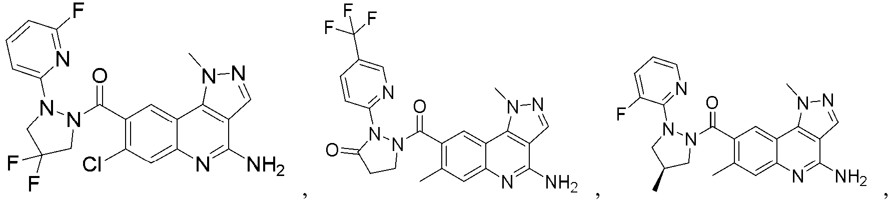

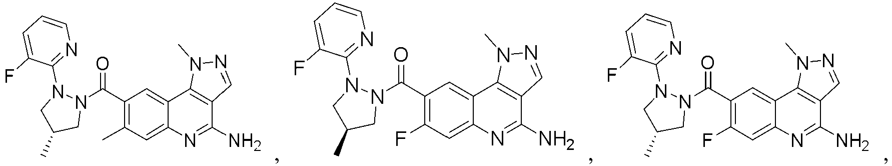

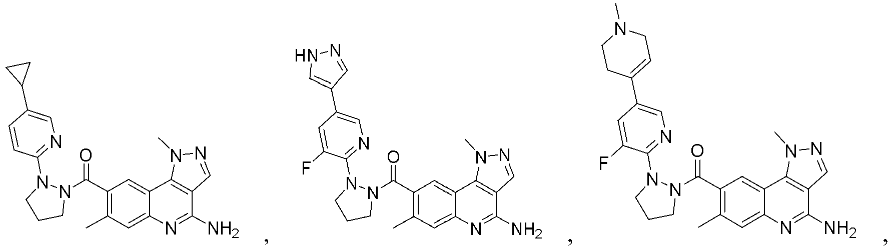

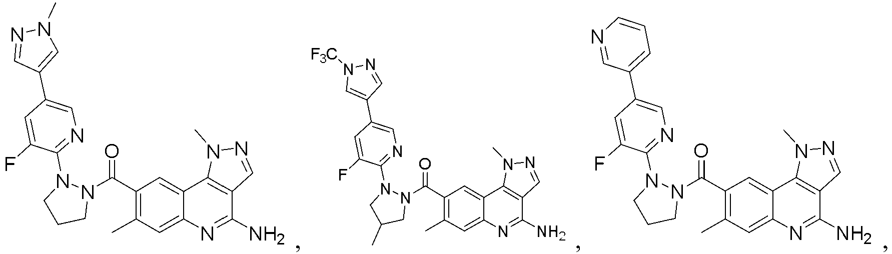

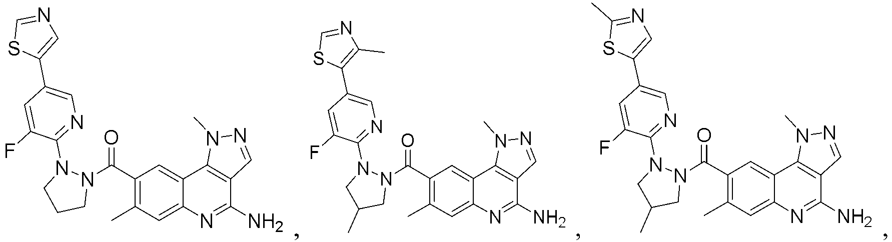

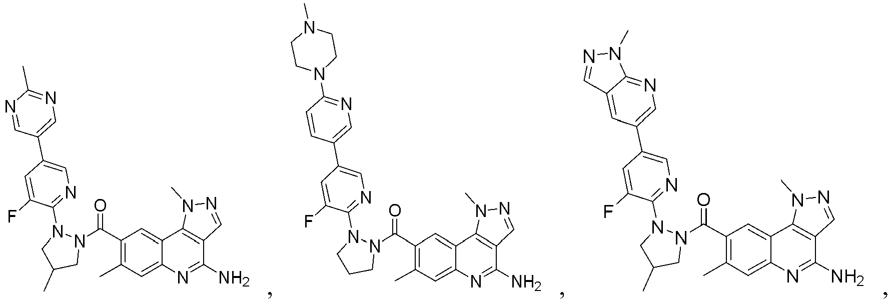

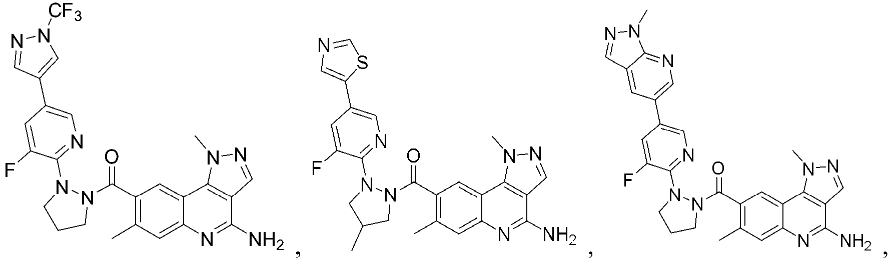

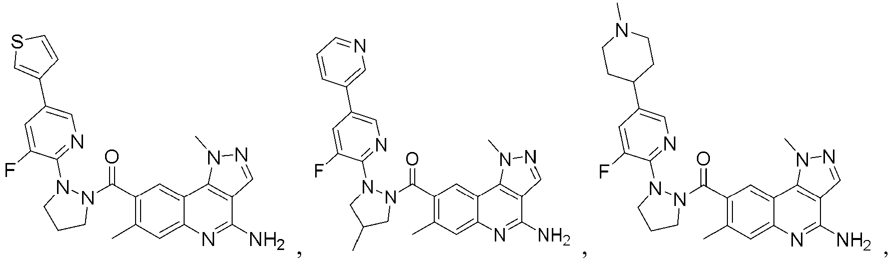

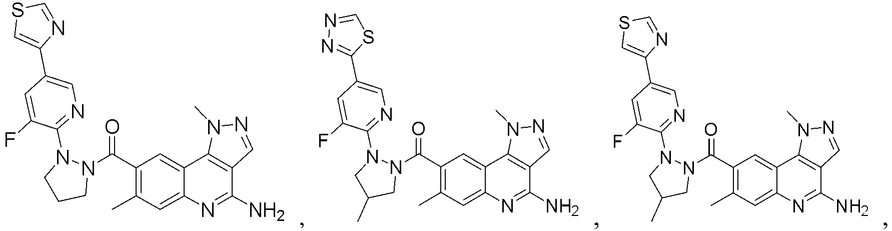

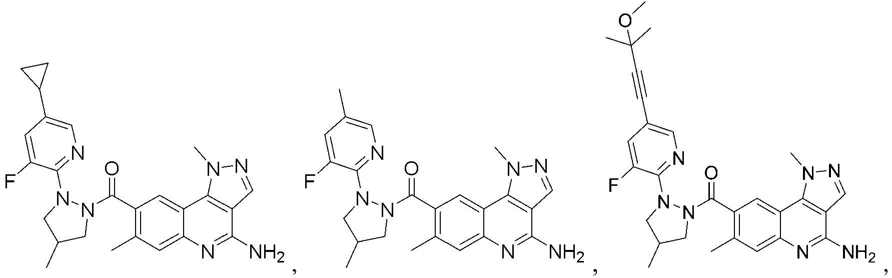

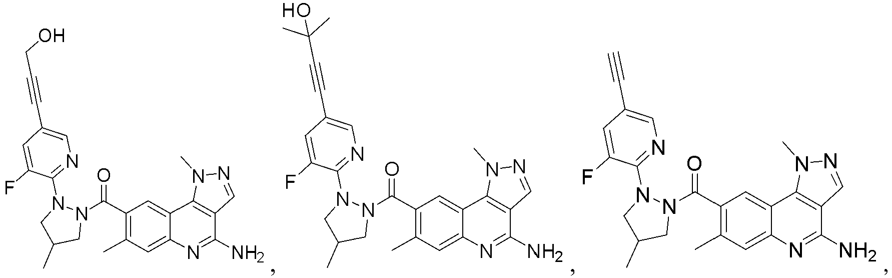

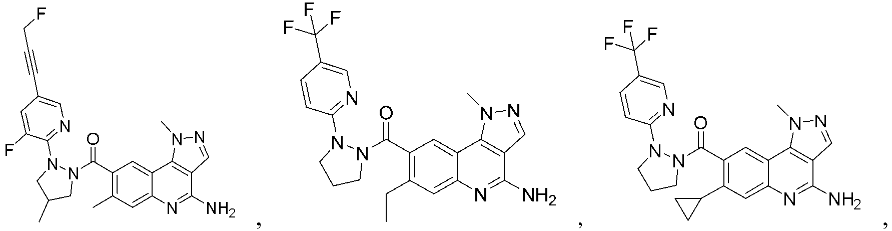

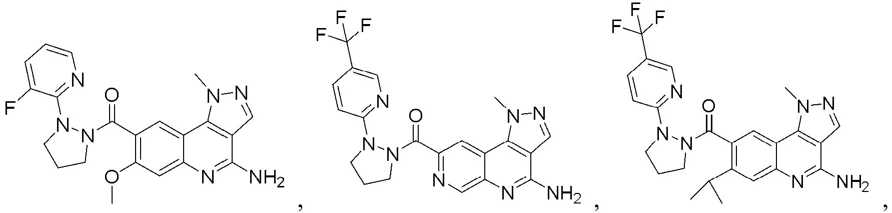

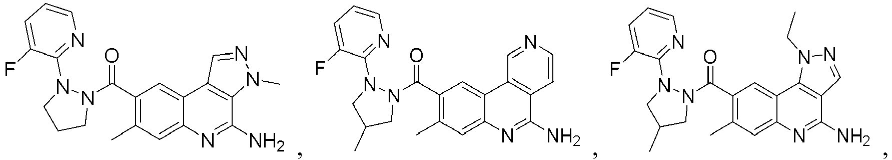

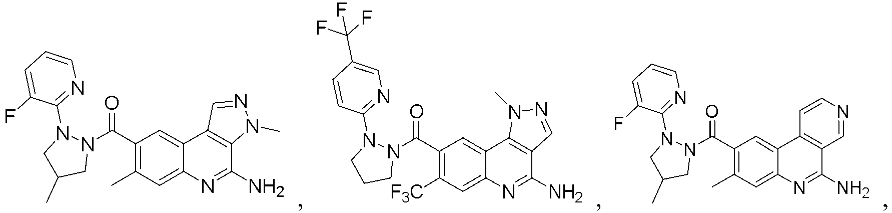

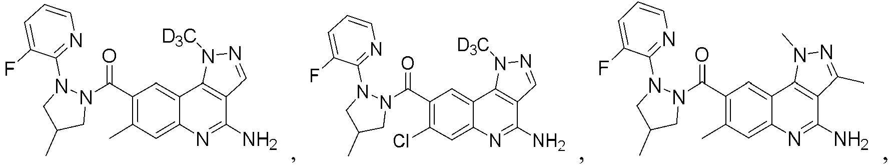

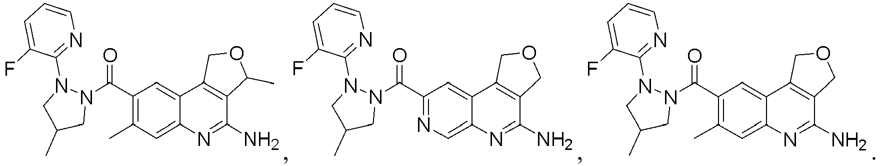

31. A pharmaceutical composition comprising the compound of general formula (I) or a mesomer, racemate, enantiomer, diastereomer thereof, or a mixture thereof, or a pharmaceutically acceptable salt thereof according to any one of claims 1 to 30, and a pharmaceutically acceptable carrier or excipient.

32. A use of the compound of general formula (I) or a mesomer, racemate, enantiomer, diastereomer thereof, or a mixture thereof, or a pharmaceutically acceptable salt thereof according to any one of claims 1 to 30 or the pharmaceutical composition according to claim 31 in the preparation of a PRMT5 inhibitor.

33. A use of the compound of general formula (I) or a mesomer, racemate, enantiomer, diastereomer thereof, or a mixture thereof, or a pharmaceutically acceptable salt thereof according to any one of claims 1 to 30 or the pharmaceutical composition according to claim 31 in the preparation of a medicament for the prevention or/and treatment of a PRMT5-mediated disease, preferably the disease is a disease related to cancer or tumor, and preferably the disease is bladder cancer.
